# EUROPEAN PATENT SPECIFICATION

(11) **EP 3 359 568 B1**
(45) Date of publication and mention of the grant of the patent: **09.03.2022**
(21) Application number: 16774932.4
(22) Date of filing: 29.09.2016
(51) Int. Cl.: C07K 16/28, C07K 16/30, C07K 16/40

(54) **BISPECIFIC ANTIBODIES WITH TETRAVALENCY FOR A COSTIMULATORY TNF RECEPTOR**
BISPEZIFISCHE ANTIKÖRPER MIT VIERWERTIGKEIT FÜR EINEN CO-STIMULIERENDEN TNF-REZEPTOR
ANTICORPS BISPÉCIFIQUES AVEC TÉTRAVALENCE POUR UN RÉCEPTEUR DE TNF COSTIMULANT

(30) Priority: 07.10.2015 EP 15188809
(43) Date of publication of application: 15.08.2018
(73) Proprietor: F. Hoffmann-La Roche AG, 4070 Basel (CH)
(72) Inventor: AMANN, Maria, 8952 Schlieren (CH); BRUENKER, Peter, 8952 Schlieren (CH); CLAUS, Christina, 8952 Schlieren (CH); FERRARA KOLLER, Claudia, 8952 Schlieren (CH); GRAU-RICHARDS, Sandra, 8952 Schlieren (CH); HOSSE, Ralf, 8952 Schlieren (CH); KLEIN, Christian, 8952 Schlieren (CH); LEVITSKI, Viktor, 8952 Schlieren (CH); MOSER, Samuel, 8952 Schlieren (CH); UMAÑA, Pablo, 8952 Schlieren (CH)
(74) Representative: Klostermeyer-Rauber, Dörte
(86) International application number: PCT/EP2016/073198
(87) International publication number: WO 2017/060144

(56) References cited:
- WO-A1-2013/164694
- WO-A2-2014/116846
- Agnes Wyzgol: "Generierung und Charakterisierung rekombinanter TNF-Liganden", , 1 January 2012 (2012-01-01), XP055250983, Retrieved from the Internet: URL:https://opus.bibliothek.uni-wuerzburg. de/files/6444/Dissertation_Agnes_Wyzgol.pd f [retrieved on 2016-02-17]
- Nora Hornig: "Combinations of costimulatory antibody-ligand fusion proteins for targeted cancer immunotherapy", , 14 October 2013 (2013-10-14), XP055250938, Retrieved from the Internet: URL:http://elib.uni-stuttgart.de/opus/voll texte/2013/8749/pdf/Thesis_Nora_Hornig.pdf [retrieved on 2016-02-17]
- MÜLLER DAFNE ET AL: "A novel antibody-4-1BBL fusion protein for targeted costimulation in cancer immunotherapy", JOURNAL OF IMMUNOTHERAPY, LIPPINCOTT WILLIAMS & WILKINS, USA, vol. 31, no. 8, 1 October 2008 (2008-10-01), pages 714-722, XP009183889, ISSN: 1537-4513
- Marlon J Hinner ET AL: "Costimulatory T cell engagement via a novel bispecific anti-CD137/anti-HER2 protein based on Anticalin technology", , 18 September 2015 (2015-09-18), XP055250958, Retrieved from the Internet: URL:http://c.eqcdn.com/pierisag/db/164/197 5/pdf/150914 CRI Poster postFinal.pdf [retrieved on 2016-02-17]
- KO K ET AL: "TREATMENT OF ADVANCED TUMORS WITH AGONISTIC ANTI-GITR MAB AND ITS EFFECTS ON TUMOR-INFILTRATING FOXP3(+)CD25(+)CD4(+) REGULATORY T CELLS", THE JOURNAL OF EXPERIMENTAL MEDICINE, ROCKEFELLER UNIVERSITY PRESS, US, vol. 202, no. 7, 3 October 2005 (2005-10-03), pages 885-891, XP009076415, ISSN: 0022-1007, DOI: 10.1084/JEM.20050940
- A. WYZGOL ET AL: "Trimer Stabilization, Oligomerization, and Antibody-Mediated Cell Surface Immobilization Improve the Activity of Soluble Trimers of CD27L, CD40L, 41BBL, and Glucocorticoid-Induced TNF Receptor Ligand", THE JOURNAL OF IMMUNOLOGY, vol. 183, no. 3, 1 August 2009 (2009-08-01), pages 1851-1861, XP055015511, ISSN: 0022-1767, DOI: 10.4049/jimmunol.0802597
- WEINBERG A D ET AL: "Engagement of the OX-40 receptor in vivo enhances antitumor immunity", THE JOURNAL OF IMMUNOLOGY, THE AMERICAN ASSOCIATION OF IMMUNOLOGISTS, US, vol. 164, no. 4, 15 February 2000 (2000-02-15), pages 2160-2169, XP002224132, ISSN: 0022-1767
- STEFANIE N. LINCH ET AL: "OX40 agonists and combination therapy: putting the pedal to the metal", FRONTIERS IN ONCOLOGY, vol. 2, no. 1, 16 February 2015 (2015-02-16), page 143, XP055326958, DOI: 10.3389/fonc.2012.00143

## Description

### FIELD OF THE INVENTION

The invention relates to novel bispecific antigen binding molecules, comprising (a) four Fab fragments capable of specific binding to OX40, (b) at least one moiety capable of specific binding to Fibroblast Activation Protein (FAP) comprising an antibody light chain variable region (VL) and an antibody heavy chain variable region (VH), and (c) a Fc domain composed of a first and a second subunit capable of stable association, wherein the Fc domain is of human IgGI subclass with the amino acid mutations L234A, L235A and P329G (numbering according to Kabat EU index), and wherein each of the Fab fragments capable of specific binding to OX40 comprises a VH domain comprising CDR-H1 comprising the amino acid sequence of SEQ ID NO:2, CDR-H2 comprising the amino acid sequence of SEQ ID NO:4, CDR-H3 comprising the amino acid sequence of SEQ ID NO:7 and a VL domain comprising CDR-L1 comprising the amino acid sequence of SEQ ID NO: 13, CDR-L2 comprising the amino acid sequence of SEQ ID NO:16 and CDR-L3 comprising the amino acid sequence of SEQ ID NO:20, and wherein a first Fab fragment capable of specific binding to OX40 is fused at the C-terminus of the CH1 domain to the VH domain of a second Fab fragment capable of specific binding to OX40 and a third Fab fragment capable of specific binding to OX40 is fused at the C-terminus of the CH1 domain to the VH domain of a fourth Fab fragment capable of specific binding to OX40, optionally via a peptide linker and wherein each two of the four Fab fragments capable of specific binding to OX40 are further fused at its C-terminus to the N-terminus of one of the subunits of the Fc domain, optionally via a peptide linker, and wherein the VH domain of the moiety capable of specific binding to FAP is connected via a peptide linker to the C-terminus of the first subunit of the Fc domain and the VL domain is connected via a peptide linker to the C-terminus of the second subunit of the Fc domain or wherein two moieties capable of specific binding to FAP are VH-VL crossover Fab fragments and are each fused at the N-terminus of the VL domain via a peptide linker to the C-terminus of one of the subunits of the Fc domain. The invention further relates to methods of producing these molecules and to methods of using the same.

### BACKGROUND

Several members of the tumor necrosis factor receptor (TNFR) superfamily function after initial T cell activation to sustain T cell responses and thus have pivotal roles in the organization and function of the immune system. CD27, 4-1BB (CD137), OX40 (CD134), HVEM, CD30, and GITR can have costimulatory effects on T cells, meaning that they sustain T-cell responses after initial T cell activation (Watts T.H. (2005) Annu. Rev. Immunol. 23, 23-68). The effects of these costimulatory TNFR superfamily members (herein called TNFR family members) can often be functionally, temporally, or spatially segregated from those of CD28 and from each other. The sequential and transient regulation of T cell activation/survival signals by different costimulators may function to allow longevity of the response while maintaining tight control of T cell survival. Depending on the disease condition, stimulation via costimulatory TNF superfamily members can exacerbate or ameliorate disease. Despite these complexities, stimulation or blockade of TNFR family costimulators shows promise for several therapeutic applications, including cancer, infectious disease, transplantation, and autoimmunity.

Among several costimulatory molecules, the tumor necrosis factor (TNF) receptor superfamily member OX40 (CD134) plays a key role in the survival and homeostasis of effector and memory T cells (Croft M. et al. (2009), Immunological Reviews 229, 173-191). OX40 (CD134) is expressed in several types of cells and regulates immune responses against infections, tumors and self-antigens and its expression has been demonstrated on the surface of T-cells, NKT-cells and NK-cells as well as neutrophils (Baumann R. et al. (2004), Eur. J. Immunol. 34, 2268-2275) and shown to be strictly inducible or strongly upregulated in response to various stimulatory signals. Functional activity of the molecule has been demonstrated in every OX40-expressing cell type suggesting complex regulation of OX40-mediated activity *in vivo.* Combined with T-cell receptor triggering, OX40 engagement on T-cells by its natural ligand or agonistic antibodies leads to synergistic activation of the PI3K and NFκB signalling pathways (Song J. et al. (2008) J. Immunology 180(11), 7240-7248). In turn, this results in enhanced proliferation, increased cytokine receptor and cytokine production and better survival of activated T-cells. In addition to its co-stimulatory activity in effector CD4⁺ or CD8⁺ T-cells, OX40 triggering has been recently shown to inhibit the development and immunosuppressive function of T regulatory cells. This effect is likely to be responsible, at least in part, for the enhancing activity of OX40 on anti-tumor or anti-microbial immune responses. Given that OX40 engagement can expand T-cell populations, promote cytokine secretion, and support T-cell memory, agonists including antibodies and soluble forms of the ligand OX40L have been used successfully in a variety of preclinical tumor models (Weinberg et al. (2000), J. Immunol. 164, 2160-2169).

Glucocorticoid-induced TNF receptor-related gene (GITR; TNFRSF18), another receptor in the TNF receptor superfamily, is considered as a marker for Tregs and activated effector T cells (Petrillo et al. (2015), Autoimmun. Rev. 14, 117-26). GITR triggering induces both pro- and anti-apototic effects, abrogates the suppressive activity of Treg cells and costimulates responder T cells, with the latter activities over-stimulating the immune system (Nocentini et al. (2005), Eur. J. Immunol. 35, 1016-1022). It has been shown that an agonistic monoclonal antibody against murine GITR effectively induced tumor-specific immunity and reduced established tumors in a mouse syngeneic tumor model (Ko et al. (2005), J. Exp. Med. 202, 885-891). There is however a structural difference between murine and human GITR. Murine GITR is a dimer whereas human GITR is a trimer. Based on this structural complexicity it has been suggested that anti-GITR antibodies per se are poor GITR agonists and that for the activation of CD4⁺ and CD8⁺ T cells increased FcR effector function is necessary. Thus, there is a need for the provision of anti-GITR antibodies in a new format that is independent from increased FcR effector function and neverthe less able to activate trimeric human GITR.

4-1BB (CD137), a member of the TNF receptor superfamily, has been first identified as a molecule whose expression is induced by T-cell activation (Kwon Y.H. and Weissman S.M. (1989), Proc. Natl. Acad. Sci. USA 86, 1963-1967). Subsequent studies demonstrated expression of 4-1BB in T- and B-lymphocytes (Snell L.M. et al. (2011) Immunol. Rev. 244, 197-217 or Zhang X.et al. (2010), J. Immunol. 184, 787-795), NK-cells (Lin W. et al. (2008), Blood 112, 699-707, NKT-cells (Kim D.H. et al. (2008), J. Immunol. 180, 2062-2068), monocytes (Kienzle G. and von Kempis J. (2000), Int. Immunol. 12, 73-82, or Schwarz H. et al. (1995), Blood 85, 1043-1052), neutrophils (Heinisch I.V. et al. (2000), Eur. J. Immunol. 30, 3441-3446), mast (Nishimoto H. et al. (2005), Blood 106, 4241-4248), and dendritic cells as well as cells of non-hematopoietic origin such as endothelial and smooth muscle cells (Broll K. et al. (2001), Am. J. Clin. Pathol. 115, 543-549 or Olofsson P.S. et al. (2008), Circulation 117, 1292-1301). Expression of 4-1BB in different cell types is mostly inducible and driven by various stimulatory signals, such as T-cell receptor (TCR) or B-cell receptor triggering, as well as signaling induced through co-stimulatory molecules or receptors of pro-inflammatory cytokines (Diehl L. et al. (2002), J. Immunol. 168, 3755-3762; von Kempis J. et al. (1997), Osteoarthritis Cartilage 5, 394-406; Zhang X.et al. (2010), J. Immunol. 184, 787-795).

CD137 signaling is known to stimulate IFNγ secretion and proliferation of NK cells (Buechele C. et al. (2012), Eur. J. Immunol. 42, 737-748; Lin W. et al. (2008), Blood 112, 699-707; Melero I. et al. (1998), Cell Immunol. 190, 167-172) as well as to promote DC activation as indicated by their increased survival and capacity to secret cytokines and upregulate costimulatory molecules (Choi B. K. et al. (2009), J. Immunol. 182, 4107-4115; Futagawa T. et al. (2002), Int. Immunol. 14, 275-286; Wilcox R. A. et al. (2002), J. Immunol. 168, 4262-4267). However, CD137 is best characterized as a co-stimulatory molecule which modulates TCR-induced activation in both the CD4+ and CD8+ subsets of T-cells. In combination with TCR triggering, agonistic 4-1BB-specific antibodies enhance proliferation of T-cells, stimulate lymphokine secretion and decrease sensitivity of T-lymphocytes to activation-induced cells death (Snell L.M. et al. (2011) Immunol. Rev. 244, 197-217). In line with these co-stimulatory effects of 4-1BB antibodies on T-cells in vitro, their administration to tumor bearing mice leads to potent anti-tumor effects in many experimental tumor models (Melero I. et al. (1997), Nat. Med. 3, 682-685; Narazaki H. et al. (2010), Blood 115, 1941-1948). In vivo depletion experiments demonstrated that CD8+ T-cells play the most critical role in anti-tumoral effect of 4-1BB-specific antibodies. However, depending on the tumor model or combination therapy, which includes anti-4-1BB, contributions of other types of cells such as DCs, NK-cells or CD4+ T-cells have been reported (Murillo O. et al. (2009), Eur. J. Immunol. 39, 2424-2436; Stagg J. et al. (2011), Proc. Natl. Acad. Sci. USA 108, 7142-7147).

In addition to their direct effects on different lymphocyte subsets, 4-1BB agonists can also induce infiltration and retention of activated T-cells in the tumor through 4-1BB-mediated upregulation of intercellular adhesion molecule 1 (ICAM1) and vascular cell adhesion molecule 1 (VCAM1) on tumor vascular endothelium (Palazon A. et al. (2011), Cancer Res. 71, 801-811). 4-1BB triggering may also reverse the state of T-cell anergy induced by exposure to soluble antigen that may contribute to disruption of immunological tolerance in the tumor microenvironment or during chronic infections (Wilcox R.A. et al. (2004), Blood 103, 177-184).

It appears that the immunomodulatory properties of 4-1BB agonistic antibodies in vivo require the presence of the wild type Fc-portion on the antibody molecule thereby implicating Fc-receptor binding as an important event required for the pharmacological activity of such reagents as has been described for agonistic antibodies specific to other apoptosis-inducing or immunomodulatory members of the TNFR-superfamily (Li F. and Ravetch J.V. (2011), Science 333, 1030-1034; Teng M.W. et al. (2009), J. Immunol. 183, 1911-1920). However, systemic administration of 4-1BB-specific agonistic antibodies with the functionally active Fc domain also induces expansion of CD8+ T-cells associated with liver toxicity (Dubrot J. et al. (2010), Cancer Immunol. Immunother. 59, 1223-1233) that is diminished or significantly ameliorated in the absence of functional Fc-receptors in mice. In human clinical trials (ClinicalTrials.gov, NCT00309023), Fc-competent 4-1BB agonistic antibodies (BMS-663513) administered once every three weeks for 12 weeks induced stabilization of the disease in patients with melanoma, ovarian or renal cell carcinoma. However, the same antibody given in another trial (NCT00612664) caused grade 4 hepatitis leading to termination of the trial (Simeone E. and Ascierto P.A. (2012), J. Immunotoxicology 9, 241-247). Thus, there is a need for new generation agonists that should not only effectively engage 4-1BB on the surface of hematopoietic and endothelial cells but also be capable of achieving that through mechanisms other than binding to Fc-receptors in order to avoid uncontrollable side effects.

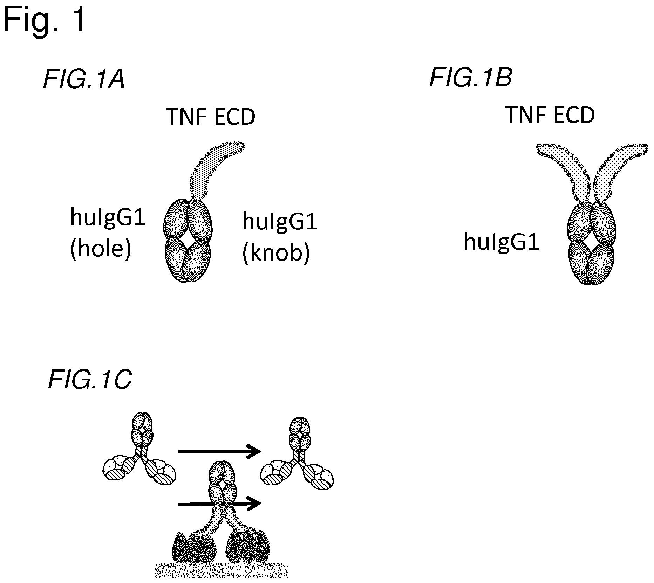

Agnes Wyzgol in her Ph.D. thesis "Generierung und Charakterisierung rekombinanter TNF-Liganden" provides fusion proteins comprising a FAP scFv domain and OX40L(52-183) or fusion proteins comprising a hu IgG1 Fc domain and OX40L(52-183). A fusion protein comprising a FAP scFv domain (scFv36) and OX40L or a fusion protein comprising a EDG (A5) scFv domain and OX40L are also described in the Ph.D. thesis "Combinations of costimulatory antibody-ligand fusion proteins for targeted cancer immunotherapy" by Nora Hornig. Müller et al., J. Immunotherapy 2008, 31(8), 714-722, disclose a fusion protein scFv36-4-1BBL (see Fig. 1) which can form a trimer as 4-1BBL can trimerize. Hinner et al. presented a "costimulatory T-cell engagement via a novel bispecific anti-CD 137 /anti-HER2 protein based on Anticalin^{®} technology" on September 2016 on a CRI cancer immunotherapy conference. WO 2014/116846 discloses multispecific binding proteins comprising a first binding site that specifically binds to a target cell antigen, a second binding site that specifically binds to a cell surface receptor on an immune cell, and a third binding site that specifically binds to a cell surface modulator on the immune cell. WO 2013/164694 relates to immunomodulatory fusion proteins comprising a targeting moiety fused with a immunomodulatory moiety.

The available pre-clinical and clinical data clearly demonstrate that there is a high clinical need for effective agonists of costimulatory TNFR family members such as OX40 and 4-1BB that are able to induce and enhance effective endogenous immune responses to cancer. However, almost never are the effects limited to a single cell type or acting via a single mechanism and studies designed to elucidate inter- and intracellular signaling mechanisms have revealed increasing levels of complexity. Thus, there is a need of "targeted" agonists that preferably act on a single cell type. The antigen binding molecules of the invention combine a moiety capable of preferred binding to tumor-specific or tumor-associated targets with a moiety capable of agonistic binding to costimulatory TNF receptors. The antigen binding molecules of this invention may be able to trigger TNF receptors not only effectively, but also very selectively at the desired site thereby reducing undesirable side effects.

### SUMMARY OF THE INVENTION

The present invention relates to bispecific antigen binding molecules combining four moieties capable of specific binding to OX40, i.e four antigen binding sites that target OX40, with at least one moiety capable of specific binding to Fibroblast Activation Protein (FAP), i.e. with at least one antigen binding side targeting FAP. These bispecific antigen binding molecules are advantageous as they will preferably activate OX40 at the site where FAP is expressed, due to their binding capability towards FAP.

In one aspect, the invention provides a bispecific antigen binding molecule, comprising
(a) four Fab fragments capable of specific binding to OX40,
(b) at least one moiety capable of specific binding to Fibroblast Activation Protein (FAP) comprising an antibody light chain variable region (VL) and an antibody heavy chain variable region (VH), and
(c) a Fc domain composed of a first and a second subunit capable of stable association, wherein the Fc domain is of human IgG1 subclass with the amino acid mutations L234A, L235A and P329G (numbering according to Kabat EU index), and
wherein each of the Fab fragments capable of specific binding to OX40 comprises a VH domain comprising CDR-H1 comprising the amino acid sequence of SEQ ID NO:2, CDR-H2 comprising the amino acid sequence of SEQ ID NO:4, CDR-H3 comprising the amino acid sequence of SEQ ID NO:7 and a VL domain comprising CDR-L1 comprising the amino acid sequence of SEQ ID NO: 13, CDR-L2 comprising the amino acid sequence of SEQ ID NO: 16 and CDR-L3 comprising the amino acid sequence of SEQ ID NO:20, and wherein a first Fab fragment capable of specific binding to OX40 is fused at the C-terminus of the CH1 domain to the VH domain of a second Fab fragment capable of specific binding to OX40 and a third Fab fragment capable of specific binding to OX40 is fused at the C-terminus of the CH1 domain to the VH domain of a fourth Fab fragment capable of specific binding to OX40, optionally via a peptide linker and wherein each two of the four Fab fragments capable of specific binding to OX40 are further fused at its C-terminus to the N-terminus of one of the subunits of the Fc domain, optionally via a peptide linker, and
wherein the VH domain of the moiety capable of specific binding to FAP is connected via a peptide linker to the C-terminus of the first subunit of the Fc domain and the VL domain is connected via a peptide linker to the C-terminus of the second subunit of the Fc domain or wherein two moieties capable of specific binding to FAP are VH-VL crossover Fab fragments and are each fused at the N-terminus of the VL domain via a peptide linker to the C-terminus of one of the subunits of the Fc domain.

Particularly, a bispecific antigen binding molecule is provided, wherein each of the Fab fragments capable of specific binding to OX40 comprises a heavy chain variable region VH comprising an amino acid sequence of SEQ ID NO:27 and a light chain variable region VL comprising an amino acid sequence of SEQ ID NO:28.

In a particular aspect, provided is a bispecific antigen binding molecule, wherein the moiety capable of specific binding to FAP comprises a VH domain comprising
(i) a CDR-H1 comprising the amino acid sequence selected from the goup consisting of SEQ ID NO:39and SEQ ID NO:40,
(ii) a CDR-H2 comprising the amino acid sequence selected from the group consisting of SEQ ID NO:41 and SEQ ID NO:42, and
(iii) a CDR-H3 comprising the amino acid sequence selected from the group consisting of SEQ ID NO:43 and SEQ ID NO:44,
   and a VL domain comprising
(iv) a CDR-L1 comprising the amino acid sequence selected from the group consisting of SEQ ID NO:45 and SEQ ID NO:46,
(v) a CDR-L2 comprising the amino acid sequence selected from the group consisting of SEQ ID NO:47 and SEQ ID NO:48, and
(vi) a CDR-L3 comprising the amino acid sequence selected from the group consisting of SEQ ID NO:49 and SEQ ID NO:50.

In another aspect, provided is a bispecific antigen binding molecule, wherein the moiety capable of specific binding to FAP comprises a VH domain comprising comprising an amino acid sequence of SEQ ID NO:51 or SEQ ID NO:53 and a light chain variable region comprising an amino acid sequence of SEQ ID NO:52 or SEQ ID NO:54.

In a further aspect, the invention thus provides a bispecific antigen binding molecule, wherein
(i) each of the Fab fragments capable of specific binding to OX40 comprises a heavy chain variable region VH comprising an amino acid sequence of SEQ ID NO: 27 and a light chain variable region comprising an amino acid sequence of SEQ ID NO: 28 and
(ii) the moiety capable of specific binding to FAP comprises a heavy chain variable region VH of SEQ ID NO:51 or SEQ ID NO:53 and a light chain variable region comprising an amino acid sequence of SEQ ID NO:52 or SEQ ID NO:54.

In another aspect, the invention provides a bispecific antigen binding molecule, wherein in the Fab fragments capable of specific binding to a costimulatory TNF receptor family member in the constant domain CL the amino acid at position 124 is substituted independently by lysine (K), arginine (R) or histidine (H) (numbering according to Kabat EU Index), and in the constant domain CH1 the amino acids at positions 147 and 213 are substituted independently by glutamic acid (E) or aspartic acid (D) (numbering according to Kabat EU index).

In a further aspect, the invention relates to a bispecific antigen binding molecule as described herein before, wherein the bispecific antigen binding molecule is tetravalent for OX40 and monovalent for FAP.

In one aspect, provided is a bispecific antigen binding molecule, wherein the moiety capable of specific binding to FAP comprises a VH and VL domain and wherein the VH domain is connected via a peptide linker to the C-terminus of the first subunit of the Fc domain and the VL domain is connected via a peptide linker to the C-terminus of the second subunit of the Fc domain.

In another aspect, the invention provides a bispecific antigen binding, wherein the Fc domain comprises a modification promoting the association of the first and second subunit of the Fc domain. In particular, the first subunit of the Fc domain comprises knobs and the second subunit of the Fc domain comprises holes according to the knobs into holes method. More particularly, the first subunit of the Fc domain comprises the amino acid substitutions S354C and T366W (numbering according to Kabat EU index) and the second subunit of the Fc domain comprises the amino acid substitutions Y349C, T366S and Y407V (numbering according to Kabat EU index).

In another aspect, provided is a bispecific antigen binding molecule of the invention, wherein said antigen binding molecule comprises
(i) a first heavy chain comprising an amino acid sequence of SEQ ID NO:213, a second heavy chain comprising an amino acid sequence of SEQ ID NO:214, and a light chain comprising an amino acid sequence of SEQ ID NO.157, or
(ii) a first heavy chain comprising an amino acid sequence of SEQ ID NO:217, a second heavy chain comprising an amino acid sequence of SEQ ID NO:218, and a light chain comprising an amino acid sequence of SEQ ID NO.157.

In addition, the invention relates to a bispecific antigen binding molecule, wherein the bispecific antigen binding molecule is tetravalent for OX40 member and bivalent for FAP.

In one aspect, provided is a bispecific antigen binding molecule, wherein the the two moieties capable of specific binding to FAP are Fab fragments or crossover Fab fragments.

In a further aspect, provided is a bispecific antigen binding molecule, wherein each of the Fab fragments or crossover Fab fragments capable of specific binding to FAP is fused at the N-terminus of the VH or VL domain via a peptide linker to the C-terminus of one of the subunits of the Fc domain.

In a particular aspect, provided is a bispecific antigen binding molecule, wherein the two moieties capable of specific binding to FAP are VH-VL crossover Fab fragments and are each fused at the N-terminus of the VL domain via a peptide linker to the C-terminus of one of the subunits of the Fc domain.

According to another aspect of the invention, there is provided an isolated polynucleotide encoding a bispecific antigen binding molecule as described herein before. The invention further provides a vector, particularly an expression vector, comprising the isolated polynucleotide of the invention and a host cell comprising the isolated polynucleotide or the vector of the invention. In some aspects the host cell is a eukaryotic cell, particularly a mammalian cell.

In another aspect, provided is a method for producing a bispecific antigen binding molecule as described herein before, comprising the steps of (i) culturing the host cell of the invention under conditions suitable for expression of the antigen binding molecule, and (ii) recovering the antigen binding molecule.

The invention further provides a pharmaceutical composition comprising a bispecific antigen binding molecule as described herein before and at least one pharmaceutically acceptable excipient.

Also encompassed by the invention is the bispecific antigen binding molecule as described herein before, or the pharmaceutical composition comprising the bispecific antigen binding molecule, for use as a medicament.

In a specific embodiment, provided is the bispecific antigen binding molecule as described herein before or the pharmaceutical composition of the invention, for use in the treatment of cancer. In another specific aspect, the invention provides the bispecific antigen binding molecule as described herein before for use in the treatment of cancer, wherein the bispecific antigen binding molecule is administered in combination with a chemotherapeutic agent, radiation and/ or other agents for use in cancer immunotherapy. Any references in the description to methods of treatment refer to the compounds, pharmaceutical compositions and medicaments of the present invention for use in a method for treatment of the human (or animal) body by therapy (or for diagnosis).

### BRIEF DESCRIPTION OF THE DRAWINGS

**Figure 1A** shows the monomeric form of Fc-linked TNF receptor antigen that was used for the preparation of TNF receptor antibodies. **Figure 1B** shows a dimeric human TNF receptor antigen Fc fusion molecule with a C-terminal Ha tag that was used for the testing of the binding of TNF receptor antibodies in the presence of TNF ligand (ligand blocking property). The schematic setup of the experiment described in Example 2.4 is shown in **Figure 1C**.

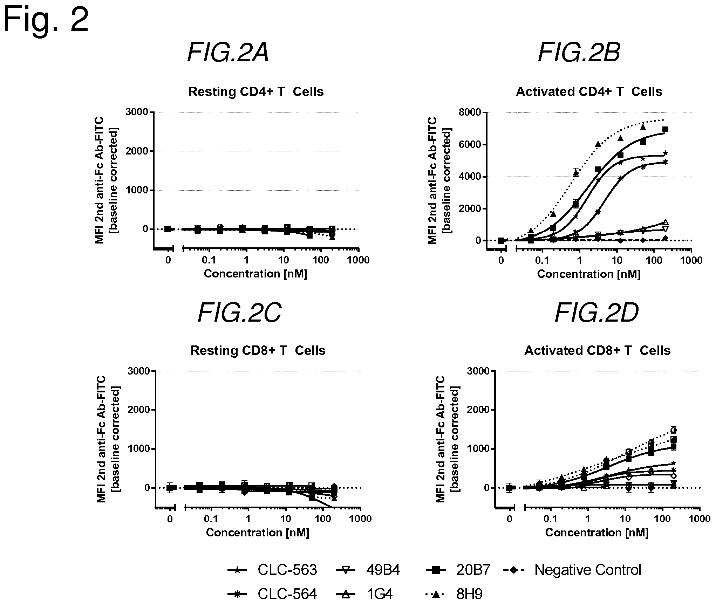

**Figures 2A-2D** show the binding of anti-OX40 antibodies to activated human CD4+ and CD8+ T cells. OX40 is not expressed on resting human PBMCs (Figures 2A and 2C). After activation of human PBMCs, OX40 is up-regulated on CD4⁺ and CD8⁺ T cells (Figures 2B and 2D). OX40 expression on human CD8⁺ T cells is lower than on CD4⁺ T cells. The depicted clones varied in their binding strength (EC₅₀ values as well as signal strength) to OX40 positive cells. Shown is the binding as median of fluorescence intensity (MFI) of FITC labeled anti-human IgG Fcy-specific goat IgG F(ab')2 fragment which is used as secondary detection antibody. MFI was measured by flow cytometry and baseline corrected by subtracting the MFI of the blank control. The x-axis shows the concentration of antibody constructs. All OX40 clones do bind to activated, OX40 expressing human CD4⁺ T cells, and to a lower extent to activated human CD8⁺ T cells

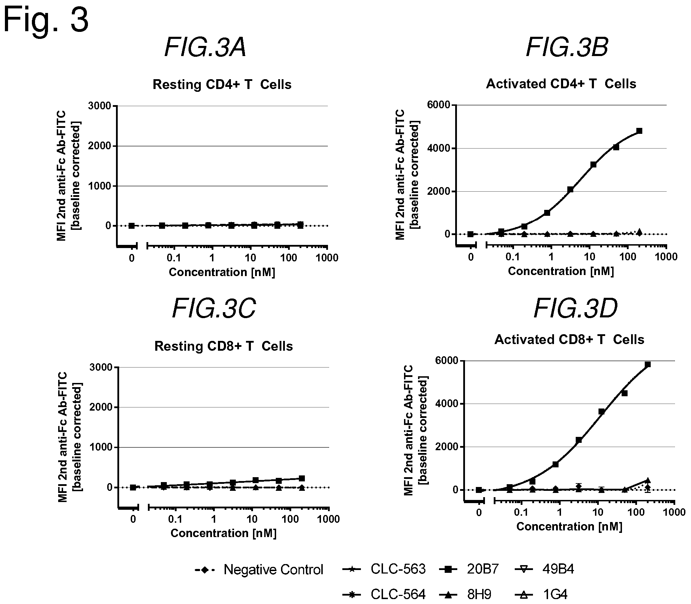

**Figures 3A-3D** show the binding of the anti-OX40 antibodies to activated mouse CD4+ and CD8⁺ T cells. OX40 was not detected on resting mouse splencoytes (Figures 3A and 3C). After activation OX40 is up-regulated on CD4⁺ and CD8⁺ T cells (Figures 3B and 3D). Mouse splenocytes were isolated by erythrolysis with ACK lysis buffer of mechanically-homogenized spleens obtained from 6-8 weeks old female C57BL/6 mice. Binding of anti-OX40 antibodies to cell surface proteins was detected with a goat anti-human IgG Fc-specific secondary antibody conjugated to FITC using FACS analysis. MFI was measured by flow cytometry and baseline corrected by subtracting the MFI of the blank control. The x-axis shows the concentration of antibody constructs. Only clone 20B7 does bind to activated, OX40 expressing mouse CD4⁺ and CD8⁺ T Cells, but not to resting T cells.

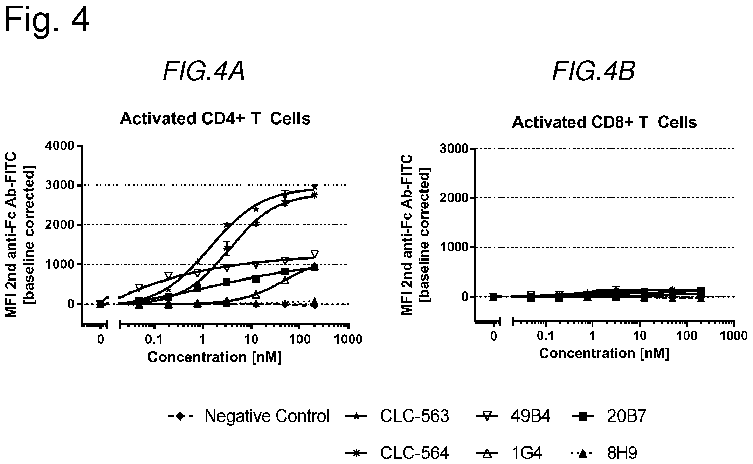

**Figures 4A and 4B** show the binding of anti-OX40 antibodies on cynomolgus activated CD4⁺ and CD8⁺ T cells, respectively. The depicted clones varied in their binding strength (EC₅₀ values as well as signal strength) to OX40 positive activated cynomolgus CD4+ T cells. OX40 expression on activated CD8+ T cells is low under this condition and hardly any binding of the selected clones was found. Binding of anti-OX40 antibodies to cell surface proteins was detected with a goat anti-human IgG Fc-specific secondary antibody conjugated to FITC using FACS analysis. MFI was measured by flow cytometry and baseline corrected by subtracting the MFI of the blank control. The x-axis shows the concentration of antibody constructs. All OX40 clones do bind to activated, OX40 expressing cynomolgus CD4⁺ T cells, and to a lower extent to activated cynomolgus CD8⁺ T cells.

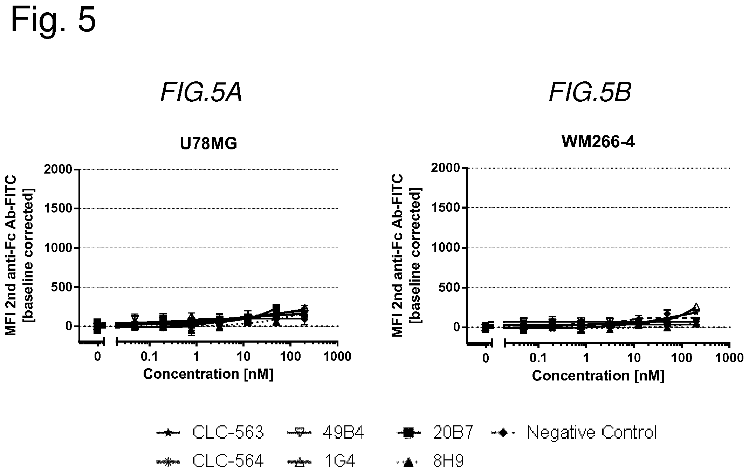

**Figures 5A and 5B** show the lack of binding to OX40 negative tumor cells. The depicted clones showed no binding to OX40 negative U-78 MG (Figure 5A) and WM266-4 tumor cells (Figure 5B). Shown is the binding as median of fluorescence intensity (MFI) of FITC labeled anti-human IgG Fcγ-specific goat IgG F(ab')2 fragment which is used as secondary detection antibody. MFI was measured by flow cytometry and baseline corrected by subtracting the MFI of the blank control. The x-axis shows the concentration of antibody constructs. All clones in an IgG format do not bind to OX40 negative tumor cells. Binding is specific for OX40 on activated leukocytes.

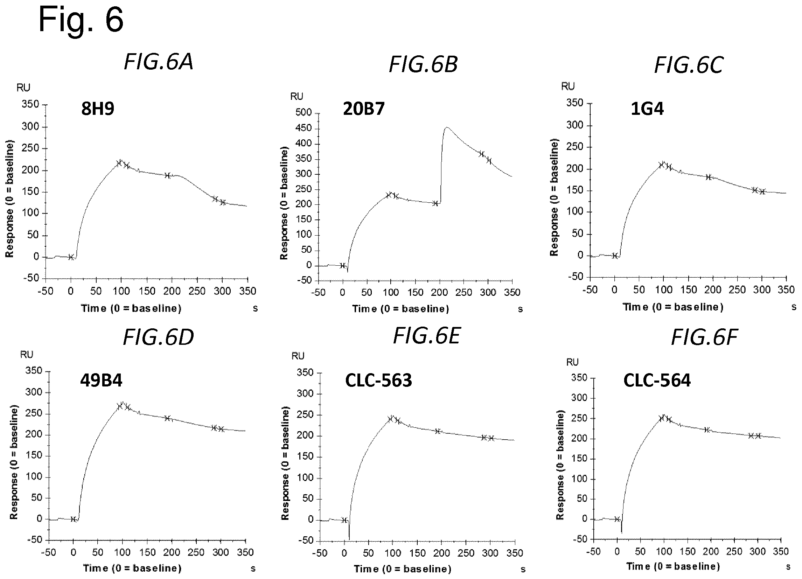

**Figures 6A to 6F** show the interaction between anti-Ox40 antibodies 8H9 (Figure 6A), 20 B7 (Figure 6B), 1G4 (Figure 6C), 49B4 (Figure 6D), CLC-563 (Figure 6E) and CLC-564 (Figure 6F) and the preformed complex huOx40 Ligand / huOx40-Fc as measured by surface plasmon resonance.

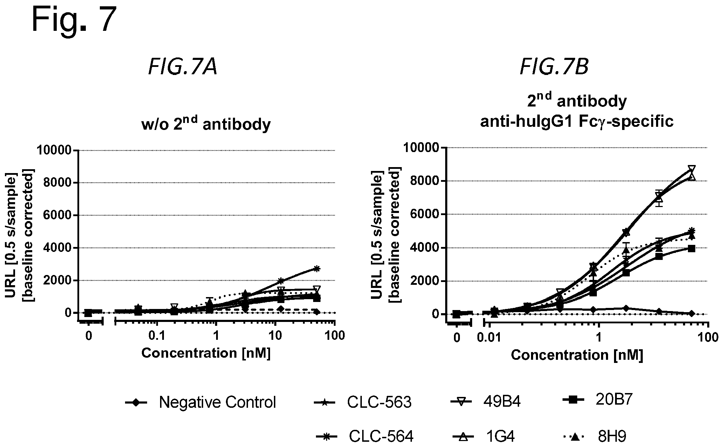

**Figures 7A and 7B** show the effect of the anti-human OX40 antibodies of the invention on HeLa cells expressing human OX40 and reporter gene NF-κB-luciferase. Shown is the activation of NF-κB signaling pathway in the reporter cell line with various anti-OX40 binders in a P329GLALA huIgG1 format w/ (Figure 7B) or w/o (Figure 7A) crosslinking by secondary-antibody. The reporter cells were cultured for 6 hours in the presence of anti-OX40 constructs at the indicated concentrations w/ or w/o crosslinking secondary poly-clonal anti-huIgG1 Fcγ-specific goat IgG F(ab)2 fragment in a 1:2 ratio. Activity is characterized by blotting the units of released light (URL) measured during 0.5 s versus the concentration in nM of tested anti-Ox40 construct. URLs are emitted due to luciferase-mediated oxidation of luciferin to oxyluciferin. All clones are able to induce NFκB activation when the OX40 axis is triggered in a human OX40⁺ reporter cell line. All clones are thus agonistic and activate in a dose dependent way. Crosslinking by secondary Fc part specific Abs strongly increases this agonism.

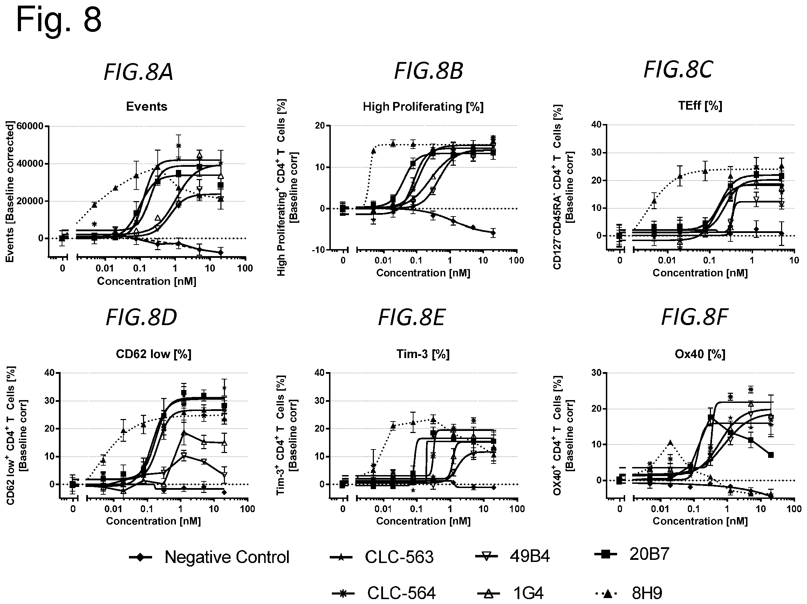

**Figures 8A to 8F** show the bioactivity of the anti-human OX40 antibodies in preactivated human CD4 T cells. Costimulation with plate-immobilized anti-OX40 binders (huIgG1 P329GLALA format) promoted cell proliferation and maturation of sub-optimally restimulated human CD4 T cells and induced an enhanced activated phenotype. PHA-L pre-activated CFSE-labeled human CD4 T cells were cultured for four days on plates pre-coated with mouse IgG Fcγ specific antibodies, human IgG Fcγ specific antibodies (both 2 µg/mL), mouse anti-human CD3 antibodies (clone OKT3, [3 ng/mL]) and titrated anti-Ox40 binders (huIgG1 P329GLALA format). Shown is the event count, the percentage of proliferating (CFSE-low) cells, the percentage of effector T cells (CD 127 low/CD45RA low) and the percentage of CD62L low, OX40 positive or Tim-3 positive cells at day 4. Baseline values of samples containing only the plate-immobilized anti-human CD3 were substracted. Therefore, the enhancing effect of Ox40 stimulation but not the effect of suboptimal anti-CD3 stimulation per se is visible here. All clones are able support suboptimal TCR stimulation in OX40 positive preactived CD4 T cells when they are coated to plate. Cells do survive better and proliferate more. In the tumor micro environment this could lead to increased anti-tumor activity of T cells.

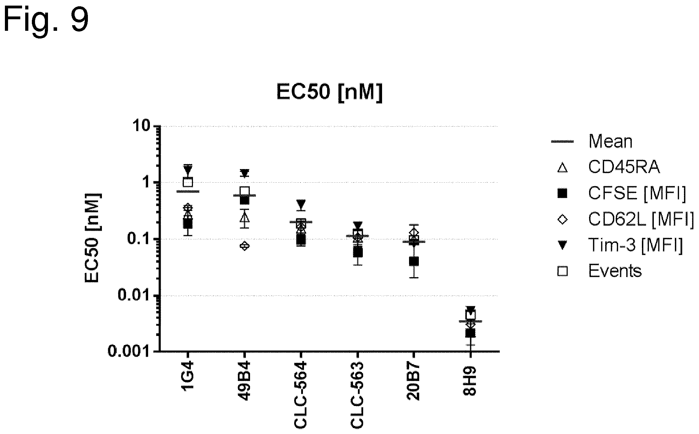

**Figure 9** summarizes the EC₅₀ values (for all biomarkers) as marker for the agonistic capacity of the respective clone (values calculated from the curves shown in Figures 8A-8F). The potency increases from left to right. The event count, the percentage of proliferating (CFSE-low) cells and the percentage of CD62L low, CD45RA low or Tim-3 positive cells at day 4 were plotted vs the anti-Ox40 antibody concentration and EC₅₀ values were calculated using the inbuilt sigmoidal dose response quotation in Prism4 (GraphPad Software, USA).

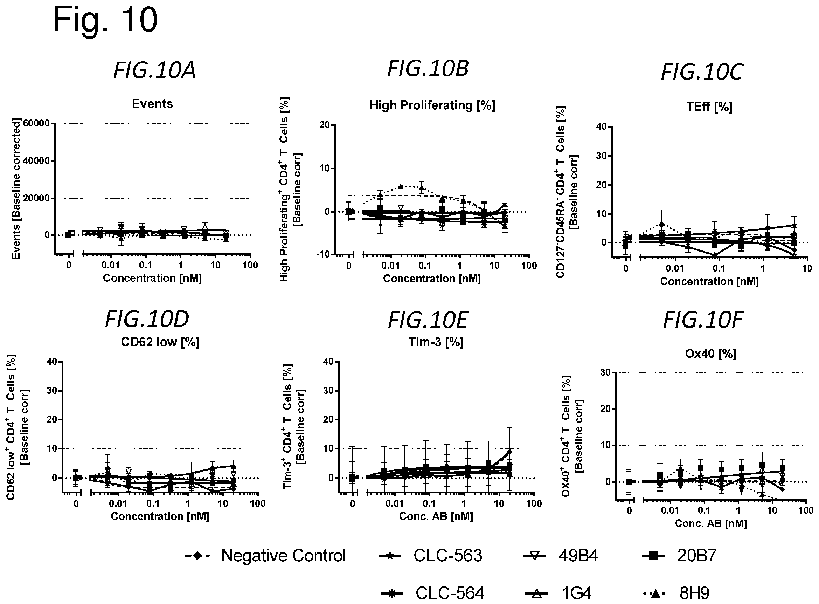

**Figures 10A to 10F** show the bioactivity of the anti- human OX40 antibodies in preactivated human CD4 T cells in solution. No effect on cell proliferation, maturation or activation status of sub-optimally restimulated human CD4 T cells was detected in the absence of plate immobilization of anti-Ox40 binders (hu IgG1 P329GLALA format). PHA-L pre-activated CFSE-labeled human CD4 T cells were cultured for four days on plates pre-coated with mouse IgG Fcγ specific antibodies and mouse anti-human CD3 antibodies (clone OKT3, [3 ng/mL]). Titrated anti-Ox40 binders (hu IgG1 P329GLALA format) were added to the media and were present in solution throughout the experiment. Shown is the event count, the percentage of proliferating (CFSE-low) cells, the percentage of effector T cells (CD127low CD45RAlow) and the percentage of CD62L low, OX40 positive or Tim-3 positive cells at day 4. Baseline values of samples containing only the plate-immobilized anti-human CD3 were substracted. Therefore, the enhancing effect of OX40 stimulation but not the effect of suboptimal anti-CD3 stimulation per se is visible here. There is no improved TCR stimulation in the absence of strong crosslinking (P329GLALA format in solution). Crosslinking is therefore essential for a bivalent aOx40 format to be agonistic on T cells. This crosslinking will be provided by FAP expressed on the cell surface of tumor or tumor-stromal cells in targeted formats.

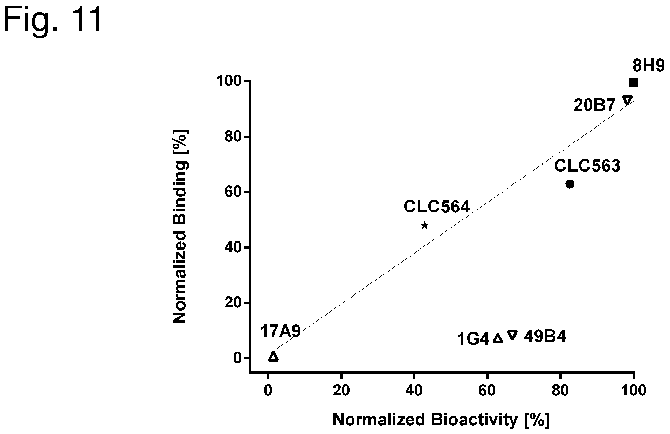

**Figure 11** shows a correlation between the binding strength and agonistic capacity of the different anti-OX40 clones. Binding of anti-OX40 clones (huIgG1 P329GLALA format) on activated CD4 T cells was performed as described in Example 2.1.2. Plateau values were normalized to the value obtained with clone 8H9 (huIgG1 P329GLALA format). Bioactivity testing of anti-Ox40 clones (huIgG1 P329GLALA format) was performed as described in Example 3.2 and plateau values of PD-1 expression were normalized to the values obtained for clone 8H9 (huIgG1 P329GLALA format). Normalized binding was plotted against normalized bioactivity, to test for a correlation between binding strength and agonistic capacity. For most clones there was a direct correlation (linear regression is shown, p value 0.96; slope 0.91). However, two clones (49B4, 1G4) showed a much stronger bioactivity then could be predicted from their binding strength. This subgroup of clones which show unexpectedly high agonistic potency in the face of low binding abilility is of particular interest for the bispecific antigen binding molecules of the invention.

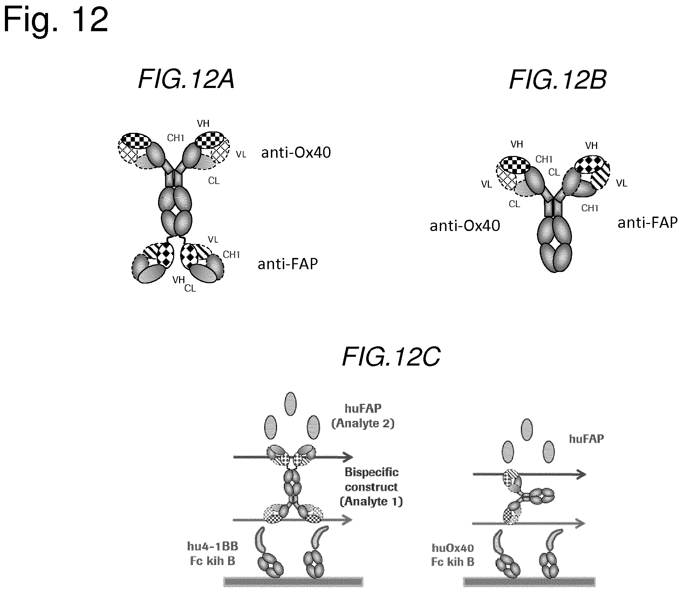

In **Figure 12A** is shown a schematic scheme of an exemplary bispecific, bivalent antigen binding molecule of the invention (2+2 format). **Figure 12B** shows a schematic scheme of an exemplary bispecific, monovalent antigen binding molecule (1+1 format) of the invention. In **Figure 12C** the setup for the SPR experiments showing simultaneous binding to immobilized human OX40 and human FAP is shown.

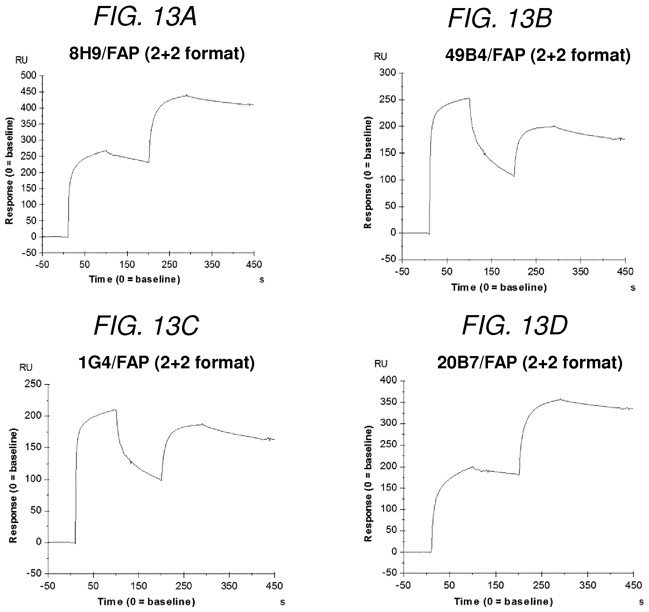

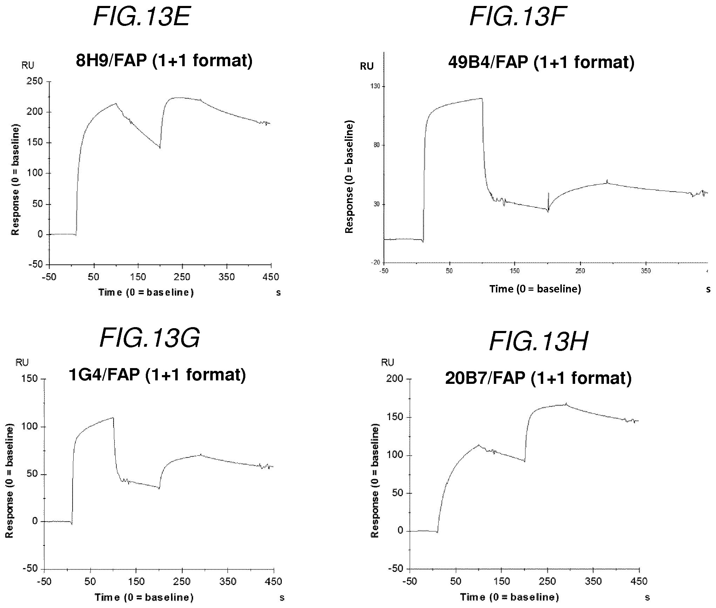

**Figures 13A-13D** show the SPR diagrams of simultaneous binding of bispecific bivalent 2+2 constructs (analyte 1) to immobilized human OX40 and human FAP (analyte 2). The SPR diagrams are shown for 2+2 constructs with OX40 clones 8H9 (Figure 13A), 49B4 (Figure 13B), 1G4 (Figure 13C) and 20B7 (Figure 13D). In **Figures 13E-13H**, the simultaneous binding of bispecific monovalent 1+1 constructs (analyte 1) to immobilized human Ox40 and human FAP (analyte 2) is shown. The SPR diagrams are shown for 1+1 constructs with OX40 clones 8H9 (Figure 13E), 49B4 (Figure 13F), 1G4 (Figure 13G) and 20B7 (Figure 13H).

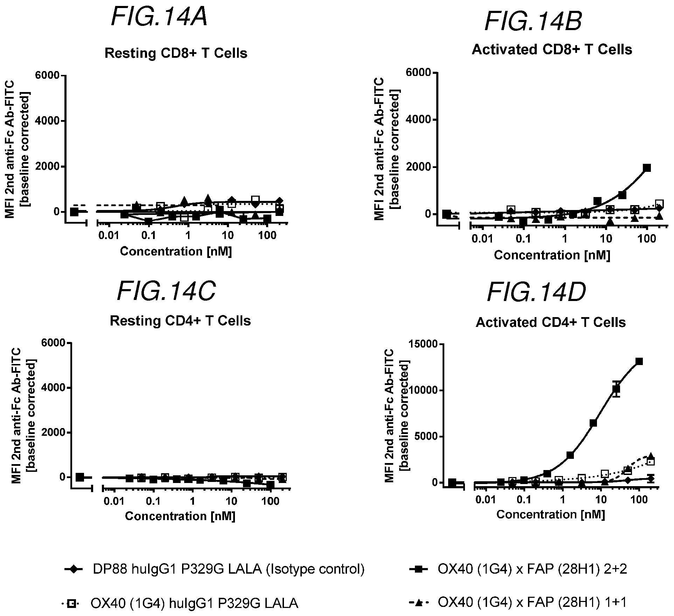

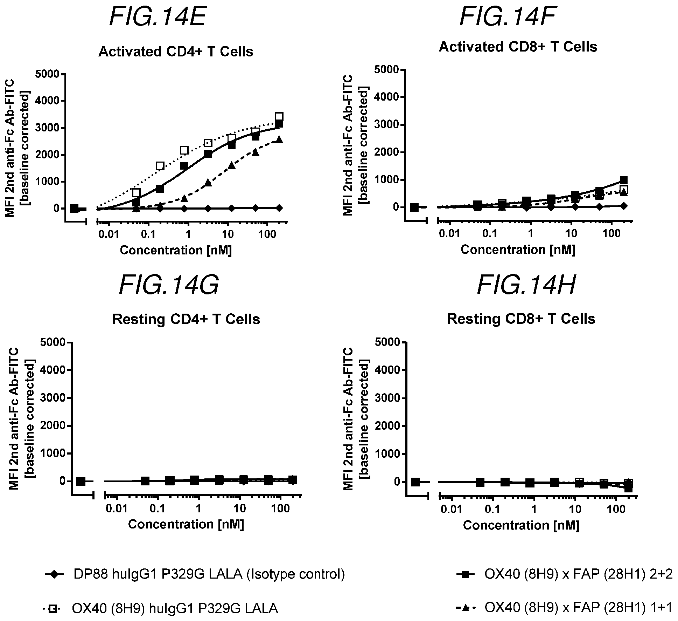

**Figures 14A-14H** show the binding of selected anti-OX40 binders (clone 8H9, 1G4) in a FAP targeted monovalent or bivalent format to resting and activated human PBMC. Binding characteristics to OX40 positive T cells (Figures 14B and 14D and Figures 14E and 14F) were comparable for clones in a conventional bivalent hu IgG format (open square) and a FAP-targeted bivalent format (filled square). Binding of the same clone in a FAP- targeted monovalent format (filled triangle) was clearly weaker due to loss of avidity binding. In the absence of human OX40 expressing cells no binding can be observed (resting cells, Figures 14A and 14C, Figures 14G and 14H). Shown is the binding as median of fluorescence intensity (MFI) of FITC labeled anti-human IgG Fcγ-specific goat IgG F(ab')2 fragment which is used as secondary detection antibody. MFI was measured by flow cytometry and baseline corrected by subtracting the MFI of the blank control. The x-axis shows the concentration of antibody constructs. In Figure 14D it can be seen that clone 1G4 binds to activated, OX40 expressing human CD4⁺ T cells, and to a lower extent to activated human CD8⁺ T cells (Figure 14B). The bivalent construct binds stronger than the monovalent construct. The constructs do not bind to OX40 negative resting T cells. Figure 14E shows that clone 8H9 binds to activated, Ox40 expressing human CD4⁺ T cells, and to a lower extent to activated human CD8⁺ T cells (Figure 14F). The bivalent construct binds stronger than the monovalent construct. The constructs do not bind to Ox40 negative resting T cells.

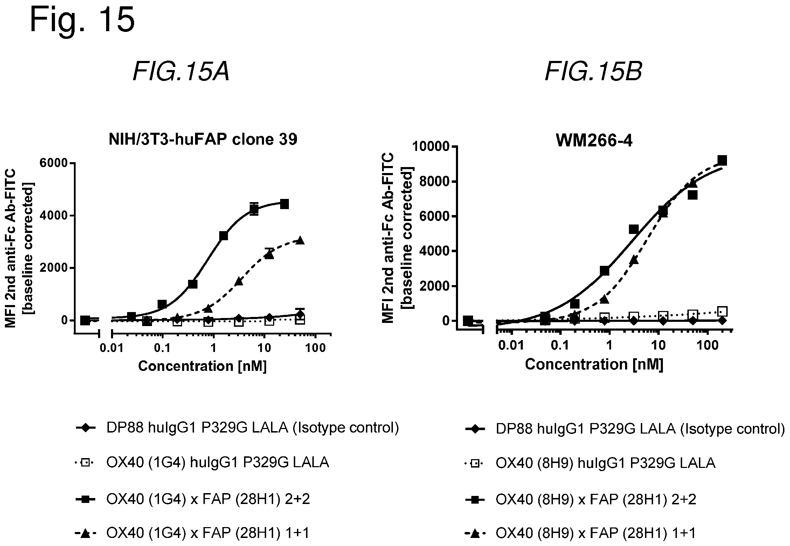

**Figures 15A and 15B** show the binding of selected anti-OX40 binders (clone 8H9, 1G4) in a FAP targeted monovalent or bivalent format to FAP positive tumor cells. Transgenic modified mouse embryonic fibroblast NIH/3T3-huFAP clone 39 or WM266-4 cells express high levels of human fibroblast activation protein (huFAP). Only FAP- targeted mono- and bivalent anti-Ox40 constructs (filled square and triangle) but not the same clone in a human IgG1 P329GLALA format (open square) binds to NIH/3T3-huFAP clone 39 cells (Figure 15A) and WM266-4 cells (Figure 15B), respectively. Shown is the binding as median of fluorescence intensity (MFI) of Fluorescein isothiocyanate (FITC)-labeled anti-human IgG Fcγ-specific goat IgG F(ab')2 fragment which is used as secondary detection antibody. MFI was measured by flow cytometry. The x-axis shows the concentration of antibody constructs. The bivalent FAP construct binds stronger than the monovalent construct.

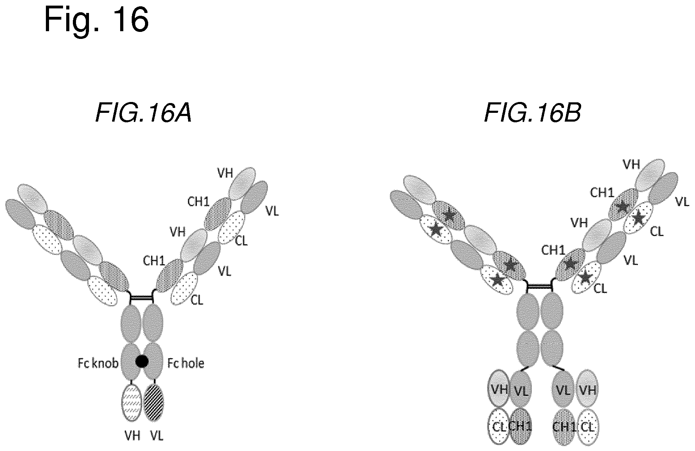

In **Figure 16A** is shown a schematic scheme of an exemplary bispecific, tetravalent antigen binding molecule of the invention with monvalency for the target cell antigen (4+1 format). **Figure 16B** shows a schematic scheme of an exemplary bispecific, tetravalent antigen binding molecule with bivalency for the target cell antigen (4+2 format) of the invention.

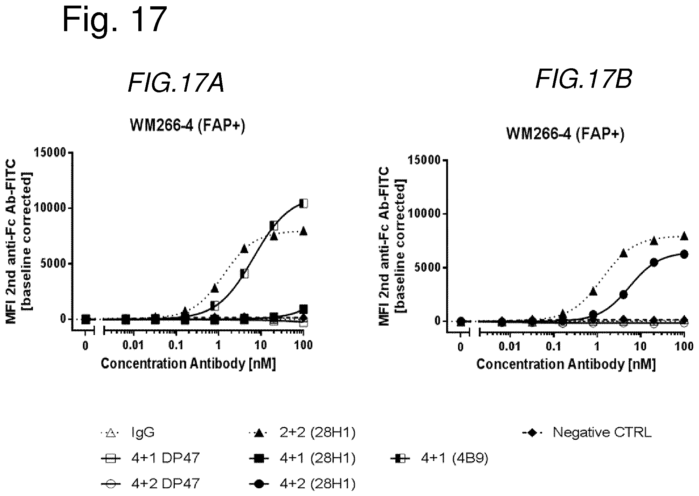

**Figures 17A and 17B** show the binding of an anti-OX40 binder (clone 49B4) in different bispecific human IgG1 P329GLALA formats to human FAP positive WM266-4 cells. WM266-4 cells express high levels of human fibroblast activation protein (huFAP). Only FAP- targeted mono- and bivalent bispecific 49B4 constructs (filled circle, square and triangle) but not untargeted 49B4 constructs (open circle, square and triangle) bound to WM266-4 cells. Shown is the binding as median of fluorescence intensity (MFI) of fluorescein isothiocyanate (FITC)-labeled anti-human IgG Fcγ-specific goat IgG F(ab')2 fragment which is used as secondary detection antibody. MFI was measured by flow cytometry. The x-axis shows the concentration of antibody constructs.

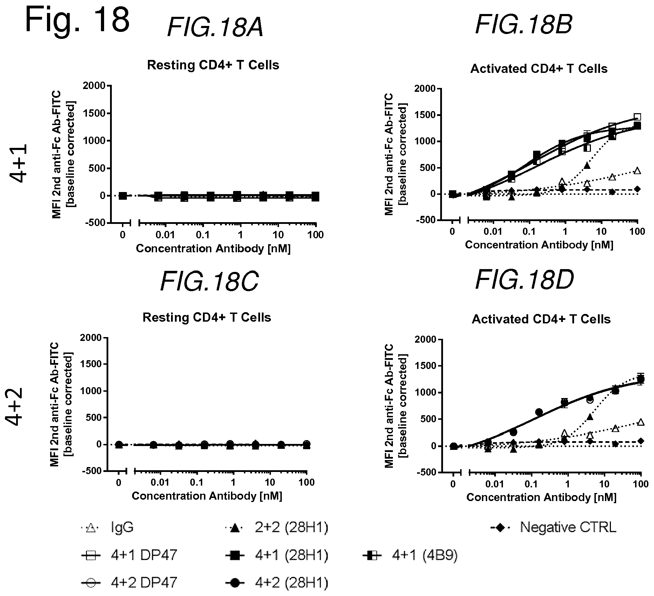

**Figures 18A-18D** show the binding of anti-OX40 binder 49B4 in different bispecific human IgG1 P329GLALA formats to resting and activated human CD4 T cells. OX40 is not expressed on resting human CD4 T cells (Figures 18A and 18C). In the absence of human OX40 expressing cells no binding was observed. After activation of human PBMCs OX40 is up-regulated on CD4+ T cells (Figures 18B and 18D). All constructs containing binder 49B4 bound to Ox40+ activated CD4+ T cells. Tetravalent OX40 binding (circle and square) strongly increased avidity for OX40, whereas the presence of a FAP binding moiety had no impact (compare open vs filled symbols). In comparison, both bivalent constructs (triangle) showed a weaker binding to CD4+ T cells. The binding of clone 49B4 in a bivalent, bispecific construct was clearly stronger than that in the conventional IgG format. Shown is the binding as median of fluorescence intensity (MFI) of FITC labeled anti-human IgG Fcγ-specific goat IgG F(ab')2 fragment which is used as secondary detection antibody. MFI was measured by flow cytometry and baseline corrected by subtracting the MFI of the blank control. The x-axis shows the concentration of antibody constructs.

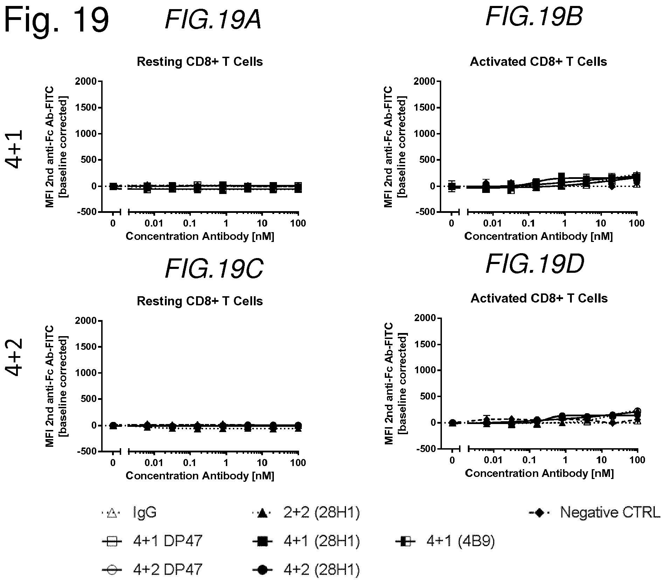

**Figures 19A-19D** show the binding of anti-OX40 binder 49B4 in different bispecific human IgG1 P329GLALA formast to resting and activated human CD8 T cells. OX40 is not expressed on resting human CD8 T cells (Figure 19A and 19C). In the absence of human OX40 expressing cells no binding was observed. After activation of human PBMCs OX40 is up-regulated on CD8⁺ T cells (Figures 19B and 19D). OX40 expression on human CD8⁺ T cells is lower than on CD4⁺ T cells and varies between donors and time points. Expression of OX40 was low on the depicted CD8 T cells. All constructs containing binder 49B4 bound to OX40⁺ activated CD8⁺ T cells. Tetravalent OX40 binding (circle and square) strongly increased avidity for OX40, whereas the presence of a FAP binding moiety had no impact (compare open vs filled symbols). In comparison, both bivalent constructs (triangle) showed a weaker binding to CD8⁺ T cells. The binding of clone 49B4 in a bivalent, bispecific construct was clearly stronger than that in the conventional IgG format. Shown is the binding as median of fluorescence intensity (MFI) of FITC labeled anti-human IgG Fcγ-specific goat IgG F(ab')2 fragment which is used as secondary detection antibody. MFI was measured by flow cytometry and baseline corrected by subtracting the MFI of the blank control. The x-axis shows the concentration of antibody constructs.

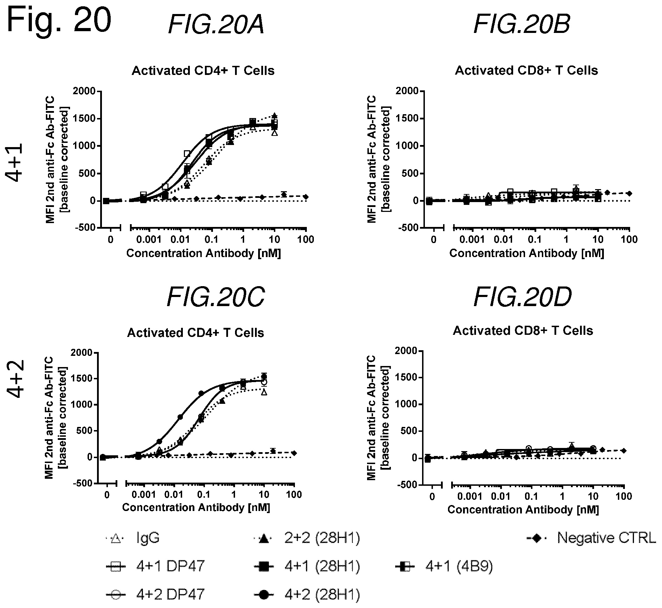

In **Figures 20A-20D** is shown the binding of anti-Ox40 binder 49B4 in different bispecific human IgG1 P329GLALA formats to activated cynomolgus T cells. The depicted constructs varied in their binding strength (EC₅₀ values) to OX40 positive activated cynomolgus CD4⁺ T cells. Constructs with a tetravalent OX40 binding moiety showed a stronger binding to OX40⁺ T cells than bivalent constructs. The gain in avidity between tetravalent and bivalent binding to OX40 was less strong than observed for human OX40. The expression on activated CD8⁺ T cells was low under this condition and only weak binding was found. Binding of anti-OX40 antibodies to cell surface proteins was detected with a goat anti-human IgG Fc-specific secondary antibody conjugated to FITC using FACS analysis. MFI was measured by flow cytometry and baseline corrected by subtracting the MFI of the blank control. The x-axis shows the concentration of antibody constructs.

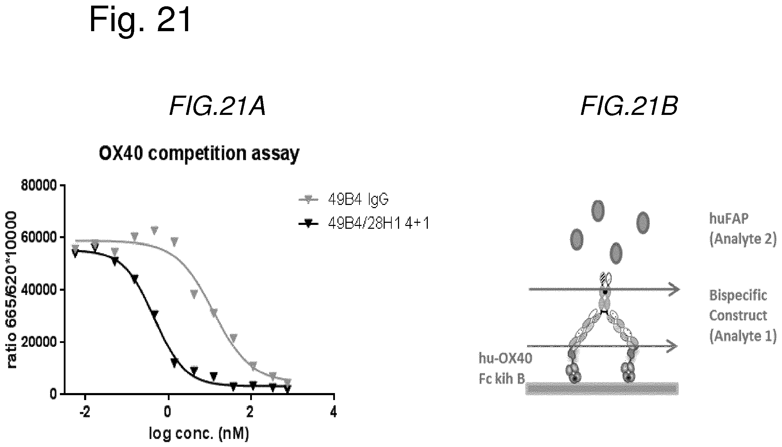

**Figure 21A** shows the results of human OX40 competition binding in a cell-based FRET assay. In **Figure 21B** the setup for the SPR experiments showing simultaneous binding to immobilized human OX40 and human FAP is shown.

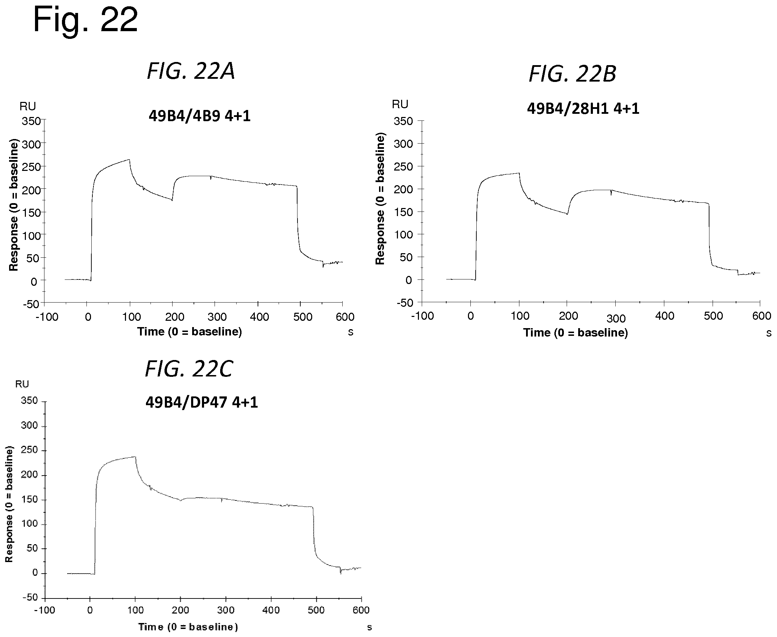

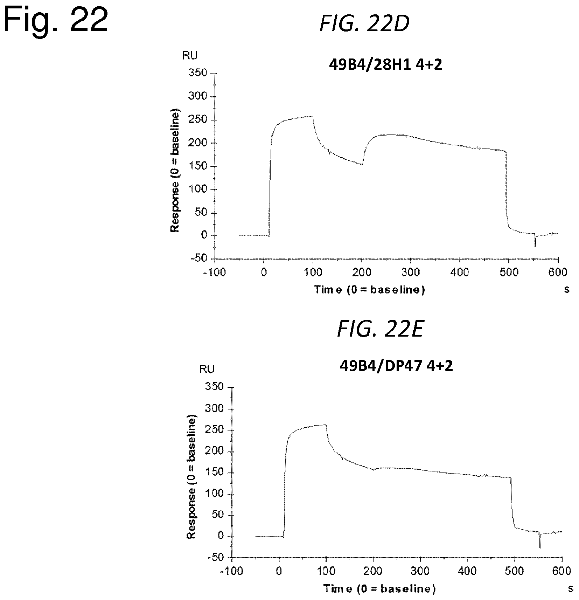

**Figures 22A, 22B and 22C** shows the SPR diagrams of simultaneous binding of bispecific tetravalent 4+1 constructs (analyte 1) to immobilized human OX40 and human FAP (analyte 2). Constructs 4+1 (49B4/28H1) (Figure 22A) and 4+1 (49B4/4B9) (Figure 22B) showed simultaneous binding, whereas construct 4+1 (49B4/DP47) showed only binding to OX40. In **Figure 22D**, the simultaneous binding of bispecific tetravalent 4+2 construct (49B4/28H1) (analyte 1) to immobilized human Ox40 and human FAP (analyte 2) is shown. Construct 4+2 (49B4/DP47) did not show simultaneous binding (**Figure 22E**).

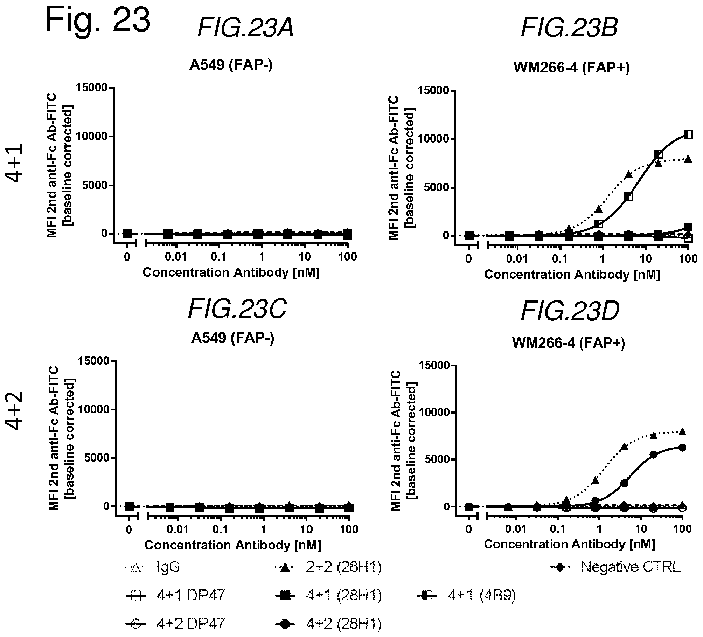

**Figures 23A-23D** relate to the binding of an anti-OX40 binder (clone 49B4) in different bispecific human IgG1 P329GLALA formats to human FAP human OX40 negative A549 cells. The depicted constructs showed no binding to OX40 negative FAP negative A549 tumor cells. Shown is the binding as median of fluorescence intensity (MFI) of FITC labeled anti-human IgG Fcγ-specific goat IgG F(ab')₂ fragment which is used as secondary detection antibody. MFI was measured by flow cytometry and baseline corrected by subtracting the MFI of the blank control. The x-axis shows the concentration of antibody constructs. For comparison, the graphs of binding to human FAP positive WM266-4 cells are shown on the right column. WM266-4 cells express high levels of human fibroblast activation protein (huFAP). Shown is the binding as median of fluorescence intensity (MFI) of fluorescein isothiocyanate (FITC)-labeled anti-human IgG Fcγ-specific goat IgG F(ab')₂ fragment which is used as secondary detection antibody. MFI was measured by flow cytometry. The x-axis shows the concentration of antibody constructs.

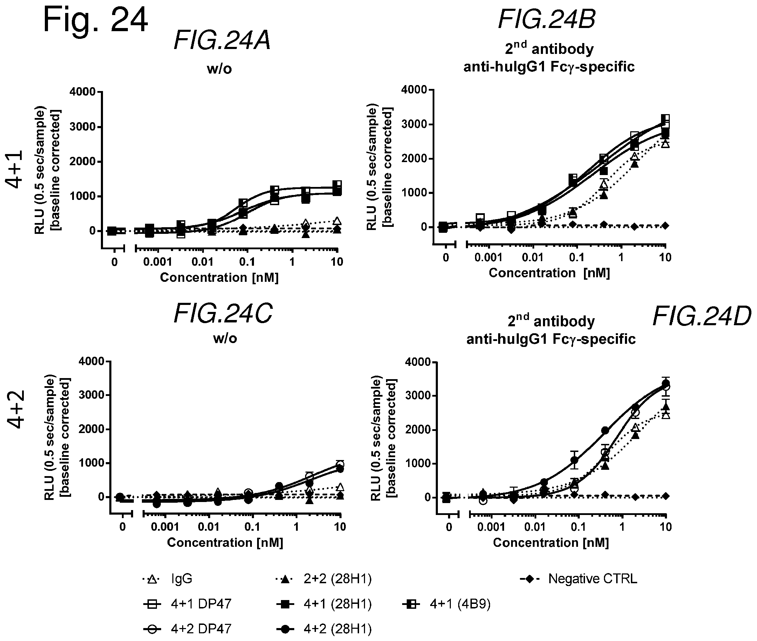

**Figures 24A-24D** show the NFκB activation by an anti-OX40 binder (clone 49B4) in different bispecific human IgG1 P329GLALA formats in HeLa_hOx40_NFkB_Luc1 reporter cells. Shown is the activation of NF-κB signaling pathway in the reporter cell line with anti-OX40 binder 49B9 in different bispecific human IgG1 P329GLALA formats with (Figures 24B and 24D) or without (Figures 24A and 24D) crosslinking by secondary-antibody. The reporter cells were cultured for 6 hours in the presence of anti-Ox40 constructs at the indicated concentrations with or without crosslinking secondary poly-clonal anti-huIgG1 Fcγ-specific goat IgG F(ab)₂ fragment in a 1:2 ratio. The NE-κB-mediated luciferase activity was characterized by blotting the units of released light (URL), measured during 0.5 s, versus the concentration in nM of tested compounds. URLs are emitted due to luciferase-mediated oxidation of luciferin to oxyluciferin. Tetravalent constructs performed slightly better than bivalent constructs.

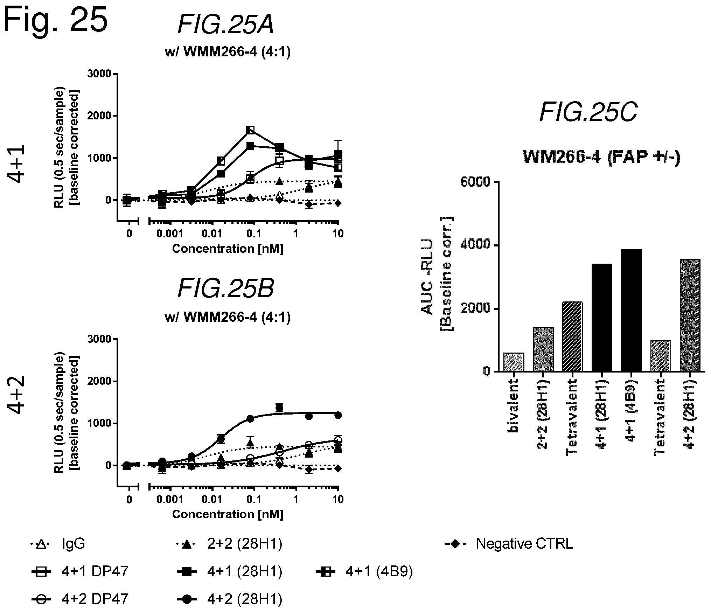

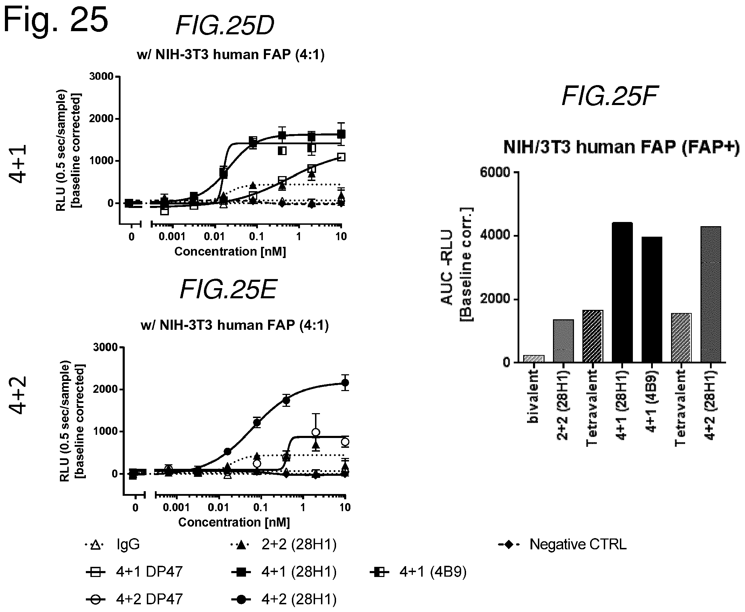

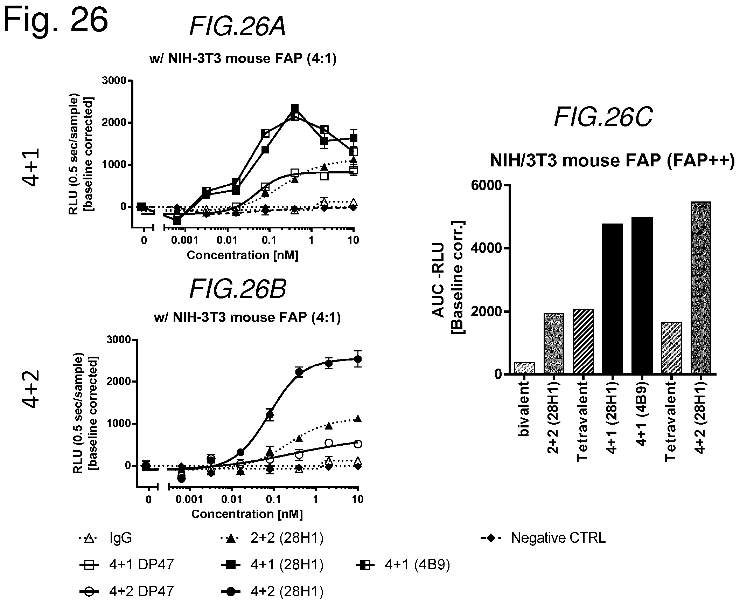

The activation of NFκB by anti-OX40 binder 49B4 in different bispecific human IgG1 P329GLALA formats in HeLa_hOx40_NFkB_Luc1 reporter cells in the presence of FAP positive cells is shown in **Figures 25A-25F** **and** **26A-26C**. Shown is the activation of NFκB signaling pathway in the reporter cells by with anti-OX40 binder 49B4 in different bispecific human IgG1 P329GLALA formats in the presence of low FAP expressing WM266-4 cells (Figures 25A-25C), intermediate FAP expressing NIH-3T3 human FAP cells (Figure 25D-25F) and high FAP expressing NIH-3T3 mouse FAP cells (Figures 26A-26C) (ratio 4 FAP+ tumor cells to 1 reporter cell). The NFκB-mediated luciferase activity was characterized by blotting the units of released light (URL), measured during 0.5 s, versus the concentration in nM of tested compounds. URLs are emitted due to luciferase-mediated oxidation of luciferin to oxyluciferin. Values are baseline corrected by subtracting the URLs of the blank control. For a better comparison of all formats the area under the curve of the respective blotted dose-response curves was quantified as a marker for the agonistic capacity of each construct. The area was calculated using GraphPad Prism. Values are baseline corrected by subtracting the value of the blank control. All constructs were able to induce NFκB activation in Ox40⁺ HeLa reporter cells. Crosslinking by addition of FAP positive cells however increased only the agonistic potential of FAP targeted molecules, but not that of the non-targeted control molecules. Tetravalent constructs performed better than bivalent constructs. Higher FAP expression provided better crosslinking and thus additionally increased OX40 agonism.

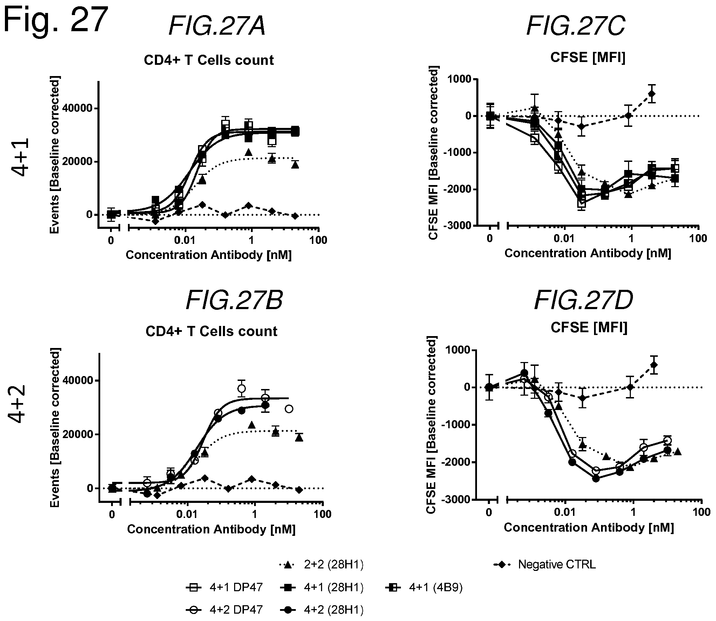

The rescue of suboptimal TCR restimulation of preactivated CD4 T cells with plate-immobilized FAP targeted mono and bivalent anti-OX40 (49B4) constructs is shown in **Figures** 27A **to 27H****.** Costimulation with plate-immobilized anti-Ox40 constructs promoted cell proliferation and maturation of sub-optimally restimulated human CD4 T cells and induced an enhanced activated phenotype. PHA-L pre-activated CFSE-labeled human CD4 T cells were cultured for four days on plates pre-coated with mouse IgG Fcγ spec. antibodies, human IgG Fcγ spec. antibodies (both 2 µg/mL), mouse anti-human CD3 antibodies (clone OKT3, [3 ng/mL]) and titrated anti-OX40 constructs. Shown is the event count, the percentage of proliferating (CFSE-low) cells, the percentage of effector T cells (CD127low CD45RAlow) and the percentage of CD62L low, OX40 positive or Tim-3 positive cells at day 4. Baseline values of samples containing only the plate-immobilized anti-human CD3 were substracted. Therefore, the enhancing effect of OX40 stimulation but not the effect of suboptimal anti-CD3 stimulation per se is visible here. In Figure 27A to 27H it can be seen that all OX40 constructs were able to rescue suboptimal TCR stimulation of preactivated, Ox40⁺ CD4 T cells when coated to plate. Cells survived and proliferation was better. All tetravalent constructs performed comparable to each other and better than bivalent constructs when coated to plate.

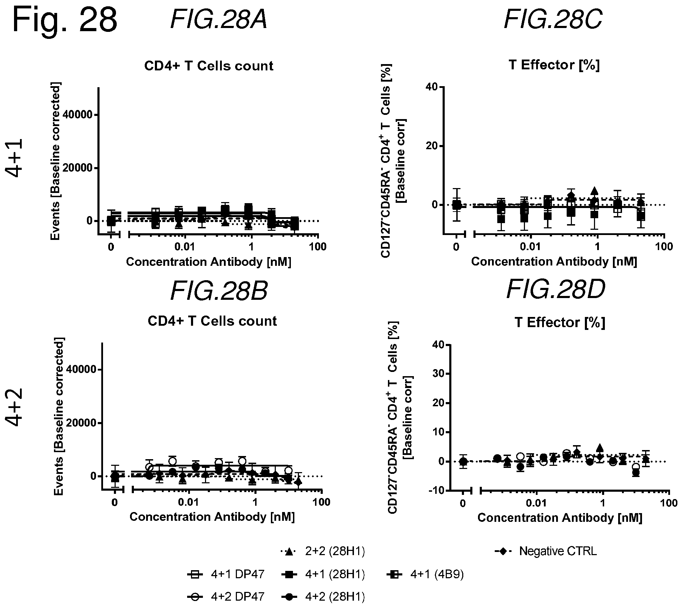

In **Figures 28A to 28D** it is shown that anti-OX40 constructs in solution did not change suboptimal TCR restimulation of preactivated CD4 T cells. No effect on cell proliferation and maturation of sub-optimally restimulated human CD4 T cells was detected in the absence of plate immobilization of anti-OX40 constructs (huIgG1 P329GLALA formats). PHA-L preactivated CFSE-labeled human CD4 T cells were cultured for four days on plates pre-coated with mouse IgG Fcγ spec. antibodies and mouse anti-human CD3 antibodies (clone OKT3, [3ng/mL]). Titrated anti-OX40 constructs (huIgG1 P329GLALA format) were added to the media and were present in solution throughout the experiment. Shown is the event count and the percentage of effector T cells (CD127_{low}CD45RA_{low}) cells at day 4. Baseline values of samples containing only the plate-immobilized anti-human CD3 were substracted. Therefore, the enhancing effect of OX40 stimulation but not the effect of suboptimal anti-CD3 stimulation per se is visible here.

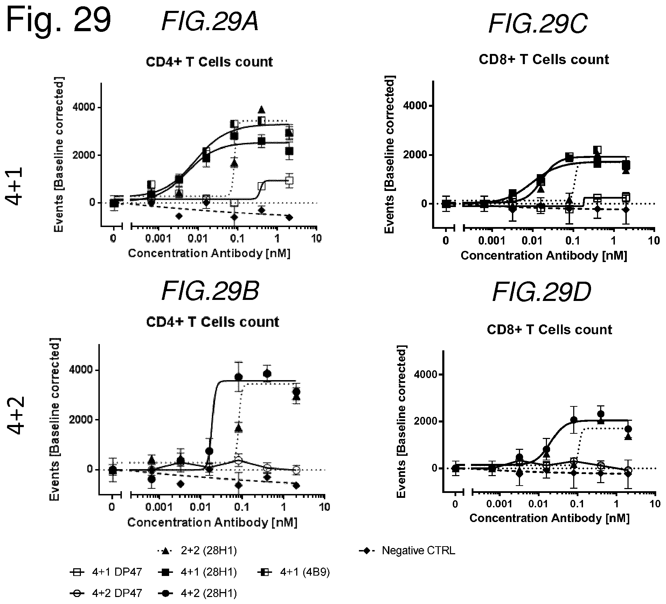

**Figures 29A to 29D** relate to the OX40 mediated costimulation of suboptimally TCR triggered resting human PBMC and hypercrosslinking by cell surface FAP. Costimulation with non-targeted tetravalent anti-Ox40 (49B4) huIgG1 P329GLALA 4+1 and 4+2 did hardly rescue suboptimally TCR stimulated CD4 and CD8 T cells. Hyper-crosslinking of the FAP targeted bi-and tetravalent anti-Ox40 constructs by the present NIH/3T3-huFAP clone 39 cells strongly promoted survival and proliferation in human CD4 and CD8 T cells. Shown is the event count of vital CD4⁺ (Figures 29A and 29B) and CD8⁺ (Figures 29C and 29D) T cells. Baseline values of samples containing only the anti-human CD3 (clone V9, huIgG1), resting human PBMC and NIH/3T3-huFAP clone 39 were substracted. Thus the enhancing effect of OX40 co-stimulation but not the effect of suboptimal anti-CD3 stimulation per se is shown here. Targeted tetravalent constructs were superior to bivalent constructs. In a FAP positive tumor micro environment this could lead to increased anti-tumor activity of T cells in the absence of systemic OX40 activation.

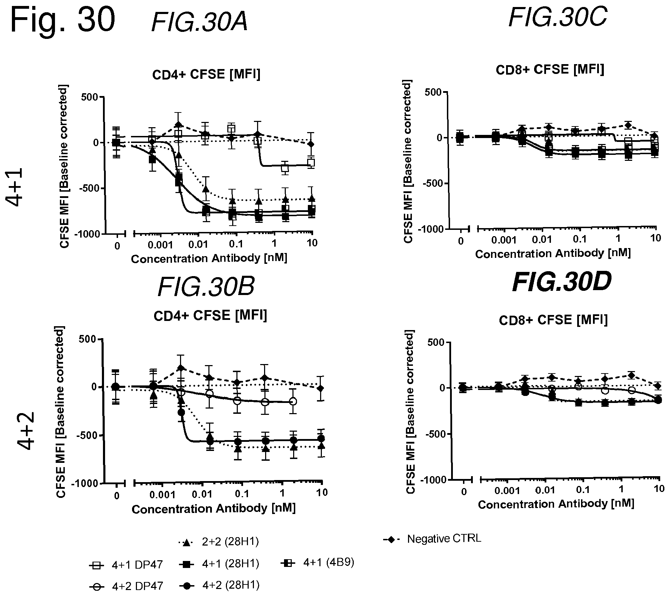

**Figures 30A to 30D** relate to the rescue of suboptimal TCR stimulation of resting human PBMC with cell surface immobilized FAP targeted tetravalent anti-Ox40 (49B4) constructs in the proliferation stage. Costimulation with non-targeted tetravalent anti-Ox40 (49B4) huIgG1 P329GLALA 4+1 and 4+2 did hardly rescue suboptimally TCR stimulated CD4 and CD8 T cells. Hyper-crosslinking of the FAP targeted bi- and tetravalent anti-Ox40 constructs by the present NIH/3T3-huFAP clone 39 cells strongly promoted survival and proliferation in human CD4 and CD8 T cells. Shown is percentage of CFSE low vital CD4⁺ (Figures 30A and 30B) and CD8⁺ (Figures 30C and 30D) T cells. Baseline values of samples containing only the anti-human CD3 (clone V9, huIgG1), resting human PBMC and NIH/3T3-huFAP clone 39 were substracted. Thus the enhancing effect of OX40 co-stimulation but not the effect of suboptimal anti-CD3 stimulation per se is shown here. The targeted tetravalent constructs were superior to the bivalent constructs.

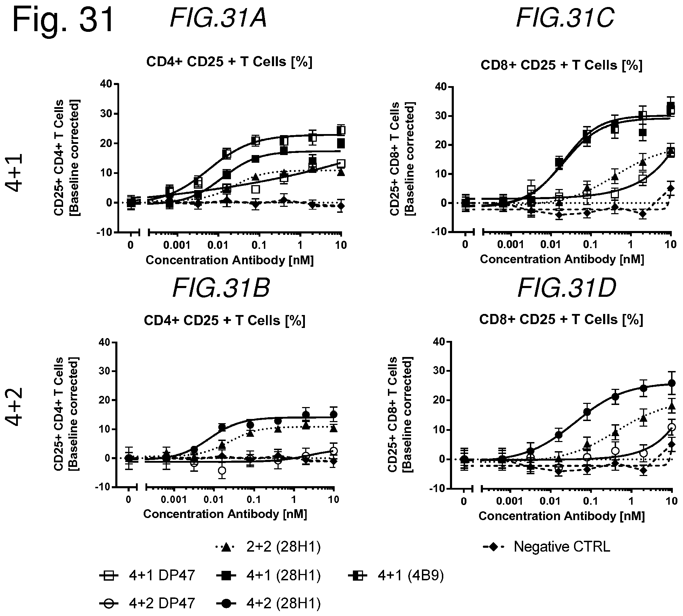

**Figures 31A to 31D** show the rescue of suboptimal TCR stimulation of resting human PBMC with cell surface immobilized FAP targeted tetravalent anti-Ox40 (49B4) constructs by enhancing activated phenotype CD25. Costimulation with non-targeted tetravalent anti-Ox40 (49B4) huIgG1 P329GLALA 4+1 and 4+2 did hardly rescue suboptimally TCR stimulated CD4 and CD8 T cells. Hyper-crosslinking of the FAP targeted bi- and tetravalent anti-Ox40 constructs by the present NIH/3T3-huFAP clone 39 cells strongly enhanced an activated phenotype in human CD4 and CD8 T cells. Shown is percentage of CD25 positive, vital CD4⁺ (Figures 31A and 31B) and CD8⁺ (Figures 31C and 31D) T cells. Baseline values of samples containing only the anti-human CD3 (clone V9, huIgG1), resting human PBMC and NIH/3T3-huFAP clone 39 were substracted. Thus the enhancing effect of OX40 co-stimulation but not the effect of suboptimal anti-CD3 stimulation per se is shown here. Targeted tetravalent constructs performed better than bivalent constructs.

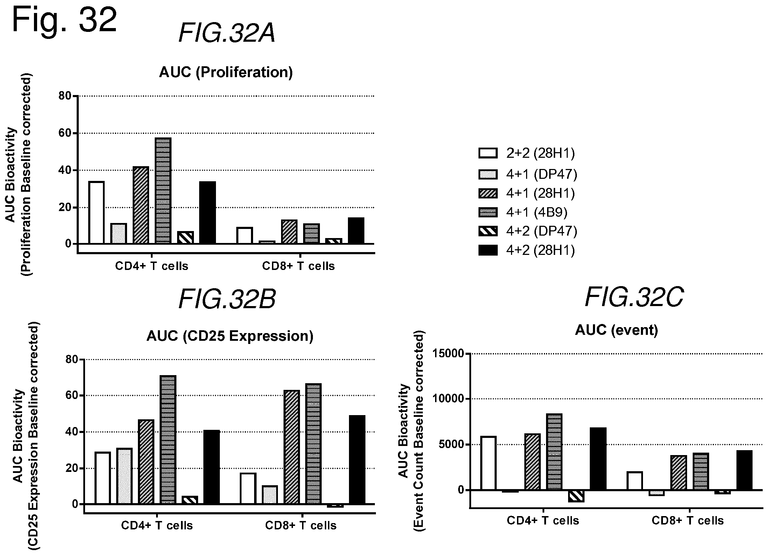

A summary is given in **Figure 32A to 32C****.** Hyper-crosslinking of the FAP targeted bi- and tetravalent anti-OX40 constructs by the present NIH/3T3-huFAP clone 39 cells strongly enhanced an activated phenotype in human CD4 and CD8 T cells and sustained survival and proliferation. For a better comparison of all formats the area under the curve of the respective blotted dose-response curves of **Figures 29A to 29D****,** **Figures 30A to 30D** **and** **Figures 31A to 31D** was quantified as a marker for the agonistic capacity of each construct. The area was calculated using GraphPad Prism. Values are baseline corrected by subtracting the value of the blank control. Only constructs containing FAP binding moiety were able to rescue suboptimal TCR stimulation of resting CD4 and CD8 T cells when crosslinking was provided by FAP positive cells (NIH). Cells showed a stronger proliferation, expansion and activation (CD25). Targeted tetravalent constructs were superior to bivalent constructs. In a FAP positive tumor micro environment this could lead to increased anti-tumor activity of T cells in the absence of systemic OX40 activation.

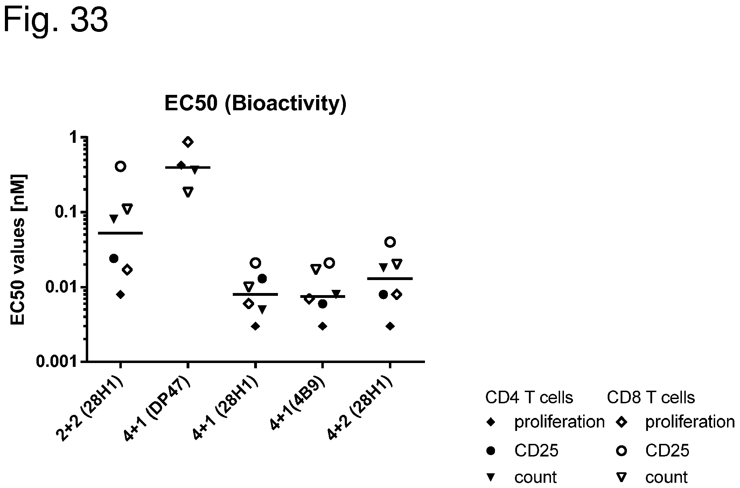

A comparison of EC₅₀ values is shown in **Figure 33**. For a better comparison of all formats the EC₅₀ values of the respective blotted dose-response curves of **Figures 29A to 29D****,** **Figures 30A to 30D** **and** **Figures 31A to 31D** was quantified as a marker for the agonistic capacity of each construct. The EC₅₀ value was calculated using GraphPad Prism. It can be easily seen that the targeted 4+1 and 4+2 constructs performed better than 2+2 construct.

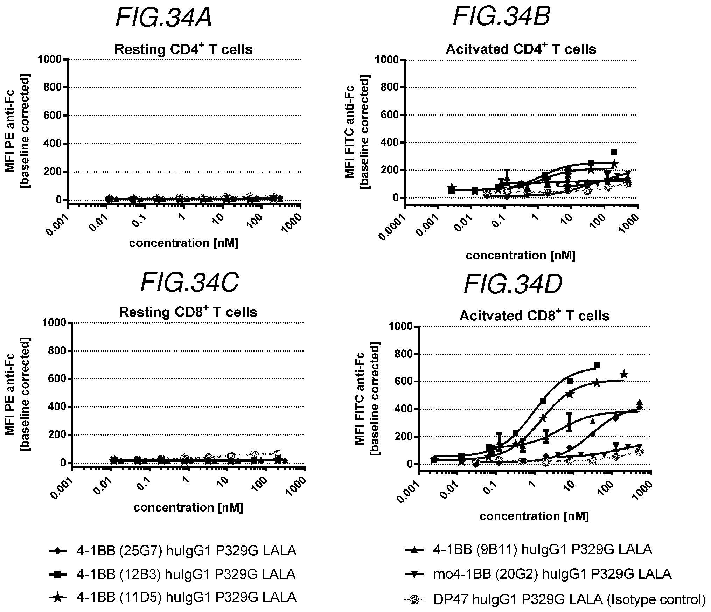

**Figures 34A-34D** show the binding to resting and activated human T cells of four anti-human 4-1BB-specific clones transferred to a huIgG1 P329G LALA format (filled diamond: clone 25G7, filled square: clone 12B3, filled star: clone 11D5, pointing-up triangle: clone 9B11) and one anti-mouse 4-1BB specific clone 20G2 transferred to a huIgG1 P329G LALA format (pointing down triangle). As negative control a non-4-1BB-specific clone DP47 huIgG1 P329G LALA antibody was used (open grey circle). The upper panels show binding to resting CD4⁺ T cells (Fig. 34A) and activated CD4⁺ T cells (Fig. 34B), whereas the lower panels show binding to resting CD8⁺ T cells (Fig. 34C) and activated CD8⁺ T cells (Fig. 34D). The binding is characterized by plotting the median of fluorescence (MFI) of FITC-labeled or PE-labeled anti-human IgG Fcγ-specific goat IgG F(ab')₂ fragment that is used as secondary detection antibody versus the concentration in nM of the tested primary anti-4-1BB-binding huIgG1 P329G LALA antibodies. MFI was measured by flow cytometry and baseline corrected by subtracting the MFI of the blank control (no primary antibody).

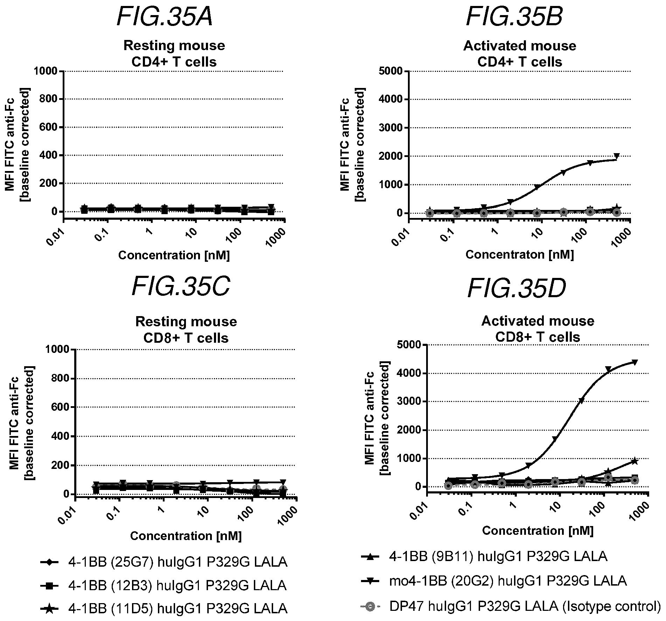

**Figures 35A-35D** show the binding to 4-1BB expressing mouse T cells. Shown is the binding to resting and activated mouse T cells of four anti-human 4-1BB binding huIgG1 P329G LALA antibody clones (filled diamond: clone 25G7, filled square: clone 12B3, filled star: clone 11D5, pointing-up triangle: clone 9B11) and one anti-mouse 4-1BB binding huIgG1 P329G LALA antibody clone 20G2 (pointing-down tringle). As negative control a non-4-1BB binding DP47 huIgG1 P329G LALA antibody was used (open grey circle). The upper panels show binding to resting mouse CD4⁺ T cells (Fig. 35A) and activated CD4⁺ T cells (Fig. 35B), whereas the lower panels show binding to resting mouse CD8⁺ T cells (Fig. 35C) and activated CD8⁺ T cells (Fig. 35D). The binding is characterized by plotting the MFI of FITC-labeled anti-human IgG Fcγ-specific goat IgG F(ab')₂ fragment that is used as secondary detection antibody versus the concentration in nM of the tested primary anti-4-1BB-binding huIgG1 P329G LALA antibodies. MFI was measured by flow cytometry and baseline corrected by subtracting the MFI of the blank control (no primary antibody).

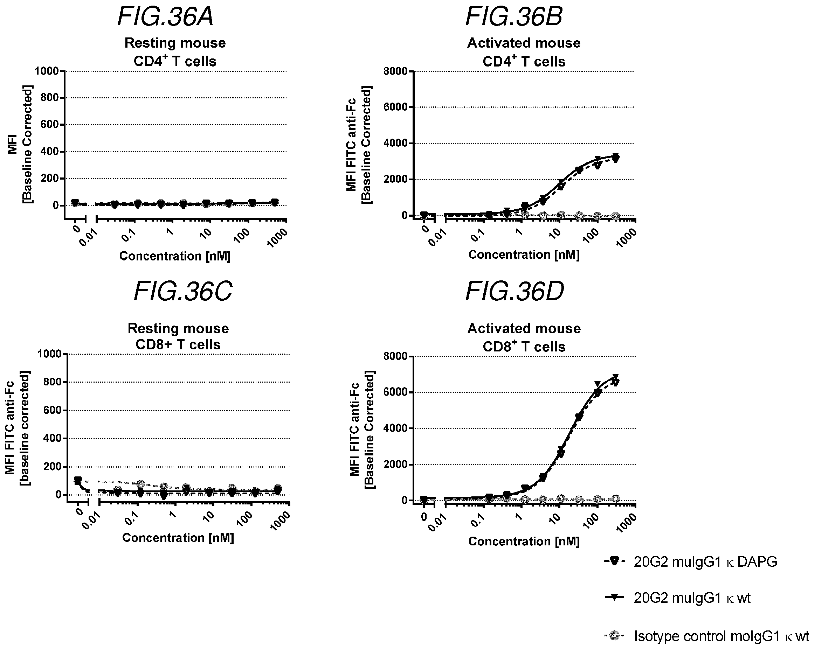

**Figures 36A-36D** show the binding of mouse IgGs to 4-1BB expressing mouse T cells. Shown is the binding to resting and activated mouse T cells of the anti-mouse 4-1BB binding clone 20G2 transferred to the formats mouse IgG1 DAPG and mouse IgG1 wildtype (wt). As negative control a commercial non-4-1BB binding mouse IgG1 wt isotype control was used (open grey circle, BioLegend, Cat.-No. 400153). In the upper panels binding to resting CD4⁺ T cells (Fig. 36A) and activated CD4⁺ T cells (Fig. 36B) is shown, whereas in the lower panels binding to resting CD8⁺ T cells (Fig. 36C) and activated CD8⁺ T cells (Fig. 36D) is shown. The binding is characterized by plotting the median of fluorescence of intensity (MFI) of FITC-labeled anti-mouse IgG Fcγ-specific goat IgG F(ab')2 fragment that is used as secondary detection antibody versus the concentration in nM of the tested primary anti -4-1BB-binding moIgG antibodies. MFI was measured by flow cytometry and baseline corrected by subtracting the MFI of the blank control (no primary antibody).

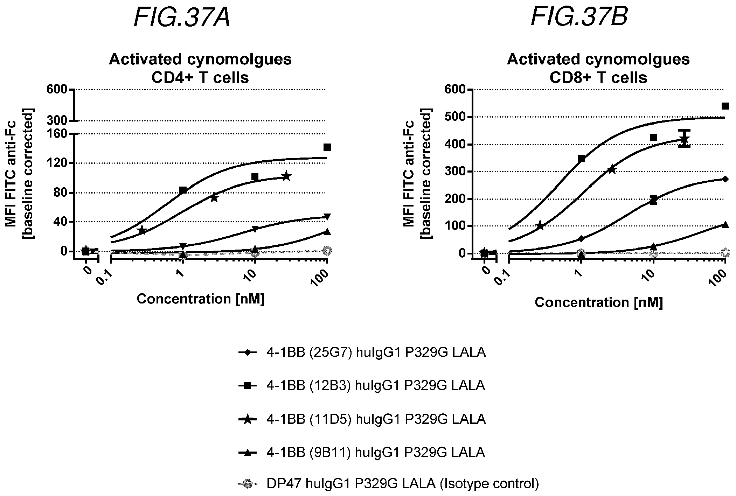

**Figures 37A and 37B** show the binding to 4-1BB expressing cynomolgus T cells. Shown is the binding to activated cynomolgus T cells of four anti-human 4-1BB binding huIgG1 P329G LALA antibody clones (filled diamond: clone 25G7, filled square: clone 12B3, filled star: clone 11D5, pointing-up triangle: clone 9B11). As negative control a non-4-1BB binding DP47 huIgG1 P329G LALA antibody was used (open grey circle). Shown is the binding to activated CD4⁺ T cells (Fig. 37A) and to activated CD8⁺ T cells (Fig. 37B), respectively. The binding is characterized by plotting the median of fluorescence of intensity (MFI) of FITC-labeled anti-human IgG Fcγ-specific goat IgG F(ab')2 fragment that is used as secondary detection antibody versus the concentration in nM of the tested primary anti-4-1BB-binding huIgG1 P329G LALA antibodies. MFI was measured by flow cytometry and baseline corrected by subtracting the MFI of the blank control (no primary antibody).

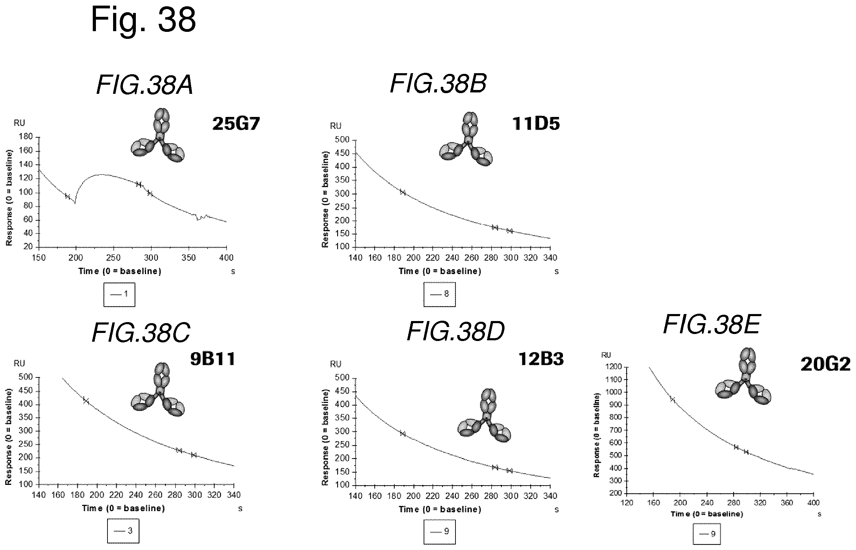

**Figures 38A-38E** refer to ligand binding properties of the anti-4-1BB antibodies of the invention as determined by surface plasmon resonance. The interaction between human anti-4-1BB IgG 25G7 (Fig. 38A), 11D5 (Fig. 38B), 9B11 (Fig. 38C) and 12B3 (Fig. 38D) and the preformed complex hu4-1BB Ligand / hu4-1BB is shown as well as the interaction of mouse anti-4-1BB clone 20G2 (Fig. 38E) and the preformed complex mu4-1BB Ligand / mu4-1BB.

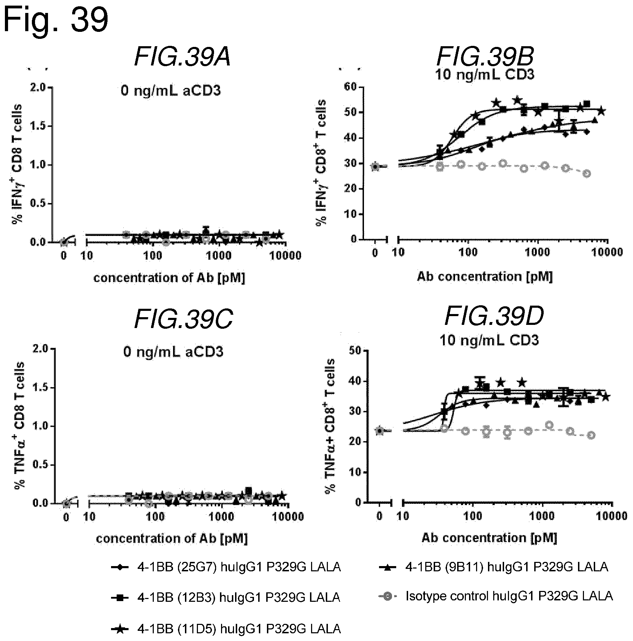

**Figures 39A-39D** show functional properties of different anti-human 4-1BB clones in vitro. Pre-activated human CD8⁺ T cells were activated with different concentrations of surface immobilized anti-human-4-1BB-specific huIgG1 P329G LALA antibodies in the absence of anti-human CD3 antibody (Figures 39A and 39C) or in the presence of sub-optimal concentration of surface immobilized anti-human CD3 antibody (Figures 39B and 39D). Shown is the frequency of IFNγ⁺ (A and B) and TNFα⁺ (C and D) CD8⁺ T cells in the total CD8⁺ T cell population versus the concentration of surface immobilized 4-1BB-binding huIgG1 P329G LALA in pM. In the presence of CD3-stimulation 4-1BB-co-stimulation increased IFNγ (Fig. 39B) and TNFα (Fig. 39D) secretion in a concentration dependent manner. In the absence of CD3-stimulation, activation of 4-1BB had no effect on IFNγ (Fig. 39A) and TNFα (Fig. 39C) secretion.

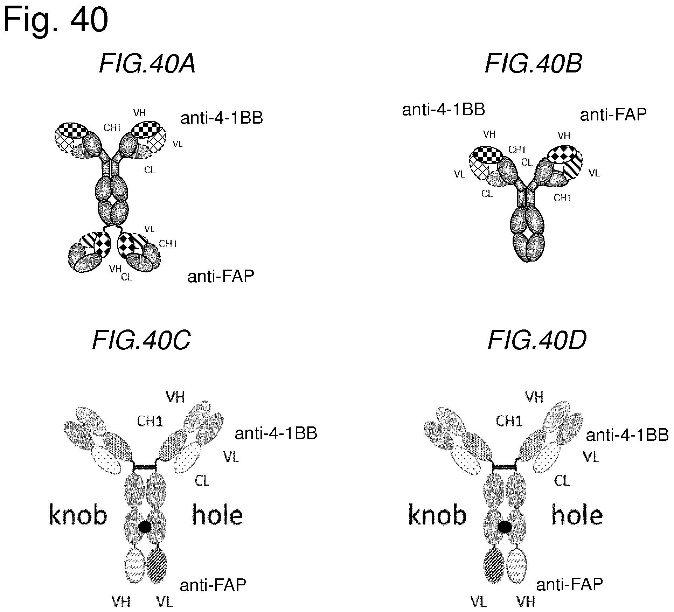

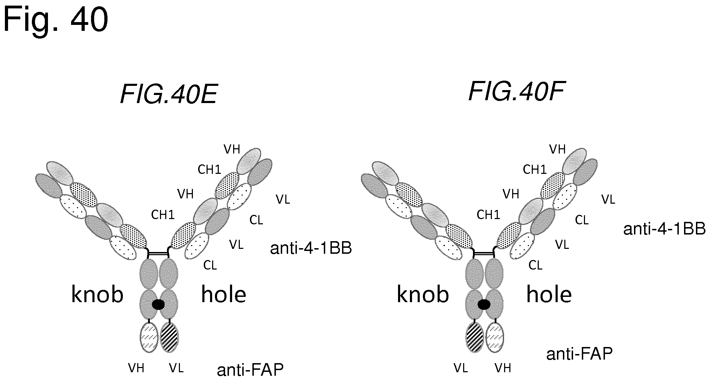

In **Figure 40A** is shown a schematic scheme of an exemplary bispecific, bivalent antigen binding molecule of the invention (2+2 format) comprising two anti-4-1BB Fab fragments and two anti-FAP Fab fragments. **Figure 40B** shows a schematic scheme of an exemplary bispecific, monovalent antigen binding molecule (1+1 format) of the invention comprising one anti-4-1BB Fab fragment and one anti-FAP Fab fragment. In **Figure 40C** a schematic scheme of an exemplary bispecific, bivalent antigen binding molecule (2+1 format) of the invention comprising two anti-4-1BB Fab fragment and a VH and VL domain capable of specific binding to FAP is shown. The filled circle symbolizes the knob into hole mutation. In the construct shown in Figure 40C the VH domain of the FAP antibody is fused to the C-terminus of the Fc knob heavy chain, whereas in the construct shown in **Figure 40D** the VH domain of the FAP antibody is bound to the C-terminus of the Fc hole chain. In **Figures 40E and 40F** are shown a schematic scheme of an exemplary bispecific, tetravalent antigen binding molecule of the invention with monovalency for FAP (4+1 format).

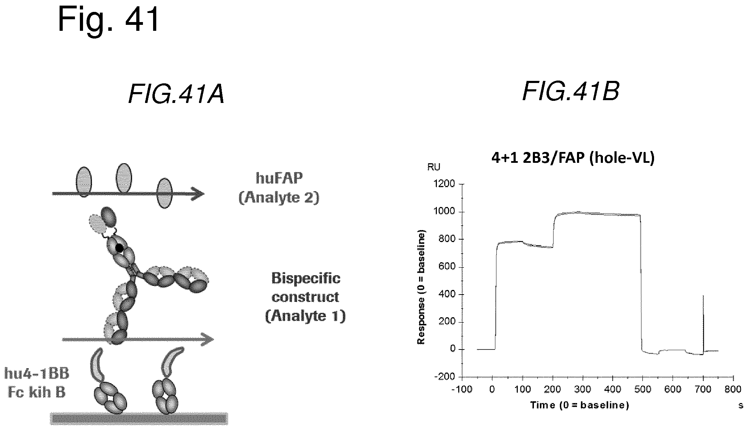

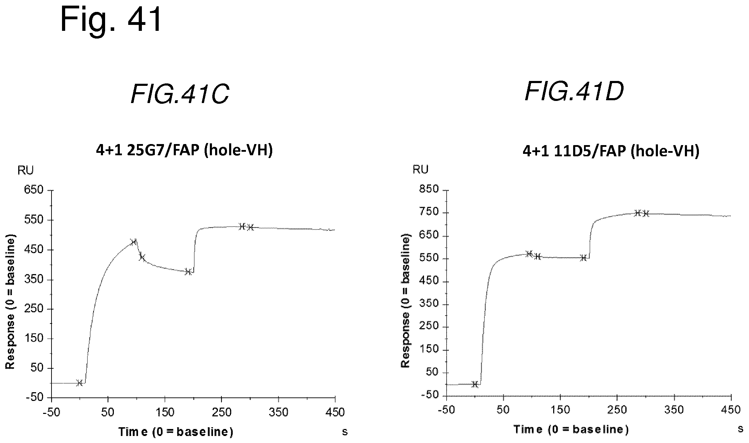

**Figures 41A to 41D** illustrate the simultaneous binding of bispecific anti-4-1BB x anti-FAP antigen binding molecules (4+1 constructs). In **Figure 41A** the setup of the assay is shown. In **Figures 41B to 41D** simultaneous binding of bispecific constructs (analyte 1) to immobilized human 4-1BB and human FAP (analyte 2) is shown. **Figure 41B** shows the binding of the bispecific 4+1 construct with clone 12B3, **Figure 41C** for the construct with clone 25G7 and **Figure 41D** for the construct with clone 11D5.

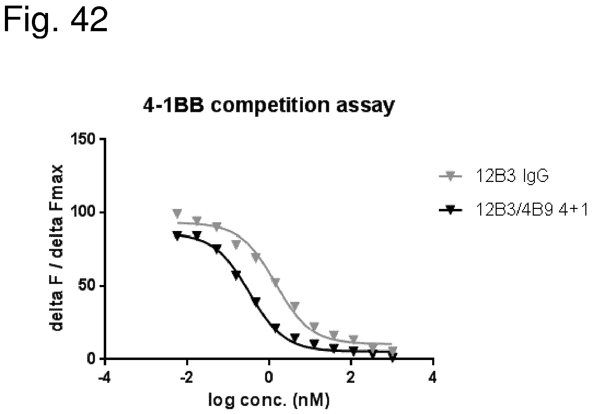

**Figure 42** shows a FRET based competition assay to assess the binding of bivalent (IgG) and tetravalent (4+1) 4-1BB × FAP constructs to membrane bound 4-1BB. For anti-4-1BB binder 12B3 the tetravalent 4+1 format competes better for binding than the respective bivalent IgG antibody.

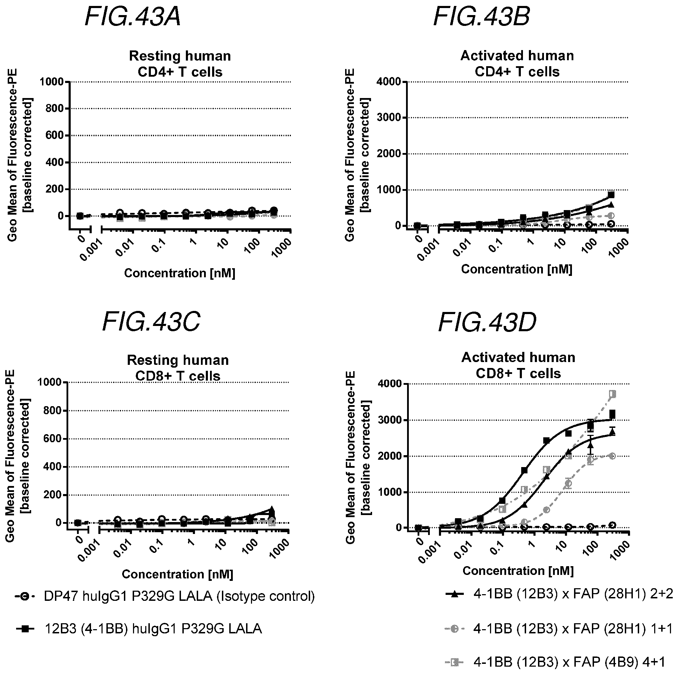

In **Figures 43A to 43D** the binding to resting and activated human T cells is shown. The concentration of 4-1BB-binding molecules is blotted against the geo mean of fluorescence intensity (MFI) of the PE-conjugated secondary detection antibody. All values are baseline corrected by subtracting the baseline value of the blank control (e.g. no primary only secondary detection antibody). In Figure 43A the binding to resting CD4 T cells and in Figure 43B to activated CD4⁺T cells is shown. In Figure 43C the binding to resting CD8 T cells andin Figure 43D to activated CD8⁺ T cells is shown. Only 4-1BB-binding-domain-containing constructs like 12B3 huIgG1 P329G LALA (black filled square), 12B3 × FAP (28H1) 2+2 antigen binding molecule (black filled triangle), 12B3 × FAP (28H1) 1+1 antigen binding molecule (half filled grey circle) or 12B3 × FAP (4B9) 4+1 antigen binding molecule (grey half-filled square) bind efficiently to 4-1BB-expressing cells. No binding could be detected with the germline control molecule DP47 huIgG1 P329G LALA (open black circles, dotted line).

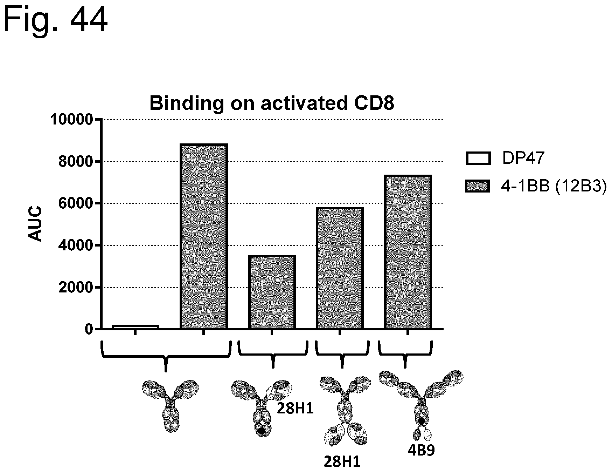

**Figure 44** summarizes the binding to activated human CD8⁺ T cells. Shown is the area under the curve (AUC) of binding curves to high-4-1BB-expressing activated CD8⁺ T cells against the different 4-1BB-specific molecules. The formats and the respective FAP-specific clones (28H1 or 4B9) are indicated below the graph as pictograms. The 4-1BB-binding clone 12B3 is indicated by grey columns whereas the isotype control DP47 huIgG1 P329G LALA is indicated as white column (only IgG, left column).

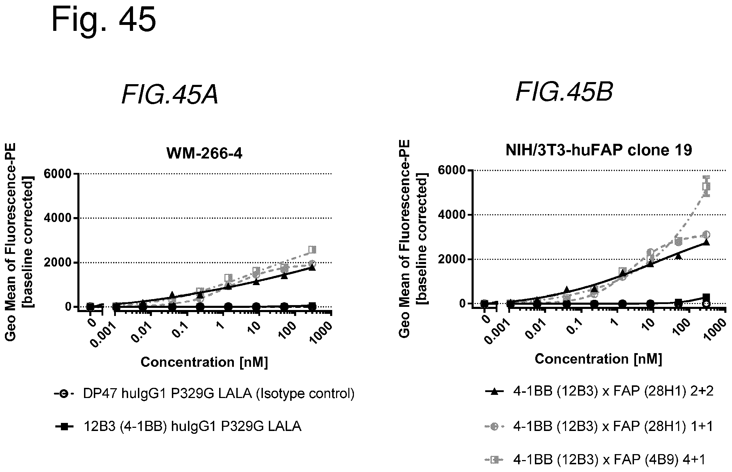

**Figures 45A and 45B** relate to the binding to human FAP-expressing cells. The concentration of 4-1BB-binding molecules is blotted against the geo mean of fluorescence intensity (MFI) of the PE-conjugated secondary detection antibody. All values are baseline corrected by subtracting the baseline values of the blank control (e.g. no primary only secondary detection antibody). In Figure 45A binding to intermediate-FAP-expressing human melanoma cell line WM-266-4 and in Figure 45B binding to high-FAP-expressing NIH/3T3-huFAP clone 19 cells are shown. Only FAP-binding-domain-containing constructs like 12B3 × FAP (28H1) 2+2 antigen binding molecule (black filled triangle), 12B3 × FAP (28H1) 1+1 antigen binding molecule (half filled grey circle) or 12B3 × FAP (4B9) 4+1 antigen binding molecule (grey half-filled square) bind efficiently to FAP-expressing cells. No binding could be detected with non FAP-targeted molecules 12B3 huIgG1 P329G LALA (black filled square) and the control molecule DP47 huIgG1 P329G LALA (open black circles, dotted line).

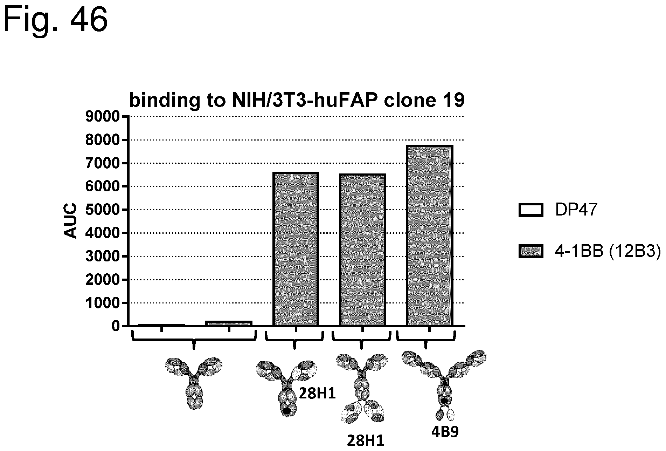

**Figure 46** summarizes the binding to human FAP-expressing NIH/3T3-huFAP cells. Shown is the area under the curve (AUC) of the binding curves to FAP-high-expressing NIH/3T3-huFAP cells blotted against the different 4-1BB-specific molecules. The formats and the respective FAP-specific clone (28H1 or 4B9) are indicated below the graph as pictograms. The 4-1BB-binding clones are indicated by the column color, whereby the isotype control DP47 is indicated in white and molecules containing the 4-1BB-specific clone 12B3 are indicated in grey.

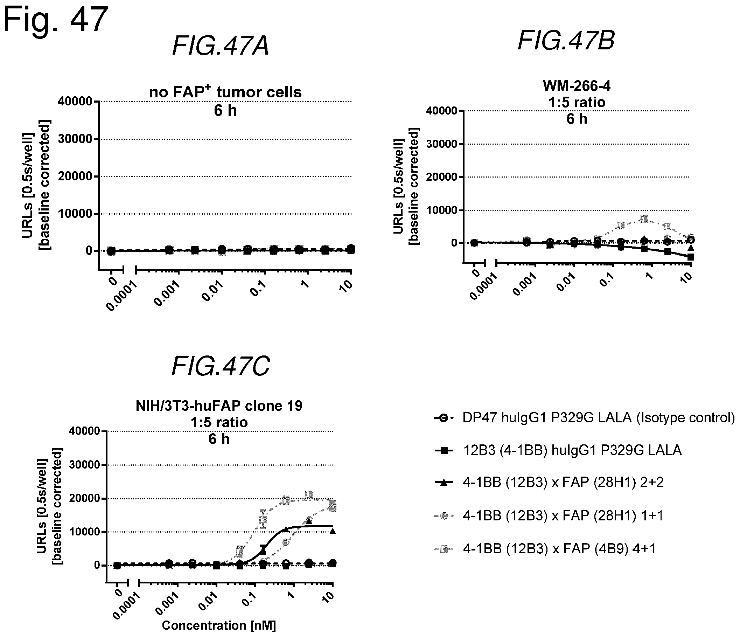

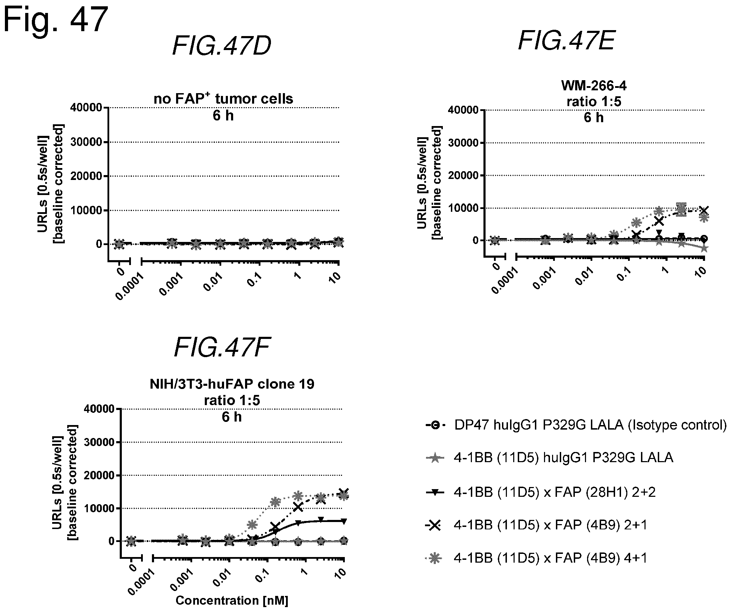

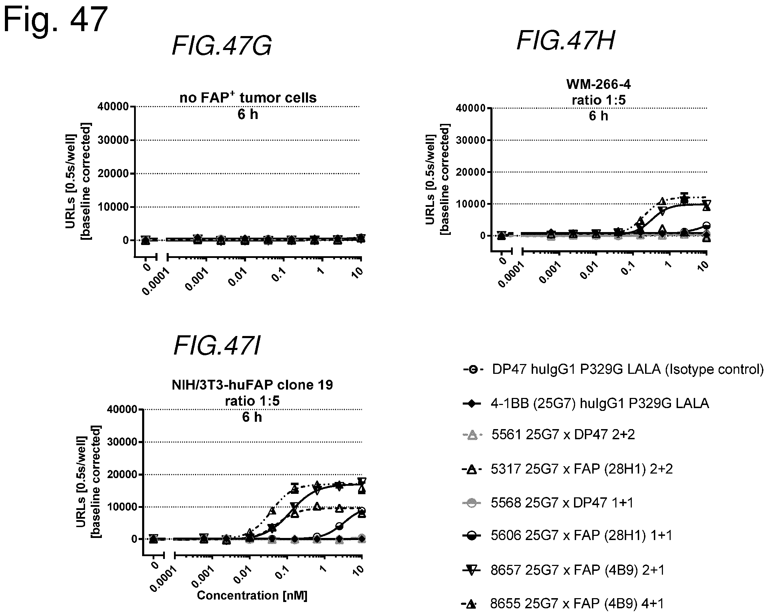

**Figures 47A to 47I** relate to NFκB-controlled luciferase expression in 4-1BB expressing reporter cell line (Example 10). The concentration of 4-1BB-binding molecules is blotted against the units of released light (URL) measured after 6 h of incubation. All values are baseline corrected by subtracting the baseline values of the blank control (e.g. no antibodies added). In **Figures 47A****, D and G** FAP-target-independent 4-1BB activation is shown, whereby 4-1BB-binding induces NFκB-controlled luciferase expression in the reporter cell line without any FAP-mediated crosslinking. No construct is able to induce FAP-targeted-independent activation. In **Figures 47B****,** **47E** **and** **47H** FAP-intermediate expressing human melanoma cell line WM-266-4 and in **Figures 47C****,** **47F** **and** **47I** high-FAP-expressing cell line NIH/3T3-huFAP clone 19 was added in a ratio 5:1 to the reporter cell line. The FAP-expressing cells lead to a crosslinking of FAP-targeted 4-1BB-binding molecules and increase their potential to induce NFkB/luficerase activation in the 4-1BB-expressing reporter cell line. The tetravalent 4-1BB FAP-targeted 4+1 molecules show the strongest activation as soon as FAP-expressing cells are present. This is true for clone 12B3 (half-filled grey square, half-dotted line, **Figure 47B and C**), clone 11D5 (grey star, dotted line, **Figure 47E** **anf F**) and clone 25G7 (half-filled triangle, dotted line, **Figure 47H and I**).

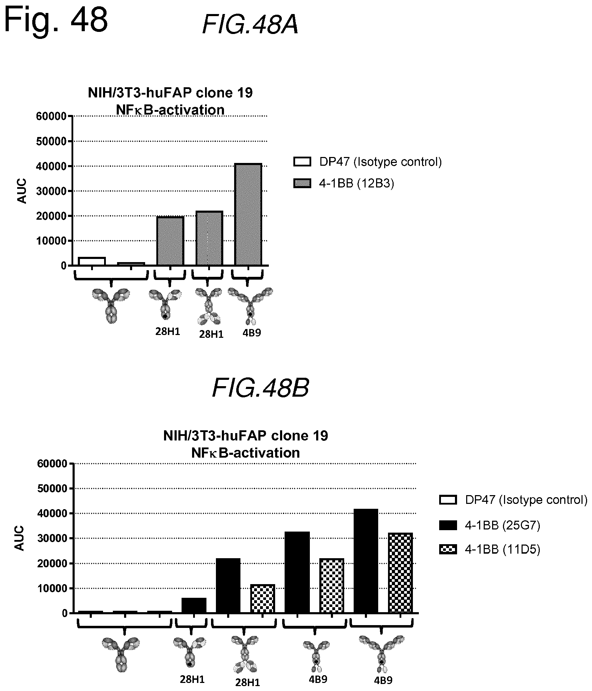

**Figure 48** summarizes the NFκB-mediated luciferase activity in the 4-1BB-expressing reporter cell line in the presence of NIH/3T3-huFAP cells. Shown is the area under the curve (AUC) of the activation curves in the presence of NIH/3T3-huFAP cells (shown in Figures 47C, F and I). Used antibody formats and anti-FAP clones (28H1 or 4B9) are indicated as pictograms under the graph, the different agonistic 4-1BB clones are indicated with different column colors. The Isotype control DP47 is indicated in white (baseline), the 4-1BB-specific clone 12B3 is indicated in grey, the 4-1BB-specific clone 25G7 in black and the 4-1BB-specific clone 11D5 in back-white-check. The graph shows that only FAP-targeted molecules can induce a strong activation above background and that the FAP (4B9)-targeted 4+1 constructs are superior to all other tested molecules.

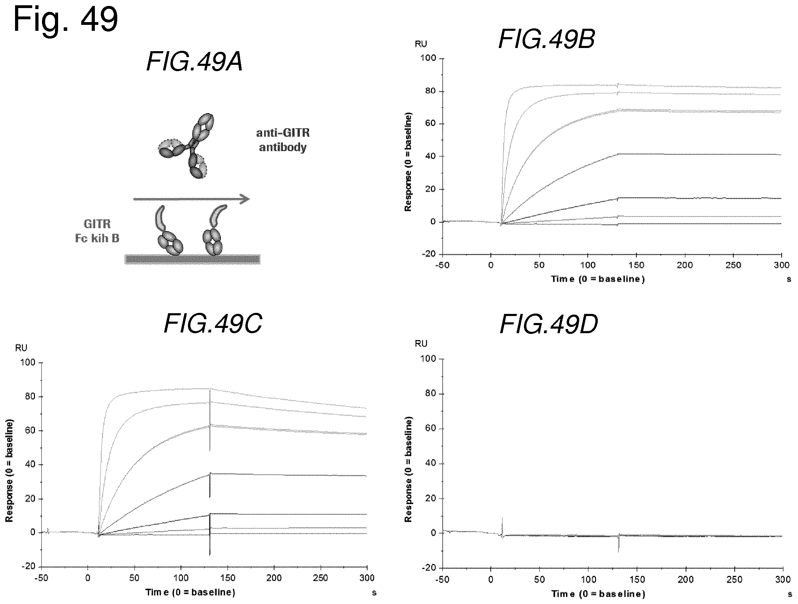

**Figures 49A to 49D** relate to the measurement of cross-specificity for anti-GITR antibody 8A06 as described in Example 11.4.1.1. Figure 49A is a schematic scheme of the assay set up for the determination of species cross-reactivity. Figure 49B shows the specificity of antibody 8A06 for human GITR. The specificity for cynomolgus GITR is shown in Figure 49C and Figure 49D shows that 8A06 does not bind to murine GITR.

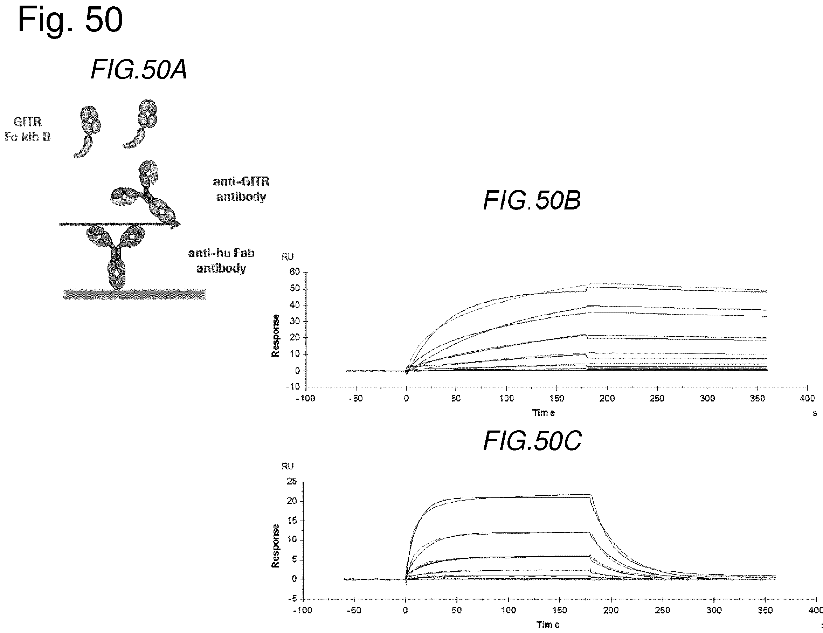

The Assay set up for determination of affinity of GITR specific antibody 8A06 to human and cynomolgus GITR is shown in **Figure 50A. Figure 50B** shows the results for human GITR, whereas the results for cynomolgus GITR are shown in **Figure 50C****.**

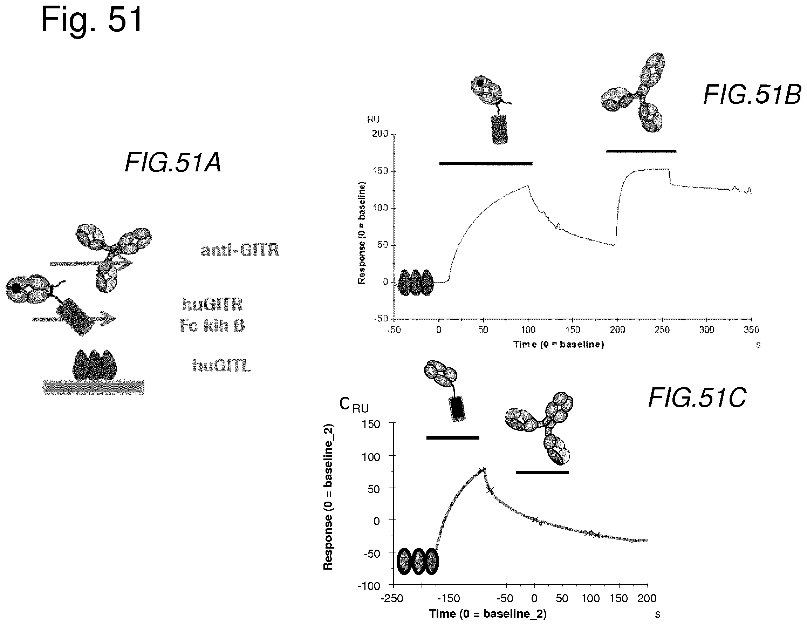

**Figures 51A to 51C** show the ligand blocking property of the anti-GITR clone 8A06 determined by surface plasmon resonance. The setup of the experiment (see Example 11.4.1.2) is shown in Figure 51A and the observed interaction between anti-GITR IgG 8A06 and the preformed complex huGITR / huGITR ligand can be seen in Figure 51B. Figure 51C shows the interaction between anti.GITR IgG 6C8 and and the preformed complex huGITR / huGITR ligand.

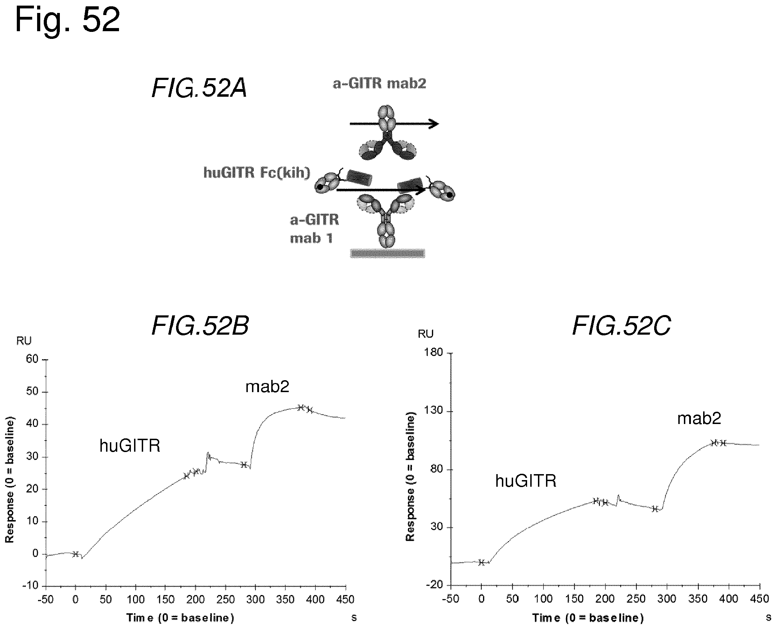

**Figures 52A to 52C** relate to the epitope binning described in Example 11.4.1.3. Measured was the binding of anti-GITR antibodies to preformed antibody complexed with GITR. In **Figure 52A** the setup of the assay is shown, the interaction between anti-GITR 8A06 IgG and the preformed complex of clone 6C8 / huGITR is shown in **Figure 52B** and the interaction between anti-GITR 6C8 IgG and the preformed complex of clone 8A06 / huGITR is presented in **Figure 52C****.**

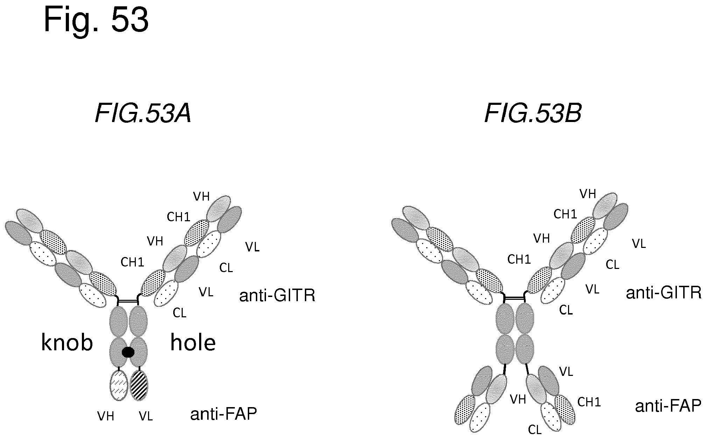

In **Figure 53A** is shown a schematic scheme of an exemplary bispecific, tetravalent anti-GITR antigen binding molecule of the invention with monovalency for FAP (4+1 format). The black point symbolizes the knob-into-hole modifications to allow better pairing of the two deiffernt heavy chains. **Figure 53B** is a schematic scheme of an exemplary bispecific, tetravalent antigen binding molecule with bivalency for FAP (4+2 format) of the invention. The constant regions of the anti-GITR Fab fragments may contain modifications (charged residues) in order to allow better pairing with the light chains.

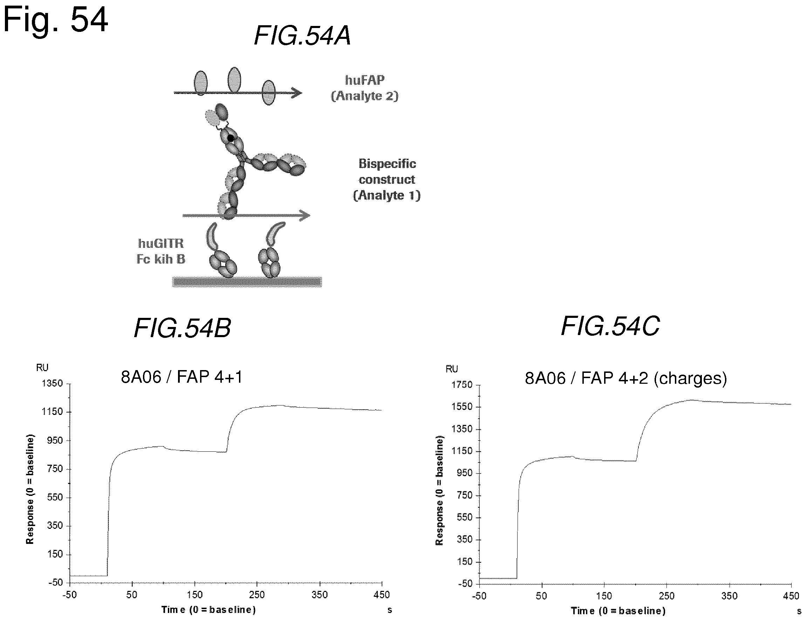

**Figures 54A to 54C** show the simultaneous binding of bispecific 4+1 and 4+2 anti-GITR x anti-FAP constructs. The setup of the experiment is shown in **Figure 54A**. Simultaneous binding of the bispecific 4+1 construct containing anti-GITR clone 8A06 (analyte 1) to immobilized human GITR and human FAP (analyte 2) is shown in **Figure 54B****.** Simultaneous binding of the bispecific 4+2 construct containing anti-GITR clone 8A06 (analyte 1) to immobilized human GITR and human FAP (analyte 2) is illustrated in **Figure 54C**.

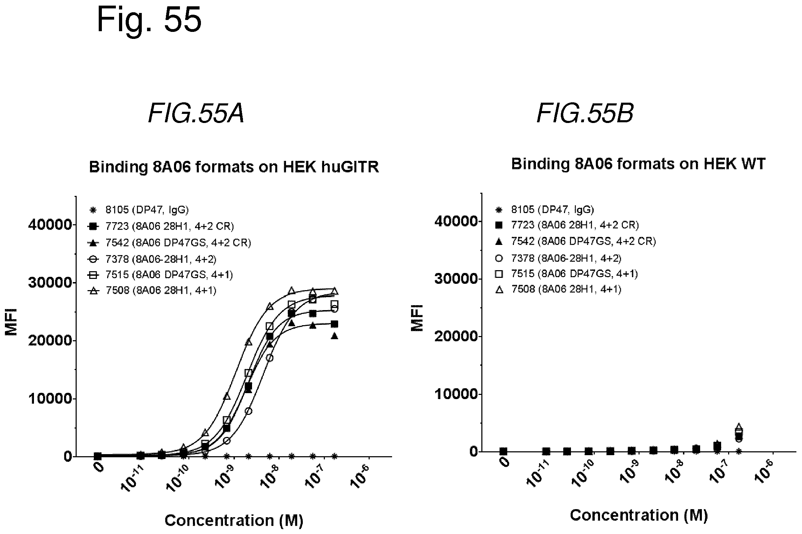

**Figures 55A** and **55B** relate to the binding to GITR-expressing HEK cells. As shown in **Figure 55A**, all tested bispecific anti-GITR constructs bound efficiently to human GITR expressing HEK cells, but not, or at negligible rates, to GITR negative control HEK cells **(****Figure 55B****).** The GITR specificity of the anti-GITR clone is thus maintained also in the tetravalent, bispecific, FAP targeted format. All constructs are made with anti-GITR clone 8A06 and anti-FAP clone 28H1 or, in a untargeted version, with germline control DP47GS. 4+2 means a tetravalent bispecific antigen binding molecule with 4 anti-GITR moieties and 2 anti-FAP moieties as disclosed herein, whereas 4+2 CR refers to the molecule with additional charged residues as described herein.

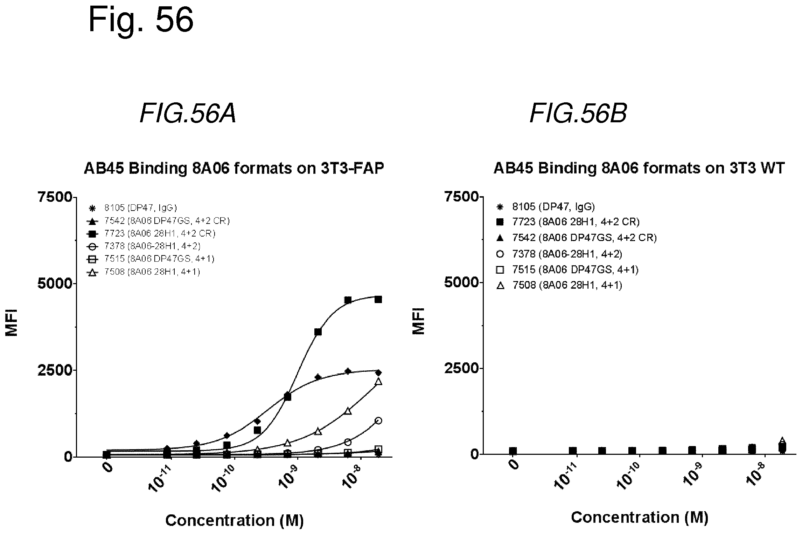

**Figures 56A** and **56B** relate to the binding to FAP-expressing 3T3 cells. As shown in **Figure 56A**, tested bispecific anti-GITR x anti-FAP constructs were able to bind to human FAP expressing 3T3 cells, but not, or at negligible rates, to FAP-negative control 3T3 cells **(****Figure 56B****).** The FAP specificity of the anti-FAP clone is thus maintained also in the tetravalent, bispecific format with a monovalent or bivalent FAP targeting moiety at the C-terminus.

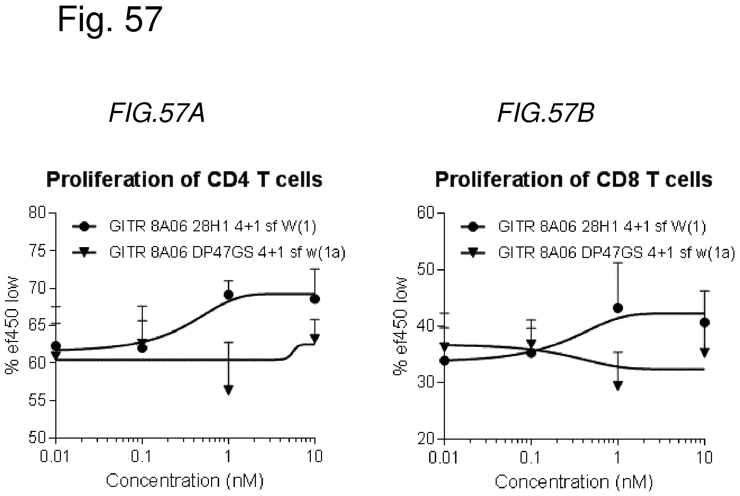

**Figures 57A and 57B** show the GITR-mediated costimulation of suboptimal stimulated TCR. Costimulation with non-targeted tetravalent anti-GITR antigen binding molecule (GITR 8A06 DP47GS 4+1 sf w(1a)) (triangle) did not have an effect on the proliferative capacity of suboptimally TCR stimulated CD4 (Figure 57A) and CD8 T cells (Figure 57B), whereas hypercrosslinking of the FAP- targeted anti-GITR construct (GITR 8A06 28H1 4+1 sf W(1)) (circle) by the present NIH/3T3-huFAP cells is needed for T cell costimulation and promoted proliferation.

### DETAILED DESCRIPTION OF THE INVENTION

### Definitions

Unless defined otherwise, technical and scientific terms used herein have the same meaning as generally used in the art to which this invention belongs. For purposes of interpreting this specification, the following definitions will apply and whenever appropriate, terms used in the singular will also include the plural and vice versa.

As used herein, the term **"antigen binding molecule"** refers in its broadest sense to a molecule that specifically binds an antigenic determinant. Examples of antigen binding molecules are antibodies, antibody fragments and scaffold antigen binding proteins.

As used herein, the term "moiety **capable of specific binding to a target cell antigen"** refers to a polypeptide molecule that specifically binds to an antigenic determinant. In one aspect, the antigen binding moiety is able to activate signaling through its target cell antigen. In a particular aspect, the antigen binding moiety is able to direct the entity to which it is attached (e.g. the TNF family ligand trimer) to a target site, for example to a specific type of tumor cell or tumor stroma bearing the antigenic determinant. Moieties capable of specific binding to a target cell antigen include antibodies and fragments thereof as further defined herein. In addition, moieties capable of specific binding to a target cell antigen include scaffold antigen binding proteins as further defined herein, e.g. binding domains which are based on designed repeat proteins or designed repeat domains (see e.g. WO 2002/020565).

In relation to an antibody or fragment thereof, the term "moiety capable of specific binding to a target cell antigen" refers to the part of the molecule that comprises the area which specifically binds to and is complementary to part or all of an antigen. A moiety capable of specific antigen binding may be provided, for example, by one or more antibody variable domains (also called antibody variable regions). Particularly, a moiety capable of specific antigen binding comprises an antibody light chain variable region (VL) and an antibody heavy chain variable region (VH). In one aspect, the "moiety capable of specific binding to a target cell antigen " is a Fab fragment or a cross-Fab fragment.

The term **"moiety capable of specific binding to a costimulatory TNF receptor family member** " refers to a polypeptide molecule that specifically binds to a costimulatory TNF receptor superfamily member. In one aspect, the antigen binding moiety is able to activate signaling through a costimulatory TNF receptor family member. Moieties capable of specific binding to a target cell antigen include antibodies and fragments thereof as further defined herein. In addition, moieties capable of specific binding to a costimulatory TNF receptor family member include scaffold antigen binding proteins as further defined herein, e.g. binding domains which are based on designed repeat proteins or designed repeat domains (see e.g. WO 2002/020565). Particularly, a moiety capable of specific binding to a costimulatory TNF receptor family member comprises an antibody light chain variable region (VL) and an antibody heavy chain variable region (VH). In a particular aspect, the "moiety capable of specific binding to a costimulatory TNF receptor family member" is an antibody fragment. In one aspect, the "moiety capable of specific binding to a costimulatory TNF receptor family member" is selected from the group consisting of single-chain antibody molecules (e.g. scFv), dual variable domains (DVD) and single domain antibodies, Fab fragments and cross-Fab fragments. More particularly, the "moiety capable of specific binding to a costimulatory TNF receptor family member" is a Fab fragment or a cross-Fab fragment.

The term **"antibody"** herein is used in the broadest sense and encompasses various antibody structures, including but not limited to monoclonal antibodies, polyclonal antibodies, monospecific and multispecific antibodies (e.g., bispecific antibodies), and antibody fragments so long as they exhibit the desired antigen-binding activity.

The term **"monoclonal antibody"** as used herein refers to an antibody obtained from a population of substantially homogeneous antibodies, i.e., the individual antibodies comprising the population are identical and/or bind the same epitope, except for possible variant antibodies, e.g. containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen.

The term **"monospecific"** antibody as used herein denotes an antibody that has one or more binding sites each of which bind to the same epitope of the same antigen. The term **"bispecific"** means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term **"valent"** as used within the current application denotes the presence of a specified number of binding sites specific for one distinct antigenic determinant in an antigen binding molecule that are specific for one distinct antigenic determinant. As such, the terms "bivalent", "tetravalent", and "hexavalent" denote the presence of two binding sites, four binding sites, and six binding sites specific for a certain antigenic determinant, respectively, in an antigen binding molecule. In particular aspects of the invention, the bispecific antigen binding molecules according to the invention can be monovalent for a certain antigenic determinant, meaning that they have only one binding site for said antigenic determinant or they can be bivalent for a certain antigenic determinant, meaning that they have two binding sites for said antigenic determinant. Particular molecules of the invention are **tetravalent** for a costimulatory TNF receptor, meaning that they have four binding sites for a costimulatory TNF receptor.

The terms "full length antibody", "intact antibody", and "whole antibody" are used herein interchangeably to refer to an antibody having a structure substantially similar to a native antibody structure. **"Native antibodies"** refer to naturally occurring immunoglobulin molecules with varying structures. For example, native IgG-class antibodies are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a light chain constant domain (CL), also called a light chain constant region. The heavy chain of an antibody may be assigned to one of five types, called α (IgA), δ (IgD), ε (IgE), γ (IgG), or µ (IgM), some of which may be further divided into subtypes, e.g. γ1 (IgG1), γ2 (IgG2), γ3 (IgG3), γ4 (IgG4), α1 (IgA1) and α2 (IgA2). The light chain of an antibody may be assigned to one of two types, called kappa (κ) and lambda (λ), based on the amino acid sequence of its constant domain.

An "**antibody fragment**" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies, triabodies, tetrabodies, cross-Fab fragments; linear antibodies; single-chain antibody molecules (e.g. scFv), dual variable domain (DVD) and single domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046. Diabodies are antibody fragments with two antigen-binding sites that may be bivalent or bispecific, see, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

Papain digestion of intact antibodies produces two identical antigen-binding fragments, called "Fab" fragments containing each the heavy- and light-chain variable domains and also the constant domain of the light chain and the first constant domain (CH1) of the heavy chain. As used herein, Thus, the term "**Fab fragment**" refers to an antibody fragment comprising a light chain fragment comprising a VL domain and a constant domain of a light chain (CL), and a VH domain and a first constant domain (CH1) of a heavy chain. Fab' fragments differ from Fab fragments by the addition of a few residues at the carboxy terminus of the heavy chain CH1 domain including one or more cysteins from the antibody hinge region. Fab'-SH are Fab' fragments wherein the cysteine residue(s) of the constant domains bear a free thiol group. Pepsin treatment yields an F(ab')₂ fragment that has two antigen-combining sites (two Fab fragments) and a part of the Fc region. According to the present invention, the term "Fab fragment" also includes "cross-Fab fragments" or "crossover Fab fragments" as defined below.

The term **"cross-Fab fragment"** or "xFab fragment" or "crossover Fab fragment" refers to a Fab fragment, wherein either the variable regions or the constant regions of the heavy and light chain are exchanged. Two different chain compositions of a crossover Fab molecule are possible and comprised in the bispecific antibodies of the invention: On the one hand, the variable regions of the Fab heavy and light chain are exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as CrossFab _{(VLVH)}. On the other hand, when the constant regions of the Fab heavy and light chain are exchanged, the crossover Fab molecule comprises a peptide chain composed of the heavy chain variable region (VH) and the light chain constant region (CL), and a peptide chain composed of the light chain variable region (VL) and the heavy chain constant region (CH1). This crossover Fab molecule is also referred to as CrossFab _{(CLCH1)}.

A "single chain Fab fragment" or "**scFab**" is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CH1-linker-VL-CL, b) VL-CL-linker-VH-CH1, c) VH-CL-linker-VL-CH1 or d) VL-CH1-linker-VH-CL; and wherein said linker is a polypeptide of at least 30 amino acids, preferably between 32 and 50 amino acids. Said single chain Fab fragments are stabilized via the natural disulfide bond between the CL domain and the CH1 domain. In addition, these single chain Fab molecules might be further stabilized by generation of interchain disulfide bonds via insertion of cysteine residues (e.g. position 44 in the variable heavy chain and position 100 in the variable light chain according to Kabat numbering).

A "crossover single chain Fab fragment" or **"x-scFab"** is a is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CL-linker-VL-CH1 and b) VL-CH1-linker-VH-CL; wherein VH and VL form together an antigen-binding site which binds specifically to an antigen and wherein said linker is a polypeptide of at least 30 amino acids. In addition, these x-scFab molecules might be further stabilized by generation of interchain disulfide bonds via insertion of cysteine residues (e.g. position 44 in the variable heavy chain and position 100 in the variable light chain according to Kabat numbering).

A "**single-chain variable fragment** (**scFv**)" is a fusion protein of the variable regions of the heavy (V_{H}) and light chains (V_{L}) of an antibody, connected with a short linker peptide of ten to about 25 amino acids. The linker is usually rich in glycine for flexibility, as well as serine or threonine for solubility, and can either connect the N-terminus of the V_{H} with the C-terminus of the V_{L}, or *vice versa.* This protein retains the specificity of the original antibody, despite removal of the constant regions and the introduction of the linker. scFv antibodies are, e.g. described in Houston, J. S., Methods in Enzymol. 203 (1991) 46-96). In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full length antibodies.

The term **"Dual Variable Domain"** or "DVD" refers to antigen binding molecule comprising two heavy chain polypeptides with two variable domains and two light chain polypeptides with two variable domains. In an DVD antibody ("DVD-Ig") each half comprises a heavy chain polypeptide and a light chain polypeptide with two target binding sites. Each binding site comprises a heavy chain variable domain and a light chain variable domain with a total of 6 CDRs involved in target binding. Each variable domain (VD) in a DVD-Ig protein may be obtained from one or more "parent" monoclonal antibodies (mAbs) that bind one or more desired antigens or epitopes. The resulting DVD-Ig molecule retains activities of both parental mAbs. For further details see e.g. WO 2008/024188.

**"Scaffold antigen binding proteins"** are known in the art, for example, fibronectin and designed ankyrin repeat proteins (DARPins) have been used as alternative scaffolds for antigen-binding domains, see, e.g., Gebauer and Skerra, Engineered protein scaffolds as next-generation antibody therapeutics. Curr Opin Chem Biol 13:245-255 (2009) and Stumpp et al., Darpins: A new generation of protein therapeutics. Drug Discovery Today 13: 695-701 (2008). In one aspect of the invention, a scaffold antigen binding protein is selected from the group consisting of CTLA-4 (Evibody), Lipocalins (Anticalin), a Protein A-derived molecule such as Z-domain of Protein A (Affibody), an A-domain (Avimer/Maxibody), a serum transferrin *(trans-body);* a designed ankyrin repeat protein (DARPin), a variable domain of antibody light chain or heavy chain (single-domain antibody, sdAb), a variable domain of antibody heavy chain (nanobody, aVH), V_{NAR} fragments, a fibronectin (AdNectin), a C-type lectin domain (Tetranectin); a variable domain of a new antigen receptor beta-lactamase (V_{NAR} fragments), a human gammacrystallin or ubiquitin (Affilin molecules); a kunitz type domain of human protease inhibitors, microbodies such as the proteins from the knottin family, peptide aptamers and fibronectin (adnectin). CTLA-4 (Cytotoxic T Lymphocyte-associated Antigen 4) is a CD28-family receptor expressed on mainly CD4+ T-cells. Its extracellular domain has a variable domain- like Ig fold. Loops corresponding to CDRs of antibodies can be substituted with heterologous sequence to confer different binding properties. CTLA-4 molecules engineered to have different binding specificities are also known as Evibodies (e.g. US7166697B1). Evibodies are around the same size as the isolated variable region of an antibody (e.g. a domain antibody). For further details see Journal of Immunological Methods 248 (1-2), 31-45 (2001). Lipocalins are a family of extracellular proteins which transport small hydrophobic molecules such as steroids, bilins, retinoids and lipids. They have a rigid beta-sheet secondary structure with a number of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633. An affibody is a scaffold derived from Protein A of Staphylococcus aureus which can be engineered to bind to antigen. The domain consists of a three-helical bundle of approximately 58 amino acids. Libraries have been generated by randomization of surface residues. For further details see Protein Eng. Des. Sel. 2004, 17, 455-462 and EP 1641818A1. Avimers are multidomain proteins derived from the A-domain scaffold family. The native domains of approximately 35 amino acids adopt a defined disulfide bonded structure. Diversity is generated by shuffling of the natural variation exhibited by the family of A-domains. For further details see Nature Biotechnology 23(12), 1556 - 1561 (2005) and Expert Opinion on Investigational Drugs 16(6), 909-917 (June 2007). A transferrin is a monomeric serum transport glycoprotein. Transferrins can be engineered to bind different target antigens by insertion of peptide sequences in a permissive surface loop. Examples of engineered transferrin scaffolds include the Trans-body. For further details see J. Biol. Chem 274, 24066-24073 (1999). Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two alpha-helices and a beta-turn. They can be engineered to bind different target antigens by randomizing residues in the first alpha-helix and a beta-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028A1. A single-domain antibody is an antibody fragment consisting of a single monomeric variable antibody domain. The first single domains were derived from the variable domain of the antibody heavy chain from camelids (nanobodies or V_{H}H fragments). Furthermore, the term single-domain antibody includes an autonomous human heavy chain variable domain (aVH) or V_{NAR} fragments derived from sharks. Fibronectin is a scaffold which can be engineered to bind to antigen. Adnectins consists of a backbone of the natural amino acid sequence of the 10th domain of the 15 repeating units of human fibronectin type III (FN3). Three loops at one end of the .beta.-sandwich can be engineered to enable an Adnectin to specifically recognize a therapeutic target of interest. For further details see Protein Eng. Des. Sel. 18, 435- 444 (2005), US20080139791, WO2005056764 and US6818418B1. Peptide aptamers are combinatorial recognition molecules that consist of a constant scaffold protein, typically thioredoxin (TrxA) which contains a constrained variable peptide loop inserted at the active site. For further details see Expert Opin. Biol. Ther. 5, 783-797 (2005). Microbodies are derived from naturally occurring microproteins of 25-50 amino acids in length which contain 3-4 cysteine bridges - examples of microproteins include KalataBI and conotoxin and knottins. The microproteins have a loop which can beengineered to include upto 25 amino acids without affecting the overall fold of the microprotein. For further details of engineered knottin domains, see WO2008098796.

An "**antigen binding molecule that binds to the same epitope**" as a reference molecule refers to an antigen binding molecule that blocks binding of the reference molecule to its antigen in a competition assay by 50% or more, and conversely, the reference molecule blocks binding of the antigen binding molecule to its antigen in a competition assay by 50% or more.

The term **"antigen binding domain"** or "antigen-binding site" refers to the part of an antigen binding molecule that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more variable domains (also called variable regions). Preferably, an antigen binding domain comprises an antibody light chain variable region (VL) and an antibody heavy chain variable region (VH).

As used herein, the term **"antigenic determinant"** is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins useful as antigens herein can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants.

By **"specific binding"** is meant that the binding is selective for the antigen and can be discriminated from unwanted or non-specific interactions. The ability of an antigen binding molecule to bind to a specific antigen can be measured either through an enzyme-linked immunosorbent assay (ELISA) or other techniques familiar to one of skill in the art, e.g. Surface Plasmon Resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding molecule to an unrelated protein is less than about 10% of the binding of the antigen binding molecule to the antigen as measured, e.g. by SPR. In certain embodiments, an molecule that binds to the antigen has a dissociation constant (Kd) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g. from 10⁻⁹ M to 10⁻¹³ M).

"**Affinity**" or "binding affinity" refers to the strength of the sum total of non-covalent interactions between a single binding site of a molecule (e.g. an antibody) and its binding partner (e.g. an antigen). Unless indicated otherwise, as used herein, "binding affinity" refers to intrinsic binding affinity which reflects a 1:1 interaction between members of a binding pair (e.g. antibody and antigen). The affinity of a molecule X for its partner Y can generally be represented by the dissociation constant (Kd), which is the ratio of dissociation and association rate constants (koff and kon, respectively). Thus, equivalent affinities may comprise different rate constants, as long as the ratio of the rate constants remains the same. Affinity can be measured by common methods known in the art, including those described herein. A particular method for measuring affinity is Surface Plasmon Resonance (SPR).

An **"affinity matured"** antibody refers to an antibody with one or more alterations in one or more hypervariable regions (HVRs), compared to a parent antibody which does not possess such alterations, such alterations resulting in an improvement in the affinity of the antibody for antigen.

A **"target cell antigen"** as used herein refers to an antigenic determinant presented on the surface of a target cell, for example a cell in a tumor such as a cancer cell or a cell of the tumor stroma. In certain embodiments, the target cell antigen is an antigen on the surface of a tumor cell. In one embodiment, target cell antigen is selected from the group consisting of Fibroblast Activation Protein (FAP), Carcinoembryonic Antigen (CEA), Melanoma-associated Chondroitin Sulfate Proteoglycan (MCSP), Epidermal Growth Factor Receptor (EGFR), CD19, CD20 and CD33. In particular, the target cell antigen is Fibroblast Activation Protein (FAP).

The term **"Fibroblast activation protein (FAP)",** also known as Prolyl endopeptidase FAP or Seprase (EC 3.4.21), refers to any native FAP from any vertebrate source, including mammals such as primates (e.g. humans) non-human primates (e.g. cynomolgus monkeys) and rodents (e.g. mice and rats), unless otherwise indicated. The term encompasses "full-length," unprocessed FAP as well as any form of FAP that results from processing in the cell. The term also encompasses naturally occurring variants of FAP, e.g., splice variants or allelic variants. In one embodiment, the antigen binding molecule of the invention is capable of specific binding to human, mouse and/or cynomolgus FAP. The amino acid sequence of human FAP is shown in UniProt (www.uniprot.org) accession no. Q12884 (version 149, SEQ ID NO:55), or NCBI (www.ncbi.nlm.nih.gov/) RefSeq NP_004451.2. The extracellular domain (ECD) of human FAP extends from amino acid position 26 to 760. The amino acid and nucleotide sequences of a His-tagged human FAP ECD is shown in SEQ ID NOs 56 and 57, respectively. The amino acid sequence of mouse FAP is shown in UniProt accession no. P97321 (version 126, SEQ ID NO:58), or NCBI RefSeq NP_032012.1. The extracellular domain (ECD) of mouse FAP extends from amino acid position 26 to 761. SEQ ID NOs 59 and 60 show the amino acid and nucleotide sequences, respectively, of a His-tagged mouse FAP ECD. SEQ ID NOs 61 and 62 show the amino acid and nucleotide sequences, respectively, of a His-tagged cynomolgus FAP ECD. Preferably, an anti-FAP binding molecule of the invention binds to the extracellular domain of FAP.

The term **"Carcinoembroynic antigen (CEA)",** also known as Carcinoembryonic antigen-related cell adhesion molecule 5 (CEACAM5), refers to any native CEA from any vertebrate source, including mammals such as primates (e.g. humans) non-human primates (e.g. cynomolgus monkeys) and rodents (e.g. mice and rats), unless otherwise indicated. The amino acid sequence of human CEA is shown in UniProt accession no. P06731 (version 151, SEQ ID NO:63). The term **"Melanoma-associated Chondroitin Sulfate Proteoglycan (MCSP)",** also known as Chondroitin Sulfate Proteoglycan 4 (CSPG4) refers to any native MCSP from any vertebrate source, including mammals such as primates (e.g. humans) non-human primates (e.g. cynomolgus monkeys) and rodents (e.g. mice and rats), unless otherwise indicated. The amino acid sequence of human MCSP is shown in UniProt accession no. Q6UVK1 (version 103, SEQ ID NO:64). The term "**Epidermal Growth Factor Receptor (EGFR)**", also named Protooncogene c-ErbB-1 or Receptor tyrosine-protein kinase erbB-1, refers to any native EGFR from any vertebrate source, including mammals such as primates (e.g. humans) non-human primates (e.g. cynomolgus monkeys) and rodents (e.g. mice and rats), unless otherwise indicated. The amino acid sequence of human EGFR is shown in UniProt accession no. P00533 (version 211, SEQ ID NO:65). The term "**CD19**" refers to B-lymphocyte antigen CD19, also known as B-lymphocyte surface antigen B4 or T-cell surface antigen Leu-12 and includes any native CD19 from any vertebrate source, including mammals such as primates (e.g. humans) non-human primates (e.g. cynomolgus monkeys) and rodents (e.g. mice and rats), unless otherwise indicated. The amino acid sequence of human CD19 is shown in Uniprot accession no. P15391 (version 160, SEQ ID NO:66). "**CD20**" refers to B-lymphocyte antigen CD20, also known as membranespanning 4-domains subfamily A member 1 (MS4A1), B-lymphocyte surface antigen B1 or Leukocyte surface antigen Leu-16, and includes any native CD20 from any vertebrate source, including mammals such as primates (e.g. humans) non-human primates (e.g. cynomolgus monkeys) and rodents (e.g. mice and rats), unless otherwise indicated. The amino acid sequence of human CD20 is shown in Uniprot accession no. P11836 (version 149, SEQ ID NO:67). "**CD33**" refers to Myeloid cell surface antigen CD33, also known as SIGLEC3 or gp67, and includes any native CD33 from any vertebrate source, including mammals such as primates (e.g. humans) non-human primates (e.g. cynomolgus monkeys) and rodents (e.g. mice and rats), unless otherwise indicated. The amino acid sequence of human CD33 is shown in Uniprot accession no. P20138 (version 157, SEQ ID NO:68).

The term **"variable region"** or "variable domain" refers to the domain of an antibody heavy or light chain that is involved in binding the antigen binding molecule to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity.

The term **"hypervariable region"** or "HVR," as used herein refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (HI), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) Hypervariable regions (HVRs) are also referred to as complementarity determining regions (CDRs), and these terms are used herein interchangeably in reference to portions of the variable region that form the antigen binding regions. This particular region has been described by Kabat et al., U.S. Dept. of Health and Human Services, "Sequences of Proteins of Immunological Interest" (1983) and by Chothia et al., J. Mol. Biol. 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table A as a comparison. The exact residue numbers which encompass a particular CDR will vary depending on the sequence and size of the CDR. Those skilled in the art can routinely determine which residues comprise a particular CDR given the variable region amino acid sequence of the antibody.

**TABLE A. CDR Definitions¹**

| **CDR** | **Kabat** | **Chothia** | **AbM²** |
|---|---|---|---|
| V_{H} CDR1 | 31-35 | 26-32 | 26-35 |
| V_{H} CDR2 | 50-65 | 52-58 | 50-58 |
| V_{H} CDR3 | 95-102 | 95-102 | 95-102 |
| V_{L} CDR1 | 24-34 | 26-32 | 24-34 |
| V_{L} CDR2 | 50-56 | 50-52 | 50-56 |
| V_{L} CDR3 | 89-97 | 91-96 | 89-97 |

| | | | |
|---|---|---|---|
| ¹ Numbering of all CDR definitions in Table A is according to the numbering conventions set forth by Kabat et al. (see below). ² "AbM" with a lowercase "b" as used in Table A refers to the CDRs as defined by Oxford Molecular's "AbM" antibody modeling software. | | | |

Kabat *et al.* also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., *supra.*

"**Framework**" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The term "**chimeric**" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g. IgG₁, IgG₂, IgG₃, IgG₄, IgA₁, and IgA₂. The heavy chain constant domains that correspond to the different classes of immunoglobulins are called α, δ, ε, γ, and µ respectively..

A "**humanized**" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A **"humanized form"** of an antibody, e.g., a non-human antibody, refers to an antibody that has undergone humanization. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

A **"human"** antibody is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "Fc domain" or **"Fc region"** herein is used to define a C-terminal region of an antibody heavy chain that contains at least a portion of the constant region. The term includes native sequence Fc regions and variant Fc regions. An IgG Fc region comprises an IgG CH2 and an IgG CH3 domain. The "CH2 domain" of a human IgG Fc region usually extends from an amino acid residue at about position 231 to an amino acid residue at about position 340. In one embodiment, a carbohydrate chain is attached to the CH2 domain. The CH2 domain herein may be a native sequence CH2 domain or variant CH2 domain. The "CH3 domain" comprises the stretch of residues C-terminal to a CH2 domain in an Fc region (i.e. from an amino acid residue at about position 341 to an amino acid residue at about position 447 of an IgG). The CH3 region herein may be a native sequence CH3 domain or a variant CH3 domain (e.g. a CH3 domain with an introduced "protuberance" ("knob") in one chain thereof and a corresponding introduced "cavity" ("hole") in the other chain thereof; see US Patent No. 5,821,333, expressly incorporated herein by reference). Such variant CH3 domains may be used to promote heterodimerization of two non-identical antibody heavy chains as herein described. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The **"knob-into-hole"** technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine). The protuberance and cavity can be made by altering the nucleic acid encoding the polypeptides, e.g. by site-specific mutagenesis, or by peptide synthesis. In a specific embodiment a knob modification comprises the amino acid substitution T366W in one of the two subunits of the Fc domain, and the hole modification comprises the amino acid substitutions T366S, L368A and Y407V in the other one of the two subunits of the Fc domain. In a further specific embodiment, the subunit of the Fc domain comprising the knob modification additionally comprises the amino acid substitution S354C, and the subunit of the Fc domain comprising the hole modification additionally comprises the amino acid substitution Y349C. Introduction of these two cysteine residues results in the formation of a disulfide bridge between the two subunits of the Fc region, thus further stabilizing the dimer (Carter, J Immunol Methods 248, 7-15 (2001)).

A "region equivalent to the Fc region of an immunoglobulin" is intended to include naturally occurring allelic variants of the Fc region of an immunoglobulin as well as variants having alterations which produce substitutions, additions, or deletions but which do not decrease substantially the ability of the immunoglobulin to mediate effector functions (such as antibody-dependent cellular cytotoxicity). For example, one or more amino acids can be deleted from the N-terminus or C-terminus of the Fc region of an immunoglobulin without substantial loss of biological function. Such variants can be selected according to general rules known in the art so as to have minimal effect on activity (see, e.g., Bowie, J. U. et al., Science 247:1306-10 (1990)).

The term "**effector functions**" refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

Fc receptor binding dependent effector functions can be mediated by the interaction of the Fc-region of an antibody with Fc receptors (FcRs), which are specialized cell surface receptors on hematopoietic cells. Fc receptors belong to the immunoglobulin superfamily, and have been shown to mediate both the removal of antibody-coated pathogens by phagocytosis of immune complexes, and the lysis of erythrocytes and various other cellular targets (e.g. tumor cells) coated with the corresponding antibody, via antibody dependent cell mediated cytotoxicity (ADCC) (see e.g. Van de Winkel, J.G. and Anderson, C.L., J. Leukoc. Biol. 49 (1991) 511-524). FcRs are defined by their specificity for immunoglobulin isotypes: Fc receptors for IgG antibodies are referred to as FcyR. Fc receptor binding is described e.g. in Ravetch, J.V. and Kinet, J.P., Annu. Rev. Immunol. 9 (1991) 457-492; Capel, P.J., et al., Immunomethods 4 (1994) 25-34; de Haas, M., et al., J. Lab. Clin. Med. 126 (1995) 330-341; and Gessner, J.E., et al., Ann. Hematol. 76 (1998) 231-248.

Cross-linking of receptors for the Fc-region of IgG antibodies (FcyR) triggers a wide variety of effector functions including phagocytosis, antibody-dependent cellular cytotoxicity, and release of inflammatory mediators, as well as immune complex clearance and regulation of antibody production. In humans, three classes of FcyR have been characterized, which are:
- FcyRI (CD64) binds monomeric IgG with high affinity and is expressed on macrophages, monocytes, neutrophils and eosinophils. Modification in the Fc-region IgG at least at one of the amino acid residues E233-G236, P238, D265, N297, A327 and P329 (numbering according to EU index of Kabat) reduce binding to FcγRI. IgG2 residues at positions 233-236, substituted into IgG1 and IgG4, reduced binding to FcγRI by 10³-fold and eliminated the human monocyte response to antibody-sensitized red blood cells (Armour, K.L., et al., Eur. J. Immunol. 29 (1999) 2613-2624).
- FcyRII (CD32) binds complexed IgG with medium to low affinity and is widely expressed. This receptor can be divided into two sub-types, FcyRIIA and FcyRIIB. FcyRIIA is found on many cells involved in killing (e.g. macrophages, monocytes, neutrophils) and seems able to activate the killing process. FcγRIIB seems to play a role in inhibitory processes and is found on B cells, macrophages and on mast cells and eosinophils. On B-cells it seems to function to suppress further immunoglobulin production and isotype switching to, for example, the IgE class. On macrophages, FcγRIIB acts to inhibit phagocytosis as mediated through FcγRIIA. On eosinophils and mast cells the B-form may help to suppress activation of these cells through IgE binding to its separate receptor. Reduced binding for FcγRIIA is found e.g. for antibodies comprising an IgG Fc-region with mutations at least at one of the amino acid residues E233-G236, P238, D265, N297, A327, P329, D270, Q295, A327, R292, and K414 (numbering according to EU index of Kabat).
- FcγRIII (CD16) binds IgG with medium to low affinity and exists as two types. FcγRIIIA is found on NK cells, macrophages, eosinophils and some monocytes and T cells and mediates ADCC. Fc γ RIIIB is highly expressed on neutrophils. Reduced binding to FcyRIIIA is found e.g. for antibodies comprising an IgG Fc-region with mutation at least at one of the amino acid residues E233-G236, P238, D265, N297, A327, P329, D270, Q295, A327, S239, E269, E293, Y296, V303, A327, K338 and D376 (numbering according to EU index of Kabat).

Mapping of the binding sites on human IgG1 for Fc receptors, the above mentioned mutation sites and methods for measuring binding to FcyRI and FcyRIIA are described in Shields, R.L., et al. J. Biol. Chem. 276 (2001) 6591-6604.

The term **"ADCC"** or "antibody-dependent cellular cytotoxicity" is a function mediated by Fc receptor binding and refers to lysis of target cells by an antibody as reported herein in the presence of effector cells. The capacity of the antibody to induce the initial steps mediating ADCC is investigated by measuring their binding to Fcγ receptors expressing cells, such as cells, recombinantly expressing FcγRI and/or FcγRIIA or NK cells (expressing essentially FcγRIIIA). In particular, binding to FcγR on NK cells is measured.

An "**activating Fc receptor**" is an Fc receptor that following engagement by an Fc region of an antibody elicits signaling events that stimulate the receptor-bearing cell to perform effector functions. Activating Fc receptors include FcγRIIIa (CD16a), FcγRI (CD64), FcγRIIa (CD32), and FcaRI (CD89). A particular activating Fc receptor is human FcyRIIIa (see UniProt accession no. P08637, version 141).

The "Tumor Necrosis factor receptor superfamily" or **"TNF receptor superfamily"** currently consists of 27 receptors. It is a group of cytokine receptors characterized by the ability to bind tumor necrosis factors (TNFs) via an extracellular cysteine-rich domain (CRD). These pseudorepeats are defined by intrachain disulphides generated by highly conserved cysteine residues within the receptor chains. With the exception of nerve growth factor (NGF), all TNFs are homologous to the archetypal TNF-alpha. In their active form, the majority of TNF receptors form trimeric complexes in the plasma membrame. Accordingly, most TNF receptors contain transmembrane domains (TMDs). Several of these receptors also contain intracellular death domains (DDs) that recruit caspase-interacting proteins following ligand binding to initiate the extrinsic pathway of caspase activation. Other TNF superfamily receptors that lack death domains bind TNF receptor-associated factors and activate intracellular signaling pathways that can lead to proliferation or differentiation. These receptors can also initiate apoptosis, but they do so via indirect mechanisms. In addition to regulating apoptosis, several TNF superfamily receptors are involved in regulating immune cell functions such as B cell homeostasis and activation, natural killer cell activation, and T cell co-stimulation. Several others regulate cell type-specific responses such as hair follicle development and osteoclast development. Members of the TNF receptor superfamily include the following: Tumor necrosis factor receptor 1(1A) (TNFRSF1A, CD120a), Tumor necrosis factor receptor 2 (1B) (TNFRSF1B, CD120b), Lymphotoxin beta receptor (LTBR, CD18), OX40 (TNFRSF4, CD134), CD40 (Bp50), Fas receptor (Apo-1, CD95, FAS), Decoy receptor 3 (TR6, M68, TNFRSF6B), CD27 (S152, Tp55), CD30 (Ki-1, TNFRSF8), 4-1BB (CD137, TNFRSF9), DR4 (TRAILR1, Apo-2, CD261, TNFRSF10A), DR5 (TRAILR2, CD262, TNFRSF10B), Decoy Receptor 1 (TRAILR3, CD263, TNFRSF10C), Decoy Receptor 2 (TRAILR4, CD264, TNFRSF10D), RANK (CD265, TNFRSF11A), Osteoprotegerin (OCIF, TR1, TNFRSF11B), TWEAK receptor (Fn14, CD266, TNFRSF12A), TACI (CD267, TNFRSF13B), BAFF receptor (CD268, TNFRSF13C), Herpesvirus entry mediator (HVEM, TR2, CD270, TNFRSF14), Nerve growth factor receptor (p75NTR, CD271, NGFR), B-cell maturation antigen (CD269, TNFRSF17), Glucocorticoid-induced TNFR-related (GITR, AITR, CD357, TNFRSF18), TROY (TNFRSF19), DR6 (CD358, TNFRSF21), DR3 (Apo-3, TRAMP, WS-1, TNFRSF25) and Ectodysplasin A2 receptor (XEDAR, EDA2R).

Several members of the tumor necrosis factor receptor (TNFR) family function after initial T cell activation to sustain T cell responses. The term **"costimulatory TNF receptor family member"** or "costimulatory TNF receptor superfamily member" or "costimulatory TNF superfamily receptor" refers to a subgroup of TNF receptor superfamily members, which are able to costimulate proliferation and cytokine production of T-cells. The term refers to any native TNF family receptor from any vertebrate source, including mammals such as primates (e.g. humans), non-human primates (e.g. cynomolgus monkeys) and rodents (e.g. mice and rats), unless otherwise indicated. In specific embodiments of the invention, costimulatory TNF receptor superfamily members are selected from the group consisting of OX40 (CD134), 4-1BB (CD137), CD27, HVEM (CD270), CD30, and GITR, all of which can have costimulatory effects on T cells. More particularly, the costimulatory TNF receptor superfamily member is selected from the group consisting of OX40 and 4-1BB.

Further information, in particular sequences, of the TNF receptor superfamily members may be obtained from publically accessible databases such as Uniprot (www.uniprot.org). For instance, the human costimulatory TNF receptors have the following amino acid sequences: human OX40 (UniProt accession no. P43489, SEQ ID NO:69), human 4-1BB (UniProt accession no. Q07011, SEQ ID NO:70), human CD27 (UniProt accession no. P26842, SEQ ID NO:71), human HVEM (UniProt accession no. Q92956, SEQ ID NO:72), human CD30 (UniProt accession no. P28908, SEQ ID NO:73), and human GITR (UniProt accession no. Q9Y5U5, SEQ ID NO:74).

The term "**OX40**", as used herein, refers to any native OX40 from any vertebrate source, including mammals such as primates (e.g. humans) and rodents (e.g., mice and rats), unless otherwise indicated. The term encompasses "full-length," unprocessed OX40 as well as any form of OX40 that results from processing in the cell. The term also encompasses naturally occurring variants of OX40, e.g., splice variants or allelic variants. The amino acid sequence of an exemplary human OX40 is shown in SEQ ID NO: 1 (Uniprot P43489, version 112) and the amino acid sequence of an exemplary murine OX40 is shown in SEQ ID NO: 75 (Uniprot P47741, version 101).

The terms "**anti-OX40 antibody**", "anti-OX40", "OX40 antibody and "an antibody that specifically binds to OX40" refer to an antibody that is capable of binding OX40 with sufficient affinity such that the antibody is useful as a diagnostic and/or therapeutic agent in targeting OX40. In one embodiment, the extent of binding of an anti-OX40 antibody to an unrelated, non-OX40 protein is less than about 10% of the binding of the antibody to OX40 as measured, e.g., by a radioimmunoassay (RIA) or flow cytometry (FACS). In certain embodiments, an antibody that binds to OX40 has a dissociation constant ( K_{D}) of ≤ 1µM, ≤ 100 nM, ≤ 10 nM, ≤ 1 nM, ≤ 0.1 nM, ≤ 0.01 nM, or ≤ 0.001 nM (e.g. 10⁻⁶ M or less, e.g. from 10⁻⁶⁸ M to 10⁻¹³ M, e.g., from 10⁻⁸ M to 10⁻¹⁰ M).

The term "**4-1BB**", as used herein, refers to any native 4-1BB from any vertebrate source, including mammals such as primates (e.g. humans) and rodents (e.g., mice and rats), unless otherwise indicated. The term encompasses "full-length," unprocessed 4-1BB as well as any form of 4-1BB that results from processing in the cell. The term also encompasses naturally occurring variants of 4-1BB, e.g., splice variants or allelic variants. The amino acid sequence of an exemplary human 4-1BB is shown in SEQ ID NO: 70 (Uniprot accession no. Q07011), the amino acid sequence of an exemplary murine 4-1BB is shown in SEQ ID NO: 76 (Uniprot accession no. P20334) and the amino acid sequence of an exemplary cynomolgous 4-1BB (from Macaca mulatta) is shown in SEQ ID NO:77 (Uniprot accession no. F6W5G6).

The terms "**anti-4-1BB antibody**", "anti-4-1BB", "4-1BB antibody and "an antibody that specifically binds to 4-1BB" refer to an antibody that is capable of binding 4-1BB with sufficient affinity such that the antibody is useful as a diagnostic and/or therapeutic agent in targeting 4-1BB. In one embodiment, the extent of binding of an anti-4-1BB antibody to an unrelated, non-4-1BB protein is less than about 10% of the binding of the antibody to 4-1BB as measured, e.g., by a radioimmunoassay (RIA) or flow cytometry (FACS). In certain embodiments, an antibody that binds to 4-1BB has a dissociation constant (K_{D}) of ≤ 1µM, ≤ 100 nM, ≤ 10 nM, ≤ 1 nM, ≤ 0.1 nM, ≤ 0.01 nM, or ≤ 0.001 nM (e.g. 10⁻⁶ M or less, e.g. from 10⁻⁶⁸ M to 10⁻¹³ M, e.g., from 10⁻⁸ M to 10⁻¹⁰ M).

The term "**GITR**", as used herein, refers to any native GITR from any vertebrate source, including mammals such as primates (e.g. humans) and rodents (e.g., mice and rats), unless otherwise indicated. The term encompasses "full-length," unprocessed GITR as well as any form of GITR that results from processing in the cell. The term also encompasses naturally occurring variants of GITR, e.g., splice variants or allelic variants. The amino acid sequence of an exemplary human GITR is shown in SEQ ID NO: 74 (Uniprot P43489, version 112).

The terms **"anti-GITR antibody",** "anti-GITR", "GITR antibody and "an antibody that specifically binds to GITR" refer to an antibody that is capable of binding GITR with sufficient affinity such that the antibody is useful as a diagnostic and/or therapeutic agent in targeting GITR. In one aspect, the extent of binding of an anti-GITR antibody to an unrelated, non-GITR protein is less than about 10% of the binding of the antibody to GITR as measured, e.g., by a radioimmunoassay (RIA) or flow cytometry (FACS). In certain embodiments, an antibody that binds to GITR has a dissociation constant (K_{D}) of ≤ 1µM, ≤ 100 nM, ≤ 10 nM, ≤ 1 nM, ≤ 0.1 nM, ≤ 0.01 nM, or ≤ 0.001 nM (e.g. 10⁻⁶ M or less, e.g. from 10⁻⁶⁸ M to 10⁻¹³ M, e.g., from 10⁻⁸ M to 10⁻¹⁰ M).

The term "**peptide linker**" refers to a peptide comprising one or more amino acids, typically about 2 to 20 amino acids. Peptide linkers are known in the art or are described herein. Suitable, non-immunogenic linker peptides are, for example, (G₄S)ₙ, (SG₄)ₙ or G₄(SG₄)ₙ peptide linkers, wherein "n" is generally a number between 1 and 10, typically between 2 and 4, in particular 2, i.e. the peptides selected from the group consisting of GGGGS (SEQ ID NO: 78) GGGGSGGGGS (SEQ ID NO:79), SGGGGSGGGG (SEQ ID NO:80) and GGGGSGGGGSGGGG (SEQ ID NO:81), but also include the sequences GSPGSSSSGS (SEQ ID NO:82), (G4S)₃ (SEQ ID NO:83), (G4S)₄ (SEQ ID NO:84), GSGSGSGS (SEQ ID NO:85), GSGSGNGS (SEQ ID NO:86), GGSGSGSG (SEQ ID NO:87), GGSGSG (SEQ ID NO:88), GGSG (SEQ ID NO:89), GGSGNGSG (SEQ ID NO:90), GGNGSGSG (SEQ ID NO:91) and GGNGSG (SEQ ID NO:92). Peptide linkers of particular interest are (G4S) (SEQ ID NO:78), (G₄S)₂ or GGGGSGGGGS (SEQ ID NO:79) and (G4S)₄ (SEQ ID NO:84).

The term "**amino acid**" as used within this application denotes the group of naturally occurring carboxy α-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

By **"fused"** or **"connected"** is meant that the components (e.g. a heavy chain of an antibody and a Fab fragment) are linked by peptide bonds, either directly or via one or more peptide linkers.

**"Percent (%) amino acid sequence identity"** with respect to a reference polypeptide (protein) sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN. SAWI or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence

A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

In certain embodiments, **amino acid sequence variants** of the TNF ligand trimer-containing antigen binding molecules provided herein are contemplated. For example, it may be desirable to improve the binding affinity and/or other biological properties of the TNF ligand trimer-containing antigen binding molecules. Amino acid sequence variants of the TNF ligand trimer-containing antigen binding molecules may be prepared by introducing appropriate modifications into the nucleotide sequence encoding the molecules, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding. Sites of interest for substitutional mutagenesis include the HVRs and Framework (FRs). Conservative substitutions are provided in Table B under the heading "Preferred Substitutions" and further described below in reference to amino acid side chain classes (1) to (6). Amino acid substitutions may be introduced into the molecule of interest and the products screened for a desired activity, e.g., retained/improved antigen binding, decreased immunogenicity, or improved ADCC or CDC.

**TABLE B**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

The term **"amino acid sequence variants"** includes substantial variants wherein there are amino acid substitutions in one or more hypervariable region residues of a parent antigen binding molecule (*e.g.* a humanized or human antibody). Generally, the resulting variant(s) selected for further study will have modifications (e.g., improvements) in certain biological properties (e.g., increased affinity, reduced immunogenicity) relative to the parent antigen binding molecule and/or will have substantially retained certain biological properties of the parent antigen binding molecule. An exemplary substitutional variant is an affinity matured antibody, which may be conveniently generated, e.g., using phage display-based affinity maturation techniques such as those described herein. Briefly, one or more HVR residues are mutated and the variant antigen binding molecules displayed on phage and screened for a particular biological activity (e.g. binding affinity). In certain embodiments, substitutions, insertions, or deletions may occur within one or more HVRs so long as such alterations do not substantially reduce the ability of the antigen binding molecule to bind antigen. For example, conservative alterations (e.g., conservative substitutions as provided herein) that do not substantially reduce binding affinity may be made in HVRs. A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antigen binding molecule complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include bispecific antigen binding molecules of the invention with an N-terminal methionyl residue. Other insertional variants of the molecule include the fusion to the N- or C-terminus to a polypeptide which increases the serum half-life of the bispecific antigen binding molecules.

In certain aspects, the bispecific antigen binding molecules provided herein are altered to increase or decrease the extent to which the antibody is glycosylated. Glycosylation variants of the molecules may be conveniently obtained by altering the amino acid sequence such that one or more glycosylation sites is created or removed. Where the TNF ligand trimer-containing antigen binding molecule comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in TNF family ligand trimer-containing antigen binding molecule may be made in order to create variants with certain improved properties. In one aspect, variants of bispecific antigen binding molecules or antibodies of the invention are provided having a carbohydrate structure that lacks fucose attached (directly or indirectly) to an Fc region. Such fucosylation variants may have improved ADCC function, see e.g. US Patent Publication Nos. US 2003/0157108 (Presta, L.) or US 2004/0093621 (Kyowa Hakko Kogyo Co., Ltd). In another aspect, variants of the bispecific antigen binding molecules or antibodies of the invention are provided with bisected oligosaccharides, e.g., in which a biantennary oligosaccharide attached to the Fc region is bisected by GlcNAc. Such variants may have reduced fucosylation and/or improved ADCC function., see for example WO 2003/011878 (Jean-Mairet et al.); US Patent No. 6,602,684 (Umana et al.); and US 2005/0123546 (Umana et al.). Variants with at least one galactose residue in the oligosaccharide attached to the Fc region are also provided. Such antibody variants may have improved CDC function and are described, e.g., in WO 1997/30087 (Patel et al.); WO 1998/58964 (Raju, S.); and WO 1999/22764 (Raju, S.).

In certain aspects, it may be desirable to create cysteine engineered variants of the bispecific antigen binding molecules of the invention, e.g., "thioMAbs," in which one or more residues of the molecule are substituted with cysteine residues. In particular aspects, the substituted residues occur at accessible sites of the molecule. By substituting those residues with cysteine, reactive thiol groups are thereby positioned at accessible sites of the antibody and may be used to conjugate the antibody to other moieties, such as drug moieties or linker-drug moieties, to create an immunoconjugate. In certain aspects, any one or more of the following residues may be substituted with cysteine: V205 (Kabat numbering) of the light chain; A118 (EU numbering) of the heavy chain; and S400 (EU numbering) of the heavy chain Fc region. Cysteine engineered antigen binding molecules may be generated as described, e.g., in U.S. Patent No. 7,521,541.

The term **"polynucleotide"** refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By **"isolated"** nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present invention. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include in vivo or in vitro RNA transcripts of the present invention, as well as positive and negative strand forms, and double-stranded forms. Isolated polynucleotides or nucleic acids according to the present invention further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence of the present invention can be determined conventionally using known computer programs, such as the ones discussed above for polypeptides (e.g. ALIGN-2).

The term **"expression cassette"** refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain embodiments, the expression cassette of the invention comprises polynucleotide sequences that encode bispecific antigen binding molecules of the invention or fragments thereof.

The term **"vector"** or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector of the present invention comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one embodiment, the expression vector of the invention comprises an expression cassette that comprises polynucleotide sequences that encode bispecific antigen binding molecules of the invention or fragments thereof.

The terms **"host cell",** "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding molecules of the present invention. Host cells include cultured cells, e.g. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An **"effective amount"** of an agent refers to the amount that is necessary to result in a physiological change in the cell or tissue to which it is administered.

A **"therapeutically effective amount"** of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An **"individual"** or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term **"pharmaceutical composition"** refers to a preparation which is in such form as to permit the biological activity of an active ingredient contained therein to be effective, and which contains no additional components which are unacceptably toxic to a subject to which the formulation would be administered.

A **"pharmaceutically acceptable excipient"** refers to an ingredient in a pharmaceutical composition, other than an active ingredient, which is nontoxic to a subject. A pharmaceutically acceptable excipient includes, but is not limited to, a buffer, a stabilizer, or a preservative.

The term **"package insert"** is used to refer to instructions customarily included in commercial packages of therapeutic products, that contain information about the indications, usage, dosage, administration, combination therapy, contraindications and/or warnings concerning the use of such therapeutic products.

As used herein, **"treatment"** (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, the molecules of the invention are used to delay development of a disease or to slow the progression of a disease.

The term **"cancer"** as used herein refers to proliferative diseases, such as lymphomas, lymphocytic leukemias, lung cancer, non-small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

### Bispecific antibodies of the invention

The invention provides novel bispecific antigen binding molecules with particularly advantageous properties such as producibility, stability, binding affinity, biological activity, targeting efficiency and reduced toxicity.

### Exemplary bispecific antigen binding molecules

In one aspect, the invention provides bispecific antigen binding molecules, comprising
(a) four Fab fragments capable of specific binding to OX40,
(b) at least one moiety capable of specific binding to Fibroblast Activation Protein (FAP) comprising an antibody light chain variable region (VL) and an antibody heavy chain variable region (VH), and
(c) a Fc domain composed of a first and a second subunit capable of stable association, wherein the Fc domain is of human IgG1 subclass with the amino acid mutations L234A, L235A and P329G (numbering according to Kabat EU index), and
wherein each of the Fab fragments capable of specific binding to OX40 comprises a VH domain comprising CDR-H1 comprising the amino acid sequence of SEQ ID NO:2, CDR-H2 comprising the amino acid sequence of SEQ ID NO:4, CDR-H3 comprising the amino acid sequence of SEQ ID NO:7 and a VL domain comprising CDR-L1 comprising the amino acid sequence of SEQ ID NO: 13, CDR-L2 comprising the amino acid sequence of SEQ ID NO: 16 and CDR-L3 comprising the amino acid sequence of SEQ ID NO:20, and wherein a first Fab fragment capable of specific binding to OX40 is fused at the C-terminus of the CH1 domain to the VH domain of a second Fab fragment capable of specific binding to OX40 and a third Fab fragment capable of specific binding to OX40 is fused at the C-terminus of the CH1 domain to the VH domain of a fourth Fab fragment capable of specific binding to OX40, optionally via a peptide linker and wherein each two of the four Fab fragments capable of specific binding to OX40 are further fused at its C-terminus to the N-terminus of one of the subunits of the Fc domain, optionally via a peptide linker, and
wherein the VH domain of the moiety capable of specific binding to FAP is connected via a peptide linker to the C-terminus of the first subunit of the Fc domain and the VL domain is connected via a peptide linker to the C-terminus of the second subunit of the Fc domain or wherein two moieties capable of specific binding to FAP are VH-VL crossover Fab fragments and are each fused at the N-terminus of the VL domain via a peptide linker to the C-terminus of one of the subunits of the Fc domain.

In a particular aspect, these bispecific antigen binding molecules are characterized by agonistic binding to OX40.

### Bispecific tetravalent antigen binding molecules binding to OX40

In one aspect, the costimulatory TNF receptor family member is OX40. Particularly, the invention provides bispecific antigen binding molecules, wherein the moiety capable of specific binding to a costimulatory TNF receptor family member binds to a polypeptide comprising the amino acid sequence of SEQ ID NO:1.

In particular, provided is a bispecific antigen binding molecule, comprising four Fab fragments capable of specific binding to OX40, wherein said Fab fragments comprise
a VH domain comprising CDR-H1 comprising the amino acid sequence of SEQ ID NO:2, CDR-H2 comprising the amino acid sequence of SEQ ID NO:4, CDR-H3 comprising the amino acid sequence of SEQ ID NO:7 and a VL domain comprising CDR-L1 comprising the amino acid sequence of SEQ ID NO: 13, CDR-H2 comprising the amino acid sequence of SEQ ID NO: 16 and CDR-H3 comprising the amino acid sequence of SEQ ID NO:20.

In another aspect, provided is a bispecific antigen binding molecule, wherein the Fab fragments capable of specific binding to OX40 comprise a heavy chain variable region VH comprising an amino acid sequence of SEQ ID NO: 27 and a light chain variable region VL comprising an amino acid sequence of SEQ ID NO: 28.

The bispecific antigen binding molecules are further characterized by comprising at least one moiety capable of specific binding to a target cell antigen. In a particular aspect, the target cell antigen is Fibroblast Activation Protein (FAP). FAP binding moieties have been described in WO 2012/02006. FAP binding moieties of particular interest are described below.

In one aspect, provided is a bispecific antigen binding molecule, wherein the moiety capable of specific binding to FAP comprises a VH domain comprising
(i) a CDR-H1 comprising the amino acid sequence selected from the goup consisting of SEQ ID NO:39 and SEQ ID NO:40,
(ii) a CDR-H2 comprising the amino acid sequence selected from the group consisting of SEQ ID NO:41 and SEQ ID NO:42, and
(iii) a CDR-H3 comprising the amino acid sequence selected from the group consisting of SEQ ID NO:43 and SEQ ID NO:44,
   and a VL domain comprising
(iv) a CDR-L1 comprising the amino acid sequence selected from the group consisting of SEQ ID NO:45 and SEQ ID NO:46,
(v) a CDR-L2 comprising the amino acid sequence selected from the group consisting of SEQ ID NO:47 and SEQ ID NO:48, and
(vi) a CDR-L3 comprising the amino acid sequence selected from the group consisting of SEQ ID NO:49 and SEQ ID NO:50.

In particular, provided is a bispecific antigen binding molecule, wherein the moiety capable of specific binding to FAP comprises
(a) a VH domain comprising CDR-H1 comprising the amino acid sequence of SEQ ID NO:40, CDR-H2 comprising the amino acid sequence of SEQ ID NO:42, CDR-H3 comprising the amino acid sequence of SEQ ID NO:44 and a VL domain comprising CDR-L1 comprising the amino acid sequence of SEQ ID NO:46, CDR-H2 comprising the amino acid sequence of SEQ ID NO:48 and CDR-H3 comprising the amino acid sequence of SEQ ID NO:50, or
(b) a VH domain comprising CDR-H1 comprising the amino acid sequence of SEQ ID NO:39, CDR-H2 comprising the amino acid sequence of SEQ ID NO:41, CDR-H3 comprising the amino acid sequence of SEQ ID NO:43 and a VL domain comprising CDR-L1 comprising the amino acid sequence of SEQ ID NO:45, CDR-H2 comprising the amino acid sequence of SEQ ID NO:47 and CDR-H3 comprising the amino acid sequence of SEQ ID NO:49.

In a further aspect, provided is a bispecific antigen binding molecule, wherein
(i) the Fab fragments capable of specific binding to OX40 comprise a heavy chain variable region VH comprising an amino acid sequence of SEQ ID NO: 27 and a light chain variable region comprising an amino acid sequence of SEQ ID NO: 28 and
(ii) the moiety capable of specific binding to FAP comprises a heavy chain variable region VH comprising an amino acid sequence of SEQ ID NO: 51 or SEQ ID NO:53 and a light chain variable region comprising an amino acid sequence of SEQ ID NO:52 or SEQ ID NO:54.

More particularly, provided is a bispecific antigen binding molecule, wherein the Fab fragments capable of specific binding to OX40 comprise a heavy chain variable region VH comprising an amino acid sequence of SEQ ID NO:27 and a light chain variable region comprising an amino acid sequence of SEQ ID NO:28 and the moiety capable of specific binding to FAP comprises a heavy chain variable region VH comprising an amino acid sequence of SEQ ID NO:51 and and a light chain variable region comprising an amino acid sequence of SEQ ID NO:52, or wherein the Fab fragments capable of specific binding to OX40 comprise a heavy chain variable region VH comprising an amino acid sequence of SEQ ID NO:27 and a light chain variable region comprising an amino acid sequence of SEQ ID NO:28 and the moiety capable of specific binding to FAP comprises a heavy chain variable region VH comprising an amino acid sequence of SEQ ID NO:53 and and a light chain variable region comprising an amino acid sequence of SEQ ID NO:54.

In one aspect, provided is a bispecific antigen binding molecule, wherein each two of the four moieties capable of specific binding to OX40 are fused to each other. Optionally, they are fused to each other via a peptide linker. In particular, provided is a bispecific antigen binding molecule, wherein each two of the four moieties capable of specific binding to OX40 are fused to each other and at the C-terminus to the N-terminus of one of the subunits of the Fc domain, optionally they are connected at its C-terminus to the N-terminus of one of the subunits of the Fc domain via a peptide linker.

In a particular aspect, the invention provides a bispecific antigen binding molecule, wherein the molecule comprises two heavy chains and each of the heavy chains comprises variable domains of two Fab fragments capable of specific binding to OX40 and a variable domain of a moiety capable of specific binding to FAP.

In another aspect, provided is a bispecific antigen binding molecule as defined herein before, wherein the four moieties capable of specific binding to OX40 are Fab fragments and each two thereof are connected to each other. In a further aspect, the moieties capable of specific binding to OX40 each comprise an antibody light chain variable region (VL) and an antibody heavy chain variable region (VH).

### Tetravalent, bispecific antigen binding molecules with monovalency for the target cell antigen (4+1 format)

In one aspect, the invention relates to bispecifc antigen binding molecules, wherein the bispecific antigen binding molecule is tetravalent for OX40 and monovalent for FAP. Thus, the invention relates to bispecifc antigen binding molecules comprising (a) four Fab fragments capable of specific binding OX40, (b) one moiety capable of specific binding to FAP, and (c) a Fc domain composed of a first and a second subunit capable of stable association.

In one aspect, provided is a bispecific antigen binding molecule, wherein the moiety capable of specific binding to FAP comprises a VH and VL domain and wherein the VH domain is connected via a peptide linker to the C-terminus of the first subunit of the Fc domain and the VL domain is connected via a peptide linker to the C-terminus of the second subunit of the Fc domain. In a particular aspect, the peptide linker is (G4S)₄.

In one aspect, the invention relates to a bispecific antigen binding molecule comprising (a) four Fab fragments capable of specific binding to OX40, (b) a VH and VL domain capable of specific binding to FAP, and (c) a Fc domain composed of a first and a second subunit capable of stable association, wherein the first subunit of the Fc domain comprises knobs and the second subunit of the Fc domain comprises holes according to the knobs into holes method, and wherein the VH domain capable of specific binding to a target cell antigen is connected via a peptide linker to the C-terminus of the first subunit of the Fc domain comprising knobs and wherein the VL domain capable of specific binding to a target cell antigen is connected via a peptide linker to the C-terminus of the second subunit of the Fc domain comprising holes.

In another aspect, the invention relates to a bispecific antigen binding molecule comprising (a) four Fab fragments capable of specific binding to OX40, (b) a VH and VL domain capable of specific binding to FAP, and (c) a Fc domain composed of a first and a second subunit capable of stable association, wherein the first subunit of the Fc domain comprises knobs and the second subunit of the Fc domain comprises holes according to the knobs into holes method, and wherein the VL domain capable of specific binding to a target cell antigen is connected via a peptide linker to the C-terminus of the first subunit of the Fc domain comprising knobs and wherein the VH domain capable of specific binding to a target cell antigen is connected via a peptide linker to the C-terminus of the second subunit of the Fc domain comprising holes.

In a specific aspect, provided is a bispecific antigen binding molecule of the invention, wherein said antigen binding molecule comprises
(i) a first heavy chain comprising an amino acid sequence of SEQ ID NO:213, a second heavy chain comprising an amino acid sequence of SEQ ID NO:214, and a light chain comprising an amino acid sequence of SEQ ID NO: 157, or
(ii) a first heavy chain comprising an amino acid sequence of SEQ ID NO:217, a second heavy chain comprising an amino acid sequence of SEQ ID NO:218, and a light chain comprising an amino acid sequence of SEQ ID NO: 157.

In a further specifc aspect, provided is a bispecific antigen binding molecule of the invention, wherein said antigen binding molecule comprises a first heavy chain comprising an amino acid sequence of SEQ ID NO:233, a second heavy chain comprising an amino acid sequence of SEQ ID NO:234, and a light chain comprising an amino acid sequence of SEQ ID NO:157.

In another specifc aspect, provided is a bispecific antigen binding molecule of the invention, wherein said antigen binding molecule comprises a first heavy chain comprising an amino acid sequence of SEQ ID NO:237, a second heavy chain comprising an amino acid sequence of SEQ ID NO:238, and a light chain comprising an amino acid sequence of SEQ ID NO:157.

### Tetravalent, bispecific antigen binding molecules with bivalency for the target cell antigen (4+2 format)

In another aspect, the invention relates to a bispecific antigen binding molecule, comprising
(a) four Fab fragments capable of specific binding to OX40,
(b) two moieties capable of specific binding to FAP,
   and
(c) a Fc domain composed of a first and a second subunit capable of stable association.

In one aspect, the invention relates to a bispecific antigen binding molecule, wherein the bispecific antigen binding molecule is tetravalent for OX40 and bivalent for FAP.

In one aspect, provided is a bispecific antigen binding molecule, wherein the two moieties capable of specific binding to FAP are Fab fragments or crossover Fab fragments.

In a further aspect, provided is a bispecific antigen binding molecule, wherein each of the Fab fragments or crossover Fab fragments capable of specific binding to FAP is fused at the N-terminus of the VH or VL domain via a peptide linker to the C-terminus of one of the subunits of the Fc domain. In a particular aspect, the peptide linker is (G4S)₄.

In a particular aspect, provided is a bispecific antigen binding molecule, wherein the two moieties capable of specific binding to FAP are VH-VL crossover Fab fragments and are each fused at the N-terminus of the VL domain via a peptide linker to the C-terminus of one of the subunits of the Fc domain.

In another aspect, provided is a bispecific antigen binding molecule, wherein the two moieties capable of specific binding to FAP are CH-CL crossover Fab fragments and are each fused at the N-terminus of the VH domain via a peptide linker to the C-terminus of one of the subunits of the Fc domain.

More particularly, provided is a bispecific antigen binding molecule, wherein said antigen binding molecule comprises (i) a heavy chain comprising an amino acid sequence of SEQ ID NO:227, a first light chain of SEQ ID NO:226 and a second light chain of SEQ ID NO:228.

### Fc domain modifications reducing Fc receptor binding and/or effector function

The bispecific antigen binding molecules further comprise a Fc domain composed of a first and a second subunit capable of stable association.

In certain aspects, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

The Fc domain confers favorable pharmacokinetic properties to the bispecific antibodies of the invention, including a long serum half-life which contributes to good accumulation in the target tissue and a favorable tissue-blood distribution ratio. At the same time it may, however, lead to undesirable targeting of the bispecific antibodies of the invention to cells expressing Fc receptors rather than to the preferred antigen-bearing cells. Accordingly, in particular embodiments the Fc domain of the bispecific antibodies exhibits reduced binding affinity to an Fc receptor and/or reduced effector function, as compared to a native IgG Fc domain, in particular an IgG1 Fc domain or an IgG4 Fc domain. More particularly, the Fc domain is an IgG1 Fc domain.

In one such aspect the Fc domain (or the bispecific antigen binding molecule of the invention comprising said Fc domain) exhibits less than 50%, preferably less than 20%, more preferably less than 10% and most preferably less than 5% of the binding affinity to an Fc receptor, as compared to a native IgG1 Fc domain (or the bispecific antigen binding molecule of the invention comprising a native IgG1 Fc domain), and/or less than 50%, preferably less than 20%, more preferably less than 10% and most preferably less than 5% of the effector function, as compared to a native IgG1 Fc domain (or the bispecific antigen binding molecule of the invention comprising a native IgG1 Fc domain). In one aspect, the Fc domain (or the bispecific antigen binding molecule of the invention comprising said Fc domain) does not substantially bind to an Fc receptor and/or induce effector function. In a particular aspect the Fc receptor is an Fcγ receptor. In one aspect, the Fc receptor is a human Fc receptor. In one aspect, the Fc receptor is an activating Fc receptor. In a specific aspect, the Fc receptor is an activating human Fcγ receptor, more specifically human FcyRIIIa, FcyRI or FcyRIIa, most specifically human FcyRIIIa. In one aspect, the Fc receptor is an inhibitory Fc receptor. In a specific aspect, the Fc receptor is an inhibitory human Fcγ receptor, more specifically human FcyRIIB. In one aspect the effector function is one or more of CDC, ADCC, ADCP, and cytokine secretion. In a particular aspect, the effector function is ADCC. In one aspect, the Fc domain domain exhibits substantially similar binding affinity to neonatal Fc receptor (FcRn), as compared to a native IgG1 Fc domain. Substantially similar binding to FcRn is achieved when the Fc domain (or the the bispecific antigen binding molecule of the invention comprising said Fc domain) exhibits greater than about 70%, particularly greater than about 80%, more particularly greater than about 90% of the binding affinity of a native IgG1 Fc domain (or the the bispecific antigen binding molecule of the invention comprising a native IgG1 Fc domain) to FcRn.

In a particular aspect, the Fc domain is engineered to have reduced binding affinity to an Fc receptor and/or reduced effector function, as compared to a non-engineered Fc domain. In a particular aspect, the Fc domain of the bispecific antigen binding molecule of the invention comprises one or more amino acid mutation that reduces the binding affinity of the Fc domain to an Fc receptor and/or effector function. Typically, the same one or more amino acid mutation is present in each of the two subunits of the Fc domain. In one aspect, the amino acid mutation reduces the binding affinity of the Fc domain to an Fc receptor. In another aspect, the amino acid mutation reduces the binding affinity of the Fc domain to an Fc receptor by at least 2-fold, at least 5-fold, or at least 10-fold. In one aspect, the bispecific antigen binding molecule of the invention comprising an engineered Fc domain exhibits less than 20%, particularly less than 10%, more particularly less than 5% of the binding affinity to an Fc receptor as compared to bispecific antibodies of the invention comprising a non-engineered Fc domain. In a particular aspect, the Fc receptor is an Fcγ receptor. In other aspects, the Fc receptor is a human Fc receptor. In one aspect, the Fc receptor is an inhibitory Fc receptor. In a specific aspect, the Fc receptor is an inhibitory human Fcγ receptor, more specifically human FcyRIIB. In some aspects the Fc receptor is an activating Fc receptor. In a specific aspect, the Fc receptor is an activating human Fcγ receptor, more specifically human FcyRIIIa, FcyRI or FcyRIIa, most specifically human FcyRIIIa. Preferably, binding to each of these receptors is reduced. In some aspects, binding affinity to a complement component, specifically binding affinity to C1q, is also reduced. In one aspect, binding affinity to neonatal Fc receptor (FcRn) is not reduced. Substantially similar binding to FcRn, i.e. preservation of the binding affinity of the Fc domain to said receptor, is achieved when the Fc domain (or the bispecific antigen binding molecule of the invention comprising said Fc domain) exhibits greater than about 70% of the binding affinity of a non-engineered form of the Fc domain (or the bispecific antigen binding molecule of the invention comprising said non-engineered form of the Fc domain) to FcRn. The Fc domain, or the the bispecific antigen binding molecule of the invention comprising said Fc domain, may exhibit greater than about 80% and even greater than about 90% of such affinity. In certain embodiments the Fc domain of the bispecific antigen binding molecule of the invention is engineered to have reduced effector function, as compared to a non-engineered Fc domain. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced dendritic cell maturation, or reduced T cell priming.

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581). Certain antibody variants with improved or diminished binding to FcRs are described. (e.g. U.S. Patent No. 6,737,056; WO 2004/056312, and Shields, R.L. et al., J. Biol. Chem. 276 (2001) 6591-6604).

In one aspect, the Fc domain comprises an amino acid substitution at a position of E233, L234, L235, N297, P331 and P329. In some aspects, the Fc domain comprises the amino acid substitutions L234A and L235A ("LALA"). In one such embodiment, the Fc domain is an IgG1 Fc domain, particularly a human IgG1 Fc domain. In one aspect, the Fc domain comprises an amino acid substitution at position P329. In a more specific aspect, the amino acid substitution is P329A or P329G, particularly P329G. In one embodiment the Fc domain comprises an amino acid substitution at position P329 and a further amino acid substitution selected from the group consisting of E233P, L234A, L235A, L235E, N297A, N297D or P331S. In more particular embodiments the Fc domain comprises the amino acid mutations L234A, L235A and P329G ("P329G LALA"). The "P329G LALA" combination of amino acid substitutions almost completely abolishes Fcγ receptor binding of a human IgG1 Fc domain, as described in PCT Patent Application No. WO 2012/130831 A1. Said document also describes methods of preparing such mutant Fc domains and methods for determining its properties such as Fc receptor binding or effector functions. such antibody is an IgG1 with mutations L234A and L235A or with mutations L234A, L235A and P329G (numbering according to EU index of Kabat et al , Kabat et al., Sequences of Proteins of Immunological Interest, 5th Ed. Public Health Service, National Institutes of Health, Bethesda, MD, 1991).

In one aspect, the Fc domain is an IgG4 Fc domain. In a more specific embodiment, the Fc domain is an IgG4 Fc domain comprising an amino acid substitution at position S228 (Kabat numbering), particularly the amino acid substitution S228P. In a more specific embodiment, the Fc domain is an IgG4 Fc domain comprising amino acid substitutions L235E and S228P and P329G. This amino acid substitution reduces in vivo Fab arm exchange of IgG4 antibodies (see Stubenrauch et al., Drug Metabolism and Disposition 38, 84-91 (2010)).

Antibodies with increased half lives and improved binding to the neonatal Fc receptor (FcRn), which is responsible for the transfer of maternal IgGs to the fetus (Guyer, R.L. et al., J. Immunol. 117 (1976) 587-593, and Kim, J.K. et al., J. Immunol. 24 (1994) 2429-2434), are described in US 2005/0014934. Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826). See also Duncan, A.R. and Winter, G., Nature 322 (1988) 738-740; US 5,648,260; US 5,624,821; and WO 94/29351 concerning other examples of Fc region variants.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or cell activating bispecific antigen binding molecules comprising an Fc domain for Fc receptors may be evaluated using cell lines known to express particular Fc receptors, such as human NK cells expressing FcγIIIa receptor. Effector function of an Fc domain, or bispecific antibodies of the invention comprising an Fc domain, can be measured by methods known in the art. A suitable assay for measuring ADCC is described herein. Other examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI^{™} non-radioactive cytotoxicity assay for flow cytometry (CellTechnology, Inc. Mountain View, CA); and CytoTox 96^{®} non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed *in vivo,* e.g. in a animal model such as that disclosed in Clynes et al., Proc Natl Acad Sci USA 95, 652-656 (1998).

In particular aspect, the Fc domain is of human IgG1 subclass with the amino acid mutations L234A, L235A and P329G (numbering according to Kabat EU index).

### Fc domain modifications promoting heterodimerization

In some aspects, the bispecific antigen binding molecules comprise different antigen-binding sites, fused to one or the other of the two subunits of the Fc domain, thus the two subunits of the Fc domain may be comprised in two non-identical polypeptide chains. Recombinant co-expression of these polypeptides and subsequent dimerization leads to several possible combinations of the two polypeptides. To improve the yield and purity of the bispecific antibodies of the invention in recombinant production, it will thus be advantageous to introduce in the Fc domain of the bispecific antigen binding molecules of the invention a modification promoting the association of the desired polypeptides.

Accordingly, in particular aspects the Fc domain comprises a modification promoting the association of the first and second subunit of the Fc domain. The site of most extensive protein-protein interaction between the two subunits of a human IgG Fc domain is in the CH3 domain of the Fc domain. Thus, in one aspect said modification is in the CH3 domain of the Fc domain.

In a specific aspect said modification is a so-called "knob-into-hole" modification, comprising a "knob" modification in one of the two subunits of the Fc domain and a "hole" modification in the other one of the two subunits of the Fc domain. Thus, the invention relates to the bispecific antigen binding molecule comprising (a) four Fab fragments capable of specific binding to OX40,(b) at least one moiety capable of specific binding to FAP, and (c) a Fc domain composed of a first and a second subunit capable of stable association, wherein the first subunit of the Fc domain comprises knobs and the second subunit of the Fc domain comprises holes according to the knobs into holes method. In a particular aspect, the first subunit of the Fc domain comprises the amino acid substitutions S354C and T366W (EU numbering) and the second subunit of the Fc domain comprises the amino acid substitutions Y349C, T366S and Y407V (numbering according to Kabat EU index).

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in one aspect, in the CH3 domain of the first subunit of the Fc domain of the bispecific antigen binding molecules of the invention an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable. The protuberance and cavity can be made by altering the nucleic acid encoding the polypeptides, e.g. by site-specific mutagenesis, or by peptide synthesis. In a specific aspect, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). In one aspect, in the second subunit of the Fc domain additionally the threonine residue at position 366 is replaced with a serine residue (T366S) and the leucine residue at position 368 is replaced with an alanine residue (L368A).

In yet a further aspect, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). Introduction of these two cysteine residues results in formation of a disulfide bridge between the two subunits of the Fc domain, further stabilizing the dimer (Carter (2001), J Immunol Methods 248, 7-15). In a particular aspect, the first subunit of the Fc domain comprises the amino acid substitutions S354C and T366W (EU numbering) and the second subunit of the Fc domain comprises the amino acid substitutions Y349C, T366S and Y407V (numbering according to Kabat EU index).

In an alternative aspect, a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable.

### Modifications in the Fab domains

In one aspect, the invention relates to a bispecific antigen binding molecule comprising (a) four Fab fragments capable of specific binding to OX40, (b) two Fab fragments capable of specific binding to FAP, and (c) a Fc domain composed of a first and a second subunit capable of stable association, wherein in the four Fab fragments capable of specific binding to OX40 or in the two Fab fragments capable of specific binding to FAP either the variable domains VH and VL or the constant domains CHI and CL are exchanged. The bispecific antibodies are prepared according to the Crossmab technology.

Multispecific antibodies with a domain replacement/exchange in one binding arm (CrossMabVH-VL or CrossMabCH-CL) are described in detail in WO2009/080252 and Schaefer, W. et al, PNAS, 108 (2011) 11187-1191. They clearly reduce the byproducts caused by the mismatch of a light chain against a first antigen with the wrong heavy chain against the second antigen (compared to approaches without such domain exchange).

In one aspect, the invention relates to a bispecific antigen binding molecule comprising (a) four Fab fragments capable of specific binding to OX40, (b) two Fab fragments capable of specific binding to FAP, and (c) a Fc domain composed of a first and a second subunit capable of stable association, wherein in the two Fab fragments capable of specific binding to FAP the variable domains VL and VH are replaced by each other so that the VH domain is part of the light chain and the VL domain is part of the heavy chain.

In another aspect, the disclosure relates to a bispecific antigen binding molecule comprising (a) four Fab fragments capable of specific binding to OX40, (b) two Fab fragments capable of specific binding to FAP, and (c) a Fc domain composed of a first and a second subunit capable of stable association, wherein in the two Fab fragments capable of specific binding to FAP the constant domains CL and CHI are replaced by each other so that the CHI domain is part of the light chain and the CL domain is part of the heavy chain.

In another aspect, the disclosure relates to a bispecific antigen binding molecule, comprising (a) four light chains and two heavy chains of an antibody comprising four Fab fragments capable of specific binding to OX40 and the Fc domain, and (b) two additional Fab fragments capable of specific binding to FAP, wherein said additional Fab fragments are each connected via a peptide linker, in particular (G4S)₄, to the C-terminus of the heavy chains of (a). In a particular aspect, the additional Fab fragments are cross-Fab fragments, wherein the variable domains VL and VH are replaced by each other so that the VH domain is part of the light chain and the VL domain is part of the heavy chain, or wherein the the constant domains CL and CHI are replaced by each other so that the CHI domain is part of the light chain and the CL domain is part of the heavy chain.

Thus, in a particular aspect, the disclosure comprises a bispecific, antigen binding molecule, comprising (a) four light chains and two heavy chains of an antibody comprising four Fab fragments capable of specific binding to OX40 and the Fc domain, and (b) two additional Fab fragments capable of specific binding to FAP, wherein said two additional Fab fragments capable of specific binding to FAP are crossover Fab fragments wherein the variable domains VL and VH are replaced by each other and the VL-CH chains are each connected via a peptide linker to the C-terminus of the heavy chains of (a). In a particular aspect, the peptide linker is (G4S)₄.

In another aspect, and to further improve correct pairing of the different heavy and light chains, the bispecific antigen binding molecule comprising (a) four light chains and two heavy chains of an antibody comprising four Fab fragments capable of specific binding to OX40 and the Fc domain, and (b) two additional Fab fragments capable of specific binding to FAP, can contain different charged amino acid substitutions (so-called "charged residues"). These modifications are introduced in the crossed or non-crossed CHI and CL domains. In one aspect, in the constant domain CL of one of CL domains of the four Fab fragments capable of specific binding to a costimulatory TNF receptor family member the amino acid at position 123 is substituted by R and the amino acid as position 124 is substituted by K (numbering according to EU index of Kabat). In a further aspect, in the constant domain CHI of one of CHI domains of the four Fab fragments capable of specific binding to a costimulatory TNF receptor family member the amino acids at position 147 and 213 are substituted by E (numbering according to EU index of Kabat).

In a particular aspect, provided is a bispecific antigen binding molecule, wherein in one of CL domains of the four Fab fragments capable of specific binding to OX40 the amino acid at position 123 (EU numbering) has been replaced by arginine (R) and the amino acid at position 124 (EU numbering) has been substituted by lysine (K) and wherein in one of the CHI domains of the four Fab fragments capable of specific binding to a costimulatory TNF receptor family member the amino acids at position 147 (EU numbering) and at position 213 (EU numbering) have been substituted by glutamic acid (E).

More particularly, the invention relates to a bispecific binding molecule comprising Fab fragments, wherein in the CL domain adjacent to the variable domains capable of specific binding to OX40 the amino acid at position 123 (EU numbering) has been replaced by arginine (R) and the amino acid at position 124 (EU numbering) has been substituted by lysine (K), and wherein in the CHI domain adjacent to the TNF ligand family member the amino acids at position 147 (EU numbering) and at position 213 (EU numbering) have been substituted by glutamic acid (E).

### Polynucleotides

The invention further provides isolated polynucleotides encoding a bispecific antigen binding molecule as described herein or a fragment thereof.

The isolated polynucleotides encoding bispecific antibodies of the invention may be expressed as a single polynucleotide that encodes the entire antigen binding molecule or as multiple (e.g., two or more) polynucleotides that are co-expressed. Polypeptides encoded by polynucleotides that are co-expressed may associate through, e.g., disulfide bonds or other means to form a functional antigen binding molecule. For example, the light chain portion of an immunoglobulin may be encoded by a separate polynucleotide from the heavy chain portion of the immunoglobulin. When co-expressed, the heavy chain polypeptides will associate with the light chain polypeptides to form the immunoglobulin.

In some aspects, the isolated polynucleotide encodes a polypeptide comprised in the bispecific molecule according to the invention as described herein.

In one aspect, the present invention is directed to an isolated polynucleotide encoding a bispecific antigen binding molecule, comprising (a) four Fab fragments capable of specific binding to OX40, (b) at least one moiety capable of specific binding to FAP, and (c) a Fc domain composed of a first and a second subunit capable of stable association.

In certain embodiments the polynucleotide or nucleic acid is DNA. In other embodiments, a polynucleotide of the present invention is RNA, for example, in the form of messenger RNA (mRNA). RNA of the present invention may be single stranded or double stranded.

### Recombinant Methods

Bispecific antibodies may be obtained, for example, by solid-state peptide synthesis (e.g. Merrifield solid phase synthesis) or recombinant production. For recombinant production one or more polynucleotide encoding the bispecific antigen binding molecule or polypeptide fragments thereof, e.g., as described above, is isolated and inserted into one or more vectors for further cloning and/or expression in a host cell. Such polynucleotide may be readily isolated and sequenced using conventional procedures. In one aspect of the invention, a vector, preferably an expression vector, comprising one or more of the polynucleotides of the invention is provided. Methods which are well known to those skilled in the art can be used to construct expression vectors containing the coding sequence of the bispecific antigen binding molecule (fragment) along with appropriate transcriptional/translational control signals. These methods include in vitro recombinant DNA techniques, synthetic techniques and in vivo recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y. (1989). The expression vector can be part of a plasmid, virus, or may be a nucleic acid fragment. The expression vector includes an expression cassette into which the polynucleotide encoding the bispecific antigen binding molecule or polypeptide fragments thereof (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector of the present invention may encode one or more polypeptides, which are post- or co-translationally separated into the final proteins via proteolytic cleavage. In addition, a vector, polynucleotide, or nucleic acid of the invention may encode heterologous coding regions, either fused or unfused to a polynucleotide encoding the bispecific antigen binding molecule of the invention or polypeptide fragments thereof, or variants or derivatives thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription.

Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions of the present invention may be associated with additional coding regions which encode secretory or signal peptides, which direct the secretion of a polypeptide encoded by a polynucleotide of the present invention. For example, if secretion of the bispecific antigen binding molecule or polypeptide fragments thereof is desired, DNA encoding a signal sequence may be placed upstream of the nucleic acid encoding the bispecific antigen binding molecule of the invention or polypeptide fragments thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, e.g. an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag) or assist in labeling the fusion protein may be included within or at the ends of the polynucleotide encoding a bispecific antigen binding molecule of the invention or polypeptide fragments thereof.

In a further aspect of the invention, a host cell comprising one or more polynucleotides of the invention is provided. In certain aspects, a host cell comprising one or more vectors of the invention is provided. The polynucleotides and vectors may incorporate any of the features, singly or in combination, described herein in relation to polynucleotides and vectors, respectively. In one aspect, a host cell comprises (e.g. has been transformed or transfected with) a vector comprising a polynucleotide that encodes (part of) a bispecific antigen binding molecule of the invention of the invention. As used herein, the term "host cell" refers to any kind of cellular system which can be engineered to generate the fusion proteins of the invention or fragments thereof. Host cells suitable for replicating and for supporting expression of antigen binding molecules are well known in the art. Such cells may be transfected or transduced as appropriate with the particular expression vector and large quantities of vector containing cells can be grown for seeding large scale fermenters to obtain sufficient quantities of the antigen binding molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006).

Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. Plant cell cultures can also be utilized as hosts. See e.g. US Patent Nos. 5,959,177, 6,040,498, 6,420,548, 7,125,978, and 6,417,429 (describing PLANTIBODIES^{™} technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr- CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell). Standard technologies are known in the art to express foreign genes in these systems. Cells expressing a polypeptide comprising either the heavy or the light chain of an immunoglobulin, may be engineered so as to also express the other of the immunoglobulin chains such that the expressed product is an immunoglobulin that has both a heavy and a light chain.

In one aspect, a method of producing a bispecific antigen binding molecule of the invention or polypeptide fragments thereof is provided, wherein the method comprises culturing a host cell comprising polynucleotides encoding the bispecific antigen binding molecule of the invention or polypeptide fragments thereof, as provided herein, under conditions suitable for expression of the bispecific antigen binding molecule of the invention or polypeptide fragments thereof, and recovering the bispecific antigen binding molecule of the invention or polypeptide fragments thereof from the host cell (or host cell culture medium).

Bispecific molecules of the invention prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the bispecific antigen binding molecule binds. For example, for affinity chromatography purification of fusion proteins of the invention, a matrix with protein A or protein G may be used. Sequential Protein A or G affinity chromatography and size exclusion chromatography can be used to isolate an antigen binding molecule essentially as described in the examples. The purity of the bispecific antigen binding molecule or fragments thereof can be determined by any of a variety of well-known analytical methods including gel electrophoresis, high pressure liquid chromatography, and the like. For example, the bispecific antigen binding molecules expressed as described in the Examples were shown to be intact and properly assembled as demonstrated by reducing and non-reducing SDS-PAGE.

### Assays

The antigen binding molecules provided herein may be identified, screened for, or characterized for their physical/chemical properties and/or biological activities by various assays known in the art.

### 1. Affinity assays

The affinity of the bispecific antigen binding molecules, antibodies and antibody fragments provided herein for the corresponding TNF receptor can be determined in accordance with the methods set forth in the examples by surface plasmon resonance (SPR), using standard instrumentation such as a BIAcore instrument (GE Healthcare), and receptors or target proteins such as may be obtained by recombinant expression. The affinity of the bispecific antigen binding molecule for the target cell antigen can also be determined by surface plasmon resonance (SPR), using standard instrumentation such as a BIAcore instrument (GE Healthcare), and receptors or target proteins such as may be obtained by recombinant expression. A specific illustrative and exemplary embodiment for measuring binding affinity is described in Example 2. According to one aspect, K_{D} is measured by surface plasmon resonance using a BIACORE^{®} T100 machine (GE Healthcare) at 25 °C.

### 2. Binding assays and other assays

Binding of the bispecific antigen binding molecule provided herein to the corresponding receptor expressing cells may be evaluated using cell lines expressing the particular receptor or target antigen, for example by flow cytometry (FACS). In one aspect, peripheral blood mononuclear cells (PBMCs) expressing OX40 are used in the binding assay. These cells are used directly after isolation (naive PMBCs) or after stimulation (activated PMBCs). In another aspect, activated mouse splenocytes (expressing the OX40 molecule) were used to demonstrate the binding of the bispecific antigen binding molecule to the corresponding OX40 expressing cells. In a further aspect, PBMC isolated from heparinized blood of healthy Macaca fascicularis were used to show of the bispecific antigen binding molecule to the corresponding cynomolgus OX40 expressing cells.

In a further aspect, cancer cell lines expressing the target cell antigen, for example FAP, were used to demonstrate the binding of the antigen binding molecules to the target cell antigen.

In another aspect, competition assays may be used to identify an antigen binding molecule that competes with a specific antibody or antigen binding molecule for binding to FAP or OX40, respectively. In certain embodiments, such a competing antigen binding molecule binds to the same epitope (e.g., a linear or a conformational epitope) that is bound by a specific anti-FAP antibody or a specific anti-OX40 antibody. Detailed exemplary methods for mapping an epitope to which an antibody binds are provided in Morris (1996) "Epitope Mapping Protocols," in Methods in Molecular Biology vol. 66 (Humana Press, Totowa, NJ).

### 3. Activity assays

In one aspect, assays are provided for identifying bispecific antigen binding molecules that bind to a specific target cell antigen and to a specific TNF receptor having biological activity. Biological activity may include, e.g., agonistic signalling through the TNF receptor on cells expressing the target cell antigen. In certain aspects, a bispecific antigen binding molecule of the invention is tested for such biological activity. Furthermore, assays for detecting cell lysis (e.g. by measurement of LDH release), induced apoptosis kinetics (e.g. by measurement of Caspase 3/7 activity) or apoptosis (e.g. using the TUNEL assay) are well known in the art. In addition the biological activity of such complexes can be assessed by evaluating their effects on survival, proliferation and lymphokine secretion of various lymphocyte subsets such as NK cells, NKT-cells or γδ T-cells or assessing their capacity to modulate phenotype and function of antigen presenting cells such as dendritic cells, monocytes/macrophages or B-cells.

### Pharmaceutical Compositions, Formulations and Routes of Administation

In a further aspect, the invention provides pharmaceutical compositions comprising any of the bispecific antigen binding molecules provided herein, e.g., for use in any of the below therapeutic methods. In one embodiment, a pharmaceutical composition comprises any of the bispecific antigen binding molecules provided herein and at least one pharmaceutically acceptable excipient. In another embodiment, a pharmaceutical composition comprises any of the bispecific antigen binding molecules provided herein and at least one additional therapeutic agent, e.g., as described below.

Pharmaceutical compositions of the present invention comprise a therapeutically effective amount of one or more bispecific antigen binding molecules dissolved or dispersed in a pharmaceutically acceptable excipient. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one bispecific antigen binding molecule or antibody according to the invention and optionally an additional active ingredient will be known to those of skill in the art in light of the present disclosure, as exemplified by Remington's Pharmaceutical Sciences, 18th Ed. Mack Printing Company, 1990, incorporated herein by reference. In particular, the compositions are lyophilized formulations or aqueous solutions. As used herein, "pharmaceutically acceptable excipient" includes any and all solvents, buffers, dispersion media, coatings, surfactants, antioxidants, preservatives (e.g. antibacterial agents, antifungal agents), isotonic agents, salts, stabilizers and combinations thereof, as would be known to one of ordinary skill in the art.

Parenteral compositions include those designed for administration by injection, e.g. subcutaneous, intradermal, intralesional, intravenous, intraarterial intramuscular, intrathecal or intraperitoneal injection. For injection, the bispecific antigen binding molecules or antibodies of the invention may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the bispecific antigen binding molecules or antibodies may be in powder form for constitution with a suitable vehicle, e.g., sterile pyrogen-free water, before use. Sterile injectable solutions are prepared by incorporating the antigen binding molecules of the invention in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable excipients include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular embodiments, prolonged absorption of an injectable composition can be brought about by the use in the compositions of agents delaying absorption, such as, for example, aluminum monostearate, gelatin or combinations thereof.

Exemplary pharmaceutically acceptable excipients herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

In addition to the compositions described previously, the antigen binding molecules may also be formulated as a depot preparation. Such long acting formulations may be administered by implantation (for example subcutaneously or intramuscularly) or by intramuscular injection. Thus, for example, the fusion proteins may be formulated with suitable polymeric or hydrophobic materials (for example as emulsion in an acceptable oil) or ion exchange resins, or as sparingly soluble derivatives, for example, as a sparingly soluble salt.

Pharmaceutical compositions comprising the bispecific antigen binding molecules or antibodies of the invention may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The bispecific antigen binding molecules may be formulated into a composition in a free acid or base, neutral or salt form. Pharmaceutically acceptable salts are salts that substantially retain the biological activity of the free acid or base. These include the acid addition salts, e.g. those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

The composition herein may also contain more than one active ingredients as necessary for the particular indication being treated, preferably those with complementary activities that do not adversely affect each other. Such active ingredients are suitably present in combination in amounts that are effective for the purpose intended.

The formulations to be used for in vivo administration are generally sterile. Sterility may be readily accomplished, e.g., by filtration through sterile filtration membranes.

### Therapeutic methods and compositions

Any of the bispecific antigen binding molecules or antibodies provided herein may be used in therapeutic methods.

For use in therapeutic methods, bispecific antigen binding molecules of the invention can be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one aspect, bispecific antigen binding molecules of the invention for use as a medicament are provided.

In further aspects, bispecific antigen binding molecules of the invention for use (i) in stimulating or enhancing T cell response, (ii) for use in supporting survival of activated T cells, (iii) for use in the treatment of infections, (iv) for use in the treatment of cancer, (v) for use in delaying progression of cancer, or (vi) for use in prolonging the survival of a patient suffering from cancer, are provided. In a particular aspect, antibodies of the invention for use in treating a disease, in particular for use in the treatment of cancer, are provided.

In a further aspect, the invention provides for the use of the bispecific antigen binding molecule in the manufacture or preparation of a medicament for the treatment of a disease in an individual in need thereof. In one aspect the medicament is for use in a method of treating a disease comprising administering to an individual having the disease a therapeutically effective amount of the medicament. In certain aspects, the disease to be treated is a proliferative disorder, particularly cancer. Examples of cancers include bladder cancer, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer. Other cell proliferation disorders that can be treated using the bispecific antigen binding molecule or antibody of the invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are pre-cancerous conditions or lesions and cancer metastases. In certain embodiments the cancer is chosen from the group consisting of renal cell cancer, skin cancer, lung cancer, colorectal cancer, breast cancer, brain cancer, head and neck cancer. A skilled artisan readily recognizes that in many cases the the bispecific antigen binding molecule or antibody of the invention may not provide a cure but may provide a benefit. In some aspects, a physiological change having some benefit is also considered therapeutically beneficial. Thus, in some aspects, an amount of the bispecific antigen binding molecule or antibody of the invention that provides a physiological change is considered an "effective amount" or a "therapeutically effective amount".

For the prevention or treatment of disease, the appropriate dosage of a bispecific antigen binding molecule or antibody of the invention (when used alone or in combination with one or more other additional therapeutic agents) will depend on the type of disease to be treated, the route of administration, the body weight of the patient, the specific molecule, the severity and course of the disease, whether the the bispecific antigen binding molecule or antibody of the invention is administered for preventive or therapeutic purposes, previous or concurrent therapeutic interventions, the patient's clinical history and response to the fusion protein, and the discretion of the attending physician. The practitioner responsible for administration will, in any event, determine the concentration of active ingredient(s) in a composition and appropriate dose(s) for the individual subject. Various dosing schedules including but not limited to single or multiple administrations over various time-points, bolus administration, and pulse infusion are contemplated herein.

The bispecific antigen binding molecule or antibody of the invention is suitably administered to the patient at one time or over a series of treatments. Depending on the type and severity of the disease, about 1 µg/kg to 15 mg/kg (e.g. 0.1 mg/kg - 10 mg/kg) of TNF family ligand trimer-containing antigen binding molecule can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the the bispecific antigen binding molecule or antibody of the invention would be in the range from about 0.005 mg/kg to about 10 mg/kg. In other examples, a dose may also comprise from about 1 µg/kg body weight, about 5 µg/kg body weight, about 10 µg/kg body weight, about 50 µg/kg body weight, about 100 µg/kg body weight, about 200 µg/kg body weight, about 350 µg/kg body weight, about 500 µg/kg body weight, about 1 mg/kg body weight, about 5 mg/kg body weight, about 10 mg/kg body weight, about 50 mg/kg body weight, about 100 mg/kg body weight, about 200 mg/kg body weight, about 350 mg/kg body weight, about 500 mg/kg body weight, to about 1000 mg/kg body weight or more per administration, and any range derivable therein. In examples of a derivable range from the numbers listed herein, a range of about 5 mg/kg body weight to about 100 mg/kg body weight, about 5 µg/kg body weight to about 500 mg/kg body weight etc., can be administered, based on the numbers described above. Thus, one or more doses of about 0.5 mg/kg, 2.0 mg/kg, 5.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the fusion protein). An initial higher loading dose, followed by one or more lower doses may be administered. However, other dosage regimens may be useful. The progress of this therapy is easily monitored by conventional techniques and assays.

The bispecific antigen binding molecule or antibody of the invention will generally be used in an amount effective to achieve the intended purpose. For use to treat or prevent a disease condition, the bispecific antigen binding molecule or antibody of the invention, or pharmaceutical compositions thereof, are administered or applied in a therapeutically effective amount. Determination of a therapeutically effective amount is well within the capabilities of those skilled in the art, especially in light of the detailed disclosure provided herein.

For systemic administration, a therapeutically effective dose can be estimated initially from in vitro assays, such as cell culture assays. A dose can then be formulated in animal models to achieve a circulating concentration range that includes the IC₅₀ as determined in cell culture. Such information can be used to more accurately determine useful doses in humans.

Initial dosages can also be estimated from in vivo data, e.g., animal models, using techniques that are well known in the art. One having ordinary skill in the art could readily optimize administration to humans based on animal data.

Dosage amount and interval may be adjusted individually to provide plasma levels of the the bispecific antigen binding molecule or antibody of the invention which are sufficient to maintain therapeutic effect. Usual patient dosages for administration by injection range from about 0.1 to 50 mg/kg/day, typically from about 0.5 to 1 mg/kg/day. Therapeutically effective plasma levels may be achieved by administering multiple doses each day. Levels in plasma may be measured, for example, by HPLC.

In cases of local administration or selective uptake, the effective local concentration of the bispecific antigen binding molecule or antibody of the invention may not be related to plasma concentration. One skilled in the art will be able to optimize therapeutically effective local dosages without undue experimentation.

A therapeutically effective dose of the bispecific antigen binding molecule or antibody of the invention described herein will generally provide therapeutic benefit without causing substantial toxicity. Toxicity and therapeutic efficacy of a fusion protein can be determined by standard pharmaceutical procedures in cell culture or experimental animals. Cell culture assays and animal studies can be used to determine the LD₅₀ (the dose lethal to 50% of a population) and the ED₅₀ (the dose therapeutically effective in 50% of a population). The dose ratio between toxic and therapeutic effects is the therapeutic index, which can be expressed as the ratio LD₅₀/ED₅₀. Bispecific antigen binding molecules that exhibit large therapeutic indices are preferred. In one aspect, the the bispecific antigen binding molecule or antibody of the invention exhibits a high therapeutic index. The data obtained from cell culture assays and animal studies can be used in formulating a range of dosages suitable for use in humans. The dosage lies preferably within a range of circulating concentrations that include the ED50 with little or no toxicity. The dosage may vary within this range depending upon a variety of factors, e.g., the dosage form employed, the route of administration utilized, the condition of the subject, and the like. The exact formulation, route of administration and dosage can be chosen by the individual physician in view of the patient's condition (see, e.g., Fingl et al., 1975, in: The Pharmacological Basis of Therapeutics, Ch. 1, p. 1, incorporated herein by reference in its entirety).

The attending physician for patients treated with fusion proteins of the invention would know how and when to terminate, interrupt, or adjust administration due to toxicity, organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient.

### Other agents and treatments

The bispecific antigen binding molecule of the invention may be administered in combination with one or more other agents in therapy. For instance, the bispecific antigen binding molecule may be co-administered with at least one additional therapeutic agent. The term "therapeutic agent" encompasses any agent that can be administered for treating a symptom or disease in an individual in need of such treatment. Such additional therapeutic agent may comprise any active ingredients suitable for the particular indication being treated, preferably those with complementary activities that do not adversely affect each other. In certain embodiments, an additional therapeutic agent is another anti-cancer agent.

Such other agents are suitably present in combination in amounts that are effective for the purpose intended. The effective amount of such other agents depends on the amount of fusion protein used, the type of disorder or treatment, and other factors discussed above. The bispecific antigen binding molecules are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the bispecific antigen binding molecule or antibody of the invention can occur prior to, simultaneously, and/or following, administration of the additional therapeutic agent and/or adjuvant.

### Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper that is pierceable by a hypodermic injection needle). At least one active agent in the composition is a bispecific antigen binding molecule of the invention.

The label or package insert indicates that the composition is used for treating the condition of choice. Moreover, the article of manufacture may comprise (a) a first container with a composition contained therein, wherein the composition comprises a bispecific antigen binding molecule of the invention; and (b) a second container with a composition contained therein, wherein the composition comprises a further cytotoxic or otherwise therapeutic agent. The article of manufacture in this embodiment may further comprise a package insert indicating that the compositions can be used to treat a particular condition.

Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

**Table C (Sequences):**

| SEQ ID NO: | Name | Sequence |
|---|---|---|
| 1 | Human OX40 ECD | Uniprot No. P43489, aa 29-214 |
| 2 | OX40(8H9,49B4,1G4, 20B7) CDR-H1 | SYAIS |
| 3 | OX40(CLC-563, CLC-564, 17A9) CDR-H1 | SYAMS |
| 4 | OX40(8H9,49B4,1G4, 20B7) CDR-H2 | GIIPIFGTANYAQKFQG |
| 5 | OX40(CLC-563, CLC-564, 17A9) CDR-H2 | AISGSGGSTYYADSVKG |
| 6 | OX40(8H9) CDR-H3 | EYGWMDY |
| 7 | OX40(49B4) CDR-H3 | EYYRGPYDY |
| 8 | OX40(1G4) CDR-H3 | EYGSMDY |
| 9 | OX40(20B7) CDR-H3 | VNYPYSYWGDFDY |
| 10 | OX40(CLC-563) CDR-H3 | DVGAFDY |
| 11 | OX40(CLC-564) CDR-H3 | DVGPFDY |
| 12 | OX40(17A9)-CDR-H3 | VFYRGGVSMDY |
| 13 | OX40(8H9,49B4,1G4, 20B7) CDR-L1 | RASQSISSWLA |
| 14 | OX40(CLC-563, CLC564) CDR-L1 | RASQSVSSSYLA |
| 15 | OX40(17A9) CDR-L1 | QGDSLRSYYAS |
| 16 | OX40(8H9,49B4,1G4, 20B7) CDR-L2 | DASSLES |
| 17 | OX40(CLC-563, CLC564) CDR-L2 | GASSRAT |
| 18 | OX40(17A9) CDR-L2 | GKNNRPS |
| 19 | OX40(8H9) CDR-L3 | QQYLTYSRFT |
| 20 | OX40(49B4) CDR-L3 | QQYSSQPYT |
| 21 | OX40(1G4)CDR-L3 | QQYISYSMLT |
| 22 | OX40(20B7) CDR-L3 | QQYQAFSLT |
| 23 | OX40(CLC-563, CLC-564) CDR-L3 | QQYGSSPLT |
| 24 | OX40(17A9) CDR-L3 | NSRVMPHNRV |
| 25 | OX40(8H9) VH | QVQLVQSGAEVKKPGSSVKVSCKASGGTFSSYAISW VRQAPGQGLEWMGGIIPIFGTANYAQKFQGRVTITA DKSTSTAYMELSSLRSEDTAVYYCAREYGWMDYW GQGTTVTVSS |
| 26 | OX40(8H9) VL | DIQMTQSPSTLSASVGDRVTITCRASQSISSWLAWYQ QKPGKAPKLLIYDASSLESGVPSRFSGSGSGTEFTLTI SSLQPDDFATYYCQQYLTYSRFTFGQGTKVEIK |
| 27 | OX40(49B4) VH | QVQLVQSGAEVKKPGSSVKVSCKASGGTFSSYAISW VRQAPGQGLEWMGGIIPIFGTANYAQKFQGRVTITA DKSTSTAYMELSSLRSEDTAVYYCAREYYRGPYDY WGQGTTVTVSS |
| 28 | OX40(49B4) VL | DIQMTQSPSTLSASVGDRVTITCRASQSISSWLAWYQ QKPGKAPKLLIYDASSLESGVPSRFSGSGSGTEFTLTI SSLQPDDFATYYCQQYSSQPYTFGQGTKVEIK |
| 29 | OX40(1G4) VH | QVQLVQSGAEVKKPGSSVKVSCKASGGTFSSYAISW VRQAPGQGLEWMGGIIPIFGTANYAQKFQGRVTITA DKSTSTAYMELSSLRSEDTAVYYCAREYGSMDYWG QGTTVTVSS |
| 30 | OX40(1G4)VL | DIQMTQSPSTLSASVGDRVTITCRASQSISSWLAWYQ |
| | | QKPGKAPKLLIYDASSLESGVPSRFSGSGSGTEFTLTI SSLQPDDFATYYCQQYISYSMLTFGQGTKVEIK |
| 31 | OX40(20B7) VH | QVQLVQSGAEVKKPGSSVKVSCKASGGTFSSYAISW VRQAPGQGLEWMGGIIPIFGTANYAQKFQGRVTITA DKSTSTAYMELSSLRSEDTAVYYCARVNYPYSYWG DFDYWGQGTTVTVSS |
| 32 | OX40(20B7) VL | DIQMTQSPSTLSASVGDRVTITCRASQSISSWLAWYQ QKPGKAPKLLIYDASSLESGVPSRFSGSGSGTEFTLTI SSLQPDDFATYYCQQYQAFSLTFGQGTKVEIK |
| 33 | OX40(CLC-563) VH | EVQLLESGGGLVQPGGSLRLSCAASGFTFSSYAMSW VRQAPGKGLEWVSAISGSGGSTYYADSVKGRFTISR DNSKNTLYLQMNSLRAEDTAVYYCALDVGAFDYW GQGALVTVSS |
| 34 | OX40(CLC-563) VL | EIVLTQSPGTLSLSPGERATLSCRASQSVSSSYLAWY QQKPGQAPRLLIYGASSRATGIPDRFSGSGSGTDFTLT ISRLEPEDFAVYYCQQYGSSPLTFGQGTKVEIK |
| 35 | OX40(CLC-564) VH | EVQLLESGGGLVQPGGSLRLSCAASGFTFSSYAMSW VRQAPGKGLEWVSAISGSGGSTYYADSVKGRFTISR DNSKNTLYLQMNSLRAEDTAVYYCAFDVGPFDYWG QGTLVTVSS |
| 36 | OX40(CLC-564) VL | EIVLTQSPGTLSLSPGERATLSCRASQSVSSSYLAWY QQKPGQAPRLLIYGASSRATGIPDRFSGSGSGTDFTLT ISRLEPEDFAVYYCQQYGSSPLTFGQGTKVEIK |
| 37 | OX40(17A9) VH | EVQLLESGGGLVQPGGSLRLSCAASGFTFSSYAMSW VRQAPGKGLEWVSAISGSGGSTYYADSVKGRFTISR DNSKNTLYLQMNSLRAEDTAVYYCARVFYRGGVSM DYWGQGTLVTVSS |
| 38 | OX40(17A9) VL | SSELTQDPAVSVALGQTVRITCQGDSLRSYYASWYQ QKPGQAPVLVIYGKNNRPSGIPDRFSGSSSGNTASLTI TGAQAEDEADYYCNSRVMPHNRVFGGGTKLTV |
| 39 | FAP(28H1) CDR-H1 | SHAMS |
| 40 | FAP(4B9) CDR-H1 | SYAMS |
| 41 | FAP(28H1) CDR-H2 | AIW ASGEQYYADSVKG |
| 42 | (28H1)CDR-H2 FAP(4B9) CDR-H2 | AIIGSGASTYYADSVKG |
| 43 | FAP(28H1) CDR-H3 | GWLGNFDY |
| 44 | FAP(4B9) CDR-H3 | GWFGGFNY |
| 45 | FAP(28H1) CDR-L1 | RASQSVSRSYLA |
| 46 | FAP(4B9) CDR-L1 | RASQSVTSSYLA |
| 47 | FAP(28H1) CDR-L2 | GASTRAT |
| 48 | FAP(4B9) CDR-L2 | VGSRRAT |
| 49 | FAP(28H1) CDR-L3 | QQGQVIPPT |
| 50 | FAP(4B9) CDR-L3 | QQGIMLPPT |
| 51 | FAP(28H1) VH | EVQLLESGGGLVQPGGSLRLSCAASGFTFSSHAMSW VRQAPGKGLEWVSAIWASGEQYYADSVKGRFTISRD NSKNTLYLQMNSLRAEDTAVYYCAKGWLGNFDYW GQGTLVTVSS EIVLTQSPGTLSLSPGERATLSCRASQSVSRSYLAWY QQKPGQAPRLLIIGASTRATGIPDRFSGSGSGTDFTLTI SRLEPEDFAVYYCQQGQVIPPTFGQGTKVEIK |
| 52 | FAP(28H1) VL | [?] EIVLTQSPGTLSLSPGERATLSCRASQSVSRSYLAWY QQKPGQAPRLLIIGASTRATGIPDRFSGSGSGTDFTLTI SRLEPEDFAVYYCQQGQVIPPTFGQGTKVEIK |
| 53 | FAP(4B9) VH | EVQLLESGGGLVQPGGSLRLSCAASGFTFSSYAMSW VRQAPGKGLEWVSAIIGSGASTYYADSVKGRFTISRD NSKNTLYLQMNSLRAEDTAVYYCAKGWFGGFNYW GQGTLVTVSS |
| 54 | FAP(4B9) VL | EIVLTQSPGTLSLSPGERATLSCRASQSVTSSYLAWY QQKPGQAPRLLINVGSRRATGIPDRFSGSGSGTDFTL TISRLEPEDFAVYYCQQGIMLPPTFGQGTKVEIK |
| 55 | Human (hu) FAP | UniProt no. Q12884 760 AA |
| 56 | hu FAP ectodomain+poly-lys-tag+his₆-tag | RPSRVHNSEENTMRALTLKDILNGTFSYKTFFPNWIS GQEYLHQSADNNIVLYNIETGQSYTILSNRTMKSVNA SNYGLSPDRQFVYLESDYSKLWRYSYTATYYIYDLS NGEFVRGNELPRPIQYLCWSPVGSKLAYVYQNNIYL KQRPGDPPFQITFNGRENKIFNGIPDWVYEEEMLATK YALWWSPNGKFLAYAEFNDTDIPVIAYSYYGDEQYP RTINIPYPKAGAKNPVVRIFIIDTTYPAYVGPQEVPVP AMIASSDYYFSWLTWVTDERVCLQWLKRVQNVSVL SICDFREDWQTWDCPKTQEHIEESRTGWAGGFFVST PVFSYDAISYYKIFSDKDGYKHIHYIKDTVENAIQITS GKWEAINIFRVTQDSLFYSSNEFEEYPGRRNIYRISIGS YPPSKKCVTCHLRKERCQYYTASFSDYAKYYALVCY GPGIPISTLHDGRTDQEIKILEENKELENALKNIQLPKE EIKKLEVDEITLWYKMILPPQFDRSKKYPLLIQVYGG PCSQSVRSVFAVNWISYLASKEGMVIALVDGRGTAF QGDKLLYAVYRKLGVYEVEDQITAVRKFIEMGFIDE KRIAIWGWSYGGYVSSLALASGTGLFKCGIAVAPVSS WEYYASVYTERFMGLPTKDDNLEHYKNSTVMARAE YFRNVDYLLIHGTADDNVHFQNSAQIAKALVNAQV DFQAMWYSDQNHGLSGLSTNHLYTHMTHFLKQCFS LSDGKKKKKKGHHHHHH |
| 57 | nucleotide sequence hu FAP ectodomain+poly-lys-tag+his₆-tag | CGCCCTTCAAGAGTTCATAACTCTGAAGAAAATAC AATGAGAGCACTCACACTGAAGGATATTTTAAATG GAACATTTTCTTATAAAACATTTTTTCCAAACTGGA TTTCAGGACAAGAATATCTTCATCAATCTGCAGAT AACAATATAGTACTTTATAATATTGAAACAGGACA ATCATATACCATTTTGAGTAATAGAACCATGAAAA GTGTGAATGCTTCAAATTACGGCTTATCACCTGAT CGGCAATTTGTATATCTAGAAAGTGATTATTCAAA GCTTTGGAGATACTCTTACACAGCAACATATTACA TCTATGACCTTAGCAATGGAGAATTTGTAAGAGGA AATGAGCTTCCTCGTCCAATTCAGTATTTATGCTGG TCGCCTGTTGGGAGTAAATTAGCATATGTCTATCA AAACAATATCTATTTGAAACAAAGACCAGGAGAT CCACCTTTTCAAATAACATTTAATGGAAGAGAAAA TAAAATATTTAATGGAATCCCAGACTGGGTTTATG AAGAGGAAATGCTTGCTACAAAATATGCTCTCTGG TGGTCTCCTAATGGAAAATTTTTGGCATATGCGGA ATTTAATGATACGGATATACCAGTTATTGCCTATTC CTATTATGGCGATGAACAATATCCTAGAACAATAA ATATTCCATACCCAAAGGCTGGAGCTAAGAATCCC GTTGTTCGGATATTTATTATCGATACCACTTACCCT GCGTATGTAGGTCCCCAGGAAGTGCCTGTTCCAGC |
| | | AATGATAGCCTCAAGTGATTATTATTTCAGTTGGC TCACGTGGGTTACTGATGAACGAGTATGTTTGCAG TGGCTAAAAAGAGTCCAGAATGTTTCGGTCCTGTC TATATGTGACTTCAGGGAAGACTGGCAGACATGGG ATTGTCCAAAGACCCAGGAGCATATAGAAGAAAG CAGAACTGGATGGGCTGGTGGATTCTTTGTTTCAA CACCAGTTTTCAGCTATGATGCCATTTCGTACTACA AAATATTTAGTGACAAGGATGGCTACAAACATATT CACTATATCAAAGACACTGTGGAAAATGCTATTCA AATTACAAGTGGCAAGTGGGAGGCCATAAATATA TTCAGAGTAACACAGGATTCACTGTTTTATTCTAG CAATGAATTTGAAGAATACCCTGGAAGAAGAAAC ATCTACAGAATTAGCATTGGAAGCTATCCTCCAAG CAAGAAGTGTGTTACTTGCCATCTAAGGAAAGAAA GGTGCCAATATTACACAGCAAGTTTCAGCGACTAC GCCAAGTACTATGCACTTGTCTGCTACGGCCCAGG CATCCCCATTTCCACCCTTCATGATGGACGCACTG ATCAAGAAATTAAAATCCTGGAAGAAAACAAGGA ATTGGAAAATGCTTTGAAAAATATCCAGCTGCCTA AAGAGGAAATTAAGAAACTTGAAGTAGATGAAAT TACTTTATGGTACAAGATGATTCTTCCTCCTCAATT TGACAGATCAAAGAAGTATCCCTTGCTAATTCAAG TGTATGGTGGTCCCTGCAGTCAGAGTGTAAGGTCT GTATTTGCTGTTAATTGGATATCTTATCTTGCAAGT AAGGAAGGGATGGTCATTGCCTTGGTGGATGGTCG AGGAACAGCTTTCCAAGGTGACAAACTCCTCTATG CAGTGTATCGAAAGCTGGGTGTTTATGAAGTTGAA GACCAGATTACAGCTGTCAGAAAATTCATAGAAAT GGGTTTCATTGATGAAAAAAGAATAGCCATATGGG GCTGGTCCTATGGAGGATACGTTTCATCACTGGCC CTTGCATCTGGAACTGGTCTTTTCAAATGTGGTATA GCAGTGGCTCCAGTCTCCAGCTGGGAATATTACGC GTCTGTCTACACAGAGAGATTCATGGGTCTCCCAA CAAAGGATGATAATCTTGAGCACTATAAGAATTCA ACTGTGATGGCAAGAGCAGAATATTTCAGAAATGT AGACTATCTTCTCATCCACGGAACAGCAGATGATA ATGTGCACTTTCAAAACTCAGCACAGATTGCTAAA GCTCTGGTTAATGCACAAGTGGATTTCCAGGCAAT GTGGTACTCTGACCAGAACCACGGCTTATCCGGCC TGTCCACGAACCACTTATACACCCACATGACCCAC TTCCTAAAGCAGTGTTTCTCTTTGTCAGACGGCAA AAAGAAAAAGAAAAAGGGCCACCACCATCACCAT CAC |
| 58 | mouse FAP | UniProt no. P97321 761 AA |
| 59 | Murine FAP ectodomain+poly-lys-tag+his6-tag | RPSRVYKPEGNTKRALTLKDILNGTFSYKTYFPNWIS EQEYLHQSEDDNIVFYNIETRESYIILSNSTMKSVNAT DYGLSPDRQFVYLESDYSKLWRYSYTATYYIYDLQN GEFVRGYELPRPIQYLCWSPVGSKLAYVYQNNIYLK QRPGDPPFQITYTGRENRIFNGIPDWVYEEEMLATKY ALWWSPDGKFLAYVEFNDSDIPIIAYSYYGDGQYPR TINIPYPKAGAKNPVVRVFIVDTTYPHHVGPMEVPVP |
| | | EMIASSDYYFSWLTWVSSERVCLQWLKRVQNVSVL SICDFREDWHAWECPKNQEHVEESRTGWAGGFFVST PAFSQDATSYYKIFSDKDGYKHIHYIKDTVENAIQITS GKWEAIYIFRVTQDSLFYSSNEFEGYPGRRNIYRISIG NSPPSKKCVTCHLRKERCQYYTASFSYKAKYYALVC YGPGLPISTLHDGRTDQEIQVLEENKELENSLRNIQLP KVEIKKLKDGGLTFWYKMILPPQFDRSKKYPLLIQVY GGPCSQSVKSVFAVNWITYLASKEGIVIALVDGRGTA FQGDKFLHAVYRKLGVYEVEDQLTAVRKFIEMGFID EERIAIWGWSYGGYVSSLALASGTGLFKCGIAVAPVS SWEYYASIYSERFMGLPTKDDNLEHYKNSTVMARA EYFRNVDYLLIHGTADDNVHFQNSAQIAKALVNAQV DFQAMWYSDQNHGILSGRSQNHLYTHMTHFLKQCF SLSDGKKKKKKGHHHHHH |
| 60 | nucleotide sequence Murine FAP ectodomain+poly-lys-tag+his6-tag | CGTCCCTCAAGAGTTTACAAACCTGAAGGAAACAC AAAGAGAGCTCTTACCTTGAAGGATATTTAAATG GAACATTCTCATATAAAACATATTTCCCAACTGG ATTTCAGAACAAGAATATCTTCATCAATCTGAGGA TGATAACATAGTATTTTATAATATTGAAACAAGAG AATCATATATCATTTTGAGTAATAGCACCATGAAA AGTGTGAATGCTACAGATTATGGTTTGTCACCTGA TCGGCAATTTGTGTATCTAGAAAGTGATTATTCAA AGCTCTGGCGATATTCATACACAGCGACATACTAC ATCTACGACCTTCAGAATGGGGAATTTGTAAGAGG ATACGAGCTCCCTCGTCCAATTCAGTATCTATGCT GGTCGCCTGTTGGGAGTAAATTAGCATATGTATAT CAAAACAATATTTATTTGAAACAAAGACCAGGAG ATCCACCTTTTCAAATAACTTATACTGGAAGAGAA AATAGAATATTTAATGGAATACCAGACTGGGTTTA TGAAGAGGAAATGCTTGCCACAAAATATGCTCTTT GGTGGTCTCCAGATGGAAAATTTTTGGCATATGTA GAATTTAATGATTCAGATATACCAATTATTGCCTA TTCTTATTATGGTGATGGACAGTATCCTAGAACTA TAAATATTCCATATCCAAAGGCTGGGGCTAAGAAT CCGGTTGTTCGTGTTTTTATTGTTGACACCACCTAC CCTCACCACGTGGGCCCAATGGAAGTGCCAGTTCC AGAAATGATAGCCTCAAGTGACTATTATTTCAGCT GGCTCACATGGGTGTCCAGTGAACGAGTATGCTTG CAGTGGCTAAAAAGAGTGCAGAATGTCTCAGTCCT GTCTATATGTGATTTCAGGGAAGACTGGCATGCAT GGGAATGTCCAAAGAACCAGGAGCATGTAGAAGA AAGCAGAACAGGATGGGCTGGTGGATTCTTTGTTT CGACACCAGCTTTTAGCCAGGATGCCACTTCTTAC TACAAAATATTTAGCGACAAGGATGGTTACAAACA TATTCACTACATCAAAGACACTGTGGAAAATGCTA TTCAAATTACAAGTGGCAAGTGGGAGGCCATATAT ATATTCCGCGTAACACAGGATTCACTGTTTTATTCT AGCAATGAATTTGAAGGTTACCCTGGAAGAAGAA ACATCTACAGAATTAGCATTGGAAACTCTCCTCCG AGCAAGAAGTGTGTTACTTGCCATCTAAGGAAAGA AAGGTGCCAATATTACACAGCAAGTTTCAGCTACA |
| | | AAGCCAAGTACTATGCACTCGTCTGCTATGGCCCT GGCCTCCCCATTTCCACCCTCCATGATGGCCGCAC AGACCAAGAAATACAAGTATTAGAAGAAAACAAA GAACTGGAAAATTCTCTGAGAAATATCCAGCTGCC TAAAGTGGAGATTAAGAAGCTCAAAGACGGGGGA CTGACTTTCTGGTACAAGATGATTCTGCCTCCTCAG TTTGACAGATCAAAGAAGTACCCTTTGCTAATTCA AGTGTATGGTGGTCCTTGTAGCCAGAGTGTTAAGT CTGTGTTTGCTGTTAATTGGATAACTTATCTCGCAA GTAAGGAGGGGATAGTCATTGCCCTGGTAGATGGT CGGGGCACTGCTTTCCAAGGTGACAAATTCCTGCA TGCCGTGTATCGAAAACTGGGTGTATATGAAGTTG AGGACCAGCTCACAGCTGTCAGAAAATTCATAGA AATGGGTTTCATTGATGAAGAAAGAATAGCCATAT GGGGCTGGTCCTACGGAGGTTATGTTTCATCCCTG GCCCTTGCATCTGGAACTGGTCTTTTCAAATGTGG CATAGCAGTGGCTCCAGTCTCCAGCTGGGAATATT ACGCATCTATCTACTCAGAGAGATTCATGGGCCTC CCAACAAAGGACGACAATCTCGAACACTATAAAA ATTCAACTGTGATGGCAAGAGCAGAATATTTCAGA AATGTAGACTATCTTCTCATCCACGGAACAGCAGA TGATAATGTGCACTTTCAGAACTCAGCACAGATTG CTAAAGCTTTGGTTAATGCACAAGTGGATTTCCAG GCGATGTGGTACTCTGACCAGAACCATGGTATATT ATCTGGGCGCTCCCAGAATCATTTATATACCCACA TGACGCACTTCCTCAAGCAATGCTTTTCTTTATCAG ACGGCAAAAAGAAAAAGAAAAAGGGCCACCACCA TCACCATCAC |
| 61 | Cynomolgus FAP ectodomain+poly-lys-tag+his6-tag | RPPRVHNSEENTMRALTLKDILNGTFSYKTFFPNWIS GQEYLHQSADNNIVLYNIETGQSYTILSNRTMKSVNA SNYGLSPDRQFVYLESDYSKLWRYSYTATYYIYDLS NGEFVRGNELPRPIQYLCWSPVGSKLAYVYQNNIYL KQRPGDPPFQITFNGRENKIFNGIPDWVYEEEMLATK YALWWSPNGKFLAYAEFNDTDIPVIAYSYYGDEQYP RTINIPYPKAGAKNPFVRIFIIDTTYPAYVGPQEVPVP AMIASSDYYFSWLTWVTDERVCLQWLKRVQNVSVL SICDFREDWQTWDCPKTQEHIEESRTGWAGGFFVST PVFSYDAISYYKIFSDKDGYKHIHYIKDTVENAIQITS GKWEAINIFRVTQDSLFYSSNEFEDYPGRRNIYRISIG SYPPSKKCVTCHLRKERCQYYTASFSDYAKYYALVC YGPGIPISTLHDGRTDQEIKILEENKELENALKNIQLP KEEIKKLEVDEITLWYKMILPPQFDRSKKYPLLIQVY GGPCSQSVRSVFAVNWISYLASKEGMVIALVDGRGT AFQGDKLLYAVYRKLGVYEVEDQITAVRKFIEMGFI DEKRIAIWGWSYGGYVSSLALASGTGLFKCGIAVAP VSSWEYYASVYTERFMGLPTKDDNLEHYKNSTVMA RAEYFRNVDYLLIHGTADDNVHFQNSAQIAKALVNA QVDFQAMWYSDQNHGLSGLSTNHLYTHMTHFLKQ CFSLSDGKKKKKKGHHHHHH |
| 62 | nucleotide sequence Cynomolgus FAP | CGCCCTCCAAGAGTTCATAACTCTGAAGAAAATAC AATGAGAGCACTCACACTGAAGGATATTTTAAATG |
| | ectodomain+poly-lys-tag+his6-tag | GGACATTTTCTTATAAAACATTTTTCCAAACTGGA TTTCAGGACAAGAATATCTTCATCAATCTGCAGAT AACAATATAGTACTTTATAATATTGAAACAGGACA ATCATATACCATTTTGAGTAACAGAACCATGAAAA GTGTGAATGCTTCAAATTATGGCTTATCACCTGAT CGGCAATTTGTATATCTAGAAAGTGATTATTCAAA GCTTTGGAGATACTCTTACACAGCAACATATTACA TCTATGACCTTAGCAATGGAGAATTTGTAAGAGGA AATGAGCTTCCTCGTCCAATTCAGTATTTATGCTGG TCGCCTGTTGGGAGTAAATTAGCATATGTCTATCA AAACAATATCTATTTGAAACAAAGACCAGGAGAT CCACCTTTTCAAATAACATTTAATGGAAGAGAAAA TAAAATATTTAATGGAATCCCAGACTGGGTTTATG AAGAGGAAATGCTTGCTACAAAATATGCTCTCTGG TGGTCTCCTAATGGAAAATTTTTGGCATATGCGGA ATTTAATGATACAGATATACCAGTTATTGCCTATTC CTATTATGGCGATGAACAATATCCCAGAACAATAA ATATTCCATACCCAAAGGCCGGAGCTAAGAATCCT TTTGTTCGGATATTTATTATCGATACCACTTACCCT GCGTATGTAGGTCCCCAGGAAGTGCCTGTTCCAGC AATGATAGCCTCAAGTGATTATTATTTCAGTTGGC TCACGTGGGTTACTGATGAACGAGTATGTTTGCAG TGGCTAAAAAGAGTCCAGAATGTTTCGGTCTTGTC TATATGTGATTTCAGGGAAGACTGGCAGACATGGG ATTGTCCAAAGACCCAGGAGCATATAGAAGAAAG CAGAACTGGATGGGCTGGTGGATTCTTTGTTTCAA CACCAGTTTTCAGCTATGATGCCATTTCATACTACA AAATATTTAGTGACAAGGATGGCTACAAACATATT CACTATATCAAAGACACTGTGGAAAATGCTATTCA AATTACAAGTGGCAAGTGGGAGGCCATAAATATA TTCAGAGTAACACAGGATTCACTGTTTTATTCTAG CAATGAATTTGAAGATTACCCTGGAAGAAGAAAC ATCTACAGAATTAGCATTGGAAGCTATCCTCCAAG CAAGAAGTGTGTTACTTGCCATCTAAGGAAAGAAA GGTGCCAATATTACACAGCAAGTTTCAGCGACTAC GCCAAGTACTATGCACTTGTCTGCTATGGCCCAGG CATCCCCATTTCCACCCTTCATGACGGACGCACTG ATCAAGAAATTAAAATCCTGGAAGAAAACAAGGA ATTGGAAAATGCTTTGAAAAATATCCAGCTGCCTA AAGAGGAAATTAAGAAACTTGAAGTAGATGAAAT TACTTTATGGTACAAGATGATTCTTCCTCCTCAATT TGACAGATCAAAGAAGTATCCCTTGCTAATTCAAG TGTATGGTGGTCCCTGCAGTCAGAGTGTAAGGTCT GTATTTGCTGTTAATTGGATATCTTATCTTGCAAGT AAGGAAGGGATGGTCATTGCCTTGGTGGATGGTCG GGGAACAGCTTTCCAAGGTGACAAACTCCTGTATG CAGTGTATCGAAAGCTGGGTGTTTATGAAGTTGAA GACCAGATTACAGCTGTCAGAAAATTCATAGAAAT GGGTTTCATTGATGAAAAAAGAATAGCCATATGGG GCTGGTCCTATGGAGGATATGTTTCATCACTGGCC CTTGCATCTGGAACTGGTCTTTTCAAATGTGGGAT |
| | | AGCAGTGGCTCCAGTCTCCAGCTGGGAATATTACG CGTCTGTCTACACAGAGAGATTCATGGGTCTCCCA ACAAAGGATGATAATCTTGAGCACTATAAGAATTC AACTGTGATGGCAAGAGCAGAATATTTCAGAAAT GTAGACTATCTTCTCATCCACGGAACAGCAGATGA TAATGTGCACTTTCAAAACTCAGCACAGATTGCTA AAGCTCTGGTTAATGCACAAGTGGATTTCCAGGCA ATGTGGTACTCTGACCAGAACCACGGCTTATCCGG CCTGTCCACGAACCACTTATACACCCACATGACCC ACTTCCTAAAGCAGTGTTTCTCTTTGTCAGACGGC AAAAAGAAAAAGAAAAAGGGCCACCACCATCACC ATCAC |
| 63 | human CEA | UniProt no. P06731, 702 AA |
| 64 | human MCSP | UniProt no. Q6UVK1, 2322 AA |
| 65 | human EGFR | UniProt no. P00533, 1210AA |
| 66 | human CD 19 | UniProt no. P15391, 556AA |
| 67 | human CD20 | Uniprot no. P11836, 297 AA |
| 68 | human CD33 | UniProt no. P20138, 364AA |
| 69 | human OX40 | UniProt no. P43489, 277AA |
| 70 | human 4-1BB | UniProt no. Q07011, 255AA |
| 71 | human CD27 | UniProt no. P26842, 260AA |
| 72 | human HVEM | UniProt no. Q92956, 283AA |
| 73 | human CD30 | UniProt no. P28908, 595AA |
| 74 | human GITR | UniProt no. Q9Y5U5, 241AA |
| 75 | murine OX40 | UniProt no. P47741, 272AA |
| 76 | murine 4-1BB | UniProt no. P20334, 256AA |
| 77 | cynomolgus 4-1BB | Uniprot no. F6W5G6, 254AA |
| 78 | Peptide linker (G4S) | GGGGS |
| 79 | Peptide linker (G4S)₂ | GGGGSGGGGS |
| 80 | Peptide linker (SG4)₂ | SGGGGSGGGG |
| 81 | Peptide linker G4(SG4)₂ | GGGGSGGGGSGGGG |
| 82 | Peptide linker | GSPGSSSSGS |
| 83 | Peptide linker (G4S)₃ | GGGGSGGGGSGGGGS |
| 84 | Peptide linker (G4S)₄ | GGGGSGGGGSGGGGSGGGGS |
| 85 | Peptide linker | GSGSGSGS |
| 86 | Peptide linker | GSGSGNGS |
| 87 | Peptide linker | GGSGSGSG |
| 88 | Peptide linker | GGSGSG |
| 89 | Peptide linker | GGSG |
| 90 | Peptide linker | GGSGNGSG |
| 91 | Peptide linker | GGNGSGSG |
| 92 | Peptide linker | GGNGSG |
| 93 | cynomolgus Ox40 ECD | aa 29-214 |
| 94 | murine OX40 ECD | aa 10-211 |
| 95 | Nucleotide sequence Fc hole chain | see Table 2 |
| 96 | Nucleotide sequence human OX40 antigen Fc knob chain | see Table 2 |
| 97 | Nucleotide sequence cynomolgus OX40 antigen Fc knob chain | see Table 2 |
| 98 | Nucleotide sequence murine OX40 antigen Fc knob chain | see Table 2 |
| 99 | Fc hole chain | see Table 2 |
| 100 | human OX40 antigen Fc knob chain | see Table 2 |
| 101 | cynomolgus OX40 antigen Fc knob chain | see Table 2 |
| 102 | murine OX40 antigen Fc knob chain | see Table 2 |
| 103 | nucleotide sequence of library DP88-4 | see Table 3 |
| 104 | nucleotide sequence of Fab light chain Vk1 5 | see Table 4 |
| 105 | Fab light chain Vk1 5 | see Table 4 |
| 106 | nucleotide sequence of Fab heavy chain VH1 69 | see Table 4 |
| 107 | Fab heavy chain VH1 69 | see Table 4 |
| 108 | LMB3 | see Table 5 |
| 109 | Vk1 5 L3r S | see Table 5 |
| 110 | Vk1 5 L3r SY | see Table 5 |
| 111 | Vk1 5 L3r SPY | see Table 5 |
| 112 | RJH31 | see Table 5 |
| 113 | RJH32 | see Table 5 |
| 114 | DP88-v4-4 | see Table 5 |
| 115 | DP88-v4-6 | see Table 5 |
| 116 | DP88-v4-8 | see Table 5 |
| 117 | fdseqlong | see Table 5 |
| 118 | (Vk3_20/VH3_23) template | see Table 6 |
| 119 | nucleotide sequence of Fab light chain Vk3_20 | see Table 7 |
| 120 | Fab light chain Vk3 20 | see Table 7 |
| 121 | nucleotide sequence of Fab heavy chain VH3 23 | see Table 7 |
| 122 | Fab heavy chain VH3_23 (DP47) | see Table 7 |
| 123 | MS64 | see Table 8 |
| 124 | DP47CDR3_ba (mod.) | see Table 8 |
| 125 | DP47-v4-4 | see Table 8 |
| 126 | DP47-v4-6 | see Table 8 |
| 127 | DP47-v4-8 | see Table 8 |
| 128 | fdseqlong | see Table 8 |
| 129 | V13_19/VH3_23 library template | see Table 9 |
| 130 | nucleotide sequence of Fab light chain Vl3_19 | see Table 10 |
| 131 | Fab light chain Vl3_19 | see Table 10 |
| 132 | LMB3 | see Table 11 |
| 133 | Vl_3_19_L3r_V | see Table 11 |
| 134 | Vl_3_19_L3r_HV | see Table 11 |
| 135 | Vl_3_19_L3r_HLV | see Table 11 |
| 136 | RJH80 | see Table 11 |
| 137 | Nucleotide sequence OX40(8H9) VL | see Table 12 |
| 138 | Nucleotide sequence OX40(8H9) VH | see Table 12 |
| 139 | Nucleotide sequence OX40(49B4) VL | see Table 12 |
| 140 | Nucleotide sequence OX40(49B4) VH | see Table 12 |
| 141 | Nucleotide sequence OX40(1G4) VL | see Table 12 |
| 142 | Nucleotide sequence OX40(1G4) VH | see Table 12 |
| 143 | Nucleotide sequence OX40(20B7) VL | see Table 12 |
| 144 | Nucleotide sequence OX40(20B7) VH | see Table 12 |
| 145 | Nucleotide sequence OX40(CLC-563) VL | see Table 12 |
| 146 | Nucleotide sequence OX40(CLC-563) VH | see Table 12 |
| 147 | Nucleotide sequence OX40(CLC-564) VL | see Table 12 |
| 148 | Nucleotide sequence OX40(CLC-564) VH | see Table 12 |
| 149 | Nucleotide sequence OX40(17A9) VL | see Table 12 |
| 150 | Nucleotide sequence OX40(17A9) VH | see Table 12 |
| 151 | 8H9 P329GLALA IgG1 (light chain) | nucleotide sequence, see Table 13 |
| 152 | 8H9 P329GLALA IgG1 (heavy chain) | nucleotide sequence, see Table 13 |
| 153 | 8H9 P329GLALA IgG1 (light chain) | see Table 13 |
| 154 | 8H9 P329GLALA IgG1 (heavy chain) | see Table 13 |
| 155 | 49B4 P329GLALA IgG1 (light chain) | nucleotide sequence, see Table 13 |
| 156 | 49B4 P329GLALA IgG1 (heavy chain) | nucleotide sequence, see Table 13 |
| 157 | 49B4 P329GLALA IgG1 (light chain) | see Table 13 |
| 158 | 49B4 P329GLALA IgG1 (heavy chain) | see Table 13 |
| 159 | 1G4 P329GLALA IgG1 (light chain) | nucleotide sequence, see Table 13 |
| 160 | 1G4 P329GLALA IgG1 (heavy chain) | nucleotide sequence, see Table 13 |
| 161 | 1G4 P329GLALA IgG1 (light chain) | see Table 13 |
| 162 | 1G4 P329GLALA IgG1 (heavy chain) | see Table 13 |
| 163 | 20B7 P329GLALA IgG1 (light chain) | nucleotide sequence, see Table 13 |
| 164 | 20B7 P329GLALA IgG1 (heavy chain) | nucleotide sequence, see Table 13 |
| 165 | 20B7 P329GLALA IgG1 (light chain) | see Table 13 |
| 166 | 20B7 P329GLALA IgG1 (heavy chain) | see Table 13 |
| 167 | CLC-563 P329GLALA IgG1 (light chain) | nucleotide sequence, see Table 13 |
| 168 | CLC-563 P329GLALA IgG1 (heavy chain) | nucleotide sequence, see Table 13 |
| 169 | CLC-563 P329GLALA IgG1 (light chain) | see Table 13 |
| 170 | CLC-563 P329GLALA IgG1 (heavy chain) | see Table 13 |
| 171 | CLC-564 P329GLALA IgG1 (light chain) | nucleotide sequence, see Table 13 |
| 172 | CLC-564 P329GLALA IgG1 (heavy chain) | nucleotide sequence, see Table 13 |
| 173 | CLC-564 P329GLALA IgG1 (light chain) | see Table 13 |
| 174 | CLC-564 P329GLALA IgG1 (heavy chain) | see Table 13 |
| 175 | 17A9 P329GLALA IgG1 (light chain) | nucleotide sequence, see Table 13 |
| 176 | 17A9 P329GLALA IgG1 (heavy chain) | nucleotide sequence, see Table 13 |
| 177 | 17A9 P329GLALA IgG1 (light chain) | see Table 13 |
| 178 | 17A9 P329GLALA IgG1 (heavy chain) | see Table 13 |
| 179 | human OX40 His | see Table 15 |
| 180 | murine OX40 His | see Table 15 |
| 181 | Nucleotide sequence dimeric human OX40 antigen Fc | see Table 21 |
| 182 | dimeric human OX40 antigen Fc | see Table 21 |
| 183 | (8H9) VHCH1-Heavy chain-(28H1) VHCL | nucleotide sequence, see Table 25 |
| 184 | VLCH1-Light chain 2 (28H1) | nucleotide sequence, see Table 25 |
| 185 | (8H9) VHCH1-Heavy chain-(28H1) VHCL | see Table 25 |
| 186 | VLCH1-Light chain 2 (28H1) | see Table 25 |
| 187 | (49B4) VHCH1-Heavy chain-(28H1) VHCL | nucleotide sequence, see Table 25 |
| 188 | (49B4) VHCH1-Heavy chain-(28H1) VHCL | see Table 25 |
| 189 | (1G4) VHCH1-Heavy chain-(28H1) VHCL | nucleotide sequence, see Table 25 |
| 190 | (1G4) VHCH1-Heavy chain-(28H1) VHCL | see Table 25 |
| 191 | (20B7) VHCH1-Heavy chain-(28H1) VHCL | nucleotide sequence, see Table 25 |
| 192 | (20B7) VHCH1-Heavy chain-(28H1) VHCL | see Table 25 |
| 193 | (CLC-563) VHCH1-Heavy chain-(28H1) VHCL | nucleotide sequence, see Table 25 |
| 194 | (CLC-563) VHCH1-Heavy chain-(28H1) VHCL | see Table 25 |
| 195 | (CLC-564) VHCH1-Heavy chain-(28H1) VHCL | nucleotide sequence, see Table 25 |
| 196 | (CLC-564) VHCH1-Heavy chain-(28H1) VHCL | see Table 25 |
| 197 | (28H1) VHCL-heavy chain hole | nucleotide sequence, see Table 27 |
| 198 | (28H1) VLCH1-light chain 2 | see Table 27 |
| 199 | (8H9) VHCH1-heavy chain knob | nucleotide sequence, see Table 27 |
| 200 | (8H9) VHCH1-heavy chain knob | see Table 27 |
| 201 | (49B4) VHCH1-heavy chain knob | nucleotide sequence, see Table 27 |
| 202 | (49B4) VHCH1-heavy chain knob | see Table 27 |
| 203 | (1G4) VHCH1-heavy chain knob | nucleotide sequence, see Table 27 |
| 204 | (1G4) VHCH1-heavy chain knob | see Table 27 |
| 205 | (20B7) VHCH1-heavy chain knob | nucleotide sequence, see Table 27 |
| 206 | (20B7) VHCH1-heavy chain knob | see Table 27 |
| 207 | (CLC-563) VHCH1-heavy chain knob | nucleotide sequence, see Table 27 |
| 208 | (CLC-563) VHCH1-heavy chain knob | see Table 27 |
| 209 | (CLC-564) VHCH1-heavy chain knob | nucleotide sequence, see Table 27 |
| 210 | (CLC-564) VHCH1-heavy chain knob | see Table 27 |
| 211 | HC 1 (49B4) VHCH1_VHCH1 Fc knob VH (4B9) (nucleotide sequence) | see Table 29 |
| 212 | HC 2 (49B4) VHCH1_VHCH1 Fc hole VL (4B9) (nucleotide sequence) | see Table 29 |
| 213 | HC 1 (49B4) VHCH1_VHCH1 Fc knob VH (4B9) | see Table 29 |
| 214 | HC 2 (49B4) VHCH1_VHCH1 Fc hole VL (4B9) | see Table 29 |
| 215 | HC 1 (49B4) VHCH1_VHCH1 Fc knob VH (28H1) (nucleotide sequence) | see Table 29 |
| 216 | HC 2 (49B4) VHCH1_VHCH1 Fc hole VL (28H1) (nucleotide sequence) | see Table 29 |
| 217 | HC 1 (49B4) VHCH1_VHCH1 Fc knob VH (28H1) | see Table 29 |
| 218 | HC 2 (49B4) VHCH1_VHCH1 Fc hole VL (28H1) | see Table 29 |
| 219 | HC 1 (49B4) VHCH1_VHCH1 Fc knob VH (DP47) (nucleotide sequence) | see Table 29 |
| 220 | HC 2 (49B4) VHCH1_VHCH1 Fc hole VL (DP47) (nucleotide sequence) | see Table 29 |
| 221 | HC 1 (49B4) VHCH1_VHCH1 Fc knob VH (DP47) | see Table 29 |
| 222 | HC 2 (49B4) VHCH1_VHCH1 Fc hole VL (DP47) | see Table 29 |
| 223 | (49B4) VLCL^{∗}-light chain 1 ^{∗}E123R/Q124K (nucleotide sequence) | see Table 34 |
| 224 | heavy chain (49B4) VHCH1^{∗}_VHCH1^{∗} Fc knob VLCH1 (28H1) ^{∗}K147E/K213E (nucleotide sequence) | see Table 34 |
| 225 | (28H1) VHCL- light chain 2 (nucleotide sequence) | see Table 34 |
| 226 | (49B4) VLCL^{∗}-light chain 1 ^{∗}E123R/Q124K | see Table 34 |
| 227 | heavy chain (49B4) VHCH1^{∗}_VHCH1^{∗} Fc knob VLCH1 (28H1) ^{∗}K147E/K213E | see Table 34 |
| 228 | (28H1) VHCL- light chain 2 | see Table 34 |
| 229 | heavy chain (49B4) VHCH1^{∗}_VHCH1^{∗} Fc knob VLCH1 (DP47) ^{∗}K147E/K213E (nucleotide sequence) | see Table 34 |
| 230 | (DP47) VHCL- light chain 2 (nucleotide sequence) | see Table 34 |
| 231 | heavy chain (49B4) VHCH1^{∗}_VHCH1^{∗} Fc knob VLCH1 (DP47) ^{∗}K147E/K213E | see Table 34 |
| 232 | (DP47) VHCL- light chain 2 | see Table 34 |
| 233 | HC 1 (49B4) VHCH1_VHCH1 Fc knob VL (4B9) | see Table 31 |
| 234 | HC 2 (49B4) VHCH1_VHCH1 Fc hole VH (4B9) | see Table 31 |
| 235 | HC 1 (49B4) VHCH1_VHCH1 Fc wt knob VH (4B9) (nucleotide sequence) | see Table 32 |
| 236 | HC 2 (49B4) VHCH1_VHCH1 Fc wt hole VL (4B9) (nucleotide sequence) | see Table 32 |
| 237 | HC 1 (49B4) VHCH1_VHCH1 Fc wt knob VH (4B9) | see Table 32 |
| 238 | HC 2 (49B4) VHCH1_VHCH1 Fc wt hole VL (4B9) | see Table 32 |
| 239 | human 4-1BB ECD | Uniprot Q07011, aa 24-186, Table 39 |
| 240 | cynomolgus 4-1BB ECD | aa 24-186, Table 39 |
| 241 | murine 4-1BB ECD | Uniprot P20334, aa 24-187, Table 39 |
| 242 | Nucleotide sequence of human 4-1BB antigen Fc knob chain | see Table 40 |
| 243 | Nucleotide sequence of cynomolgus 4-1BB antigen Fc knob chain | see Table 40 |
| 244 | Nucleotide sequence of murine 4-1BB antigen Fc knob chain | see Table 40 |
| 245 | human 4-1BB antigen Fc knob chain | see Table 40 |
| 246 | cynomolgus 4-1BB antigen Fc knob chain | see Table 40 |
| 247 | murine 4-1BB antigen Fc knob chain | see Table 40 |
| 248 | MS63 Primer | see Table 46, TTTCGCACAGTAATATACGGCCGTGTCC |
| 249 | 4-1BB(12B3,11D5, 9B11, 20G2) CDR-H1 | SYAIS |
| 250 | 4-1BB(25G7) CDR-H1 | SYAMS |
| 251 | 4-1BB(12B3,11D5, 9B11, 20G2) CDR-H2 | GIIPIFGTANYAQKFQG |
| 252 | 4-1BB(25G7) CDR-H2 | AISGSGGSTYYADSVKG |
| 253 | 4-1BB(12B3) CDR-H3 | SEFRFYADFDY |
| 254 | 4-1BB(25G7) CDR-H3 | DDPWPPFDY |
| 255 | 4-1BB(11D5) CDR-H3 | STLIYGYFDY |
| 256 | 4-1BB(9B11) CDR-H3 | S SGAYPGYFDY |
| 257 | 4-1BB(20G2) CDR-H3 | SYYWESYPFDY |
| 258 | 4-1BB(12B3,11D5,9B11, 20G2) CDR-L1 | RASQSISSWLA |
| 259 | 4-1BB(25G7) CDR-L1 | QGDSLRSYYAS |
| 260 | 4-1BB(12B3,11D5,9B11, 20G2) CDR-L2 | DASSLES |
| 261 | 4-1BB(25G7) CDR-L2 | GKNNRPS |
| 262 | 4-1BB(12B3) CDR-L3 | QQYHSYPQT |
| 263 | 4-1BB(25G7) CDR-L3 | NSLDRRGMWV |
| 264 | 4-1BB(11D5) CDR-L3 | QQLNSYPQT |
| 265 | 4-1BB(9B11) CDR-L3 | QQVNSYPQT |
| 266 | 4-1BB(20G2) CDR-L3 | QQQHSYYT |
| 267 | 4-1BB(12B3) VH | QVQLVQSGAEVKKPGSSVKVSCKASGGTFSSYAISW VRQAPGQGLEWMGGIIPIFGTANYAQKFQGRVTITA DKSTSTAYMELSSLRSEDTAVYYCARSEFRFYADFD YWGQGTTVTVSS |
| 268 | 4-1BB(12B3) VL | DIQMTQSPSTLSASVGDRVTITCRASQSISSWLAWYQ |
| | | QKPGKAPKLLIYDASSLESGVPSRFSGSGSGTEFTLTI SSLQPDDFATYYCQQYHSYPQTFGQGTKVEIK |
| 269 | 4-1BB(25G7) VH | EVQLLESGGGLVQPGGSLRLSCAASGFTFSSYAMSW VRQAPGKGLEWVSAISGSGGSTYYADSVKGRFTISR DNSKNTLYLQMNSLRAEDTAVYYCARDDPWPPFDY WGQGTLVTVSS |
| 270 | 4-1BB(25G7) VL | SSELTQDPAVSVALGQTVRITCQGDSLRSYYASWYQ QKPGQAPVLVIYGKNNRPSGIPDRFSGSSSGNTASLTI TGAQAEDEADYYCNSLDRRGMWVFGGGTKLTV |
| 271 | 4-1BB(11D5) VH | QVQLVQSGAEVKKPGSSVKVSCKASGGTFSSYAISW VRQAPGQGLEWMGGIIPIFGTANYAQKFQGRVTITA DKSTSTAYMELSSLRSEDTAVYYCARSTLIYGYFDY WGQGTTVTVSS |
| 272 | 4-1BB(11D5) VL | DIQMTQSPSTLSASVGDRVTITCRASQSISSWLAWYQ QKPGKAPKLLIYDASSLESGVPSRFSGSGSGTEFTLTI SSLQPDDFATYYCQQLNSYPQTFGQGTKVEIK |
| 273 | 4-1BB(9B11) VH | QVQLVQSGAEVKKPGSSVKVSCKASGGTFSSYAISW VRQAPGQGLEWMGGIIPIFGTANYAQKFQGRVTITA DKSTSTAYMELSSLRSEDTAVYYCARSSGAYPGYFD YWGQGTTVTVSS |
| 274 | 4-1BB(9B11) VL | DIQMTQSPSTLSASVGDRVTITCRASQSISSWLAWYQ QKPGKAPKLLIYDASSLESGVPSRFSGSGSGTEFTLTI SSLQPDDFATYYCQQVNSYPQTFGQGTKVEIK |
| 275 | 4-1BB(20G2) VH | QVQLVQSGAEVKKPGSSVKVSCKASGGTFSSYAISW VRQAPGQGLEWMGGIIPIFGTANYAQKFQGRVTITA DKSTSTAYMELSSLRSEDTAVYYCARSYYWESYPFD YWGQGTTVTVSS |
| 276 | 4-1BB(20G2) VL | DIQMTQSPSTLSASVGDRVTITCRASQSISSWLAWYQ QKPGKAPKLLIYDASSLESGVPSRFSGSGSGTEFTLTI SSLQPDDFATYYCQQHSYYTFGQGTKVEIK |
| 277 | Nucleotide sequence of 4-1BB(12B3) VH | see Table 47 |
| 278 | Nucleotide sequence of 4-1BB(12B3) VL | see Table 47 |
| 279 | Nucleotide sequence of 4-1BB(25G7) VH | see Table 47 |
| 280 | Nucleotide sequence of 4-1BB(25G7) VL | see Table 47 |
| 281 | Nucleotide sequence of 4-1BB(11D5) VH | see Table 47 |
| 282 | Nucleotide sequence of 4-1BB(11D5) VL | see Table 47 |
| 283 | Nucleotide sequence of 4-1BB(9B11) VH | see Table 47 |
| 284 | Nucleotide sequence of 4-1BB(9B11) VL | see Table 47 |
| 285 | Nucleotide sequence of 4-1BB(20G2) VH | see Table 47 |
| 286 | Nucleotide sequence of 4-1BB(20G2) VL | see Table 47 |
| 287 | nucleotide sequence of 12B3 P329GLALA IgG1 (light chain) | see Table 48 |
| 288 | nucleotide sequence of 12B3 P329GLALA IgG1 (heavy chain) | see Table 48 |
| 289 | 12B3 P329GLALA IgG1 (light chain) | see Table 48 |
| 290 | 12B3 P329GLALA IgG1 (heavy chain) | see Table 48 |
| 291 | nucleotide sequence of 25G7 P329GLALA IgG1 (light chain) | see Table 48 |
| 292 | nucleotide sequence of 25G7 P329GLALA IgG1 (heavy chain) | see Table 48 |
| 293 | 25G7 P329GLALA IgG1 (light chain) | see Table 48 |
| 294 | 25G7 P329GLALA IgG1 (heavy chain) | see Table 48 |
| 295 | nucleotide sequence of 11D5 P329GLALA IgG1 (light chain) | see Table 48 |
| 296 | nucleotide sequence of 11D5 P329GLALA IgG1 (heavy chain) | see Table 48 |
| 297 | 11D5 P329GLALA IgG1 (light chain) | see Table 48 |
| 298 | 11D5 P329GLALA IgG1 (heavy chain) | see Table 48 |
| 299 | nucleotide sequence of 9B11 P329GLALA IgG1 (light chain) | see Table 48 |
| 300 | nucleotide sequence of 9B11 P329GLALA IgG1 (heavy chain) | see Table 48 |
| 301 | 9B11 P329GLALA IgG1 (light chain) | see Table 48 |
| 302 | 9B11 P329GLALA IgG1 (heavy chain) | see Table 48 |
| 303 | nucleotide sequence of 20G2 P329GLALA IgG1 (light chain) | see Table 48 |
| 304 | nucleotide sequence of 20G2 P329GLALA IgG1 (heavy chain) | see Table 48 |
| 305 | 20G2 P329GLALA IgG1 (light chain) | see Table 48 |
| 306 | 20G2 P329GLALA IgG1 (heavy chain) | see Table 48 |
| 307 | (12B3) VHCH1-Heavy chain-(28H1) VHCL (nucleotide sequence) | see Table 59 |
| 308 | (12B3) VHCH1-Heavy chain-(28H1) VHCL | see Table 59 |
| 309 | (25G7) VHCH1-Heavy chain-(28H1) VHCL (nucleotide sequence) | see Table 59 |
| 310 | (25G7) VHCH1-Heavy chain-(28H1) VHCL | see Table 59 |
| 311 | (11D5) VHCH1-Heavy chain-(28H1) VHCL (nucleotide sequence) | see Table 59 |
| 312 | (11D5) VHCH1-Heavy chain-(28H1) VHCL | see Table 59 |
| 313 | (12B3) VHCH1-heavy chain knob (nucleotide sequence) | see Table 61 |
| 314 | (12B3) VHCH1-heavy chain knob | see Table 61 |
| 315 | (25G7) VHCH1-heavy chain knob (nucleotide sequence) | see Table 61 |
| 316 | (25G7) VHCH1-heavy chain knob | see Table 61 |
| 317 | (25G7) VHCH1 Fc knob VH (4B9) (nucleotide sequence, heavy chain 1) | see Table 63 |
| 318 | (25G7) VHCH1 Fc hole VL (4B9) (nucleotide sequence, heavy chain 2) | see Table 63 |
| 319 | (25G7) VHCH1 Fc knob VH (4B9) (heavy chain 1) | see Table 63 |
| 320 | (25G7) VHCH1 Fc hole VL (4B9) (heavy chain 2) | see Table 63 |
| 321 | (11D5) VHCH1 Fc knob VH (4B9) (nucleotide sequence, heavy chain 1) | see Table 63 |
| 322 | (11D5) VHCH1 Fc hole VL (4B9) (nucleotide sequence, heavy chain 2) | see Table 63 |
| 323 | (11D5) VHCH1 Fc knob VH (4B9) (heavy chain 1) | see Table 63 |
| 324 | (11D5) VHCH1 Fc hole VL (4B9) (heavy chain 2) | see Table 63 |
| 325 | (12B3) VHCH1_VHCH1 Fc knob VH (4B9) (nucleotide sequence) | see Table 65 |
| 326 | (12B3) VHCH1_VHCH1 Fc hole VL (4B9) (nucleotide sequence) | see Table 65 |
| 327 | (12B3) VHCH1_VHCH1 Fc knob VH (4B9) | see Table 65 |
| 328 | (12B3) VHCH1_VHCH1 Fc hole VL (4B9) | see Table 65 |
| 329 | (25G7) VHCH1_VHCH1 Fc knob VH (4B9) (nucleotide sequence) | see Table 65 |
| 330 | (25G7) VHCH1_VHCH1 Fc hole VL (4B9) (nucleotide sequence) | see Table 65 |
| 331 | (25G7) VHCH1_VHCH1 Fc knob VH (4B9) | see Table 65 |
| 332 | (25G7) VHCH1_VHCH1 Fc hole VL (4B9) | see Table 65 |
| 333 | (11D5) VHCH1_VHCH1 Fc knob VH (4B9) (nucleotide sequence) | see Table 65 |
| 334 | (11D5) VHCH1_VHCH1 Fc hole VL (4B9) (nucleotide sequence) | see Table 65 |
| 335 | (11D5) VHCH1_VHCH1 Fc knob VH (4B9) | see Table 65 |
| 336 | (11D5) VHCH1_VHCH1 Fc hole VL (4B9) | see Table 65 |
| 337 | (9B11) VHCH1_VHCH1 Fc knob VH (4B9) (nucleotide sequence) | see Table 65 |
| 338 | (9B11) VHCH1_VHCH1 Fc hole VL (4B9) (nucleotide sequence) | see Table 65 |
| 339 | (9B11) VHCH1_VHCH1 Fc knob VH (4B9) | see Table 65 |
| 340 | (9B11) VHCH1_VHCH1 Fc hole VL (4B9) | see Table 65 |
| 341 | (12B3) VHCH1_VHCH1 Fc knob VL (4B9) (nucleotide sequence) | see Table 66 |
| 342 | (12B3) VHCH1_VHCH1 Fc hole VH (4B9) (nucleotide sequence) | see Table 66 |
| 343 | (12B3) VHCH1_VHCH1 Fc knob VL (4B9) | see Table 66 |
| 344 | (12B3) VHCH1_VHCH1 Fc hole VH (4B9) | see Table 66 |
| 345 | (25G7) VHCH1_VHCH1 Fc knob VL (4B9) (nucleotide sequence) | see Table 66 |
| 346 | (25G7) VHCH1_VHCH1 Fc hole VH (4B9) (nucleotide sequence) | see Table 66 |
| 347 | (25G7) VHCH1_VHCH1 Fc knob VL (4B9) | see Table 66 |
| 348 | (25G7) VHCH1_VHCH1 Fc hole VH (4B9) | see Table 66 |
| 349 | (11D5) VHCH1_VHCH1 Fc knob VL (4B9) (nucleotide sequence) | see Table 66 |
| 350 | (11D5) VHCH1_VHCH1 Fc hole VH (4B9) (nucleotide sequence) | see Table 66 |
| 351 | (11D5) VHCH1_VHCH1 Fc knob VL (4B9) | see Table 66 |
| 352 | (11D5) VHCH1_VHCH1 Fc hole VH (4B9) | see Table 66 |
| 353 | (9B11) VHCH1_VHCH1 Fc knob VL (4B9) (nucleotide sequence) | see Table 66 |
| 354 | (9B11) VHCH1_VHCH1 Fc hole VH (4B9) (nucleotide sequence) | see Table 66 |
| 355 | (9B11) VHCH1_VHCH1 Fc knob VL (4B9) | see Table 66 |
| 356 | (9B11) VHCH1_VHCH1 Fc hole VH (4B9) | see Table 66 |
| 357 | human GITR ECD | Q9Y5U5, aa 26-162, see Table 72 |
| 358 | cynomolgus GITR ECD | aa 20-156, see Table 72 |
| 359 | murine GITR ECD | 035714, aa 20-153, see Table 72 |
| 360 | Nucleotide sequence human GITR antigen Fc knob chain | see Table 73 |
| 361 | Nucleotide sequence cynomolgus GITR antigen Fc knob chain | see Table 73 |
| 362 | Nucleotide sequence murine GITR antigen Fc knob chain | see Table 73 |
| 363 | human GITR antigen Fc knob chain | see Table 73 |
| 364 | cynomolgus GITR antigen Fc knob chain | see Table 73 |
| 365 | murine GITR antigen Fc knob chain | see Table 73 |
| 366 | Fab light chain Vl3_19 template | see Table 75 |
| 367 | Fab heavy chain VH1_69 template | see Table 75 |
| 368 | nucleotide sequence of Fab light chain Vl3_19 template | see Table 75 |
| 369 | nucleotide sequence of Fab heavy chain VH1_69 template | see Table 75 |
| 370 | Complete pRJH52 Fab sequence | see Table 75 |
| 371 | GITR(8A06) CDR-H1 | SYAIS |
| 372 | GITR(8A06) CDR-H2 | GIIPIFGTANYAQKFQG |
| 373 | GITR(8A06) CDR-H3 | GYYAIDY |
| 374 | GITR(8A06) CDR-L1 | QGDSLRSYYAS |
| 375 | GITR(8A06) CDR-L2 | GKNNRPS |
| 376 | GITR(8A06) CDR-L3 | NSPQTSGSAV |
| 377 | GITR(6C8) CDR-H1 | TSGMGVG |
| 378 | GITR(6C8) CDR-H2 | HIWWDDDKYYQPSLKS |
| 379 | GITR(6C8) CDR-H3 | TRRYFPFAY |
| 380 | GITR(6C8) CDR-L1 | KASQNVGTNVA |
| 381 | GITR(6C8) CDR-L2 | SASYRYS |
| 382 | GITR(6C8) CDR-L3 | QQYNTDPLT |
| 383 | GITR(8A06) VH | QVQLVQSGAEVKKPGSSVKVSCKASGGTFSSYAISW VRQAPGQGLEWMGGIIPIFGTANYAQKFQGRVTITA DKSTSTAYMELSSLRSEDTAVYYCARGYYAIDYWG QGTTVTVSS |
| 384 | GITR(8A06) VL | SSELTQDPAVSVALGQTVRITCQGDSLRSYYASWYQ QKPGQAPVLVIYGKNNRPSGIPDRFSGSSSGNTASLTI TGAQAEDEADYYCNSPQTSGSAVFGGGTKLTVL |
| 385 | GITR(6C8) VH | QVTLRESGPALVKPTQTLTLTCTFSGFSLSTSGMGVG WIRQPPGKALEWLAHIWWDDDKYYQPSLKSRLTISK DTSKNQVVLTMTNMDPVDTATYYCARTRRYFPFAY WGQGTLVTVSS |
| 386 | GITR(6C8) VL | EIVMTQSPATLSVSPGERATLSCKASQNVGTNVAWY QQKPGQAPRLLIYSASYRYSGIPARFSGSGSGTEFTLT ISSLQSEDFAVYYCQQYNTDPLTFGGGTKVEIK |
| 387 | nucleotide sequence of GITR(8A06) VL | see Table 77 |
| 388 | nucleotide sequence of GITR(8A06) VH | see Table 77 |
| 389 | nucleotide sequence of GITR(6C8) VL | see Table 77 |
| 390 | nucleotide sequence of GITR(6C8) VH | see Table 77 |
| 391 | nucleotide sequence of 8A06 P329GLALA IgG1 (light chain) | see Table 78 |
| 392 | nucleotide sequence of 8A06 P329GLALA IgG1 (heavy chain) | see Table 78 |
| 393 | 8A06 P329GLALA IgG1 (light chain) | see Table 78 |
| 394 | 8A06 P329GLALA IgG1 (heavy chain) | see Table 78 |
| 395 | nucleotide sequence of 6C8 P329GLALA IgG1 (light chain) | see Table 78 |
| 396 | nucleotide sequence of 6C8 P329GLALA IgG1 (heavy chain) | see Table 78 |
| 397 | 6C8 P329GLALA IgG1 (light chain) | see Table 78 |
| 398 | 6C8 P329GLALA IgG1 (heavy chain) | see Table 78 |
| 399 | HC 1 (8A06) VHCH1_VHCH1 Fc knob VH (28H1) (nucleotide sequence) | see Table 83 |
| 400 | HC 2 (8A06) VHCH1_VHCH1 Fc hole VL (28H1) (nucleotide sequence) | see Table 83 |
| 401 | HC 1 (8A06) VHCH1_VHCH1 Fc knob VH (28H1) | see Table 83 |
| 402 | HC 2 (8A06) VHCH1_VHCH1 Fc hole VL (28H1) | see Table 83 |
| 403 | HC 1 (6C8) VHCH1_VHCH1 Fc knob VH (28H1) (nucleotide sequence) | see Table 83 |
| 404 | HC 2 (6C8) VHCH1_VHCH1 Fc hole VL (28H1) (nucleotide sequence) | see Table 83 |
| 405 | HC 1 (6C8) VHCH1_VHCH1 Fc knob VH (28H1) | see Table 83 |
| 406 | HC 2 (6C8) VHCH1_VHCH1 Fc hole VL (28H1) | see Table 83 |
| 407 | heavy chain (8A06) VHCH1_VHCH1Fc VHCL (28H1) (nucleotide sequence) | see Table 84 |
| 408 | (28H1) VLCH1- light chain 2 (nucleotide sequence) | see Table 84 |
| 409 | heavy chain (8A06) VHCH1_VHCH1Fc VHCL (28H1) | see Table 84 |
| 410 | (28H1) VLCH1- light chain 2 | see Table 84 |
| 411 | heavy chain (6C8) VHCH1_VHCH1Fc VHCL (28H1) (nucleotide sequence) | see Table 84 |
| 412 | heavy chain (6C8) VHCH1_VHCH1Fc VHCL (28H1) | see Table 84 |

All nucleotide sequences are presented without the respective stop codon sequences.

### EXAMPLES

The following are examples of methods and compositions of the invention. It is understood that various other embodiments may be practiced, given the general description provided above.

### Recombinant DNA techniques

Standard methods were used to manipulate DNA as described in Sambrook et al., Molecular cloning: A laboratory manual; Cold Spring Harbor Laboratory Press, Cold Spring Harbor, New York, 1989. The molecular biological reagents were used according to the manufacturer's instructions. General information regarding the nucleotide sequences of human immunoglobulin light and heavy chains is given in: Kabat, E.A. et al., (1991) Sequences of Proteins of Immunological Interest, Fifth Ed., NIH Publication No 91-3242.

### DNA sequencing

DNA sequences were determined by double strand sequencing.

### Gene synthesis

Desired gene segments were either generated by PCR using appropriate templates or were synthesized by Geneart AG (Regensburg, Germany) from synthetic oligonucleotides and PCR products by automated gene synthesis. In cases where no exact gene sequence was available, oligonucleotide primers were designed based on sequences from closest homologues and the genes were isolated by RT-PCR from RNA originating from the appropriate tissue. The gene segments flanked by singular restriction endonuclease cleavage sites were cloned into standard cloning / sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow sub-cloning into the respective expression vectors. All constructs were designed with a 5'-end DNA sequence coding for a leader peptide which targets proteins for secretion in eukaryotic cells.

### Protein purification

Proteins were purified from filtered cell culture supernatants referring to standard protocols. In brief, antibodies were applied to a Protein A Sepharose column (GE healthcare) and washed with PBS. Elution of antibodies was achieved at pH 2.8 followed by immediate neutralization of the sample. Aggregated protein was separated from monomeric antibodies by size exclusion chromatography (Superdex 200, GE Healthcare) in PBS or in 20 mM Histidine, 150 mM NaCl pH 6.0. Monomeric antibody fractions were pooled, concentrated (if required) using e.g., a MILLIPORE Amicon Ultra (30 MWCO) centrifugal concentrator, frozen and stored at -20°C or -80°C. Part of the samples were provided for subsequent protein analytics and analytical characterization e.g. by SDS-PAGE, size exclusion chromatography (SEC) or mass spectrometry.

### SDS-PAGE

The NuPAGE^{®} Pre-Cast gel system (Invitrogen) was used according to the manufacturer's instruction. In particular, 10% or 4-12% NuPAGE^{®} Novex^{®} Bis-TRIS Pre-Cast gels (pH 6.4) and a NuPAGE^{®} MES (reduced gels, with NuPAGE^{®} Antioxidant running buffer additive) or MOPS (non-reduced gels) running buffer was used.

### Analytical size exclusion chromatography

Size exclusion chromatography (SEC) for the determination of the aggregation and oligomeric state of antibodies was performed by HPLC chromatography. Briefly, Protein A purified antibodies were applied to a Tosoh TSKgel G3000SW column in 300 mM NaCl, 50 mM KH₂PO₄/K₂HPO₄, pH 7.5 on an Agilent HPLC 1100 system or to a Superdex 200 column (GE Healthcare) in 2 x PBS on a Dionex HPLC-System. The eluted protein was quantified by UV absorbance and integration of peak areas. BioRad Gel Filtration Standard 151-1901 served as a standard.

### Mass spectrometry

This section describes the characterization of the multispecific antibodies with VH/VL or CH/CL exchange (CrossMabs) with emphasis on their correct assembly. The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

### Example 1

### Generation of Ox40 antibodies and tool binders

### 1.1 Preparation, purification and characterization of antigens and screening tools for the generation of novel OX40 binders by Phage Display

DNA sequences encoding the ectodomains of human, mouse or cynomolgus OX40 (Table 1) were subcloned in frame with the human IgG1 heavy chain CH2 and CH3 domains on the knob (Merchant et al., 1998). An AcTEV protease cleavage site was introduced between an antigen ectodomain and the Fc of human IgG1. An Avi tag for directed biotinylation was introduced at the C-terminus of the antigen-Fc knob. Combination of the antigen-Fc knob chain containing the S354C/T366W mutations, with a Fc hole chain containing the Y349C/T366S/L368A/Y407V mutations allows generation of a heterodimer which includes a single copy of the OX40 ectodomain containing chain, thus creating a monomeric form of Fc-linked antigen (**Figure 1A**). **Table 1** shows the amino acid sequences of the various OX40 ectodomains. **Table 2** the cDNA and amino acid sequences of monomeric antigen Fc(kih) fusion molecules as depicted in Figure 1.

**Table 1: Amino acid numbering of antigen ectodomains (ECD) and their origin**

| SEQ ID NO: | Construct | Origin | ECD |
|---|---|---|---|
| 1 | **human OX40 ECD** | Synthetized according to P43489 | aa 29-214 |
| 93 | **cynomolgus OX40 ECD** | Isolated from cynomolgus blood | aa 29-214 |
| 94 | **murine OX40 ECD** | Synthetized according to P47741 | aa 10-211 |

**Table 2: cDNA and amino acid sequences of monomeric antigen Fc(kih) fusion molecules (produced by combination of one Fc hole chain with one antigen Fc knob chain)**

| SEQ ID NO: | Antigen | Sequence |
|---|---|---|
| 95 | Nucleotide sequence Fc hole chain | GACAAAACTCACACATGCCCACCGTGCCCAGCACCTGAA CTCCTGGGGGGACCGTCAGTCTTCCTCTTCCCCCCAAAAC CCAAGGACACCCTCATGATCTCCCGGACCCCTGAGGTCAC ATGCGTGGTGGTGGACGTGAGCCACGAAGACCCTGAGGT CAAGTTCAACTGGTACGTGGACGGCGTGGAGGTGCATAA TGCCAAGACAAAGCCGCGGGAGGAGCAGTACAACAGCAC GTACCGTGTGGTCAGCGTCCTCACCGTCCTGCACCAGGAC TGGCTGAATGGCAAGGAGTACAAGTGCAAGGTCTCCAAC AAAGCCCTCCCAGCCCCCATCGAGAAAACCATCTCCAAA GCCAAAGGGCAGCCCCGAGAACCACAGGTGTACACCCTG CCCCCATCCCGGGATGAGCTGACCAAGAACCAGGTCAGC |
| | | CTCTCGTGCGCAGTCAAAGGCTTCTATCCCAGCGACATCG CCGTGGAGTGGGAGAGCAATGGGCAGCCGGAGAACAACT ACAAGACCACGCCTCCCGTGCTGGACTCCGACGGCTCCTT CTTCCTCGTGAGCAAGCTCACCGTGGACAAGAGCAGGTG GCAGCAGGGGAACGTCTTCTCATGCTCCGTGATGCATGAG GCTCTGCACAACCACTACACGCAGAAGAGCCTCTCCCTGT CTCCGGGTAAA |
| 96 | Nucleotide sequence human OX40 antigen Fc knob chain | CTGCACTGCGTGGGCGACACCTACCCCAGCAACGACCGG TGCTGCCACGAGTGCAGACCCGGCAACGGCATGGTGTCC CGGTGCAGCCGGTCCCAGAACACCGTGTGCAGACCTTGC GGCCCTGGCTTCTACAACGACGTGGTGTCCAGCAAGCCCT GCAAGCCTTGTACCTGGTGCAACCTGCGGAGCGGCAGCG AGCGGAAGCAGCTGTGTACCGCCACCCAGGATACCGTGT GCCGGTGTAGAGCCGGCACCCAGCCCCTGGACAGCTACA AACCCGGCGTGGACTGCGCCCCTTGCCCTCCTGGCCACTT CAGCCCTGGCGACAACCAGGCCTGCAAGCCTTGGACCAA CTGCACCCTGGCCGGCAAGCACACCCTGCAGCCCGCCAG CAATAGCAGCGACGCCATCTGCGAGGACCGGGATCCTCC TGCCACCCAGCCTCAGGAAACCCAGGGCCCTCCCGCCAG ACCCATCACCGTGCAGCCTACAGAGGCCTGGCCCAGAAC CAGCCAGGGGCCTAGCACCAGACCCGTGGAAGTGCCTGG CGGCAGAGCCGTCGACGAACAGTTATATTTCAGGGCGG CTCACCCAAATCTGCAGACAAAACTCACACATGCCCACC GTGCCCAGCACCTGAACTCCTGGGGGGACCGTCAGTCTTC CTCTTCCCCCCAAAACCCAAGGACACCCTCATGATCTCCC GGACCCCTGAGGTCACATGCGTGGTGGTGGACGTGAGCC ACGAAGACCCTGAGGTCAAGTTCAACTGGTACGTGGACG GCGTGGAGGTGCATAATGCCAAGACAAAGCCGCGGGAGG AGCAGTACAACAGCACGTACCGTGTGGTCAGCGTCCTCA CCGTCCTGCACCAGGACTGGCTGAATGGCAAGGAGTACA AGTGCAAGGTCTCCAACAAAGCCCTCCCAGCCCCCATCG AGAAAACCATCTCCAAAGCCAAAGGGCAGCCCCGAGAAC CACAGGTGTACACCCTGCCCCCATGCCGGGATGAGCTGA CCAAGAACCAGGTCAGCCTGTGGTGCCTGGTCAAAGGCT TCTATCCCAGCGACATCGCCGTGGAGTGGGAGAGCAATG GGCAGCCGGAGAACAACTACAAGACCACGCCTCCCGTGC TGGACTCCGACGGCTCCTTCTTCCTCTACAGCAAGCTCAC CGTGGACAAGAGCAGGTGGCAGCAGGGGAACGTCTTCTC ATGCTCCGTGATGCATGAGGCTCTGCACAACCACTACACG CAGAAGAGCCTCTCCCTGTCTCCGGGTAAATCCGGAGGCC TGAACGACATCTTCGAGGCCCAGAAGATTGAATGGCACG AG |
| 97 | Nucleotide sequence cynomolgus OX40 antigen Fc knob chain | CTCCACTGTGTCGGGACACCTACCCCAGCAACGACCGGT GCTGTCAGGAGTGCAGGCCAGGCAACGGATGGTGAGCC GCTGCAACCGCTCCCAGAACACGGTGTGCCGTCCGTGCG GGCCCGGCTTCTACAACGACGTGGTCAGCGCCAAGCCCT GCAAGGCCTGCACATGGTGCAACCTCAGAAGTGGGAGTG AGCGGAAACAGCCGTGCACGGCCACACAGGACACAGTCT GCCGCTGCCGGGCGGGCACCCAGCCCCTGGACAGCTACA AGCCTGGAGTTGACTGTGCCCCCTGCCCTCCAGGGCACTT CTCCCCGGGCGACAACCAGGCCTGCAAGCCCTGGACCAA CTGCACCTTGGCCGGGAAGCACACCCTGCAGCCAGCCAG |
| | | CAATAGCTCGGACGCCATCTGTGAGGACAGGGACCCCCC ACCCACACAGCCCCAGGAGACCCAGGGCCCCCGGCCAG GCCCACCACTGTCCAGCCCACTGAAGCCTGGCCCAGAAC CTCACAGAGACCCTCCACCCGGCCCGTGGAGGTCCCCAG GGGCCCTGCGGTCGACGAACAGTTATATTTCAGGGCGGC TCACCCAAATCTGCAGACAAAACTCACACATGCCCACCGT GCCCAGCACCTGAACTCCTGGGGGGACCGTCAGTCTTCCT CTTCCCCCCAAAACCCAAGGACACCCTCATGATCTCCCGG ACCCCTGAGGTCACATGCGTGGTGGTGGACGTGAGCCAC GAAGACCCTGAGGTCAAGTTCAACTGGTACGTGGACGGC GTGGAGGTGCATAATGCCAAGACAAAGCCGCGGGAGGAG CAGTACAACAGCACGTACCGTGTGGTCAGCGTCCTCACCG TCCTGCACCAGGACTGGCTGAATGGCAAGGAGTACAAGT GCAAGGTCTCCAACAAAGCCCTCCCAGCCCCCATCGAGA AAACCATCTCCAAAGCCAAAGGGCAGCCCCGAGAACCAC AGGTGTACACCCTGCCCCCATGCCGGGATGAGCTGACCA AGAACCAGGTCAGCCTGTGGTGCCTGGTCAAAGGCTTCTA TCCCAGCGACATCGCCGTGGAGTGGGAGAGCAATGGGCA GCCGGAGAACAACTACAAGACCACGCCTCCCGTGCTGGA CTCCGACGGCTCCTTCTTCCTCTACAGCAAGCTCACCGTG GACAAGAGCAGGTGGCAGCAGGGGAACGTCTTCTCATGC TCCGTGATGCATGAGGCTCTGCACAACCACTACACGCAG AAGAGCCTCTCCCTGTCTCCGGGTAAATCCGGAGGCCTGA ACGACATCTTCGAGGCCCAGAAGATTGAATGGCACGAG |
| 98 | Nucleotide sequence murine OX40 antigen Fc knob chain | GTGACCGCCAGACGGCTGAACTGCGTGAAGCACACCTAC CCCAGCGGCCACAAGTGCTGCAGAGAGTGCCAGCCCGGC CACGGCATGGTGTCCAGATGCGACCACACACGGACACC CTGTGCCACCCTTGCGAGACAGGCTTCTACAACGAGGCCG TGAACTACGATACCTGCAAGCAGTGCACCCAGTGCAACC ACAGAAGCGGCAGCGAGCTGAAGCAGAACTGCACCCCCA CCCAGGATACCGTGTGCAGATGCAGACCCGGCACCCAGC CCAGACAGGACAGCGGCTACAAGCTGGGCGTGGACTGCG TGCCCTGCCCTCCTGGCCACTTCAGCCCCGGCAACAACCA GGCCTGCAAGCCCTGGACCAACTGCACCCTGAGCGGCAA GCAGACCAGACACCCCGCCAGCGACAGCCTGGATGCCGT GTGCGAGGACAGAAGCCTGCTGGCCACCCTGCTGTGGGA GACACAGCGGCCCACCTTCAGACCCACCACCGTGCAGAG CACCACCGTGTGGCCCAGAACCAGCGAGCTGCCCAGTCC TCCTACCCTCGTGACACCTGAGGGCCCCGTCGACGAACAG TTATATTTTCAGGGCGGCTCACCCAAATCTGCAGACAAAA CTCACACATGCCCACCGTGCCCAGCACCTGAACTCCTGGG GGGACCGTCAGTCTTCCTCTTCCCCCCAAAACCCAAGGAC ACCCTCATGATCTCCCGGACCCCTGAGGTCACATGCGTGG TGGTGGACGTGAGCCACGAAGACCCTGAGGTCAAGTTCA ACTGGTACGTGGACGGCGTGGAGGTGCATAATGCCAAGA CAAAGCCGCGGGAGGAGCAGTACAACAGCACGTACCGTG TGGTCAGCGTCCTCACCGTCCTGCACCAGGACTGGCTGAA TGGCAAGGAGTACAAGTGCAAGGTCTCCAACAAAGCCCT CCCAGCCCCCATCGAGAAAACCATCTCCAAAGCCAAAGG GCAGCCCCGAGAACCACAGGTGTACACCCTGCCCCCATG CCGGGATGAGCTGACCAAGAACCAGGTCAGCCTGTGGTG CCTGGTCAAAGGCTTCTATCCCAGCGACATCGCCGTGGAG |
| | | TGGGAGAGCAATGGGCAGCCGGAGAACAACTACAAGACC ACGCCTCCCGTGCTGGACTCCGACGGCTCCTTCTTCCTCT ACAGCAAGCTCACCGTGGACAAGAGCAGGTGGCAGCAGG GGAACGTCTTCTCATGCTCCGTGATGCATGAGGCTCTGCA CAACCACTACACGCAGAAGAGCCTCTCCCTGTCTCCGGGT AAATCCGGAGGCCTGAACGACATCTTCGAGGCCCAGAAG ATTGAATGGCACGAG |
| 99 | Fc hole chain | DKTHTCPPCPAPELLGGPSVFLFPPKPKDTLMISRTPEVTCVV VDVSHEDPEVKFNWYVDGVEVHNAKTKPREEQYNSTYRV VSVLTVLHQDWLNGKEYKCKVSNKALPAPIEKTISKAKGQP REPQVCTLPPSRDELTKNQVSLSCAVKGFYPSDIAVEWESNG QPENNYKTTPPVLDSDGSFFLVSKLTVDKSRWQQGNVFSCS VMHEALHNHYTQKSLSLSPGK |
| 100 | human OX40 antigen Fc knob chain | LHCVGDTYPSNDRCCHECRPGNGMVSRCSRSQNTVCRPCGP GFYNDVVSSKPCKPCTWCNLRSGSERKQLCTATQDTVCRCR AGTQPLDSYKPGVDCAPCPPGHFSPGDNQACKPWTNCTLA GKHTLQPASNSSDAICEDRDPPATQPQETQGPPARPITVQPTE AWPRTSQGPSTRPVEVPGGRAVDEQLYFQGGSPKSADKTHT CPPCPAPELLGGPSVFLFPPKPKDTLMISRTPEVTCVVVDVSH EDPEVKFNWYVDGVEVHNAKTKPREEQYNSTYRVVSVLTV LHQDWLNGKEYKCKVSNKALPAPIEKTISKAKGQPREPQVY TLPPCRDELTKNQVSLWCLVKGFYPSDIAVEWESNGQPENN YKTTPPVLDSDGSFFLYSKLTVDKSRWQQGNVFSCSVMHEA LHNHYTQKSLSLSPGKSGGLNDIFEAQKIEWHE |
| 101 | cynomolgus OX40 antigen Fc knob chain | LHCVGDTYPSNDRCCQECRPGNGMVSRCNRSQNTVCRPCG PGFYNDVVSAKPCKACTWCNLRSGSERKQPCTATQDTVCR CRAGTQPLDSYKPGVDCAPCPPGHFSPGDNQACKPWTNCTL AGKHTLQPASNSSDAICEDRDPPPTQPQETQGPPARPTTVQP TEAWPRTSQRPSTRPVEVPRGPAVDEQLYFQGGSPKSADKT HTCPPCPAPELLGGPSVFLFPPKPKDTLMISRTPEVTCVVVDV SHEDPEVKFNWYVDGVEVHNAKTKPREEQYNSTYRVVSVL TVLHQDWLNGKEYKCKVSNKALPAPIEKTISKAKGQPREPQ VYTLPPCRDELTKNQVSLWCLVKGFYPSDIAVEWESNGQPE NNYKTTPPVLDSDGSFFLYSKLTVDKSRWQQGNVFSCSVM HEALHNHYTQKSLSLSPGKSGGLNDIFEAQKIEWHE |
| 102 | murine OX40 antigen Fc knob chain | VTARRLNCVKHTYPSGHKCCRECQPGHGMVSRCDHTRDTL CHPCETGFYNEAVNYDTCKQCTQCNHRSGSELKQNCTPTQ DTVCRCRPGTQPRQDSGYKLGVDCVPCPPGHFSPGNNQACK PWTNCTLSGKQTRHPASDSLDAVCEDRSLLATLLWETQRPT FRPTTVQSTTVWPRTSELPSPPTLVTPEGPVDEQLYFQGGSP KSADKTHTCPPCPAPELLGGPSVFLFPPKPKDTLMISRTPEVT CVVVDVSHEDPEVKFNWYVDGVEVHNAKTKPREEQYNST YRVVSVLTVLHQDWLNGKEYKCKVSNKALPAPIEKTISKAK GQPREPQVYTLPPCRDELTKNQVSLWCLVKGFYPSDIAVEW ESNGQPENNYKTTPPVLDSDGSFFLYSKLTVDKSRWQQGNV FSCSVMHEALHNHYTQKSLSLSPGKSGGLNDIFEAQKIEWH E |

All OX40-Fc-fusion encoding sequences were cloned into a plasmid vector driving expression of the insert from an MPSV promoter and containing a synthetic polyA signal sequence located at the 3' end of the CDS. In addition, the vector contained an EBV OriP sequence for episomal maintenance of the plasmid.

For preparation of the biotinylated monomeric antigen/Fc fusion molecules, exponentially growing suspension HEK293 EBNA cells were co-transfected with three vectors encoding the two components of fusion protein (knob and hole chains) as well as BirA, an enzyme necessary for the biotinylation reaction. The corresponding vectors were used at a 2 : 1 : 0.05 ratio ("antigen ECD-AcTEV- Fc knob" : "Fc hole" : "BirA").

For protein production in 500 ml shake flasks, 400 million HEK293 EBNA cells were seeded 24 hours before transfection. For transfection cells were centrifuged for 5 minutes at 210 g, and supernatant was replaced by pre-warmed CD CHO medium. Expression vectors were resuspended in 20 mL of CD CHO medium containing 200 µg of vector DNA. After addition of 540 µL of polyethylenimine (PEI), the solution was vortexed for 15 seconds and incubated for 10 minutes at room temperature. Afterwards, cells were mixed with the DNA/PEI solution, transferred to a 500 mL shake flask and incubated for 3 hours at 37 °C in an incubator with a 5% CO₂ atmosphere. After the incubation, 160 mL of F17 medium was added and cells were cultured for 24 hours. One day after transfection, 1 mM valproic acid and 7% Feed were added to the culture. After 7 days of culturing, the cell supernatant was collected by spinning down cells for 15 min at 210 g. The solution was sterile filtered (0.22 µm filter), supplemented with sodium azide to a final concentration of 0.01 % (w/v), and kept at 4 °C.

Secreted proteins were purified from cell culture supernatants by affinity chromatography using Protein A, followed by size exclusion chromatography. For affinity chromatography, the supernatant was loaded on a HiTrap ProteinA HP column (CV = 5 mL, GE Healthcare) equilibrated with 40 mL 20 mM sodium phosphate, 20 mM sodium citrate pH 7.5. Unbound protein was removed by washing with at least 10 column volumes of a buffer containing 20 mM sodium phosphate, 20 mM sodium citrate and 0.5 M sodium chloride (pH 7.5). The bound protein was eluted using a linear pH-gradient of sodium chloride (from 0 to 500 mM) created over 20 column volumes of 20 mM sodium citrate, 0.01% (v/v) Tween-20, pH 3.0. The column was then washed with 10 column volumes of a solution containing 20 mM sodium citrate, 500 mM sodium chloride and 0.01% (v/v) Tween-20, pH 3.0.

The pH of the collected fractions was adjusted by adding 1/40 (v/v) of 2M Tris, pH8.0. The protein was concentrated and filtered prior to loading on a HiLoad Superdex 200 column (GE Healthcare) equilibrated with 2 mM MOPS, 150 mM sodium chloride, 0.02% (w/v) sodium azide solution of pH 7.4.

### 1.2 Selection of Ox40-specific 8H9, 20B7, 49B4, 1G4, CLC-563, CLC-564 and 17A9 antibodies from generic Fab and common light chain libraries

Anti-OX40 antibodies were selected from three different generic phage display libraries: DP88-4 (clones 20B7, 8H9 1G4 and 49B4), the common light chain library Vk3_20/VH3_23 (clones CLC-563 and CLC-564) and lambda-DP47 (clone 17A9).

The **DP88-4 library** was constructed on the basis of human germline genes using the V-domain pairing Vk1_5 (kappa light chain) and VH1_69 (heavy chain) comprising randomized sequence space in CDR3 of the light chain (L3, 3 different lengths) and CDR3 of the heavy chain (H3, 3 different lengths). Library generation was performed by assembly of 3 PCR-amplified fragments applying splicing by overlapping extension (SOE) PCR. Fragment 1 comprises the 5' end of the antibody gene including randomized L3, fragment 2 is a central constant fragment spanning from L3 to H3 whereas fragment 3 comprises randomized H3 and the 3' portion of the antibody gene. The following primer combinations were used to generate these library fragments for DP88-4 library: fragment 1 (forward primer LMB3 combined with reverse primers Vk1_5_L3r_S or Vk1_5_L3r_SY or Vk1_5_L3r_SPY), fragment 2 (forward primer RJH31 combined with reverse primer RJH32) and fragment 3 (forward primers DP88-v4-4 or DP88-v4-6 or DP88-v4-8 combined with reverse primer fdseqlong), respectively. PCR parameters for production of library fragments were 5 min initial denaturation at 94 °C, 25 cycles of 1 min 94 °C, 1 min 58 °C, 1 min 72 °C and terminal elongation for 10 min at 72 °C. For assembly PCR, using equimolar ratios of the gel-purified single fragments as template, parameters were 3 min initial denaturation at 94 °C and 5 cycles of 30 s 94 °C, 1 min 58 °C, 2 min 72 °C. At this stage, outer primers (LMB3 and fdseqlong) were added and additional 20 cycles were performed prior to a terminal elongation for 10 min at 72 °C. After assembly of sufficient amounts of full length randomized Fab constructs, they were digested NcoI / NheI and ligated into similarly treated acceptor phagemid vector. Purified ligations were used for ~60 transformations into electrocompetent E. coli TG1. Phagemid particles displaying the Fab library were rescued and purified by PEG/NaCl purification to be used for selections. These library construction steps were repeated three times to obtain a final library size of 4.4 × 109. Percentages of functional clones, as determined by C-terminal tag detection in dot blot, were 92.6% for the light chain and 93.7% for the heavy chain, respectively.

The common light chain library Vk3_20/VH3_23 was constructed on the basis of human germline genes using the V-domain pairing Vk3_20 (kappa light chain) and VH3_23 (heavy chain) comprising a constant non-randomized common light chain Vk3_20 and randomized sequence space in CDR3 of the heavy chain (H3, 3 different lengths). Library generation was performed by assembly of 2 PCR-amplified fragments applying splicing by overlapping extension (SOE) PCR. Fragment 1 is a constant fragment spanning from L3 to H3 whereas fragment 2 comprises randomized H3 and the 3' portion of the antibody gene. The following primer combinations were used to generate these library fragments for the Vk3_20/VH3_23 common light chain library: fragment 1 (forward primer MS64 combined with reverse primer DP47CDR3_ba (mod.)) and fragment 2 (forward primers DP47-v4-4, DP47-v4-6, DP47-v4-8 combined with reverse primer fdseqlong), respectively. PCR parameters for production of library fragments were 5 min initial denaturation at 94 °C, 25 cycles of 1 min 94 °C, 1 min 58 °C, 1 min 72 °C and terminal elongation for 10 min at 72 °C. For assembly PCR, using equimolar ratios of the gel-purified single fragments as template, parameters were 3 min initial denaturation at 94 °C and 5 cycles of 30 s 94 °C, 1 min 58 °C, 2 min 72 °C. At this stage, outer primers (MS64 and fdseqlong) were added and additional 18 cycles were performed prior to a terminal elongation for 10 min at 72 °C. After assembly of sufficient amounts of full length randomized VH constructs, they were digested MunI / NotI and ligated into similarly treated acceptor phagemid vector. Purified ligations were used for ~60 transformations into electrocompetent E. coli TG1. Phagemid particles displaying the Fab library were rescued and purified by PEG/NaCl purification to be used for selections. A final library size of 3.75 × 109 was obtained. Percentages of functional clones, as determined by C-terminal tag detection in dot blot, were 98.9% for the light chain and 89.5% for the heavy chain, respectively.

The **lambda-DP47** library was constructed on the basis of human germline genes using the following V-domain pairings: Vl3_19 lambda light chain with VH3_23 heavy chain. The library was randomized in CDR3 of the light chain (L3) and CDR3 of the heavy chain (H3) and was assembled from 3 fragments by "splicing by overlapping extension" (SOE) PCR. Fragment 1 comprises the the 5' end of the antibody gene including randomized L3, fragment 2 is a central constant fragment spanning from the end of L3 to the beginning of H3 whereas fragment 3 comprises randomized H3 and the 3' portion of the Fab fragment. The following primer combinations were used to generate library fragments for library: fragment 1 (LMB3 - Vl_3_19_L3r_V / Vl_3_19_L3r_HV / Vl_3_19_L3r_HLV), fragment 2 (RJH80 - DP47CDR3_ba (mod)) and fragment 3 (DP47-v4-4 / DP47-v4-6 / DP47-v4-8 - fdseqlong). PCR parameters for production of library fragments were 5 min initial denaturation at 94 °C, 25 cycles of 60 sec at 94 °C, 60 sec at 55 °C, 60 sec at 72 °C and terminal elongation for 10 min at 72 °C. For assembly PCR, using equimolar ratios of the 3 fragments as template, parameters were 3 min initial denaturation at 94 °C and 5 cycles of 60 sec at 94 °C, 60 sec at 55°C, 120 sec at 72 °C. At this stage, outer primers were added and additional 20 cycles were performed prior to a terminal elongation for 10 min at 72 °C. After assembly of sufficient amounts of full length randomized Fab fragments, they were digested with NcoI / NheI alongside with similarly treated acceptor phagemid vector. 15ug of Fab library insert were ligated with 13.3ug of phagemid vector. Purified ligations were used for 60 transformations resulting in 1.5 × 10⁹ transformants. Phagemid particles displaying the Fab library were rescued and purified by PEG/NaCl purification to be used for selections.

Human OX40 (CD134) as antigen for the phage display selections was transiently expressed as N-terminal monomeric Fc-fusion in HEK EBNA cells and *in vivo* site-specifically biotinylated via co-expression of BirA biotin ligase at the avi-tag recognition sequence located a the C-terminus of the Fc portion carrying the receptor chain (Fc knob chain).

Selection rounds (biopanning) were performed in solution according to the following pattern:
1. Pre-clearing of - 1012 phagemid particles on maxisorp plates coated with 10ug/ml of an unrelated human IgG to deplete the libraries of antibodies recognizing the Fc-portion of the antigen,
2. incubation of the non-binding phagemid particles with 100nM biotinylated human OX40 for 0.5 h in the presence of 100nM unrelated non-biotinylated Fc knob-into-hole construct for further depletion of Fc-binders in a total volume of 1 ml,
3. capture of biotinylated hu OX40 and attached specifically binding phage by transfer to 4 wells of a neutravidin pre-coated microtiter plate for 10 min (in rounds 1 & 3),
4. washing of respective wells using 5x PBS/Tween20 and 5x PBS,
5. elution of phage particles by addition of 250ul 100mM TEA (triethylamine) per well for 10 min and neutralization by addition of 500ul 1M Tris/HCl pH 7.4 to the pooled eluates from 4 wells,
6. post-clearing of neutralized eluates by incubation on neutravidin pre-coated microtiter plate with 100nM biotin-captured Fc knob-into-hole construct for final removal of Fc-binders,
7. re-infection of log-phase E. coli TG1 cells with the supernatant of eluted phage particles, infection with helperphage VCSM13, incubation on a shaker at 30°C over night and subsequent PEG/NaCl precipitation of phagemid particles to be used in the next selection round.

Selections were carried out over 3 or 4 rounds using constant antigen concentrations of 100 nM. In order to increase the likelihood for binders that are cross-reactive not only to cynomolgus OX40 but also murine OX40, in some selection rounds the murine target was used instead of the human OX40. In rounds 2 and 4, in order to avoid enrichment of binders to neutravidin, capture of antigen : phage complexes was performed by addition of 5.4 × 107 streptavidin-coated magnetic beads. Specific binders were identified by ELISA as follows: 100ul of 25nM biotinylated human OX40 and 10ug/ml of human IgG were coated on neutravidin plates and maxisorp plates, respectively. Fab-containing bacterial supernatants were added and binding Fabs were detected via their Flag-tags using an anti-Flag/HRP secondary antibody. Clones exhibiting signals on human OX40 and being negative on human IgG were short-listed for further analyses and were also tested in a similar fashion against cynomolgus and murine OX40. They were bacterially expressed in a 0.5 liter culture volume, affinity purified and further characterized by SPR-analysis using BioRad's ProteOn XPR36 biosensor.

**Table 3** shows the sequence of generic phage-displayed antibody library (DP88-4), **Table 4** provides cDNA and amino acid sequences of library DP88-4 germline template and **Table 5** shows the Primer sequences used for generation of DP88-4 germline template.

**Table 3: Sequence of generic phage-displayed antibody library (DP88-4)**

| SEQ ID NO: | Description | Sequence |
|---|---|---|
| 103 | nucleotide sequence of | TGAAATACCTATTGCCTACGGCAGCCGCTGGATTGTTATTACTCG CGGCCCAGCCGGCCATGGCCGACATCCAGATGACCCAGTCTCCT TCCACCCTGTCTGCATCTGTAGGAGACCGTGTCACCATCACTTGC CGTGCCAGTCAGAGTATTAGTAGCTGGTTGGCCTGGTATCAGCA GAAACCAGGGAAAGCCCCTAAGCTCCTGATCTATGATGCCTCCA GTTTGGAAAGTGGGGTCCCATCACGTTTCAGCGGCAGTGGATCC GGGACAGAATTCACTCTCACCATCAGCAGCTTGCAGCCTGATGA TTTTGCAACTTATTACTGCCAACAGTATAATAGTTATTCTACGTT TGGCCAGGGCACCAAAGTCGAGATCAAGCGTACGGTGGCTGCA CCATCTGTCTTCATCTTCCCGCCATCTGATGAGCAGTTGAAATCT GGAACTGCCTCTGTTGTGTGCCTGCTGAATAACTTCTATCCCAGA GAGGCCAAAGTACAGTGGAAGGTGGATAACGCCCTCCAATCGG GTAACTCCCAGGAGAGTGTCACAGAGCAGGACAGCAAGGACAG CACCTACAGCCTCAGCAGCACCCTGACGCTGAGCAAAGCAGACT ACGAGAAACACAAAGTCTACGCCTGCGAAGTCACCCATCAGGG CCTGAGCTCGCCCGTCACAAAGAGCTTCAACAGGGGAGAGTGTG GAGCCGCAGAACAAAAACTCATCTCAGAAGAGGATCTGAATGG AGCCGCAGACTACAAGGACGACGACGACAAGGGTGCCGCATAA TAAGGCGCGCCAATTCTATTTCAAGGAGACAGTCATATGAAATA CCTGCTGCCGACCGCTGCTGCTGGTCTGCTGCTCCTCGCTGCCCA GCCGGCGATGGCCCAGGTGCAATTGGTGCAGTCTGGGGCTGAGG TGAAGAAGCCTGGGTCCTCGGTGAAGGTCTCCTGCAAGGCCTCC GGAGGCACATTCAGCAGCTACGCTATAAGCTGGGTGCGACAGGC CCCTGGACAAGGGCTCGAGTGGATGGGAGGGATCATCCCTATCT TTGGTACAGCAAACTACGCACAGAAGTTCCAGGGCAGGGTCACC ATTACTGCAGACAAATCCACGAGCACAGCCTACATGGAGCTGAG CAGCCTGAGATCTGAGGACACCGCCGTGTATTACTGTGCGAGAC TATCCCCAGGCGGTTACTATGTTATGGATGCCTGGGGCCAAGGG ACCACCGTGACCGTCTCCTCAGCTAGCACCAAAGGCCCATCGGT CTTCCCCCTGGCACCCTCCTCCAAGAGCACCTCTGGGGGCACAG CGGCCCTGGGCTGCCTGGTCAAGGACTACTTCCCCGAACCGGTG ACGGTGTCGTGGAACTCAGGCGCCCTGACCAGCGGCGTGCACAC CTTCCCGGCTGTCCTACAGTCCTCAGGACTCTACTCCCTCAGCAG CGTGGTGACCGTGCCCTCCAGCAGCTTGGGCACCCAGACCTACA TCTGCAACGTGAATCACAAGCCCAGCAACACCAAAGTGGACAA GAAAGTTGAGCCCAAATCTTGTGACGCGGCCGCAAGCACTAGTG CCCATCACCATCACCATCACGCCGCGGCA |
| | pRJH33 library template DP88-4 library; complete Fab coding region comprising PelB leader sequence + Vk1_5 kappa V-domain + CL constant domain for light chain and PelB + VH1_69 V-domain + CH1 constant domain for heavy chain including tags | |

**Table 4: cDNA and amino acid sequences of library DP88-4 germline template**

| SEQ ID NO: | Description | Sequence |
|---|---|---|
| 104 | nucleotide sequence of Fab light chain Vk1_5 | GACATCCAGATGACCCAGTCTCCTTCCACCCTGTCTGC ATCTGTAGGAGACCGTGTCACCATCACTTGCCGTGCCA GTCAGAGTATTAGTAGCTGGTTGGCCTGGTATCAGCAG AAACCAGGGAAAGCCCCTAAGCTCCTGATCTATGATGC CTCCAGTTTGGAAAGTGGGGTCCCATCACGTTTCAGCG GCAGTGGATCCGGGACAGAATTCACTCTCACCATCAGC AGCTTGCAGCCTGATGATTTTGCAACTTATTACTGCCA ACAGTATAATAGTTATTCTACGTTTGGCCAGGGCACCA AAGTCGAGATCAAGCGTACGGTGGCTGCACCATCTGTC TTCATCTTCCCGCCATCTGATGAGCAGTTGAAATCTGG AACTGCCTCTGTTGTGTGCCTGCTGAATAACTTCTATCC CAGAGAGGCCAAAGTACAGTGGAAGGTGGATAACGCC CTCCAATCGGGTAACTCCCAGGAGAGTGTCACAGAGC AGGACAGCAAGGACAGCACCTACAGCCTCAGCAGCAC CCTGACGCTGAGCAAAGCAGACTACGAGAAACACAAA GTCTACGCCTGCGAAGTCACCCATCAGGGCCTGAGCTC GCCCGTCACAAAGAGCTTCAACAGGGGAGAGTGTGGA GCCGCAGAACAAAAACTCATCTCAGAAGAGGATCTGA ATGGAGCCGCAGACTACAAGGACGACGACGACAAGGG TGCCGCA |
| 105 | Fab light chain Vk1_5 | DIQMTQSPSTLSASVGDRVTITCRASQSISSWLAWYQQKP GKAPKLLIYDASSLESGVPSRFSGSGSGTEFTLTISSLQPDD FATYYCQQYNSYSTFGQGTKVEIKRTVAAPSVFIFPPSDE QLKSGTASVVCLLNNFYPREAKVQWKVDNALQSGNSQE SVTEQDSKDSTYSLSSTLTLSKADYEKHKVYACEVTHQG LSSPVTKSFNRGECGAAEQKLISEEDLNGAADYKDDDDK GAA |
| 106 | nucleotide sequence of Fab heavy chain VH1_69 | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGACTATCC CCAGGCGGTTACTATGTTATGGATGCCTGGGGCCAAGG GACCACCGTGACCGTCTCCTCAGCTAGCACCAAAGGCC CATCGGTCTTCCCCCTGGCACCCTCCTCCAAGAGCACC TCTGGGGGCACAGCGGCCCTGGGCTGCCTGGTCAAGG ACTACTTCCCCGAACCGGTGACGGTGTCGTGGAACTCA GGCGCCCTGACCAGCGGCGTGCACACCTTCCCGGCTGT CCTACAGTCCTCAGGACTCTACTCCCTCAGCAGCGTGG TGACCGTGCCCTCCAGCAGCTTGGGCACCCAGACCTAC ATCTGCAACGTGAATCACAAGCCCAGCAACACCAAAG TGGACAAGAAAGTTGAGCCCAAATCTTGTGACGCGGC CGCAAGCACTAGTGCCCATCACCATCACCATCACGCCG |
| | | CGGCA |
| 107 | Fab heavy chain VH1_69 | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARLSPGGYYVMDAWGQGTTVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDAAASTSAHHHH HHAAA |

**Table 5: Primer sequences used for generation of DP88-4 library**

| **SEQ ID NO:** | **Primer name** | **Primer sequence 5' - 3'** |
|---|---|---|
| 108 | LMB3 | CAGGAAACAGCTATGACCATGATTAC |
| 109 | Vk1_5_L3r_S | CTCGACTTTGGTGCCCTGGCCAAACGTSBAATA CGAATTATACTGTTGGCAGTAATAAGTTGCAAAATCAT |
| | | underlined: 60% original base and 40% randomization as M. |
| | | bolded and italic: 60% original base and 40% randomization as N |
| 110 | Vk1_5_L3r_SY | CTCGACTTTGGTGCCCTGGCCAAACGTMHRSGRATACGA ATTATACTGTTGGCAGTAATAAGTTGCAAAATCAT |
| | | underlined: 60% original base and 40% randomization as M. |
| | | bolded and italic: 60% original base and 40% randomization as N |
| 111 | Vk1_5_L3r_SPY | CTCGACTTTGGTGCCCTGGCCAAACGTMHHMSSSGRATA CGAATTATACTGTTGGCAGTAATAAGTTGCAAAATCAT |
| | | underlined: 60% original base and 40% randomization as M. |
| | | bolded and italic: 60% original base and 40% randomization as N |
| 112 | RJH31 | ACGTTTGGCCAGGGCACCAAAGTCGAG |
| 113 | RJH32 | TCTCGCACAGTAATACACGGCGGTGTCC |
| 114 | DP88-v4-4 | GGACACCGCCGTGTATTACTGTGCGAGA-1-2-2-3-4-GAC- TAC-TGGGGCCAAGGGACCACCGTGACCGTCTCC |
| | | 1: G/D = 20%, E/V/S = 10%, A/P/R/L/T/Y=5%; 2: G/Y/S=15%, A/D/T/R/P/L/V/N/W/F/I/E = 4,6%; 3: G/A/Y = 20%, P/W/S/D/T = 8%; 4: F = 46%, L/M = 15%, G/I/Y = 8%. |
| 115 | DP88-v4-6 | GGACACCGCCGTGTATTACTGTGCGAGA-1-2-2-2-2-3-4- GAC-TAC-TGGGGCCAAGGGACCACCGTGACCGTCTCC |
| | | 1: G/D = 20%, E/V/S = 10%, A/P/R/L/T/Y=5%; 2: G/Y/S=15%, A/D/T/R/P/L/V/N/W/F/I/E = 4,6%; 3: G/A/Y = 20%, P/W/S/D/T = 8%; 4: F = 46%, L/M = 15%, G/I/Y = 8%. |
| 116 | DP88-v4-8 | GGACACCGCCGTGTATTACTGTGCGAGA-1-2-2-2-2-2-2-3- 4-GAC-TAC-TGGGGCCAAGGGACCACCGTGACCGTCTCC |
| | | 1: G/D = 20%, E/V/S = 10%, A/P/R/L/T/Y=5%; 2: G/Y/S=15%, A/D/T/R/P/L/V/N/W/F/I/E = 4,6%; 3: G/A/Y = 20%, P/W/S/D/T = 8%; 4: F = 46%, L/M = 15%, G/I/Y = 8%. |
| 117 | fdseqlong | GACGTTAGTAAATGAATTTTCTGTATGAGG |

**Table 6** shows the sequence of generic phage-displayed antibody common light chain library (Vk3_20/VH3_23). **Table 7** provides cDNA and amino acid sequences of common light chain library (Vk3_20/VH3_23) germline template and **Table 8** shows the Primer sequences used for generation of common light chain library (Vk3_20/VH3_23).

**Table 6: Sequence of generic phage- displayed antibody common light chain library (Vk3_20/VH3_23) template used for PCR**

| SEQ ID NO: | Description | Sequence |
|---|---|---|
| 118 | pRJH110 library template of common light chain library **Vk3_20/V H3_23**; complete Fab coding region comprising PelB leader sequence + Vk3_20 kappa V-domain + CL constant domain for light chain and PelB + VH3_23 V-domain + CH1 constant domain for heavy chain including tags | ATGAAATACCTATTGCCTACGGCAGCCGCTGGATTGTTATTACTC GCGGCCCAGCCGGCCATGGCCGAAATCGTGTTAACGCAGTCTCC AGGCACCCTGTCTTTGTCTCCAGGGGAAAGAGCCACCCTCTCTT GCAGGGCCAGTCAGAGTGTTAGCAGCAGCTACTTAGCCTGGTAC CAGCAGAAACCTGGCCAGGCTCCCAGGCTCCTCATCTATGGAGC ATCCAGCAGGGCCACTGGCATCCCAGACAGGTTCAGTGGCAGTG GATCCGGGACAGACTTCACTCTCACCATCAGCAGACTGGAGCCT GAAGATTTTGCAGTGTATTACTGTCAGCAGTATGGTAGCTCACC GCTGACGTTCGGCCAGGGGACCAAAGTGGAAATCAAACGTACG GTGGCTGCACCATCTGTCTTCATCTTCCCGCCATCTGATGAGCAG TTGAAATCTGGAACTGCCTCTGTTGTGTGCCTGCTGAATAACTTC TATCCCAGAGAGGCCAAAGTACAGTGGAAGGTGGATAACGCCC TCCAATCGGGTAACTCCCAGGAGAGTGTCACAGAGCAGGACAG CAAGGACAGCACCTACAGCCTCAGCAGCACCCTGACGCTGAGC AAAGCAGACTACGAGAAACACAAAGTCTACGCCTGCGAAGTCA CCCATCAGGGCCTGAGCTCGCCCGTCACAAAGAGCTTCAACAGG GGAGAGTGTGGAGCCGCACATCACCATCACCATCACGGAGCCG CAGACTACAAGGACGACGACGACAAGGGTGCCGCATAATAAGG CGCGCCAATTCTATTTCAAGGAGACAGTCATATGAAATACCTGC TGCCGACCGCTGCTGCTGGTCTGCTGCTCCTCGCTGCCCAGCCGG CGATGGCCGAGGTGCAATTGCTGGAGTCTGGGGGAGGCTTGGTA CAGCCTGGGGGGTCCCTGAGACTCTCCTGTGCAGCCTCCGGATT CACCTTTAGCAGTTATGCCATGAGCTGGGTCCGCCAGGCTCCAG GGAAGGGGCTGGAGTGGGTCTCAGCTATTAGTGGTAGTGGTGGT AGCACATACTACGCAGACTCCGTGAAGGGCCGGTTCACCATCTC CAGAGACAATTCCAAGAACACGCTGTATCTGCAGATGAACAGCC TGAGAGCCGAGGACACGGCCGTATATTACTGTGCGAAACCGTTT CCGTATTTTGACTACTGGGGCCAAGGAACCCTGGTCACCGTCTC GAGTGCTAGCACCAAAGGCCCATCGGTCTTCCCCCTGGCACCCT CCTCCAAGAGCACCTCTGGGGGCACAGCGGCCCTGGGCTGCCTG GTCAAGGACTACTTCCCCGAACCGGTGACGGTGTCGTGGAACTC AGGCGCCCTGACCAGCGGCGTGCACACCTTCCCGGCTGTCCTAC AGTCCTCAGGACTCTACTCCCTCAGCAGCGTGGTGACCGTGCCC TCCAGCAGCTTGGGCACCCAGACCTACATCTGCAACGTGAATCA CAAGCCCAGCAACACCAAAGTGGACAAGAAAGTTGAGCCCAAA TCTTGTGACGCGGCCGCAGAACAAAAACTCATCTCAGAAGAGGA TCTGAATGCCGCGGCA |

**Table 7: cDNA and amino acid sequences of common light chain library (Vk3_20NH3_23) germline template**

| SEQ ID NO: | Description | Sequence |
|---|---|---|
| 119 | nucleotide sequence of Fab light chain Vk3_20 | GAAATCGTGTTAACGCAGTCTCCAGGCACCCTGTCTTTGT CTCCAGGGGAAAGAGCCACCCTCTCTTGCAGGGCCAGTC AGAGTGTTAGCAGCAGCTACTTAGCCTGGTACCAGCAGA AACCTGGCCAGGCTCCCAGGCTCCTCATCTATGGAGCATC CAGCAGGGCCACTGGCATCCCAGACAGGTTCAGTGGCAG TGGATCCGGGACAGACTTCACTCTCACCATCAGCAGACTG GAGCCTGAAGATTTTGCAGTGTATTACTGTCAGCAGTATG GTAGCTCACCGCTGACGTTCGGCCAGGGGACCAAAGTGG AAATCAAACGTACGGTGGCTGCACCATCTGTCTTCATCTT CCCGCCATCTGATGAGCAGTTGAAATCTGGAACTGCCTCT GTTGTGTGCCTGCTGAATAACTTCTATCCCAGAGAGGCCA AAGTACAGTGGAAGGTGGATAACGCCCTCCAATCGGGTA ACTCCCAGGAGAGTGTCACAGAGCAGGACAGCAAGGACA GCACCTACAGCCTCAGCAGCACCCTGACGCTGAGCAAAG CAGACTACGAGAAACACAAAGTCTACGCCTGCGAAGTCA CCCATCAGGGCCTGAGCTCGCCCGTCACAAAGAGCTTCA ACAGGGGAGAGTGTGGAGCCGCACATCACCATCACCATC ACGGAGCCGCAGACTACAAGGACGACGACGACAAGGGT GCCGCA |
| 120 | Fab light chain Vk3_20 | EIVLTQSPGTLSLSPGERATLSCRASQSVSSSYLAWYQQKPG QAPRLLIYGASSRATGIPDRFSGSGSGTDFTLTISRLEPEDFAV YYCQQYGSSPLTFGQGTKVEIKRTVAAPSVFIFPPSDEQLKS GTASVVCLLNNFYPREAKVQWKVDNALQSGNSQESVTEQD SKDSTYSLSSTLTLSKADYEKHKVYACEVTHQGLSSPVTKSF NRGECGAAHHHHHHGAADYKDDDDKGAA |
| 121 | nucleotide sequence of Fab heavy chain VH3_23 | GAGGTGCAATTGCTGGAGTCTGGGGGAGGCTTGGTACAG CCTGGGGGGTCCCTGAGACTCTCCTGTGCAGCCTCCGGAT TCACCTTTAGCAGTTATGCCATGAGCTGGGTCCGCCAGGC TCCAGGGAAGGGGCTGGAGTGGGTCTCAGCTATTAGTGG TAGTGGTGGTAGCACATACTACGCAGACTCCGTGAAGGG CCGGTTCACCATCTCCAGAGACAATTCCAAGAACACGCTG TATCTGCAGATGAACAGCCTGAGAGCCGAGGACACGGCC GTATATTACTGTGCGAAACCGTTTCCGTATTTTGACTACT GGGGCCAAGGAACCCTGGTCACCGTCTCGAGTGCTAGCA CCAAAGGCCCATCGGTCTTCCCCCTGGCACCCTCCTCCAA GAGCACCTCTGGGGGCACAGCGGCCCTGGGCTGCCTGGT CAAGGACTACTTCCCCGAACCGGTGACGGTGTCGTGGAA CTCAGGCGCCCTGACCAGCGGCGTGCACACCTTCCCGGCT GTCCTACAGTCCTCAGGACTCTACTCCCTCAGCAGCGTGG TGACCGTGCCCTCCAGCAGCTTGGGCACCCAGACCTACAT CTGCAACGTGAATCACAAGCCCAGCAACACCAAAGTGGA CAAGAAAGTTGAGCCCAAATCTTGTGACGCGGCCGCAGA ACAAAAACTCATCTCAGAAGAGGATCTGAATGCCGCGGC A |
| 122 | Fab heavy chain VH3_23 (DP47) | EVQLLESGGGLVQPGGSLRLSCAASGFTFSSYAMSWVRQAP GKGLEWVSAISGSGGSTYYADSVKGRFTISRDNSKNTLYLQ MNSLRAEDTAVYYCAKPFPYFDYWGQGTLVTVSSASTKGP SVFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDAAAEQKLISEEDLNAAA |

**Table 8: Primer sequences used for generation of DP88-4 library**

| **SEQ ID NO:** | **Primer name** | **Primer sequence 5' - 3'** |
|---|---|---|
| 123 | MS64 | ACGTTCGGCCAGGGGACCAAAGTGG |
| 124 | DP47CDR3_ba (mod.) | CGCACAGTAATATACGGCCGTGTCC |
| 125 | DP47-v4-4 | CGAGGACACGGCCGTATATTACTGTGCG-5-1–2-2-3-4-GAC- TAC-TGGGGCCAAGGAACCCTGGTCACCGTCTCG |
| 126 | DP47-v4-6 | CGAGGACACGGCCGTATATTACTGTGCG-5-1–2-2-2-2-3-4- GAC-TAC- TGGGGCCAAGGAACCCTGGTCACCGTCTCG |
| 127 | DP47-v4-8 | CGAGGACACGGCCGTATATTACTGTGCG-5-1–2-2-2-2-2-2-3-4- GAC-TAC- TGGGGCCAAGGAACCCTGGTCACCGTCTCG |
| 128 | fdseqlong | GACGTTAGTAAATGAATTTTCTGTATGAGG |

| | | |
|---|---|---|
| 1: G/D = 20%, E/V/S = 10%, A/P/R/L/T/Y=5%; 2: G/Y/S=15%, A/D/T/R/P/L/V/N/W/F/I/E = 4,6%; 3: G/A/Y = 20%, P/W/S/D/T = 8%; 4: F = 46%, L/M = 15%, G/I/Y = 8%; 5: K=70%, R =30%. | | |

**Table 9** shows the sequence of generic phage-displayed lambda-DP47 library (V13_19/VH3_23) template used for PCRs. **Table 10** provides cDNA and amino acid sequences of lambda-DP47 library (V13_19/VH3_23) germline template and **Table 11** shows the Primer sequences used for generation of lambda-DP47 library (Vl3_19/VH3_23).

**Table 9: Sequence of generic phage- displayed lambda-DP47 library (V13_19/VH3_23) template used for PCRs**

| SEQ ID NO: | Description | Sequence |
|---|---|---|
| 129 | pRJH53 library template of **lambda-DP47 library Vl3_19/VH 3_23;** complete Fab coding region comprising PelB leader sequence + V13_19 lambda V-domain + CL constant domain for | ATGAAATACCTATTGCCTACGGCAGCCGCTGGATTGTTATTACTC GCGGCCCAGCCGGCCATGGCCTCGTCTGAGCTGACTCAGGACCC TGCTGTGTCTGTGGCCTTGGGACAGACAGTCAGGATCACATGCC AAGGAGACAGCCTCAGAAGTTATTATGCAAGCTGGTACCAGCAG AAGCCAGGACAGGCCCCTGTACTTGTCATCTATGGTAAAAACAA CCGGCCCTCAGGGATCCCAGACCGATTCTCTGGCTCCAGCTCAG GAAACACAGCTTCCTTGACCATCACTGGGGCTCAGGCGGAAGAT GAGGCTGACTATTACTGTAACTCCCGTGATAGTAGCGGTAATCA TGTGGTATTCGGCGGAGGGACCAAGCTGACCGTCCTAGGACAAC CCAAGGCTGCCCCCAGCGTGACCCTGTTCCCCCCCAGCAGCGAG GAATTGCAGGCCAACAAGGCCACCCTGGTCTGCCTGATCAGCGA CTTCTACCCAGGCGCCGTGACCGTGGCCTGGAAGGCCGACAGCA GCCCCGTGAAGGCCGGCGTGGAGACCACCACCCCCAGCAAGCA GAGCAACAACAAGTACGCCGCCAGCAGCTACCTGAGCCTGACC CCCGAGCAGTGGAAGAGCCACAGGTCCTACAGCTGCCAGGTGA CCCACGAGGGCAGCACCGTGGAGAAAACCGTGGCCCCCACCGA GTGCAGCGGAGCCGCAGAACAAAAACTCATCTCAGAAGAGGAT CTGAATGGAGCCGCAGACTACAAGGACGACGACGACAAGGGTG CCGCATAATAAGGCGCGCCAATTCTATTTCAAGGAGACAGTCAT |
| | light chain and PelB + VH3_23 V-domain + CH1 constant domain for heavy chain including tags | ATGAAATACCTGCTGCCGACCGCTGCTGCTGGTCTGCTGCTCCTC GCTGCCCAGCCGGCGATGGCCGAGGTGCAATTGCTGGAGTCTGG GGGAGGCTTGGTACAGCCTGGGGGGTCCCTGAGACTCTCCTGTG CAGCCTCCGGATTCACCTTTAGCAGTTATGCCATGAGCTGGGTCC GCCAGGCTCCAGGGAAGGGGCTGGAGTGGGTCTCAGCTATTAGT GGTAGTGGTGGTAGCACATACTACGCAGACTCCGTGAAGGGCCG GTTCACCATCTCCAGAGACAATTCCAAGAACACGCTGTATCTGC AGATGAACAGCCTGAGAGCCGAGGACACGGCCGTATATTACTGT GCGAAACCGTTTCCGTATTTTGACTACTGGGGCCAAGGAACCCT GGTCACCGTCTCGAGTGCTAGCACCAAAGGCCCATCGGTCTTCC CCCTGGCACCCTCCTCCAAGAGCACCTCTGGGGGCACAGCGGCC CTGGGCTGCCTGGTCAAGGACTACTTCCCCGAACCGGTGACGGT GTCGTGGAACTCAGGCGCCCTGACCAGCGGCGTGCACACCTTCC CGGCTGTCCTACAGTCCTCAGGACTCTACTCCCTCAGCAGCGTG GTGACCGTGCCCTCCAGCAGCTTGGGCACCCAGACCTACATCTG CAACGTGAATCACAAGCCCAGCAACACCAAAGTGGACAAGAAA GTTGAGCCCAAATCTTGTGACGCGGCCGCAAGCACTAGTGCCCA TCACCATCACCATCACGCCGCGGCA |

**Table 10: cDNA and amino acid sequences of lambda-DP47 library (Vl3_19/VH3_23) germline template**

| SEQ ID NO: | Description | Sequence |
|---|---|---|
| 130 | nucleotide sequence of Fab light chain V13_19 | TCGTCTGAGCTGACTCAGGACCCTGCTGTGTCTGTGGCCT TGGGACAGACAGTCAGGATCACATGCCAAGGAGACAGCC TCAGAAGTTATTATGCAAGCTGGTACCAGCAGAAGCCAG GACAGGCCCCTGTACTTGTCATCTATGGTAAAAACAACCG GCCCTCAGGGATCCCAGACCGATTCTCTGGCTCCAGCTCA GGAAACACAGCTTCCTTGACCATCACTGGGGCTCAGGCG GAAGATGAGGCTGACTATTACTGTAACTCCCGTGATAGTA GCGGTAATCATGTGGTATTCGGCGGAGGGACCAAGCTGA CCGTCCTAGGACAACCCAAGGCTGCCCCCAGCGTGACCCT GTTCCCCCCCAGCAGCGAGGAATTGCAGGCCAACAAGGC CACCCTGGTCTGCCTGATCAGCGACTTCTACCCAGGCGCC GTGACCGTGGCCTGGAAGGCCGACAGCAGCCCCGTGAAG GCCGGCGTGGAGACCACCACCCCCAGCAAGCAGAGCAAC AACAAGTACGCCGCCAGCAGCTACCTGAGCCTGACCCCC GAGCAGTGGAAGAGCCACAGGTCCTACAGCTGCCAGGTG ACCCACGAGGGCAGCACCGTGGAGAAAACCGTGGCCCCC ACCGAGTGCAGCGGAGCCGCAGAACAAAAACTCATCTCA GAAGAGGATCTGAATGGAGCCGCAGACTACAAGGACGAC GACGACAAGGGTGCCGCA |
| 131 | Fab light chain V13_19 | SSELTQDPAVSVALGQTVRITCQGDSLRSYYASWYQQKPGQ APVLVIYGKNNRPSGIPDRFSGSSSGNTASLTITGAQAEDEAD YYCNSRDSSGNHVVFGGGTKLTVLGQPKAAPSVTLFPPSSEE LQANKATLVCLISDFYPGAVTVAWKADSSPVKAGVETTTPS KQSNNKYAASSYLSLTPEQWKSHRSYSCQVTHEGSTVEKTV APTECSGAAEQKLISEEDLNGAADYKDDDDKGAA |
| 121 | nucleotide sequence of Fab heavy chain VH3_23 | see Table 7 |
| 122 | Fab heavy chain VH3_23 (DP47) | see Table 7 |

**Table 11: Primer sequences used for generation of lambda-DP47 library (V13_19/VH3_23)**

| **SEQ ID NO:** | **Primer name** | **Primer sequence 5' - 3'** |
|---|---|---|
| 132 | LMB3 | CAGGAAACAGCTATGACCATGATTAC |
| 133 | Vl_3_19_L3r_V | GGACGGTCAGCTTGGTCCCTCCGCCGAATAC VHV ATT ACC GCT ACT ATC ACG GGAGTTACAGTAATAGTCAGCCTCATCTTCCGC |
| | | underlined: 60% original base and 40% randomization as M |
| | | bold and italic: 60% original base and 40% randomization as N |
| 134 | Vl_3_19_L3r_HV | GGACGGTCAGCTTGGTCCCTCCGCCGAATAC CMM ATG ATT ACC GCT ACT ATC ACG GGAGTTACAGTAATAGTCAGCCTCATCTTCCGC |
| | | underlined: 60% original base and 40% randomization as M |
| | | bolded and italic: 60% original base and 40% randomization as N |
| 135 | Vl_3_19_L3r_HL V | GGACGGTCAGCTTGGTCCCTCCGCCGAATAC RHM VWG ATG ATT ACC GCT ACT ATC ACG GGAGTTACAGTAATAGTCAGCCTCATCTTC CGC |
| | | underlined: 60% original base and 4U% randomization as M |
| | | bolded and italic: 60% original base and 40% randomization as N |
| 136 | RJH80 | TTCGGCGGAGGGACCAAGCTGACCGTCC |

| | | |
|---|---|---|
| Additional primers used for construction of the lambda-DP47 library, i.e. DP47CDR3_ba (mod.), DP47-v4-4, DP47-v4-6, DP47-v4-8 and fdseqlong, are identical to the primers used for the construction of the common light chain library (Vk320/VH3_23) and have already been listed in Table 8. | | |

Clones 8H9, 20B7, 49B4, 1G4, CLC-563, CLC-564 and 17A9 were identified as human Ox40-specific binders through the procedure described above. The cDNA sequences of their variable regions are shown in **Table 12** below, the corresponding amino acid sequences can be found in Table C.

**Table 12: Variable region base pair sequences for phage-derived anti-Ox40 antibodies. Underlined are the complementary determining regions (CDRs).**

| Clone | SEQ ID NO: | Sequence |
|---|---|---|
| 8H9 | 137 (VL) | TCGTCTGAGCTGACTCAGGACCCTGCTGTGTCTGTGGCCTTGGGACA GACAGTCAGGATCACATGCCAAGGAGACAGCCTCAGAAGTTATTAT GCAAGCTGGTACCAGCAGAAGCCAGGACAGGCCCCTGTACTTGTCA TCTATGGTAAAAACAACCGGCCCTCAGGGATCCCAGACCGATTCTC TGGCTCCAGCTCAGGAAACACAGCTTCCTTGACCATCACTGGGGCT CAGGCGGAAGATGAGGCTGACTATTACTGTAACTCCCGTGTTATGC CTCATAATCGCGTATTCGGCGGAGGGACCAAGCTGACCGTC |
| | 138 (VH) | GAGGTGCAATTGTTGGAGTCTGGGGGAGGCTTGGTACAGCCTGGGG GGTCCCTGAGACTCTCCTGTGCAGCCTCCGGATTCACCTTTAGCAGT TATGCCATGAGCTGGGTCCGCCAGGCTCCAGGGAAGGGGCTGGAGT GGGTCTCAGCTATTAGTGGTAGTGGTGGTAGCACATACTACGCAGA CTCCGTGAAGGGCCGGTTCACCATCTCCAGAGACAATTCCAAGAAC ACGCTGTATCTGCAGATGAACAGCCTGAGAGCCGAGGACACGGCCG TATATTACTGTGCGCGTGTTTCTACCGTGGTGGTGTTTCTATGGAC TACTGGGGCCAAGGAACCCTGGTCACCGTCTCGAGT |
| 49B4 | 139 (VL) | GACATCCAGATGACCCAGTCTCCTTCCACCCTGTCTGCATCTGTAGG AGACCGTGTCACCATCACTTGCCGTGCCAGTCAGAGTATTAGTAGC TGGTTGGCCTGGTATCAGCAGAAACCAGGGAAAGCCCCTAAGCTCC TGATCTATGATGCCTCCAGTTTGGAAAGTGGGGTCCCATCACGTTTC AGCGGCAGTGGATCCGGGACAGAATTCACTCTCACCATCAGCAGCT TGCAGCCTGATGATTTTGCAACTTATTACTGCCAACAGTATAGTTCG CAGCCGTATACGTTTGGCCAGGGCACCAAAGTCGAGATCAAG |
| | 140 (VH) | CAGGTGCAATTGGTGCAGTCTGGGGCTGAGGTGAAGAAGCCTGGGT CCTCGGTGAAGGTCTCCTGCAAGGCCTCCGGAGGCACATTCAGCAG CTACGCTATAAGCTGGGTGCGACAGGCCCCTGGACAAGGGCTCGAG TGGATGGGAGGGATCATCCCTATCTTTGGTACAGCAAACTACGCAC AGAAGTTCCAGGGCAGGGTCACCATTACTGCAGACAAATCCACGAG CACAGCCTACATGGAGCTGAGCAGCCTGAGATCTGAGGACACCGCC GTGTATTACTGTGCGAGAGAATACTACCGTGGTCCGTACGACTACT GGGGCCAAGGGACCACCGTGACCGTCTCCTCA |
| 1G4 | 141 (VL) | GACATCCAGATGACCCAGTCTCCTTCCACCCTGTCTGCATCTGTAGG AGACCGTGTCACCATCACTTGCCGTGCCAGTCAGAGTATTAGTAGC TGGTTGGCCTGGTATCAGCAGAAACCAGGGAAAGCCCCTAAGCTCC TGATCTATGATGCCTCCAGTTTGGAAAGTGGGGTCCCATCACGTTTC AGCGGCAGTGGATCCGGGACAGAATTCACTCTCACCATCAGCAGCT TGCAGCCTGATGATTTTGCAACTTATTACTGCCAACAGTATATTTCG TATTCCATGTTGACGTTTGGCCAGGGCACCAAAGTCGAGATCAAG |
| | 142 (VH) | CAGGTGCAATTGGTGCAGTCTGGGGCTGAGGTGAAGAAGCCTGGGT CCTCGGTGAAGGTCTCCTGCAAGGCCTCCGGAGGCACATTCAGCAG CTACGCTATAAGCTGGGTGCGACAGGCCCCTGGACAAGGGCTCGAG TGGATGGGAGGGATCATCCCTATCTTTGGTACAGCAAACTACGCAC AGAAGTTCCAGGGCAGGGTCACCATTACTGCAGACAAATCCACGAG CACAGCCTACATGGAGCTGAGCAGCCTGAGATCTGAGGACACCGCC GTGTATTACTGTGCGAGAGAATACGGTTCTATGGACTACTGGGGCC AAGGGACCACCGTGACCGTCTCCTCA |
| 20B7 | 143 (VL) | GACATCCAGATGACCCAGTCTCCTTCCACCCTGTCTGCATCTGTAGG AGACCGTGTCACCATCACTTGCCGTGCCAGTCAGAGTATTAGTAGC TGGTTGGCCTGGTATCAGCAGAAACCAGGGAAAGCCCCTAAGCTCC |
| | | TGATCTATGATGCCTCCAGTTTGGAAAGTGGGGTCCCATCACGTTTC AGCGGCAGTGGATCCGGGACAGAATTCACTCTCACCATCAGCAGCT TGCAGCCTGATGATTTTGCAACTTATTACTGCCAACAGTATCAGGCT TTTTCGCTTACGTTTGGCCAGGGCACCAAAGTCGAGATCAAG |
| | 144 (VH) | CAGGTGCAATTGGTGCAGTCTGGGGCTGAGGTGAAGAAGCCTGGGT CCTCGGTGAAGGTCTCCTGCAAGGCCTCCGGAGGCACATTCAGCAG CTACGCTATAAGCTGGGTGCGACAGGCCCCTGGACAAGGGCTCGAG TGGATGGGAGGGATCATCCCTATCTTTGGTACAGCAAACTACGCAC AGAAGTTCCAGGGCAGGGTCACCATTACTGCAGACAAATCCACGAG CACAGCCTACATGGAGCTGAGCAGCCTGAGATCTGAGGACACCGCC GTGTATTACTGTGCGAGAGTTAACTACCCGTACTCTTACTGGGGTGA CTTCGACTACTGGGGCCAAGGGACCACCGTGACCGTCTCCTCA |
| CLC-563 | 145 (VL) | GAGATCGTGCTGACCCAGAGCCCCGGCACACTCTCCCTGTCTCCTG GGGAAAGGGCCACCCTTTCATGCAGAGCCAGCCAGTCCGTCTCTAG TAGCTACCTGGCATGGTATCAGCAGAAGCCAGGACAAGCCCCCCGC CTCCTGATTTACGGCGCTTCCTCTCGGGCAACTGGTATCCCTGACAG GTTCTCAGGGAGCGGAAGCGGAACAGATTTTACCTTGACTATTTCT AGACTGGAGCCAGAGGACTTCGCCGTGTATTACTGTCAGCAGTACG GTAGTAGCCCCCTCACCTTTGGCCAGGGGACAAAAGTCGAAATCAA G GAGGTGCAATTGTTGGAGTCTGGGGGAGGCTTGGTACAGCCTGGGG GGTCCCTGAGACTCTCCTGTGCAGCCTCCGGATTCACCTTTAGCAGT TATGCCATGAGCTGGGTCCGCCAGGCTCCAGGGAAGGGGCTGGAGT GGGTCTCAGCTATTAGTGGTAGTGGTGGTAGCACATACTACGCAGA CTCCGTGAAGGGCCGGTTCACCATCTCCAGAGACAATTCCAAGAAC ACGCTGTATCTGCAGATGAACAGCCTGAGAGCCGAGGACACGGCCG TATATTACTGTGCGCTTGACGTTGGTGCTTTCGACTACTGGGGCCAA GGAGCCCTGGTCACCGTCTCGAGT |
| | 146 (VH) | GAGGTGCAATTGTTGGAGTCTGGGGGAGGCTTGGTACAGCCTGGGG GGTCCCTGAGACTCTCCTGTGCAGCCTCCGGATTCACCTTTAGCAGT TATGCCATGAGCTGGGTCCGCCAGGCTCCAGGGAAGGGGCTGGAGT GGGTCTCAGCTATTAGTGGTAGTGGTGGTAGCACATACTACGCAGA CTCCGTGAAGGGCCGGTTCACCATCTCCAGAGACAATTCCAAGAAC ACGCTGTATCTGCAGATGAACAGCCTGAGAGCCGAGGACACGGCCG TATATTACTGTGCGCTTGACGTTGGTGCTTTCGACTACTGGGGCCAA GGAGCCCTGGTCACCGTCTCGAGT |
| CLC-564 | 147 (VL) | GAGATCGTGCTGACCCAGAGCCCCGGCACACTCTCCCTGTCTCCTG GGGAAAGGGCCACCCTTTCATGCAGAGCCAGCCAGTCCGTCTCTAG TAGCTACCTGGCATGGTATCAGCAGAAGCCAGGACAAGCCCCCCGC CTCCTGATTTACGGCGCTTCCTCTCGGGCAACTGGTATCCCTGACAG GTTCTCAGGGAGCGGAAGCGGAACAGATTTTACCTTGACTATTTCT AGACTGGAGCCAGAGGACTTCGCCGTGTATTACTGTCAGCAGTACG GTAGTAGCCCCCTCACCTTTGGCCAGGGGACAAAAGTCGAAATCAA G |
| | 148 (VH) | GAGGTGCAATTGTTGGAGTCTGGGGGAGGCTTGGTACAGCCTGGGG GGTCCCTGAGACTCTCCTGTGCAGCCTCCGGATTCACCTTTAGCAGT TATGCCATGAGCTGGGTCCGCCAGGCTCCAGGGAAGGGGCTGGAGT GGGTCTCAGCTATTAGTGGTAGTGGTGGTAGCACATACTACGCAGA CTCCGTGAAGGGCCGGTTCACCATCTCCAGAGACAATTCCAAGAAC ACGCTGTATCTGCAGATGAACAGCCTGAGAGCCGAGGACACGGCCG TATATTACTGTGCGTTCGACGTTGGTCCGTTCGACTACTGGGGCCAA GGAACCCTGGTCACCGTCTCGAGT |
| 17A9 | 149 (VL) | TCGTCTGAGCTGACTCAGGACCCTGCTGTGTCTGTGGCCTTGGACA GACAGTCAGGATCACATGCCAAGGAGACAGCCTCAGAAGTTATTAT GCAAGCTGGTACCAGCAGAAGCCAGGACAGGCCCCTGTACTTGTCA TCTATGGTAAAAACAACCGGCCCTCAGGGATCCCAGACCGATTCTC TGGCTCCAGCTCAGGAAACACAGCTTCCTTGACCATCACTGGGGCT CAGGCGGAAGATGAGGCTGACTATTACTGTAACTCCCGTGTTATGC CTCATAATCGCGTATTCGGCGGAGGGACCAAGCTGACCGTC GAGGTGCAATTGTTGGAGTCTGGGGGAGGCTTGGTACAGCCTGGGG GGTCCCTGAGACTCTCCTGTGCAGCCTCCGGATTCACCTTTAGCAGT TATGCCATGAGCTGGGTCCGCCAGGCTCCAGGGAAGGGGCTGGAGT |
| | 150 (VH) | GAGGTGCAATTGTTGGAGTCTGGGGGAGGCTTGGTACAGCCTGGGG GGTCCCTGAGACTCTCCTGTGCAGCCTCCGGATTCACCTTTAGCAGT TATGCCATGAGCTGGGTCCGCCAGGCTCCAGGGAAGGGGCTGGAGT |
| | | GGGTCTCAGCTATTAGTGGTAGTGGTGGTAGCACATACTACGCAGA CTCCGTGAAGGGCCGGTTCACCATCTCCAGAGACAATTCCAAGAAC ACGCTGTATCTGCAGATGAACAGCCTGAGAGCCGAGGACACGGCCG TATATTACTGTGCGCGTGTTTCTACCGTGGTGGTGTTTCTATGGAC TACTGGGGCCAAGGAACCCTGGTCACCGTCTCGAGT |

### 1.3 Preparation, purification and characterization of anti-Ox40 IgG1 P329G LALA antibodies

The variable regions of heavy and light chain DNA sequences of selected anti-Ox40 binders were subcloned in frame with either the constant heavy chain or the constant light chain of human IgG1. The Pro329Gly, Leu234Ala and Leu235Ala mutations have been introduced in the constant region of the knob and hole heavy chains to abrogate binding to Fc gamma receptors according to the method described in International Patent Appl. Publ. No. WO 2012/130831 A1.

The cDNA and amino acid sequences of the anti-Ox40 clones are shown in **Table 13.** All anti-Ox40-Fc-fusion encoding sequences were cloned into a plasmid vector, which drives expression of the insert from an MPSV promoter and contains a synthetic polyA signal sequence located at the 3' end of the CDS. In addition, the vector contains an EBV OriP sequence for episomal maintenance of the plasmid.

**Table 13: Sequences of anti-Ox40 clones in P329GLALA human IgG1 format**

| **Clone** | **SEQ ID No.** | **Sequence** |
|---|---|---|
| **8B9** | 151 (nucleotide sequence light chain) | GACATCCAGATGACCCAGTCTCCTTCCACCCTGTCTGCATCTGT AGGAGACCGTGTCACCATCACTTGCCGTGCCAGTCAGAGTATT AGTAGCTGGTTGGCCTGGTATCAGCAGAAACCAGGGAAAGCC CCTAAGCTCCTGATCTATGATGCCTCCAGTTTGGAAAGTGGGG TCCCATCACGTTTCAGCGGCAGTGGATCCGGGACAGAATTCAC TCTCACCATCAGCAGCTTGCAGCCTGATGATTTTGCAACTTATT ACTGCCAACAGTATTTGACGTATTCGCGGTTTACGTTTGGCCAG GGCACCAAAGTCGAGATCAAGGTACGGTGGCTGCACCATCTGT CTTCATCTTCCCGCCATCTGATGAGCAGTTGAAATCTGGAACTG CCTCTGTTGTGTGCCTGCTGAATAACTTCTATCCCAGAGAGGCC AAAGTACAGTGGAAGGTGGATAACGCCCTCCAATCGGGTAACT CCCAGGAGAGTGTCACAGAGCAGGACAGCAAGGACAGCACCT ACAGCCTCAGCAGCACCCTGACGCTGAGCAAAGCAGACTACG AGAAACACAAAGTCTACGCCTGCGAAGTCACCCATCAGGGCCT GAGCTCGCCCGTCACAAAGAGCTTCAACAGGGGAGAGTGT |
| | 152 (nucleotide sequence heavy chain) | CAGGTGCAATTGGTGCAGTCTGGGGCTGAGGTGAAGAAGCCTG GGTCCTCGGTGAAGGTCTCCTGCAAGGCCTCCGGAGGCACATT CAGCAGCTACGCTATAAGCTGGGTGCGACAGGCCCCTGGACAA GGGCTCGAGTGGATGGGAGGGATCATCCCTATCTTTGGTACAG CAAACTACGCACAGAAGTTCCAGGGCAGGGTCACCATTACTGC AGACAAATCCACGAGCACAGCCTACATGGAGCTGAGCAGCCT GAGATCTGAGGACACCGCCGTGTATTACTGTGCGAGAGAATAC GGTTGGATGGACTACTGGGGCCAAGGGACCACCGTGACCGTCT CCTCAGCTAGCACCAAGGGCCCATCGGTCTTCCCCCTGGCACC |
| | | CTCCTCCAAGAGCACCTCTGGGGGCACAGCGGCCCTGGGCTGC CTGGTCAAGGACTACTTCCCCGAACCGGTGACGGTGTCGTGGA ACTCAGGCGCCCTGACCAGCGGCGTGCACACCTTCCCGGCTGT CCTACAGTCCTCAGGACTCTACTCCCTCAGCAGCGTGGTGACC GTGCCCTCCAGCAGCTTGGGCACCCAGACCTACATCTGCAACG TGAATCACAAGCCCAGCAACACCAAGGTGGACAAGAAAGTTG AGCCCAAATCTTGTGACAAAACTCACACATGCCCACCGTGCCC AGCACCTGAAGCTGCAGGGGGACCGTCAGTCTTCCTCTTCCCC CCAAAACCCAAGGACACCCTCATGATCTCCCGGACCCCTGAGG TCACATGCGTGGTGGTGGACGTGAGCCACGAAGACCCTGAGGT CAAGTTCAACTGGTACGTGGACGGCGTGGAGGTGCATAATGCC AAGACAAAGCCGCGGGAGGAGCAGTACAACAGCACGTACCGT GTGGTCAGCGTCCTCACCGTCCTGCACCAGGACTGGCTGAATG GCAAGGAGTACAAGTGCAAGGTCTCCAACAAAGCCCTCGGCG CCCCCATCGAGAAAACCATCTCCAAAGCCAAAGGGCAGCCCC GAGAACCACAGGTGTACACCCTGCCCCCATCCCGGGATGAGCT GACCAAGAACCAGGTCAGCCTGACCTGCCTGGTCAAAGGCTTC TATCCCAGCGACATCGCCGTGGAGTGGGAGAGCAATGGGCAG CCGGAGAACAACTACAAGACCACGCCTCCCGTGCTGGACTCCG ACGGCTCCTTCTTCCTCTACAGCAAGCTCACCGTGGACAAGAG CAGGTGGCAGCAGGGGAACGTCTTCTCATGCTCCGTGATGCAT GAGGCTCTGCACAACCACTACACGCAGAAGAGCCTCTCCCTGT CTCCGGGTAAA |
| | 153 (Light chain) | DIQMTQSPSTLSASVGDRVTITCRASQSISSWLAWYQQKPGKAPK LLIYDASSLESGVPSRFSGSGSGTEFTLTISSLQPDDFATYYCQQYL TYSRFTFGQGTKVEIKRTVAAPSVFIFPPSDEQLKSGTASVVCLLN NFYPREAKVQWKVDNALQSGNSQESVTEQDSKDSTYSLSSTLTLS KADYEKHKVYACEVTHQGLSSPVTKSFNRGEC |
| | 154 (Heavy chain) | QVQLVQSGAEVKKPGSSVKVSCKASGGTFSSYAISWVRQAPGQG LEWMGGIIPIFGTANYAQKFQGRVTITADKSTSTAYMELSSLRSED TAVYYCAREYGWMDYWGQGTTVTVSSASTKGPSVFPLAPSSKST SGGTAALGCLVKDYFPEPVTVSWNSGALTSGVHTFPAVLQSSGL YSLSSVVTVPSSSLGTQTYICNVNHKPSNTKVDKKVEPKSCDKTH TCPPCPAPEAAGGPSVFLFPPKPKDTLMISRTPEVTCVVVDVSHED PEVKFNWYVDGVEVHNAKTKPREEQYNSTYRVVSVLTVLHQDW LNGKEYKCKVSNKALGAPIEKTISKAKGQPREPQVYTLPPSRDEL TKNQVSLTCLVKGFYPSDIAVEWESNGQPENNYKTTPPVLDSDGS FFLYSKLTVDKSRWQQGNVFSCSVMHEALHNHYTQKSLSLSPGK |
| **49B4** | 155 (nucleotide sequence light chain) | GACATCCAGATGACCCAGTCTCCTTCCACCCTGTCTGCATCTGT AGGAGACCGTGTCACCATCACTTGCCGTGCCAGTCAGAGTATT AGTAGCTGGTTGGCCTGGTATCAGCAGAAACCAGGGAAAGCC CCTAAGCTCCTGATCTATGATGCCTCCAGTTTGGAAAGTGGGG TCCCATCACGTTTCAGCGGCAGTGGATCCGGGACAGAATTCAC TCTCACCATCAGCAGCTTGCAGCCTGATGATTTTGCAACTTATT ACTGCCAACAGTATAGTTCGCAGCCGTATACGTTTGGCCAGGG CACCAAAGTCGAGATCAAGCGTACGGTGGCTGCACCATCTGTC TTCATCTTCCCGCCATCTGATGAGCAGTTGAAATCTGGAACTGC CTCTGTTGTGTGCCTGCTGAATAACTTCTATCCCAGAGAGGCCA AAGTACAGTGGAAGGTGGATAACGCCCTCCAATCGGGTAACTC CCAGGAGAGTGTCACAGAGCAGGACAGCAAGGACAGCACCTA CAGCCTCAGCAGCACCCTGACGCTGAGCAAAGCAGACTACGA GAAACACAAAGTCTACGCCTGCGAAGTCACCCATCAGGGCCTG AGCTCGCCCGTCACAAAGAGCTTCAACAGGGGAGAGTGT |
| | 156 (nucleotide sequence heavy chain) | CAGGTGCAATTGGTGCAGTCTGGGGCTGAGGTGAAGAAGCCTG GGTCCTCGGTGAAGGTCTCCTGCAAGGCCTCCGGAGGCACATT CAGCAGCTACGCTATAAGCTGGGTGCGACAGGCCCCTGGACAA GGGCTCGAGTGGATGGGAGGGATCATCCCTATCTTTGGTACAG CAAACTACGCACAGAAGTTCCAGGGCAGGGTCACCATTACTGC AGACAAATCCACGAGCACAGCCTACATGGAGCTGAGCAGCCT GAGATCTGAGGACACCGCCGTGTATTACTGTGCGAGAGAATAC TACCGTGGTCCGTACGACTACTGGGGCCAAGGGACCACCGTGA CCGTCTCCTCAGCTAGCACCAAGGGCCCATCGGTCTTCCCCCTG GCACCCTCCTCCAAGAGCACCTCTGGGGGCACAGCGGCCCTGG GCTGCCTGGTCAAGGACTACTTCCCCGAACCGGTGACGGTGTC GTGGAACTCAGGCGCCCTGACCAGCGGCGTGCACACCTTCCCG GCTGTCCTACAGTCCTCAGGACTCTACTCCCTCAGCAGCGTGGT GACCGTGCCCTCCAGCAGCTTGGGCACCCAGACCTACATCTGC AACGTGAATCACAAGCCCAGCAACACCAAGGTGGACAAGAAA GTTGAGCCCAAATCTTGTGACAAAACTCACACATGCCCACCGT GCCCAGCACCTGAAGCTGCAGGGGGACCGTCAGTCTTCCTCTT CCCCCCAAAACCCAAGGACACCCTCATGATCTCCCGGACCCCT GAGGTCACATGCGTGGTGGTGGACGTGAGCCACGAAGACCCT GAGGTCAAGTTCAACTGGTACGTGGACGGCGTGGAGGTGCATA ATGCCAAGACAAAGCCGCGGGAGGAGCAGTACAACAGCACGT ACCGTGTGGTCAGCGTCCTCACCGTCCTGCACCAGGACTGGCT GAATGGCAAGGAGTACAAGTGCAAGGTCTCCAACAAAGCCCT CGGCGCCCCCATCGAGAAAACCATCTCCAAAGCCAAAGGGCA GCCCCGAGAACCACAGGTGTACACCCTGCCCCCATCCCGGGAT GAGCTGACCAAGAACCAGGTCAGCCTGACCTGCCTGGTCAAAG GCTTCTATCCCAGCGACATCGCCGTGGAGTGGGAGAGCAATGG GCAGCCGGAGAACAACTACAAGACCACGCCTCCCGTGCTGGA CTCCGACGGCTCCTTCTTCCTCTACAGCAAGCTCACCGTGGACA AGAGCAGGTGGCAGCAGGGGAACGTCTTCTCATGCTCCGTGAT GCATGAGGCTCTGCACAACCACTACACGCAGAAGAGCCTCTCC CTGTCTCCGGGTAAA |
| | 157 (Light chain) | DIQMTQSPSTLSASVGDRVTITCRASQSISSWLAWYQQKPGKAPK LLIYDASSLESGVPSRFSGSGSGTEFTLTISSLQPDDFATYYCQQYS SQPYTFGQGTKVEIKRTVAAPSVFIFPPSDEQLKSGTASVVCLLNN FYPREAKVQWKVDNALQSGNSQESVTEQDSKDSTYSLSSTLTLSK ADYEKHKVYACEVTHQGLSSPVTKSFNRGEC |
| | 158 (Heavy chain) | QVQLVQSGAEVKKPGSSVKVSCKASGGTFSSYAISWVRQAPGQG LEWMGGIIPIFGTANYAQKFQGRVTITADKSTSTAYMELSSLRSED TAVYYCAREYYRGPYDYWGQGTTVTVSSASTKGPSVFPLAPSSK STSGGTAALGCLVKDYFPEPVTVSWNSGALTSGVHTFPAVLQSSG LYSLSSVVTVPSSSLGTQTYICNVNHKPSNTKVDKKVEPKSCDKT HTCPPCPAPEAAGGPSVFLFPPKPKDTLMISRTPEVTCVVVDVSHE DPEVKFNWYVDGVEVHNAKTKPREEQYNSTYRVVSVLTVLHQD WLNGKEYKCKVSNKALGAPIEKTISKAKGQPREPQVYTLPPSRDE LTKNQVSLTCLVKGFYPSDIAVEWESNGQPENNYKTTPPVLDSDG SFFLYSKLTVDKSRWQQGNVFSCSVMHEALHNHYTQKSLSLSPG K |
| **1G4** | 159 (nucleotide sequence light chain) | GACATCCAGATGACCCAGTCTCCTTCCACCCTGTCTGCATCTGT AGGAGACCGTGTCACCATCACTTGCCGTGCCAGTCAGAGTATT AGTAGCTGGTTGGCCTGGTATCAGCAGAAACCAGGGAAAGCC CCTAAGCTCCTGATCTATGATGCCTCCAGTTTGGAAAGTGGGG TCCCATCACGTTTCAGCGGCAGTGGATCCGGGACAGAATTCAC |
| | | TCTCACCATCAGCAGCTTGCAGCCTGATGATTTTGCAACTTATT ACTGCCAACAGTATATTTCGTATTCCATGTTGACGTTTGGCCAG GGCACCAAAGTCGAGATCAAGCGTACGGTGGCTGCACCATCTG TCTTCATCTTCCCGCCATCTGATGAGCAGTTGAAATCTGGAACT GCCTCTGTTGTGTGCCTGCTGAATAACTTCTATCCCAGAGAGGC CAAAGTACAGTGGAAGGTGGATAACGCCCTCCAATCGGGTAA CTCCCAGGAGAGTGTCACAGAGCAGGACAGCAAGGACAGCAC CTACAGCCTCAGCAGCACCCTGACGCTGAGCAAAGCAGACTAC GAGAAACACAAAGTCTACGCCTGCGAAGTCACCCATCAGGGC CTGAGCTCGCCCGTCACAAAGAGCTTCAACAGGGGAGAGTGT |
| | 160 (nucleotide sequence heavy chain) | CAGGTGCAATTGGTGCAGTCTGGGGCTGAGGTGAAGAAGCCTG GGTCCTCGGTGAAGGTCTCCTGCAAGGCCTCCGGAGGCACATT CAGCAGCTACGCTATAAGCTGGGTGCGACAGGCCCCTGGACAA GGGCTCGAGTGGATGGGAGGGATCATCCCTATCTTTGGTACAG CAAACTACGCACAGAAGTTCCAGGGCAGGGTCACCATTACTGC AGACAAATCCACGAGCACAGCCTACATGGAGCTGAGCAGCCT GAGATCTGAGGACACCGCCGTGTATTACTGTGCGAGAGAATAC GGTTCTATGGACTACTGGGGCCAAGGGACCACCGTGACCGTCT CCTCAGCTAGCACCAAGGGCCCATCGGTCTTCCCCCTGGCACC CTCCTCCAAGAGCACCTCTGGGGGCACAGCGGCCCTGGGCTGC CTGGTCAAGGACTACTTCCCCGAACCGGTGACGGTGTCGTGGA ACTCAGGCGCCCTGACCAGCGGCGTGCACACCTTCCCGGCTGT CCTACAGTCCTCAGGACTCTACTCCCTCAGCAGCGTGGTGACC GTGCCCTCCAGCAGCTTGGGCACCCAGACCTACATCTGCAACG TGAATCACAAGCCCAGCAACACCAAGGTGGACAAGAAAGTTG AGCCCAAATCTTGTGACAAAACTCACACATGCCCACCGTGCCC AGCACCTGAAGCTGCAGGGGGACCGTCAGTCTTCCTCTTCCCC CCAAAACCCAAGGACACCCTCATGATCTCCCGGACCCCTGAGG TCACATGCGTGGTGGTGGACGTGAGCCACGAAGACCCTGAGGT CAAGTTCAACTGGTACGTGGACGGCGTGGAGGTGCATAATGCC AAGACAAAGCCGCGGGAGGAGCAGTACAACAGCACGTACCGT GTGGTCAGCGTCCTCACCGTCCTGCACCAGGACTGGCTGAATG GCAAGGAGTACAAGTGCAAGGTCTCCAACAAAGCCCTCGGCG CCCCCATCGAGAAAACCATCTCCAAAGCCAAAGGGCAGCCCC GAGAACCACAGGTGTACACCCTGCCCCCATCCCGGGATGAGCT GACCAAGAACCAGGTCAGCCTGACCTGCCTGGTCAAAGGCTTC TATCCCAGCGACATCGCCGTGGAGTGGGAGAGCAATGGGCAG CCGGAGAACAACTACAAGACCACGCCTCCCGTGCTGGACTCCG ACGGCTCCTTCTTCCTCTACAGCAAGCTCACCGTGGACAAGAG CAGGTGGCAGCAGGGGAACGTCTTCTCATGCTCCGTGATGCAT GAGGCTCTGCACAACCACTACACGCAGAAGAGCCTCTCCCTGT CTCCGGGTAAA |
| | 161 (Light chain) | DIQMTQSPSTLSASVGDRVTITCRASQSISSWLAWYQQKPGKAPK LLIYDASSLESGVPSRFSGSGSGTEFTLTISSLQPDDFATYYCQQYIS YSMLTFGQGTKVEIKRTVAAPSVFIFPPSDEQLKSGTASVVCLLNN FYPREAKVQWKVDNALQSGNSQESVTEQDSKDSTYSLSSTLTLSK ADYEKHKVYACEVTHQGLSSPVTKSFNRGEC |
| | 162 (Heavy chain) | QVQLVQSGAEVKKPGSSVKVSCKASGGTFSSYAISWVRQAPGQG LEWMGGIIPIFGTANYAQKFQGRVTITADKSTSTAYMELSSLRSED TAVYYCAREYGSMDYWGQGTTVTVSSASTKGPSVFPLAPSSKST SGGTAALGCLVKDYFPEPVTVSWNSGALTSGVHTFPAVLQSSGL YSLSSVVTVPSSSLGTQTYICNVNHKPSNTKVDKKVEPKSCDKTH TCPPCPAPEAAGGPSVFLFPPKPKDTLMISRTPEVTCVVVDVSHED |
| | | PEVKFNWYVDGVEVHNAKTKPREEQYNSTYRVVSVLTVLHQDW LNGKEYKCKVSNKALGAPIEKTISKAKGQPREPQVYTLPPSRDEL TKNQVSLTCLVKGFYPSDIAVEWESNGQPENNYKTTPPVLDSDGS FFLYSKLTVDKSRWQQGNVFSCSVMHEALHNHYTQKSLSLSPGK |
| **20B7** | 163 (nucleotide sequence light chain) | GACATCCAGATGACCCAGTCTCCTTCCACCCTGTCTGCATCTGT AGGAGACCGTGTCACCATCACTTGCCGTGCCAGTCAGAGTATT AGTAGCTGGTTGGCCTGGTATCAGCAGAAACCAGGGAAAGCC CCTAAGCTCCTGATCTATGATGCCTCCAGTTTGGAAAGTGGGG TCCCATCACGTTTCAGCGGCAGTGGATCCGGGACAGAATTCAC TCTCACCATCAGCAGCTTGCAGCCTGATGATTTTGCAACTTATT ACTGCCAACAGTATCAGGCTTTTTCGCTTACGTTTGGCCAGGGC ACCAAAGTCGAGATCAAGCGTACGGTGGCTGCACCATCTGTCT TCATCTTCCCGCCATCTGATGAGCAGTTGAAATCTGGAACTGC CTCTGTTGTGTGCCTGCTGAATAACTTCTATCCCAGAGAGGCCA AAGTACAGTGGAAGGTGGATAACGCCCTCCAATCGGGTAACTC CCAGGAGAGTGTCACAGAGCAGGACAGCAAGGACAGCACCTA CAGCCTCAGCAGCACCCTGACGCTGAGCAAAGCAGACTACGA GAAACACAAAGTCTACGCCTGCGAAGTCACCCATCAGGGCCTG AGCTCGCCCGTCACAAAGAGCTTCAACAGGGGAGAGTGT |
| | 164 (nucleotide sequence heavy chain) | CAGGTGCAATTGGTGCAGTCTGGGGCTGAGGTGAAGAAGCCTG GGTCCTCGGTGAAGGTCTCCTGCAAGGCCTCCGGAGGCACATT CAGCAGCTACGCTATAAGCTGGGTGCGACAGGCCCCTGGACAA GGGCTCGAGTGGATGGGAGGGATCATCCCTATCTTTGGTACAG CAAACTACGCACAGAAGTTCCAGGGCAGGGTCACCATTACTGC AGACAAATCCACGAGCACAGCCTACATGGAGCTGAGCAGCCT GAGATCTGAGGACACCGCCGTGTATTACTGTGCGAGAGTTAAC TACCCGTACTCTTACTGGGGTGACTTCGACTACTGGGGCCAAG GGACCACCGTGACCGTCTCCTCAGCTAGCACCAAGGGCCCATC GGTCTTCCCCCTGGCACCCTCCTCCAAGAGCACCTCTGGGGGC ACAGCGGCCCTGGGCTGCCTGGTCAAGGACTACTTCCCCGAAC CGGTGACGGTGTCGTGGAACTCAGGCGCCCTGACCAGCGGCGT GCACACCTTCCCGGCTGTCCTACAGTCCTCAGGACTCTACTCCC TCAGCAGCGTGGTGACCGTGCCCTCCAGCAGCTTGGGCACCCA GACCTACATCTGCAACGTGAATCACAAGCCCAGCAACACCAAG GTGGACAAGAAAGTTGAGCCCAAATCTTGTGACAAAACTCACA CATGCCCACCGTGCCCAGCACCTGAAGCTGCAGGGGGACCGTC AGTCTTCCTCTTCCCCCCAAAACCCAAGGACACCCTCATGATCT CCCGGACCCCTGAGGTCACATGCGTGGTGGTGGACGTGAGCCA CGAAGACCCTGAGGTCAAGTTCAACTGGTACGTGGACGGCGTG GAGGTGCATAATGCCAAGACAAAGCCGCGGGAGGAGCAGTAC AACAGCACGTACCGTGTGGTCAGCGTCCTCACCGTCCTGCACC AGGACTGGCTGAATGGCAAGGAGTACAAGTGCAAGGTCTCCA ACAAAGCCCTCGGCGCCCCCATCGAGAAAACCATCTCCAAAGC CAAAGGGCAGCCCCGAGAACCACAGGTGTACACCCTGCCCCC ATCCCGGGATGAGCTGACCAAGAACCAGGTCAGCCTGACCTGC CTGGTCAAAGGCTTCTATCCCAGCGACATCGCCGTGGAGTGGG AGAGCAATGGGCAGCCGGAGAACAACTACAAGACCACGCCTC CCGTGCTGGACTCCGACGGCTCCTTCTTCCTCTACAGCAAGCTC ACCGTGGACAAGAGCAGGTGGCAGCAGGGGAACGTCTTCTCA TGCTCCGTGATGCATGAGGCTCTGCACAACCACTACACGCAGA AGAGCCTCTCCCTGTCTCCGGGTAAA |
| | 165 (Light chain) | DIQMTQSPSTLSASVGDRVTITCRASQSISSWLAWYQQKPGKAPK LLIYDASSLESGVPSRFSGSGSGTEFTLTISSLQPDDFATYYCQQYQ |
| | | AFSLTFGQGTKVEIKRTVAAPSVFIFPPSDEQLKSGTASVVCLLNN FYPREAKVQWKVDNALQSGNSQESVTEQDSKDSTYSLSSTLTLSK ADYEKHKVYACEVTHQGLSSPVTKSFNRGEC |
| | 166 (Heavy chain) | QVQLVQSGAEVKKPGSSVKVSCKASGGTFSSYAISWVRQAPGQG LEWMGGIIPIFGTANYAQKFQGRVTITADKSTSTAYMELSSLRSED TAVYYCARVNYPYSYWGDFDYWGQGTTVTVSSASTKGPSVFPL APSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPEAAGGPSVFLFPPKPKDTLMISRTPEVTCVVVD VSHEDPEVKFNWYVDGVEVHNAKTKPREEQYNSTYRVVSVLTV LHQDWLNGKEYKCKVSNKALGAPIEKTISKAKGQPREPQVYTLPP SRDELTKNQVSLTCLVKGFYPSDIAVEWESNGQPENNYKTTPPVL DSDGSFFLYSKLTVDKSRWQQGNVFSCSVMHEALHNHYTQKSLS LSPGK |
| **CLC-563** | 167 (nucleotide sequence light chain) | GAGATCGTGCTGACCCAGAGCCCCGGCACACTCTCCCTGTCTC CTGGGGAAAGGGCCACCCTTTCATGCAGAGCCAGCCAGTCCGT CTCTAGTAGCTACCTGGCATGGTATCAGCAGAAGCCAGGACAA GCCCCCCGCCTCCTGATTTACGGCGCTTCCTCTCGGGCAACTGG TATCCCTGACAGGTTCTCAGGGAGCGGAAGCGGAACAGATTTT ACCTTGACTATTTCTAGACTGGAGCCAGAGGACTTCGCCGTGT ATTACTGTCAGCAGTACGGTAGTAGCCCCCTCACCTTTGGCCA GGGGACAAAAGTCGAAATCAAGCGTACGGTGGCTGCACCATC TGTCTTCATCTTCCCGCCATCTGATGAGCAGTTGAAATCTGGAA CTGCCTCTGTTGTGTGCCTGCTGAATAACTTCTATCCCAGAGAG GCCAAAGTACAGTGGAAGGTGGATAACGCCCTCCAATCGGGT AACTCCCAGGAGAGTGTCACAGAGCAGGACAGCAAGGACAGC ACCTACAGCCTCAGCAGCACCCTGACGCTGAGCAAAGCAGACT ACGAGAAACACAAAGTCTACGCCTGCGAAGTCACCCATCAGG GCCTGAGCTCGCCCGTCACAAAGAGCTTCAACAGGGGAGAGT GT |
| | 168 (nucleotide sequence heavy chain) | GAGGTGCAATTGTTGGAGTCTGGGGGAGGCTTGGTACAGCCTG GGGGGTCCCTGAGACTCTCCTGTGCAGCCTCCGGATTCACCTTT AGCAGTTATGCCATGAGCTGGGTCCGCCAGGCTCCAGGGAAGG GGCTGGAGTGGGTCTCAGCTATTAGTGGTAGTGGTGGTAGCAC ATACTACGCAGACTCCGTGAAGGGCCGGTTCACCATCTCCAGA GACAATTCCAAGAACACGCTGTATCTGCAGATGAACAGCCTGA GAGCCGAGGACACGGCCGTATATTACTGTGCGCTTGACGTTGG TGCTTTCGACTACTGGGGCCAAGGAGCCCTGGTCACCGTCTCG AGTGCTAGCACCAAGGGCCCATCGGTCTTCCCCCTGGCACCCT CCTCCAAGAGCACCTCTGGGGGCACAGCGGCCCTGGGCTGCCT GGTCAAGGACTACTTCCCCGAACCGGTGACGGTGTCGTGGAAC TCAGGCGCCCTGACCAGCGGCGTGCACACCTTCCCGGCTGTCC TACAGTCCTCAGGACTCTACTCCCTCAGCAGCGTGGTGACCGT GCCCTCCAGCAGCTTGGGCACCCAGACCTACATCTGCAACGTG AATCACAAGCCCAGCAACACCAAGGTGGACAAGAAAGTTGAG CCCAAATCTTGTGACAAAACTCACACATGCCCACCGTGCCCAG CACCTGAAGCTGCAGGGGGACCGTCAGTCTTCCTCTTCCCCCC AAAACCCAAGGACACCCTCATGATCTCCCGGACCCCTGAGGTC ACATGCGTGGTGGTGGACGTGAGCCACGAAGACCCTGAGGTC AAGTTCAACTGGTACGTGGACGGCGTGGAGGTGCATAATGCCA AGACAAAGCCGCGGGAGGAGCAGTACAACAGCACGTACCGTG TGGTCAGCGTCCTCACCGTCCTGCACCAGGACTGGCTGAATGG CAAGGAGTACAAGTGCAAGGTCTCCAACAAAGCCCTCGGCGC |
| | | CCCCATCGAGAAAACCATCTCCAAAGCCAAAGGGCAGCCCCG AGAACCACAGGTGTACACCCTGCCCCCATCCCGGGATGAGCTG ACCAAGAACCAGGTCAGCCTGACCTGCCTGGTCAAAGGCTTCT ATCCCAGCGACATCGCCGTGGAGTGGGAGAGCAATGGGCAGC CGGAGAACAACTACAAGACCACGCCTCCCGTGCTGGACTCCGA CGGCTCCTTCTTCCTCTACAGCAAGCTCACCGTGGACAAGAGC AGGTGGCAGCAGGGGAACGTCTTCTCATGCTCCGTGATGCATG AGGCTCTGCACAACCACTACACGCAGAAGAGCCTCTCCCTGTC TCCGGGTAAA |
| | 169 (Light chain) | EIVLTQSPGTLSLSPGERATLSCRASQSVSSSYLAWYQQKPGQAPR LLIYGASSRATGIPDRFSGSGSGTDFTLTISRLEPEDFAVYYCQQYG SSPLTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| | 170 (Heavy chain) | EVQLLESGGGLVQPGGSLRLSCAASGFTFSSYAMSWVRQAPGKG LEWVSAISGSGGSTYYADSVKGRFTISRDNSKNTLYLQMNSLRAE DTAVYYCALDVGAFDYWGQGALVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTH TCPPCPAPEAAGGPSVFLFPPKPKDTLMISRTPEVTCVVVDVSHED PEVKFNWYVDGVEVHNAKTKPREEQYNSTYRVVSVLTVLHQDW LNGKEYKCKVSNKALGAPIEKTISKAKGQPREPQVYTLPPSRDEL TKNQVSLTCLVKGFYPSDIAVEWESNGQPENNYKTTPPVLDSDGS FFLYSKLTVDKSRWQQGNVFSCSVMHEALHNHYTQKSLSLSPGK |
| **CLC-564** | 171 (nucleotide sequence light chain) | GAGATCGTGCTGACCCAGAGCCCCGGCACACTCTCCCTGTCTC CTGGGGAAAGGGCCACCCTTTCATGCAGAGCCAGCCAGTCCGT CTCTAGTAGCTACCTGGCATGGTATCAGCAGAAGCCAGGACAA GCCCCCCGCCTCCTGATTTACGGCGCTTCCTCTCGGGCAACTGG TATCCCTGACAGGTTCTCAGGGAGCGGAAGCGGAACAGATTTT ACCTTGACTATTTCTAGACTGGAGCCAGAGGACTTCGCCGTGT ATTACTGTCAGCAGTACGGTAGTAGCCCCCTCACCTTTGGCCA GGGGACAAAAGTCGAAATCAAGCGTACGGTGGCTGCACCATC TGTCTTCATCTTCCCGCCATCTGATGAGCAGTTGAAATCTGGAA CTGCCTCTGTTGTGTGCCTGCTGAATAACTTCTATCCCAGAGAG GCCAAAGTACAGTGGAAGGTGGATAACGCCCTCCAATCGGGT AACTCCCAGGAGAGTGTCACAGAGCAGGACAGCAAGGACAGC ACCTACAGCCTCAGCAGCACCCTGACGCTGAGCAAAGCAGACT ACGAGAAACACAAAGTCTACGCCTGCGAAGTCACCCATCAGG GCCTGAGCTCGCCCGTCACAAAGAGCTTCAACAGGGGAGAGT GT |
| | 172 (nucleotide sequence heavy chain) | GAGGTGCAATTGTTGGAGTCTGGGGGAGGCTTGGTACAGCCTG GGGGGTCCCTGAGACTCTCCTGTGCAGCCTCCGGATTCACCTTT AGCAGTTATGCCATGAGCTGGGTCCGCCAGGCTCCAGGGAAGG GGCTGGAGTGGGTCTCAGCTATTAGTGGTAGTGGTGGTAGCAC ATACTACGCAGACTCCGTGAAGGGCCGGTTCACCATCTCCAGA GACAATTCCAAGAACACGCTGTATCTGCAGATGAACAGCCTGA GAGCCGAGGACACGGCCGTATATTACTGTGCGTTCGACGTTGG TCCGTTCGACTACTGGGGCCAAGGAACCCTGGTCACCGTCTCG AGTGCTAGCACCAAGGGCCCATCGGTCTTCCCCCTGGCACCCT CCTCCAAGAGCACCTCTGGGGGCACAGCGGCCCTGGGCTGCCT GGTCAAGGACTACTTCCCCGAACCGGTGACGGTGTCGTGGAAC TCAGGCGCCCTGACCAGCGGCGTGCACACCTTCCCGGCTGTCC TACAGTCCTCAGGACTCTACTCCCTCAGCAGCGTGGTGACCGT |
| | | GCCCTCCAGCAGCTTGGGCACCCAGACCTACATCTGCAACGTG AATCACAAGCCCAGCAACACCAAGGTGGACAAGAAAGTTGAG CCCAAATCTTGTGACAAAACTCACACATGCCCACCGTGCCCAG CACCTGAAGCTGCAGGGGGACCGTCAGTCTTCCTCTTCCCCCC AAAACCCAAGGACACCCTCATGATCTCCCGGACCCCTGAGGTC ACATGCGTGGTGGTGGACGTGAGCCACGAAGACCCTGAGGTC AAGTTCAACTGGTACGTGGACGGCGTGGAGGTGCATAATGCCA AGACAAAGCCGCGGGAGGAGCAGTACAACAGCACGTACCGTG TGGTCAGCGTCCTCACCGTCCTGCACCAGGACTGGCTGAATGG CAAGGAGTACAAGTGCAAGGTCTCCAACAAAGCCCTCGGCGC CCCCATCGAGAAAACCATCTCCAAAGCCAAAGGGCAGCCCCG AGAACCACAGGTGTACACCCTGCCCCCATCCCGGGATGAGCTG ACCAAGAACCAGGTCAGCCTGACCTGCCTGGTCAAAGGCTTCT ATCCCAGCGACATCGCCGTGGAGTGGGAGAGCAATGGGCAGC CGGAGAACAACTACAAGACCACGCCTCCCGTGCTGGACTCCGA CGGCTCCTTCTTCCTCTACAGCAAGCTCACCGTGGACAAGAGC AGGTGGCAGCAGGGGAACGTCTTCTCATGCTCCGTGATGCATG AGGCTCTGCACAACCACTACACGCAGAAGAGCCTCTCCCTGTC TCCGGGTAAA |
| | 173 (Light chain) | EIVLTQSPGTLSLSPGERATLSCRASQSVSSSYLAWYQQKPGQAPR LLIYGASSRATGIPDRFSGSGSGTDFTLTISRLEPEDFAVYYCQQYG SSPLTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| | 174 (Heavy chain) | EVQLLESGGGLVQPGGSLRLSCAASGFTFSSYAMSWVRQAPGKG LEWVSAISGSGGSTYYADSVKGRFTISRDNSKNTLYLQMNSLRAE DTAVYYCAFDVGPFDYWGQGTLVTVSSASTKGPSVFPLAPSSKST SGGTAALGCLVKDYFPEPVTVSWNSGALTSGVHTFPAVLQSSGL YSLSSVVTVPSSSLGTQTYICNVNHKPSNTKVDKKVEPKSCDKTH TCPPCPAPEAAGGPSVFLFPPKPKDTLMISRTPEVTCVVVDVSHED PEVKFNWYVDGVEVHNAKTKPREEQYNSTYRVVSVLTVLHQDW LNGKEYKCKVSNKALGAPIEKTISKAKGQPREPQVYTLPPSRDEL TKNQVSLTCLVKGFYPSDIAVEWESNGQPENNYKTTPPVLDSDGS FFLYSKLTVDKSRWQQGNVFSCSVMHEALHNHYTQKSLSLSPGK |
| **17A9** | 175 (nucleotide sequence light chain) | TCGTCTGAGCTGACTCAGGACCCTGCTGTGTCTGTGGCCTTGGG ACAGACAGTCAGGATCACATGCCAAGGAGACAGCCTCAGAAG TTATTATGCAAGCTGGTACCAGCAGAAGCCAGGACAGGCCCCT GTACTTGTCATCTATGGTAAAAACAACCGGCCCTCAGGGATCC CAGACCGATTCTCTGGCTCCAGCTCAGGAAACACAGCTTCCTT GACCATCACTGGGGCTCAGGCGGAAGATGAGGCTGACTATTAC TGTAACTCCCGTGTTATGCCTCATAATCGCGTATTCGGCGGAG GGACCAAGCTGACCGTCCTAGGTCAACCCAAGGCTGCCCCCAG CGTGACCCTGTTCCCCCCCAGCAGCGAGGAACTGCAGGCCAAC AAGGCCACCCTGGTCTGCCTGATCAGCGACTTCTACCCAGGCG CCGTGACCGTGGCCTGGAAGGCCGACAGCAGCCCCGTGAAGG CCGGCGTGGAGACCACCACCCCCAGCAAGCAGAGCAACAACA AGTACGCCGCCAGCAGCTACCTGAGCCTGACCCCCGAGCAGTG GAAGAGCCACAGGTCCTACAGCTGCCAGGTGACCCACGAGGG CAGCACCGTGGAGAAAACCGTGGCCCCCACCGAGTGCAGC |
| | 176 (nucleotide sequence heavy chain) | GAGGTGCAATTGTTGGAGTCTGGGGGAGGCTTGGTACAGCCTG GGGGGTCCCTGAGACTCTCCTGTGCAGCCTCCGGATTCACCTTT AGCAGTTATGCCATGAGCTGGGTCCGCCAGGCTCCAGGGAAGG GGCTGGAGTGGGTCTCAGCTATTAGTGGTAGTGGTGGTAGCAC |
| | | ATACTACGCAGACTCCGTGAAGGGCCGGTTCACCATCTCCAGA GACAATTCCAAGAACACGCTGTATCTGCAGATGAACAGCCTGA GAGCCGAGGACACGGCCGTATATTACTGTGCGCGTGTTTCTA CCGTGGTGGTGTTTCTATGGACTACTGGGGCCAAGGAACCCTG GTCACCGTCTCGAGTGCTAGCACCAAGGGCCCATCGGTCTTCC CCCTGGCACCCTCCTCCAAGAGCACCTCTGGGGGCACAGCGGC CCTGGGCTGCCTGGTCAAGGACTACTTCCCCGAACCGGTGACG GTGTCGTGGAACTCAGGCGCCCTGACCAGCGGCGTGCACACCT TCCCGGCTGTCCTACAGTCCTCAGGACTCTACTCCCTCAGCAGC GTGGTGACCGTGCCCTCCAGCAGCTTGGGCACCCAGACCTACA TCTGCAACGTGAATCACAAGCCCAGCAACACCAAGGTGGACA AGAAAGTTGAGCCCAAATCTTGTGACAAAACTCACACATGCCC ACCGTGCCCAGCACCTGAAGCTGCAGGGGGACCGTCAGTCTTC CTCTTCCCCCCAAAACCCAAGGACACCCTCATGATCTCCCGGA CCCCTGAGGTCACATGCGTGGTGGTGGACGTGAGCCACGAAGA CCCTGAGGTCAAGTTCAACTGGTACGTGGACGGCGTGGAGGTG CATAATGCCAAGACAAAGCCGCGGGAGGAGCAGTACAACAGC ACGTACCGTGTGGTCAGCGTCCTCACCGTCCTGCACCAGGACT GGCTGAATGGCAAGGAGTACAAGTGCAAGGTCTCCAACAAAG CCCTCGGCGCCCCCATCGAGAAAACCATCTCCAAAGCCAAAGG GCAGCCCCGAGAACCACAGGTGTACACCCTGCCCCCATCCCGG GATGAGCTGACCAAGAACCAGGTCAGCCTGACCTGCCTGGTCA AAGGCTTCTATCCCAGCGACATCGCCGTGGAGTGGGAGAGCAA TGGGCAGCCGGAGAACAACTACAAGACCACGCCTCCCGTGCTG GACTCCGACGGCTCCTTCTTCCTCTACAGCAAGCTCACCGTGG ACAAGAGCAGGTGGCAGCAGGGGAACGTCTTCTCATGCTCCGT GATGCATGAGGCTCTGCACAACCACTACACGCAGAAGAGCCTC TCCCTGTCTCCGGGTAAA |
| | 177 (Light chain) | SSELTQDPAVSVALGQTVRITCQGDSLRSYYASWYQQKPGQAPV LVIYGKNNRPSGIPDRFSGSSSGNTASLTITGAQAEDEADYYCNSR VMPHNRVFGGGTKLTVLGQPKAAPSVTLFPPSSEELQANKATLVC LISDFYPGAVTVAWKADSSPVKAGVETTTPSKQSNNKYAASSYLS LTPEQWKSHRSYSCQVTHEGSTVEKTVAPTECS |
| | 178 (Heavy chain) | EVQLLESGGGLVQPGGSLRLSCAASGFTFSSYAMSWVRQAPGKG LEWVSAISGSGGSTYYADSVKGRFTISRDNSKNTLYLQMNSLRAE DTAVYYCARVFYRGGVSMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSCD KTHTCPPCPAPEAAGGPSVFLFPPKPKDTLMISRTPEVTCVVVDVS HEDPEVKFNWYVDGVEVHNAKTKPREEQYNSTYRVVSVLTVLH QDWLNGKEYKCKVSNKALGAPIEKTISKAKGQPREPQVYTLPPSR DELTKNQVSLTCLVKGFYPSDIAVEWESNGQPENNYKTTPPVLDS DGSFFLYSKLTVDKSRWQQGNVFSCSVMHEALHNHYTQKSLSLS PGK |

The anti-Ox40 antibodies were produced by co-transfecting HEK293-EBNA cells with the mammalian expression vectors using polyethylenimine. The cells were transfected with the corresponding expression vectors in a 1:1 ratio ("vector heavy chain" : "vector light chain").

For production in 500 mL shake flasks, 400 million HEK293 EBNA cells were seeded 24 hours before transfection. For transfection cells were centrifuged for 5 minutes at 210 x g, and the supernatant was replaced by pre-warmed CD CHO medium. Expression vectors (200 µg of total DNA) were mixed in 20 mL CD CHO medium. After addition of 540 µL PEI, the solution was vortexed for 15 seconds and incubated for 10 minutes at room temperature. Afterwards, cells were mixed with the DNA/PEI solution, transferred to a 500 mL shake flask and incubated for 3 hours at 37°C in an incubator with a 5% CO₂ atmosphere. After the incubation, 160 mL of F17 medium was added and cells were cultured for 24 hours. One day after transfection 1 mM valproic acid and 7% Feed with supplements were added. After culturing for 7 days, the supernatant was collected by centrifugation for 15 minutes at 210 x g. The solution was sterile filtered (0.22 µm filter), supplemented with sodium azide to a final concentration of 0.01 % (w/v), and kept at 4°C.

Purification of antibody molecules from cell culture supernatants was carried out by affinity chromatography using Protein A as described above for purification of antigen Fc fusions.

The protein was concentrated and filtered prior to loading on a HiLoad Superdex 200 column (GE Healthcare) equilibrated with 20mM Histidine, 140mM NaCl solution of pH 6.0.

The protein concentration of purified antibodies was determined by measuring the OD at 280 nm, using the molar extinction coefficient calculated on the basis of the amino acid sequence. Purity and molecular weight of the antibodies were analyzed by CE-SDS in the presence and absence of a reducing agent (Invitrogen, USA) using a LabChipGXII (Caliper). The aggregate content of antibody samples was analyzed using a TSKgel G3000 SW XL analytical size-exclusion column (Tosoh) equilibrated in a 25 mM K₂HPO₄, 125 mM NaCl, 200mM L-Arginine Monohydrocloride, 0.02 % (w/v) NaN₃, pH 6.7 running buffer at 25°C.

**Table 14** summarizes the yield and final content of the anti-Ox40 P329G LALA IgG1 antibodies.

**Table 14: Biochemical analysis of anti-Ox40 P329G LALA IgG1 clones**

| Clone | Yield [mg/l] | Monomer [%] | CE-SDS (non red) | CE-SDS (red) |
|---|---|---|---|---|
| 8H9 P329GLALA IgG1 | 7 | 100 | 1.2% (176kDa) | 66.9% (54kDa) |
| | | | 96.1% (158kDa) | 28.9% (25kDa) |
| | | | 1.3% (142kDa) | |
| 49B4 P329GLALA IgG1 | 7.5 | 100 | 99% (163kDa) | 81% (61.7kDa) |
| | | | 1% (149kDa) | 18% (28.9kDa) |
| 1G4 P329GLALA IgG1 | 1 | 100 | 98.9% | 80% (63.4kDa) |
| | | | (167.4kDa) | 19% (28.9kDa) |
| | | | 1.1% (151kDa) | |
| 20B7 P329GLALA IgG1 | 17 | 93 | 97.9% (174kDa) | 79.8% (65.4kDa) |
| | | | | 19.9% (29.5kDa) |
| CLC-563 P329GLALA IgG1 | 6.2 | 100 | 97.7% (160kDa) | 77.7% (60kDa) |
| | | | | 19.8% (26.4kDa) |
| CLC-564 P329GLALA IgG1 | 13.5 | 100 | 98.4% (155kDa) | 79.3% (60.1kDa) |
| | | | | 19.8% (26.5kDa) |
| 17A9 P329GLALA IgG1 | 7.5 | 100 | 98.6% (175kDa) | 74.1% (61kDa) |
| | | | 1.4% (153kDa) | 25.5% (38kDa) |

### Example 2

### Characterization of anti-OX40 antibodies

### 2.1 Binding on human OX40

### 2.1.1 Surface plasmon resonance (avidity + affinity)

Binding of phage-derived OX40-specific antibodies to the recombinant OX40 Fc(kih) was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany).

In the same experiment, the species selectivity and the avidity of the interaction between the phage display derived anti- OX40 clones 8H9, 49B4, 1G4, 20B7, CLC-563, CLC-564 and 17A9 (all human IgG1 P329GLALA), and recombinant OX40 (human, cyno and murine) was determined. Biotinylated human, cynomolgus and murine OX40 Fc(kih) were directly coupled to different flow cells of a streptavidin (SA) sensor chip. Immobilization levels up to 600 resonance units (RU) were used.

Phage display derived anti-OX40 human IgG1 P329GLALA antibodies were passed at a concentration range from 2 to 500 nM (3-fold dilution) with a flow of 30 µL/minute through the flow cells over 120 seconds. Complex dissociation was monitored for 210 seconds. Bulk refractive index differences were corrected for by subtracting the response obtained in a reference flow cell, where no protein was immobilized.

Kinetic constants were derived using the Biacore T200 Evaluation Software (vAA, Biacore AB, Uppsala/Sweden), to fit rate equations for 1:1 Langmuir binding by numerical integration and used to estimate qualitatively the avidity (**Table 16**).

In the same experiment, the affinities of the interaction between phage display derived antibodies 8H9, 49B4, 1G4, 20B7, CLC-563 and CLC-564 (human IgG1 P329GLALA) to recombinant OX40 were determined. For this purpose, the ectodomain of human or murine Ox40 was also subcloned in frame with an avi (GLNDIFEAQKIEWHE) and a hexahistidine tag (for the sequences see **Table 15**) or obtained by cleavage with AcTEV protease and removal of Fc by chromatographical method.

**Table 15: Nucleotide and amino acid sequences of monomeric human and murine Ox40 His tag**

| SEQ ID NO: | Antigen | Sequence |
|---|---|---|
| 179 | human OX40 His | LHCVGDTYPSNDRCCHECRPGNGMVSRCSRSQNTVCRPC GPGFYNDVVSSKPCKPCTWCNLRSGSERKQLCTATQDTV CRCRAGTQPLDSYKPGVDCAPCPPGHFSPGDNQACKPWT NCTLAGKHTLQPASNSSDAICEDRDPPATQPQETQGPPAR PITVQPTEAWPRTSQGPSTRPVEVPGGRAVDEQLYFQGGS GLNDIFEAQKIEWHEARAHHHHHH |
| 180 | murine OX40 His | TARRLNCVKHTYPSGHKCCRECQPGHGMVSRCDHTRDT LCHPCETGFYNEAVNYDTCKQCTQCNHRSGSELKQNCTP TQDTVCRCRPGTQPRQDSGYKLGVDCVPCPPGHFSPGNN QACKPWTNCTLSGKQTRHPASDSLDAVCEDRSLLATLL WETQRPTFRPTTVQSTTVWPRTSELPSPPTLVTPEGPVDE QLYFQGGSGLNDIFEAQKIEWHEARAHHHHHH |

Protein production was performed as described above for the Fc-fusion protein. Secreted proteins were purified from cell culture supernatants by chelating chromatography, followed by size exclusion chromatography.

The first chromatographic step was performed on a NiNTA Superflow Cartridge (5ml, Qiagen) equilibrated in 20mM sodium phosphate, 500nM sodium chloride, pH7.4. Elution was performed by applying a gradient over 12 column volume from 5% to 45% of elution buffer (20mM sodium phosphate, 500nM sodium chloride, 500mM Imidazole, pH7.4).

The protein was concentrated and filtered prior to loading on a HiLoad Superdex 75 column (GE Healthcare) equilibrated with 2mM MOPS, 150mM sodium chloride, 0.02% (w/v) sodium azide solution of pH 7.4.

Affinity determination was performed using two setups.

Setup 1) Anti-human Fab antibody (Biacore, Freiburg/Germany) was directly coupled on a CM5 chip at pH 5.0 using the standard amine coupling kit (Biacore, Freiburg/Germany). The immobilization level was approximately 9000 RU. Phage display derived antibodies to OX40 were captured for 60 seconds at concentrations of 25 to 50 nM. Recombinant human OX40 Fc(kih) was passed at a concentration range from 4 to 1000 nM with a flow of 30 µL/minutes through the flow cells over 120 seconds. The dissociation was monitored for 120 seconds. Bulk refractive index differences were corrected for by subtracting the response obtained on reference flow cell. Here, the antigens were flown over a surface with immobilized anti-human Fab antibody but on which HBS-EP has been injected rather than the antibodies.

Setup 2) Anti-human Fc antibody (Biacore, Freiburg/Germany) was directly coupled on a CM5 chip at pH 5.0 using the standard amine coupling kit (Biacore, Freiburg/Germany). The immobilization level was approximately 8000 RU. Phage display derived antibodies to Ox40 were captured for 60 seconds at concentrations of 20 nM. Recombinant human Ox40 avi His was passed at a concentration range from 2.3 to 600 nM with a flow of 30 µL/minutes through the flow cells over 120 seconds. The dissociation was monitored for 120 seconds. Bulk refractive index differences were corrected for by subtracting the response obtained on reference flow cell. Here, the antigens were flown over a surface with immobilized anti-human Fab antibody but on which HBS-EP has been injected rather than the antibodies.

Kinetic constants were derived using the Biacore T200 Evaluation Software (vAA, Biacore AB, Uppsala/Sweden), to fit rate equations for 1:1 Langmuir binding by numerical integration.

Clones 49B4, 1G4 and CLC-564 bind human Ox40 Fc(kih) with a lower affinity than clones 8H9, 20B7 and CLC-563.

Affinity constants for the interaction between anti-OX40 P329GLALA IgG1 and human OX40 Fc(kih) were determined by fitting to a 1:1 Langmuir binding.

**Table 16: Binding of anti-OX40 antibodies to recombinant human OX40**

| Clone | Recombinant human OX40 (avidity format) | Recombinant human OX40 | | | Recombinant human OX40 His | | |
|---|---|---|---|---|---|---|---|
| | | Fc(kih) (affinity format) | | | (affinity format) | | |
| | | ka (1/Ms) | kd (1/s) | KD (M) | ka (1/Ms) | kd (1/s) | KD (M) |
| 8H9 | ++++ | 1.6E+05 | 4.5E-03 | 2.8E-08 | 6.5E+04 | 2.0E-03 | 3.1E-08 |
| 49B4 | ++ | 2.5E+05 | 1.3E-01 | 5.1E-07 | 1.4E+06 | 6.7E-01 | 4.6E-07 |
| 1G4 | ++ | 3.0E+05 | 8.4E-08 | 2.8E-07 | 2.3E+06 | 5.7E-01 | 2.5E-07 |
| 20B7 | +++ | 3.2E+04 | 1.3E-03 | 4.2E-08 | 1.2E+05 | 6.6E-04 | 5.6E-09 |
| CLC-563 | ++ | 3.6E+04 | 3.2E-03 | 8.9E-08 | 4.0E+04 | 3.6E-03 | 8.9E-08 |
| CLC-564 | ++++ | 3.2E+04 | 4.2E-03 | 1.3E-07 | 3.8E+05 | 5.3E-03 | 1.4E-08 |

### 2.1.2 Binding to human Ox40 expressing cells: naïve and activated human peripheral mononuclear blood leukocytes (PBMC)

Buffy coats were obtained from the Zurich blood donation center. To isolate fresh peripheral blood mononuclear cells (PBMCs) the buffy coat was diluted with the same volume of DPBS (Gibco by Life Technologies, Cat. No. 14190 326). 50 mL polypropylene centrifuge tubes (TPP, Cat.-No. 91050) were supplied with 15 mL Histopaque 1077 (SIGMA Life Science, Cat.-No. 10771, polysucrose and sodium diatrizoate, adjusted to a density of 1.077 g/mL) and the buffy coat solution was layered above the Histopaque 1077. The tubes were centrifuged for 30 min at 400 x g, room temperature and with low acceleration and no break. Afterwards the PBMCs were collected from the interface, washed three times with DPBS and resuspended in T cell medium consisting of RPMI 1640 medium (Gibco by Life Technology, Cat. No. 42401-042) supplied with 10 % Fetal Bovine Serum (FBS, Gibco by Life Technology, Cat. No. 16000-044, Lot 941273, gamma-irradiated, mycoplasma-free and heat inactivated at 56 °C for 35 min), 1 % (v/v) GlutaMAX I (GIBCO by Life Technologies, Cat. No. 35050 038), 1 mM Sodium-Pyruvat (SIGMA, Cat. No. S8636), 1 % (v/v) MEM non-essential amino acids (SIGMA, Cat.-No. M7145) and 50 µM β-Mercaptoethanol (SIGMA, M3148).

PBMCs were used directly after isolation (binding on naive human PBMCs) or they were stimulated to receive a strong human Ox40 expression on the cell surface of T cells (binding on activated human PBMCs). Therefore naive PBMCs were cultured for five days in T cell medium supplied with 200 U/mL Proleukin and 2 ug/mL PHA-L in 6-well tissue culture plate and then 1 day on pre-coated 6-well tissue culture plates [2 ug/mL anti-human CD3 (clone OKT3) and 2 ug/mL anti-human CD28 (clone CD28.2)] in T cell medium supplied with 200 U/mL Proleukin at 37 °C and 5% CO₂.

For detection of Ox40 naïve human PBMC and activated human PBMC were mixed. To enable distinction of naive from activated human PBMC resting cells were labeled prior to the binding assay using the eFluor670 cell proliferation dye (eBioscience, Cat.-No.65-0840-85).

For labeling cells were harvested, washed with pre-warmed (37°C) DPBS and adjusted to a cell density of 1 × 10⁷ cells/mL in DPBS. eFluor670 cell proliferation dye (eBioscience, Cat.-No.65-0840-85 ) was added to the suspension of naive human PBMC at a final concentration of 2.5 mM and a final cell density of 0.5 × 10⁷ cells/mL in DPBS. Cells were then incubated for 10 min at room temperature in the dark. To stop labeling reaction 2 mL FBS were added and cells were washed three times with T cell medium. A one to one mixture of 1 × 10⁵ naive, eFluor670 labeled human PBMC and unlabeled activated human PBMC were then added to each well of a round-bottom suspension cell 96-well plates (Greiner bio-one, Cellstar, Cat. No. 650185).

Plates were centrifuged 4 minutes with 400 x g and at 4 °C and supernatant was flicked off. Cell were washed once with 200 µL 4 °C cold FACS buffer (DPBS supplied with 2 % FBS, 5 mM EDTA pH8 (Amresco, Cat. No. E177) and 7.5 mM Sodium azide (Sigma-Aldrich S2002)). Cells were incubated in 50 µL/well of 4 °C cold FACS buffer containing titrated anti -Ox40 antibody constructs for 120 minutes at 4 °C. Plates were washed four times with 200 µL/well 4 °C FACS buffer to remove unbound construct.

Cells were stained for 30 minutes at 4°C in the dark in 25 µL/well 4 °C cold FACS buffer containing fluorescently labeled anti-human CD4 (clone RPA-T4, mouse IgG1 k, BioLegend, Cat.-No. 300532), anti-human CD8 (clone RPa-T8, mouse IgG1k, BioLegend, Cat.-No. 3010441), anti-human CD45 (clone HI30, mouse IgG1k, BioLegend, Cat.-No. 304028), and Fluorescein isothiocyanate (FITC)-conjugated AffiniPure anti-human IgG Fcy-fragment-specific goat IgG F(ab')₂ fragment (Jackson ImmunoResearch, Cat.-No. 109-096-098).

Plates where washed twice with 200 µL/well 4 °C FACS buffer, were finally resuspended in 80 µL/well FACS-buffer containing 0.2 µg/mL DAPI (Santa Cruz Biotec, Cat. No. Sc-3598) and acquired the same day using 5-laser LSR-Fortessa (BD Bioscience with DIVA software).

As shown in **Figure 2**, no antibody construct specific for Ox40 bound to resting human CD4+ T-cells or CD8+ T-cells, which do not express Ox40. In contrast, all constructs bound to activated CD8+ or *CD4+* T-cells, which do express Ox40. Binding to CD4+ T-cells was much stronger than that to CD8+ T cells. Activated human CD8+ T cells do express only a fraction of the Ox40 levels detected on activated CD4+ T cells. The difference is donor as well as time dependent. The analyzed anti-Ox40 clones varied in their binding strength. The EC₅₀ values are shown in **Table 17**. For further evaluation of bivalent and monovalent FAP targeted constructs clones with high (8H9) and low (49B4/ 1G4) binding capacity were chosen.

**Table 17: EC₅₀ values of binding to activated human CD4 T cells**

| Clone | EC₅₀ [nM] |
|---|---|
| 8H9 | 0.59 |
| CLC563 | 1.59 |
| 20B7 | 1.64 |
| 49B4 | 4.19 |
| CLC-564 | 4.63 |
| 1G4 | n.a. |

### 2.2 Binding on murine OX40

### 2.2.1 Surface plasmon resonance (avidity + affinity)

Binding of the phage-derived OX40 specific antibody 20B7 to recombinant murine OX40 Fc(kih) was assessed by surface plasmon resonance as described above for human OX40 Fc(kih) (see Example 2.1.1). Kinetic constants were derived using the Biacore T200 Evaluation Software (vAA, Biacore AB, Uppsala/Sweden), to fit rate equations for 1:1 Langmuir binding by numerical integration and used to estimate qualitatively the avidity **(Table 18**).

For affinity determination, due to an unspecific interaction of the Fc fusion protein to the reference flow cell, murine Ox40 His (see Example 2.1.2) or Ox40 Fc(kih) cleaved with AcTEV protease was used. Anti-human Fc antibody (Biacore, Freiburg/Germany) was directly coupled on a CM5 chip at pH 5.0 using the standard amine coupling kit (Biacore, Freiburg/Germany). The immobilization level was approximately 8000 RU. Phage display derived antibodies to OX40 were captured for 60 seconds at concentrations of 25 nM. Recombinant murine OX40 (cleaved by AcTEV digestion following the distributor instruction) was passed at a concentration range from 4.1 to 1000 nM with a flow of 30 µL/minutes through the flow cells over 120 seconds. The dissociation was monitored for 120 seconds. Bulk refractive index differences were corrected for by subtracting the response obtained on reference flow cell. Here, the antigens were flown over a surface with immobilized anti-human Fab antibody but on which HBS-EP has been injected rather than the antibodies.

Kinetic constants were derived using the Biacore T200 Evaluation Software (vAA, Biacore AB, Uppsala/Sweden), to fit rate equations for 1:1 Langmuir binding by numerical integration. It was shown that clone 20B7 binds murine OX40 **(Table 18**).

Affinity constants of interaction between anti-OX40 P329GLALA IgG1 molecules and murine OX40 were derived using the Biacore T200 Evaluation Software (vAA, Biacore AB, Uppsala/Sweden), to fit rate equations for 1:1 Langmuir binding by numerical integration.

**Table 18: Binding of anti-Ox40 antibody 20B7 to murine OX40**

| Clone | Origin | Recombinant murine OX40 (avidity format) | Recombinant murine OX40 (affinity format) | | |
|---|---|---|---|---|---|
| | | | ka (1/Ms) | kd (1/s) | KD (M) |
| 20B7 | Phage display | ++ | 4.9E+04 | 1.8E-02 | 3.6E-07 |

### 2.2.2 Binding to mouse OX40 expressing cells: naïve and activated mouse splenocytes (selected clones)

Mouse spleens were collected in 3 mL PBS and a single cell suspension was generated using the gentle MACS tubes (Miltenyi Biotec Cat.-No. 130-096-334) and gentleMACS Octo Dissociator (Miltenyi Biotec). Afterwards splenocytes were filtered through a 30 µm pre-separation filters (Miltenyi Biotec Cat.-No. 130-041-407) and centrifuged for 7 min at 350 x g and 4°C. Supernatant was aspirated and cells were resuspended in RPMI 1640 medium supplied with 10 % (v/v) FBS, 1 % (v/v) GlutaMAX-I, 1 mM Sodium-Pyruvate, 1 % (v/v) MEM non-essential amino acids, 50 µM β-Mercaptoethanol and 10 % Penicillin-Streptomycin (SIGMA, Cat.-No. P4333). 10⁶ cells/mL were cultured for 3 days in a 6-well tissue culture plate coated with 10 µg/mL anti-mouse CD3ε Armenian hamster IgG (clone 145-2C11, BioLegend, Cat.-No. 100331) and 2 µg/mL anti-mouse CD28 Syrian hamster IgG (clone 37.51, BioLegend, Cat.-No. 102102). Activated or fresh mouse splenocytes were harvested, washed in DPBS, counted and 0.1 × 10⁶ cells were transferred to each well of a 96 U-bottom non-tissue culture treated plate. Cells were washed with DPBS and stained in 50 uL FACS buffer containing different concentration of anti-OX40 human IgG1 P329GLALA antibodies (selected binders only). Cells were incubated for 120 min at 4°C. Then cells were washed twice with FACS buffer and stained in 25 µL/well FACS buffer containing anti-mouse CD8b rat IgG2bκ-APC-Cy7 (BioLegend, Cat.-No. 100714, clone53-6.7), anti-mouse CD4 rat IgG2bκ-PE-Cy7 (BioLegend, Cat.-No. 100422, clone GK1.5) and FITC-conjugated AffiniPure anti-human IgG Fcy-fragment-specific goat IgG F(ab')₂ fragment (Jackson ImmunoResearch, Cat.-No. 109-096-098) for 30 min at 4°C. Plates where washed twice with 200 µL/well 4 °C FACS buffer, cells were finally resuspended in 80 µL/well FACS-buffer containing 0.2 µg/mL DAPI (Santa Cruz Biotec, Cat. No. Sc-3598) and acquired the same day using 5-laser LSR-Fortessa (BD Bioscience with DIVA software).

As shown in **Figure 3**, only clone 20B7 and the well characterized mouse specific benchmark antibody OX86 showed binding to activated mouse CD4+ and CD8+ T cells. No binding was observed on resting mouse splenocytes.

### 2.3 Binding on cynomolgus OX40

To test the reactivity of selected anti-OX40 binders with cynomolgus cells, PBMC of healthy Macaca fascicularis were isolated from heparinized blood using density gradient centrifugation as described for human cells with minor modifications. Cynomolgus PBMC were isolated with density gradient centrifugation from heparinized fresh blood using lymphoprep medium (90% v/v, Axon Lab, Cat. No. 1114545) diluted with DPBS. Centrifugation was performed at 520xg, without brake at room temperature for 30 minutes. Adjacent centrifugation at 150xg at room temperature for 15 minutes was performed to reduce platelets count followed by several centrifugation steps with 400xg at room temperature for 10 minutes to wash PBMC with sterile DPBS. PBMCs were stimulated to receive a strong Ox40 expression on the cell surface of T cells (binding on activated cynomolgus PBMCs). Therefore naive PBMCs were cultured for 72 hrs on pre-coated 12-well tissue culture plates [10 ug/mL cynomolgus cross-reactive anti-human CD3 (clone clone SP34)] and 2 ug/mL cynomolgus cross-reactive anti-human CD28 (clone CD28.2)] in T cell medium supplied with 200 U/mL Proleukin at 37 °C and 5% CO₂.

0.5 × 10⁵ activated cynomolgus PBMC were then added to each well of a round-bottom suspension cell 96-well plates (greiner bio-one, cellstar, Cat. No. 650185). Cell were washed once with 200 µL 4 °C cold FACS buffer and were incubated in 50 µL/well of 4 °C cold FACS containing titrated anti -Ox40 antibody constructs for 120 minutes at 4 °C. Then, plates were washed four times with 200 µL/well 4 °C FACS buffer. Cells were resuspended in 25 µL/well 4 °C cold FACS buffer containing fluorescently labeled, cynomolgus cross-reactive anti-human CD4 (clone OKT-4, mouse IgG1 k, BD, Cat.-No. 317428), anti-human CD8 (clone HIT8a, mouse IgG1k, BD, Cat.-No. 555369) and FITC-conjugated AffiniPure anti-human IgG Fcγ-fragment-specific goat IgG F(ab')₂ fragment (Jackson ImmunoResearch, Cat.-No. 109-096-098) and incubated for 30 minutes at 4 °C in the dark. Plates where washed twice with 200 µL/well 4 °C FACS buffer, cells were finally resuspended in 80 µL/well FACS-buffer containing 0.2 µg/mL DAPI (Santa Cruz Biotec, Cat. No. Sc-3598) and acquired the same day using 5-laser LSR-Fortessa (BD Bioscience with DIVA software).

As shown in **Figure 4****,** most constructs bound to activated CD4⁺ cynomolgus T-cells. Binding to CD4⁺ T-cells was much stronger than that to CD8⁺ T cells. Expression levels for OX40 are depending on kinetic and strength of stimulation and were optimized for CD4⁺ cynomolgus T cells but not for CD8+ cynomolgus T cells, so that only little OX40 expression was induced on CD8⁺ T cells. The analyzed anti-OX40 clones varied in their binding strength. The EC₅₀ values are shown in **Table 19**. Due to untypical curve fit no EC₅₀ value could be calculated for clones 8H9, 49B4, 21H4.

**Table 19: EC₅₀ values of binding to activated cynomolgus CD4 T cells**

| Clone | EC₅₀ [nM] |
|---|---|
| 8H9 | n.d. |
| CLC563 | 1.41 |
| 20B7 | 1.52 |
| 49B4 | n.d. |
| CLC-564 | 3.50 |
| 1G4 | 48.20 |

### 2.3.1 Surface plasmon resonance (avidity + affinity)

Binding of phage-derived OX40-specific antibodies (all human IgG1 P329GLALA) to the recombinant cynomolgus OX40 Fc(kih) was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany).

Biotinylated cynomolgus OX40 Fc(kih) was directly coupled to different flow cells of a streptavidin (SA) sensor chip. Immobilization levels up to 800 resonance units (RU) were used.

Phage display derived anti-OX40 human IgG1 P329GLALA antibodies were passed at a concentration range from 2 to 500 nM (3-fold dilution) with a flow of 30 µL/minute through the flow cells over 120 seconds. Complex dissociation was monitored for 210 seconds. Bulk refractive index differences were corrected for by subtracting the response obtained in a reference flow cell, where no protein was immobilized.

Kinetic constants were derived using the Biacore T200 Evaluation Software (vAA, Biacore AB, Uppsala/Sweden), to fit rate equations for 1:1 Langmuir binding by numerical integration and used to estimate qualitatively the avidity (**Table 20**).

In the same experiment, the affinities of the interaction between phage display derived antibodies (human IgG1 P329GLALA) to recombinant cynomolgus OX40 Fc(kih) were determined. Anti-human Fab antibody (Biacore, Freiburg/Germany) was directly coupled on a CM5 chip at pH 5.0 using the standard amine coupling kit (Biacore, Freiburg/Germany). The immobilization level was approximately 9000 RU. Phage display derived antibodies to Ox40 were captured for 60 seconds at concentrations of 25 to 50 nM. Recombinant cynomolgus Ox40 Fc(kih) was passed at a concentration range from 4 to 1000 nM with a flow of 30 µL/minutes through the flow cells over 120 seconds. The dissociation was monitored for 120 seconds. Bulk refractive index differences were corrected for by subtracting the response obtained on reference flow cell. Here, the antigens were flown over a surface with immobilized anti-human Fab antibody but on which HBS-EP has been injected rather than the antibodies.

Kinetic constants were derived using the Biacore T200 Evaluation Software (vAA, Biacore AB, Uppsala/Sweden), to fit rate equations for 1:1 Langmuir binding by numerical integration (**Table 20**).

Clones 49B4, 1G4 and CLC-564 bind cynomolgus OX40 Fc(kih) with a lower affinity than clones 8H9, 20B7 and CLC-563.

Affinity constants of interaction between anti-OX40 P329GLALA IgG1 and cynomolgus OX40 Fc(kih) were derived using the Biacore T100 Evaluation Software (vAA, Biacore AB, Uppsala/Sweden), to fit rate equations for 1:1 Langmuir binding by numerical integration.

**Table 20: Binding of anti-OX40 antibodies to recombinant cynomolgus OX40 Fc(kih)**

| Clone | Origin | Recombinant cynomolgus OX40 (avidity format) | Recombinant cynomolgus OX40 (affinity format) | | |
|---|---|---|---|---|---|
| | | | ka (1/Ms) | kd (1/s) | KD (M) |
| 8H9 | Phage display | ++++ | 1.4E+05 | 9.6E-02 | 6.7E-07 |
| 20B7 | Phage display | +++ | 1.57E+04 | 1.66E-02 | 1.1E-06 |
| 49B4 | Phage display | ++ | 1.1E+05 | 3.8E-02 | 3.5E-07 |
| 1G4 | Phage display | + | Too low to be detected | | |
| CLC-563 | Phage display | +++ | 2.8E+04 | 6.9E-04 | 2.5E-08 |
| CLC-564 | Phage display | +++ | 2.1E+04 | 7.2E-04 | 3.4E-08 |

### 2.3.2 Binding on cynomolgus OX40 expressing cells: activated cynomolgus peripheral mononuclear blood leukocytes (PBMC)

### Binding to Ox40 negative tumor cells

The lack of binding to Ox40 negative tumor cells was tested using WM266-4 cells (ATCC CRL-1676) and U-87 MG (ATCC HTB-14) tumor cells. To allow separation of both tumor cells, WM266-4 cells were pre-labeled with PKH-26 Red Fluorescence Cell linker Kit (Sigma, Cat.-No. PKH26GL). Cells were harvested and washed three times with RPMI 1640 medium. Pellet was stained for 5 minutes at room temperature in the dark at a final cell density of 1 × 10⁷ cells in freshly prepared PKH26-Red-stain solution (final concentration [1 nM] in provided diluent C). Excess FBS was added to stop labeling reaction and cell were washed four times with RPMI 1640 medium supplemented with 10 % (v/v) FBS, 1 % (v/v) GlutaMAX-I to remove excess dye.

A mixture of 5 × 10⁴ PKH26 labeled WM266-4 cells and unlabeled U-87 MG cells in DPBS were added to each well of a round-bottom suspension cell 96-well plates. Plates were centrifuged 4 minutes, 400 x g, 4 °C and supernatant were flicked off. Cells were washed once with 200 µL DPBS and pellets were resuspended by a short and gentle vortex. All samples were resuspended in 50 µL/well of 4 °C cold FACS buffer containing titrated concentrations of anti - Ox40 human IgG1 P329GLALA antibody constructs for 120 minutes at 4 °C. Plates were washed four times with 200 µL/well 4 °C FACS buffer. Cells were resuspended in 25 µL/well 4 °C cold FACS buffer containing FITC-conjugated AffiniPure anti-human IgG Fcγ-fragment-specific goat IgG F(ab')₂ fragment (Jackson ImmunoResearch, Cat.-No. 109-096-098) and incubated for 30 minutes at 4 °C in the dark. Plates where washed twice with 200 µL/well 4 °C FACS buffer, were finally resuspended in 80 µL/well FACS-buffer containing 0.2 µg/mL DAPI (Santa Cruz Biotec, Cat. No. Sc-3598) and acquired the same day using 5-laser LSR-Fortessa (BD Bioscience with DIVA software).

As shown in **Figure 5**, no antibody construct specific for OX40 bound to OX40 negative human tumor cells WM266-4 and U-78 MG.

### 2.4 Ligand blocking property

To determine the capacity of OX40-specific human IgG1 P329GLALA antibody molecules to interfere with OX40/OX40-ligand interactions human OX40 ligand (R&D systems) was used. Due to the low affinity of the interaction between OX40 and OX40 ligand, a dimeric human OX40 Fc fusion with a C-terminal Ha tag was prepared (**Figure 1B**). The nucleotide and amino acid sequences of this dimeric human Ox40 Fc fusion molecule are shown in **Table 21**. Production and purification were performed as described for the monomeric OX40 Fc(kih) in Example 1.1.

**Table 21: cDNA and Amino acid sequences of dimeric human OX40 Fc fusion molecule (composed by 2 Fc chains)**

| SEQ ID NO: | Antigen | Sequence |
|---|---|---|
| 181 | Nucleotide sequence dimeric human OX40 antigen Fc | CTGCACTGCGTGGGCGACACCTACCCCAGCAACGACC GGTGCTGCCACGAGTGCAGACCCGGCAACGGCATGGT GTCCCGGTGCAGCCGGTCCCAGAACACCGTGTGCAGA CCTTGCGGCCCTGGCTTCTACAACGACGTGGTGTCCAG CAAGCCCTGCAAGCCTTGTACCTGGTGCAACCTGCGG AGCGGCAGCGAGCGGAAGCAGCTGTGTACCGCCACCC AGGATACCGTGTGCCGGTGTAGAGCCGGCACCCAGCC CCTGGACAGCTACAAACCCGGCGTGGACTGCGCCCCT TGCCCTCCTGGCCACTTCAGCCCTGGCGACAACCAGG CCTGCAAGCCTTGGACCAACTGCACCCTGGCCGGCAA GCACACCCTGCAGCCCGCCAGCAATAGCAGCGACGCC ATCTGCGAGGACCGGGATCCTCCTGCCACCCAGCCTC AGGAAACCCAGGGCCCTCCCGCCAGACCCATCACCGT GCAGCCTACAGAGGCCTGGCCCAGAACCAGCCAGGGG CCTAGCACCAGACCCGTGGAAGTGCCTGGCGGCAGAG |
| | | CCGTCGACGAACAGTTATATTTCAGGCGGCTCACCC AAATCTGCAGACAAAACTCACACATGCCCACCGTGCC CAGCACCTGAACTCCTGGGGGGACCGTCAGTCTTCCTC TTCCCCCCAAAACCCAAGGACACCCTCATGATCTCCCG GACCCCTGAGGTCACATGCGTGGTGGTGGACGTGAGC CACGAAGACCCTGAGGTCAAGTTCAACTGGTACGTGG ACGGCGTGGAGGTGCATAATGCCAAGACAAAGCCGCG GGAGGAGCAGTACAACAGCACGTACCGTGTGGTCAGC GTCCTCACCGTCCTGCACCAGGACTGGCTGAATGGCA AGGAGTACAAGTGCAAGGTCTCCAACAAAGCCCTCGG CGCCCCCATCGAGAAAACCATCTCCAAAGCCAAAGGG CAGCCCCGAGAACCACAGGTGTACACCCTGCCCCCAT CCCGGGATGAGCTGACCAAGAACCAGGTCAGCCTGAC CTGCCTGGTCAAAGGCTTCTATCCCAGCGACATCGCCG TGGAGTGGGAGAGCAATGGGCAGCCGGAGAACAACT ACAAGACCACGCCTCCCGTGCTGGACTCCGACGGCTC CTTCTTCCTCTACAGCAAGCTCACCGTGGACAAGAGC AGGTGGCAGCAGGGGAACGTCTTCTCATGCTCCGTGA TGCATGAGGCTCTGCACAACCACTACACGCAGAAGAG CCTCTCCCTGTCTCCGGGTAAATCCGGCTACCCATACG ATGTTCCAGATTACGCT |
| 182 | dimeric human OX40 antigen Fc | LHCVGDTYPSNDRCCHECRPGNGMVSRCSRSQNTVCRP CGPGFYNDVVSSKPCKPCTWCNLRSGSERKQLCTATQD TVCRCRAGTQPLDSYKPGVDCAPCPPGHFSPGDNQACKP WTNCTLAGKHTLQPASNSSDAICEDRDPPATQPQETQGP PARPITVQPTEAWPRTSQGPSTRPVEVPGGRAVDEQLYF QGGSPKSADKTHTCPPCPAPELLGGPSVFLFPPKPKDTLM ISRTPEVTCVVVDVSHEDPEVKFNWYVDGVEVHNAKTK PREEQYNSTYRVVSVLTVLHQDWLNGKEYKCKVSNKA LGAPIEKTISKAKGQPREPQVYTLPPSRDELTKNQVSLTC LVKGFYPSDIAVEWESNGQPENNYKTTPPVLDSDGSFFL YSKLTVDKSRWQQGNVFSCSVMHEALHNHYTQKSLSLS PGKSGYPYDVPDYA |

Human OX40 ligand (R&D systems) was directly coupled to two flow cells of a CM5 chip at approximately 2500 RU by pH 5.0 using the standard amine coupling kit (Biacore, Freiburg/Germany). Recombinant human Ox40 Fc was passed on the second flow cell at a concentration of 200 nM with a flow of 30 µL/minute over 90 seconds. The dissociation was omitted and the phage derived anti-Ox40 human IgG1P329LALA was passed on both flow cells at a concentration of 500 nM with a flow of 30 µL/ minute over 90 seconds. The dissociation was monitored for 60 seconds. Bulk refractive index differences were corrected for by subtracting the response obtained on reference flow cell. Here, the antibodies were flown over a surface with immobilized human OX40 ligand but on which HBS-EP has been injected instead of recombinant human OX40 Fc. **Figure 1C** shows the design of the experiment.

The phage-derived clone 20B7 bound to the complex of human OX40 with its OX40 ligand (**Table 22,** **Figure 6**). Thus, this antibody does not compete with the ligand for binding to human OX40 and is therefore termed "non-ligand blocking". On the contrary, clones 8H9, 1G4, 49B4, CLC-563 and CLC-564 did not bind to human OX40 in complex with its ligand and are therefore termed "ligand blocking".

**Table 22: Ligand binding property of the anti-OX40 clones determined by surface plasmon resonance**

| Clone | Origin | First injection | Second injection (anti-Ox40 clone) | Ligand blocking |
|---|---|---|---|---|
| 8H9 | Phage display | human OX40 Fc | Not binding | YES |
| 20B7 | Phage display | human OX40 Fc | Binding | NO |
| 1G4 | Phage display | human OX40 Fc | Not binding | YES |
| 49B4 | Phage display | human OX40 Fc | Not binding | YES |
| CLC-564 | Phage display | human OX40 Fc | Not binding | YES |
| CLC-564 | Phage display | human OX40 Fc | Not binding | YES |

### Example 3

### Functional properties of anti-human OX40 binding clones

### 3.1 HeLa cells expressing human OX40 and reporter gene NF-KB-luciferase

Agonstic binding of OX40 to its ligand induces downstream signaling via activation of nuclear factor kappa B (NFκB) (A. D. Weinberg et al., J. Leukoc. Biol. 2004, 75(6), 962-972). The recombinant reporter cell line HeLa_hOx40_NFkB_Luc1 was generated to express human Ox40 on its surface. Additionally, it harbors a reporter plasmid containing the luciferase gene under the control of an NFκB-sensitive enhancer segment. Ox40 triggering induces dose-dependent activation of NFκB, which translocates in the nucleus, where it binds on the NPκB sensitive enhancer of the reporter plasmid to increase expression of the luciferase protein. Luciferase catalyzes luciferin-oxidation resulting in oxyluciferin which emits light. This can be quantified by a luminometer. The scope of one experiment was to test the capacity of the various anti-Ox40 binders in a P329GLALA huIgG1 format to induce NPκB activation in HeLa_hOx40_NFκB_Luc1 reporter cells.

Adherent HeLa_hOx40_NFκB_Luc1 cells were harvested using cell dissociation buffer (Invitrogen, Cat.-No. 13151-014) for 10 minutes at 37 °C. Cells were washed once with DPBS and were adjusted to a cell density of 2×10⁵ in assay media comprising of MEM (Invitrogen, Cat.-No. 22561-021), 10 % (v/v) heat-inactivated FBS, 1 mM Sodium-Pyruvat and 1% (v/v) non-essential amino acids. Cells were seeded in a density of 0.3^{∗}10⁵ cells per well in a sterile white 96-well flat bottom tissue culture plate with lid (greiner bio-one, Cat. No. 655083) and kept over night at 37 °C and 5% CO₂ in an incubator (Hera Cell 150).

The next day, HeLa_hOX40_NFκB_Luc1 were stimulated for 6 hours adding assay medium containing various titrated anti-OX40 binders in a P329GLALA huIgG1 format. For testing the effect of hyper-crosslinking on anti-OX40 antibodies, 50 µL/well of medium containing secondary antibody anti-human IgG Fcy-fragment-specific goat IgG F(ab')₂ fragment (Jackson ImmunoResearch, 109-006-098) were added in a 1:2 ratio (2 times more secondary antibody than the primary single anti-OX40 P329GLALA huIgG1). After incubation, supernatant was aspirated and plates washed two times with DPBS. Quantification of light emission was done using the luciferase 1000 assay system and the reporter lysis buffer (both Promega, Cat.-No. E4550 and Cat-No: E3971) according to manufacturer instructions. Briefly, cells were lysed for 10 minutes at -20 °C by addition of 30 uL per well 1x lysis buffer. Cells were thawed for 20 minutes at 37 °C before 90 uL per well provided luciferase assay reagent was added. Light emission was quantified immediately with a SpectraMax M5/M5e microplate reader (Molecular Devices, USA) using 500ms integration time, without any filter to collect all wavelengths. Emitted relative light units (URL) were corrected by basal luminescence of HeLa_hOX40_NFκB_Luc1 cells and were blotted against the logarithmic primary antibody concentration using Prism4 (GraphPad Software, USA). Curves were fitted using the inbuilt sigmoidal dose response.

As shown in **Figure 7**, a limited, dose dependent NFκB activation was induced already by addition of anti-OX40 P329GLALA huIgG1 antibodies (left side) to the reporter cell line. Hyper- crosslinking of anti-OX40 antibodies by anti-human IgG specific secondary antibodies strongly increased the induction of NFκB-mediated luciferase-activation in a concentration-dependent manner (right side). The EC₅₀ values of activation are summarized in **Table 23**.

**Table 23: EC₅₀ values of NFκB activation in the HeLa_hOx40_NFκB_luc1 reporter cell line co-incubated with anti-Ox40 binders (huIgG1 P329GLALA format) and secondary anti-human IgG Fcγ spec. antibodies**

| Clone | EC₅₀ [nM] |
|---|---|
| 8H9 | 0.66 |
| CLC563 | 1.69 |
| 20B7 | 2.27 |
| 49B4 | 2.42 |
| CLC-564 | 3.23 |
| 1G4 | 3.59 |

### 3.2 OX40 mediated costimulation of suboptimally TCR triggered pre-activated human CD4 T cells

Ligation of OX40 provides a synergistic co-stimulatory signal promoting division and survival of T-cells following suboptimal T-cell receptor (TCR) stimulation (M. Croft et al., Immunol. Rev. 2009, 229(1), 173-191). Additionally, production of several cytokines and surface expression of T-cell activation markers is increased (I. Gramaglia et al., J. Immunol. 1998, 161(12), 6510-6517; S. M. Jensen et al., Seminars in Oncology 2010, 37(5), 524-532).

To test agonistic properties of various anti-OX40 binders, pre-activated Ox40 positive CD4 T-cells were stimulated for 72 hours with a suboptimal concentration of plate-immobilized anti-CD3 antibodies in the presence of anti-OX40 antibodies, either in solution or immobilized on the plate surface. Effects on T-cell survival and proliferation were analyzed through monitoring of total cell counts and CFSE dilution in living cells by flow cytometry. Additionally, cells were co-stained with fluorescently-labeled antibodies against T-cell activation and differentiation markers, e.g. CD127, CD45RA, Tim-3, CD62L and OX40 itself.

Human PBMCs were isolated via ficoll density centrifugation and were simulated for three days with PHA-L [2 µg/mL] and Proleukin [200 U/mL] as described under Example 2.1.2. Cells were then labeled with CFSE at a cell density of 1×10⁶ cells/ mL with CFDA-SE (Sigma-Aldrich, Cat.-No. 2188) at a final concentration of [50 nM] for 10 minutes at 37 °C. Thereafter, cells were washed twice with excess DPBS containing FBS (10% v/v). Labeled cells were rested in T-cell media at 37 °C for 30 minutes. Thereafter, non-converted CFDA-SE was removed by two additional washing steps with DPBS. CD4 T-cell isolation from pre-activated CFSE-labeled human PBMC was performed using the MACS negative CD4 T-cell isolation kit (Miltenyi Biotec) according to manufacturer instructions.

Morris et al. showed that agonistic co-stimulation with conventional anti-Ox40 antibodies relied on surface immobilization (N. P. Morris et al., Mol. Immunol. 2007, 44(12), 3112-3121). Thus, goat anti-mouse Fcy-specific antibodies (Jackson ImmunoResearch, Cat.No. 111-500-5008) were coated to the surface of a 96 well U-bottom cell culture plate (Greiner Bio One) at a concentration of [2 µg/mL] in PBS over night at 4 °C in the presence (surface immobilized anti-OX40) or absence (anti-OX40 in solution) of goat anti-human Fcy-specific antibody (Jackson ImmunoResearch, Ca.No. 109-006-098). Thereafter, the plate surface was blocked with DPBS containing BSA (1 % v/w). All following incubation steps were done at 37 °C for 90 minutes in PBS containing BSA (1 % v/w). Between the incubation steps, plates were washed with DPBS.

Mouse anti-human CD3 antibody (clone OKT3, eBioscience, Ca.No. 16-0037-85, fixed concentration [3 ng/mL]) was captured in a subsequent incubation step via the surface coated anti-mouse Fcγ- specific antibodies. In one experiment titrated human anti-OX40 antibodies (human IgG₁ P329G LALA) were then immobilized on plate by an additional incubation step in DPBS. In a second experiment anti-OX40 antibodies were added during the activation assay directly to the media to plates not pre-coated with anti-human IgG Fc specific antibodies.

CFSE-labeled preactivated CD4+ T cells were added to the pre-coated plates at a cell density of 0.6^{∗}10⁵ cells per well in 200 µL T-cell media and cultured for 96 hours. Cells were stained with a combination of fluorochrome-labeled mouse anti-human Ox40 (clone BerACT35, Bioledgend, Ca.No. 35008), TIM-3 (clone F38-2E2, Biolegend, Ca.No.345008), CD127 (clone A019D5, Biolegend, Ca.No.351234), CD62L (clone DREG 56, Biolegend, Ca.No.304834) and CD45RA (clone HI100, BD Biosciences, Ca.No.555489) for 20 minutes at 4 °C in the dark. Plates where washed twice with 200 µL/well 4 °C FACS buffer, were finally resuspended in 80 µL/well FACS-buffer containing 0.2 µg/mL DAPI (Santa Cruz Biotec, Cat. No. Sc-3598) and acquired the same day using 5-laser LSR-Fortessa (BD Bioscience with DIVA software).

DAPI negative living cells were analyzed for decrease in median CFSE fluorescence as a marker for proliferation. The percentage of OX40 positive, CD62L low and TIM-3 positive T cells was monitored as a marker for T-cell activation. The expression of CD45RA and CD127 was analyzed to determine changes in maturation status of T cell, whereby CD45RA low CD127 low cells were categorized as effector T cells.

Co-stimulation with plate-immobilized antibodies strongly enhanced suboptimal stimulation of pre-activated human CD4 T cells with plate-immobilized anti-human CD3 in a dose dependent manner **(****Figure 8**). T-cells proliferated stronger, showed a more mature phenotype with a higher percentage of effector T cells and had higher percentages of CD62L low, Tim-3 positive and OX40 positive activated cells. Some clones (8H9, 20B7) out-competed the commercially available detection antibody in binding to cellular OX40. For those no EC₅₀ value calculation was possible and thus all EC₅₀ values for OX40 induction were excluded from overall EC₅₀ value calculation. Half-maximal changes in all other parameters of T-cell activation were achieved at concentrations ranging from 3 to 700 pM and are summarized in **Figure 9** and **Table 24**. No enhancement in suboptimal TCR stimulation was seen when anti-Ox40 antibodies were added in solution in the absence of surface immobilization **(****Figure 10**). This demonstrated again the strong dependency of Ox40 axis activation on hypercrosslinking of the OX40 receptor.

A correlation between the binding strength and the agonistic activity (bioactivity) of the anti-OX40 antibodies (hu IgG1 P329GLALA format) is shown in **Figure 11**. For most clones there was a direct correlation, however surprisingly two clones (49B4, 1G4) showed a much stronger bioactivity then was predicted from their binding strength.

**Table 24: EC₅₀ values of of rescuing suboptimal TCR stimulation with plate-immobilized anti-OX40 binders huIG1 P329GLALA format**

| Clone | EC₅₀ [nM] | SEM (+/-) |
|---|---|---|
| 8H9 | 0.003 | 0.001 |
| 20B7 | 0.090 | 0.015 |
| CLC-563 | 0.114 | 0.018 |
| CLC-564 | 0.202 | 0.053 |
| 49B4 | 0.591 | 0.237 |
| 1G4 | 0.697 | 0.278 |

### Example 4

### Generation of bispecific constructs targeting Ox40 and fibroblast activation protein (FAP) 4.1 Generation of bispecific bivalent antigen binding molecules targeting Ox40 and fibroblast activation protein (FAP) (2+2 format, Comparative Examples)

Bispecific agonistic Ox40 antibodies with bivalent binding for Ox40 and for FAP were prepared. The crossmab technology in accordance with International patent application No. WO 2010/145792 A1 was applied to reduce the formation of wrongly paired light chains.

The generation and preparation of the FAP binders is described in WO 2012/020006 A2.

In this example, a crossed Fab unit (VHCL) of the FAP binder 28H1 was C-terminally fused to the heavy chain of an anti-OX40 huIgG1 using a (G4S)₄ connector sequence. This heavy chain fusion was co-expressed with the light chain of the anti-OX40 and the corresponding FAP crossed light chain (VLCH1). The Pro329Gly, Leu234Ala and Leu235Ala mutations have been introduced in the constant region of the heavy chains to abrogate binding to Fc gamma receptors according to the method described in International Patent Appl. Publ. No. WO 2012/130831 A1. The resulting bispecific, bivalent construct is depicted in **Figure 12A**.

**Table 25** shows, respectively, the nucleotide and amino acid sequences of mature bispecific, bivalent anti-OX40/anti-FAP human IgG1 P329GLALA antibodies.

**Table 25: Sequences of bispecific, bivalent anti-OX40/anti-FAP human IgG1 P329GLALA antigen binding molecules**

| SEQ ID NO: | Description | Sequence |
|---|---|---|
| 183 | (8H9) VHCH1-Heavy chain-(28H1) VHCL (nucleotide sequence) | CAGGTGCAATTGGTGCAGTCTGGGGCTGAGGTGAAGAAG CCTGGGTCCTCGGTGAAGGTCTCCTGCAAGGCCTCCGGAG GCACATTCAGCAGCTACGCTATAAGCTGGGTGCGACAGG CCCCTGGACAAGGGCTCGAGTGGATGGGAGGGATCATCC CTATCTTTGGTACAGCAAACTACGCACAGAAGTTCCAGGG CAGGGTCACCATTACTGCAGACAAATCCACGAGCACAGC CTACATGGAGCTGAGCAGCCTGAGATCTGAGGACACCGC CGTGTATTACTGTGCGAGAGAATACGGTTGGATGGACTAC TGGGGCCAAGGGACCACCGTGACCGTCTCCTCAGCTAGC ACCAAGGGCCCATCCGTGTTCCCTCTGGCCCCTTCCAGCA AGTCTACCTCTGGCGGCACAGCCGCTCTGGGCTGCCTCGT GAAGGACTACTTCCCCGAGCCTGTGACAGTGTCCTGGAAC TCTGGCGCCCTGACATCCGGCGTGCACACCTTTCCAGCTG TGCTGCAGTCCTCCGGCCTGTACTCCCTGTCCTCCGTCGTG ACAGTGCCCTCCAGCTCTCTGGGCACCCAGACCTACATCT GCAACGTGAACCACAAGCCCTCCAACACCAAGGTGGACA AGAAGGTGGAACCCAAGTCCTGCGACAAGACCCACACCT GTCCCCCTTGTCCTGCCCCTGAAGCTGCTGGCGGCCCTAG CGTGTTCCTGTTCCCCCCAAAGCCCAAGGACACCCTGATG ATCTCCCGGACCCCCGAAGTGACCTGCGTGGTGGTGGATG TGTCCCACGAGGACCCTGAAGTGAAGTTCAATTGGTACGT GGACGGCGTGGAAGTGCACAATGCCAAGACCAAGCCTAG AGAGGAACAGTACAACTCCACCTACCGGGTGGTGTCCGT GCTGACCGTGCTGCACCAGGATTGGCTGAACGGCAAAGA GTACAAGTGCAAGGTGTCCAACAAGGCCCTGGGAGCCCC CATCGAAAAGACCATCTCCAAGGCCAAGGGCCAGCCTCG CGAGCCTCAGGTGTACACCCTGCCCCCTAGCAGAGATGA GCTGACCAAGAACCAGGTGTCCCTGACCTGTCTCGTGAAA GGCTTCTACCCCTCCGATATCGCCGTGGAATGGGAGAGCA ACGGCCAGCCCGAGAACAACTACAAGACCACCCCCCCTG TGCTGGACTCCGACGGCTCATTCTTCCTGTACTCTAAGCT GACAGTGGACAAGTCCCGGTGGCAGCAGGGCAACGTGTT CTCCTGCTCCGTGATGCACGAGGCCCTGCACAACCACTAC ACCCAGAAGTCCCTGTCCCTGTCTCCCGGGGGAGGCGGA GGATCTGGCGGAGGCGGATCCGGTGGTGGCGGATCTGGG GGCGGTGGATCTGAGGTGCAGCTGCTGGAATCTGGGGGA GGACTGGTGCAGCCAGGCGGATCTCTGAGGCTGTCCTGC GCTGCTTCCGGCTTTACCTTCTCCAGCCACGCCATGAGTT GGGTGCGCCAGGCACCCGGAAAAGGACTGGAATGGGTGT CAGCCATCTGGGCCTCCGGCGAGCAGTACTACGCCGATA GCGTGAAGGGCCGGTTCACCATCTCTCGGGATAACAGCA AGAATACTCTGTACCTGCAGATGAACTCCCTGCGCGCTGA AGATACCGCTGTGTATTACTGCGCCAAGGGCTGGCTGGGC AACTTCGATTACTGGGGCCAGGGAACCCTCGTGACTGTCT CGAGCGCTTCTGTGGCCGCTCCCTCCGTGTTCATCTTCCCA CCTTCCGACGAGCAGCTGAAGTCCGGCACTGCCTCTGTCG TGTGCCTGCTGAACAACTTCTACCCTCGGGAAGCCAAGGT GCAGTGGAAAGTGGATAACGCCCTGCAGTCCGGCAACTC CCAGGAATCCGTGACCGAGCAGGACTCCAAGGACAGCAC |
| | | CTACTCCCTGAGCAGCACCCTGACCCTGTCCAAGGCCGAC TACGAGAAGCACAAGGTGTACGCCTGTGAAGTGACCCAC CAGGGCCTGTCCAGCCCCGTGACCAAGTCCTTCAACCGGG GCGAGTGC |
| 151 | VLCL- Light chain 1 (8H9) (nucleotide sequence) | see Table 13 |
| 184 | VLCH1-Light chain 2 (28H1) (nucleotide sequence) | GAGATCGTGCTGACCCAGTCTCCCGGCACCCTGAGCCTGA GCCCTGGCGAGAGAGCCACCCTGAGCTGCAGAGCCAGCC AGAGCGTGAGCCGGAGCTACCTGGCCTGGTATCAGCAGA AGCCCGGCCAGGCCCCCAGACTGCTGATCATCGGCGCCA GCACCCGGGCCACCGGCATCCCCGATAGATTCAGCGGCA GCGGCTCCGGCACCGACTTCACCCTGACCATCAGCCGGCT GGAACCCGAGGACTTCGCCGTGTACTACTGCCAGCAGGG CCAGGTGATCCCCCCCACCTTCGGCCAGGGCACCAAGGT GGAAATCAAGAGCTCCGCTAGCACCAAGGGCCCCTCCGT GTTTCCTCTGGCCCCCAGCAGCAAGAGCACCTCTGGCGGA ACAGCCGCCCTGGGCTGCCTGGTGAAAGACTACTTCCCCG AGCCCGTGACCGTGTCCTGGAACTCTGGCGCCCTGACCAG CGGCGTGCACACCTTTCCAGCCGTGCTGCAGAGCAGCGG CCTGTACTCCCTGAGCAGCGTGGTGACAGTGCCCTCCAGC AGCCTGGGCACCCAGACCTACATCTGCAACGTGAACCAC AAGCCCAGCAACACCAAAGTGGACAAGAAGGTGGAACCC AAGAGCTGCGAC |
| 185 | (8H9) VHCH1-Heavy chain-(28H1) VHCL | QVQLVQSGAEVKKPGSSVKVSCKASGGTFSSYAISWVRQAP GQGLEWMGGIIPIFGTANYAQKFQGRVTITADKSTSTAYME LSSLRSEDTAVYYCAREYGWMDYWGQGTTVTVSSASTKGP SVFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPEAAGGPSVFLFPPKPKD TLMISRTPEVTCVVVDVSHEDPEVKFNWYVDGVEVHNAKT KPREEQYNSTYRVVSVLTVLHQDWLNGKEYKCKVSNKALG APIEKTISKAKGQPREPQVYTLPPSRDELTKNQVSLTCLVKG FYPSDIAVEWESNGQPENNYKTTPPVLDSDGSFFLYSKLTVD KSRWQQGNVFSCSVMHEALHNHYTQKSLSLSPGGGGGSGG GGSGGGGSGGGGSEVQLLESGGGLVQPGGSLRLSCAASGFT FSSHAMSWVRQAPGKGLEWVSAIWASGEQYYADSVKGRFT ISRDNSKNTLYLQMNSLRAEDTAVYYCAKGWLGNFDYWG QGTLVTVSSASVAAPSVFIFPPSDEQLKSGTASVVCLLNNFY PREAKVQWKVDNALQSGNSQESVTEQDSKDSTYSLSSTLTL SKADYEKHKVYACEVTHQGLSSPVTKSFNRGEC |
| 153 | VLCL- Light chain 1 (8H9) | see Table 13 |
| 186 | VLCH1-Light chain 2 (28H1) | EIVLTQSPGTLSLSPGERATLSCRASQSVSRSYLAWYQQKPG QAPRLLIIGASTRATGIPDRFSGSGSGTDFTLTISRLEPEDFAV YYCQQGQVIPPTFGQGTKVEIKSSASTKGPSVFPLAPSSKSTS GGTAALGCLVKDYFPEPVTVSWNSGALTSGVHTFPAVLQSS GLYSLSSVVTVPSSSLGTQTYICNVNHKPSNTKVDKKVEPKS CD |
| 187 | (49B4) VHCH1-Heavy chain-(28H1) VHCL | CAGGTGCAATTGGTGCAGTCTGGGGCTGAGGTGAAGAAG CCTGGGTCCTCGGTGAAGGTCTCCTGCAAGGCCTCCGGAG GCACATTCAGCAGCTACGCTATAAGCTGGGTGCGACAGG |
| | (nucleotide sequence) | CCCCTGGACAAGGGCTCGAGTGGATGGGAGGGATCATCC CTATCTTTGGTACAGCAAACTACGCACAGAAGTTCCAGGG CAGGGTCACCATTACTGCAGACAAATCCACGAGCACAGC CTACATGGAGCTGAGCAGCCTGAGATCTGAGGACACCGC CGTGTATTACTGTGCGAGAGAATACTACCGTGGTCCGTAC GACTACTGGGGCCAAGGGACCACCGTGACCGTCTCCTCA GCTAGCACCAAGGGCCCATCCGTGTTCCCTCTGGCCCCTT CCAGCAAGTCTACCTCTGGCGGCACAGCCGCTCTGGGCTG CCTCGTGAAGGACTACTTCCCCGAGCCTGTGACAGTGTCC TGGAACTCTGGCGCCCTGACATCCGGCGTGCACACCTTTC CAGCTGTGCTGCAGTCCTCCGGCCTGTACTCCCTGTCCTC CGTCGTGACAGTGCCCTCCAGCTCTCTGGGCACCCAGACC TACATCTGCAACGTGAACCACAAGCCCTCCAACACCAAG GTGGACAAGAAGGTGGAACCCAAGTCCTGCGACAAGACC CACACCTGTCCCCCTTGTCCTGCCCCTGAAGCTGCTGGCG GCCCTAGCGTGTTCCTGTTCCCCCCAAAGCCCAAGGACAC CCTGATGATCTCCCGGACCCCCGAAGTGACCTGCGTGGTG GTGGATGTGTCCCACGAGGACCCTGAAGTGAAGTTCAATT GGTACGTGGACGGCGTGGAAGTGCACAATGCCAAGACCA AGCCTAGAGAGGAACAGTACAACTCCACCTACCGGGTGG TGTCCGTGCTGACCGTGCTGCACCAGGATTGGCTGAACGG CAAAGAGTACAAGTGCAAGGTGTCCAACAAGGCCCTGGG AGCCCCCATCGAAAAGACCATCTCCAAGGCCAAGGGCCA GCCTCGCGAGCCTCAGGTGTACACCCTGCCCCCTAGCAGA GATGAGCTGACCAAGAACCAGGTGTCCCTGACCTGTCTCG TGAAAGGCTTCTACCCCTCCGATATCGCCGTGGAATGGGA GAGCAACGGCCAGCCCGAGAACAACTACAAGACCACCCC CCCTGTGCTGGACTCCGACGGCTCATTCTTCCTGTACTCTA AGCTGACAGTGGACAAGTCCCGGTGGCAGCAGGGCAACG TGTTCTCCTGCTCCGTGATGCACGAGGCCCTGCACAACCA CTACACCCAGAAGTCCCTGTCCCTGTCTCCCGGGGGAGGC GGAGGATCTGGCGGAGGCGGATCCGGTGGTGGCGGATCT GGGGGCGGTGGATCTGAGGTGCAGCTGCTGGAATCTGGG GGAGGACTGGTGCAGCCAGGCGGATCTCTGAGGCTGTCC TGCGCTGCTTCCGGCTTTACCTTCTCCAGCCACGCCATGA GTTGGGTGCGCCAGGCACCCGGAAAAGGACTGGAATGGG TGTCAGCCATCTGGGCCTCCGGCGAGCAGTACTACGCCGA TAGCGTGAAGGGCCGGTTCACCATCTCTCGGGATAACAG CAAGAATACTCTGTACCTGCAGATGAACTCCCTGCGCGCT GAAGATACCGCTGTGTATTACTGCGCCAAGGGCTGGCTG GGCAACTTCGATTACTGGGGCCAGGGAACCCTCGTGACT GTCTCGAGCGCTTCTGTGGCCGCTCCCTCCGTGTTCATCTT CCCACCTTCCGACGAGCAGCTGAAGTCCGGCACTGCCTCT GTCGTGTGCCTGCTGAACAACTTCTACCCTCGGGAAGCCA AGGTGCAGTGGAAAGTGGATAACGCCCTGCAGTCCGGCA ACTCCCAGGAATCCGTGACCGAGCAGGACTCCAAGGACA GCACCTACTCCCTGAGCAGCACCCTGACCCTGTCCAAGGC CGACTACGAGAAGCACAAGGTGTACGCCTGTGAAGTGAC CCACCAGGGCCTGTCCAGCCCCGTGACCAAGTCCTTCAAC CGGGGCGAGTGC |
| 155 | VLCL- Light chain 1 (49B4) (nucleotide sequence) | see Table 13 |
| 184 | VLCH1-Light chain 2 (28H1) (nucleotide sequence) | see above |
| 188 | (49B4) VHCH1-Heavy chain-(28H1) VHCL | QVQLVQSGAEVKKPGSSVKVSCKASGGTFSSYAISWVRQAP GQGLEWMGGIIPIFGTANYAQKFQGRVTITADKSTSTAYME LSSLRSEDTAVYYCAREYYRGPYDYWGQGTTVTVSSASTK GPSVFPLAPSSKSTSGGTAALGCLVKDYFPEPVTVSWNSGAL TSGVHTFPAVLQSSGLYSLSSVVTVPSSSLGTQTYICNVNHK PSNTKVDKKVEPKSCDKTHTCPPCPAPEAAGGPSVFLFPPKP KDTLMISRTPEVTCVVVDVSHEDPEVKFNWYVDGVEVHNA KTKPREEQYNSTYRVVSVLTVLHQDWLNGKEYKCKVSNKA LGAPIEKTISKAKGQPREPQVYTLPPSRDELTKNQVSLTCLV KGFYPSDIAVEWESNGQPENNYKTTPPVLDSDGSFFLYSKLT VDKSRWQQGNVFSCSVMHEALHNHYTQKSLSLSPGGGGGS GGGGSGGGGSGGGGSEVQLLESGGGLVQPGGSLRLSCAAS GFTFSSHAMSWVRQAPGKGLEWVSAIWASGEQYYADSVKG RFTISRDNSKNTLYLQMNSLRAEDTAVYYCAKGWLGNFDY WGQGTLVTVSSASVAAPSVFIFPPSDEQLKSGTASVVCLLNN FYPREAKVQWKVDNALQSGNSQESVTEQDSKDSTYSLSSTL TLSKADYEKHKVYACEVTHQGLSSPVTKSFNRGEC |
| 157 | VLCL-Light chain 1 (49B4) | see Table 13 |
| 186 | VLCH1-Light chain 2 (28H1) | see above |
| 189 | (1G4) VHCH1-Heavy chain-(28H1) VHCL (nucleotide sequence) | CAGGTGCAATTGGTGCAGTCTGGGGCTGAGGTGAAGAAG CCTGGGTCCTCGGTGAAGGTCTCCTGCAAGGCCTCCGGAG GCACATTCAGCAGCTACGCTATAAGCTGGGTGCGACAGG CCCCTGGACAAGGGCTCGAGTGGATGGGAGGGATCATCC CTATCTTTGGTACAGCAAACTACGCACAGAAGTTCCAGGG CAGGGTCACCATTACTGCAGACAAATCCACGAGCACAGC CTACATGGAGCTGAGCAGCCTGAGATCTGAGGACACCGC CGTGTATTACTGTGCGAGAGAATACGGTTCTATGGACTAC TGGGGCCAAGGGACCACCGTGACCGTCTCCTCAGCTAGC ACCAAGGGCCCATCCGTGTTCCCTCTGGCCCCTTCCAGCA AGTCTACCTCTGGCGGCACAGCCGCTCTGGGCTGCCTCGT GAAGGACTACTTCCCCGAGCCTGTGACAGTGTCCTGGAAC TCTGGCGCCCTGACATCCGGCGTGCACACCTTTCCAGCTG TGCTGCAGTCCTCCGGCCTGTACTCCCTGTCCTCCGTCGTG ACAGTGCCCTCCAGCTCTCTGGGCACCCAGACCTACATCT GCAACGTGAACCACAAGCCCTCCAACACCAAGGTGGACA AGAAGGTGGAACCCAAGTCCTGCGACAAGACCCACACCT GTCCCCCTTGTCCTGCCCCTGAAGCTGCTGGCGGCCCTAG CGTGTTCCTGTTCCCCCCAAAGCCCAAGGACACCCTGATG ATCTCCCGGACCCCCGAAGTGACCTGCGTGGTGGTGGATG TGTCCCACGAGGACCCTGAAGTGAAGTTCAATTGGTACGT GGACGGCGTGGAAGTGCACAATGCCAAGACCAAGCCTAG AGAGGAACAGTACAACTCCACCTACCGGGTGGTGTCCGT GCTGACCGTGCTGCACCAGGATTGGCTGAACGGCAAAGA GTACAAGTGCAAGGTGTCCAACAAGGCCCTGGGAGCCCC CATCGAAAAGACCATCTCCAAGGCCAAGGGCCAGCCTCG CGAGCCTCAGGTGTACACCCTGCCCCCTAGCAGAGATGA GCTGACCAAGAACCAGGTGTCCCTGACCTGTCTCGTGAAA |
| | | GGCTTCTACCCCTCCGATATCGCCGTGGAATGGGAGAGCA ACGGCCAGCCCGAGAACAACTACAAGACCACCCCCCCTG TGCTGGACTCCGACGGCTCATTCTTCCTGTACTCTAAGCT GACAGTGGACAAGTCCCGGTGGCAGCAGGGCAACGTGTT CTCCTGCTCCGTGATGCACGAGGCCCTGCACAACCACTAC ACCCAGAAGTCCCTGTCCCTGTCTCCCGGGGGAGGCGGA GGATCTGGCGGAGGCGGATCCGGTGGTGGCGGATCTGGG GGCGGTGGATCTGAGGTGCAGCTGCTGGAATCTGGGGGA GGACTGGTGCAGCCAGGCGGATCTCTGAGGCTGTCCTGC GCTGCTTCCGGCTTTACCTTCTCCAGCCACGCCATGAGTT GGGTGCGCCAGGCACCCGGAAAAGGACTGGAATGGGTGT CAGCCATCTGGGCCTCCGGCGAGCAGTACTACGCCGATA GCGTGAAGGGCCGGTTCACCATCTCTCGGGATAACAGCA AGAATACTCTGTACCTGCAGATGAACTCCCTGCGCGCTGA AGATACCGCTGTGTATTACTGCGCCAAGGGCTGGCTGGGC AACTTCGATTACTGGGGCCAGGGAACCCTCGTGACTGTCT CGAGCGCTTCTGTGGCCGCTCCCTCCGTGTTCATCTTCCCA CCTTCCGACGAGCAGCTGAAGTCCGGCACTGCCTCTGTCG TGTGCCTGCTGAACAACTTCTACCCTCGGGAAGCCAAGGT GCAGTGGAAAGTGGATAACGCCCTGCAGTCCGGCAACTC CCAGGAATCCGTGACCGAGCAGGACTCCAAGGACAGCAC CTACTCCCTGAGCAGCACCCTGACCCTGTCCAAGGCCGAC TACGAGAAGCACAAGGTGTACGCCTGTGAAGTGACCCAC CAGGGCCTGTCCAGCCCCGTGACCAAGTCCTTCAACCGGG GCGAGTGC |
| 159 | VLCL- Light chain 1 (1G4) (nucleotide sequence) | see Table 13 |
| 184 | VLCH1-Light chain 2 (28H1) (nucleotide sequence) | see above |
| 190 | (1G4) VHCH1-Heavy chain-(28H1) VHCL | QVQLVQSGAEVKKPGSSVKVSCKASGGTFSSYAISWVRQAP GQGLEWMGGIIPIFGTANYAQKFQGRVTITADKSTSTAYME LSSLRSEDTAVYYCAREYGSMDYWGQGTTVTVSSASTKGPS VFPLAPSSKSTSGGTAALGCLVKDYFPEPVTVSWNSGALTSG VHTFPAVLQSSGLYSLSSVVTVPSSSLGTQTYICNVNHKPSN TKVDKKVEPKSCDKTHTCPPCPAPEAAGGPSVFLFPPKPKDT LMISRTPEVTCVVVDVSHEDPEVKFNWYVDGVEVHNAKTK PREEQYNSTYRVVSVLTVLHQDWLNGKEYKCKVSNKALGA PIEKTISKAKGQPREPQVYTLPPSRDELTKNQVSLTCLVKGF YPSDIAVEWESNGQPENNYKTTPPVLDSDGSFFLYSKLTVDK SRWQQGNVFSCSVMHEALHNHYTQKSLSLSPGGGGGSGGG GSGGGGSGGGGSEVQLLESGGGLVQPGGSLRLSCAASGFTF SSHAMSWVRQAPGKGLEWVSAIWASGEQYYADSVKGRFTI SRDNSKNTLYLQMNSLRAEDTAVYYCAKGWLGNFDYWGQ GTLVTVSSASVAAPSVFIFPPSDEQLKSGTASVVCLLNNFYPR EAKVQWKVDNALQSGNSQESVTEQDSKDSTYSLSSTLTLSK ADYEKHKVYACEVTHQGLSSPVTKSFNRGEC |
| 161 | VLCL- Light chain 1 (1G4) | see Table 13 |
| 186 | VLCH1-Light chain 2 (28H1) | see above |
| 191 | (20B7) VHCH1-Heavy chain-(28H1) VHCL (nucleotide sequence) | CAGGTGCAATTGGTGCAGTCTGGGGCTGAGGTGAAGAAG CCTGGGTCCTCGGTGAAGGTCTCCTGCAAGGCCTCCGGAG GCACATTCAGCAGCTACGCTATAAGCTGGGTGCGACAGG CCCCTGGACAAGGGCTCGAGTGGATGGGAGGGATCATCC CTATCTTTGGTACAGCAAACTACGCACAGAAGTTCCAGGG CAGGGTCACCATTACTGCAGACAAATCCACGAGCACAGC CTACATGGAGCTGAGCAGCCTGAGATCTGAGGACACCGC CGTGTATTACTGTGCGAGAGTTAACTACCCGTACTCTTAC TGGGGTGACTTCGACTACTGGGGCCAAGGGACCACCGTG ACCGTCTCCTCAGCTAGCACCAAGGGCCCATCCGTGTTCC CTCTGGCCCCTTCCAGCAAGTCTACCTCTGGCGGCACAGC CGCTCTGGGCTGCCTCGTGAAGGACTACTTCCCCGAGCCT GTGACAGTGTCCTGGAACTCTGGCGCCCTGACATCCGGCG TGCACACCTTTCCAGCTGTGCTGCAGTCCTCCGGCCTGTA CTCCCTGTCCTCCGTCGTGACAGTGCCCTCCAGCTCTCTG GGCACCCAGACCTACATCTGCAACGTGAACCACAAGCCC TCCAACACCAAGGTGGACAAGAAGGTGGAACCCAAGTCC TGCGACAAGACCCACACCTGTCCCCCTTGTCCTGCCCCTG AAGCTGCTGGCGGCCCTAGCGTGTTCCTGTTCCCCCCAAA GCCCAAGGACACCCTGATGATCTCCCGGACCCCCGAAGT GACCTGCGTGGTGGTGGATGTGTCCCACGAGGACCCTGA AGTGAAGTTCAATTGGTACGTGGACGGCGTGGAAGTGCA CAATGCCAAGACCAAGCCTAGAGAGGAACAGTACAACTC CACCTACCGGGTGGTGTCCGTGCTGACCGTGCTGCACCAG GATTGGCTGAACGGCAAAGAGTACAAGTGCAAGGTGTCC AACAAGGCCCTGGGAGCCCCCATCGAAAAGACCATCTCC AAGGCCAAGGGCCAGCCTCGCGAGCCTCAGGTGTACACC CTGCCCCCTAGCAGAGATGAGCTGACCAAGAACCAGGTG TCCCTGACCTGTCTCGTGAAAGGCTTCTACCCCTCCGATA TCGCCGTGGAATGGGAGAGCAACGGCCAGCCCGAGAACA ACTACAAGACCACCCCCCCTGTGCTGGACTCCGACGGCTC ATTCTTCCTGTACTCTAAGCTGACAGTGGACAAGTCCCGG TGGCAGCAGGGCAACGTGTTCTCCTGCTCCGTGATGCACG AGGCCCTGCACAACCACTACACCCAGAAGTCCCTGTCCCT GTCTCCCGGGGGAGGCGGAGGATCTGGCGGAGGCGGATC CGGTGGTGGCGGATCTGGGGGCGGTGGATCTGAGGTGCA GCTGCTGGAATCTGGGGGAGGACTGGTGCAGCCAGGCGG ATCTCTGAGGCTGTCCTGCGCTGCTTCCGGCTTTACCTTCT CCAGCCACGCCATGAGTTGGGTGCGCCAGGCACCCGGAA AAGGACTGGAATGGGTGTCAGCCATCTGGGCCTCCGGCG AGCAGTACTACGCCGATAGCGTGAAGGGCCGGTTCACCA TCTCTCGGGATAACAGCAAGAATACTCTGTACCTGCAGAT GAACTCCCTGCGCGCTGAAGATACCGCTGTGTATTACTGC GCCAAGGGCTGGCTGGGCAACTTCGATTACTGGGGCCAG GGAACCCTCGTGACTGTCTCGAGCGCTTCTGTGGCCGCTC CCTCCGTGTTCATCTTCCCACCTTCCGACGAGCAGCTGAA GTCCGGCACTGCCTCTGTCGTGTGCCTGCTGAACAACTTC TACCCTCGGGAAGCCAAGGTGCAGTGGAAAGTGGATAAC GCCCTGCAGTCCGGCAACTCCCAGGAATCCGTGACCGAG CAGGACTCCAAGGACAGCACCTACTCCCTGAGCAGCACC CTGACCCTGTCCAAGGCCGACTACGAGAAGCACAAGGTG TACGCCTGTGAAGTGACCCACCAGGGCCTGTCCAGCCCCG TGACCAAGTCCTTCAACCGGGGCGAGTGC |
| 163 | VLCL- Light chain 1 (20B7) (nucleotide sequence) | see Table 13 |
| 184 | VLCH1-Light chain 2 (28H1) (nucleotide sequence) | see above |
| 192 | (20B7) VHCH1-Heavy chain-(28H1) VHCL | QVQLVQSGAEVKKPGSSVKVSCKASGGTFSSYAISWVRQAP GQGLEWMGGIIPIFGTANYAQKFQGRVTITADKSTSTAYME LSSLRSEDTAVYYCARVNYPYSYWGDFDYWGQGTTVTVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKDTLMISRTPEVTCVVVDVSHEDPEVKFNWYVDGVEV HNAKTKPREEQYNSTYRVVSVLTVLHQDWLNGKEYKCKVS NKALGAPIEKTISKAKGQPREPQVYTLPPSRDELTKNQVSLT CLVKGFYPSDIAVEWESNGQPENNYKTTPPVLDSDGSFFLYS KLTVDKSRWQQGNVFSCSVMHEALHNHYTQKSLSLSPGGG GGSGGGGSGGGGSGGGGSEVQLLESGGGLVQPGGSLRLSC AASGFTFSSHAMSWVRQAPGKGLEWVSAIWASGEQYYADS VKGRFTISRDNSKNTLYLQMNSLRAEDTAVYYCAKGWLGN FDYWGQGTLVTVSSASVAAPSVFIFPPSDEQLKSGTASVVCL LNNFYPREAKVQWKVDNALQSGNSQESVTEQDSKDSTYSL SSTLTLSKADYEKHKVYACEVTHQGLSSPVTKSFNRGEC |
| 165 | VLCL- Light chain 1 (20B7) | see Table 13 |
| 186 | VLCH1-Light chain 2 (28H1) | see above |
| 193 | (CLC-563) VHCH1-Heavy chain-(28H1) VHCL (nucleotide sequence) | GAGGTGCAATTGTTGGAGTCTGGGGGAGGCTTGGTACAG CCTGGGGGGTCCCTGAGACTCTCCTGTGCAGCCTCCGGAT TCACCTTTAGCAGTTATGCCATGAGCTGGGTCCGCCAGGC TCCAGGGAAGGGGCTGGAGTGGGTCTCAGCTATTAGTGG TAGTGGTGGTAGCACATACTACGCAGACTCCGTGAAGGG CCGGTTCACCATCTCCAGAGACAATTCCAAGAACACGCTG TATCTGCAGATGAACAGCCTGAGAGCCGAGGACACGGCC GTATATTACTGTGCGCTTGACGTTGGTGCTTTCGACTACT GGGGCCAAGGAGCCCTGGTCACCGTCTCGAGTGCTAGCA CCAAGGGCCCATCCGTGTTCCCTCTGGCCCCTTCCAGCAA GTCTACCTCTGGCGGCACAGCCGCTCTGGGCTGCCTCGTG AAGGACTACTTCCCCGAGCCTGTGACAGTGTCCTGGAACT CTGGCGCCCTGACATCCGGCGTGCACACCTTTCCAGCTGT GCTGCAGTCCTCCGGCCTGTACTCCCTGTCCTCCGTCGTG ACAGTGCCCTCCAGCTCTCTGGGCACCCAGACCTACATCT GCAACGTGAACCACAAGCCCTCCAACACCAAGGTGGACA AGAAGGTGGAACCCAAGTCCTGCGACAAGACCCACACCT GTCCCCCTTGTCCTGCCCCTGAAGCTGCTGGCGGCCCTAG CGTGTTCCTGTTCCCCCCAAAGCCCAAGGACACCCTGATG ATCTCCCGGACCCCCGAAGTGACCTGCGTGGTGGTGGATG TGTCCCACGAGGACCCTGAAGTGAAGTTCAATTGGTACGT GGACGGCGTGGAAGTGCACAATGCCAAGACCAAGCCTAG AGAGGAACAGTACAACTCCACCTACCGGGTGGTGTCCGT GCTGACCGTGCTGCACCAGGATTGGCTGAACGGCAAAGA |
| | | GTACAAGTGCAAGGTGTCCAACAAGGCCCTGGAGCCCC CATCGAAAAGACCATCTCCAAGGCCAAGGGCCAGCCTCG CGAGCCTCAGGTGTACACCCTGCCCCCTAGCAGAGATGA GCTGACCAAGAACCAGGTGTCCCTGACCTGTCTCGTGAAA GGCTTCTACCCCTCCGATATCGCCGTGGAATGGGAGAGCA ACGGCCAGCCCGAGAACAACTACAAGACCACCCCCCCTG TGCTGGACTCCGACGGCTCATTCTTCCTGTACTCTAAGCT GACAGTGGACAAGTCCCGGTGGCAGCAGGGCAACGTGTT CTCCTGCTCCGTGATGCACGAGGCCCTGCACAACCACTAC ACCCAGAAGTCCCTGTCCCTGTCTCCCGGGGGAGGCGGA GGATCTGGCGGAGGCGGATCCGGTGGTGGCGGATCTGGG GGCGGTGGATCTGAGGTGCAGCTGCTGGAATCTGGGGGA GGACTGGTGCAGCCAGGCGGATCTCTGAGGCTGTCCTGC GCTGCTTCCGGCTTTACCTTCTCCAGCCACGCCATGAGTT GGGTGCGCCAGGCACCCGGAAAAGGACTGGAATGGGTGT CAGCCATCTGGGCCTCCGGCGAGCAGTACTACGCCGATA GCGTGAAGGGCCGGTTCACCATCTCTCGGGATAACAGCA AGAATACTCTGTACCTGCAGATGAACTCCCTGCGCGCTGA AGATACCGCTGTGTATTACTGCGCCAAGGGCTGGCTGGGC AACTTCGATTACTGGGGCCAGGGAACCCTCGTGACTGTCT CGAGCGCTTCTGTGGCCGCTCCCTCCGTGTTCATCTTCCCA CCTTCCGACGAGCAGCTGAAGTCCGGCACTGCCTCTGTCG TGTGCCTGCTGAACAACTTCTACCCTCGGGAAGCCAAGGT GCAGTGGAAAGTGGATAACGCCCTGCAGTCCGGCAACTC CCAGGAATCCGTGACCGAGCAGGACTCCAAGGACAGCAC CTACTCCCTGAGCAGCACCCTGACCCTGTCCAAGGCCGAC TACGAGAAGCACAAGGTGTACGCCTGTGAAGTGACCCAC CAGGGCCTGTCCAGCCCCGTGACCAAGTCCTTCAACCGGG GCGAGTGC |
| 167 | VLCL- Light chain 1 (CLC-563) (nucleotide sequence) | see Table 13 |
| 184 | VLCH1-Light chain 2 (28H1) (nucleotide sequence) | see above |
| 194 | (CLC-563) VHCH1-Heavy chain-(28H1) VHCL | EVQLLESGGGLVQPGGSLRLSCAASGFTFSSYAMSWVRQAP GKGLEWVSAISGSGGSTYYADSVKGRFTISRDNSKNTLYLQ MNSLRAEDTAVYYCALDVGAFDYWGQGALVTVSSASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPEAAGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL GAPIEKTISKAKGQPREPQVYTLPPSRDELTKNQVSLTCLVK GFYPSDIAVEWESNGQPENNYKTTPPVLDSDGSFFLYSKLTV DKSRWQQGNVFSCSVMHEALHNHYTQKSLSLSPGGGGGSG GGGSGGGGSGGGGSEVQLLESGGGLVQPGGSLRLSCAASGF TFSSHAMSWVRQAPGKGLEWVSAIWASGEQYYADSVKGRF TISRDNSKNTLYLQMNSLRAEDTAVYYCAKGWLGNFDYW GQGTLVTVSSASVAAPSVFIFPPSDEQLKSGTASVVCLLNNF |
| | | YPREAKVQWKVDNALQSGNSQESVTEQDSKDSTYSLSSTLT LSKADYEKHKVYACEVTHQGLSSPVTKSFNRGEC |
| 169 | VLCL- Light chain 1 (CLC-563) | see Table 13 |
| 186 | VLCH1-Light chain 2 (28H1) | see above |
| 195 | (CLC-564) VHCH1-Heavy chain-(28H1) VHCL (nucleotide sequence) | GAGGTGCAATTGTTGGAGTCTGGGGGAGGCTTGGTACAG CCTGGGGGGTCCCTGAGACTCTCCTGTGCAGCCTCCGGAT TCACCTTTAGCAGTTATGCCATGAGCTGGGTCCGCCAGGC TCCAGGGAAGGGGCTGGAGTGGGTCTCAGCTATTAGTGG TAGTGGTGGTAGCACATACTACGCAGACTCCGTGAAGGG CCGGTTCACCATCTCCAGAGACAATTCCAAGAACACGCTG TATCTGCAGATGAACAGCCTGAGAGCCGAGGACACGGCC GTATATTACTGTGCGTTCGACGTTGGTCCGTTCGACTACT GGGGCCAAGGAACCCTGGTCACCGTCTCGAGTGCTAGCA CCAAGGGCCCATCCGTGTTCCCTCTGGCCCCTTCCAGCAA GTCTACCTCTGGCGGCACAGCCGCTCTGGGCTGCCTCGTG AAGGACTACTTCCCCGAGCCTGTGACAGTGTCCTGGAACT CTGGCGCCCTGACATCCGGCGTGCACACCTTTCCAGCTGT GCTGCAGTCCTCCGGCCTGTACTCCCTGTCCTCCGTCGTG ACAGTGCCCTCCAGCTCTCTGGGCACCCAGACCTACATCT GCAACGTGAACCACAAGCCCTCCAACACCAAGGTGGACA AGAAGGTGGAACCCAAGTCCTGCGACAAGACCCACACCT GTCCCCCTTGTCCTGCCCCTGAAGCTGCTGGCGGCCCTAG CGTGTTCCTGTTCCCCCCAAAGCCCAAGGACACCCTGATG ATCTCCCGGACCCCCGAAGTGACCTGCGTGGTGGTGGATG TGTCCCACGAGGACCCTGAAGTGAAGTTCAATTGGTACGT GGACGGCGTGGAAGTGCACAATGCCAAGACCAAGCCTAG AGAGGAACAGTACAACTCCACCTACCGGGTGGTGTCCGT GCTGACCGTGCTGCACCAGGATTGGCTGAACGGCAAAGA GTACAAGTGCAAGGTGTCCAACAAGGCCCTGGGAGCCCC CATCGAAAAGACCATCTCCAAGGCCAAGGGCCAGCCTCG CGAGCCTCAGGTGTACACCCTGCCCCCTAGCAGAGATGA GCTGACCAAGAACCAGGTGTCCCTGACCTGTCTCGTGAAA GGCTTCTACCCCTCCGATATCGCCGTGGAATGGGAGAGCA ACGGCCAGCCCGAGAACAACTACAAGACCACCCCCCCTG TGCTGGACTCCGACGGCTCATTCTTCCTGTACTCTAAGCT GACAGTGGACAAGTCCCGGTGGCAGCAGGGCAACGTGTT CTCCTGCTCCGTGATGCACGAGGCCCTGCACAACCACTAC ACCCAGAAGTCCCTGTCCCTGTCTCCGGGGGGAGGCGGA GGATCTGGCGGAGGCGGATCCGGTGGTGGCGGATCTGGG GGCGGTGGATCTGAGGTGCAGCTGCTGGAATCTGGGGGA GGACTGGTGCAGCCAGGCGGATCTCTGAGGCTGTCCTGC GCTGCTTCCGGCTTTACCTTCTCCAGCCACGCCATGAGTT GGGTGCGCCAGGCACCCGGAAAGGACTGGAATGGGTGT CAGCCATCTGGGCCTCCGGCGAGCAGTACTACGCCGATA GCGTGAAGGGCCGGTTCACCATCTCTCGGGATAACAGCA AGAATACTCTGTACCTGCAGATGAACTCCCTGCGCGCTGA AGATACCGCTGTGTATTACTGCGCCAAGGGCTGGCTGGGC AACTTCGATTACTGGGGCCAGGGAACCCTCGTGACTGTCT CGAGCGCTTCTGTGGCCGCTCCCTCCGTGTTCATCTTCCCA CCTTCCGACGAGCAGCTGAAGTCCGGCACTGCCTCTGTCG TGTGCCTGCTGAACAACTTCTACCCTCGGGAAGCCAAGGT |
| | | GCAGTGGAAAGTGGATAACGCCCTGCAGTCCGGCAACTC CCAGGAATCCGTGACCGAGCAGGACTCCAAGGACAGCAC CTACTCCCTGAGCAGCACCCTGACCCTGTCCAAGGCCGAC TACGAGAAGCACAAGGTGTACGCCTGTGAAGTGACCCAC CAGGGCCTGTCCAGCCCCGTGACCAAGTCCTTCAACCGGG GCGAGTGC |
| 171 | VLCL- Light chain 1 (CLC-564) (nucleotide sequence) | see Table 13 |
| 184 | VLCH1-Light chain 2 (28H1) (nucleotide sequence) | see above |
| 196 | (CLC-564) VHCH1-Heavy chain-(28H1) VHCL | EVQLLESGGGLVQPGGSLRLSCAASGFTFSSYAMSWVRQAP GKGLEWVSAISGSGGSTYYADSVKGRFTISRDNSKNTLYLQ MNSLRAEDTAVYYCAFDVGPFDYWGQGTLVTVSSASTKGP SVFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPEAAGGPSVFLFPPKPKD TLMISRTPEVTCVVVDVSHEDPEVKFNWYVDGVEVHNAKT KPREEQYNSTYRVVSVLTVLHQDWLNGKEYKCKVSNKALG APIEKTISKAKGQPREPQVYTLPPSRDELTKNQVSLTCLVKG FYPSDIAVEWESNGQPENNYKTTPPVLDSDGSFFLYSKLTVD KSRWQQGNVFSCSVMHEALHNHYTQKSLSLSPGGGGGSGG GGSGGGGSGGGGSEVQLLESGGGLVQPGGSLRLSCAASGFT FSSHAMSWVRQAPGKGLEWVSAIWASGEQYYADSVKGRFT ISRDNSKNTLYLQMNSLRAEDTAVYYCAKGWLGNFDYWG QGTLVTVSSASVAAPSVFIFPPSDEQLKSGTASVVCLLNNFY PREAKVQWKVDNALQSGNSQESVTEQDSKDSTYSLSSTLTL SKADYEKHKVYACEVTHQGLSSPVTKSFNRGEC |
| 173 | VLCL- Light chain 1 (CLC-564) | see Table 13 |
| 186 | VLCH1-Light chain 2 (28H1) | see above |

All genes were transiently expressed under control of a chimeric MPSV promoter consisting of the MPSV core promoter combined with the CMV promoter enhancer fragment. The expression vector also contains the oriP region for episomal replication in EBNA (Epstein Barr Virus Nuclear Antigen) containing host cells.

The bispecific anti-Ox40, anti-FAP constructs were produced by co-transfecting HEK293-EBNA cells with the mammalian expression vectors using polyethylenimine. The cells were transfected with the corresponding expression vectors in a 1:1:1 ratio ("vector heavy chain" : "vector light chain 1" : "vector light chain2").

For production in 500 mL shake flasks, 400 million HEK293 EBNA cells were seeded 24 hours before transfection. For transfection cells were centrifuged for 5 minutes by 210 x g, and supernatant was replaced by pre-warmed CD CHO medium. Expression vectors were mixed in 20 mL CD CHO medium to a final amount of 200 µg DNA. After addition of 540 µL PEI, the solution was vortexed for 15 seconds and incubated for 10 minutes at room temperature. Afterwards, cells were mixed with the DNA/PEI solution, transferred to a 500 mL shake flask and incubated for 3 hours at 37°C in an incubator with a 5% CO₂ atmosphere. After the incubation, 160 mL F17 medium was added and cells were cultured for 24 hours. One day after transfection 1 mM valproic acid and 7% Feed were added. After culturing for 7 days, the cell supernatant was collected by centrifugation for 15 minutes at 210 × g. The solution was sterile filtered (0.22 µm filter), supplemented with sodium azide to a final concentration of 0.01 % (w/v), and kept at 4°C.

Purification of bispecific constructs from cell culture supernatants was carried out by affinity chromatography using Protein A as described above for purification of antigen-Fc fusions and antibodies.

The protein was concentrated and filtered prior to loading on a HiLoad Superdex 200 column (GE Healthcare) equilibrated with 20mM Histidine, 140mM NaCl solution of pH 6.0.

The protein concentration of purified bispecific constructs was determined by measuring the OD at 280 nm, using the molar extinction coefficient calculated on the basis of the amino acid sequence. Purity and molecular weight of the bispecific constructs were analyzed by CE-SDS in the presence and absence of a reducing agent (Invitrogen, USA) using a LabChipGXII (Caliper). The aggregate content of bispecific constructs was analyzed using a TSKgel G3000 SW XL analytical size-exclusion column (Tosoh) equilibrated in a 25 mM K₂HPO₄, 125 mM NaCl, 200mM L-Arginine Monohydrocloride, 0.02 % (w/v) NaN₃, pH 6.7 running buffer at 25°C (Table 26).

**Table 26: Biochemical analysis of exemplary bispecific, bivalent anti-Ox40/anti-FAP IgG1 P329G LALA antigen binding molecules**

| Clone | Yield [mg/l] | Monomer [%] | CE-SDS (non red) | CE-SDS (red) |
|---|---|---|---|---|
| 8H9/FAP P329GLALA IgG1 2+2 | 58 | 100 | 95.3% (254kDa) | 3.2% (114kDa) |
| | | | 3% (237kDa) | 71.3% (90.7kDa) |
| | | | | 13.3% (28.9kDa) |
| | | | | 11.9% (26.2kDa) |
| 49B4/FAP P329GLALA IgG1 2+2 | 17 | 99 | 98.9% (253kDa) | 3.% (116kDa) |
| | | | | 71.4% (92kDa) |
| | | | | 12.9% (28.9kDa) |
| | | | | 12.1% (25.7kDa) |
| 1G4/FAP P329GLALA IgG1 2+2 | 0.5 | 99.1 | 93.9% (234kDa) | 55.5% (90.6kDa) |
| | | | 3.2% (242kDa) | 20.7% (27kDa) |
| | | | 1.2% (244kDa) | 21.6% (25kDa) |
| 20B7/FAP P329GLALA IgG1 2+2 | 14 | 97.2 | 91.5% (244kDa) | 54.1% (89kDa) |
| | | | 2.3% (227kDa) | 19% (27kDa) |
| | | | 1.4% (218kDa) | 25% (24kDa) |
| | | | 1.5% (202kDa) | |

### 4.2 Generation of bispecific monovalent antigen binding molecules targeting Ox40 and fibroblast activation protein (FAP) (1+1 format, Comparative Examples)

Bispecific agonistic Ox40 antibodies with monovalent binding for Ox40 and for FAP were prepared by applying the crossmab technology according to International patent application No. WO 2010/145792 A1 to reduce the formation of wrongly paired light chains.

In this example, a crossed Fab unit (VHCL) of the FAP binder 28H1 was fused to the hole heavy chain of a huIgG1. The Fab against anti-Ox40 was fused to the knob heavy chain. Combination of the targeted anti-FAP-Fc hole with the anti-Ox40-Fc knob chain allows generation of a heterodimer, which includes a FAP binding Fab and an Ox40 binding Fab (**Figure 12B**).

The Pro329Gly, Leu234Ala and Leu235Ala mutations have been introduced in the constant region of the heavy chains to abrogate binding to Fc gamma receptors according to the method described in International Patent Appl. Publ. No. WO 2012/130831 A1.

The resulting bispecific, monovalent construct is depicted in **Figure 12B** and the nucleotide and amino acid sequences can be found in **Table 27**.

**Table 27: cDNA and amino acid sequences of mature bispecific monovalent anti-Ox40/ anti-FAP huIgG1 P329GLALA kih antibodies**

| SEQ ID NO: | Description | Sequence |
|---|---|---|
| 197 | (28H1) VHCL-heavy chain hole (nucleotide sequence) | GAAGTGCAGCTGCTGGAATCCGGCGGAGGCCTGGTGC AGCCTGGCGGATCTCTGAGACTGTCCTGCGCCGCCTCC GGCTTCACCTTCTCCTCCCACGCCATGTCCTGGGTCCG ACAGGCTCCTGGCAAAGGCCTGGAATGGGTGTCCGCC ATCTGGGCCTCCGGCGAGCAGTACTACGCCGACTCTGT GAAGGGCCGGTTCACCATCTCCCGGGACAACTCCAAG AACACCCTGTACCTGCAGATGAACTCCCTGCGGGCCGA GGACACCGCCGTGTACTACTGTGCCAAGGGCTGGCTGG GCAACTTCGACTACTGGGGACAGGGCACCCTGGTCACC GTGTCCAGCGCTAGCGTGGCCGCTCCCAGCGTGTTCAT CTTCCCACCCAGCGACGAGCAGCTGAAGTCCGGCACA GCCAGCGTGGTGTGCCTGCTGAACAACTTCTACCCCCG CGAGGCCAAGGTGCAGTGGAAGGTGGACAACGCCCTG |
| | | CAGAGCGGCAACAGCCAGGAATCCGTGACCGAGCAGG ACAGCAAGGACTCCACCTACAGCCTGAGCAGCACCCT GACCCTGAGCAAGGCCGACTACGAGAAGCACAAGGTG TACGCCTGCGAAGTGACCCACCAGGGCCTGTCCAGCCC CGTGACCAAGAGCTTCAACCGGGGCGAGTGCGACAAG ACCCACACCTGTCCCCCTTGCCCTGCCCCTGAAGCTGC TGGTGGCCCTTCCGTGTTCCTGTTCCCCCCAAAGCCCA AGGACACCCTGATGATCAGCCGGACCCCCGAAGTGAC CTGCGTGGTGGTCGATGTGTCCCACGAGGACCCTGAAG TGAAGTTCAATTGGTACGTGGACGGCGTGGAAGTGCA CAATGCCAAGACCAAGCCGCGGGAGGAGCAGTACAAC AGCACGTACCGTGTGGTCAGCGTCCTCACCGTCCTGCA CCAGGACTGGCTGAATGGCAAGGAGTACAAGTGCAAG GTCTCCAACAAAGCCCTCGGCGCCCCCATCGAGAAAA CCATCTCCAAAGCCAAAGGGCAGCCCCGAGAACCACA GGTGTGCACCCTGCCCCCATCCCGGGATGAGCTGACCA AGAACCAGGTCAGCCTCTCGTGCGCAGTCAAAGGCTTC TATCCCAGCGACATCGCCGTGGAGTGGGAGAGCAATG GGCAGCCGGAGAACAACTACAAGACCACGCCTCCCGT GCTGGACTCCGACGGCTCCTTCTTCCTCGTGAGCAAGC TCACCGTGGACAAGAGCAGGTGGCAGCAGGGGAACGT CTTCTCATGCTCCGTGATGCATGAGGCTCTGCACAACC ACTACACGCAGAAGAGCCTCTCCCTGTCTCCGGGTAAA |
| 184 | (28H1) VLCH1-Light chain 2 (nucleotide sequence) | see Table 25 |
| 198 | (28H1) VHCL-heavy chain hole | EVQLLESGGGLVQPGGSLRLSCAASGFTFSSHAMSWVRQ APGKGLEWVSAIWASGEQYYADSVKGRFTISRDNSKNTL YLQMNSLRAEDTAVYYCAKGWLGNFDYWGQGTLVTVS SASVAAPSVFIFPPSDEQLKSGTASVVCLLNNFYPREAKV QWKVDNALQSGNSQESVTEQDSKDSTYSLSSTLTLSKAD YEKHKVYACEVTHQGLSSPVTKSFNRGECDKTHTCPPCP APEAAGGPSVFLFPPKPKDTLMISRTPEVTCVVVDVSHED PEVKFNWYVDGVEVHNAKTKPREEQYNSTYRVVSVLTV LHQDWLNGKEYKCKVSNKALGAPIEKTISKAKGQPREPQ VCTLPPSRDELTKNQVSLSCAVKGFYPSDIAVEWESNGQP ENNYKTTPPVLDSDGSFFLVSKLTVDKSRWQQGNVFSCS VMHEALHNHYTQKSLSLSPGK |
| 186 | (28H1) VLCH1-Light chain 2 | see Table 25 |
| 199 | (8H9) VHCH1-heavy chain knob (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGAGAATAC GGTTGGATGGACTACTGGGGCCAAGGGACCACCGTGA CCGTCTCCTCA GCTAGCACCAAGGGCCCTAGCGTGTTCCCTCTGGCCCC TAGCAGCAAGAGCACAAGTGGAGGAACAGCCGCCCTG GGCTGCCTGGTCAAGGACTACTTCCCCGAGCCCGTGAC CGTGTCCTGGAATTCTGGCGCCCTGACAAGCGGCGTGC |
| | | ACACATTTCCAGCCGTGCTGCAGAGCAGCGGCCTGTAC TCTCTGAGCAGCGTCGTGACCGTGCCCTCTAGCTCTCT GGGCACCCAGACCTACATCTGCAACGTGAACCACAAG CCCAGCAACACCAAAGTGGACAAGAAGGTGGAACCCA AGAGCTGCGACAAGACCCACACCTGTCCCCCTTGCCCT GCCCCTGAAGCTGCTGGTGGCCCTTCCGTGTTCCTGTTC CCCCCAAAGCCCAAGGACACCCTGATGATCAGCCGGA CCCCCGAAGTGACCTGCGTGGTGGTCGATGTGTCCCAC GAGGACCCTGAAGTGAAGTTCAATTGGTACGTGGACG GCGTGGAAGTGCACAATGCCAAGACCAAGCCGCGGGA GGAGCAGTACAACAGCACGTACCGTGTGGTCAGCGTC CTCACCGTCCTGCACCAGGACTGGCTGAATGGCAAGG AGTACAAGTGCAAGGTCTCCAACAAAGCCCTCGGCGC CCCCATCGAGAAAACCATCTCCAAAGCCAAAGGGCAG CCCCGAGAACCACAGGTGTACACCCTGCCCCCATGCCG GGATGAGCTGACCAAGAACCAGGTCAGCCTGTGGTGC CTGGTCAAAGGCTTCTATCCCAGCGACATCGCCGTGGA GTGGGAGAGCAATGGGCAGCCGGAGAACAACTACAAG ACCACGCCTCCCGTGCTGGACTCCGACGGCTCCTTCTT CCTCTACAGCAAGCTCACCGTGGACAAGAGCAGGTGG CAGCAGGGGAACGTCTTCTCATGCTCCGTGATGCATGA GGCTCTGCACAACCACTACACGCAGAAGAGCCTCTCCC TGTCTCCGGGTAAA |
| 151 | (8H9) VLCL-Light chain 1 (nucleotide sequence) | see Table 13 |
| 200 | (8H9) VHCH1-heavy chain knob | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYGWMDYWGQGTTVTVSS ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALGAPIEKTISKAKGQPREPQVYTLPP CRDELTKNQVSLWCLVKGFYPSDIAVEWESNGQPENNY KTTPPVLDSDGSFFLYSKLTVDKSRWQQGNVFSCSVMHE ALHNHYTQKSLSLSPGK |
| 153 | (8H9) VLCL-Light chain 1 | see Table 13 |
| 201 | (49B4) VHCH1-heavy chain knob (nucleotide sequence) | AGGTGCAATTGGTGCAGTCTGGGGCTGAGGTGAAGAA GCCTGGGTCCTCGGTGAAGGTCTCCTGCAAGGCCTCCG GAGGCACATTCAGCAGCTACGCTATAAGCTGGGTGCG ACAGGCCCCTGGACAAGGGCTCGAGTGGATGGGAGGG ATCATCCCTATCTTTGGTACAGCAAACTACGCACAGAA GTTCCAGGGCAGGGTCACCATTACTGCAGACAAATCCA CGAGCACAGCCTACATGGAGCTGAGCAGCCTGAGATC TGAGGACACCGCCGTGTATTACTGTGCGAGAGAATACT ACCGTGGTCCGTACGACTACTGGGGCCAAGGGACCAC CGTGACCGTCTCCTCA GCTAGCACCAAGGGCCCTAGCGTGTTCCCTCTGGCCCC TAGCAGCAAGAGCACAAGTGGAGGAACAGCCGCCCTG GGCTGCCTGGTCAAGGACTACTTCCCCGAGCCCGTGAC CGTGTCCTGGAATTCTGGCGCCCTGACAAGCGGCGTGC |
| | | ACACATTTCCAGCCGTGCTGCAGAGCAGCGGCCTGTAC TCTCTGAGCAGCGTCGTGACCGTGCCCTCTAGCTCTCT GGGCACCCAGACCTACATCTGCAACGTGAACCACAAG CCCAGCAACACCAAAGTGGACAAGAAGGTGGAACCCA AGAGCTGCGACAAGACCCACACCTGTCCCCCTTGCCCT GCCCCTGAAGCTGCTGGTGGCCCTTCCGTGTTCCTGTTC CCCCCAAAGCCCAAGGACACCCTGATGATCAGCCGGA CCCCCGAAGTGACCTGCGTGGTGGTCGATGTGTCCCAC GAGGACCCTGAAGTGAAGTTCAATTGGTACGTGGACG GCGTGGAAGTGCACAATGCCAAGACCAAGCCGCGGGA GGAGCAGTACAACAGCACGTACCGTGTGGTCAGCGTC CTCACCGTCCTGCACCAGGACTGGCTGAATGGCAAGG AGTACAAGTGCAAGGTCTCCAACAAAGCCCTCGGCGC CCCCATCGAGAAAACCATCTCCAAAGCCAAAGGGCAG CCCCGAGAACCACAGGTGTACACCCTGCCCCCATGCCG GGATGAGCTGACCAAGAACCAGGTCAGCCTGTGGTGC CTGGTCAAAGGCTTCTATCCCAGCGACATCGCCGTGGA GTGGGAGAGCAATGGGCAGCCGGAGAACAACTACAAG ACCACGCCTCCCGTGCTGGACTCCGACGGCTCCTTCTT CCTCTACAGCAAGCTCACCGTGGACAAGAGCAGGTGG CAGCAGGGGAACGTCTTCTCATGCTCCGTGATGCATGA GGCTCTGCACAACCACTACACGCAGAAGAGCCTCTCCC TGTCTCCGGGTAAA |
| 155 | (49B4) VLCL-Light chain 1 (nucleotide sequence) | see Table 13 |
| 202 | (49B4) VHCH1-heavy chain knob | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYYRGPYDYWGQGTTVTVS S ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALGAPIEKTISKAKGQPREPQVYTLPP CRDELTKNQVSLWCLVKGFYPSDIAVEWESNGQPENNY KTTPPVLDSDGSFFLYSKLTVDKSRWQQGNVFSCSVMHE ALHNHYTQKSLSLSPGK |
| 157 | (49B4) VLCL-Light chain 1 | see Table 13 |
| 203 | (1G4) VHCH1-heavy chain knob (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGAGAATAC GGTTCTATGGACTACTGGGGCCAAGGGACCACCGTGA CCGTCTCCTCA GCTAGCACCAAGGGCCCTAGCGTGTTCCCTCTGGCCCC TAGCAGCAAGAGCACAAGTGGAGGAACAGCCGCCCTG GGCTGCCTGGTCAAGGACTACTTCCCCGAGCCCGTGAC |
| | | CGTGTCCTGGAATTCTGGCGCCCTGACAAGCGGCGTGC ACACATTTCCAGCCGTGCTGCAGAGCAGCGGCCTGTAC TCTCTGAGCAGCGTCGTGACCGTGCCCTCTAGCTCTCT GGGCACCCAGACCTACATCTGCAACGTGAACCACAAG CCCAGCAACACCAAAGTGGACAAGAAGGTGGAACCCA AGAGCTGCGACAAGACCCACACCTGTCCCCCTTGCCCT GCCCCTGAAGCTGCTGGTGGCCCTTCCGTGTTCCTGTTC CCCCCAAAGCCCAAGGACACCCTGATGATCAGCCGGA CCCCCGAAGTGACCTGCGTGGTGGTCGATGTGTCCCAC GAGGACCCTGAAGTGAAGTTCAATTGGTACGTGGACG GCGTGGAAGTGCACAATGCCAAGACCAAGCCGCGGGA GGAGCAGTACAACAGCACGTACCGTGTGGTCAGCGTC CTCACCGTCCTGCACCAGGACTGGCTGAATGGCAAGG AGTACAAGTGCAAGGTCTCCAACAAAGCCCTCGGCGC CCCCATCGAGAAAACCATCTCCAAAGCCAAAGGGCAG CCCCGAGAACCACAGGTGTACACCCTGCCCCCATGCCG GGATGAGCTGACCAAGAACCAGGTCAGCCTGTGGTGC CTGGTCAAAGGCTTCTATCCCAGCGACATCGCCGTGGA GTGGGAGAGCAATGGGCAGCCGGAGAACAACTACAAG ACCACGCCTCCCGTGCTGGACTCCGACGGCTCCTTCTT CCTCTACAGCAAGCTCACCGTGGACAAGAGCAGGTGG CAGCAGGGGAACGTCTTCTCATGCTCCGTGATGCATGA GGCTCTGCACAACCACTACACGCAGAAGAGCCTCTCCC TGTCTCCGGGTAAA |
| 159 | (1G4) VLCL-Light chain 1 (nucleotide sequence) | see Table 13 |
| 204 | (1G4) VHCH1-heavy chain knob | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYGSMDYWGQGTTVTVSS ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALGAPIEKTISKAKGQPREPQVYTLPP CRDELTKNQVSLWCLVKGFYPSDIAVEWESNGQPENNY KTTPPVLDSDGSFFLYSKLTVDKSRWQQGNVFSCSVMHE ALHNHYTQKSLSLSPGK |
| 161 | (1G4) VLCL-Light chain 1 | see Table 13 |
| 205 | (20B7) VHCH1-heavy chain knob (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGAGTTAAC TACCCGTACTCTTACTGGGGTGACTTCGACTACTGGGG CCAAGGGACCACCGTGACCGTCTCCTCA GCTAGCACCAAGGGCCCTAGCGTGTTCCCTCTGGCCCC TAGCAGCAAGAGCACAAGTGGAGGAACAGCCGCCCTG GGCTGCCTGGTCAAGGACTACTTCCCCGAGCCCGTGAC |
| | | CGTGTCCTGGAATTCTGGCGCCCTGACAAGCGGCGTGC ACACATTTCCAGCCGTGCTGCAGAGCAGCGGCCTGTAC TCTCTGAGCAGCGTCGTGACCGTGCCCTCTAGCTCTCT GGGCACCCAGACCTACATCTGCAACGTGAACCACAAG CCCAGCAACACCAAAGTGGACAAGAAGGTGGAACCCA AGAGCTGCGACAAGACCCACACCTGTCCCCCTTGCCCT GCCCCTGAAGCTGCTGGTGGCCCTTCCGTGTTCCTGTTC CCCCCAAAGCCCAAGGACACCCTGATGATCAGCCGGA CCCCCGAAGTGACCTGCGTGGTGGTCGATGTGTCCCAC GAGGACCCTGAAGTGAAGTTCAATTGGTACGTGGACG GCGTGGAAGTGCACAATGCCAAGACCAAGCCGCGGGA GGAGCAGTACAACAGCACGTACCGTGTGGTCAGCGTC CTCACCGTCCTGCACCAGGACTGGCTGAATGGCAAGG AGTACAAGTGCAAGGTCTCCAACAAAGCCCTCGGCGC CCCCATCGAGAAAACCATCTCCAAAGCCAAAGGGCAG CCCCGAGAACCACAGGTGTACACCCTGCCCCCATGCCG GGATGAGCTGACCAAGAACCAGGTCAGCCTGTGGTGC CTGGTCAAAGGCTTCTATCCCAGCGACATCGCCGTGGA GTGGGAGAGCAATGGGCAGCCGGAGAACAACTACAAG ACCACGCCTCCCGTGCTGGACTCCGACGGCTCCTTCTT CCTCTACAGCAAGCTCACCGTGGACAAGAGCAGGTGG CAGCAGGGGAACGTCTTCTCATGCTCCGTGATGCATGA GGCTCTGCACAACCACTACACGCAGAAGAGCCTCTCCC TGTCTCCGGGTAAA |
| 163 | (20B7) VLCL-Light chain 1 (nucleotide sequence) | see Table 13 |
| 206 | (20B7) VHCH1-heavy chain knob | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARVNYPYSYWGDFDYWGQGT TVTVSS ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALGAPIEKTISKAKGQPREPQVYTLPP CRDELTKNQVSLWCLVKGFYPSDIAVEWESNGQPENNY KTTPPVLDSDGSFFLYSKLTVDKSRWQQGNVFSCSVMHE ALHNHYTQKSLSLSPGK |
| 165 | (20B7) VLCL-Light chain 1 | see Table 13 |
| 207 | (CLC-563) VHCH1-heavy chain knob (nucleotide sequence) | GAGGTGCAATTGTTGGAGTCTGGGGGAGGCTTGGTAC AGCCTGGGGGGTCCCTGAGACTCTCCTGTGCAGCCTCC GGATTCACCTTTAGCAGTTATGCCATGAGCTGGGTCCG CCAGGCTCCAGGGAAGGGGCTGGAGTGGGTCTCAGCT ATTAGTGGTAGTGGTGGTAGCACATACTACGCAGACTC CGTGAAGGGCCGGTTCACCATCTCCAGAGACAATTCCA AGAACACGCTGTATCTGCAGATGAACAGCCTGAGAGC CGAGGACACGGCCGTATATTACTGTGCGCTTGACGTTG GTGCTTTCGACTACTGGGGCCAAGGAGCCCTGGTCACC GTCTCGAGT GCTAGCACCAAGGGCCCTAGCGTGTTCCCTCTGGCCCC TAGCAGCAAGAGCACAAGTGGAGGAACAGCCGCCCTG |
| | | GGCTGCCTGGTCAAGGACTACTTCCCCGAGCCGTGAC CGTGTCCTGGAATTCTGGCGCCCTGACAAGCGGCGTGC ACACATTTCCAGCCGTGCTGCAGAGCAGCGGCCTGTAC TCTCTGAGCAGCGTCGTGACCGTGCCCTCTAGCTCTCT GGGCACCCAGACCTACATCTGCAACGTGAACCACAAG CCCAGCAACACCAAAGTGGACAAGAAGGTGGAACCCA AGAGCTGCGACAAGACCCACACCTGTCCCCCTTGCCCT GCCCCTGAAGCTGCTGGTGGCCCTTCCGTGTTCCTGTTC CCCCCAAAGCCCAAGGACACCCTGATGATCAGCCGGA CCCCCGAAGTGACCTGCGTGGTGGTCGATGTGTCCCAC GAGGACCCTGAAGTGAAGTTCAATTGGTACGTGGACG GCGTGGAAGTGCACAATGCCAAGACCAAGCCGCGGGA GGAGCAGTACAACAGCACGTACCGTGTGGTCAGCGTC CTCACCGTCCTGCACCAGGACTGGCTGAATGGCAAGG AGTACAAGTGCAAGGTCTCCAACAAAGCCCTCGGCGC CCCCATCGAGAAAACCATCTCCAAAGCCAAAGGGCAG CCCCGAGAACCACAGGTGTACACCCTGCCCCCATGCCG GGATGAGCTGACCAAGAACCAGGTCAGCCTGTGGTGC CTGGTCAAAGGCTTCTATCCCAGCGACATCGCCGTGGA GTGGGAGAGCAATGGGCAGCCGGAGAACAACTACAAG ACCACGCCTCCCGTGCTGGACTCCGACGGCTCCTTCTT CCTCTACAGCAAGCTCACCGTGGACAAGAGCAGGTGG CAGCAGGGGAACGTCTTCTCATGCTCCGTGATGCATGA GGCTCTGCACAACCACTACACGCAGAAGAGCCTCTCCC TGTCTCCGGGTAAA |
| 167 | (CLC-563) VLCL-Light chain 1 (nucleotide sequence) | see Table 13 |
| 208 | (CLC-563) VHCH1-heavy chain knob | EVQLLESGGGLVQPGGSLRLSCAASGFTFSSYAMSWVRQ APGKGLEWVSAISGSGGSTYYADSVKGRFTISRDNSKNTL YLQMNSLRAEDTAVYYCALDVGAFDYWGQGALVTVSS ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALGAPIEKTISKAKGQPREPQVYTLPP CRDELTKNQVSLWCLVKGFYPSDIAVEWESNGQPENNY KTTPPVLDSDGSFFLYSKLTVDKSRWQQGNVFSCSVMHE ALHNHYTQKSLSLSPGK |
| 169 | (CLC-563) VLCL-Light chain 1 | see Table 13 |
| 209 | (CLC-564) VHCH1-heavy chain knob (nucleotide sequence) | GAGGTGCAATTGTTGGAGTCTGGGGGAGGCTTGGTAC AGCCTGGGGGGTCCCTGAGACTCTCCTGTGCAGCCTCC GGATTCACCTTTAGCAGTTATGCCATGAGCTGGGTCCG CCAGGCTCCAGGGAAGGGGCTGGAGTGGGTCTCAGCT ATTAGTGGTAGTGGTGGTAGCACATACTACGCAGACTC CGTGAAGGGCCGGTTCACCATCTCCAGAGACAATTCCA AGAACACGCTGTATCTGCAGATGAACAGCCTGAGAGC CGAGGACACGGCCGTATATTACTGTGCGTTCGACGTTG GTCCGTTCGACTACTGGGGCCAAGGAACCCTGGTCACC GTCTCGAGT GCTAGCACCAAGGGCCCTAGCGTGTTCCCTCTGGCCCC TAGCAGCAAGAGCACAAGTGGAGGAACAGCCGCCCTG |
| | | GGCTGCCTGGTCAAGGACTACTTCCCCGAGCCGTGAC CGTGTCCTGGAATTCTGGCGCCCTGACAAGCGGCGTGC ACACATTTCCAGCCGTGCTGCAGAGCAGCGGCCTGTAC TCTCTGAGCAGCGTCGTGACCGTGCCCTCTAGCTCTCT GGGCACCCAGACCTACATCTGCAACGTGAACCACAAG CCCAGCAACACCAAAGTGGACAAGAAGGTGGAACCCA AGAGCTGCGACAAGACCCACACCTGTCCCCCTTGCCCT GCCCCTGAAGCTGCTGGTGGCCCTTCCGTGTTCCTGTTC CCCCCAAAGCCCAAGGACACCCTGATGATCAGCCGGA CCCCCGAAGTGACCTGCGTGGTGGTCGATGTGTCCCAC GAGGACCCTGAAGTGAAGTTCAATTGGTACGTGGACG GCGTGGAAGTGCACAATGCCAAGACCAAGCCGCGGGA GGAGCAGTACAACAGCACGTACCGTGTGGTCAGCGTC CTCACCGTCCTGCACCAGGACTGGCTGAATGGCAAGG AGTACAAGTGCAAGGTCTCCAACAAAGCCCTCGGCGC CCCCATCGAGAAAACCATCTCCAAAGCCAAAGGGCAG CCCCGAGAACCACAGGTGTACACCCTGCCCCCATGCCG GGATGAGCTGACCAAGAACCAGGTCAGCCTGTGGTGC CTGGTCAAAGGCTTCTATCCCAGCGACATCGCCGTGGA GTGGGAGAGCAATGGGCAGCCGGAGAACAACTACAAG ACCACGCCTCCCGTGCTGGACTCCGACGGCTCCTTCTT CCTCTACAGCAAGCTCACCGTGGACAAGAGCAGGTGG CAGCAGGGGAACGTCTTCTCATGCTCCGTGATGCATGA GGCTCTGCACAACCACTACACGCAGAAGAGCCTCTCCC TGTCTCCGGGTAAA |
| 171 | (CLC-564) VLCL-Light chain 1 (nucleotide sequence) | see Table 13 |
| 210 | (CLC-564) VHCH1-heavy chain knob | EVQLLESGGGLVQPGGSLRLSCAASGFTFSSYAMSWVRQ APGKGLEWVSAISGSGGSTYYADSVKGRFTISRDNSKNTL YLQMNSLRAEDTAVYYCAFDVGPFDYWGQGTLVTVSS ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALGAPIEKTISKAKGQPREPQVYTLPP CRDELTKNQVSLWCLVKGFYPSDIAVEWESNGQPENNY KTTPPVLDSDGSFFLYSKLTVDKSRWQQGNVFSCSVMHE ALHNHYTQKSLSLSPGK |
| 173 | (CLC-564) VLCL-Light chain 1 | see Table 13 |

All genes were transiently expressed under control of a chimeric MPSV promoter consisting of the MPSV core promoter combined with the CMV promoter enhancer fragment. The expression vector also contains the oriP region for episomal replication in EBNA (Epstein Barr Virus Nuclear Antigen) containing host cells.

The bispecific anti-Ox40, anti-FAP constructs were produced by co-transfecting HEK293-EBNA cells with the mammalian expression vectors using polyethylenimine. The cells were transfected with the corresponding expression vectors in a 1:1:1:1 ratio ("vector heavy chain hole" : "vector heavy chain knob" : "vector light chain 1" : "vector light chain2").

For production in 500 mL shake flasks, 400 million HEK293 EBNA cells were seeded 24 hours before transfection. For transfection cells were centrifuged for 5 minutes by 210 x g, and supernatant was replaced by pre-warmed CD CHO medium. Expression vectors were mixed in 20 mL CD CHO medium to a final amount of 200 µg DNA. After addition of 540 µL PEI, the solution was vortexed for 15 seconds and incubated for 10 minutes at room temperature. Afterwards, cells were mixed with the DNA/PEI solution, transferred to a 500 mL shake flask and incubated for 3 hours at 37°C in an incubator with a 5% CO₂ atmosphere. After the incubation, 160 mL F17 medium was added and cells were cultured for 24 hours. One day after transfection 1 mM valproic acid and 7% Feed were added. After culturing for 7 days, the cell supernatant was collected by centrifugation for 15 minutes at 210 x g. The solution was sterile filtered (0.22 µm filter), supplemented with sodium azide to a final concentration of 0.01 % (w/v), and kept at 4°C.

Purification of the bispecific antigen binding molecules from cell culture supernatants was carried out by affinity chromatography using Protein A as described above for purification of antigen-Fc fusions and antibodies.

The protein was concentrated and filtered prior to loading on a HiLoad Superdex 200 column (GE Healthcare) equilibrated with 20mM Histidine, 140mM NaCl solution of pH 6.0.

The protein concentration of purified bispecific constructs was determined by measuring the OD at 280 nm, using the molar extinction coefficient calculated on the basis of the amino acid sequence. Purity and molecular weight of the bispecific constructs were analyzed by CE-SDS in the presence and absence of a reducing agent (Invitrogen, USA) using a LabChipGXII (Caliper). The aggregate content of bispecific constructs was analyzed using a TSKgel G3000 SW XL analytical size-exclusion column (Tosoh) equilibrated in a 25 mM K₂HPO₄, 125 mM NaCl, 200mM L-Arginine Monohydrocloride, 0.02 % (w/v) NaN₃, pH 6.7 running buffer at 25°C.

**Table 28** summarizes the biochemical analysis of bispecific, monovalent anti-Ox40 / anti-FAP IgG1 P329G LALA kih antigen binding molecules.

**Table 28: Biochemical analysis of bispecific monovalent anti-Ox40/anti-FAP IgG1 P329G LALA kih antigen binding molecules**

| Clone | Yield [mg/l] | Monomer [%] | CE-SDS (non red) | CE-SDS (red) |
|---|---|---|---|---|
| 8H9/FAP P329GLALA IgG1 1+1 | 16.5 | 100 | 92.1% (164kDa) | 67.7% (63.6kDa) |
| | | | 1.9% (145kDa) | 13.3% (28.5kDa) |
| | | | 3.6% (120.1kDa) | 16.5% (25.7kDa) |
| 1G4/FAP P329GLALA IgG1 1+1 | 12.5 | 98.5 | 85.2% (157kDa) | 69.5% (64.2kDa) |
| | | | 7.4% (151kDa) | 13.1% (28.8kDa) |
| | | | 2.8% (139.5kDa) | 16.7% (26.2kDa) |
| 49B4/FAP P329GLALA IgG1 1+1 | 2.3 | 97.9 | 80% (153kDa) | 70.4% (63.5kDa) |
| | | | 11.9% (141kDa) | 14.7% (28kDa) |
| | | | 4.3% (120kDa) | 13.7% (25kDa) |
| 20B7/FAP P329GLALA IgG1 1+1 | 22 | 100 | 97.5% (166kDa) | 82.7% (56.2kDa) |
| | | | 1.3% (149kDa) | 8.2% (27.2kDa) |
| | | | | 8.1% (24.3kDa) |

### 4.3 Characterization of bispecific, bivalent constructs targeting Ox40 and FAP

### 4.3.1 Surface plasmon resonance (simultaneous binding)

The capacity of binding simultaneously human Ox40 Fc(kih) and human FAP was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany). Biotinylated human Ox40 Fc(kih) was directly coupled to a flow cell of a streptavidin (SA) sensor chip. Immobilization levels up to 1000 resonance units (RU) were used.

The bispecific constructs targeting Ox40 and FAP were passed at a concentration range of 250 nM with a flow of 30 µL/minute through the flow cells over 90 seconds and dissociation was set to zero sec. Human FAP was injected as second analyte with a flow of 30 µL/minute through the flow cells over 90 seconds at a concentration of 250 nM (**Figure 12C**). The dissociation was monitored for 120 sec. Bulk refractive index differences were corrected for by subtracting the response obtained in a reference flow cell, where no protein was immobilized.

As can be seen in the graphs of **Figures 13A-13D** for the 2+2 constructs and in **Figures 13E-13H****,** all bispecific constructs could bind simultaneously human Ox40 and human FAP.

### 4.3.2 Binding on cells

### 4.3.2.1 Binding to naïve versus activated human PBMCs of FAP-targeted anti-Ox40 antibodies

Human PBMC were isolated by ficoll density gradient centrifugation as described in Example 2.1.2. PBMCs were used directly after isolation (binding on resting human PBMCs) or they were stimulated to receive a strong human Ox40 expression on the cell surface of T cells (binding on activated human PBMCs). Therefore naive PBMCs were cultured for four to seven days in T cell medium supplied with 200 U/mL Proleukin and 2 ug/mL PHA-L in 6-well tissue culture plate and then 1 day on pre-coated 6-well tissue culture plates [10 ug/mL anti-human CD3 (clone OKT3) and 2 ug/mL anti-human CD28 (clone CD28.2)] in T cell medium supplied with 200 U/mL Proleukin at 37 °C and 5% CO₂.

For detection of Ox40 naive human PBMC and activated human PBMC were mixed. To enable distinction of naive from activated human PBMC naive cells were labeled prior to the binding assay using the eFluor670 cell proliferation dye (eBioscience, Cat.-No.65-0840-85). A 1 to 1 mixture of 1 × 10⁵ naïve, eFluor670 labeled human PBMC and unlabeled activated human PBMC were then added to each well of a round-bottom suspension cell 96-well plates (greiner bio-one, cellstar, Cat. No. 650185) and the binding assay was performed as described in Example 2.1.2. A 1 to 1 mixture of 1 x 105 naive, eFluor670 labeled human PBMC and unlabeled activated human PBMC were then added to each well of a round-bottom suspension cell 96-well plates (greiner bio-one, cellstar, Cat. No. 650185) and binding assay was performed as described in section 2.1.2.

Primary antibodies were titrated anti -Ox40 antibody constructs, incubated for 120 minutes at 4 °C. Secondary antibody solution was a mixture of fluorescently labeled anti-human CD4 (clone RPA-T4, mouse IgG1 k, BioLegend, Cat.-No. 300532), anti-human CD8 (clone RPa-T8, mouse IgGlk, BioLegend, Cat.-No. 3010441) and Fluorescein isothiocyanate (FITC)-conjugated AffiniPure anti-human IgG Fcγ-fragment-specific goat IgG F(ab')₂ fragment (Jackson ImmunoResearch, Cat.-No. 109-096-098), incubated for 30 minutes at 4 °C in the dark. Plates were finally resuspended in 80 µL/well FACS-buffer containing 0.2 µg/mL DAPI (Santa Cruz Biotec, Cat. No. Sc-3598) and acquired the same day using 5-laser LSR-Fortessa (BD Bioscience with DIVA software).

As shown in **Figure 14A and 14B****,** no antigen binding molecule specific for Ox40 bound to resting human CD4+ T-cells or CD8+ T-cells. In contrast, all antigen binding molecules bound to activated CD8+ or CD4+ T-cells. Binding to CD4+ T-cells was much stronger than that to CD8+ T cells similar to what was described already in Example 1.4.1.2. As shown in **Figures 14A and 14B****,** bivalent FAP targeted Ox40 construct showed similar binding characteristics to Ox40 positive and negative cells as respective clones in a conventional IgG antibody format, whereas monovalent antibodies had a clearly reduced capacity to bind to Ox40 positive cells due to the loss of avidity.

### 4.3.2.2 Binding to human FAP-expressing tumor cells

The binding to cell surface FAP was tested using human fibroblast activating protein (huFAP) expressing cells NIH/3T3-huFAP clone 39 or WM266-4 cells (ATCC CRL-1676). NIH/3T3-huFAP clone 39 was generated by the transfection of the mouse embryonic fibroblast NIH/3T3 cell line (ATCC CRL-1658) with the expression vector pETR4921 to express huFAP under 1.5 µg/mL Puromycin selection. In some assays WM266-4 cells were pre- labeled with PKH-26 Red Fluorescence Cell linker Kit (Sigma, Cat.-No. PKH26GL) as described in Example 2.3.2 to allow separation of these tumor cells from other cells present (e.g. human PBMC).

0.5 × 10⁵ NIH/3T3-huFAP clone 39 or WM266-4 cells were then added to each well of a round-bottom suspension cell 96-well plates (greiner bio-one, cellstar, Cat. No. 650185) and the binding assay was performed in a similar manner as described in Example 2.3.2. Plates were centrifuged 4 minutes, 400 × g at 4 °C and supernatants were flicked off. Cells were washed once with 200 µL DPBS and pellets were resuspended by a short and gentle vortex. All samples were resuspended in 50 µL/well of 4 °C cold FACS buffer containing the bispecific antigen binding molecules (primary antibody) at the indicated range of concentrations (titrated) and incubated for 120 minutes at 4 °C. Afterwards the cells were washed four times with 200 µL 4°C FACS buffer and resuspended by a short vortex. Cells were further stained with 25 µL/well of 4 °C cold secondary antibody solution containing Fluorescein isothiocyanate (FITC)-conjugated AffiniPure anti-human IgG Fcγ-fragment-specific goat IgG F(ab')₂ fragment (Jackson ImmunoResearch, Cat. No. 109-096-098) and incubated for 30 minutes at 4 °C in the dark. Plates were finally resuspended in 80 µL/well FACS-buffer containing 0.2 µg/mL DAPI (Santa Cruz Biotec, Cat. No. Sc-3598) and acquired the same day using 5-laser LSR-Fortessa (BD Bioscience with DIVA software).

As shown in **Figure 15A**, the FAP-targeted mono- and bivalent anti- Ox40 antigen binding molecules but not the same clones in the huIgG1 P329GLALA format efficiently bound to human FAP-expressing target cells. Therefore only FAP- targeted mono- and bivalent anti-Ox40 antigen binding molecules show direct tumor-targeting properties. The bivalent construct (filled square) showed stronger binding to FAP than the monovalent constructs explained by a gain of avidity in the bivalent relative to the monovalent format. This was more prominent in the high FAP expressing NIH/3T3-huFAP clone 39 cells (left graph) than in the lower FAP expressing WM266-4 cells. A lower density of surface FAP on WM266-4 cells might not provide the close proximity of FAP molecules to always allow bivalent binding of the anti- OX40 constructs. EC₅₀ values of binding to activated human CD4 T cells and FAP positive tumor cells are summarized in **Table 29**.

**Table 29: EC₅₀ values for binding of selected aOx40 binder (clone 8H9, 1G4) in a FAP targeted mono or bivalent format to cell surface human FAP and human Ox40**

| Clone | Format | FAP+ cell EC₅₀ [nM] | OX40+ cell EC₅₀ [nM] |
|---|---|---|---|
| 8H9 (WM266-4) | hu IgG1 | n.a. | 0.59 |
| | FAP 1+1 | 5.99 | 8.20 |
| | FAP 2+2 | 2.88 | 0.93 |
| 1G4 (NIH/3T3 huFAP clone 39) | hu IgG1 | n.a. | n.a. |
| | FAP 1+1 | 3.55 | 49.07 |
| | FAP 2+2 | 0.77 | 9.37 |

### 4.4 Generation of bispecific tetravalent antigen binding molecules targeting Ox40 and fibroblast activation protein (FAP) (4+1 format)

Bispecific agonistic Ox40 antibodies with tetravalent binding for Ox40 and monovalent binding for FAP were prepared by applying the knob-into-hole technology to allow the assembling of two different heavy chains.

In this example, the first heavy chain (HC 1) was comprised of two Fab units (VHCH1_VHCH1) of the anti-OX40 binder 49B4 followed by Fc knob chain fused by a (G4S) linker to a VH domain of the anti-FAP binder 28H1 or 4B9. The second heavy chain (HC 2) of the construct was comprised of two Fab units (VHCH1_VHCH1) of the anti-OX40 binder 49B4 followed Fc hole chain fused by a (G4S) linker to a VL domain of the anti-FAP binder 28H1 or 4B9.

The generation and preparation of the FAP binders is described in WO 2012/020006 A2, which is incorporated herein by reference.

The Pro329Gly, Leu234Ala and Leu235Ala mutations were introduced in the constant region of the heavy chains to abrogate binding to Fc gamma receptors according to the method described in International Patent Appl. Publ. No. WO 2012/130831 A1. The heavy chain fusion proteins were co-expressed with the light chain of the anti-OX40 binder 49B4 (CLVL). The resulting bispecific, tetravalent construct is depicted in **Figure 16A** and the nucleotide and amino acid sequences can be found in **Table 30**.

In addition, an "untargeted" 4+1 construct was prepared, wherein the VH and VL domain of the anti-FAP binder were replaced by a germline control, termed DP47, not binding to the antigen.

**Table 30: cDNA and amino acid sequences of mature bispecific tetravalent anti-Ox40/ monovalent anti-FAP huIgG1 P329GLALA kih antibodies (4+1 format) and untargeted (DP47) 4+1 construct**

| SEQ ID NO: | Description | Sequence |
|---|---|---|
| 155 | (49B4) VLCL-light chain (nucleotide sequence) | see Table 13 |
| 211 | HC 1 (49B4) _VHCH1 _VHCH1 Fc knob VH (4B9) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGAGAATAC TACCGTGGTCCGTACGACTACTGGGGCCAAGGGACCA CCGTGACCGTCTCCTCAGCTAGCACAAAGGGACCTAGC GTGTTCCCCCTGGCCCCCAGCAGCAAGTCTACATCTGG CGGAACAGCCGCCCTGGGCTGCCTCGTGAAGGACTACT TTCCCGAGCCCGTGACCGTGTCCTGGAACTCTGGCGCT CTGACAAGCGGCGTGCACACCTTTCCAGCCGTGCTGCA GAGCAGCGGCCTGTACTCTCTGAGCAGCGTCGTGACAG TGCCCAGCAGCTCTCTGGGCACCCAGACCTACATCTGC AACGTGAACCACAAGCCCAGCAACACCAAGGTGGACA AGAAGGTGGAACCCAAGAGCTGCGACGGCGGAGGGG GATCTGGCGGCGGAGGATCCCAGGTGCAATTGGTGCA GTCTGGGGCTGAGGTGAAGAAGCCTGGGTCCTCGGTG AAGGTCTCCTGCAAGGCCTCCGGAGGCACATTCAGCA GCTACGCTATAAGCTGGGTGCGACAGGCCCCTGGACA AGGGCTCGAGTGGATGGGAGGGATCATCCCTATCTTTG GTACAGCAAACTACGCACAGAAGTTCCAGGGCAGGGT CACCATTACTGCAGACAAATCCACGAGCACAGCCTAC ATGGAGCTGAGCAGCCTGAGATCTGAGGACACCGCCG TGTATTACTGTGCGAGAGAATACTACCGTGGTCCGTAC GACTACTGGGGCCAAGGGACCACCGTGACCGTCTCCTC AGCTAGCACCAAGGGCCCATCGGTCTTCCCCCTGGCAC CCTCCTCCAAGAGCACCTCTGGGGGCACAGCGGCCCTG GGCTGCCTGGTCAAGGACTACTTCCCCGAACCGGTGAC GGTGTCGTGGAACTCAGGCGCCCTGACCAGCGGCGTG CACACCTTCCCGGCTGTCCTACAGTCCTCAGGACTCTA CTCCCTCAGCAGCGTGGTGACCGTGCCCTCCAGCAGCT TGGGCACCCAGACCTACATCTGCAACGTGAATCACAA GCCCAGCAACACCAAGGTGGACAAGAAAGTTGAGCCC AAATCTTGTGACAAAACTCACACATGCCCACCGTGCCC AGCACCTGAAGCTGCAGGGGGACCGTCAGTCTTCCTCT TCCCCCCAAAACCCAAGGACACCCTCATGATCTCCCGG ACCCCTGAGGTCACATGCGTGGTGGTGGACGTGAGCC ACGAAGACCCTGAGGTCAAGTTCAACTGGTACGTGGA CGGCGTGGAGGTGCATAATGCCAAGACAAAGCCGCGG GAGGAGCAGTACAACAGCACGTACCGTGTGGTCAGCG TCCTCACCGTCCTGCACCAGGACTGGCTGAATGGCAAG GAGTACAAGTGCAAGGTCTCCAACAAAGCCCTCGGCG CCCCCATCGAGAAAACCATCTCCAAAGCCAAAGGGCA GCCCCGAGAACCACAGGTGTACACCCTGCCCCCCTGCA GAGATGAGCTGACCAAGAACCAGGTGTCCCTGTGGTG TCTGGTCAAGGGCTTCTACCCCAGCGATATCGCCGTGG AGTGGGAGAGCAACGGCCAGCCTGAGAACAACTACAA GACCACCCCCCCTGTGCTGGACAGCGACGGCAGCTTCT TCCTGTACTCCAAACTGACCGTGGACAAGAGCCGGTGG CAGCAGGGCAACGTGTTCAGCTGCAGCGTGATGCACG AGGCCCTGCACAACCACTACACCCAGAAGTCCCTGAG |
| | | CCTGAGCCCGGCGGAGGCGGCGGAAGCGGAGGAGGA GGATCTGGGGGCGGAGGTTCCGGAGGCGGAGGATCCG AGGTGCAGCTGCTCGAAAGCGGCGGAGGACTGGTGCA GCCTGGCGGCAGCCTGAGACTGTCTTGCGCCGCCAGCG GCTTCACCTTCAGCAGCTACGCCATGAGCTGGGTCCGC CAGGCCCCTGGCAAGGGACTGGAATGGGTGTCCGCCA TCATCGGCTCTGGCGCCAGCACCTACTACGCCGACAGC GTGAAGGGCCGGTTCACCATCAGCCGGGACAACAGCA AGAACACCCTGTACCTGCAGATGAACAGCCTGCGGGC CGAGGACACCGCCGTGTACTACTGCGCCAAGGATGG TTCGGCGGCTTCAACTACTGGGGACAGGGCACCCTGGT CACCGTGTCCAGC |
| 212 | HC 2 (49B4) _VHCH1 _VHCH1 Fc hole VL (4B9) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGAGAATAC TACCGTGGTCCGTACGACTACTGGGGCCAAGGGACCA CCGTGACCGTCTCCTCAGCTAGCACAAAGGGACCTAGC GTGTTCCCCCTGGCCCCCAGCAGCAAGTCTACATCTGG CGGAACAGCCGCCCTGGGCTGCCTCGTGAAGGACTACT TTCCCGAGCCCGTGACCGTGTCCTGGAACTCTGGCGCT CTGACAAGCGGCGTGCACACCTTTCCAGCCGTGCTGCA GAGCAGCGGCCTGTACTCTCTGAGCAGCGTCGTGACAG TGCCCAGCAGCTCTCTGGGCACCCAGACCTACATCTGC AACGTGAACCACAAGCCCAGCAACACCAAGGTGGACA AGAAGGTGGAACCCAAGAGCTGCGACGGCGGAGGGG GATCTGGCGGCGGAGGATCCCAGGTGCAATTGGTGCA GTCTGGGGCTGAGGTGAAGAAGCCTGGGTCCTCGGTG AAGGTCTCCTGCAAGGCCTCCGGAGGCACATTCAGCA GCTACGCTATAAGCTGGGTGCGACAGGCCCCTGGACA AGGGCTCGAGTGGATGGGAGGGATCATCCCTATCTTTG GTACAGCAAACTACGCACAGAAGTTCCAGGGCAGGGT CACCATTACTGCAGACAAATCCACGAGCACAGCCTAC ATGGAGCTGAGCAGCCTGAGATCTGAGGACACCGCCG TGTATTACTGTGCGAGAGAATACTACCGTGGTCCGTAC GACTACTGGGGCCAAGGGACCACCGTGACCGTCTCCTC AGCTAGCACCAAGGGCCCATCGGTCTTCCCCCTGGCAC CCTCCTCCAAGAGCACCTCTGGGGGCACAGCGGCCCTG GGCTGCCTGGTCAAGGACTACTTCCCCGAACCGGTGAC GGTGTCGTGGAACTCAGGCGCCCTGACCAGCGGCGTG CACACCTTCCCGGCTGTCCTACAGTCCTCAGGACTCTA CTCCCTCAGCAGCGTGGTGACCGTGCCCTCCAGCAGCT TGGGCACCCAGACCTACATCTGCAACGTGAATCACAA GCCCAGCAACACCAAGGTGGACAAGAAAGTTGAGCCC AAATCTTGTGACAAAACTCACACATGCCCACCGTGCCC AGCACCTGAAGCTGCAGGGGGACCGTCAGTCTTCCTCT TCCCCCCAAAACCCAAGGACACCCTCATGATCTCCCGG ACCCCTGAGGTCACATGCGTGGTGGTGGACGTGAGCC ACGAAGACCCTGAGGTCAAGTTCAACTGGTACGTGGA CGGCGTGGAGGTGCATAATGCCAAGACAAAGCCGCGG |
| | | GAGGAGCAGTACAACAGCACGTACCGTGTGGTCAGCG TCCTCACCGTCCTGCACCAGGACTGGCTGAATGGCAAG GAGTACAAGTGCAAGGTCTCCAACAAAGCCCTCGGCG CCCCCATCGAGAAAACCATCTCCAAAGCCAAAGGGCA GCCCCGAGAACCACAGGTGTGCACCCTGCCCCCATCCC GGGATGAGCTGACCAAGAACCAGGTCAGCCTCTCGTG CGCAGTCAAAGGCTTCTATCCCAGCGACATCGCCGTGG AGTGGGAGAGCAATGGGCAGCCGGAGAACAACTACAA GACCACGCCTCCCGTGCTGGACTCCGACGGCTCCTTCT TCCTCGTGAGCAAGCTCACCGTGGACAAGAGCAGGTG GCAGCAGGGGAACGTCTTCTCATGCTCCGTGATGCATG AGGCTCTGCACAACCACTACACGCAGAAGAGCCTCTCC CTGTCTCCGGGTGGAGGCGGCGGAAGCGGAGGAGGAG GATCCGGCGGCGGAGGTTCCGGAGGCGGTGGATCTGA GATCGTGCTGACCCAGTCTCCCGGCACCCTGTCTCTGA GCCCTGGCGAGAGAGCCACCCTGTCCTGCAGAGCCTCC CAGTCCGTGACCTCCTCCTACCTCGCCTGGTATCAGCA GAAGCCCGGCCAGGCCCCTCGGCTGCTGATCAACGTG GGCAGTCGGAGAGCCACCGGCATCCCTGACCGGTTCTC CGGCTCTGGCTCCGGCACCGACTTCACCCTGACCATCT CCCGGCTGGAACCCGAGGACTTCGCCGTGTACTACTGC CAGCAGGGCATCATGCTGCCCCCCACCTTTGGCCAGGG CACCAAGGTGGAAATCAAG |
| 157 | (49B4) VLCL-light chain | see Table 13 |
| 213 | HC 1 (49B4) _VHCH1 _VHCH1 Fc knob VH (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYYRGPYDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCAREYYRGPYDYWGQGTTVTVSSAS TKGPSVFPLAPSSKSTSGGTAALGCLVKDYFPEPVTVSWN SGALTSGVHTFPAVLQSSGLYSLSSVVTVPSSSLGTQTYIC NVNHKPSNTKVDKKVEPKSCDKTHTCPPCPAPEAAGGPS VFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVYTLPPCRD ELTKNQVSLWCLVKGFYPSDIAVEWESNGQPENNYKTTP PVLDSDGSFFLYSKLTVDKSRWQQGNVFSCSVMHEALH NHYTQKSLSLSPGGGGGSGGGGSGGGGSGGGGSEVQLLE SGGGLVQPGGSLRLSCAASGFTFSSYAMSWVRQAPGKGL EWVSAIIGSGASTYYADSVKGRFTISRDNSKNTLYLQMNS LRAEDTAVYYCAKGWFGGFNYWGQGTLVTVSS |
| 214 | HC 2 (49B4) _VHCH1 _VHCH1 Fc hole VL (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYYRGPYDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM |
| | | ELSSLRSEDTAVYYCAREYYRGPYDYWGQGTTVTVSSAS TKGPSVFPLAPSSKSTSGGTAALGCLVKDYFPEPVTVSWN SGALTSGVHTFPAVLQSSGLYSLSSVVTVPSSSLGTQTYIC NVNHKPSNTKVDKKVEPKSCDKTHTCPPCPAPEAAGGPS VFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVCTLPPSRD ELTKNQVSLSCAVKGFYPSDIAVEWESNGQPENNYKTTP PVLDSDGSFFLVSKLTVDKSRWQQGNVFSCSVMHEALH NHYTQKSLSLSPGGGGGSGGGGSGGGGSGGGGSEIVLTQ SPGTLSLSPGERATLSCRASQSVTSSYLAWYQQKPGQAPR LLINVGSRRATGIPDRFSGSGSGTDFTLTISRLEPEDFAVY YCQQGIMLPPTFGQGTKVEIK |
| 155 | (49B4) VLCL-light chain (nucleotide sequence) | see Table 13 |
| 215 | HC 1 (49B4) _VHCH1 _VHCH1 Fc knob VH (28H1) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGAGAATAC TACCGTGGTCCGTACGACTACTGGGGCCAAGGGACCA CCGTGACCGTCTCCTCAGCTAGCACAAAGGGACCTAGC GTGTTCCCCCTGGCCCCCAGCAGCAAGTCTACATCTGG CGGAACAGCCGCCCTGGGCTGCCTCGTGAAGGACTACT TTCCCGAGCCCGTGACCGTGTCCTGGAACTCTGGCGCT CTGACAAGCGGCGTGCACACCTTTCCAGCCGTGCTGCA GAGCAGCGGCCTGTACTCTCTGAGCAGCGTCGTGACAG TGCCCAGCAGCTCTCTGGGCACCCAGACCTACATCTGC AACGTGAACCACAAGCCCAGCAACACCAAGGTGGACA AGAAGGTGGAACCCAAGAGCTGCGACGGCGGAGGGG GATCTGGCGGCGGAGGATCCCAGGTGCAATTGGTGCA GTCTGGGGCTGAGGTGAAGAAGCCTGGGTCCTCGGTG AAGGTCTCCTGCAAGGCCTCCGGAGGCACATTCAGCA GCTACGCTATAAGCTGGGTGCGACAGGCCCCTGGACA AGGGCTCGAGTGGATGGGAGGGATCATCCCTATCTTTG GTACAGCAAACTACGCACAGAAGTTCCAGGGCAGGGT CACCATTACTGCAGACAAATCCACGAGCACAGCCTAC ATGGAGCTGAGCAGCCTGAGATCTGAGGACACCGCCG TGTATTACTGTGCGAGAGAATACTACCGTGGTCCGTAC GACTACTGGGGCCAAGGGACCACCGTGACCGTCTCCTC AGCTAGCACCAAGGGCCCATCGGTCTTCCCCCTGGCAC CCTCCTCCAAGAGCACCTCTGGGGGCACAGCGGCCCTG GGCTGCCTGGTCAAGGACTACTTCCCCGAACCGGTGAC GGTGTCGTGGAACTCAGGCGCCCTGACCAGCGGCGTG CACACCTTCCCGGCTGTCCTACAGTCCTCAGGACTCTA CTCCCTCAGCAGCGTGGTGACCGTGCCCTCCAGCAGCT TGGGCACCCAGACCTACATCTGCAACGTGAATCACAA GCCCAGCAACACCAAGGTGGACAAGAAAGTTGAGCCC AAATCTTGTGACAAAACTCACACATGCCCACCGTGCCC AGCACCTGAAGCTGCAGGGGGACCGTCAGTCTTCCTCT |
| | | TCCCCCCAAAACCCAAGGACACCCTCATGATCTCCCGG ACCCCTGAGGTCACATGCGTGGTGGTGGACGTGAGCC ACGAAGACCCTGAGGTCAAGTTCAACTGGTACGTGGA CGGCGTGGAGGTGCATAATGCCAAGACAAAGCCGCGG GAGGAGCAGTACAACAGCACGTACCGTGTGGTCAGCG TCCTCACCGTCCTGCACCAGGACTGGCTGAATGGCAAG GAGTACAAGTGCAAGGTCTCCAACAAAGCCCTCGGCG CCCCCATCGAGAAAACCATCTCCAAAGCCAAAGGGCA GCCCCGAGAACCACAGGTGTACACCCTGCCCCCCTGCA GAGATGAGCTGACCAAGAACCAGGTGTCCCTGTGGTG TCTGGTCAAGGGCTTCTACCCCAGCGATATCGCCGTGG AGTGGGAGAGCAACGGCCAGCCTGAGAACAACTACAA GACCACCCCCCCTGTGCTGGACAGCGACGGCAGCTTCT TCCTGTACTCCAAACTGACCGTGGACAAGAGCCGGTGG CAGCAGGGCAACGTGTTCAGCTGCAGCGTGATGCACG AGGCCCTGCACAACCACTACACCCAGAAGTCCCTGAG CCTGAGCCCCGGCGGAGGCGGCGGAAGCGGAGGAGGA GGATCCGGAGGAGGGGGAAGTGGCGGCGGAGGATCTG AGGTGCAGCTGCTGGAATCCGGCGGAGGCCTGGTGCA GCCTGGCGGATCTCTGAGACTGTCCTGCGCCGCCTCCG GCTTCACCTTCTCCTCCCACGCCATGTCCTGGGTCCGAC AGGCTCCTGGCAAAGGCCTGGAATGGGTGTCCGCCATC TGGGCCTCCGGCGAGCAGTACTACGCCGACTCTGTGAA GGGCCGGTTCACCATCTCCCGGGACAACTCCAAGAAC ACCCTGTACCTGCAGATGAACTCCCTGCGGGCCGAGGA CACCGCCGTGTACTACTGTGCCAAGGGCTGGCTGGGCA ACTTCGACTACTGGGGCCAGGGCACCCTGGTCACCGTG TCCAGC |
| 216 | HC 2 (49B4) _VHCH1 _VHCH1 Fc hole VL (28H1) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGAGAATAC TACCGTGGTCCGTACGACTACTGGGGCCAAGGGACCA CCGTGACCGTCTCCTCAGCTAGCACAAAGGGACCTAGC GTGTTCCCCCTGGCCCCCAGCAGCAAGTCTACATCTGG CGGAACAGCCGCCCTGGGCTGCCTCGTGAAGGACTACT TCCCGAGCCCGTGACCGTGTCCTGGAACTCTGGCGCT CTGACAAGCGGCGTGCACACCTTTCCAGCCGTGCTGCA GAGCAGCGGCCTGTACTCTCTGAGCAGCGTCGTGACAG TGCCCAGCAGCTCTCTGGGCACCCAGACCTACATCTGC AACGTGAACCACAAGCCCAGCAACACCAAGGTGGACA AGAAGGTGGAACCCAAGAGCTGCGACGGCGGAGGGG GATCTGGCGGCGGAGGATCCCAGGTGCAATTGGTGCA GTCTGGGGCTGAGGTGAAGAAGCCTGGGTCCTCGGTG AAGGTCTCCTGCAAGGCCTCCGGAGGCACATTCAGCA GCTACGCTATAAGCTGGGTGCGACAGGCCCCTGGACA AGGGCTCGAGTGGATGGGAGGGATCATCCCTATCTTTG GTACAGCAAACTACGCACAGAAGTTCCAGGGCAGGGT CACCATTACTGCAGACAAATCCACGAGCACAGCCTAC ATGGAGCTGAGCAGCCTGAGATCTGAGGACACCGCCG |
| | | TGTATTACTGTGCGAGAGAATACTACCGTGGTCCGTAC GACTACTGGGGCCAAGGGACCACCGTGACCGTCTCCTC AGCTAGCACCAAGGGCCCATCGGTCTTCCCCCTGGCAC CCTCCTCCAAGAGCACCTCTGGGGGCACAGCGGCCCTG GGCTGCCTGGTCAAGGACTACTTCCCCGAACCGGTGAC GGTGTCGTGGAACTCAGGCGCCCTGACCAGCGGCGTG CACACCTTCCCGGCTGTCCTACAGTCCTCAGGACTCTA CTCCCTCAGCAGCGTGGTGACCGTGCCCTCCAGCAGCT TGGGCACCCAGACCTACATCTGCAACGTGAATCACAA GCCCAGCAACACCAAGGTGGACAAGAAAGTTGAGCCC AAATCTTGTGACAAAACTCACACATGCCCACCGTGCCC AGCACCTGAAGCTGCAGGGGGACCGTCAGTCTTCCTCT TCCCCCCAAAACCCAAGGACACCCTCATGATCTCCCGG ACCCCTGAGGTCACATGCGTGGTGGTGGACGTGAGCC ACGAAGACCCTGAGGTCAAGTTCAACTGGTACGTGGA CGGCGTGGAGGTGCATAATGCCAAGACAAAGCCGCGG GAGGAGCAGTACAACAGCACGTACCGTGTGGTCAGCG TCCTCACCGTCCTGCACCAGGACTGGCTGAATGGCAAG GAGTACAAGTGCAAGGTCTCCAACAAAGCCCTCGGCG CCCCCATCGAGAAAACCATCTCCAAAGCCAAAGGGCA GCCCCGAGAACCACAGGTGTGCACCCTGCCCCCATCCC GGGATGAGCTGACCAAGAACCAGGTCAGCCTCTCGTG CGCAGTCAAAGGCTTCTATCCCAGCGACATCGCCGTGG AGTGGGAGAGCAATGGGCAGCCGGAGAACAACTACAA GACCACGCCTCCCGTGCTGGACTCCGACGGCTCCTTCT TCCTCGTGAGCAAGCTCACCGTGGACAAGAGCAGGTG GCAGCAGGGGAACGTCTTCTCATGCTCCGTGATGCATG AGGCTCTGCACAACCACTACACGCAGAAGAGCCTCTCC CTGTCTCCGGGTGGAGGCGGCGGAAGCGGAGGAGGAG GATCCGGTGGTGGCGGATCTGGGGGCGGTGGATCTGA GATCGTGCTGACCCAGTCTCCCGGCACCCTGAGCCTGA GCCCTGGCGAGAGAGCCACCCTGAGCTGCAGAGCCAG CCAGAGCGTGAGCCGGAGCTACCTGGCCTGGTATCAG CAGAAGCCCGGCCAGGCCCCCAGACTGCTGATCATCG GCGCCAGCACCCGGGCCACCGGCATCCCCGATAGATTC AGCGGCAGCGGCTCCGGCACCGACTTCACCCTGACCAT CAGCCGGCTGGAACCCGAGGACTTCGCCGTGTACTACT GCCAGCAGGGCCAGGTGATCCCCCCCACCTTCGGCCAG GGCACCAAGGTGGAAATCAAG |
| 157 | (49B4) VLCL-light chain | see Table 13 |
| 217 | HC 1 (49B4) _VHCH1 _VHCH1 Fc knob VH (28H1) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYYRGPYDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCAREYYRGPYDYWGQGTTVTVSSAS TKGPSVFPLAPSSKSTSGGTAALGCLVKDYFPEPVTVSWN SGALTSGVHTFPAVLQSSGLYSLSSVVTVPSSSLGTQTYIC NVNHKPSNTKVDKKVEPKSCDKTHTCPPCPAPEAAGGPS VFLFPPKPKDTLMISRTPEVTCVVVDVSHEDPEVKFNWY |
| | | VDGVEVHNAKTKPREEQYNSTYRVVSVLTVLHQDWLNG KEYKCKVSNKALGAPIEKTISKAKGQPREPQVYTLPPCRD ELTKNQVSLWCLVKGFYPSDIAVEWESNGQPENNYKTTP PVLDSDGSFFLYSKLTVDKSRWQQGNVFSCSVMHEALH NHYTQKSLSLSPGGGGGSGGGGSGGGGSGGGGSEVQLLE SGGGLVQPGGSLRLSCAASGFTFSSHAMSWVRQAPGKGL EWVSAIWASGEQYYADSVKGRFTISRDNSKNTLYLQMNS LRAEDTAVYYCAKGWLGNFDYWGQGTLVTVSS |
| 218 | HC 2 (49B4) _VHCH1 _VHCH1 Fc hole VL (28H1) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYYRGPYDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCAREYYRGPYDYWGQGTTVTVSSAS TKGPSVFPLAPSSKSTSGGTAALGCLVKDYFPEPVTVSWN SGALTSGVHTFPAVLQSSGLYSLSSVVTVPSSSLGTQTYIC NVNHKPSNTKVDKKVEPKSCDKTHTCPPCPAPEAAGGPS VFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVCTLPPSRD ELTKNQVSLSCAVKGFYPSDIAVEWESNGQPENNYKTTP PVLDSDGSFFLVSKLTVDKSRWQQGNVFSCSVMHEALH NHYTQKSLSLSPGGGGGSGGGGSGGGGSGGGGSEIVLTQ SPGTLSLSPGERATLSCRASQSVSRSYLAWYQQKPGQAPR LLIIGASTRATGIPDRFSGSGSGTDFTLTISRLEPEDFAVYY CQQGQVIPPTFGQGTKVEIK |
| 155 | (49B4) VLCL-light chain (nucleotide sequence) | see Table 13 |
| 219 | HC 1 (49B4) _VHCH1 _VHCH1 Fc knob VH (DP47) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGAGAATAC TACCGTGGTCCGTACGACTACTGGGGCCAAGGGACCA CCGTGACCGTCTCCTCAGCTAGCACAAAGGGACCTAGC GTGTTCCCCCTGGCCCCCAGCAGCAAGTCTACATCTGG CGGAACAGCCGCCCTGGGCTGCCTCGTGAAGGACTACT TTCCCGAGCCCGTGACCGTGTCCTGGAACTCTGGCGCT CTGACAAGCGGCGTGCACACCTTTCCAGCCGTGCTGCA GAGCAGCGGCCTGTACTCTCTGAGCAGCGTCGTGACAG TGCCCAGCAGCTCTCTGGGCACCCAGACCTACATCTGC AACGTGAACCACAAGCCCAGCAACACCAAGGTGGACA AGAAGGTGGAACCCAAGAGCTGCGACGGCGGAGGGG GATCTGGCGGCGGAGGATCCCAGGTGCAATTGGTGCA GTCTGGGGCTGAGGTGAAGAAGCCTGGGTCCTCGGTG AAGGTCTCCTGCAAGGCCTCCGGAGGCACATTCAGCA GCTACGCTATAAGCTGGGTGCGACAGGCCCCTGGACA |
| | | AGGGCTCGAGTGGATGGGAGGGATCATCCCTATCTTTG GTACAGCAAACTACGCACAGAAGTTCCAGGGCAGGGT CACCATTACTGCAGACAAATCCACGAGCACAGCCTAC ATGGAGCTGAGCAGCCTGAGATCTGAGGACACCGCCG TGTATTACTGTGCGAGAGAATACTACCGTGGTCCGTAC GACTACTGGGGCCAAGGGACCACCGTGACCGTCTCCTC AGCTAGCACCAAGGGCCCATCGGTCTTCCCCCTGGCAC CCTCCTCCAAGAGCACCTCTGGGGGCACAGCGGCCCTG GGCTGCCTGGTCAAGGACTACTTCCCCGAACCGGTGAC GGTGTCGTGGAACTCAGGCGCCCTGACCAGCGGCGTG CACACCTTCCCGGCTGTCCTACAGTCCTCAGGACTCTA CTCCCTCAGCAGCGTGGTGACCGTGCCCTCCAGCAGCT TGGGCACCCAGACCTACATCTGCAACGTGAATCACAA GCCCAGCAACACCAAGGTGGACAAGAAAGTTGAGCCC AAATCTTGTGACAAAACTCACACATGCCCACCGTGCCC AGCACCTGAAGCTGCAGGGGGACCGTCAGTCTTCCTCT TCCCCCCAAAACCCAAGGACACCCTCATGATCTCCCGG ACCCCTGAGGTCACATGCGTGGTGGTGGACGTGAGCC ACGAAGACCCTGAGGTCAAGTTCAACTGGTACGTGGA CGGCGTGGAGGTGCATAATGCCAAGACAAAGCCGCGG GAGGAGCAGTACAACAGCACGTACCGTGTGGTCAGCG TCCTCACCGTCCTGCACCAGGACTGGCTGAATGGCAAG GAGTACAAGTGCAAGGTCTCCAACAAAGCCCTCGGCG CCCCCATCGAGAAAACCATCTCCAAAGCCAAAGGGCA GCCCCGAGAACCACAGGTGTACACCCTGCCCCCCTGCA GAGATGAGCTGACCAAGAACCAGGTGTCCCTGTGGTG TCTGGTCAAGGGCTTCTACCCCAGCGATATCGCCGTGG AGTGGGAGAGCAACGGCCAGCCTGAGAACAACTACAA GACCACCCCCCCTGTGCTGGACAGCGACGGCAGCTTCT TCCTGTACTCCAAACTGACCGTGGACAAGAGCCGGTGG CAGCAGGGCAACGTGTTCAGCTGCAGCGTGATGCACG AGGCCCTGCACAACCACTACACCCAGAAGTCCCTGAG CCTGAGCCCCGGCGGAGGCGGCGGAAGCGGAGGAGGA GGATCCGGAGGAGGGGGAAGTGGCGGCGGAGGATCTG AGGTGCAATTGTTGGAGTCTGGGGGAGGCTTGGTACA GCCTGGGGGGTCCCTGAGACTCTCCTGTGCAGCCAGCG GATTCACCTTTAGCAGTTATGCCATGAGCTGGGTCCGC CAGGCTCCAGGGAAGGGGCTGGAGTGGGTCTCAGCTA TTAGTGGTAGTGGTGGTAGCACATACTACGCAGACTCC GTGAAGGGCCGGTTCACCATCTCCAGAGACAATTCCAA GAACACGCTGTATCTGCAGATGAACAGCCTGAGAGCC GAGGACACGGCCGTATATTACTGTGCGAAAGGCAGCG GATTTGACTACTGGGGCCAAGGAACCCTGGTCACCGTC TCGAGC |
| 220 | HC 2 (49B4) _VHCH1 _VHCH1 Fc hole VL (DP47) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGAGAATAC TACCGTGGTCCGTACGACTACTGGGGCCAAGGGACCA CCGTGACCGTCTCCTCAGCTAGCACAAAGGGACCTAGC |
| | | GTGTTCCCCCTGGCCCCCAGCAGCAAGTCTACATCTGG CGGAACAGCCGCCCTGGGCTGCCTCGTGAAGGACTACT TTCCCGAGCCCGTGACCGTGTCCTGGAACTCTGGCGCT CTGACAAGCGGCGTGCACACCTTTCCAGCCGTGCTGCA GAGCAGCGGCCTGTACTCTCTGAGCAGCGTCGTGACAG TGCCCAGCAGCTCTCTGGGCACCCAGACCTACATCTGC AACGTGAACCACAAGCCCAGCAACACCAAGGTGGACA AGAAGGTGGAACCCAAGAGCTGCGACGGCGGAGGGG GATCTGGCGGCGGAGGATCCCAGGTGCAATTGGTGCA GTCTGGGGCTGAGGTGAAGAAGCCTGGGTCCTCGGTG AAGGTCTCCTGCAAGGCCTCCGGAGGCACATTCAGCA GCTACGCTATAAGCTGGGTGCGACAGGCCCCTGGACA AGGGCTCGAGTGGATGGGAGGGATCATCCCTATCTTTG GTACAGCAAACTACGCACAGAAGTTCCAGGGCAGGGT CACCATTACTGCAGACAAATCCACGAGCACAGCCTAC ATGGAGCTGAGCAGCCTGAGATCTGAGGACACCGCCG TGTATTACTGTGCGAGAGAATACTACCGTGGTCCGTAC GACTACTGGGGCCAAGGGACCACCGTGACCGTCTCCTC AGCTAGCACCAAGGGCCCATCGGTCTTCCCCCTGGCAC CCTCCTCCAAGAGCACCTCTGGGGGCACAGCGGCCCTG GGCTGCCTGGTCAAGGACTACTTCCCCGAACCGGTGAC GGTGTCGTGGAACTCAGGCGCCCTGACCAGCGGCGTG CACACCTTCCCGGCTGTCCTACAGTCCTCAGGACTCTA CTCCCTCAGCAGCGTGGTGACCGTGCCCTCCAGCAGCT TGGGCACCCAGACCTACATCTGCAACGTGAATCACAA GCCCAGCAACACCAAGGTGGACAAGAAAGTTGAGCCC AAATCTTGTGACAAAACTCACACATGCCCACCGTGCCC AGCACCTGAAGCTGCAGGGGGACCGTCAGTCTTCCTCT TCCCCCCAAAACCCAAGGACACCCTCATGATCTCCCGG ACCCCTGAGGTCACATGCGTGGTGGTGGACGTGAGCC ACGAAGACCCTGAGGTCAAGTTCAACTGGTACGTGGA CGGCGTGGAGGTGCATAATGCCAAGACAAAGCCGCGG GAGGAGCAGTACAACAGCACGTACCGTGTGGTCAGCG TCCTCACCGTCCTGCACCAGGACTGGCTGAATGGCAAG GAGTACAAGTGCAAGGTCTCCAACAAAGCCCTCGGCG CCCCCATCGAGAAAACCATCTCCAAAGCCAAAGGGCA GCCCCGAGAACCACAGGTGTGCACCCTGCCCCCATCCC GGGATGAGCTGACCAAGAACCAGGTCAGCCTCTCGTG CGCAGTCAAAGGCTTCTATCCCAGCGACATCGCCGTGG AGTGGGAGAGCAATGGGCAGCCGGAGAACAACTACAA GACCACGCCTCCCGTGCTGGACTCCGACGGCTCCTTCT TCCTCGTGAGCAAGCTCACCGTGGACAAGAGCAGGTG GCAGCAGGGGAACGTCTTCTCATGCTCCGTGATGCATG AGGCTCTGCACAACCACTACACGCAGAAGAGCCTCTCC CTGTCTCCGGGTGGAGGCGGCGGAAGCGGAGGAGGAG GATCCGGAGGCGGCGGAAGCGGAGGGGGAGGCTCTGA AATTGTGCTGACCCAGAGCCCCGGCACCCTGTCACTGT CTCCAGGCGAAAGAGCCACCCTGAGCTGCAGAGCCAG CCAGAGCGTGTCCAGCTCTTACCTGGCCTGGTATCAGC AGAAGCCCGGACAGGCCCCCAGACTGCTGATCTACGG CGCCTCTTCTAGAGCCACCGGCATCCCCGATAGATTCA GCGGCAGCGGCTCCGGCACCGACTTCACCCTGACAATC AGCAGACTGGAACCCGAGGACTTTGCCGTGTATTACTG CCAGCAGTACGGCAGCAGCCCCCTGACCTTTGGCCAGG |
| | | GCACCAAGGTGGAAATCAAA |
| 157 | (49B4) VLCL-light chain | see Table 13 |
| 221 | HC 1 (49B4) _VHCH1 _VHCH1 Fc knob VH (DP47) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYYRGPYDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCAREYYRGPYDYWGQGTTVTVSSAS TKGPSVFPLAPSSKSTSGGTAALGCLVKDYFPEPVTVSWN SGALTSGVHTFPAVLQSSGLYSLSSVVTVPSSSLGTQTYIC NVNHKPSNTKVDKKVEPKSCDKTHTCPPCPAPEAAGGPS VFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVYTLPPCRD ELTKNQVSLWCLVKGFYPSDIAVEWESNGQPENNYKTTP PVLDSDGSFFLYSKLTVDKSRWQQGNVFSCSVMHEALH NHYTQKSLSLSPGGGGGSGGGGSGGGGSGGGGSEVQLLE SGGGLVQPGGSLRLSCAASGFTFSSYAMSWVRQAPGKGL EWVSAISGSGGSTYYADSVKGRFTISRDNSKNTLYLQMN SLRAEDTAVYYCAKGSGFDYWGQGTLVTVSS |
| 222 | HC 2 (49B4) _VHCH1 _VHCH1 Fc hole VL (DP47) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYYRGPYDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCAREYYRGPYDYWGQGTTVTVSSAS TKGPSVFPLAPSSKSTSGGTAALGCLVKDYFPEPVTVSWN SGALTSGVHTFPAVLQSSGLYSLSSVVTVPSSSLGTQTYIC NVNHKPSNTKVDKKVEPKSCDKTHTCPPCPAPEAAGGPS VFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVCTLPPSRD ELTKNQVSLSCAVKGFYPSDIAVEWESNGQPENNYKTTP PVLDSDGSFFLVSKLTVDKSRWQQGNVFSCSVMHEALH NHYTQKSLSLSPGGGGGSGGGGSGGGGSGGGGSEIVLTQ SPGTLSLSPGERATLSCRASQSVSSSYLAWYQQKPGQAPR LLIYGASSRATGIPDRFSGSGSGTDFTLTISRLEPEDFAVYY CQQYGSSPLTFGQGTKVEIK |

In a further example, the first heavy chain (HC 1) is comprised of two Fab units (VHCH1VHCH1) of the anti-OX40 binder 49B4 followed by Fc knob chain fused by a (G4S) linker to a VL domain of the anti-FAP binder 4B9. The second heavy chain (HC 2) of the construct is comprised of two Fab units (VHCH1VHCH1) of the anti-OX40 binder 49B4 followed Fc hole chain fused by a (G4S) linker to a VH domain of the anti-FAP binder 4B9.

The amino acid sequences for a 4+1 anti-Ox40, anti-FAP construct with a-FAP VL fused to knob and VH fused to hole chain can be found respectively in Table 31.

**Table 31: Amino acid sequences of mature bispecific tetravalent anti-Ox40/ monovalent anti-FAP huIgG1 P329GLALA kih antibody (4+1 format) anti-FAP VH fused to hole and anti-FAP VL fused to knob chain**

| SEQ ID NO: | Description | Sequence |
|---|---|---|
| 157 | (49B4) VLCL-light chain | see Table 13 |
| 233 | HC 1 (49B4) _VHCH1 _VHCH1 Fc knob VL (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYYRGPYDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCAREYYRGPYDYWGQGTTVTVSSAS TKGPSVFPLAPSSKSTSGGTAALGCLVKDYFPEPVTVSWN SGALTSGVHTFPAVLQSSGLYSLSSVVTVPSSSLGTQTYIC NVNHKPSNTKVDKKVEPKSCDKTHTCPPCPAPEAAGGPS VFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVYTLPPCRD ELTKNQVSLWCLVKGFYPSDIAVEWESNGQPENNYKTTP PVLDSDGSFFLYSKLTVDKSRWQQGNVFSCSVMHEALH NHYTQKSLSLSPGGGGGSGGGGSGGGGSGGGGSEIVLTQ SPGTLSLSPGERATLSCRASQSVTSSYLAWYQQKPGQAPR LLINVGSRRATGIPDRFSGSGSGTDFTLTISRLEPEDFAVY YCQQGIMLPPTFGQGTKVEIK |
| 234 | HC 2 (49B4) _VHCH1 _VHCH1 Fc hole VH (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYYRGPYDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCAREYYRGPYDYWGQGTTVTVSSAS TKGPSVFPLAPSSKSTSGGTAALGCLVKDYFPEPVTVSWN SGALTSGVHTFPAVLQSSGLYSLSSVVTVPSSSLGTQTYIC NVNHKPSNTKVDKKVEPKSCDKTHTCPPCPAPEAAGGPS VFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVCTLPPSRD ELTKNQVSLSCAVKGFYPSDIAVEWESNGQPENNYKTTP PVLDSDGSFFLVSKLTVDKSRWQQGNVFSCSVMHEALH NHYTQKSLSLSPGGGGGSGGGGSGGGGSGGGGSEVQLLE SGGGLVQPGGSLRLSCAASGFTFSSYAMSWVRQAPGKGL EWVSAIIGSGASTYYADSVKGRFTISRDNSKNTLYLQMNS |
| | | LRAEDTAVYYCAKGWFGGFNYWGQGTLVTVSS |

In addition, the base pair and amino acid sequences for a 4+1 anti-OX40, anti-FAP construct, that does not contain the P329G LALA mutations, can be found respectively in Table 32.

**Table 32: cDNA and Amino acid sequences of mature bispecific tetravalent anti-Ox40/ monovalent anti-FAP huIgG1 wt kih antibody (4+1 format) with anti-FAP VH fused to knob and anti-FAP VL fused to hole chain**

| SEQ ID NO: | Description | Sequence |
|---|---|---|
| 155 | (49B4) VLCL-light chain (nucleotide sequence) | see Table 13 |
| 235 | HC 1 (49B4) _VHCH1 _VHCH1 Fc wt knob VH (4B9) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGAGAATAC TACCGTGGTCCGTACGACTACTGGGGCCAAGGGACCA CCGTGACCGTCTCCTCAGCTAGCACAAAGGGACCTAGC GTGTTCCCCCTGGCCCCCAGCAGCAAGTCTACATCTGG CGGAACAGCCGCCCTGGGCTGCCTCGTGAAGGACTACT TTCCCGAGCCCGTGACCGTGTCCTGGAACTCTGGCGCT CTGACAAGCGGCGTGCACACCTTTCCAGCCGTGCTGCA GAGCAGCGGCCTGTACTCTCTGAGCAGCGTCGTGACAG TGCCCAGCAGCTCTCTGGGCACCCAGACCTACATCTGC AACGTGAACCACAAGCCCAGCAACACCAAGGTGGACA AGAAGGTGGAACCCAAGAGCTGCGACGGCGGAGGGG GATCTGGCGGCGGAGGATCCCAGGTGCAATTGGTGCA GTCTGGGGCTGAGGTGAAGAAGCCTGGGTCCTCGGTG AAGGTCTCCTGCAAGGCCTCCGGAGGCACATTCAGCA GCTACGCTATAAGCTGGGTGCGACAGGCCCCTGGACA AGGGCTCGAGTGGATGGGAGGGATCATCCCTATCTTTG GTACAGCAAACTACGCACAGAAGTTCCAGGGCAGGGT CACCATTACTGCAGACAAATCCACGAGCACAGCCTAC ATGGAGCTGAGCAGCCTGAGATCTGAGGACACCGCCG TGTATTACTGTGCGAGAGAATACTACCGTGGTCCGTAC GACTACTGGGGCCAAGGGACCACCGTGACCGTCTCCTC AGCTAGCACCAAGGGCCCATCGGTCTTCCCCCTGGCAC CCTCCTCCAAGAGCACCTCTGGGGGCACAGCGGCCCTG GGCTGCCTGGTCAAGGACTACTTCCCCGAACCGGTGAC GGTGTCGTGGAACTCAGGCGCCCTGACCAGCGGCGTG CACACCTTCCCGGCTGTCCTACAGTCCTCAGGACTCTA CTCCCTCAGCAGCGTGGTGACCGTGCCCTCCAGCAGCT TGGGCACCCAGACCTACATCTGCAACGTGAATCACAA GCCCAGCAACACCAAGGTGGACAAGAAAGTTGAGCCC AAATCTTGTGACAAAACTCACACATGCCCACCGTGCCC |
| | | AGCACCTGAACTCCTGGGGGGACCGTCAGTCTTCCTCT TCCCCCCAAAACCCAAGGACACCCTCATGATCTCCCGG ACCCCTGAGGTCACATGCGTGGTGGTGGACGTGAGCC ACGAAGACCCTGAGGTCAAGTTCAACTGGTACGTGGA CGGCGTGGAGGTGCATAATGCCAAGACAAAGCCGCGG GAGGAGCAGTACAACAGCACGTACCGTGTGGTCAGCG TCCTCACCGTCCTGCACCAGGACTGGCTGAATGGCAAG GAGTACAAGTGCAAGGTCTCCAACAAAGCCCTCCCAG CCCCCATCGAGAAAACCATCTCCAAAGCCAAAGGGCA GCCCCGAGAACCACAGGTGTACACCCTGCCCCCCTGCA GAGATGAGCTGACCAAGAACCAGGTGTCCCTGTGGTG TCTGGTCAAGGGCTTCTACCCCAGCGATATCGCCGTGG AGTGGGAGAGCAACGGCCAGCCTGAGAACAACTACAA GACCACCCCCCCTGTGCTGGACAGCGACGGCAGCTTCT TCCTGTACTCCAAACTGACCGTGGACAAGAGCCGGTGG CAGCAGGGCAACGTGTTCAGCTGCAGCGTGATGCACG AGGCCCTGCACAACCACTACACCCAGAAGTCCCTGAG CCTGAGCCCCGGCGGAGGCGGCGGAAGCGGAGGAGGA GGATCTGGGGGCGGAGGTTCCGGAGGCGGAGGATCCG AGGTGCAGCTGCTCGAAAGCGGCGGAGGACTGGTGCA GCCTGGCGGCAGCCTGAGACTGTCTTGCGCCGCCAGCG GCTTCACCTTCAGCAGCTACGCCATGAGCTGGGTCCGC CAGGCCCCTGGCAAGGGACTGGAATGGGTGTCCGCCA TCATCGGCTCTGGCGCCAGCACCTACTACGCCGACAGC GTGAAGGGCCGGTTCACCATCAGCCGGGACAACAGCA AGAACACCCTGTACCTGCAGATGAACAGCCTGCGGGC CGAGGACACCGCCGTGTACTACTGCGCCAAGGGATGG TTCGGCGGCTTCAACTACTGGGGACAGGGCACCCTGGT CACCGTGTCCAGC |
| 236 | HC 2 (49B4) _VHCH1 _VHCH1 Fc wt hole VL (4B9) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGAGAATAC TACCGTGGTCCGTACGACTACTGGGGCCAAGGGACCA CCGTGACCGTCTCCTCAGCTAGCACAAAGGGACCTAGC GTGTTCCCCCTGGCCCCCAGCAGCAAGTCTACATCTGG CGGAACAGCCGCCCTGGGCTGCCTCGTGAAGGACTACT TTCCCGAGCCCGTGACCGTGTCCTGGAACTCTGGCGCT CTGACAAGCGGCGTGCACACCTTTCCAGCCGTGCTGCA GAGCAGCGGCCTGTACTCTCTGAGCAGCGTCGTGACAG TGCCCAGCAGCTCTCTGGGCACCCAGACCTACATCTGC AACGTGAACCACAAGCCCAGCAACACCAAGGTGGACA AGAAGGTGGAACCCAAGAGCTGCGACGGCGGAGGGG GATCTGGCGGCGGAGGATCCCAGGTGCAATTGGTGCA GTCTGGGGCTGAGGTGAAGAAGCCTGGGTCCTCGGTG AAGGTCTCCTGCAAGGCCTCCGGAGGCACATTCAGCA GCTACGCTATAAGCTGGGTGCGACAGGCCCCTGGACA AGGGCTCGAGTGGATGGGAGGGATCATCCCTATCTTTG GTACAGCAAACTACGCACAGAAGTTCCAGGGCAGGGT CACCATTACTGCAGACAAATCCACGAGCACAGCCTAC |
| | | ATGGAGCTGAGCAGCCTGAGATCTGAGGACACCGCCG TGTATTACTGTGCGAGAGAATACTACCGTGGTCCGTAC GACTACTGGGGCCAAGGGACCACCGTGACCGTCTCCTC AGCTAGCACCAAGGGCCCATCGGTCTTCCCCCTGGCAC CCTCCTCCAAGAGCACCTCTGGGGGCACAGCGGCCCTG GGCTGCCTGGTCAAGGACTACTTCCCCGAACCGGTGAC GGTGTCGTGGAACTCAGGCGCCCTGACCAGCGGCGTG CACACCTTCCCGGCTGTCCTACAGTCCTCAGGACTCTA CTCCCTCAGCAGCGTGGTGACCGTGCCCTCCAGCAGCT TGGGCACCCAGACCTACATCTGCAACGTGAATCACAA GCCCAGCAACACCAAGGTGGACAAGAAAGTTGAGCCC AAATCTTGTGACAAAACTCACACATGCCCACCGTGCCC AGCACCTGAACTCCTGGGGGGACCGTCAGTCTTCCTCT TCCCCCCAAAACCCAAGGACACCCTCATGATCTCCCGG ACCCCTGAGGTCACATGCGTGGTGGTGGACGTGAGCC ACGAAGACCCTGAGGTCAAGTTCAACTGGTACGTGGA CGGCGTGGAGGTGCATAATGCCAAGACAAAGCCGCGG GAGGAGCAGTACAACAGCACGTACCGTGTGGTCAGCG TCCTCACCGTCCTGCACCAGGACTGGCTGAATGGCAAG GAGTACAAGTGCAAGGTCTCCAACAAAGCCCTCCCAG CCCCCATCGAGAAAACCATCTCCAAAGCCAAAGGGCA GCCCCGAGAACCACAGGTGTGCACCCTGCCCCCATCCC GGGATGAGCTGACCAAGAACCAGGTCAGCCTCTCGTG CGCAGTCAAAGGCTTCTATCCCAGCGACATCGCCGTGG AGTGGGAGAGCAATGGGCAGCCGGAGAACAACTACAA GACCACGCCTCCCGTGCTGGACTCCGACGGCTCCTTCT TCCTCGTGAGCAAGCTCACCGTGGACAAGAGCAGGTG GCAGCAGGGGAACGTCTTCTCATGCTCCGTGATGCATG AGGCTCTGCACAACCACTACACGCAGAAGAGCCTCTCC CTGTCTCCGGGTGGAGGCGGCGGAAGCGGAGGAGGAG GATCCGGCGGCGGAGGTTCCGGAGGCGGTGGATCTGA GATCGTGCTGACCCAGTCTCCCGGCACCCTGTCTCTGA GCCCTGGCGAGAGAGCCACCCTGTCCTGCAGAGCCTCC CAGTCCGTGACCTCCTCCTACCTCGCCTGGTATCAGCA GAAGCCCGGCCAGGCCCCTCGGCTGCTGATCAACGTG GGCAGTCGGAGAGCCACCGGCATCCCTGACCGGTTCTC CGGCTCTGGCTCCGGCACCGACTTCACCCTGACCATCT CCCGGCTGGAACCCGAGGACTTCGCCGTGTACTACTGC CAGCAGGGCATCATGCTGCCCCCCACCTTTGGCCAGGG CACCAAGGTGGAAATCAAG |
| 157 | (49B4) VLCL-light chain | see Table 13 |
| 237 | HC 1 (49B4) _VHCH1 _VHCH1 Fc wt knob VH (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYYRGPYDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCAREYYRGPYDYWGQGTTVTVSSAS TKGPSVFPLAPSSKSTSGGTAALGCLVKDYFPEPVTVSWN SGALTSGVHTFPAVLQSSGLYSLSSVVTVPSSSLGTQTYIC NVNHKPSNTKVDKKVEPKSCDKTHTCPPCPAPELLGGPS |
| | | VFLFPPKPKDTLMISRTPEVTCVVVDVSHEDPEVKFNWY VDGVEVHNAKTKPREEQYNSTYRVVSVLTVLHQDWLNG KEYKCKVSNKALPAPIEKTISKAKGQPREPQVYTLPPCRD ELTKNQVSLWCLVKGFYPSDIAVEWESNGQPENNYKTTP PVLDSDGSFFLYSKLTVDKSRWQQGNVFSCSVMHEALH NHYTQKSLSLSPGGGGGSGGGGSGGGGSGGGGSEVQLLE SGGGLVQPGGSLRLSCAASGFTFSSYAMSWVRQAPGKGL EWVSAIIGSGASTYYADSVKGRFTISRDNSKNTLYLQMNS LRAEDTAVYYCAKGWFGGFNYWGQGTLVTVSS |
| 238 | HC 2 (49B4) _VHCH1 _VHCH1 Fc wt hole VL (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCAREYYRGPYDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCAREYYRGPYDYWGQGTTVTVSSAS TKGPSVFPLAPSSKSTSGGTAALGCLVKDYFPEPVTVSWN SGALTSGVHTFPAVLQSSGLYSLSSVVTVPSSSLGTQTYIC NVNHKPSNTKVDKKVEPKSCDKTHTCPPCPAPELLGGPS VFLFPPKPKDTLMISRTPEVTCVVVDVSHEDPEVKFNWY VDGVEVHNAKTKPREEQYNSTYRVVSVLTVLHQDWLNG KEYKCKVSNKALPAPIEKTISKAKGQPREPQVCTLPPSRD ELTKNQVSLSCAVKGFYPSDIAVEWESNGQPENNYKTTP PVLDSDGSFFLVSKLTVDKSRWQQGNVFSCSVMHEALH NHYTQKSLSLSPGGGGGSGGGGSGGGGSGGGGSEIVLTQ SPGTLSLSPGERATLSCRASQSVTSSYLAWYQQKPGQAPR LLINVGSRRATGIPDRFSGSGSGTDFTLTISRLEPEDFAVY YCQQGIMLPPTFGQGTKVEIK |

All genes were transiently expressed under control of a chimeric MPSV promoter consisting of the MPSV core promoter combined with the CMV promoter enhancer fragment. The expression vector also contains the oriP region for episomal replication in EBNA (Epstein Barr Virus Nuclear Antigen) containing host cells.

The bispecific anti-Ox40/ anti-FAP 4+1 constructs were produced by co-transfecting HEK293-EBNA cells with the mammalian expression vectors using polyethylenimine (PEI; Polysciences Inc.). The cells were transfected with the corresponding expression vectors in a 1:1:4 ratio ("vector HC1" : "vector HC2" : "vector LC").

For a 200 mL production in 500 mL shake flasks, 250 million HEK293 EBNA cells were seeded 24 hours before transfection in Excell media (Sigma) with supplements. For transfection, the cells were centrifuged for 5 minutes at 210 x g, and supernatant was replaced by pre-warmed CD-CHO medium (Gibco). Expression vectors were mixed in 20 mL CD-CHO medium to a final amount of 200 µg DNA. After addition of 540 µL PEI (1 mg/mL) (Polysciences Inc.), the solution was vortexed for 15 seconds and incubated for 10 minutes at room temperature. Afterwards, cells were mixed with the DNA/PEI solution, transferred to a 500 mL shake flask and incubated for 3 hours at 37 °C in an incubator with a 5% CO₂ atmosphere and shaking at 165 rpm. After the incubation, 160 mL Excell medium with supplements (1 mM valproic acid, 5g/l PepSoy, 6 mM L-Glutamine) was added and cells were cultured for 24 hours. 24h after transfection the cells were supplemented with an amino acid and glucose feed at 12% final volume (24 mL) and 3 g/L glucose (1.2 mL from 500 g/L stock). After culturing for 7 days, the cell supernatant was collected by centrifugation for 45 minutes at 2000-3000 × g. The solution was sterile filtered (0.22 µm filter), supplemented with sodium azide to a final concentration of 0.01 % (w/v), and kept at 4 °C.

Purification of the bispecific constructs from cell culture supernatants was carried out by affinity chromatography using MabSelectSure. The protein was concentrated and filtered prior to loading on a HiLoad Superdex 200 column (GE Healthcare) equilibrated with 20 mM histidine, 140 mM NaCl , 0.01% Tween-20 solution of pH 6.0.

For affinity chromatography, the supernatant was loaded on a ProtA MabSelect Sure column (CV = 5 mL, GE Healthcare) equilibrated with 30 mL 20 mM Sodium Citrate, 20 mM Sodium Phosphate, pH 7.5. Unbound protein was removed by washing with 6- 10 column volumes of a buffer containing 20 mM sodium phosphate, 20 mM sodium citrate (pH 7.5). The bound protein was eluted using either a step or a linear pH-gradient of 15 CVs (from 0 to 100%) of 20 mM Sodium Citrate, 100mM Sodium Chloride, 100 mM Glycine, 0.01% (v/v) Tween-20, pH 3.0. The column was then washed with 10 column volumes of a solution containing 20 mM Sodium Citrate, 100 mM sodium chloride, 100 mM glycine, 0.01% (v/v) Tween-20, pH 3.0 followed by a re-equilibration step.

The pH of the collected fractions was adjusted by adding 1/10 (v/v) of 0.5 M sodium phosphate, pH8.0. The protein was concentrated and filtered prior to loading on a HiLoad Superdex 200 column (GE Healthcare) equilibrated with 20 mM histidine, 140 mM sodium chloride, pH 6.0, 0.01% Tween20.

The protein concentration of purified bispecific constructs was determined by measuring the OD at 280 nm, using the molar extinction coefficient calculated on the basis of the amino acid sequence. Purity and molecular weight of the bispecific constructs were analyzed by CE-SDS in the presence and absence of a reducing agent (Invitrogen) using a LabChipGXII (Caliper). The aggregate content of bispecific constructs was analyzed using a TSKgel G3000 SW XL analytical size-exclusion column (Tosoh) equilibrated in a 25 mM potassium phosphate, 125 mM sodium chloride, 200mM L-arginine monohydrochloride, 0.02 % (w/v) NaN₃, pH 6.7 running buffer at 25 °C (Table 31).

**Table 33: Biochemical analysis of exemplary bispecific, tetravalent anti-Ox40/anti-FAP IgG1 P329G LALA antigen binding molecules (4+1 constructs)**

| Clone | Yield [mg/l] | Monomer [%] | CE-SDS (non red) | CE-SDS (red) |
|---|---|---|---|---|
| OX40(49B4) /FAP(28H1) P329GLALA IgG1 4+1 | 10.24 | 99.14 | 96.95% (269 kDa) | 0.46% (136.4 kDa) |
| | | | 1.61% (261.3 kDa) | 54.4% (103.7 kDa) |
| | | 0.86% | 0.23% (253 kDa) | 0.34% (77.6 kDa) |
| | | HMW | 0.47% (224 kDa) | 3.03% (30.8 kDa) |
| | | | 0.73 % (32 kDa) | 41.74% (28 kDa) |
| OX40(49B4) /FAP(4B9) P329GLALA IgG1 4+1 | 9.22 | 100 | 95.3% (264.2 kDa) | |
| | | | 3.04% (258.8 kDa) | 0.29% (144.9 kDa) |
| | | | 0.56% (253 kDa) | 58.53% (104.9 kDa) |
| | | | 0.33% (225.8kDa) | |
| | | | 0.04% (192 kDa) | 1.05% (31.6 kDa) |
| | | | 0.22% (155.8 kDa) | 40.14 (29.15 kDa) |
| | | | 0.5% (33.4 kDa) | |
| OX40(49B4) / DP47 P329GLALA IgG1 4+1 | 30.88 | 98.29% | 100% (266.9 kDa) | 52.43% (105 kDa) |
| | | 1.71% | | 0.53% (30.9 kDa) |
| | | HMW | | 47.04% (28.4 kDa) |

### 4.5 Generation of bispecific tetravalent antigen binding molecules targeting OX40 and fibroblast activation protein (FAP) (4+2 format)

Bispecific agonistic OX40 antibodies with tetravalent binding for OX40 and with bivalent binding for FAP were prepared. The crossmab technology in accordance with International patent application No. WO 2010/145792 A1 was applied to reduce the formation of wrongly paired light chains.

The generation and preparation of the FAP binders is described in WO 2012/020006 A2.

In this example, a crossed Fab unit (VLCH) of the FAP binder 28H1 was fused to the C-terminus of the Fc part of both heavy chains. Two Fabs against OX40 were fused to the N-terminus of each heavy chain as described in Example 4.3. The CH and CL of the anti-OX40 Fabs contained amino acid mutations (so-called charged residues) in the CHI domain (K147E, K213E, numbering according Kabat EU index) and the CL of the anti-OX40 binder 49B4 (E123R and Q124K, numbering according to Kabat EU index) to prevent the generation of Bence Jones proteins and to further stabilize the correct pairing of the light chains. In this case the introduction of a knob into hole was not necessary as both heavy chains contained the same domains.

The Pro329Gly, Leu234Ala and Leu235Ala mutations were introduced in the constant region of the heavy chains to abrogate binding to Fc gamma receptors according to the method described in International Patent Appl. Publ. No. WO 2012/130831 A1. The resulting bispecific, tetravalent construct is depicted in **Figure 16B**.

**Table 34** shows, respectively, the nucleotide and amino acid sequences of mature bispecific, tetravalent anti-OX40/anti-FAP human IgG1 P329GLALA antibodies.

In addition, an "untargeted" 4+2 construct was prepared, wherein the Fab domains of the anti-FAP binder were replaced by Fab domain of a germline control, termed DP47, not binding to the antigen.

**Table 34: Sequences of bispecific, tetravalent anti-OX40/anti-FAP human IgG1 P329GLALA antigen binding molecules (4+2 format)**

| SEQ ID NO: | Description | Sequence |
|---|---|---|
| 223 | (49B4) VLCL^{∗}-light chain 1 ^{∗}E123R/Q124K (nucleotide sequence) | GACATCCAGATGACCCAGTCTCCTTCCACCCTGTCTGCAT CTGTAGGAGACCGTGTCACCATCACTTGCCGTGCCAGTCA GAGTATTAGTAGCTGGTTGGCCTGGTATCAGCAGAAACC AGGGAAAGCCCCTAAGCTCCTGATCTATGATGCCTCCAGT TTGGAAAGTGGGGTCCCATCACGTTTCAGCGGCAGTGGAT CCGGGACAGAATTCACTCTCACCATCAGCAGCTTGCAGCC TGATGATTTTGCAACTTATTACTGCCAACAGTATAGTTCG CAGCCGTATACGTTTGGCCAGGGCACCAAAGTCGAGATC AAGCGTACGGTGGCTGCACCATCTGTCTTCATCTTCCCGC CATCTGATCGGAAGTTGAAATCTGGAACTGCCTCTGTTGT GTGCCTGCTGAATAACTTCTATCCCAGAGAGGCCAAAGTA CAGTGGAAGGTGGATAACGCCCTCCAATCGGGTAACTCC CAGGAGAGTGTCACAGAGCAGGACAGCAAGGACAGCAC CTACAGCCTCAGCAGCACCCTGACGCTGAGCAAAGCAGA CTACGAGAAACACAAAGTCTACGCCTGCGAAGTCACCCA TCAGGGCCTGAGCTCGCCCGTCACAAAGAGCTTCAACAG GGGAGAGTGT |
| 224 | heavy chain (49B4) _VHCH1 ^{∗}_VHCH 1^{∗} Fc knob VLCH1 (28H1) ^{∗}K147E/K213E (nucleotide sequence) | CAGGTGCAGCTGGTGCAGTCTGGCGCCGAAGTGAAGAAA CCCGGCAGCAGCGTGAAGGTGTCCTGCAAGGCTTCCGGC GGCACCTTCAGCAGCTACGCCATTTCTTGGGTGCGCCAGG CCCCTGGACAGGGCCTGGAATGGATGGGCGGCATCATCC CCATCTTCGGCACCGCCAACTACGCCCAGAAATTCCAGGG CAGAGTGACCATCACCGCCGACAAGAGCACCAGCACCGC CTACATGGAACTGAGCAGCCTGCGGAGCGAGGACACCGC CGTGTACTACTGCGCCAGAGAGTACTACAGAGGCCCCTA CGACTACTGGGGCCAGGGCACAACCGTGACCGTGTCTAG CGCCAGCACAAAGGGCCCCAGCGTGTTCCCTCTGGCCCCT AGCAGCAAGAGCACATCTGGCGGAACAGCCGCCCTGGGC TGCCTGGTGGAAGATTACTTCCCCGAGCCCGTGACAGTGT CCTGGAACTCTGGCGCCCTGACAAGCGGCGTGCACACCTT TCCAGCCGTGCTGCAGAGCAGCGGCCTGTACTCACTGTCC AGCGTCGTGACTGTGCCCAGCAGCAGCCTGGGAACCCAG ACCTACATCTGCAACGTGAACCACAAGCCCAGCAACACC AAGGTGGACGAGAAGGTGGAACCCAAGAGCTGCGACGG CGGAGGCGGATCTGGCGGCGGAGGATCCCAGGTGCAGCT GGTGCAGAGCGGAGCTGAAGTGAAAAAGCCTGGCTCCTC |
| | | CGTGAAAGTGTCTTGTAAAGCCAGCGGCGGCACATTCTCA TCCTACGCCATCAGCTGGGTGCGGCAGGCTCCAGGCCAG GGACTGGAATGGATGGGAGGAATTATCCCTATTTTGGGA CAGCCAATTATGCTCAGAAATTTCAGGGGCGCGTGACAA TTACAGCCGACAAGTCCACCTCTACAGCTTATATGGAACT GTCCTCCCTGCGCTCCGAGGATACAGCTGTGTATTATTGT GCCCGCGAGTACTACCGGGGACCTTACGATTATTGGGGA CAGGGAACCACAGTGACTGTGTCCTCCGCTAGCACCAAG GGACCTTCCGTGTTTCCCCTGGCTCCCAGCTCCAAGTCTA CCTCTGGGGGCACAGCTGCTCTGGGATGTCTGGTGGAAG ATTATTTCCTGAACCTGTGACCGTGTCATGGAACAGCGG AGCCCTGACCTCCGGGGTGCACACATTCCCTGCTGTGCTG CAGTCCTCCGGCCTGTATAGCCTGAGCAGCGTCGTGACCG TGCCTTCCAGCTCTCTGGGCACACAGACATATATCTGTAA TGTGAATCACAAACCCTCTAATACCAAAGTGGATGAGAA AGTGGAACCTAAGTCCTGCGACAAGACCCACACCTGTCC CCCTTGTCCTGCCCCTGAAGCTGCTGGCGGCCCATCTGTG TTTCTGTTCCCCCCAAAGCCCAAGGACACCCTGATGATCA GCCGGACCCCCGAAGTGACCTGCGTGGTGGTGGATGTGT CCCACGAGGACCCAGAAGTGAAGTTCAATTGGTACGTGG ACGGCGTGGAAGTGCACAACGCCAAGACCAAGCCGCGGG AAGAACAGTACAACAGCACCTACCGGGTGGTGTCCGTGC TGACAGTGCTGCACCAGGACTGGCTGAACGGCAAAGAGT ACAAGTGCAAGGTGTCCAACAAGGCCCTGGGAGCCCCCA TCGAGAAAACCATCAGCAAGGCCAAGGGCCAGCCCCGCG AACCTCAGGTGTACACCCTGCCCCCAAGCAGGGACGAGC TGACCAAGAACCAGGTGTCCCTGACCTGTCTCGTGAAGG GCTTCTACCCCTCCGATATCGCCGTGGAATGGGAGAGCAA CGGCCAGCCCGAGAACAACTACAAGACCACCCCCCCTGT GCTGGACAGCGACGGCTCATTCTTCCTGTACTCCAAGCTG ACCGTGGACAAGAGCCGGTGGCAGCAGGGCAACGTGTTC AGCTGCAGCGTGATGCACGAGGCCCTGCACAACCACTAC ACACAGAAGTCTCTGAGCCTGAGCCCTGGCGGAGGGGGA GGATCTGGGGGAGGCGGAAGTGGGGGAGGGGGTTCCGG AGGCGGCGGATCAGAAATTGTGCTGACCCAGTCCCCCGG CACCCTGTCACTGTCTCCAGGCGAAAGAGCCACCCTGAGC TGTAGGGCCTCCCAGAGCGTGTCCAGAAGCTATCTGGCCT GGTATCAGCAGAAGCCCGGACAGGCCCCCAGACTGCTGA TCATTGGCGCCTCTACCAGAGCCACCGGCATCCCCGATAG ATTCAGCGGCTCTGGCAGCGGCACCGACTTCACCCTGACC ATCTCCAGACTGGAACCCGAGGACTTTGCCGTGTACTATT GCCAGCAGGGCCAAGTGATCCCCCCCACCTTTGGCCAGG GAACAAAGGTGGAAATCAAGTCCAGCGCTTCCACCAAGG GCCCCTCAGTGTTCCCACTGGCACCATCCAGCAAGTCCAC AAGCGGAGGAACCGCCGCTCTGGGCTGTCTCGTGAAAGA CTACTTTCCAGAGCCAGTGACCGTGTCCTGGAATAGTGGC GCTCTGACTTCTGGCGTGCACACTTTCCCCGCAGTGCTGC AGAGTTCTGGCCTGTACTCCCTGAGTAGCGTCGTGACAGT GCCCTCCTCTAGCCTGGGCACTCAGACTTACATCTGCAAT GTGAATCATAAGCCTTCCAACACAAAAGTGGACAAAAAA GTGGAACCCAAATCTTGC |
| 225 | (28H1) VHCL-light chain 2 (nucleotide sequence) | GAAGTGCAGCTGCTGGAATCCGGCGGAGGCCTGGTGCAG CCTGGCGGATCTCTGAGACTGTCCTGCGCCGCCTCCGGCT TCACCTTCTCCTCCCACGCCATGTCCTGGGTCCGACAGGC |
| | | TCCTGGCAAAGGCCTGGAATGGGTGTCCGCCATCTGGGCC TCCGGCGAGCAGTACTACGCCGACTCTGTGAAGGGCCGG TTCACCATCTCCCGGGACAACTCCAAGAACACCCTGTACC TGCAGATGAACTCCCTGCGGGCCGAGGACACCGCCGTGT ACTACTGTGCCAAGGGCTGGCTGGGCAACTTCGACTACTG GGGACAGGGCACCCTGGTCACCGTGTCCAGCGCTAGCGT GGCCGCTCCCTCCGTGTTCATCTTCCCACCTTCCGACGAG CAGCTGAAGTCCGGCACCGCTTCTGTCGTGTGCCTGCTGA ACAACTTCTACCCCCGCGAGGCCAAGGTGCAGTGGAAGG TGGACAACGCCCTGCAGTCCGGCAACAGCCAGGAATCCG TGACCGAGCAGGACTCCAAGGACAGCACCTACTCCCTGT CCTCCACCCTGACCCTGTCCAAGGCCGACTACGAGAAGC ACAAGGTGTACGCCTGCGAAGTGACCCACCAGGGCCTGT CTAGCCCCGTGACCAAGTCTTTCAACCGGGGCGAGTGC |
| 226 | (49B4) VLCL^{∗}-light chain 1 ^{∗}E123R/Q124K | DIQMTQSPSTLSASVGDRVTITCRASQSISSWLAWYQQKPGK APKLLIYDASSLESGVPSRFSGSGSGTEFTLTISSLQPDDFATY YCQQYSSQPYTFGQGTKVEIKRTVAAPSVFIFPPSDRKLKSG TASVVCLLNNFYPREAKVQWKVDNALQSGNSQESVTEQDS KDSTYSLSSTLTLSKADYEKHKVYACEVTHQGLSSPVTKSF NRGEC |
| 227 | heavy chain (49B4) _VHCH1 ^{∗}_VHCH 1^{∗} Fc knob VLCH1 (28H1) ^{∗}K147E/K213E | QVQLVQSGAEVKKPGSSVKVSCKASGGTFSSYAISWVRQAP GQGLEWMGGIIPIFGTANYAQKFQGRVTITADKSTSTAYME LSSLRSEDTAVYYCAREYYRGPYDYWGQGTTVTVSSASTK GPSVFPLAPSSKSTSGGTAALGCLVEDYFPEPVTVSWNSGAL TSGVHTFPAVLQSSGLYSLSSVVTVPSSSLGTQTYICNVNHK PSNTKVDEKVEPKSCDGGGGSGGGGSQVQLVQSGAEVKKP GSSVKVSCKASGGTFSSYAISWVRQAPGQGLEWMGGIIPIFG TANYAQKFQGRVTITADKSTSTAYMELSSLRSEDTAVYYCA REYYRGPYDYWGQGTTVTVSSASTKGPSVFPLAPSSKSTSG GTAALGCLVEDYFPEPVTVSWNSGALTSGVHTFPAVLQSSG LYSLSSVVTVPSSSLGTQTYICNVNHKPSNTKVDEKVEPKSC DKTHTCPPCPAPEAAGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALGAPIEKTISKAKG QPREPQVYTLPPSRDELTKNQVSLTCLVKGFYPSDIAVEWES NGQPENNYKTTPPVLDSDGSFFLYSKLTVDKSRWQQGNVFS CSVMHEALHNHYTQKSLSLSPGGGGGSGGGGSGGGGSGGG GSEIVLTQSPGTLSLSPGERATLSCRASQSVSRSYLAWYQQK PGQAPRLLIIGASTRATGIPDRFSGSGSGTDFTLTISRLEPEDF AVYYCQQGQVIPPTFGQGTKVEIKSSASTKGPSVFPLAPSSKS TSGGTAALGCLVKDYFPEPVTVSWNSGALTSGVHTFPAVLQ SSGLYSLSSVVTVPSSSLGTQTYICNVNHKPSNTKVDKKVEP KSC |
| 228 | (28H1) VHCL-light chain 2 | EVQLLESGGGLVQPGGSLRLSCAASGFTFSSHAMSWVRQAP GKGLEWVSAIWASGEQYYADSVKGRFTISRDNSKNTLYLQ MNSLRAEDTAVYYCAKGWLGNFDYWGQGTLVTVSSASVA APSVFIFPPSDEQLKSGTASVVCLLNNFYPREAKVQWKVDN ALQSGNSQESVTEQDSKDSTYSLSSTLTLSKADYEKHKVYA CEVTHQGLSSPVTKSFNRGEC |
| 223 | (49B4) VLCL^{∗}-light chain 1 ^{∗}E123R/Q124K (nucleotide sequence) | see above |
| 229 | heavy chain (49B4) _VHCH1 ^{∗}_VHCH 1^{∗} Fc knob VLCH1 (DP47) ^{∗}K147E/K213E (nucleotide sequence) | CAGGTGCAGCTGGTGCAGTCTGGCGCCGAAGTGAAGAAA CCCGGCAGCAGCGTGAAGGTGTCCTGCAAGGCTTCCGGC GGCACCTTCAGCAGCTACGCCATTTCTTGGGTGCGCCAGG CCCCTGGACAGGGCCTGGAATGGATGGGCGGCATCATCC CCATCTTCGGCACCGCCAACTACGCCCAGAAATTCCAGGG CAGAGTGACCATCACCGCCGACAAGAGCACCAGCACCGC CTACATGGAACTGAGCAGCCTGCGGAGCGAGGACACCGC CGTGTACTACTGCGCCAGAGAGTACTACAGAGGCCCCTA CGACTACTGGGGCCAGGGCACAACCGTGACCGTGTCTAG CGCCAGCACAAAGGGCCCCAGCGTGTTCCCTCTGGCCCCT AGCAGCAAGAGCACATCTGGCGGAACAGCCGCCCTGGGC TGCCTGGTGGAAGATTACTTCCCCGAGCCCGTGACAGTGT CCTGGAACTCTGGCGCCCTGACAAGCGGCGTGCACACCTT TCCAGCCGTGCTGCAGAGCAGCGGCCTGTACTCACTGTCC AGCGTCGTGACTGTGCCCAGCAGCAGCCTGGGAACCCAG ACCTACATCTGCAACGTGAACCACAAGCCCAGCAACACC AAGGTGGACGAGAAGGTGGAACCCAAGAGCTGCGACGG CGGAGGCGGATCTGGCGGCGGAGGATCCCAGGTGCAGCT GGTGCAGAGCGGAGCTGAAGTGAAAAAGCCTGGCTCCTC CGTGAAAGTGTCTTGTAAAGCCAGCGGCGGCACATTCTCA TCCTACGCCATCAGCTGGGTGCGGCAGGCTCCAGGCCAG GGACTGGAATGGATGGGAGGAATTATCCCTATTTTGGGA CAGCCAATTATGCTCAGAAATTTCAGGGGCGCGTGACAA TTACAGCCGACAAGTCCACCTCTACAGCTTATATGGAACT GTCCTCCCTGCGCTCCGAGGATACAGCTGTGTATTATTGT GCCCGCGAGTACTACCGGGGACCTTACGATTATTGGGGA CAGGGAACCACAGTGACTGTGTCCTCCGCTAGCACCAAG GGACCTTCCGTGTTTCCCCTGGCTCCCAGCTCCAAGTCTA CCTCTGGGGGCACAGCTGCTCTGGGATGTCTGGTGGAAG ATTATTTTCCTGAACCTGTGACCGTGTCATGGAACAGCGG AGCCCTGACCTCCGGGGTGCACACATTCCCTGCTGTGCTG CAGTCCTCCGGCCTGTATAGCCTGAGCAGCGTCGTGACCG TGCCTTCCAGCTCTCTGGGCACACAGACATATATCTGTAA TGTGAATCACAAACCCTCTAATACCAAAGTGGATGAGAA AGTGGAACCTAAGTCCTGCGACAAGACCCACACCTGTCC CCCTTGTCCTGCCCCTGAAGCTGCTGGCGGCCCATCTGTG TTTCTGTTCCCCCCAAAGCCCAAGGACACCCTGATGATCA GCCGGACCCCCGAAGTGACCTGCGTGGTGGTGGATGTGT CCCACGAGGACCCAGAAGTGAAGTTCAATTGGTACGTGG ACGGCGTGGAAGTGCACAACGCCAAGACCAAGCCGCGGG AAGAACAGTACAACAGCACCTACCGGGTGGTGTCCGTGC TGACAGTGCTGCACCAGGACTGGCTGAACGGCAAAGAGT ACAAGTGCAAGGTGTCCAACAAGGCCCTGGGAGCCCCCA TCGAGAAAACCATCAGCAAGGCCAAGGGCCAGCCCCGCG AACCTCAGGTGTACACCCTGCCCCCAAGCAGGGACGAGC TGACCAAGAACCAGGTGTCCCTGACCTGTCTCGTGAAGG GCTTCTACCCCTCCGATATCGCCGTGGAATGGGAGAGCAA CGGCCAGCCCGAGAACAACTACAAGACCACCCCCCCTGT GCTGGACAGCGACGGCTCATTCTTCCTGTACTCCAAGCTG ACCGTGGACAAGAGCCGGTGGCAGCAGGGCAACGTGTTC AGCTGCAGCGTGATGCACGAGGCCCTGCACAACCACTAC ACACAGAAGTCTCTGAGCCTGAGCCCTGGCGGAGGGGGA GGATCTGGGGGAGGCGGAAGTGGGGGAGGGGGTTCCGG AGGCGGAGGATCCGAAATCGTGTTAACGCAGTCTCCAGG |
| | | CACCCTGTCTTTGTCTCCAGGGGAAAGAGCCACCCTCTCT TGCAGGGCCAGTCAGAGTGTTAGCAGCAGCTACTTAGCCT GGTACCAGCAGAAACCTGGCCAGGCTCCCAGGCTCCTCA TCTATGGAGCATCCAGCAGGGCCACTGGCATCCCAGACA GGTTCAGTGGCAGTGGATCCGGGACAGACTTCACTCTCAC CATCAGCAGACTGGAGCCTGAAGATTTTGCAGTGTATTAC TGTCAGCAGTATGGTAGCTCACCGCTGACGTTCGGCCAGG GGACCAAAGTGGAAATCAAAAGCAGCGCTTCCACCAAGG GCCCCTCAGTGTTCCCACTGGCACCATCCAGCAAGTCCAC AAGCGGAGGAACCGCCGCTCTGGGCTGTCTCGTGAAAGA CTACTTTCCAGAGCCAGTGACCGTGTCCTGGAATAGTGGC GCTCTGACTTCTGGCGTGCACACTTTCCCCGCAGTGCTGC AGAGTTCTGGCCTGTACTCCCTGAGTAGCGTCGTGACAGT GCCCTCCTCTAGCCTGGGCACTCAGACTTACATCTGCAAT GTGAATCATAAGCCTTCCAACACAAAAGTGGACAAAAAA GTGGAACCCAAATCTTGC |
| 230 | (DP47) VHCL-light chain 2 (nucleotide sequence) | GAGGTGCAATTGTTGGAGTCTGGGGGAGGCTTGGTACAG CCTGGGGGGTCCCTGAGACTCTCCTGTGCAGCCTCCGGAT TCACCTTTAGCAGTTATGCCATGAGCTGGGTCCGCCAGGC TCCAGGGAAGGGGCTGGAGTGGGTCTCAGCTATTAGTGG TAGTGGTGGTAGCACATACTACGCAGACTCCGTGAAGGG CCGGTTCACCATCTCCAGAGACAATTCCAAGAACACGCTG TATCTGCAGATGAACAGCCTGAGAGCCGAGGACACGGCC GTATATTACTGTGCGAAAGGCAGCGGATTTGACTACTGGG GCCAAGGAACCCTGGTCACCGTCTCGAGTGCTAGCGTGG CCGCTCCCTCCGTGTTCATCTTCCCACCTTCCGACGAGCA GCTGAAGTCCGGCACCGCTTCTGTCGTGTGCCTGCTGAAC AACTTCTACCCCCGCGAGGCCAAGGTGCAGTGGAAGGTG GACAACGCCCTGCAGTCCGGCAACAGCCAGGAATCCGTG ACCGAGCAGGACTCCAAGGACAGCACCTACTCCCTGTCCT CCACCCTGACCCTGTCCAAGGCCGACTACGAGAAGCACA AGGTGTACGCCTGCGAAGTGACCCACCAGGGCCTGTCTA GCCCCGTGACCAAGTCTTTCAACCGGGGCGAGTGC |
| 226 | (49B4) VLCL^{∗}-light chain 1 ^{∗}E123R/Q124K | see above |
| 231 | heavy chain (49B4) _VHCH1 ^{∗}_VHCH 1^{∗} Fc knob VLCH1 (DP47) ^{∗}K147E/K213E | QVQLVQSGAEVKKPGSSVKVSCKASGGTFSSYAISWVRQAP GQGLEWMGGIIPIFGTANYAQKFQGRVTITADKSTSTAYME LSSLRSEDTAVYYCAREYYRGPYDYWGQGTTVTVSSASTK GPSVFPLAPSSKSTSGGTAALGCLVEDYFPEPVTVSWNSGAL TSGVHTFPAVLQSSGLYSLSSVVTVPSSSLGTQTYICNVNHK PSNTKVDEKVEPKSCDGGGGSGGGGSQVQLVQSGAEVKKP GSSVKVSCKASGGTFSSYAISWVRQAPGQGLEWMGGIIPIFG TANYAQKFQGRVTITADKSTSTAYMELSSLRSEDTAVYYCA REYYRGPYDYWGQGTTVTVSSASTKGPSVFPLAPSSKSTSG GTAALGCLVEDYFPEPVTVSWNSGALTSGVHTFPAVLQSSG LYSLSSVVTVPSSSLGTQTYICNVNHKPSNTKVDEKVEPKSC DKTHTCPPCPAPEAAGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALGAPIEKTISKAKG QPREPQVYTLPPSRDELTKNQVSLTCLVKGFYPSDIAVEWES NGQPENNYKTTPPVLDSDGSFFLYSKLTVDKSRWQQGNVFS CSVMHEALHNHYTQKSLSLSPGGGGGSGGGGSGGGGSGGG GSEIVLTQSPGTLSLSPGERATLSCRASQSVSSSYLAWYQQK |
| | | PGQAPRLLIYGASSRATGIPDRFSGSGSGTDFTLTISRLEPEDF AVYYCQQYGSSPLTFGQGTKVEIKSSASTKGPSVFPLAPSSK STSGGTAALGCLVKDYFPEPVTVSWNSGALTSGVHTFPAVL QSSGLYSLSSVVTVPSSSLGTQTYICNVNHKPSNTKVDKKVE PKSC |
| 232 | (DP47) VHCL-light chain 2 | EVQLLESGGGLVQPGGSLRLSCAASGFTFSSYAMSWVRQAP GKGLEWVSAISGSGGSTYYADSVKGRFTISRDNSKNTLYLQ MNSLRAEDTAVYYCAKGSGFDYWGQGTLVTVSSASVAAPS VFIFPPSDEQLKSGTASVVCLLNNFYPREAKVQWKVDNALQ SGNSQESVTEQDSKDSTYSLSSTLTLSKADYEKHKVYACEV THQGLSSPVTKSFNRGEC |

All genes were transiently expressed under control of a chimeric MPSV promoter consisting of the MPSV core promoter combined with the CMV promoter enhancer fragment. The expression vector also contains the oriP region for episomal replication in EBNA (Epstein Barr Virus Nuclear Antigen) containing host cells.

The bispecific anti-Ox40/ anti-FAP constructs were produced by co-transfecting HEK293-EBNA cells with the mammalian expression vectors using polyethylenimine (PEI; Polysciences Inc.). The cells were transfected with the corresponding expression vectors in a 1:2:1 ratio ("vector heavy chain" : "vector light chain 1 " : "vector light chain 2 ").

For a 200 mL production in 500 mL shake flasks, 250 million HEK293 EBNA cells were seeded 24 hours before transfection in Excell (Sigma) media with supplements. For transfection, the cells were centrifuged for 5 minutes at 210 x g, and supernatant was replaced by pre-warmed CD-CHO medium (Gibco). Expression vectors were mixed in 20 mL CD-CHO medium (Gibco) to a final amount of 200 µg DNA. After addition of 540 µL PEI (1 mg/mL) (Polysciences Inc.), the solution was vortexed for 15 seconds and incubated for 10 minutes at room temperature. Afterwards, cells were mixed with the DNA/PEI solution, transferred to a 500 mL shake flask and incubated for 3 hours at 37°C in an incubator with a 5% CO₂ atmosphere and shaking at 165 rpm. After the incubation, 160 mL Excell medium (Sigma) with supplements (1 mM valproic acid, 5g/l PepSoy, 6 mM L-Glutamine) was added and cells were cultured for 24 hours. 24h after transfection the cells were supplement with an amino acid and glucose feed at 12% final volume (24 mL) and 3 g/L glucose (1.2 mL from 500 g/L stock). After culturing for 7 days, the cell supernatant was collected by centrifugation for 45 minutes at 2000-3000 x g. The solution was sterile filtered (0.22 µm filter), supplemented with sodium azide to a final concentration of 0.01 % (w/v), and kept at 4°C.

For affinity chromatography, the supernatant was loaded on a ProtA MabSelect Sure column (CV = 5 mL, GE Healthcare) equilibrated with 30 mL 20 mM sodium citrate, 20 mM sodium phosphate, pH 7.5. Unbound protein was removed by washing with 6-10 column volumes of a buffer containing 20 mM sodium phosphate, 20 mM sodium citrate, pH 7.5. The bound protein was eluted using a step elution with 8 CV of 20 mM Sodium Citrate, 100mM Sodium Chloride, 100 mM Glycine, pH 3.0.

The pH of the collected fractions was adjusted by adding 1/10 (v/v) of 0.5 M sodium phosphate pH8.0. The protein was concentrated and filtered prior to loading on a HiLoad Superdex 200 column (GE Healthcare) equilibrated with 20 mM histidine, 140 mM NaCl, 0.01% Tween20, pH 6.0.

The protein concentration of purified bispecific tetravalent 4+2 constructs was determined by measuring the OD at 280 nm, using the molar extinction coefficient calculated on the basis of the amino acid sequence. Purity and molecular weight of the bispecific constructs were analyzed by CE-SDS in the presence and absence of a reducing agent (Invitrogen) using a LabChipGXII (Caliper). The aggregate content of bispecific constructs was analyzed using a TSKgel G3000 SW XL analytical size-exclusion column (Tosoh) equilibrated in a 25 mM potassiumphosphate, 125 mM sodiumchloride, 200 mM L-arginine monohydrocloride, 0.02 % (w/v) NaN₃, pH 6.7 running buffer at 25°C (Table 35).

**Table 35: Biochemical analysis of exemplary bispecific, tetravalent anti-Ox40/anti-FAP IgG1 P329G LALA antigen binding molecules (4+2 constructs)**

| Construct | Yield [mg/l] | Monomer [%] | CE-SDS (non red) | CE-SDS (red) |
|---|---|---|---|---|
| OX40(49B4) /FAP(28H1) P329GLALA IgG1 4+2 | 1.21 | 99.3 0.7 HMW | 96.2 % | 3.74% (145.6 kDa) |
| | | | | 33.06% (121.34 kDa) |
| | | | | 0.46% (31.6 kDa) |
| | | | | 45.48% (29.2 kDa) |
| | | | | 16.93% (26.65 kDa) |
| OX40(49B4) / DP47 P329GLALA IgG1 4+2 | 1.32 | 99.2 0.8 HMW | 90.4 % | 4.24% (145.28 kDa) |
| | | | | 31.28% (121.3 kDa) |
| | | | | 0.5% (31.65 kDa) |
| | | | | 0.38% (30.25 kDa) |
| | | | | 48.93% (29.22 kDa) |
| | | | | 14.67% (26.89 kDa) |

### 4.6 Determination of the aggregation temperature of the bispecific tetravalent 4+1 and 4+2 anti-OX40 antigen binding molecules

For direct comparison of all formats the thermal stability was monitored by Static Light Scattering (SLS) and by measuring the intrinsic protein fluorescence in response to applied temperature stress. 30 µg of filtered protein sample with a protein concentration of 1 mg/ml was applied in duplicate to an Optim 2 instrument (Avacta Analytical Ltd). The temperature was ramped from 25 to 85 °C at 0.1 °C/min, with the radius and total scattering intensity being collected. For determination of intrinsic protein fluorescence the sample was excited at 266 nm and emission was collected between 275 nm and 460 nm.

For all constructs the aggregation temperature was between 49°C and 67 °C depending on the binder combination and format.

### 4.7 Characterization of bispecific, tetravalent constructs targeting Ox40 and FAP

### 4.7.1 Binding to human FAP-expressing tumor cells

The binding to cell surface FAP was tested using WM266-4 cells (ATCC CRL-1676). 0.5 × 10⁵ WM266-4 cells were added to each well of a round-bottom suspension cell 96-well plates (greiner bio-one, cellstar, Cat. No. 650185). Cells were stained for 120 minutes at 4°C in the dark in 50 µL/well 4 °C cold FACS buffer (DPBS (Gibco by Life Technologies, Cat. No. 14190 326) w/ BSA (0.1 % v/w, Sigma-Aldrich, Cat. No. A9418) containing titrated anti-OX40 antibody constructs. After three times washing with excess FACS buffer, cells were stained for 45 minutes at 4°C in the dark in 25 µL/well 4 °C cold FACS buffer containing Fluorescein isothiocyanate (FITC)-conjugated AffiniPure anti-human IgG Fcy-fragment-specific goat IgG F(ab')₂ fragment (Jackson ImmunoResearch, Cat.-No. 109-096-098).

Plates were finally resuspended in 85 µL/well FACS-buffer containing 0.2 µg/mL DAPI (Santa Cruz Biotec, Cat. No. Sc-3598) and acquired the same day using 5-laser LSR-Fortessa (BD Bioscience with DIVA software).

As shown in **Figure 17****,** the FAP-targeted bispecific tetravalent OX40 constructs but not the untargeted OX40 constructs efficiently bound to human FAP-expressing target cells. Therefore only FAP- targeted anti-OX40 constructs show direct tumor-targeting properties. For the 4+1 format, FAP clone 4B9 had a much stronger binding to human FAP than FAP clone 28H1, where under used washing conditions hardly any binding was observed with flow cytometric analysis. For clone 28H1, the 2+2 and 4+2 constructs (filled circle and triangle) showed stronger binding to FAP than the 4+1 constructs (square) explained by a gain of avidity in the bivalent relative to the monovalent format. The difference in FAP binding between the tetravalent and the bivalent 49B4 construct ( filled triangle versus circle), which contain the same bivalent FAP binding moiety, hints that there are already steric hindrances in FAP binding, maybe due to the bigger size of the tetravalent construct. EC₅₀ values of binding to activated human and cynomolgus CD4 T cells (measured as described in Examples 4.3.2.1 and 4.3.2.2) and FAP positive tumor cells are summarized in **Table 36**.

**Table 36: EC₅₀ values for binding of selected aOX40 binder (clone 49B9) in different constructs to cell surface human FAP and human Ox40**

| Construct | anti-FAP clone | human FAP+ cell EC₅₀ [nM] | human OX40+ cell EC₅₀ [nM] | cynomolgus OX40+ cell EC₅₀ [nM] |
|---|---|---|---|---|
| hu IgG1 | n.t. | - | 907.17 | 0.05 |
| 2+2 | 28H1 | 1.31 | 5.34 | 0.13 |
| 4+1 | 28H1 | n.d. | 0.08 | 0.02 |
| 4+1 | 4B9 | 6.66 | 0.16 | 0.03 |
| 4+1 | n.t. | - | 0.13 | 0.01 |
| 4+2 | 28H1 | 5.51 | 0.13 | 0.01 |
| 4+2 | n.t. | - | 0.11 | 0.07 |

| | | | | |
|---|---|---|---|---|
| n.t. = non targeted, n.d. = not detected | | | | |

### 4.7.2 Surface plasmon resonance

The capacity of the bispecific constructs to bind human, murine and cynomolgus FAP was assessed by surface plasmon resonance (SPR). All SPR experiments were performed on a Biacore T200 (Biacore) at 25 °C with HBS-EP as running buffer (0.01 M HEPES pH 7.4, 0.15 M NaCl, 3 mM EDTA, 0.005% Surfactant P20, (Biacore).

His-tagged human, murine or cynomolgus monkey dimeric FAP was captured on a CM5 chip (GE Healthcare) immobilized with anti-His antibody (Qiagen Cat. No. 34660) by injection of 500 nM huFAP for 60 s at a flow rate of 10 uL/min, 10 nM murine FAP for 20 s at a flow rate of 20 uL/min and 10 nM cynoFAP for 20s at a flow rate of 20 Ll/min. Immobilization levels for the anti-His antibody of up to 18000 resonance units (RU) were used.

Following the capture step, the bispecific constructs as well as control molecules were immediately passed over the chip surface at a concentration ranging from 0.006-100 nM with a flow rate of 30 µL/minute for 280 s and a dissociation phase of 180 s. Bulk refractive index differences were corrected for by subtracting the response obtained in a reference flow cell, where no FAP was immobilized. Affinity was determined using the Langmuir 1:1 curve fitting. For bivalent binding the same 1:1 fitting was used leading to an apparent K_{D} value.

**Table 37: Binding of exemplary bispecific anti-Ox40/anti-FAP antigen binding molecules to recombinant human FAP, murine FAP and cynomolgus FAP**

| Construct | hu FAP K_{D} (M) | mu FAP K_{D} (M) | cyno FAP K_{D} (M) |
|---|---|---|---|
| OX40(49B4) /FAP(28H1) P329GLALA IgG1 4+1 | 2.44E-08 | 5.40E-11 | 4.19E-08 |
| OX40(49B4) /FAP(4B9) P329GLALA IgG1 4+1 | 1.69E-09 | 9.38E-08 | 1.54E-09 |
| OX40(49B4) /FAP(28H1) P329GLALA IgG1 4+2 | 6.38E-10 | 4.41E-12 | 6.10E-10 |
| OX40(49B4) /FAP(28H1) P329GLALA IgG1 1+1 | 3.60E-09 | 1.63E-12 | 4.37E-09 |
| OX40(49B4) /FAP(4B9) P329GLALA IgG1 1+1 | 7.52E-10 | 4.79E-09 | 5.32E-10 |
| 28H1 IgG | 9.33E-10 | 4.90E-13 | 6.69E-10 |
| 49B4 IgG | 9.98E-11 | 6.44E-11 | 4.84E-11 |

### 4.7.3 Binding to OX40

### 4.7.3.1 Binding to human Ox40 expressing cells: naïve and activated human peripheral mononuclear blood leukocytes (PBMC)

Buffy coats were obtained from the Zurich blood donation center. To isolate fresh peripheral blood mononuclear cells (PBMCs) the buffy coat was diluted with the same volume of DPBS (Gibco by Life Technologies, Cat. No. 14190 326). 50 mL polypropylene centrifuge tubes (TPP, Cat.-No. 91050) were supplied with 15 mL Histopaque 1077 (SIGMA Life Science, Cat.-No. 10771, polysucrose and sodium diatrizoate, adjusted to a density of 1.077 g/mL) and the buffy coat solution was layered above the Histopaque 1077. The tubes were centrifuged for 30 min at 400 x g, room temperature and with low acceleration and no break. Afterwards the PBMCs were collected from the interface, washed three times with DPBS and resuspended in T cell medium consisting of RPMI 1640 medium (Gibco by Life Technology, Cat. No. 42401-042) supplied with 10 % Fetal Bovine Serum (FBS, Gibco by Life Technology, Cat. No. 16000-044, Lot 941273, gamma-irradiated, mycoplasma-free and heat inactivated at 56 °C for 35 min), 1 % (v/v) GlutaMAX I (GIBCO by Life Technologies, Cat. No. 35050 038), 1 mM Sodium-Pyruvat (SIGMA, Cat. No. S8636), 1 % (v/v) MEM non-essential amino acids (SIGMA, Cat.-No. M7145) and 50 µM · -Mercaptoethanol (SIGMA, M3148).

PBMCs were used directly after isolation (binding on resting human PBMCs) or they were stimulated to receive a strong human Ox40 expression on the cell surface of T cells (binding on activated human PBMCs). Therefore naive PBMCs were cultured for four days in T cell medium supplied with 200 U/mL Proleukin (Novartis) and 2 ug/mL PHA-L (Sigma-Aldrich, L2769-10) in 6-well tissue culture plate and then 1 day on pre-coated 6-well tissue culture plates [4 ug/mL] anti-human CD3 (clone OKT3, eBioscience, Ca.No. 16-0037-85) and [2 ug/mL] anti-human CD28 (clone CD28.2, eBioscience, Cat No. 16-0289-85] in T cell medium supplied with 200 U/mL Proleukin at 37 °C and 5% CO₂.

For detection of OX40 naïve human PBMC and activated human PBMC were mixed. To enable distinction of naive from activated human PBMC naive cells were labeled prior to the binding assay using the eFluor670 cell proliferation dye (eBioscience, Cat.-No.65-0840-85).

For labeling cells were harvested, washed with pre-warmed (37°C) DPBS and adjusted to a cell density of 1 × 10⁷ cells/mL in DPBS. eFluor670 cell proliferation dye (eBioscience, Cat.-No.65-0840-85 ) was added to the suspension of naive human PBMC at a final concentration of 2.5 mM and a final cell density of 0.5 × 107 cells/mL in DPBS. Cells were then incubated for 10 min at room temperature in the dark. To stop labeling reaction 4 mL heat inactivated FBS were added and cells were washed three times with T cell medium. A two to one mixture of 1 × 105 resting eFluor670 labeled human PBMC and 0.5 × 105 unlabeled activated human PBMC were then added to each well of a round-bottom suspension cell 96-well plates (greiner bio-one, cellstar, Cat. No. 650185).

Cells were stained for 120 minutes at 4 °C in the dark in 50 µL/well 4 °C cold FACS buffer containing titrated anti -Ox40 antibody constructs. After three times washing with excess FACS buffer, cells were stained for 45 minutes at 4°C in the dark in 25 µL/well 4 °C cold FACS buffer containing a mixture of fluorescently labeled anti-human CD4 (clone RPA-T4, mouse IgG1 k, BioLegend, Cat.-No. 300532), anti-human CD8 (clone RPa-T8, mouse IgGlk, BioLegend, Cat.-No. 3010441) and Fluorescein isothiocyanate (FITC)-conjugated AffiniPure anti-human IgG Fcy-fragment-specific goat IgG F(ab')2 fragment (Jackson ImmunoResearch, Cat. No. 109 096 098).

Plates were finally resuspended in 85 µL/well FACS-buffer containing 0.2 µg/mL DAPI (Santa Cruz Biotec, Cat. No. Sc-3598) and acquired the same day using 5-laser LSR-Fortessa (BD Bioscience with DIVA software).

As shown in **Figure 18** and **Figure 19**, no antibody construct specific for OX40 bound to resting human CD4+ T-cells or CD8+ T-cells, which are negative for OX40. In contrast, all constructs bound to activated CD8+ or CD4+ T-cells, which do express OX40. Binding to CD4+ T-cells was much stronger than that to CD8+ T cells. Activated human CD8+ T cells do express only a fraction of the OX40 levels detected on activated CD4+ T cells. Expression levels for OX40 are depending on kinetic and strength of stimulation and conditions were here optimized for OX40 expression on CD4+ T cells but not for CD8+ T cells. Thus, only little Ox40 expression was induced on CD8 T cells. The analyzed bispecific anti-OX40 constructs varied in their binding strength (EC50 values see Table 34). Tetravalent OX40 binding (circle and square) strongly increased avidity for OX40. In comparison, both bivalent constructs (triangle) showed a weaker binding to CD4+ T cells. The presence of a FAP binding moiety had no impact on OX40 binding for the tetravalent OX40 binders (e.g. by sterical hindrance, compare open vs filled symbols). The binding of clone 49B9 in a bivalent, bispecific construct however was clearly stronger than that in the conventional IgG format.

### 4.7.3.2 Binding to cynomolgus OX40 expressing cells: activated cynomolgus peripheral mononuclear blood leukocytes (PBMC)

To test the reactivity of anti-OX40 constructs with cynomolgus cells, PBMC of healthy Macaca fascicularis were isolated from heparinized blood using density gradient centrifugation as described for human cells in section 4.7.3.1 with minor modifications. Cynomolgus PBMC were isolated with density gradient centrifugation from heparinized fresh blood using Histopaque 1077 (SIGMA Life Science, Cat.-No. 10771, polysucrose and sodium diatrizoate, adjusted to a density of 1.077 g/mL) diluted with DPBS (90% v/v). Centrifugation was performed at 520 x g, without brake at room temperature for 30 minutes. PBMCs were stimulated to receive a strong OX40 expression on the cell surface of T cells (binding on activated cynomolgus PBMCs). Therefore naive PBMCs were cultured for 72 hrs on pre-coated 12-well tissue culture plates [10 ug/mL] cynomolgus cross-reactive anti-human CD3 (clone SP34-2, BDBioscience, Cat No. 551916) and [2 ug/mL] cynomolgus cross-reactive anti-human CD28 (clone CD28.2, eBioscience, Cat No. 16-0289-85] in T cell medium supplied with 200 U/mL Proleukin at 37 °C and 5% CO2.

0.5 × 105 activated cynomolgus PBMC were then added to each well of a round-bottom suspension cell 96-well plates (greiner bio-one, cellstar, Cat. No. 650185). Cell were washed once with 200 µL 4 °C cold FACS buffer and were incubated in 50 µL/well of 4 °C cold FACS containing titrated anti -Ox40 antibody constructs for 120 minutes at 4 °C. Then, plates were washed four times with 200 µL/well 4 °C FACS buffer. Cells were resuspended in 25 µL/well 4 °C cold FACS buffer containing fluorescently labeled, cynomolgus cross-reactive anti-human CD4 (clone OKT-4, mouse IgG1 k, BD, Cat.-No. 317428), anti-human CD8 (clone HIT8a, mouse IgGlk, BD, Cat.-No. 555369) and FITC-conjugated AffiniPure anti-human IgG Fcγ-fragment-specific goat IgG F(ab')2 fragment (Jackson ImmunoResearch, Cat. No. 109 096 098) and incubated for 60 minutes at 4 °C in the dark. Plates where washed twice with 200 µL/well 4 °C FACS buffer, were finally resuspended in 80 µL/well FACS-buffer containing 0.2 µg/mL DAPI (Santa Cruz Biotec, Cat. No. Sc-3598) and acquired the same day using 5-laser LSR-Fortessa (BD Bioscience with DIVA software).

As shown in **Figure 20****,** all 49B4 constructs bound to activated CD4+ cynomolgus T-cells. The analyzed anti-OX40 antibody constructs varied in their binding strength (EC50 values summarized in Table 34). Constructs with a tetravalent OX40 binding moiety showed a stronger binding to Ox40+ T cells than bivalent constructs. The gain in avidity between tetravalent and bivalent binding to OX40 was less strong than what was observed for human OX40 (~5x gain versus ~50x gain, compare values in Table 34). Binding to CD4+ T-cells was much stronger than that to CD8+ T cells. Expression levels for OX40 are depending on kinetic and strength of stimulation and were optimized for CD4+ cynomolgus T cells but not for CD8+ cynomolgus T cells, so that only little OX40 expression was induced on CD8+ T cells.

### 4.7.3.3 Binding to humanOX40 - competition binding of bivalent vs tetravalent Ox40 binding

To confirm the ability of all four anti-OX40 Fab domains to bind to huOX40, a cell-based FRET assay (TagLite) was applied. Therefore, 900 Hek293 EBNA cells/well transfected with huOX40-SNAP fusion and labeled with the FRET donor Terbium (Cisbio) were mixed with 1.56 nM (49B4) IgG labeled with the FRET acceptor d2 (Cisbio). Additionally, a concentration dilution ranging from 0.01-750 nM from either (49B4) IgG or bispecific construct 49B4/28H1 (4+1) was added and incubated for 2-4 hours at RT. The fluorescent signal was measured at 620 nm for the fluorescent donor (Terbium) and at 665 nm for the fluorescent acceptor dye (M100 Pro, Tecan). The ratio of 665/620^{∗}1000 was calculated, and the reference (cells only) was subtracted (**Figure 21A**). For EC₅₀ determination the results were analysed in Graph Pad Prism5. The observed EC₅₀ values are shown in Table 38.

**Table 38: EC₅₀ values for competition binding of of bivalent vs tetravalent OX40 antigen binding molecules**

| Construct | EC₅₀ (nM) |
|---|---|
| 49B4 IgG1 | 12.7 (5.8-25) |
| OX40(49B4) /FAP(28H1) P329GLALA IgG1 4+1 | 0.45 (0.35-0.58) |

### 4.7.4 Simultaneous binding to OX40 and FAP

The capacity of binding simultaneously human OX40 Fc (kih) and human FAP was assessed by surface plasmon resonance (SPR). All SPR experiments were performed on a Biacore T200 (Biacore) at 25 °C with HBS-EP as running buffer (0.01 M HEPES pH 7.4, 0.15 M NaCl, 3 mM EDTA, 0.005% Surfactant P20 (Biacore).

Biotinylated human OX40 Fc (kih) was directly coupled to a flow cell of a streptavidin (SA) sensor chip. Immobilization levels up to 1000 resonance units (RU) were used.

The bispecific antibodies targeting OX40 and FAP were passed over the chip surface at a concentration of 250 nM with a flow rate of 30 µL/minute for 90 seconds and dissociation was set to zero sec. Human FAP was injected as second analyte with a flow rate of 30 µL/minute for 90 seconds at a concentration of 250 nM (see **Figure 21B**). The dissociation was monitored for 120 sec. Bulk refractive index differences were corrected for by subtracting the response obtained in a reference flow cell, where no protein was immobilized.

All bispecific constructs could bind simultaneously to human OX40 and human FAP apart from the DP47 control molecules **(****Figure 22****).**

### 4.7.5 Binding to OX40 and FAP negative tumor cells

The lack of binding to OX40 negative FAP negative tumor cells was tested using A549 NucLight^{™} Red Cells (Essenbioscience, Cat. No. 4491) expressing the NucLight Red fluorescent protein restricted to the nucleus to allow separation from unlabeled human FAP positive WM266-4 cells. Parental A549 (ATCC CCL-185) were transduced with the Essen CellPlayer NucLight Red Lentivirus (Essenbioscience, Cat. No. 4476; EF1α, puromycin) at an MOI of 3 (TU/cell) in the presence of 8 µg/ml polybrene following the standard Essen protocol. This resulted in ≥70% transduction efficiency.

A mixture of 5 × 104 unlabeled WM266-4 cells and unlabeled A549 NucLight^{™} Red Cells in FACS buffer were added to each well of a round-bottom suspension cell 96-well plates and the binding assay was performed as described in section 4.7.1.

As shown in **Figure 23****,** no 49B4 antibody construct bound to Ox40 negative FAP negative human tumor cells.

### Example 5

### Functional properties of bispecific anti-human OX40 binding molecules

### 5.1 HeLa cells expressing human OX40 and reporter gene NFκB-luciferase

Agonstic binding of Ox40 to its ligand induces downstream signaling via activation of nuclear factor kappa B (NFκB) (A. D. Weinberg et al., J. Leukoc. Biol. 2004, 75(6), 962-972). The recombinant reporter cell line HeLa_hOx40_NFkB_Luc1 was generated to express human OX40 on its surface. Additionally, it harbors a reporter plasmid containing the luciferase gene under the control of an NFκB-sensitive enhancer segment. OX40 triggering induces dosedependent activation of NFκB, which translocates in the nucleus, where it binds on the NFκB sensitive enhancer of the reporter plasmid to increase expression of the luciferase protein. Luciferase catalyzes luciferin-oxidation resulting in oxyluciferin which emits light. This can be quantified by a luminometer. Thus, the capacity of the various anti-OX40 antigen binding molecules to induce NFκB activation in HeLa_hOx40_NFkB_Luc1 reporter cells was analyzed as a measure for bioactivity.

Adherent HeLa_hOx40_NFkB_Luc1 cells were harvested using cell dissociation buffer (Invitrogen, Cat.-No. 13151-014) for 10 minutes at 37 °C. Cells were washed once with DPBS and were adjusted to a cell density of 0.64^{∗}10⁵ in assay media comprising of MEM (Invitrogen, Cat.-No. 22561-021), 10 % (v/v) heat-inactivated FBS, 1 mM Sodium-Pyruvat and 1% (v/v) non-essential amino acids. Cells were seeded in a density of 0.1^{∗}10⁵ cells per well in a sterile white 96-well flat bottom tissue culture plate with lid (greiner bio-one, Cat. No. 655083) and kept over night at 37 °C and 5% CO₂ in an incubator (Hera Cell 150).

The next day, HeLa_hOx40_NFkB_Luc1 were stimulated for 6 hours by adding assay medium containing various titrated bispecific anti-OX40 (clone 49B4) antibody constructs in a P329GLALA huIgG1 format. For testing the effect of hyper-crosslinking on anti-Ox4O antibodies, 25 µL/well of medium containing secondary antibody anti-human IgG Fcγ-fragment-specific goat IgG F(ab')₂ fragment (Jackson ImmunoResearch, 109-006-098) were added in a 1:2 ratio (2 times more secondary antibody than the primary anti-Ox40 P329GLALA huIgG1). After incubation, supernatant was aspirated and plates washed two times with DPBS. Quantification of light emission was done using the luciferase 100 assay system and the reporter lysis buffer (both Promega, Cat.-No. E4550 and Cat-No: E3971) according to manufacturer instructions. Briefly, cells were lysed for 10 minutes at -20 °C by addition of 30 uL per well 1x lysis buffer. Cells were thawed for 20 minutes at 37 °C before 90 uL per well provided luciferase assay reagent was added. Light emission was quantified immediately with a SpectraMax M5/M5e microplate reader (Molecular Devices, USA) using 500ms integration time, without any filter to collect all wavelengths. Emitted relative light units (URL) were corrected by basal luminescence of HeLa_hOx40_NFkB_Luc1 cells and were blotted against the logarithmic primary antibody concentration using Prism4 (GraphPad Software, USA). Curves were fitted using the inbuilt sigmoidal dose response.

As shown in **Figure 24****,** a limited, dose dependent NFκB activation was induced already by addition of anti-OX40 P329GLALA huIgG1 antibodies (left side) to the reporter cell line. Constructs with a tetravalent OX40 binding moiety induced a stronger NFkB activation than constructs with a bivalent OX40 binding moiety. This is in line with the finding that the minimal signaling unit of the OX40 receptor is a trimer (M. Croft, Nat rev Immunol. 2009, 9, 271- 285). Hyper-crosslinking of anti-OX40 antibodies by anti-human IgG specific secondary antibodies strongly increased the induction of NFκB-mediated luciferase-activation in a concentrationdependent manner (right side). The gain was within the tetravalent and bivalent group of constructs.

Consequently, the NFkB activating capacity of aOx40 binder 49B9 in different bispecific human IgG1 P329GLALA formats with hyper-crosslinking of the constructs by FAP+ tumor cell lines was tested.

Tested tumor cell lines were WM266-4 cells (ATCC CRL-1676), NIH/3T3-moFAP clone 26 and NIH/3T3-huFAP clone 39. NIH/3T3-huFAP clone 39 and NIH/3T3-moFAP clone 26 was generated by the transfection of the mouse embryonic fibroblast NIH/3T3 cell line (ATCC CRL-1658) with the expression vector pETR4921 to express huFAP and expression vector pETR4906 to express moFAP, all under 1.5 µg/mL Puromycin selection. The surface expression of FAP was quantified using the Quifikit (Dako Cat. No. K0078) according to manufacturer's instructions. The primary antibody used to detect cell surface FAP expression was the human/ mouse crossreactive clone F11-24 (mouse IgG1, Calbiochem, Ca. No. OP188). The surface expression on WM266-4 cells was in average app 40000 huFAP per cell (low expression, FAP+/-), for NIH/3T3-huFAP clone 39 app. 90000 huFAP per cell (intermediate expression, FAP+) and for NIH/3T3-moFAP clone 26 app. 160000 moFAP per cell (high expression, FAP++) .

As described herein before, adherent HeLa_hOx40_NFkB_Luc1 cells were cultured over night at a cell density of 0.1^{∗}105 cells per well and were stimulated for 5 to 6 hours with assay medium containing titrated anti-OX40 constructs. To test the effect of hyper-crosslinking by cell surface FAP binding 25 µL/well of medium containing FAP+ tumor cells (WM266-4, NIH/3T3-huFAP clone 39, NIH/3T3-moFAP cl 26) were co-cultured in a 4 to 1 ratio (four times as much FAP+ tumor cells than reporter cells per well). Activated NFκB was quantified by measuring light emission using luciferase 100 assay system and the reporter lysis buffer (both Promega, Cat. No. E4550 and Cat-No: E3971) as described herein before.

As shown in **Figure 25** and **Figure 26****,** the presence of all anti-OX40 constructs induced a limited NFκB activation. FAP-expressing tumor cells strongly increased induction of NFκB-mediated luciferase-activation when FAP targeted molecules (filled circle, square and triangle) were used but not when non-targeted bispecific constructs (compare respective open circle, square and triangle) were added.

For a better comparison of all formats the area under the curve of the respective blotted dose-response curves was quantified as a marker for the agonistic capacity of each construct. As shown in **Figure 26****,** the tetravalent, FAP-targeted constructs were superior to the bivalent FAP-targeted molecules. A high expression of FAP ensured higher cross-linking and thus a better agonistic effect of the FAP-targeted constructs (compare low FAP expressing WM266-4 cells, with intermediate FAP expressing NIH-3T3 human FAP cells with high FAP expressing NIH-3T3 mouse FAP cells).

### 5.2 OX40 mediated costimulation of suboptimally TCR triggered pre-activated human CD4 T cells

Ligation of OX40 provides a synergistic co-stimulatory signal promoting division and survival of T-cells following suboptimal T-cell receptor (TCR) stimulation (M. Croft et al., Immunol. Rev. 2009, 229(1), 173-191). Additionally, production of several cytokines and surface expression of T-cell activation markers is increased (I. Gramaglia et al., J. Immunol. 1998, 161(12), 6510-6517; S. M. Jensen et al., Seminars in Oncology 2010, 37(5), 524-532).

To test agonistic properties of clone 49B9 in different bispecific human IgG1 P329GLALA formats, pre-activated OX40 positive CD4 T-cells were stimulated for 72 hours with a suboptimal concentration of plate-immobilized anti-CD3 antibodies in the presence of anti-Ox40 antibodies, either in solution or immobilized on the plate surface. Effects on T-cell survival and proliferation were analyzed through monitoring of total cell counts and CFSE dilution in living cells by flow cytometry. Additionally, cells were co-stained with fluorescently-labeled antibodies against T-cell activation and differentiation markers, e.g. CD127, CD45RA, Tim-3, CD62L and OX40 itself.

Human PBMCs were isolated via ficoll density centrifugation and were simulated for three days with PHA-L [2 µg/mL] and Proleukin [200 U/mL] as described under Example 4.7.3.1. Cells were then labeled with CFSE at a cell density of 1×10⁶ cells/ mL with CFDA-SE (Sigma-Aldrich, Cat.-No. 2188) at a final concentration of [50 nM] for 10 minutes at 37 °C. Thereafter, cells were washed twice with excess DPBS containing FBS (10% v/v). Labeled cells were rested in T-cell media at 37 °C for 30 minutes. Thereafter, non-converted CFDA-SE was removed by two additional washing steps with DPBS. CD4 T-cell isolation from pre-activated CFSE-labeled human PBMC was performed using the MACS negative CD4 T-cell isolation kit (Miltenyi Biotec, Cat. No. 130-096.533) according to manufacturer's instructions.

Morris et al. showed that agonistic co-stimulation with conventional anti-Ox40 antibodies relied on surface immobilization (N. P. Morris et al., Mol. Immunol. 2007, 44(12), 3112-3121). Thus, goat anti-mouse Fcy-specific antibodies (Jackson ImmunoResearch, Ca.No. 111-500-5008) were coated to the surface of a 96 well U-bottom cell culture plate (Greiner Bio One) at a concentration of [2 µg/mL] in PBS over night at 4 °C in the presence (surface immobilized anti-OX40) or absence (anti-OX40 in solution) of goat anti-human Fcy-specific antibody (Jackson ImmunoResearch, Ca.No. 109-006-098). Thereafter, the plate surface was blocked with DPBS containing BSA (1 % v/w). All following incubation steps were done at 37 °C for 90 minutes in PBS containing BSA (1 % v/w). Between the incubation steps, plates were washed with DPBS.

Mouse anti-human CD3 antibody (clone OKT3, eBioscience, Ca.No. 16-0037-85, fixed concentration [3 ng/mL]) was captured in a subsequent incubation step via the surface coated anti-mouse Fcγ- specific antibodies. In one experiment titrated human anti-OX40 antigen binding molecules were then immobilized on plate by an additional incubation step in DPBS. In a second experiment anti-OX40 antigen binding molecules were added during the activation assay directly to the media to plates not pre-coated with anti-human IgG Fc spec. antibodies.

CFSE-labeled preactivated CD4⁺ T cells were added to the pre-coated plates at a cell density of 0.5^{∗}10⁵ cells per well in 200 µL T-cell media and cultured for 96 hours. Cells were stained with a combination of fluorochrome-labeled mouse anti-human Ox40 (clone BerACT35, Bioledgend, Ca.No. 35008), TIM-3 (clone F38-2E2, Bioledgend, Ca.No.345008), CD127 (clone A019D5, Bioledgend, Ca.No.351234), CD25 (clone M-AQ251, Bioledgend, Cat. No. **356112)** and CD45RA (clone HI100, BD Biosciences, Ca.No.555489) for 20 minutes at 4 °C in the dark. Plates where washed twice with 200 µL/well 4 °C FACS buffer, were finally resuspended in 85 µL/well FACS-buffer containing 0.2 µg/mL DAPI (Santa Cruz Biotec, Cat. No. Sc-3598) and acquired the same day using 5-laser LSR-Fortessa (BD Bioscience with DIVA software).

DAPI-negative living cells were analyzed for decrease in median CFSE fluorescence as a marker for proliferation. The percentage of OX40 positive, CD25 high and TIM-3 positive T cells was monitored as a marker for T-cell activation. The expression of CD45RA and CD127 was analyzed to determine changes in maturation status of T cell, whereby CD45RA low CD127 low cells were categorized as effector T cells.

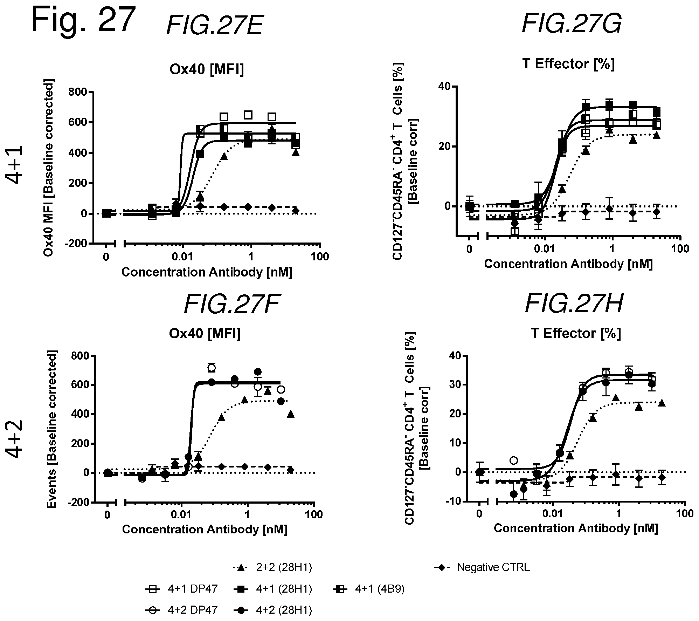

Co-stimulation with plate-immobilized antibodies strongly enhanced suboptimal stimulation of pre-activated human CD4 T cells with plate-immobilized anti-human CD3 in a dose dependent manner **(****Figure 27****).** T-cells proliferated stronger, showed a more mature phenotype with a higher percentage of effector T cells and had higher percentages of Ox40 positive activated cells. Molecules with tetravalent OX40 binding moieties were stronger agonistic than molecules with only bivalent binding to OX40. All five tetravalent molecules and the two bivalent molecules were able to enhance TCR stimulation to a similar extent. Thus, differences in the activating potential in the presence of FAP+ tumor cells within these two groups were mediated by different FAP binding capacity and crosslinking and not because of different ability to address OX40. No enhancement in suboptimal TCR stimulation was seen when anti-OX40 antigen binding molecules were added in solution in the absence of surface immobilization **(****Figure 28****).** This demonstrated again the strong dependency of OX40 axis activation on hypercrosslinking of the OX40 receptor.

### 5.3 Ox40 mediated costimulation of suboptimally TCR triggered resting human PBMC and hypercrosslinking by cell surface FAP

It was shown in Example 5.1 that addition of FAP+ tumor cells can strongly increase the NFkB activity induced by FAP targeted tetravalent anti-OX40 construct in human OX40 positive reporter cell lines by providing strong oligomerization of OX40 receptors. Likewise, we tested FAP targeted tetravalent anti-OX40 constructs in the presence of NIH/3T3-huFAP clone 39 cells for their ability to rescue suboptimal TCR stimulation of resting human PBMC cells.

Human PBMC preparations contain (1) resting OX40 negative CD4+ and CD8+ T cells and (2) antigen presenting cells with various Fc-y receptor molecules on their cell surface e.g. B cells and monocytes. Anti-human CD3 antibody of human IgG1 isotype can bind with its Fc part to the present Fc-y receptor molecules and mediate a prolonged TCR activation on resting Ox40 negative CD4+ and CD8+ T cells. These cells then start to express OX40 within several hours. Functional agonistic compounds against OX40 can signal via the OX40 receptor present on activated CD8+ and CD4+ T cells and support TCR-mediated stimulation.

Resting CFSE-labeled human PBMC were stimulated for five days with a suboptimal concentration of anti-CD3 antibody in the presence of irradiated FAP+ NIH/3T3-huFAP clone 39 cells and titrated anti-OX40 constructs. Effects on T-cell survival and proliferation were analyzed through monitoring of total cell counts and CFSE dilution in living cells by flow cytometry. Additionally, cells were co-stained with fluorescently-labeled antibodies against T-cell activation marker CD25.

Mouse embryonic fibroblast NIH/3T3-huFAP clone 39 cells (see Example 4.3.2.2) were harvested using cell dissociation buffer (Invitrogen, Cat.-No. 13151-014) for 10 minutes at 37 °C. Cells were washed once with DPBS. NIH/3T3-huFAP clone 39 cells were cultured at a density of 0.2^{∗}10⁵ cells per well in T cell media in a sterile 96-well round bottom adhesion tissue culture plate (TPP, Cat. No. 92097) over night at 37 °C and 5% CO₂ in an incubator (Hera Cell 150). The next day they were irradiated in an xRay irradiator using a dose of 4500 RAD to prevent later overgrowth of human PBMC by the tumor cell line.

Human PBMCs were isolated by ficoll density centrifugation and were labeled with CFSE as described in Example 4.3.2.1. Cells were added to each well at a density of 0.5^{∗}10⁵ cells per well. Anti-human CD3 antibody (clone V9, human IgG1, described in Rodrigues et al., Int J Cancer Suppl 7, 45-50 (1992) and US patent no. 6,054,297) at a final concentration of [10 nM] and anti-Ox40 constructs were added at the indicated concentrations. Cells were activated for five days at 37 °C and 5% CO₂ in an incubator (Hera Cell 150). Then, Cells were surface-stained with fluorescent dye-conjugated antibodies anti-human CD4 (clone RPA-T4, BioLegend, Cat.-No. 300532), CD8 (clone RPa-T8, BioLegend, Cat.-No. 3010441) and CD25 (clone M-A251, BioLegend, Cat.-No. 356112) for 20 min at 4°C. For permeabilizing the cell membrane, cell pellets were washed twice with FACS buffer, then resuspended in 50 µL/well freshly prepared FoxP3 Fix/Perm buffer (eBioscience Cat.-No. 00-5123 and 00-5223) for 45 min at room temperature in the dark. After three times washing with Perm-Wash buffer (eBioscience, Cat.-No. 00-8333-56), cells were stained intracellular with 25 µL/well Perm-Wash Buffer containing anti-human GranzymeB antibody (clone GB-11, BD Bioscience, Cat. No. 561142) for 1 h at room temperature in the dark. Cells were resuspended in 85 µL/well FACS buffer and acquired using a 5-laser Fortessa flow cytometer (BD Bioscience with DIVA software).

As shown in **Figures 29****,** **30** **and** **31****,** costimulation with non-targeted tetravalent anti-OX40 (49B4) 4+1 construct (open square) and OX40 (49B4) 4+2 (open circle) only slightly rescued suboptimally TCR stimulated CD4 and CD8 T cells. Hyper-crosslinking of the FAP targeted 4+1 (filled and semi-filled square) and 4+2 (filled circle) anti-OX40 constructs by the present NIH/3T3-huFAP clone 39 cells strongly promoted proliferation (Figure 29), survival (Figure 30) and induced an enhanced activated phenotype (Figure 31) in human CD4 and CD8 T cells. The FAP-targeted tetravalent OX40 binders were clearly superior to the FAP-targeted bivalent OX40 binder (Figures 29 to 30: filled triangle, for comparison see **Figures 32** **and** **33****).** Cell surface immobilization of a bivalent OX40 agonist however, was more effective in supporting activation than addition of non-targeted tetravalent OX40 agonist (compare 2+2 (28H1) vs 4+1 (DP47)/ 4+2 (DP47) in Figures 32 and 33). This finding in a primary cell setting was slightly different to the observations made in the NFκB reporter assay (Figure 26). Here, tetravalency for OX40 resulted in stronger NFκB activation compared to bivalency, even when not surfaceimmobilized. So, for obtaining optimal OX40 agonism for T cells, not only sufficient oligomerization of the OX40 receptor needs to be obtained, but additionally, cell surface immobilization of OX40 receptor oligomers. Bivalent binding to FAP reduced the the agonistic capacity of tetravalent OX40 molecules compared to monovalent binding to FAP (compare 4+1 (28H1) vs 4+2 (28H1) in Figure 31). This might be due to a reduced number of FAP targeted OX40 molecules, which can be bound per FAP positive cells. Higher affinity to FAP for a monovalent FAP binder additionally increased the agonistic capacity of the tetravalent OX40 agonist (compare 4+1 (28H1) vs 4+1 (4B9) in Figure 31), most likely by optimized hypercrosslinking of OX40 receptor oligomers.

### Example 6

### Generation of 4-1BB antibodies and tool binders

### 6.1 Preparation, purification and characterization of antigens and screening tools for the generation of novel 4-1BB binders by Phage Display

DNA sequences encoding the ectodomains of human, mouse or cynomolgus 4-1BB **(Table 39)** were subcloned in frame with the human IgG1 heavy chain CH2 and CH3 domains on the knob (Merchant et al., 1998). An AcTEV protease cleavage site was introduced between an antigen ectodomain and the Fc of human IgG1. An Avi tag for directed biotinylation was introduced at the C-terminus of the antigen-Fc knob. Combination of the antigen-Fc knob chain containing the S354C/T366W mutations, with a Fc hole chain containing the Y349C/T366S/L368A/Y407V mutations allows generation of a heterodimer which includes a single copy of 4-1BB ectodomain containing chain, thus creating a monomeric form of Fc-linked antigen **(****Figure 1A****). Table 40** shows the cDNA and amino acid sequences of the antigen Fc-fusion constructs.

**Table 39: Amino acid numbering of antigen ectodomains (ECD) and their origin**

| SEQ ID NO: | Construct | Origin | ECD |
|---|---|---|---|
| 239 | human 4-1BB ECD | Synthetized according to Q07011 | aa 24-186 |
| 240 | cynomolgus 4-1BB ECD | isolated from cynomolgus blood | aa 24-186 |
| 241 | murine 4-1BB ECD | Synthetized according to P20334 | aa 24-187 |

**Table 40: cDNA and amino acid sequences of monomeric antigen Fc(kih) fusion molecules (produced by combination of one Fc hole chain with one antigen Fc knob chain)**

| SEQ ID NO: | Antigen | Sequence |
|---|---|---|
| 95 | Nucleotide sequence Fc hole chain | see Table 2 |
| 242 | Nucleotide sequence of human 4-1BB antigen Fc knob chain | CTGCAGGACCCCTGCAGCAACTGCCCTGCCGGCACCTTCT GCGACAACAACCGGAACCAGATCTGCAGCCCCTGCCCCC CCAACAGCTTCAGCTCTGCCGGCGGACAGCGGACCTGCG ACATCTGCAGACAGTGCAAGGGCGTGTTCAGAACCCGGA AAGAGTGCAGCAGCACCAGCAACGCCGAGTGCGACTGCA CCCCCGGCTTCCATTGTCTGGGAGCCGGCTGCAGCATGTG CGAGCAGGACTGCAAGCAGGGCCAGGAACTGACCAAGA AGGGCTGCAAGGACTGCTGCTTCGGCACCTTCAACGACC AGAAGCGGGGCATCTGCCGGCCCTGGACCAACTGTAGCC TGGACGGCAAGAGCGTGCTGGTCAACGGCACCAAAGAAC |
| | | GGGACGTCGTGTGCGGCCCCAGCCCTGCTGATCTGTCTCC TGGGGCCAGCAGCGTGACCCCTCCTGCCCCTGCCAGAGA GCCTGGCCACTCTCCTCAGGTCGACGAACAGTTATATTTT CAGGGCGGCTCACCCAAATCTGCAGACAAAACTCACACA TGCCCACCGTGCCCAGCACCTGAACTCCTGGGGGGACCGT CAGTCTTCCTCTTCCCCCCAAAACCCAAGGACACCCTCAT GATCTCCCGGACCCCTGAGGTCACATGCGTGGTGGTGGAC GTGAGCCACGAAGACCCTGAGGTCAAGTTCAACTGGTAC GTGGACGGCGTGGAGGTGCATAATGCCAAGACAAAGCCG CGGGAGGAGCAGTACAACAGCACGTACCGTGTGGTCAGC GTCCTCACCGTCCTGCACCAGGACTGGCTGAATGGCAAG GAGTACAAGTGCAAGGTCTCCAACAAAGCCCTCCCAGCC CCCATCGAGAAAACCATCTCCAAAGCCAAAGGGCAGCCC CGAGAACCACAGGTGTACACCCTGCCCCCATGCCGGGAT GAGCTGACCAAGAACCAGGTCAGCCTGTGGTGCCTGGTC AAAGGCTTCTATCCCAGCGACATCGCCGTGGAGTGGGAG AGCAATGGGCAGCCGGAGAACAACTACAAGACCACGCCT CCCGTGCTGGACTCCGACGGCTCCTTCTTCCTCTACAGCA AGCTCACCGTGGACAAGAGCAGGTGGCAGCAGGGGAACG TCTTCTCATGCTCCGTGATGCATGAGGCTCTGCACAACCA CTACACGCAGAAGAGCCTCTCCCTGTCTCCGGGTAAATCC GGAGGCCTGAACGACATCTTCGAGGCCCAGAAGATTGAA TGGCACGAG |
| 243 | Nucleotide sequence of cynomolgus 4-1BB antigen Fc knob chain | TTGCAGGATCTGTGTAGTAACTGCCCAGCTGGTACATTCT GTGATAATAACAGGAGTCAGATTTGCAGTCCCTGTCCTCC AAATAGTTTCTCCAGCGCAGGTGGACAAAGGACCTGTGA CATATGCAGGCAGTGTAAAGGTGTTTCAAGACCAGGAA GGAGTGTTCCTCCACCAGCAATGCAGAGTGTGACTGCATT TCAGGGTATCACTGCCTGGGGGCAGAGTGCAGCATGTGT GAACAGGATTGTAAACAAGGTCAAGAATTGACAAAAAAA GGTTGTAAAGACTGTTGCTTTGGGACATTTAATGACCAGA AACGTGGCATCTGTCGCCCCTGGACAAACTGTTCTTTGGA TGGAAAGTCTGTGCTTGTGAATGGGACGAAGGAGAGGGA CGTGGTCTGCGGACCATCTCCAGCCGACCTCTCTCCAGGA GCATCCTCTGCGACCCCGCCTGCCCCTGCGAGAGAGCCAG GACACTCTCCGCAGGTCGACGAACAGTTATATTTCAGGG CGGCTCACCCAAATCTGCAGACAAAACTCACACATGCCC ACCGTGCCCAGCACCTGAACTCCTGGGGGGACCGTCAGT CTTCCTCTTCCCCCCAAAACCCAAGGACACCCTCATGATC TCCCGGACCCCTGAGGTCACATGCGTGGTGGTGGACGTG AGCCACGAAGACCCTGAGGTCAAGTTCAACTGGTACGTG GACGGCGTGGAGGTGCATAATGCCAAGACAAAGCCGCGG GAGGAGCAGTACAACAGCACGTACCGTGTGGTCAGCGTC CTCACCGTCCTGCACCAGGACTGGCTGAATGGCAAGGAG TACAAGTGCAAGGTCTCCAACAAAGCCCTCCCAGCCCCC ATCGAGAAAACCATCTCCAAAGCCAAAGGGCAGCCCCGA GAACCACAGGTGTACACCCTGCCCCCATGCCGGGATGAG CTGACCAAGAACCAGGTCAGCCTGTGGTGCCTGGTCAAA GGCTTCTATCCCAGCGACATCGCCGTGGAGTGGGAGAGC AATGGGCAGCCGGAGAACAACTACAAGACCACGCCTCCC GTGCTGGACTCCGACGGCTCCTTCTTCCTCTACAGCAAGC TCACCGTGGACAAGAGCAGGTGGCAGCAGGGGAACGTCT TCTCATGCTCCGTGATGCATGAGGCTCTGCACAACCACTA |
| | | CACGCAGAAGAGCCTCTCCCTGTCTCCGGGTAAATCCGGA GGCCTGAACGACATCTTCGAGGCCCAGAAGATTGAATGG CACGAG |
| 244 | Nucleotide sequence of murine 4-1BB antigen Fc knob chain | GTGCAGAACAGCTGCGACAACTGCCAGCCCGGCACCTTC TGCCGGAAGTACAACCCCGTGTGCAAGAGCTGCCCCCCC AGCACCTTCAGCAGCATCGGCGGCCAGCCCAACTGCAAC ATCTGCAGAGTGTGCGCCGGCTACTTCCGGTTCAAGAAGT TCTGCAGCAGCACCCACAACGCCGAGTGCGAGTGCATCG AGGGCTTCCACTGCCTGGGCCCCCAGTGCACCAGATGCG AGAAGGACTGCAGACCCGGCCAGGAACTGACCAAGCAGG GCTGTAAGACCTGCAGCCTGGGCACCTTCAACGACCAGA ACGGGACCGGCGTGTGCCGGCCTTGGACCAATTGCAGCC TGGACGGGAGAAGCGTGCTGAAAACCGGCACCACCGAGA AGGACGTCGTGTGCGGCCCTCCCGTGGTGTCCTTCAGCCC TAGCACCACCATCAGCGTGACCCCTGAAGGCGGCCCTGG CGGACACTCTCTGCAGGTCCTGGTCGACGAACAGTTATAT TTTCAGGGCGGCTCACCCAAATCTGCAGACAAAACTCAC ACATGCCCACCGTGCCCAGCACCTGAACTCCTGGGGGGA CCGTCAGTCTTCCTCTTCCCCCCAAAACCCAAGGACACCC TCATGATCTCCCGGACCCCTGAGGTCACATGCGTGGTGGT GGACGTGAGCCACGAAGACCCTGAGGTCAAGTTCAACTG GTACGTGGACGGCGTGGAGGTGCATAATGCCAAGACAAA GCCGCGGGAGGAGCAGTACAACAGCACGTACCGTGTGGT CAGCGTCCTCACCGTCCTGCACCAGGACTGGCTGAATGGC AAGGAGTACAAGTGCAAGGTCTCCAACAAAGCCCTCCCA GCCCCCATCGAGAAAACCATCTCCAAAGCCAAAGGGCAG CCCCGAGAACCACAGGTGTACACCCTGCCCCCATGCCGG GATGAGCTGACCAAGAACCAGGTCAGCCTGTGGTGCCTG GTCAAAGGCTTCTATCCCAGCGACATCGCCGTGGAGTGG GAGAGCAATGGGCAGCCGGAGAACAACTACAAGACCAC GCCTCCCGTGCTGGACTCCGACGGCTCCTTCTTCCTCTAC AGCAAGCTCACCGTGGACAAGAGCAGGTGGCAGCAGGGG AACGTCTTCTCATGCTCCGTGATGCATGAGGCTCTGCACA ACCACTACACGCAGAAGAGCCTCTCCCTGTCTCCGGGTAA ATCCGGAGGCCTGAACGACATCTTCGAGGCCCAGAAGAT TGAATGGCACGAG |
| 99 | Fc hole chain | see Table 2 |
| 245 | human 4-1BB antigen Fc knob chain | LQDPCSNCPAGTFCDNNRNQICSPCPPNSFSSAGGQRTCDIC RQCKGVFRTRKECSSTSNAECDCTPGFHCLGAGCSMCEQDC KQGQELTKKGCKDCCFGTFNDQKRGICRPWTNCSLDGKSV LVNGTKERDVVCGPSPADLSPGASSVTPPAPAREPGHSPQVD EQLYFQGGSPKSADKTHTCPPCPAPELLGGPSVFLFPPKPKD TLMISRTPEVTCVVVDVSHEDPEVKFNWYVDGVEVHNAKT KPREEQYNSTYRVVSVLTVLHQDWLNGKEYKCKVSNKALP APIEKTISKAKGQPREPQVYTLPPCRDELTKNQVSLWCLVKG FYPSDIAVEWESNGQPENNYKTTPPVLDSDGSFFLYSKLTVD KSRWQQGNVFSCSVMHEALHNHYTQKSLSLSPGKSGGLNDI FEAQKIEWHE |
| 246 | cynomolgus 4-1BB antigen Fc knob chain | LQDLCSNCPAGTFCDNNRSQICSPCPPNSFSSAGGQRTCDICR QCKGVFKTRKECSSTSNAECDCISGYHCLGAECSMCEQDCK QGQELTKKGCKDCCFGTFNDQKRGICRPWTNCSLDGKSVL VNGTKERDVVCGPSPADLSPGASSATPPAPAREPGHSPQVDE QLYFQGGSPKSADKTHTCPPCPAPELLGGPSVFLFPPKPKDT |
| | | LMISRTPEVTCVVVDVSHEDPEVKFNWYVDGVEVHNAKTK PREEQYNSTYRVVSVLTVLHQDWLNGKEYKCKVSNKALPA PIEKTISKAKGQPREPQVYTLPPCRDELTKNQVSLWCLVKGF YPSDIAVEWESNGQPENNYKTTPPVLDSDGSFFLYSKLTVDK SRWQQGNVFSCSVMHEALHNHYTQKSLSLSPGKSGGLNDIF EAQKIEWHE |
| 247 | murine 4-1BB antigen Fc knob chain | VQNSCDNCQPGTFCRKYNPVCKSCPPSTFSSIGGQPNCNICR VCAGYFRFKKFCSSTHNAECECIEGFHCLGPQCTRCEKDCRP GQELTKQGCKTCSLGTFNDQNGTGVCRPWTNCSLDGRSVL KTGTTEKDVVCGPPVVSFSPSTTISVTPEGGPGGHSLQVLVD EQLYFQGGSPKSADKTHTCPPCPAPELLGGPSVFLFPPKPKD TLMISRTPEVTCVVVDVSHEDPEVKFNWYVDGVEVHNAKT KPREEQYNSTYRVVSVLTVLHQDWLNGKEYKCKVSNKALP APIEKTISKAKGQPREPQVYTLPPCRDELTKNQVSLWCLVKG FYPSDIAVEWESNGQPENNYKTTPPVLDSDGSFFLYSKLTVD KSRWQQGNVFSCSVMHEALHNHYTQKSLSLSPGKSGGLNDI FEAQKIEWHE |

All 4-1BB-Fc-fusion molecule encoding sequences were cloned into a plasmid vector, which drives expression of the insert from an MPSV promoter and contains a synthetic polyA signal sequence located at the 3' end of the CDS. In addition, the vector contains an EBV OriP sequence for episomal maintenance of the plasmid.

For preparation of the biotinylated monomeric antigen/Fc fusion molecules, exponentially growing suspension HEK293 EBNA cells were co-transfected with three vectors encoding the two components of fusion protein (knob and hole chains) as well as BirA, an enzyme necessary for the biotinylation reaction. The corresponding vectors were used at a 2 : 1 : 0.05 ratio ("antigen ECD-AcTEV- Fc knob" : "Fc hole" : "BirA").

For protein production in 500 ml shake flasks, 400 million HEK293 EBNA cells were seeded 24 hours before transfection. For transfection cells were centrifuged for 5 minutes at 210 g, and the supernatant was replaced by pre-warmed CD CHO medium. Expression vectors were resuspended in 20 mL of CD CHO medium containing 200 µg of vector DNA. After addition of 540 µL of polyethylenimine (PEI), the solution was vortexed for 15 seconds and incubated for 10 minutes at room temperature. Afterwards, cells were mixed with the DNA/PEI solution, transferred to a 500 mL shake flask and incubated for 3 hours at 37°C in an incubator with a 5 % CO₂ atmosphere. After the incubation, 160 mL of F17 medium was added and cells were cultured for 24 hours. The production medium was supplemented with 5 µM kifunensine. One day after transfection, 1 mM valproic acid and 7 % Feed were added to the culture. After 7 days of culturing, the cell supernatant was collected by spinning down cells for 15 min at 210 g. The solution was sterile filtered (0.22 µm filter), supplemented with sodium azide to a final concentration of 0.01 % (w/v), and kept at 4°C.

Secreted proteins were purified from cell culture supernatants by affinity chromatography using Protein A, followed by size exclusion chromatography. For affinity chromatography, the supernatant was loaded on a HiTrap ProteinA HP column (CV = 5 mL, GE Healthcare) equilibrated with 40 mL 20 mM sodium phosphate, 20 mM sodium citrate pH 7.5. Unbound protein was removed by washing with at least 10 column volumes of 20 mM sodium phosphate, 20 mM sodium citrate, 0.5 M sodium chloride containing buffer (pH 7.5). The bound protein was eluted using a linear pH-gradient of sodium chloride (from 0 to 500 mM) created over 20 column volumes of20 mM sodium citrate, 0.01 % (v/v) Tween-20, pH 3.0 . The column was then washed with 10 column volumes of 20 mM sodium citrate, 500 mM sodium chloride, 0.01 % (v/v) Tween-20, pH 3.0. The pH of collected fractions was adjusted by adding 1/40 (v/v) of 2M Tris, pH8.0. The protein was concentrated and filtered prior to loading on a HiLoad Superdex 200 column (GE Healthcare) equilibrated with 2mM MOPS, 150 mM sodium chloride, 0.02 % (w/v) sodium azide solution of pH 7.4.

### 6.2 Selection of 4-1BB-specific 12B3, 25G7, 11D5, 9B11 and 20G2 antibodies from generic F(ab) libraries

The antibodies **11D5, 9B11,** and **12B3** with specificity for human and cynomolgus 4-1BB were selected from a generic phage-displayed antibody library (DP88-4) in the Fab format. From the same library, an additional antibody, clone **20G2,** with reactivity to murine 4-1BB was selected as well. This library was constructed on the basis of human germline genes using the V-domain pairing Vkl_5 (kappa light chain) and VH1_69 (heavy chain) comprising randomized sequence space in CDR3 of the light chain (L3, 3 different lengths) and CDR3 of the heavy chain (H3, 3 different lengths). Library generation was performed by assembly of 3 PCR-amplified fragments applying splicing by overlapping extension (SOE) PCR. Fragment 1 comprises the 5' end of the antibody gene including randomized L3, fragment 2 is a central constant fragment spanning from L3 to H3 whereas fragment 3 comprises randomized H3 and the 3' portion of the antibody gene. The following primer combinations were used to generate these library fragments for DP88-4 library: fragment 1 (forward primer LMB3 combined with reverse primers Vk1_5_L3r_S or Vk1_5_L3r_SY or Vk1_5_L3r_SPY), fragment 2 (forward primer RJH31 combined with reverse primer RJH32) and fragment 3 (forward primers DP88-v4-4 or DP88-v4-6 or DP88-v4-8 combined with reverse primer fdseqlong), respectively. PCR parameters for production of library fragments were 5 min initial denaturation at 94 °C, 25 cycles of 1 min 94 °C, 1 min 58 °C, 1 min 72 °C and terminal elongation for 10 min at 72 °C. For assembly PCR, using equimolar ratios of the gel-purified single fragments as template, parameters were 3 min initial denaturation at 94 °C and 5 cycles of 30 s 94 °C, 1 min 58 °C, 2 min 72 °C. At this stage, outer primers (LMB3 and fdseqlong) were added and additional 20 cycles were performed prior to a terminal elongation for 10 min at 72 °C. After assembly of sufficient amounts of full length randomized Fab constructs, they were digested *Nco*I / *NheI* and ligated into similarly treated acceptor phagemid vector. Purified ligations were used for ~60 transformations into electrocompetent *E. coli* TG1. Phagemid particles displaying the Fab library were rescued and purified by PEG/NaCl purification to be used for selections. These library construction steps were repeated three times to obtain a final library size of 4.4 × 10⁹. Percentages of functional clones, as determined by C-terminal tag detection in dot blot, were 92.6% for the light chain and 93.7% for the heavy chain, respectively.

The antibody **25G7** with specificity for human and cynomolgus 4-1BB was selected from a generic phage-displayed antibody library (λ-DP47 in the Fab format. This library was constructed on the basis of human germline genes using the V-domain pairing Vl3_19 (lambda light chain) and VH3 23 (heavy chain) comprising randomized sequence space in CDR3 of the light chain (L3, 3 different lengths) and CDR3 of the heavy chain (H3, 3 different lengths). Library generation was performed by assembly of 3 PCR-amplified fragments applying splicing by overlapping extension (SOE) PCR. Fragment 1 comprises the 5' end of the antibody gene including randomized L3, fragment 2 is a central constant fragment spanning from L3 to H3 whereas fragment 3 comprises randomized H3 and the 3' portion of the antibody gene. The following primer combinations were used to generate these library fragments for λ-DP47 library: fragment 1 (forward primer LMB3 combined with reverse primers Vl_3_19_L3r_V or Vl_3_19_L3r_HV or Vl_3_19_L3r_HLV), fragment 2 (forward primer RJH80 combined with reverse primer MS63) and fragment 3 (forward primers DP47-v4-4 or DP47-v4-6 or DP47-v4-8 combined with reverse primer fdseqlong), respectively. PCR parameters for production of library fragments were 5 min initial denaturation at 94 °C, 25 cycles of 1 min 94 °C, 1 min 58 °C, 1 min 72 °C and terminal elongation for 10 min at 72 °C. For assembly PCR, using equimolar ratios of the gel-purified single fragments as template, parameters were 3 min initial denaturation at 94 °C and 5 cycles of 30 s 94 °C, 1 min 58 °C, 2 min 72 °C. At this stage, outer primers (LMB3 and fdseqlong) were added and additional 20 cycles were performed prior to a terminal elongation for 10 min at 72 °C. After assembly of sufficient amounts of full length randomized Fab constructs, they were digested *Ncol* / *NheI* and ligated into similarly treated acceptor phagemid vector. Purified ligations were used for ~60 transformations into electrocompetent *E. coli* TG1. Phagemid particles displaying the Fab library were rescued and purified by PEG/NaCl purification to be used for selections. A final library size of 9.5 × 10⁹ was obtained. Percentages of functional clones, as determined by C-terminal tag detection in dot blot, were 81.1% for the light chain and 83.2% for the heavy chain, respectively.

**Table 41** shows the sequence of generic phage-displayed antibody library (DP88-4), **Table 42** provides cDNA and amino acid sequences of library DP88-4 germline template and **Table 43** shows the Primer sequences used for generation of DP88-4 germline template.

**Table 41: Sequence of generic phage-displayed antibody library (DP88-4)**

| SEQ ID NO: | Description | Sequence |
|---|---|---|
| 103 | nucleotide sequence of pRJH33 library template DP88-4 library; complete Fab coding region comprising PelB leader sequence + Vkl_5 kappa V-domain + CL constant domain for light chain and PelB + VH1_69 V-domain + CH1 constant domain for heavy chain including tags | TGAAATACCTATTGCCTACGGCAGCCGCTGGATTGTTATTACTCG CGGCCCAGCCGGCCATGGCCGACATCCAGATGACCCAGTCTCCT TCCACCCTGTCTGCATCTGTAGGAGACCGTGTCACCATCACTTGC CGTGCCAGTCAGAGTATTAGTAGCTGGTTGGCCTGGTATCAGCA GAAACCAGGGAAAGCCCCTAAGCTCCTGATCTATGATGCCTCCA GTTTGGAAAGTGGGGTCCCATCACGTTTCAGCGGCAGTGGATCC GGGACAGAATTCACTCTCACCATCAGCAGCTTGCAGCCTGATGA TTTTGCAACTTATTACTGCCAACAGTATAATAGTTATTCTACGTT TGGCCAGGGCACCAAAGTCGAGATCAAGCGTACGGTGGCTGCA CCATCTGTCTTCATCTTCCCGCCATCTGATGAGCAGTTGAAATCT GGAACTGCCTCTGTTGTGTGCCTGCTGAATAACTTCTATCCCAGA GAGGCCAAAGTACAGTGGAAGGTGGATAACGCCCTCCAATCGG GTAACTCCCAGGAGAGTGTCACAGAGCAGGACAGCAAGGACAG CACCTACAGCCTCAGCAGCACCCTGACGCTGAGCAAAGCAGACT ACGAGAAACACAAAGTCTACGCCTGCGAAGTCACCCATCAGGG CCTGAGCTCGCCCGTCACAAAGAGCTTCAACAGGGGAGAGTGTG GAGCCGCAGAACAAAAACTCATCTCAGAAGAGGATCTGAATGG AGCCGCAGACTACAAGGACGACGACGACAAGGGTGCCGCATAA TAAGGCGCGCCAATTCTATTTCAAGGAGACAGTCATATGAAATA CCTGCTGCCGACCGCTGCTGCTGGTCTGCTGCTCCTCGCTGCCCA GCCGGCGATGGCCCAGGTGCAATTGGTGCAGTCTGGGGCTGAGG TGAAGAAGCCTGGGTCCTCGGTGAAGGTCTCCTGCAAGGCCTCC GGAGGCACATTCAGCAGCTACGCTATAAGCTGGGTGCGACAGGC CCCTGGACAAGGGCTCGAGTGGATGGGAGGGATCATCCCTATCT TTGGTACAGCAAACTACGCACAGAAGTTCCAGGGCAGGGTCACC ATTACTGCAGACAAATCCACGAGCACAGCCTACATGGAGCTGAG CAGCCTGAGATCTGAGGACACCGCCGTGTATTACTGTGCGAGAC TATCCCCAGGCGGTTACTATGTTATGGATGCCTGGGGCCAAGGG ACCACCGTGACCGTCTCCTCAGCTAGCACCAAAGGCCCATCGGT CTTCCCCCTGGCACCCTCCTCCAAGAGCACCTCTGGGGGCACAG CGGCCCTGGGCTGCCTGGTCAAGGACTACTTCCCCGAACCGGTG ACGGTGTCGTGGAACTCAGGCGCCCTGACCAGCGGCGTGCACAC CTTCCCGGCTGTCCTACAGTCCTCAGGACTCTACTCCCTCAGCAG CGTGGTGACCGTGCCCTCCAGCAGCTTGGGCACCCAGACCTACA TCTGCAACGTGAATCACAAGCCCAGCAACACCAAAGTGGACAA GAAAGTTGAGCCCAAATCTTGTGACGCGGCCGCAAGCACTAGTG CCCATCACCATCACCATCACGCCGCGGCA |

**Table 42: cDNA and amino acid sequences of library DP88-4 germline template**

| SEQ ID NO: | Description | Sequence |
|---|---|---|
| 104 | nucleotide sequence of Fab light chain Vkl_5 | see Table 4 |
| 105 | Fab light chain Vkl_5 | see Table 4 |
| 106 | nucleotide sequence of Fab heavy chain VH1_69 | see Table 4 |
| 107 | Fab heavy chain VH1_69 | see Table 4 |

**Table 43: Primer sequences used for generation of DP88-4 library**

| **SEQ ID NO:** | **Primer name** | **Primer sequence 5' - 3'** |
|---|---|---|
| 108 | LMB3 | CAGGAAACAGCTATGACCATGATTAC |
| 109 | Vk1_5_L3r_S | CTCGACTTTGGTGCCCTGGCCAAACGTSBAATA CGAATTATACTGTTGGCAGTAATAAGTTGCAAAATCAT |
| | | underlined: 60% original base and 40% randomization as M. |
| | | bolded and italic: 60% original base and 40% randomization as N |
| 110 | Vk1_5_L3r_SY | CTCGACTTTGGTGCCCTGGCCAAACGTMHRSGRATACGA ATTATACTGTTGGCAGTAATAAGTTGCAAAATCAT |
| | | underlined: 60% original base and 40% randomization as M. |
| | | bolded and italic: 60% original base and 40% randomization as N |
| 111 | Vk1_5_L3r_SPY | CTCGACTTTGGTGCCCTGGCCAAACGTMHHMSSSGRATA CGAATTATACTGTTGGCAGTAATAAGTTGCAAAATCAT |
| | | underlined: 60% original base and 40% randomization as M. |
| | | bolded and italic: 60% original base and 40% randomization as N |
| 112 | RJH31 | ACGTTTGGCCAGGGCACCAAAGTCGAG |
| 113 | RJH32 | TCTCGCACAGTAATACACGGCGGTGTCC |
| 114 | DP88-v4-4 | GGACACCGCCGTGTATTACTGTGCGAGA-1-2-2-3-4-GAC- TAC-TGGGGCCAAGGGACCACCGTGACCGTCTCC |
| | | 1: G/D = 20%, E/V/S = 10%, A/P/R/L/T/Y=5%; 2: G/Y/S=15%, A/D/T/R/P/L/V/N/W/F/I/E = 4,6%; 3: G/A/Y = 20%, P/W/S/D/T = 8%; 4: F = 46%, L/M = 15%, G/I/Y = 8%. |
| 115 | DP88-v4-6 | GGACACCGCCGTGTATTACTGTGCGAGA-1-2-2-2-2-3-4- GAC-TAC-TGGGGCCAAGGGACCACCGTGACCGTCTCC |
| | | 1: G/D = 20%, E/V/S = 10%, A/P/R/L/T/Y=5%; 2: G/Y/S=15%, A/D/T/R/P/L/V/N/W/F/I/E = 4,6%; 3: G/A/Y = 20%, P/W/S/D/T = 8%; 4: F = 46%, L/M = 15%, G/I/Y = 8%. |
| 116 | DP88-v4-8 | GGACACCGCCGTGTATTACTGTGCGAGA-1-2-2-2-2-2-2-3- 4-GAC-TAC-TGGGGCCAAGGGACCACCGTGACCGTCTCC |
| | | 1: G/D = 20%, E/V/S = 10%, A/P/R/L/T/Y=5%; 2: G/Y/S=15%, A/D/T/R/P/L/V/N/W/F/I/E = 4,6%; 3: G/A/Y = 20%, P/W/S/D/T = 8%; 4: F = 46%, L/M = 15%, G/I/Y = 8%. |
| 117 | fdseqlong | GACGTTAGTAAATGAATTTTCTGTATGAGG |

**Table 44** shows the sequence of generic phage-displayed lambda-DP47 library (V13_19/VH3_23) template used for PCRs. **Table 45** provides cDNA and amino acid sequences of lambda-DP47 library (Vl3_19/VH3_23) germline template and **Table 46** shows the Primer sequences used for generation of lambda-DP47 library (Vl3_19/VH3_23).

**Table 44: Sequence of generic phage- displayed lambda-DP47 library (Vl3_19/VH3_23) template used for PCRs**

| SEQ ID NO: | Description | Sequence |
|---|---|---|
| 129 | pRJH53 library template of lambda-**DP47 library Vl3_19/VH 3_23;** complete Fab coding region comprising PelB leader sequence + Vl3_19 lambda V-domain + CL constant domain for light chain and PelB + VH3 23 V-domain + CH1 constant domain for heavy chain including tags | ATGAAATACCTATTGCCTACGGCAGCCGCTGGATTGTTATTACTC GCGGCCCAGCCGGCCATGGCCTCGTCTGAGCTGACTCAGGACCC TGCTGTGTCTGTGGCCTTGGGACAGACAGTCAGGATCACATGCC AAGGAGACAGCCTCAGAAGTTATTATGCAAGCTGGTACCAGCAG AAGCCAGGACAGGCCCCTGTACTTGTCATCTATGGTAAAAACAA CCGGCCCTCAGGGATCCCAGACCGATTCTCTGGCTCCAGCTCAG GAAACACAGCTTCCTTGACCATCACTGGGGCTCAGGCGGAAGAT GAGGCTGACTATTACTGTAACTCCCGTGATAGTAGCGGTAATCA TGTGGTATTCGGCGGAGGGACCAAGCTGACCGTCCTAGGACAAC CCAAGGCTGCCCCCAGCGTGACCCTGTTCCCCCCCAGCAGCGAG GAATTGCAGGCCAACAAGGCCACCCTGGTCTGCCTGATCAGCGA CTTCTACCCAGGCGCCGTGACCGTGGCCTGGAAGGCCGACAGCA GCCCCGTGAAGGCCGGCGTGGAGACCACCACCCCCAGCAAGCA GAGCAACAACAAGTACGCCGCCAGCAGCTACCTGAGCCTGACC CCCGAGCAGTGGAAGAGCCACAGGTCCTACAGCTGCCAGGTGA CCCACGAGGGCAGCACCGTGGAGAAAACCGTGGCCCCCACCGA GTGCAGCGGAGCCGCAGAACAAAAACTCATCTCAGAAGAGGAT CTGAATGGAGCCGCAGACTACAAGGACGACGACGACAAGGGTG CCGCATAATAAGGCGCGCCAATTCTATTTCAAGGAGACAGTCAT ATGAAATACCTGCTGCCGACCGCTGCTGCTGGTCTGCTGCTCCTC GCTGCCCAGCCGGCGATGGCCGAGGTGCAATTGCTGGAGTCTGG GGGAGGCTTGGTACAGCCTGGGGGGTCCCTGAGACTCTCCTGTG CAGCCTCCGGATTCACCTTTAGCAGTTATGCCATGAGCTGGGTCC GCCAGGCTCCAGGGAAGGGGCTGGAGTGGGTCTCAGCTATTAGT GGTAGTGGTGGTAGCACATACTACGCAGACTCCGTGAAGGGCCG GTTCACCATCTCCAGAGACAATTCCAAGAACACGCTGTATCTGC AGATGAACAGCCTGAGAGCCGAGGACACGGCCGTATATTACTGT GCGAAACCGTTTCCGTATTTTGACTACTGGGGCCAAGGAACCCT GGTCACCGTCTCGAGTGCTAGCACCAAAGGCCCATCGGTCTTCC CCCTGGCACCCTCCTCCAAGAGCACCTCTGGGGGCACAGCGGCC CTGGGCTGCCTGGTCAAGGACTACTTCCCCGAACCGGTGACGGT GTCGTGGAACTCAGGCGCCCTGACCAGCGGCGTGCACACCTTCC CGGCTGTCCTACAGTCCTCAGGACTCTACTCCCTCAGCAGCGTG GTGACCGTGCCCTCCAGCAGCTTGGGCACCCAGACCTACATCTG |
| | | CAACGTGAATCACAAGCCCAGCAACACCAAAGTGGACAAGAAA GTTGAGCCCAAATCTTGTGACGCGGCCGCAAGCACTAGTGCCCA TCACCATCACCATCACGCCGCGGCA |

**Table 45: cDNA and amino acid sequences of lambda-DP47 library (Vl3_19/VH3_23) germline template**

| SEQ ID NO: | Description | Sequence |
|---|---|---|
| 130 | nucleotide sequence of Fab light chain Vl3_19 | see Table 10 |
| 131 | Fab light chain V13 _19 | see Table 10 |
| 121 | nucleotide sequence of Fab heavy chain VH3_23 | see Table 7 |
| 122 | Fab heavy chain VH3_23 (DP47) | see Table 7 |

**Table 46: Primer sequences used for generation of lambda-DP47 library (Vl3_19/VH3_23)**

| **SEQ ID NO:** | **Primer name** | **Primer sequence 5'** - **3'** |
|---|---|---|
| 132 | LMB3 | CAGGAAACAGCTATGACCATGATTAC |
| 133 | Vl_3_19_L3r_V | GGACGGTCAGCTTGGTCCCTCCGCCGAATAC VHV ATT ACC GCT ACT ATC ACG GGAGTTACAGTAATAGTCAGCCTCATCTTCCGC |
| | | underlined: 60% original base and 40% randomization as M |
| | | bold and italic: 60% oriainal base and 40% randomization as N |
| 134 | Vl_3_19_L3r_HV | GGACGGTCAGCTTGGTCCCTCCGCCGAATAC CMM ATG ATT ACC GCT ACT ATC ACG GGAGTTACAGTAATAGTCAGCCTCATCTTCCGC |
| | | underlined: 60% original base and 40% randomization as M |
| | | bolded and italic: 60% original base and 40% randomization as N |
| 135 | Vl_3_19_L3r_HL V | GGACGGTCAGCTTGGTCCCTCCGCCGAATAC RHM VWG ATG ATT ACC GCT ACT ATC ACG GGAGTTACAGTAATAGTCAGCCTCATCTTC CGC |
| | | underlined: 60% original base and 40% randomization as M |
| | | bolded and italic: 60% original base and 40% randomization as N |
| 136 | RJH80 | TTCGGCGGAGGGACCAAGCTGACCGTCC |
| 248 | MS63 | TTTCGCACAGTAATATACGGCCGTGTCC |
| 125 | DP47-v4-4 | CGAGGACACGGCCGTATATTACTGTGCG-5-1-2-2-3-4-GAC- |
| | | TAC-TGGGGCCAAGGAACCCTGGTCACCGTCTCG |
| 126 | DP47-v4-6 | CGAGGACACGGCCGTATATTACTGTGCG-5-1–2-2-2-2-3-4- GAC-TAC- TGGGGCCAAGGAACCCTGGTCACCGTCTCG |
| 127 | DP47-v4-8 | CGAGGACACGGCCGTATATTACTGTGCG-5-1–2-2-2-2-2-2-3-4- GAC-TAC- TGGGGCCAAGGAACCCTGGTCACCGTCTCG |
| 128 | fdseqlong | GACGTTAGTAAATGAATTTTCTGTATGAGG |

| | | |
|---|---|---|
| 1: G/D = 20%, E/V/S = 10%, A/P/R/L/T/Y=5%; 2: G/Y/S=15%, A/D/T/R/P/L/V/N/W/F/I/E = 4,6%; 3: G/A/Y = 20%, P/W/S/D/T = 8%; 4: F = 46%, L/M = 15%, G/I/Y = 8%; 5: K=70%, R =30%. | | |

Human, murine and cynomolgus 4-1BB (CD137) as antigens for the phage display selections and ELISA- and SPR-based screenings were transiently expressed as N-terminal monomeric Fc-fusion in HEK EBNA cells and in vivo site-specifically biotinylated via coexpression of BirA biotin ligase at the avi-tag recognition sequence located a the C-terminus of the Fc portion carrying the receptor chain (Fc knob chain).

Selection rounds (biopanning) were performed in solution according to the following procedure. First step, pre-clearing of ~ 10¹² phagemid particles on maxisorp plates coated with 10ug/ml of an unrelated human IgG to deplete the libraries of antibodies recognizing the Fcportion of the antigen; second, incubation of the non-binding phagemid particles with 100nM biotinylated human or murine 4-1BB for 0.5 h in the presence of 100nM unrelated nonbiotinylated Fc knob-into-hole construct for further depletion of Fc-binders in a total volume of 1ml; third, capture of biotinylated hu 4-1BB and attached specifically binding phage by transfer to 4 wells of a neutravidin pre-coated microtiter plate for 10 min (in rounds 1 & 3); fourth, washing of respective wells using 5x PBS/Tween20 and 5x PBS; fifth, elution of phage particles by addition of 250ul 100mM TEA (triethylamine) per well for 10 min and neutralization by addition of 500ul 1M Tris/HCl pH 7.4 to the pooled eluates from 4 wells; sixth, post-clearing of neutralized eluates by incubation on neutravidin pre-coated microtiter plate with 100nM biotincaptured Fc knob-into-hole construct for final removal of Fc-binders; seventh, re-infection of log-phase *E. coli* TG1 cells with the supernatant of eluted phage particles, infection with helperphage VCSM13, incubation on a shaker at 30°C over night and subsequent PEG/NaCl precipitation of phagemid particles to be used in the next selection round. Selections were carried out over 3 or 4 rounds using constant antigen concentrations of 100nM. In rounds 2 and 4, in order to avoid enrichment of binders to neutravidin, capture of antigen: phage complexes was performed by addition of 5.4 × 10⁷ streptavidin-coated magnetic beads. Specific binders were identified by ELISA as follows: 100 µl of 25 nM biotinylated human or murine 4-1BB and 10ug/ml of human IgG were coated on neutravidin plates and maxisorp plates, respectively. Fabcontaining bacterial supernatants were added and binding Fabs were detected via their Flag-tags using an anti-Flag/HRP secondary antibody. Clones exhibiting signals on human or murine 4-1BB and being negative on human IgG were short-listed for further analyses and were also tested in a similar fashion against the remaining two species of 4-1BB. They were bacterially expressed in a 0.5 liter culture volume, affinity purified and further characterized by SPR-analysis using BioRad's ProteOn XPR36 biosensor.

Clones 12B3, 25G7, 11D5 and 9B11 were identified as human 4-1BB-specific binder through the procedure described above. Clone 20G2 was identified as murine 4-1BB-specific binder through the procedure described above. The cDNA sequences of their variable regions are shown in **Table 47** below, the corresponding amino acid sequences can be found in Table C.

**Table 47: Variable region base pair sequences for phage-derived anti-4-1BB antibodies. Underlined are the complementary determining regions (CDRs).**

| Clone | SEQ ID NO: | Sequence |
|---|---|---|
| 12B3 | 277 (VL) | GACATCCAGATGACCCAGTCTCCTTCCACCCTGTCTGCATCTGTAGG AGACCGTGTCACCATCACTTGCCGTGCCAGTCAGAGTATTAGTAGC TGGTTGGCCTGGTATCAGCAGAAACCAGGGAAAGCCCCTAAGCTCC TGATCTATGATGCCTCCAGTTTGGAAAGTGGGGTCCCATCACGTTTC AGCGGCAGTGGATCCGGGACAGAATTCACTCTCACCATCAGCAGCT TGCAGCCTGATGATTTTGCAACTTATTACTGCCAACAGTATCATTCG TATCCGCAGACGTTTGGCCAGGGCACCAAAGTCGAGATCAAG |
| | 278 (VH) | CAGGTGCAATTGGTGCAGTCTGGGGCTGAGGTGAAGAAGCCTGGGT CCTCGGTGAAGGTCTCCTGCAAGGCCTCCGGAGGCACATTCAGCAG CTACGCTATAAGCTGGGTGCGACAGGCCCCTGGACAAGGGCTCGAG TGGATGGGAGGGATCATCCCTATCTTTGGTACAGCAAACTACGCAC AGAAGTTCCAGGGCAGGGTCACCATTACTGCAGACAAATCCACGAG CACAGCCTACATGGAGCTGAGCAGCCTGAGATCTGAGGACACCGCC GTGTATTACTGTGCGAGATCTGAATTCCGTTTCTACGCTGACTTCGA CTACTGGGGCCAAGGGACCACCGTGACCGTCTCCTCA |
| 25G7 | 279 (VL) | TCGTCTGAGCTGACTCAGGACCCTGCTGTGTCTGTGGCCTTGGGACA GACAGTCAGGATCACATGCCAAGGAGACAGCCTCAGAAGTTATTAT GCAAGCTGGTACCAGCAGAAGCCAGGACAGGCCCCTGTACTTGTCA TCTATGGTAAAAACAACCGGCCCTCAGGGATCCCAGACCGATTCTC TGGCTCCAGCTCAGGAAACACAGCTTCCTTGACCATCACTGGGGCT CAGGCGGAAGATGAGGCTGACTATTACTGTAACTCCCTTGATAGGC GCGGTATGTGGGTATTCGGCGGAGGGACCAAGCTGACCGTC |
| | 280 (VH) | GAGGTGCAATTGTTGGAGTCTGGGGGAGGCTTGGTACAGCCTGGGG GGTCCCTGAGACTCTCCTGTGCAGCCTCCGGATTCACCTTTAGCAGT TATGCCATGAGCTGGGTCCGCCAGGCTCCAGGGAAGGGGCTGGAGT GGGTCTCAGCTATTAGTGGTAGTGGTGGTAGCACATACTACGCAGA CTCCGTGAAGGGCCGGTTCACCATCTCCAGAGACAATTCCAAGAAC ACGCTGTATCTGCAGATGAACAGCCTGAGAGCCGAGGACACGGCCG TATATTACTGTGCGCGTGACGACCCGTGGCCGCCGTTCGACTACTGG GGCCAAGGAACCCTGGTCACCGTCTCGAGT |
| 11D5 | 281 (VL) | GACATCCAGATGACCCAGTCTCCTTCCACCCTGTCTGCATCTGTAGG AGACCGTGTCACCATCACTTGCCGTGCCAGTCAGAGTATTAGTAGC TGGTTGGCCTGGTATCAGCAGAAACCAGGGAAAGCCCCTAAGCTCC |
| | | TGATCTATGATGCCTCCAGTTTGGAAAGTGGGGTCCCATCACGTTTC AGCGGCAGTGGATCCGGGACAGAATTCACTCTCACCATCAGCAGCT TGCAGCCTGATGATTTTGCAACTTATTACTGCCAACAGCTTAATTCG TATCCTCAGACGTTTGGCCAGGGCACCAAAGTCGAGATCAAG |
| | 282 (VH) | CAGGTGCAATTGGTGCAGTCTGGGGCTGAGGTGAAGAAGCCTGGGT CCTCGGTGAAGGTCTCCTGCAAGGCCTCCGGAGGCACATTCAGCAG CTACGCTATAAGCTGGGTGCGACAGGCCCCTGGACAAGGGCTCGAG TGGATGGGAGGGATCATCCCTATCTTTGGTACAGCAAACTACGCAC AGAAGTTCCAGGGCAGGGTCACCATTACTGCAGACAAATCCACGAG CACAGCCTACATGGAGCTGAGCAGCCTGAGATCTGAGGACACCGCC GTGTATTACTGTGCGAGATCTACTCTGATCTACGGTTACTTCGACTA CTGGGGCCAAGGGACCACCGTGACCGTCTCCTCA |
| 9B11 | 283 (VL) | GACATCCAGATGACCCAGTCTCCTTCCACCCTGTCTGCATCTGTAGG AGACCGTGTCACCATCACTTGCCGTGCCAGTCAGAGTATTAGTAGC TGGTTGGCCTGGTATCAGCAGAAACCAGGGAAAGCCCCTAAGCTCC TGATCTATGATGCCTCCAGTTTGGAAAGTGGGGTCCCATCACGTTTC AGCGGCAGTGGATCCGGGACAGAATTCACTCTCACCATCAGCAGCT TGCAGCCTGATGATTTTGCAACTTATTACTGCCAACAGGTTAATTCT TATCCGCAGACGTTTGGCCAGGGCACCAAAGTCGAGATCAAG |
| | 284 (VH) | CAGGTGCAATTGGTGCAGTCTGGGGCTGAGGTGAAGAAGCCTGGGT CCTCGGTGAAGGTCTCCTGCAAGGCCTCCGGAGGCACATTCAGCAG CTACGCTATAAGCTGGGTGCGACAGGCCCCTGGACAAGGGCTCGAG TGGATGGGAGGGATCATCCCTATCTTTGGTACAGCAAACTACGCAC AGAAGTTCCAGGGCAGGGTCACCATTACTGCAGACAAATCCACGAG CACAGCCTACATGGAGCTGAGCAGCCTGAGATCTGAGGACACCGCC GTGTATTACTGTGCGAGATCTTCTGGTGCTTACCCGGGTTACTTCGA CTACTGGGGCCAAGGGACCACCGTGACCGTCTCCTCA |
| 20G2 | 285 (VL) | GACATCCAGATGACCCAGTCTCCATCCACCCTGTCTGCATCTGTAGG AGACCGTGTCACCATCACTTGCCGTGCCAGTCAGAGTATTAGTAGC TGGTTGGCCTGGTATCAGCAGAAACCAGGGAAAGCCCCTAAGCTCC TGATCTATGATGCCTCCAGTTTGGAAAGTGGGGTCCCATCACGTTTC AGCGGCAGTGGATCCGGGACAGAATTCACTCTCACCATCAGCAGCT TGCAGCCTGATGATTTTGCAACTTATTACTGCCAACAGCAGCACTCG TATTATACGTTTGGCCAGGGCACCAAAGTCGAGATCAAG |
| | 286 (VH) | CAGGTGCAATTGGTGCAGTCTGGGGCTGAGGTGAAGAAGCCTGGGT CCTCGGTGAAGGTCTCCTGCAAGGCCTCCGGAGGCACATTCAGCAG CTACGCTATAAGCTGGGTGCGACAGGCCCCTGGACAAGGGCTCGAG TGGATGGGAGGGATCATCCCTATCTTTGGTACAGCAAACTACGCAC AGAAGTTCCAGGGCAGGGTCACCATTACTGCAGACAAATCCACGAG CACAGCCTACATGGAGCTGAGCAGCCTGAGATCTGAGGACACCGCC GTGTATTACTGTGCGAGATCTTACTACTGGGAATCTTACCCGTTCGA CTACTGGGGCCAAGGGACCACCGTGACCGTCTCCAGC |

### 6.3 Preparation, purification and characterization of anti-4-1BB IgG1 P329G LALA antibodies

The variable regions of heavy and light chain DNA sequences of selected anti-4-1BB binders were subcloned in frame with either the constant heavy chain or the constant light chain of human IgG1. The Pro329Gly, Leu234Ala and Leu235Ala mutations have been introduced in the constant region of the knob and hole heavy chains to abrogate binding to Fc gamma receptors according to the method described in International Patent Appl. Publ. No. WO 2012/130831 A1.

The nucleotide and amino acid sequences of the anti-4-1BB clones are shown in **Table 48.** All anti-4-1BB-Fc-fusion encoding sequences were cloned into a plasmid vector, which drives expression of the insert from an MPSV promoter and contains a synthetic polyA signal sequence located at the 3' end of the CDS. In addition, the vector contains an EBV OriP sequence for episomal maintenance of the plasmid.

**Table 48: Sequences of anti-4-1BB clones in P329GLALA human IgG1 format**

| **Clone** | **SEQ ID No.** | **Sequence** |
|---|---|---|
| **12B3** | 287 (nucleotide sequence light chain) | GACATCCAGATGACCCAGTCTCCTTCCACCCTGTCTGCATCTGT AGGAGACCGTGTCACCATCACTTGCCGTGCCAGTCAGAGTATT AGTAGCTGGTTGGCCTGGTATCAGCAGAAACCAGGGAAAGCC CCTAAGCTCCTGATCTATGATGCCTCCAGTTTGGAAAGTGGGG TCCCATCACGTTTCAGCGGCAGTGGATCCGGGACAGAATTCAC TCTCACCATCAGCAGCTTGCAGCCTGATGATTTTGCAACTTATT ACTGCCAACAGTATCATTCGTATCCGCAGACGTTTGGCCAGGG CACCAAAGTCGAGATCAAGCGTACGGTGGCTGCACCATCTGTC TTCATCTTCCCGCCATCTGATGAGCAGTTGAAATCTGGAACTGC CTCTGTTGTGTGCCTGCTGAATAACTTCTATCCCAGAGAGGCCA AAGTACAGTGGAAGGTGGATAACGCCCTCCAATCGGGTAACTC CCAGGAGAGTGTCACAGAGCAGGACAGCAAGGACAGCACCTA CAGCCTCAGCAGCACCCTGACGCTGAGCAAAGCAGACTACGA GAAACACAAAGTCTACGCCTGCGAAGTCACCCATCAGGGCCTG AGCTCGCCCGTCACAAAGAGCTTCAACAGGGGAGAGTGT |
| | 288 (nucleotide sequence heavy chain) | CAGGTGCAATTGGTGCAGTCTGGGGCTGAGGTGAAGAAGCCTG GGTCCTCGGTGAAGGTCTCCTGCAAGGCCTCCGGAGGCACATT CAGCAGCTACGCTATAAGCTGGGTGCGACAGGCCCCTGGACAA GGGCTCGAGTGGATGGGAGGGATCATCCCTATCTTTGGTACAG CAAACTACGCACAGAAGTTCCAGGGCAGGGTCACCATTACTGC AGACAAATCCACGAGCACAGCCTACATGGAGCTGAGCAGCCT GAGATCTGAGGACACCGCCGTGTATTACTGTGCGAGATCTGAA TTCCGTTTCTACGCTGACTTCGACTACTGGGGCCAAGGGACCA CCGTGACCGTCTCCTCAGCTAGCACCAAGGGCCCATCGGTCTT CCCCCTGGCACCCTCCTCCAAGAGCACCTCTGGGGGCACAGCG GCCCTGGGCTGCCTGGTCAAGGACTACTTCCCCGAACCGGTGA CGGTGTCGTGGAACTCAGGCGCCCTGACCAGCGGCGTGCACAC CTTCCCGGCTGTCCTACAGTCCTCAGGACTCTACTCCCTCAGCA GCGTGGTGACCGTGCCCTCCAGCAGCTTGGGCACCCAGACCTA CATCTGCAACGTGAATCACAAGCCCAGCAACACCAAGGTGGA CAAGAAAGTTGAGCCCAAATCTTGTGACAAAACTCACACATGC CCACCGTGCCCAGCACCTGAAGCTGCAGGGGGACCGTCAGTCT TCCTCTTCCCCCCAAAACCCAAGGACACCCTCATGATCTCCCG GACCCCTGAGGTCACATGCGTGGTGGTGGACGTGAGCCACGAA GACCCTGAGGTCAAGTTCAACTGGTACGTGGACGGCGTGGAGG TGCATAATGCCAAGACAAAGCCGCGGGAGGAGCAGTACAACA GCACGTACCGTGTGGTCAGCGTCCTCACCGTCCTGCACCAGGA CTGGCTGAATGGCAAGGAGTACAAGTGCAAGGTCTCCAACAA |
| | | AGCCCTCGGCGCCCCCATCGAGAAAACCATCTCCAAAGCCAAA GGGCAGCCCCGAGAACCACAGGTGTACACCCTGCCCCCATCCC GGGATGAGCTGACCAAGAACCAGGTCAGCCTGACCTGCCTGGT CAAAGGCTTCTATCCCAGCGACATCGCCGTGGAGTGGGAGAGC AATGGGCAGCCGGAGAACAACTACAAGACCACGCCTCCCGTG CTGGACTCCGACGGCTCCTTCTTCCTCTACAGCAAGCTCACCGT GGACAAGAGCAGGTGGCAGCAGGGGAACGTCTTCTCATGCTCC GTGATGCATGAGGCTCTGCACAACCACTACACGCAGAAGAGCC TCTCCCTGTCTCCGGGTAAA |
| | 289 (Light chain) | DIQMTQSPSTLSASVGDRVTITCRASQSISSWLAWYQQKPG KAPKLLIYDASSLESGVPSRFSGSGSGTEFTLTISSLQPDDFA TYYCQQYHSYPQTFGQGTKVEIKRTVAAPSVFIFPPSDEQLK SGTASVVCLLNNFYPREAKVQWKVDNALQSGNSQESVTEQ DSKDSTYSLSSTLTLSKADYEKHKVYACEVTHQGLSSPVTK SFNRGEC |
| | 290 (Heavy chain) | QVQLVQSGAEVKKPGSSVKVSCKASGGTFSSYAISWVRQAP GQGLEWMGGIIPIFGTANYAQKFQGRVTITADKSTSTAYME LSSLRSEDTAVYYCARSEFRFYADFDYWGQGTTVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKKVEPKSCDKTHTCPPCPAPEAAGGPSVFLFPP KPKDTLMISRTPEVTCVVVDVSHEDPEVKFNWYVDGVEVH NAKTKPREEQYNSTYRVVSVLTVLHQDWLNGKEYKCKVS NKALGAPIEKTISKAKGQPREPQVYTLPPSRDELTKNQVSLT CLVKGFYPSDIAVEWESNGQPENNYKTTPPVLDSDGSFFLY SKLTVDKSRWQQGNVFSCSVMHEALHNHYTQKSLSLSPGK |
| **25G7** | 291 (nucleotide sequence light chain) | TCGTCTGAGCTGACTCAGGACCCTGCTGTGTCTGTGGCCTTGGG ACAGACAGTCAGGATCACATGCCAAGGAGACAGCCTCAGAAG TTATTATGCAAGCTGGTACCAGCAGAAGCCAGGACAGGCCCCT GTACTTGTCATCTATGGTAAAAACAACCGGCCCTCAGGGATCC CAGACCGATTCTCTGGCTCCAGCTCAGGAAACACAGCTTCCTT GACCATCACTGGGGCTCAGGCGGAAGATGAGGCTGACTATTAC TGTAACTCCCTTGATAGGCGCGGTATGTGGGTATTCGGCGGAG GGACCAAGCTGACCGTCCTAGGTCAACCCAAGGCTGCCCCCAG CGTGACCCTGTTCCCCCCCAGCAGCGAGGAACTGCAGGCCAAC AAGGCCACCCTGGTCTGCCTGATCAGCGACTTCTACCCAGGCG CCGTGACCGTGGCCTGGAAGGCCGACAGCAGCCCCGTGAAGG CCGGCGTGGAGACCACCACCCCCAGCAAGCAGAGCAACAACA AGTACGCCGCCAGCAGCTACCTGAGCCTGACCCCCGAGCAGTG GAAGAGCCACAGGTCCTACAGCTGCCAGGTGACCCACGAGGG CAGCACCGTGGAGAAAACCGTGGCCCCCACCGAGTGCAGC |
| | 292 (nucleotide sequence heavy chain) | GAGGTGCAATTGTTGGAGTCTGGGGGAGGCTTGGTACAGCCTG GGGGGTCCCTGAGACTCTCCTGTGCAGCCTCCGGATTCACCTTT AGCAGTTATGCCATGAGCTGGGTCCGCCAGGCTCCAGGGAAGG GGCTGGAGTGGGTCTCAGCTATTAGTGGTAGTGGTGGTAGCAC ATACTACGCAGACTCCGTGAAGGGCCGGTTCACCATCTCCAGA GACAATTCCAAGAACACGCTGTATCTGCAGATGAACAGCCTGA GAGCCGAGGACACGGCCGTATATTACTGTGCGCGTGACGACCC GTGGCCGCCGTTCGACTACTGGGGCCAAGGAACCCTGGTCACC GTCTCGAGTGCTAGCACCAAGGGCCCATCGGTCTTCCCCCTGG CACCCTCCTCCAAGAGCACCTCTGGGGGCACAGCGGCCCTGGG CTGCCTGGTCAAGGACTACTTCCCCGAACCGGTGACGGTGTCG |
| | | TGGAACTCAGGCGCCCTGACCAGCGGCGTGCACACCTTCCCGG CTGTCCTACAGTCCTCAGGACTCTACTCCCTCAGCAGCGTGGTG ACCGTGCCCTCCAGCAGCTTGGGCACCCAGACCTACATCTGCA ACGTGAATCACAAGCCCAGCAACACCAAGGTGGACAAGAAAG TTGAGCCCAAATCTTGTGACAAAACTCACACATGCCCACCGTG CCCAGCACCTGAAGCTGCAGGGGGACCGTCAGTCTTCCTCTTC CCCCCAAAACCCAAGGACACCCTCATGATCTCCCGGACCCCTG AGGTCACATGCGTGGTGGTGGACGTGAGCCACGAAGACCCTG AGGTCAAGTTCAACTGGTACGTGGACGGCGTGGAGGTGCATAA TGCCAAGACAAAGCCGCGGGAGGAGCAGTACAACAGCACGTA CCGTGTGGTCAGCGTCCTCACCGTCCTGCACCAGGACTGGCTG AATGGCAAGGAGTACAAGTGCAAGGTCTCCAACAAAGCCCTC GGCGCCCCCATCGAGAAAACCATCTCCAAAGCCAAAGGGCAG CCCCGAGAACCACAGGTGTACACCCTGCCCCCATCCCGGGATG AGCTGACCAAGAACCAGGTCAGCCTGACCTGCCTGGTCAAAGG CTTCTATCCCAGCGACATCGCCGTGGAGTGGGAGAGCAATGGG CAGCCGGAGAACAACTACAAGACCACGCCTCCCGTGCTGGACT CCGACGGCTCCTTCTTCCTCTACAGCAAGCTCACCGTGGACAA GAGCAGGTGGCAGCAGGGGAACGTCTTCTCATGCTCCGTGATG CATGAGGCTCTGCACAACCACTACACGCAGAAGAGCCTCTCCC TGTCTCCGGGTAAA |
| | 293 (Light chain) | SSELTQDPAVSVALGQTVRITCQGDSLRSYYASWYQQKPGQAPV LVIYGKNNRPSGIPDRFSGSSSGNTASLTITGAQAEDEADYYCNSL DRRGMWVFGGGTKLTVLGQPKAAPSVTLFPPSSEELQANKATLV CLISDFYPGAVTVAWKADSSPVKAGVETTTPSKQSNNKYAASSYL SLTPEQWKSHRSYSCQVTHEGSTVEKTVAPTECS |
| | 294 (Heavy chain) | EVQLLESGGGLVQPGGSLRLSCAASGFTFSSYAMSWVRQAPGKG LEWVSAISGSGGSTYYADSVKGRFTISRDNSKNTLYLQMNSLRAE DTAVYYCARDDPWPPFDYWGQGTLVTVSSASTKGPSVFPLAPSS KSTSGGTAALGCLVKDYFPEPVTVSWNSGALTSGVHTFPAVLQSS GLYSLSSVVTVPSSSLGTQTYICNVNHKPSNTKVDKKVEPKSCDK THTCPPCPAPEAAGGPSVFLFPPKPKDTLMISRTPEVTCVVVDVSH EDPEVKFNWYVDGVEVHNAKTKPREEQYNSTYRVVSVLTVLHQ DWLNGKEYKCKVSNKALGAPIEKTISKAKGQPREPQVYTLPPSRD ELTKNQVSLTCLVKGFYPSDIAVEWESNGQPENNYKTTPPVLDSD GSFFLYSKLTVDKSRWQQGNVFSCSVMHEALHNHYTQKSLSLSP GK |
| **11D5** | 295 (nucleotide sequence light chain) | GACATCCAGATGACCCAGTCTCCTTCCACCCTGTCTGCATCTGT AGGAGACCGTGTCACCATCACTTGCCGTGCCAGTCAGAGTATT AGTAGCTGGTTGGCCTGGTATCAGCAGAAACCAGGGAAAGCC CCTAAGCTCCTGATCTATGATGCCTCCAGTTTGGAAAGTGGGG TCCCATCACGTTTCAGCGGCAGTGGATCCGGGACAGAATTCAC TCTCACCATCAGCAGCTTGCAGCCTGATGATTTTGCAACTTATT ACTGCCAACAGCTTAATTCGTATCCTCAGACGTTTGGCCAGGG CACCAAAGTCGAGATCAAGCGTACGGTGGCTGCACCATCTGTC TTCATCTTCCCGCCATCTGATGAGCAGTTGAAATCTGGAACTGC CTCTGTTGTGTGCCTGCTGAATAACTTCTATCCCAGAGAGGCCA AAGTACAGTGGAAGGTGGATAACGCCCTCCAATCGGGTAACTC CCAGGAGAGTGTCACAGAGCAGGACAGCAAGGACAGCACCTA CAGCCTCAGCAGCACCCTGACGCTGAGCAAAGCAGACTACGA GAAACACAAAGTCTACGCCTGCGAAGTCACCCATCAGGGCCTG AGCTCGCCCGTCACAAAGAGCTTCAACAGGGGAGAGTGT |
| | | CAGGTGCAATTGGTGCAGTCTGGGGCTGAGGTGAAGAAGCCTG |
| | 296 (nucleotide sequence heavy chain) | GGTCCTCGGTGAAGGTCTCCTGCAAGGCCTCCGGAGGCACATT CAGCAGCTACGCTATAAGCTGGGTGCGACAGGCCCCTGGACAA GGGCTCGAGTGGATGGGAGGGATCATCCCTATCTTTGGTACAG CAAACTACGCACAGAAGTTCCAGGGCAGGGTCACCATTACTGC AGACAAATCCACGAGCACAGCCTACATGGAGCTGAGCAGCCT GAGATCTGAGGACACCGCCGTGTATTACTGTGCGAGATCTACT CTGATCTACGGTTACTTCGACTACTGGGGCCAAGGGACCACCG TGACCGTCTCCTCAGCTAGCACCAAGGGCCCATCGGTCTTCCC CCTGGCACCCTCCTCCAAGAGCACCTCTGGGGGCACAGCGGCC CTGGGCTGCCTGGTCAAGGACTACTTCCCCGAACCGGTGACGG TGTCGTGGAACTCAGGCGCCCTGACCAGCGGCGTGCACACCTT CCCGGCTGTCCTACAGTCCTCAGGACTCTACTCCCTCAGCAGC GTGGTGACCGTGCCCTCCAGCAGCTTGGGCACCCAGACCTACA TCTGCAACGTGAATCACAAGCCCAGCAACACCAAGGTGGACA AGAAAGTTGAGCCCAAATCTTGTGACAAAACTCACACATGCCC ACCGTGCCCAGCACCTGAAGCTGCAGGGGGACCGTCAGTCTTC CTCTTCCCCCCAAAACCCAAGGACACCCTCATGATCTCCCGGA CCCCTGAGGTCACATGCGTGGTGGTGGACGTGAGCCACGAAGA CCCTGAGGTCAAGTTCAACTGGTACGTGGACGGCGTGGAGGTG CATAATGCCAAGACAAAGCCGCGGGAGGAGCAGTACAACAGC ACGTACCGTGTGGTCAGCGTCCTCACCGTCCTGCACCAGGACT GGCTGAATGGCAAGGAGTACAAGTGCAAGGTCTCCAACAAAG CCCTCGGCGCCCCCATCGAGAAAACCATCTCCAAAGCCAAAGG GCAGCCCCGAGAACCACAGGTGTACACCCTGCCCCCATCCCGG GATGAGCTGACCAAGAACCAGGTCAGCCTGACCTGCCTGGTCA AAGGCTTCTATCCCAGCGACATCGCCGTGGAGTGGGAGAGCAA TGGGCAGCCGGAGAACAACTACAAGACCACGCCTCCCGTGCTG GACTCCGACGGCTCCTTCTTCCTCTACAGCAAGCTCACCGTGG ACAAGAGCAGGTGGCAGCAGGGGAACGTCTTCTCATGCTCCGT GATGCATGAGGCTCTGCACAACCACTACACGCAGAAGAGCCTC TCCCTGTCTCCGGGTAAA |
| | 297 (Light chain) | DIQMTQSPSTLSASVGDRVTITCRASQSISSWLAWYQQKPGKAPK LLIYDASSLESGVPSRFSGSGSGTEFTLTISSLQPDDFATYYCQQLN SYPQTFGQGTKVEIKRTVAAPSVFIFPPSDEQLKSGTASVVCLLNN FYPREAKVQWKVDNALQSGNSQESVTEQDSKDSTYSLSSTLTLSK ADYEKHKVYACEVTHQGLSSPVTKSFNRGEC |
| | 298 (Heavy chain) | QVQLVQSGAEVKKPGSSVKVSCKASGGTFSSYAISWVRQAPGQG LEWMGGIIPIFGTANYAQKFQGRVTITADKSTSTAYMELSSLRSED TAVYYCARSTLIYGYFDYWGQGTTVTVSSASTKGPSVFPLAPSSK STSGGTAALGCLVKDYFPEPVTVSWNSGALTSGVHTFPAVLQSSG LYSLSSVVTVPSSSLGTQTYICNVNHKPSNTKVDKKVEPKSCDKT HTCPPCPAPEAAGGPSVFLFPPKPKDTLMISRTPEVTCVVVDVSHE DPEVKFNWYVDGVEVHNAKTKPREEQYNSTYRVVSVLTVLHQD WLNGKEYKCKVSNKALGAPIEKTISKAKGQPREPQVYTLPPSRDE LTKNQVSLTCLVKGFYPSDIAVEWESNGQPENNYKTTPPVLDSDG SFFLYSKLTVDKSRWQQGNVFSCSVMHEALHNHYTQKSLSLSPG K |
| **9B11** | 299 (nucleotide sequence light chain) | GACATCCAGATGACCCAGTCTCCTTCCACCCTGTCTGCATCTGT AGGAGACCGTGTCACCATCACTTGCCGTGCCAGTCAGAGTATT AGTAGCTGGTTGGCCTGGTATCAGCAGAAACCAGGGAAAGCC CCTAAGCTCCTGATCTATGATGCCTCCAGTTTGGAAAGTGGGG TCCCATCACGTTTCAGCGGCAGTGGATCCGGGACAGAATTCAC TCTCACCATCAGCAGCTTGCAGCCTGATGATTTTGCAACTTATT |
| | | ACTGCCAACAGGTTAATTCTTATCCGCAGACGTTTGGCCAGGG CACCAAAGTCGAGATCAAGCGTACGGTGGCTGCACCATCTGTC TTCATCTTCCCGCCATCTGATGAGCAGTTGAAATCTGGAACTGC CTCTGTTGTGTGCCTGCTGAATAACTTCTATCCCAGAGAGGCCA AAGTACAGTGGAAGGTGGATAACGCCCTCCAATCGGGTAACTC CCAGGAGAGTGTCACAGAGCAGGACAGCAAGGACAGCACCTA CAGCCTCAGCAGCACCCTGACGCTGAGCAAAGCAGACTACGA GAAACACAAAGTCTACGCCTGCGAAGTCACCCATCAGGGCCTG AGCTCGCCCGTCACAAAGAGCTTCAACAGGGGAGAGTGT |
| | 300 (nucleotide sequence heavy chain) | CAGGTGCAATTGGTGCAGTCTGGGGCTGAGGTGAAGAAGCCTG GGTCCTCGGTGAAGGTCTCCTGCAAGGCCTCCGGAGGCACATT CAGCAGCTACGCTATAAGCTGGGTGCGACAGGCCCCTGGACAA GGGCTCGAGTGGATGGGAGGGATCATCCCTATCTTTGGTACAG CAAACTACGCACAGAAGTTCCAGGGCAGGGTCACCATTACTGC AGACAAATCCACGAGCACAGCCTACATGGAGCTGAGCAGCCT GAGATCTGAGGACACCGCCGTGTATTACTGTGCGAGATCTTCT GGTGCTTACCCGGGTTACTTCGACTACTGGGGCCAAGGGACCA CCGTGACCGTCTCCTCAGCTAGCACCAAGGGCCCATCGGTCTT CCCCCTGGCACCCTCCTCCAAGAGCACCTCTGGGGGCACAGCG GCCCTGGGCTGCCTGGTCAAGGACTACTTCCCCGAACCGGTGA CGGTGTCGTGGAACTCAGGCGCCCTGACCAGCGGCGTGCACAC CTTCCCGGCTGTCCTACAGTCCTCAGGACTCTACTCCCTCAGCA GCGTGGTGACCGTGCCCTCCAGCAGCTTGGGCACCCAGACCTA CATCTGCAACGTGAATCACAAGCCCAGCAACACCAAGGTGGA CAAGAAAGTTGAGCCCAAATCTTGTGACAAAACTCACACATGC CCACCGTGCCCAGCACCTGAAGCTGCAGGGGGACCGTCAGTCT TCCTCTTCCCCCCAAAACCCAAGGACACCCTCATGATCTCCCG GACCCCTGAGGTCACATGCGTGGTGGTGGACGTGAGCCACGAA GACCCTGAGGTCAAGTTCAACTGGTACGTGGACGGCGTGGAGG TGCATAATGCCAAGACAAAGCCGCGGGAGGAGCAGTACAACA GCACGTACCGTGTGGTCAGCGTCCTCACCGTCCTGCACCAGGA CTGGCTGAATGGCAAGGAGTACAAGTGCAAGGTCTCCAACAA AGCCCTCGGCGCCCCCATCGAGAAAACCATCTCCAAAGCCAAA GGGCAGCCCCGAGAACCACAGGTGTACACCCTGCCCCCATCCC GGGATGAGCTGACCAAGAACCAGGTCAGCCTGACCTGCCTGGT CAAAGGCTTCTATCCCAGCGACATCGCCGTGGAGTGGGAGAGC AATGGGCAGCCGGAGAACAACTACAAGACCACGCCTCCCGTG CTGGACTCCGACGGCTCCTTCTTCCTCTACAGCAAGCTCACCGT GGACAAGAGCAGGTGGCAGCAGGGGAACGTCTTCTCATGCTCC GTGATGCATGAGGCTCTGCACAACCACTACACGCAGAAGAGCC TCTCCCTGTCTCCGGGTAAA |
| | 301 (Light chain) | DIQMTQSPSTLSASVGDRVTITCRASQSISSWLAWYQQKPGKAPK LLIYDASSLESGVPSRFSGSGSGTEFTLTISSLQPDDFATYYCQQVN SYPQTFGQGTKVEIKRTVAAPSVFIFPPSDEQLKSGTASVVCLLNN FYPREAKVQWKVDNALQSGNSQESVTEQDSKDSTYSLSSTLTLSK ADYEKHKVYACEVTHQGLSSPVTKSFNRGEC |
| | 302 (Heavy chain) | QVQLVQSGAEVKKPGSSVKVSCKASGGTFSSYAISWVRQAPGQG LEWMGGIIPIFGTANYAQKFQGRVTITADKSTSTAYMELSSLRSED TAVYYCARSSGAYPGYFDYWGQGTTVTVSSASTKGPSVFPLAPSS KSTSGGTAALGCLVKDYFPEPVTVSWNSGALTSGVHTFPAVLQSS GLYSLSSVVTVPSSSLGTQTYICNVNHKPSNTKVDKKVEPKSCDK THTCPPCPAPEAAGGPSVFLFPPKPKDTLMISRTPEVTCVVVDVSH EDPEVKFNWYVDGVEVHNAKTKPREEQYNSTYRVVSVLTVLHQ |
| | | DWLNGKEYKCKVSNKALGAPIEKTISKAKGQPREPQVYTLPPSRD ELTKNQVSLTCLVKGFYPSDIAVEWESNGQPENNYKTTPPVLDSD GSFFLYSKLTVDKSRWQQGNVFSCSVMHEALHNHYTQKSLSLSP GK |
| **20G2** | 303 (nucleotide sequence light chain) | GACATCCAGATGACCCAGTCTCCATCCACCCTGTCTGCATCTGT AGGAGACCGTGTCACCATCACTTGCCGTGCCAGTCAGAGTATT AGTAGCTGGTTGGCCTGGTATCAGCAGAAACCAGGGAAAGCC CCTAAGCTCCTGATCTATGATGCCTCCAGTTTGGAAAGTGGGG TCCCATCACGTTTCAGCGGCAGTGGATCCGGGACAGAATTCAC TCTCACCATCAGCAGCTTGCAGCCTGATGATTTTGCAACTTATT ACTGCCAACAGCAGCACTCGTATTATACGTTTGGCCAGGGCAC CAAAGTCGAGATCAAGCGTACGGTGGCTGCACCATCTGTCTTC ATCTTCCCGCCATCTGATGAGCAGTTGAAATCTGGAACTGCCT CTGTTGTGTGCCTGCTGAATAACTTCTATCCCAGAGAGGCCAA AGTACAGTGGAAGGTGGATAACGCCCTCCAATCGGGTAACTCC CAGGAGAGTGTCACAGAGCAGGACAGCAAGGACAGCACCTAC AGCCTCAGCAGCACCCTGACGCTGAGCAAAGCAGACTACGAG AAACACAAAGTCTACGCCTGCGAAGTCACCCATCAGGGCCTGA GCTCGCCCGTCACAAAGAGCTTCAACAGGGGAGAGTGT |
| | 304 (nucleotide sequence heavy chain) | CAGGTGCAATTGGTGCAGTCTGGGGCTGAGGTGAAGAAGCCTG GGTCCTCGGTGAAGGTCTCCTGCAAGGCCTCCGGAGGCACATT CAGCAGCTACGCTATAAGCTGGGTGCGACAGGCCCCTGGACAA GGGCTCGAGTGGATGGGAGGGATCATCCCTATCTTTGGTACAG CAAACTACGCACAGAAGTTCCAGGGCAGGGTCACCATTACTGC AGACAAATCCACGAGCACAGCCTACATGGAGCTGAGCAGCCT GAGATCTGAGGACACCGCCGTGTATTACTGTGCGAGATCTTAC TACTGGGAATCTTACCCGTTCGACTACTGGGGCCAAGGGACCA CCGTGACCGTCTCCAGCGCTAGCACCAAGGGCCCATCGGTCTT CCCCCTGGCACCCTCCTCCAAGAGCACCTCTGGGGGCACAGCG GCCCTGGGCTGCCTGGTCAAGGACTACTTCCCCGAACCGGTGA CGGTGTCGTGGAACTCAGGCGCCCTGACCAGCGGCGTGCACAC CTTCCCGGCTGTCCTACAGTCCTCAGGACTCTACTCCCTCAGCA GCGTGGTGACCGTGCCCTCCAGCAGCTTGGGCACCCAGACCTA CATCTGCAACGTGAATCACAAGCCCAGCAACACCAAGGTGGA CAAGAAAGTTGAGCCCAAATCTTGTGACAAAACTCACACATGC CCACCGTGCCCAGCACCTGAAGCTGCAGGGGGACCGTCAGTCT TCCTCTTCCCCCCAAAACCCAAGGACACCCTCATGATCTCCCG GACCCCTGAGGTCACATGCGTGGTGGTGGACGTGAGCCACGAA GACCCTGAGGTCAAGTTCAACTGGTACGTGGACGGCGTGGAGG TGCATAATGCCAAGACAAAGCCGCGGGAGGAGCAGTACAACA GCACGTACCGTGTGGTCAGCGTCCTCACCGTCCTGCACCAGGA CTGGCTGAATGGCAAGGAGTACAAGTGCAAGGTCTCCAACAA AGCCCTCGGCGCCCCCATCGAGAAAACCATCTCCAAAGCCAAA GGGCAGCCCCGAGAACCACAGGTGTACACCCTGCCCCCATCCC GGGATGAGCTGACCAAGAACCAGGTCAGCCTGACCTGCCTGGT CAAAGGCTTCTATCCCAGCGACATCGCCGTGGAGTGGGAGAGC AATGGGCAGCCGGAGAACAACTACAAGACCACGCCTCCCGTG CTGGACTCCGACGGCTCCTTCTTCCTCTACAGCAAGCTCACCGT GGACAAGAGCAGGTGGCAGCAGGGGAACGTCTTCTCATGCTCC GTGATGCATGAGGCTCTGCACAACCACTACACGCAGAAGAGCC TCTCCCTGTCTCCGGGTAAA |
| | 305 (Light chain) | DIQMTQSPSTLSASVGDRVTITCRASQSISSWLAWYQQKPGKAPK LLIYDASSLESGVPSRFSGSGSGTEFTLTISSLQPDDFATYYCQQH |
| | | SYYTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| | 306 (Heavy chain) | QVQLVQSGAEVKKPGSSVKVSCKASGGTFSSYAISWVRQAPGQG LEWMGGIIPIFGTANYAQKFQGRVTITADKSTSTAYMELSSLRSED TAVYYCARSYYWESYPFDYWGQGTTVTVSSASTKGPSVFPLAPS SKSTSGGTAALGCLVKDYFPEPVTVSWNSGALTSGVHTFPAVLQS SGLYSLSSVVTVPSSSLGTQTYICNVNHKPSNTKVDKKVEPKSCD KTHTCPPCPAPEAAGGPSVFLFPPKPKDTLMISRTPEVTCVVVDVS HEDPEVKFNWYVDGVEVHNAKTKPREEQYNSTYRVVSVLTVLH QDWLNGKEYKCKVSNKALGAPIEKTISKAKGQPREPQVYTLPPSR DELTKNQVSLTCLVKGFYPSDIAVEWESNGQPENNYKTTPPVLDS DGSFFLYSKLTVDKSRWQQGNVFSCSVMHEALHNHYTQKSLSLS PGK |

The anti-4-BB antibodies were produced by co-transfecting HEK293-EBNA cells with the mammalian expression vectors using polyethylenimine. The cells were transfected with the corresponding expression vectors in a 1:1 ratio ("vector heavy chain" : "vector light chain" ).

For production in 500 mL shake flasks, 400 million HEK293 EBNA cells were seeded 24 hours before transfection. For transfection cells were centrifuged for 5 minutes at 210 x g, and the supernatant was replaced by pre-warmed CD CHO medium. Expression vectors (200 µg of total DNA) were mixed in 20 mL CD CHO medium. After addition of 540 µL PEI, the solution was vortexed for 15 seconds and incubated for 10 minutes at room temperature. Afterwards, cells were mixed with the DNA/PEI solution, transferred to a 500 mL shake flask and incubated for 3 hours at 37°C in an incubator with a 5% CO2 atmosphere. After the incubation, 160 mL of F17 medium was added and cells were cultured for 24 hours. One day after transfection 1 mM valproic acid and 7% Feed with supplements were added. After culturing for 7 days, the supernatant was collected by centrifugation for 15 minutes at 210 x g. The solution was sterile filtered (0.22 µm filter), supplemented with sodium azide to a final concentration of 0.01 % (w/v), and kept at 4°C.

Purification of antibody molecules from cell culture supernatants was carried out by affinity chromatography using Protein A as described above for purification of antigen Fc fusions.

The protein was concentrated and filtered prior to loading on a HiLoad Superdex 200 column (GE Healthcare) equilibrated with 20mM Histidine, 140 mM NaCl solution of pH 6.0.

The protein concentration of purified antibodies was determined by measuring the OD at 280 nm, using the molar extinction coefficient calculated on the basis of the amino acid sequence. Purity and molecular weight of the antibodies were analyzed by CE-SDS in the presence and absence of a reducing agent (Invitrogen, USA) using a LabChipGXII (Caliper). The aggregate content of antibody samples was analyzed using a TSKgel G3000 SW XL analytical size-exclusion column (Tosoh) equilibrated in a 25 mM K₂HPO₄, 125 mM NaCl, 200mM L-Arginine Monohydrocloride, 0.02 % (w/v) NaN₃, pH 6.7 running buffer at 25°C.

**Table 49** summarizes the yield and final content of the anti-4-BB P329G LALA IgG1 antibodies.

**Table 49: Biochemical analysis of anti-4-BB P329G LALA IgG1 clones**

| Clone | Yield [mg/l] | Monomer [%] | CE-SDS (non red) | CE-SDS (red) |
|---|---|---|---|---|
| 12B3 P329GLALA IgG1 | 4 | 98 | 98.6% (173kDa) | 22.5% (29kDa) |
| | | | | 75.5% (64kDa) |
| 25G7 P329GLALA IgG1 | 25 | 100 | 99.7% (181.6kDa) | 76.8% (65kDa) |
| | | | | 23% (42kDa) |
| 11D5 P329GLALA IgG1 | 9.7 | 98.7 | 99.6% (176kDa) | tbd. |
| 9B11 P329GLALA IgG1 | 22 | 100 | 100% (153 kDa) | 2% (127 kDa) |
| | | | | 72.3 % (114 kDa) |
| | | | | 24.6 % (37.1 kDa) |
| 20G2 P329GLALA IgG1 | 11 | 100 | 98.5% (166kDa) | 80.2% (62.8kDa) |
| | | | | 18% (28.4kDa) |

### Example 7

### Characterization of anti-4-BB antibodies

### 7.1 Binding on human 4-1BB

### 7.1.1 Surface plasmon resonance (avidity + affinity)

Binding of phage-derived 4-1BB-specific antibodies to the recombinant 4-1BB Fc(kih) was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany).

In the same experiment, the species selectivity and the avidity of the interaction between the phage display derived anti-4-1BB clones 12B3, 25G7, 11D5, 9B11 and 20G2 (all human IgG1 P329GLALA), and recombinant 4-1BB (human, cyno and murine) was determined. Biotinylated human, cynomolgus and murine 4-1BB Fc(kih) were directly coupled to different flow cells of a streptavidin (SA) sensor chip. Immobilization levels up to 100 resonance units (RU) were used. Phage display derived anti-4-1BB human IgG1 P329GLALA antibodies were passed at a concentration range from 4 to 450 nM (3-fold dilution) with a flow of 30 µL/minute through the flow cells over 120 seconds. Complex dissociation was monitored for 220 seconds. Bulk refractive index differences were corrected for by subtracting the response obtained in a reference flow cell, where no protein was immobilized.

Kinetic constants were derived using the Biacore T200 Evaluation Software (vAA, Biacore AB, Uppsala/Sweden), to fit rate equations for 1:1 Langmuir binding by numerical integration and used to estimate qualitatively the avidity **(Table 50).**

In the same experiment, the affinities of the interaction between phage display derived antibodies (human IgG1 P329GLALA) to recombinant 4-1BB (human, cyno and murine) were determined. Anti-human Fab antibody (Biacore, Freiburg/Germany) was directly coupled on a CM5 chip at pH 5.0 using the standard amine coupling kit (Biacore, Freiburg/Germany). The immobilization level was approximately 7500 RU. Phage display derived antibodies to 4-1BB were captured for 60 seconds at concentrations ranging from 25 nM. Recombinant human 4-1BB Fc(kih) was passed at a concentration range from 4.1 to 1000 nM with a flow of 30 µL/minutes through the flow cells over 120 seconds. The dissociation was monitored for 120 seconds. Bulk refractive index differences were corrected for by subtracting the response obtained on reference flow cell. Here, the antigens were flown over a surface with immobilized anti-human Fab antibody but on which HBS-EP has been injected rather than the antibodies. Kinetic constants were derived using the Biacore T200 Evaluation Software (vAA, Biacore AB, Uppsala/Sweden), to fit rate equations for 1:1 Langmuir binding by numerical integration.

Clones 25G7 and 9B11 bind human 4-1BB Fc(kih) with a lower affinity than clones 12B3 and 11D5. Clone 20G2 is not binding to human 4-1BB. Affinity constants for the interaction between anti-4-1BB P329GLALA IgG1 and human 4-1BB Fc(kih) were determined by fitting to a 1:1 Langmuir binding.

**Table 50: Binding of anti-4-1BB antibodies to recombinant human 4-1BB**

| Clone | Origin | Recombinant human 4-1BB (avidity format) | Recombinant human 4-1BB (affinity format) | | |
|---|---|---|---|---|---|
| | | | ka (1/Ms) | kd (1/s) | KD (M) |
| 12B3 | Phage display | ++++ | 3.4E+04 | 1.0E-03 | 3.0E-08 |
| 25G7 | Phage display | + | 2.9E+04 | 9.9E-04 | 3.4E-08 |
| 11D5 | Phage display | +++ | 3 .2E+04 | 1.2E-03 | 3.6E-08 |
| 9B11 | Phage display | ++ | 2.7E+04 | 3.9E-03 | 1.4E-07 |

### 7.1.2 Binding to human 4-1BB expressing cells: resting and activated human peripheral mononuclear blood leukocytes (PBMC)

Expression of human 4-1BB is absent on resting and naive human T cells (Kienzle G. and von Kempis J (2000), Int. Immunol. 12(1):, 73-82, Wen T. et al. (2002), J. Immunol. 168, 4897-4906). After activation with immobilized anti-human CD3 agonistic antibody, 4-1BB is upregulated on CD4⁺ and CD8⁺ T cells. 4-1BB expression has also been reported on activated human NK cells (Baessler T. et. al. (2010) Blood 115(15), 3058-3069), activated human NKT cells (Cole S.L. et al. (2014) J. Immunol. 192(8), 3898-3907), activated human B cells (Zhang et al. (2010) J. Immunol. 184(2), 787-795), activated human eosinophils (Heinisch et al. 2001), constitutively on human neutrophils (Heinisch I.V. (2000) J Allergy Clin Immunol. 108(1), 21-28), activated human monocytes (Langstein J.et al. (1998) J Immunol. 160(5), 2488-2494, Kwajah M. and Schwarz H. (2010) Eur J Immunol. 40(7), 1938-1949), constitutively on human regulatory T cells (Bacher P. et al. (2014) Mucosal Immunol. 7(4), 916-928), human follicular dendritic cells (Pauly S. et al. (2002) J Leukoc Biol. 72(1), 35-42), activated human dendritic cells (Zhang L. et al. (2004) Cell Mol Immunol. 1(1), 71-76) and on blood vessels of malignant human tumors (Broll K. et al. (2001) Am J Clin Pathol. 115(4), 543-549).

To test binding of our anti-4-1BB clones to naturally cell-expressed human 4-1BB, fresh isolated resting peripheral blood mononuclear cells (PBMCs) or PHA-L/Proleukin pre-activated and CD3/CD28-reactivated PBMC were used. PBMCs from buffy coats obtained from the Zurich blood donation center were isolated by ficoll density centrifugation using Histopaque 1077 (SIGMA Life Science, Cat.-No. 10771, polysucrose and sodium diatrizoate, adjusted to a density of 1.077 g/mL) and resuspended in T cell medium consisting of RPMI 1640 medium (Gibco by Life Technology, Cat.-No. 42401-042) supplied with 10 % Fetal Bovine Serum (FBS, Gibco by Life Technology, Cat.-No. 16000-044, Lot 941273, gamma-irradiated, mycoplasmafree and heat inactivated at 56 °C for 35 min), 1 % (v/v) GlutaMAX-I (GIBCO by Life Technologies, Cat.-No. 35050 038), 1 mM Sodium Pyruvate (SIGMA, Cat.-No. S8636), 1 % (v/v) MEM non-essential amino acids (SIGMA, Cat.-No. M7145) and 50 µM β-Mercaptoethanol (SIGMA, M3148). PBMCs were used directly after isolation (resting cells) or stimulated to induce 4-1BB expression at the cell surface of T cells by culturing for 3 to 5 days in T cell medium supplemented with 200 U/mL Proleukin (Novartis Pharma Schweiz AG, CHCLB-P-476-700-10340) and 2 µg/mL PHA-L (SIGMA Cat.-No. L2769) in a 6-well tissue culture plate and then 2 day in a 6-well tissue culture plate coated with 10 µg/mL anti-human CD3 (clone OKT3, BioLegend, Cat.-No. 317315) and 2 µg/mL anti-human CD28 (clone CD28.2, BioLegend, Cat.-No.: 302928) in T cell medium at 37 °C and 5% CO₂.

To determine binding to human 4-1BB expressed by human PBMCs, 0.1-0.2 x 10⁶ freshly isolated or activated PBMCs were added to each well of a round-bottom suspension cell 96-well plates (Greiner bio-one, cellstar, Cat.-No. 650185). Plates were centrifuged 4 minutes with 400 x g at 4 °C and supernatant was discarded. Cells were washed with 200 µL/well DPBS and then incubated for 30 min at 4°C with 100 µL/mL DPBS containing 1:5000 diluted Fixable Viability Dye eFluor 450 (eBioscience, Cat.-No. 64-0863-18) or Fixable Viability Dye eFluor 660 (eBioscience, Cat.-No. 65-0864-18) . Afterwards cells were washed once with 200 µL/well cold FACS buffer (DPBS supplied with 2 % (v/v) FBS, 5 mM EDTA pH8 (Amresco, Cat. No. E177) and 7.5 mM sodium azide (Sigma-Aldrich S2002)). Next, 50 µL/well of 4 °C cold FACS buffer containing titrated anti-human 4-1BB binders were added and cells were incubated for 120 minutes at 4 °C. Cells were washed four times with 200 µL/well 4 °C FACS buffer to remove onbound molecules. Afterwards cells were further incubated with 50 µL/well of 4 °C cold FACS buffer containing 2.5 µg/mL PE-conjugated AffiniPure anti-human IgG Fcy-fragment-specific goat F(ab')2 fragment (Jackson ImmunoResearch, Cat.-No. 109-116-098) or 30 µg/mL FITC-conjugated AffiniPure anti-human IgG Fcy-fragment-specific goat F(ab')2 fragment (Jackson ImmunoResearch, Cat.-No. 109 096 098), anti-human CD45 AF488 (clone HI30, BioLegend, Cat.-No. 304019), 0.67 µg/mL APC/Cy7-conjugated anti-human CD3 moIgG1κ (clone UCH1, BioLegend, Cat.-No. 300426) or 0.125 µg/mL PE-conjugated anti-human CD3 mouse IgG1κ (clone SK7, BioLegend Cat.-No. 344806) or 0.67 µg/mL PerCP/Cy5.5-conjugated anti-human CD3 mouse IgG1 κ (clone UCHT1, BioLegend, Cat.-No. 300430), 0.125 µg/mL BV421-conjugated anti-human CD4 moIgG1κ (clone RPA-T4, BioLegend, Cat.-No. 300532) or 0.23 µg/mL BV421-conjugated anti-human CD4 mouse IgG2b κ (clone OKT4, BioLegend, Cat.-No. 317434) or 0.08 µg/mL PE/Cy7-conjugated anti-human CD4 mouse IgG1κ (clone SK3, BioLegend Cat.-No. 344612), 0.17 µg/mL APC/Cy7-conjugated anti-human CD8 (mouse IgG1κ, clone RPA-T8, BioLegend Cat.-No. 301016) or 0.125 µg/mL PE/Cy7-conjugated anti-human CD8a (moIgG1κ, clone RPA-T8, BioLegend, Cat.-No. 301012) or 0.33 µg/mL anti-human CD8 BV510 (moIgG1κ, clone SKI, BioLegend, Cat.-No. 344732) and 0.25 µg/mL APC-conjugated anti-human CD56 (mouse IgG1κ, clone HCD56, BioLegend, Cat.-No. 318310) or 1 µL AF488-conjugated anti-human CD56 (moIgG1κ, clone B159, BD Pharmingen, Cat.-No. 557699) and 0.67 µg/mL anti-human CD19-PE/Cy7 (moIgG1κ, clone HIB19, BioLegend, Cat.-No. 302216) and incubated for 30 minutes at 4 °C.

Cells were washed twice with 200 µL FACS buffer/well and fixated by resuspending in 50 µL/well DPBS containing 1 % Formaldehyde (Sigma, HT501320-9.5L). Cells were acquired the same or next day using a 3-laser Canto II (BD Bioscience with DIVA software) or a 5-laser Fortessa (BD Bioscience with DIVA software) or 3-laser MACSQuant Analyzer 10 (Miltenyi Biotech). Gates were set on CD8⁺ and CD4⁺ T cells and the median fluorescence intensity (MFI) or geo mean of fluorescence intensity of the secondary detection antibody was used to analyze binding of primary antibodies. Using Graph Pad Prism (Graph Pad Software Inc.) data was baselined by subtracting the blank values (no primary antibody added) and the EC50 values were calculated using non-linear regression curve fit (robust fit).

Human T cells lack 4-1BB expression in a resting status but upregulate 4-1BB after activation. Human CD8⁺ T cells show a stronger up-regulation than CD4⁺ T. The generated anti-human 4-1BB-specific antibodies can bind to human 4-1BB expressed by activated human T cells as shown in **Figures 34A-34D****.** The shown anti-human 4-1BB clones can be classified in strong binding (clones 12B3 and 11D5) and low binders (clones 25G7 and 9B11). Differences are not only seen by EC₅₀ value but also by MFI. The EC₅₀ values of binding to activated CD8⁺ T cells are shown in **Table 51.** The anti-mouse 4-1BB-specific clone 20G2 did not bind to human 4-1BB and is therefore not human-cross-reactive.

**Table 51: EC₅₀ values of binding to activated human CD8 T cells**

| Clone | EC₅₀ [nM] |
|---|---|
| 25G7 | 29 |
| 12B3 | 0.95 |
| 11D5 | 1.46 |
| 9B11 | 4.485 |
| 20G2 | n.d. |

### 7.2 Binding on murine 4-1BB

### 7.2.1 Surface plasmon resonance (avidity + affinity)

Binding of the phage-derived 4-1BB specific antibody 20G2 to recombinant murine 4-1BB Fc(kih) was assessed by surface plasmon resonance as described above for human 4-1BB Fc(kih) (see Example 7.1.1). Kinetic constants were derived using the Biacore T200 Evaluation Software (vAA, Biacore AB, Uppsala/Sweden), to fit rate equations for 1:1 Langmuir binding by numerical integration and used to estimate qualitatively the avidity **(Table 52).**

For affinity determination, due to an unspecific interaction of the Fc fusion protein to the reference flow cell, murine 4-1BB Fc(kih) was cleaved with AcTEV protease and the Fc portion removed by chromatographical method. Anti-human Fc antibody (Biacore, Freiburg/Germany) was directly coupled on a CM5 chip at pH 5.0 using the standard amine coupling kit (Biacore, Freiburg/Germany). The immobilization level was about 7500 RU. Phage display derived antibodies to 4-1BB were captured for 60 seconds at concentrations ranging from 25 nM. Recombinant murine 4-1BB AcTEV was passed at a concentration range from 4.1 to 1000 nM with a flow of 30 µL/minutes through the flow cells over 120 seconds. The dissociation was monitored for 120 seconds. Bulk refractive index differences were corrected for by subtracting the response obtained on reference flow cell. Here, the antigens were flown over a surface with immobilized anti-human Fc antibody but on which HBS-EP has been injected rather than the antibodies.

Kinetic constants were derived using the Biacore T200 Evaluation Software (vAA, Biacore AB, Uppsala/Sweden), to fit rate equations for 1:1 Langmuir binding by numerical integration. It was shown that clone 20G2 binds murine 4-1BB **(Table 52).**

Affinity constants of interaction between anti-4-1BB P329GLALA IgG1 molecules and murine 4-1BB were derived using the Biacore T200 Evaluation Software (vAA, Biacore AB, Uppsala/Sweden), to fit rate equations for 1:1 Langmuir binding by numerical integration.

**Table 52: Binding of anti-4-1BB antibody 20G2 to murine 4-1BB**

| Clone | Origin | Recombinant murine 4-1BB (avidity format) | Recombinant murine 4-1BB (affinity format) | | |
|---|---|---|---|---|---|
| | | | ka (1/Ms) | kd (1/s) | KD (M) |
| 20G2 | Phage display | | 2.4E+04 | 3.4E-04 | 1.4E-08 |

### 7.2.2 Binding to mouse 4-1BB expressing cells: resting and activated mouse splenocytes (selected clones)

Similar to human, freshly isolated resting mouse T cells do not express 4-1BB but expression can be induced by TCR activation via peptide-pulsed APCs (Cannons J.et al. (2001) J. Immunol. 167(3): 1313-1324) or immobilized anti-mouse CD3 or a combination of anti-mouse CD3 and anti-mouse CD28 antibodies (Pollok K. et al. (1995), European J. Immunol. 25(2), 488-494). After activation via surface immobilized anti-CD3 antibody 4-1BB expression is reported to be higher on CD8⁺ T cells (Shuford W, et al. (1997) J. of Experimental Med. 186(1), 47-55), but this may depend on activation protocol. Further 4-1BB expression has also been reported on activated mouse NK cells (Melero et al. (1998) Cell Immunol. 190(2), 167-172), activated mouse NKT cells (Vinay D, et al. (2004) J Immunol. 173(6), 4218-4229), activated mouse B cells (Vinay, Kwon (2011) Cell Mol Immunol. 8(4), 281-284), constitutively on mouse neutrophils (Lee S. et al. (2005) Infect Immun. 73(8), 5144-5151) and mouse regulatory T cells (Gavin et al. (2002) Nat Immunol. 3(1), 33-41), mouse IgE-stimulated mast cell (Nishimoto et al. (2005) Blood 106(13): 4241-4248), mouse myeloid-lineage cells (Lee et al. (2008) Nat Immunol. 9(8), 917-926), mouse follicular dendritic cells (Middendorp et al. (2009) Blood 114(11), 2280-2289) and activated mouse dendritic cells (Wilcox, Chapoval et al. (2002) J Immunol. 168(9),4262-4267).

Anti-4-1BB specific antibody binding was tested directly after isolation of splenocytes from healthy female C57BL/6 mice as well as after *in vitro* activation for 72 hours with surface immobilized agonistic anti-mouse CD3 and anti-mouse CD28 antibodies. Female C57BL/6 mice (age 7-9 weeks) were purchased at Charles River, France. After arrival animals were maintained for one week to get accustomed to new environment and for observation. Mice were maintained under specific-pathogen-free condition with food and water *ad libitum* and daily cycles of 12 h light /12 h darkness according to committed guidelines (GV-Solas; Felasa; TierschG). Continuous health monitoring was carried out on a regular basis. Mice were sacrificed by cervical dislocation. Spleens were dissected and stored on ice in RPMI 1640 supplemented with 10 % (v/v) heat-inactivated FBS and 1% (v/v) GlutaMAX-I. To obtain a single cell solution spleens were homogenized through a 70 µm cell strainer (BD Falcon; Germany) and subjected to erythrolysis for 10 minutes at 37°C in ACK lysis buffer (0.15M NH4CL, 10 mM KHCO3, 0.1mM EDTA in ddH₂O, pH 7.2). After two washing steps with sterile DPBS splenocytes were reconstituted in T cell medium. Either cells were used freshly (resting) or 10⁶ cells/mL splenocytes were further stimulated for 72 hours in T cell medium consisting of RPMI 1640 medium supplied with 10 % FBS, 1 % (v/v) GlutaMAX-I, 1 mM Sodium Pyruvate, 1 % (v/v) MEM non-essential amino acids and 50 µM β-Mercaptoethanol on 6-well cell culture plates coated with 1 µg/mL anti-mouse CD3 antibody (rat IgG2b, clone 17A2, BioLegend, Cat.-No. 100223) and 2 µg/mL anti-mouse CD28 antibody (syrian hamster, clone 37.51, BioLegend Cat.-No. 102112).

To test binding to mouse 4-1BB, freshly isolated or activated mouse splenocytes were resuspended in DPBS and 0.1 x 10⁶/well splenocytes were transferred to a round-bottom 96-suspension cell plate (Greiner bio-one, cellstar, Cat.-No. 650185). Cells were centrifuged 4 minutes at 4°C and 400 x g and supernatant was removed. After resuspension in 100 µ/well DPBS containing 1:5000 diluted Fixable Viability Dye eFluor 450 (eBioscience, Cat.-No. 65-0863-18) or Fixable Viability Dye eFluor 660 (eBioscience, Cat.-No. 65-0864-18) cells were incubated for 30 min at 4°C. Cells were washed with FACS buffer and 50 µL/well FACS buffer containing titrated concentrations of anti-human 4-1BB huIgG1 P329G LALA antibody-clones 12B3, 25G7, 11D5, 9B11 and anti-mouse 4-1BB-specific clone 20G2 as huIgG1 P329G LALA or mouse IgG1 or mouse IgG1 DAPG. After 1 h incubation at 4°C cells were washed four times to remove excessive antibodies. If binding of anti-mouse 20G2 as mouse IgG1 and mouse IgG1 DAPG format was tested, cells were incubated in 50 µL FACS buffer/well containing 30 µg/mL FITC-conjugated anti-mouse IgG Fcy-fragment-specific AffiniPure goat F(ab')γ fragment for 30 min at 4°C and washed twice with FACS-buffer. If binding of anti-4-1BB binders containing a human IgG1 P329G LALA Fc-fragment were tested this step was skipped. Afterwards cells were incubated in 50 µL FACS buffer/well containing 0.67 µg/mL PE-conjugated anti-mouse CD3 (rat IgG2bκ, clone 17A2, BD Pharmingen, Cat.-No. 555275) or APC-Cy7-conjugated anti-mouse CD3 (rat IgG2aκ, clone 53-6.7, BioLegend, Cat.-No. 100708), 0.67 µg/mL PE/Cy7-conjugated anti-mouse CD4 (rat IgG2bκ, clone GK1.5, BioLegend, Cat.-No. 100422), 0.67 µg/mL APC/Cy7-conjugated anti-mouse CD8 (rat IgG2aκ, clone 53-6.7, BioLegend, Cat.-No. 1007141) or PE-conjugated anti-mouse CD8 (rat IgG2aκ, clone 53-6.7, BioLegend, Cat.-No. 100708), 2 µg/mL APC-conjugated anti-mouse NK1.1 (mouse IgG2a, κ, clone PK136, BioLegend, Cat.-No. 108710) or PerCP/Cy5.5-conjugated anti-mouse NK1.1 (mouse IgG2a, κ, clone PK136, BioLegend, Cat.-No. 108728) and 10 µg/mL anti-mouse CD16/CD32 (mouse Fc-Block, rat IgG2b κ, clone 2.4G2, BD Bioscience, Cat.-No. 553142). If binding of anti-4-1BB binders containing a human IgG1 P329G LALA Fc-fragment were tested 30 µg/mL FITC-conjugated AffiniPure anti-human IgG Fcy-fragment-specific goat F(ab')2 fragment (Jackson ImmunoResearch, Cat. No. 109 096 098) were also added. Cells were incubated for 30 min at 4°C, washed twice with 200 µL/well FACS-buffer and resuspended for fixation with 50 µL/well DPBS containing 1% (v/v) formaldehyde. The next day cells were resuspended in 200 µL/well FACS buffer and acquired using the 2-laser CantoII (BD Bioscience with DIVA software) or 5-laser Fortessa (BD Bioscience with DIVA software). Gates were set on CD8⁺ and CD4⁺ T cells and the median fluorescence intensity (MFI) of the secondary detection antibody was used to analyze binding of primary antibodies. Using Graph Pad Prism (Graph Pad Software Inc.) data was baselined by subtracting the blank values (no primary antibody added) and the EC₅₀ values were calculated using non-linear regression curve fit (robust fit).

As shown in **Figure 35B and 35D****,** only the anti-mouse 4-1BB binding clone 20G2 bound to activated mouse CD8⁺ and CD4⁺ T cells, whereas the anti-human-4-1BB binding clones 9B11, 11D5, 12B3 and 25G7 did not bind to mouse-4-1BB and are therefore not mouse-cross-reactive. Only clone 20G2 from the tested clones can be used as a mouse surrogate. As expected none of the anti-4-1BB binding clones showed binding to freshly isolated resting mouse T cells **(****Figure 35A and 35C****).** Similar to the activated human CD4⁺ T cells also the activated mouse CD4⁺ T cells express less 4-1BB than the activated mouse CD8⁺ T cells. The difference however is not as strong as for human T cells, this may also be related to the different activation protocol. EC₅₀ values are shown in **Table 53.**

**Table 53: EC₅₀ values of binding to activated murine CD8 and CD4 T cells**

| Clone | EC₅₀ CD8 [nM] | EC₅₀ CD4 [nM] |
|---|---|---|
| 25G7 | n.d. | n.d. |
| 12B3 | n.d. | n.d. |
| 11D5 | n.d | n.d |
| 9B11 | n.d | n.d |
| 20G2 | 16.36 | 10.10 |

As shown in **Figures 36B and 36D****,** the anti-mouse-4-1BB binding clone 20G2 binds to activated mouse CD8⁺ and CD4⁺ T cells as moIgG1 wildtype (wt) or moIgG1 DAPG format in a similar way. The binding is similar to the binding shown in **Figures 35B and 35D****;** therefore changing of format does not influence the binding properties. We transferred the clone 20G2 to moIgG to prevent triggering of anti-drug-antibodies (ADAs) in immune competent mice. The DAPG mutation is equivalent to the P329G LALA mutation in the human IgG1 constructs, e.g. it prevents crosslinking via the FcR⁺ immune cells. EC50 values are shown in **Table 54.**

**Table 54: EC₅₀ values of binding to activated murine CD8 and CD4 T cells**

| Clone | EC₅₀ CD8 [nM] | EC₅₀ CD4 [nM] |
|---|---|---|
| 20G2 mu IgG1 κ DAPG | 17.91 | 11.22 |
| 20G2 mu IgG1 κ wt | 18.51 | 9.917 |

### 7.3 Binding on cynomolgus 4-1BB

### 7.3.1 Surface plasmon resonance (avidity + affinity)

Binding of phage-derived 4-1BB specific antibodies 12B3, 25G7, 11D5 and 9B11 to the recombinant cynomolgus 4-1BB Fc(kih) was assessed by surface plasmon resonance (SPR) as described above for human 4-1BB Fc(kih) (see Example 7.1.1). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany). Kinetic constants were derived using the Biacore T200 Evaluation Software (vAA, Biacore AB, Uppsala/Sweden), to fit rate equations for 1:1 Langmuir binding by numerical integration and used to estimate qualitatively the avidity **(Table 55).**

In the same experiment, the affinities of the interaction between phage display derived antibodies (human IgG1 P329GLALA) to recombinant cynomolgus 4-1BB Fc(kih) were determined. Anti-human Fab antibody (Biacore, Freiburg/Germany) was directly coupled on a CM5 chip at pH 5.0 using the standard amine coupling kit (Biacore, Freiburg/Germany). The immobilization level was approximately 9000 RU. Phage display derived antibodies to 4-1BB were captured using concentrations of 25 to 100 nM. The experiment was performed as described for human 4-1BB Fc(kih (Example 7.1.1).

Clones 12B3, 25G7, 11D5 and 9B11 bound cynomolgus 4-1BB Fc(kih) with similar affinities **(Table 55),** but 12B3 and 11D5 bound with higher avidity to cells expressing cynomolgus 4-1BB. Affinity constants of interaction between anti-4-1BB P329GLALA IgG1 and cynomolgus 4-1BB Fc(kih) were derived using the Biacore T100 Evaluation Software (vAA, Biacore AB, Uppsala/Sweden), to fit rate equations for 1:1 Langmuir binding by numerical integration.

**Table 55: Binding of anti-4-1BB antibodies to recombinant cynomolgus 4-1BB Fc(kih)**

| Clone | Origin | Recombinant cynomolgus 4-1BB (avidity format) | Recombinant cynomolgus 4-1BB (affinity format) | | |
|---|---|---|---|---|---|
| | | | ka (1/Ms) | kd (1/s) | KD (M) |
| 12B3 | Phage display | +++ | 3.8E+04 | 7.8E-04 | 2.0E-08 |
| 25G7 | Phage display | ++ | 2.7E+04 | 4.6E-04 | 1.7E-08 |
| 11D5 | Phage display | +++ | 3.1E+04 | 7.7E-04 | 2.4E-08 |
| 9B11 | Phage display | + | 2.6E+04 | 2.0E-03 | 7.8E-08 |

### 7.3.2 Binding on cynomolgus 4-1BB expressing cells: activated cynomolgus peripheral mononuclear blood leukocytes (PBMC)

To test the cross-reactivity of the anti-human 4-1BB binding clones to cynomolgus cells, PBMCs of healthy *cynomolgus fascicularis* were isolated from heparinized blood using density gradient centrifugation as described for human PBMCs (**7.1.2**) with minor differences. Isolated PBMC were cultured for 72 hours at a cell density of 1.5^{∗}10⁶ cells/mL in in T cell medium consisting of RPMI 1640 medium supplied with 10 % FBS, 1 % (v/v) GlutaMAX-I, 1 mM Sodium Pyruvate, 1 % (v/v) MEM non-essential amino acids and 50 µM β-Mercaptoethanol on 6-well cell culture plates (Greiner Bio-One, Germany) coated with 10 µg/mL anti-cyno-cross-reactive CD3 (mo IgG3 λ, anti-human CD3, clone SP34, BD Pharmingen, Cat.-No. 556610) and 2 µg/mL anti-cyno-cross-reactive CD28 (moIgG1κ, anti-human CD28, clone CD28.2, BioLegend, Cat.-No. 140786) antibodies. After 72 h stimulation cells were harvested and seeded to a round-bottom suspension cell 96-well plate (Greiner bio-one, cellstar, Cat.-No. 650185) at a concentration of 0. 1 × 10⁶ cells/well. Cells were incubated in 50 µL/well FACS buffer containing different concentrations of the primary anti-human 4-1BB-specific huIgG P32G LALA antibodies for 2 h at 4 °C. Afterwards cells were washed four times with 200 µL/well FACS buffer and incubated further for 30 min at 4°C with 50 µL/well FACS buffer containing 2 µL PE-conjugated anti-cyno-crossreactive CD4 (moIgG2aκ, anti-human CD4, clone M-T477, BD Pharmingen, Cat.-No. 556616), 1 µg/mL PerCP/Cy5.5-conjugated anti-cyno-cross-reactive CD8 (moIgG1κ, anti-human CD8, clone RPA-T8, BioLegend, Cat.-No. 301032) and 30 µg/mL FITC-conjugated AffiniPure anti-human IgG Fcy-fragment-specific goat F(ab')2 fragment (Jackson ImmunoResearch, Cat. No. 109 096 098). Cells were washed twice with FACS buffer and resuspended in 100 µL/well FACS buffer supplied with 0.2 µg/mL DAPI to discriminated dead from living cells. Cells were immediately acquired using a 5-laser Fortessa (BD Bioscience with DIVA software). Gates were set on CD8⁺ and CD4⁺ T cells and the median fluorescence intensity (MFI) of the secondary detection antibody was used to analyze binding of primary antibodies. Using Graph Pad Prism (Graph Pad Software Inc.) data was baselined by subtracting the blank values (no primary antibody added) and the EC₅₀ values were calculated using non-linear regression curve fit (robust fit).

As shown in **Figures 37A and 37B****,** at least three of the anti-human 4-1BB clones, namely 12B3, 11D5 and 25G7 are also cross-reactive for cynomolgus 4-1BB expressed on activated CD4⁺ and CD8⁺ T cells. Interestingly the binding curves look similar to human 4-1BB, e.g. 12B3 and 11D5 show the highest MFI and lowest EC₅₀ values of all tested constructs, whereas 25G7 is a weaker binder with lower MFI and higher EC₅₀ value (**Table 56**). The clone 9B11 is only binding at the highest concentration of 100 nM. This is contrary to binding to human 4-1BB where clone 9B11 is superior compared to clone 25G7. Further differences of 4-1BB expression levels on activated cynomolgus CD4⁺ and CD8⁺ T cells are similar to activated human T cells e.g. CD4⁺ T cells express much less 4-1BB than CD8⁺ T cells.

**Table 56: EC₅₀ values of binding to activated cynomolgus CD8 and CD4 T cells**

| Clone | EC₅₀ CD8 [nM] | EC₅₀ CD4 [nM] |
|---|---|---|
| 25G7 | 4.52 | 6.68 |
| 12B3 | 0.47 | 0.59 |
| 11D5 | 1.04 | 0.97 |
| 9B11 | n.d. | n.d. |

### 7.4 Ligand blocking property

To determine the capacity of 4-1BB-specific human IgG1 P329GLALA antibody molecules to interfere with 4-1BB/4-1BB-ligand interactions human 4-1BB ligand (R&D systems) was used. Similarly, murine 4-1BB ligand (R&D systems) was used to assess the ligand blocking property of the anti-murine 4-1BB specific IgG1 P329GLALA antibody 20G2.

Human or murine 4-1BB ligand was directly coupled to two flow cells of a CM5 chip at approximately 1500 RU by pH 5.0 using the standard amine coupling kit (Biacore, Freiburg/Germany). Recombinant human, or murine, 4-1BB Fc(kih) was passed on the second flow cell at a concentration of 500 nM with a flow of 30 µL/minute over 90 seconds. The dissociation was omitted and the phage derived anti-4-1BB human IgG1 P329GLALA was passed on both flow cells at a concentration of 200 nM with a flow of 30 uL/ minute over 90 seconds. The dissociation was monitored for 60 seconds. Bulk refractive index differences were corrected for by subtracting the response obtained on reference flow cell. Here, the antibodies were flown over a surface with immobilized human, or murine, 4-1BB ligand but on which HBS-EP has been injected instead of recombinant human 4-1BB Fc(kih).

The phage-derived clone 25G7 bound to the complex of human 4-1BB with its 4-1BB ligand **(Table 57,** **Figure 38A****).** Thus, this antibody does not compete with the ligand for binding to human 4-1BB and is therefore termed "non-ligand blocking". On the contrary, clones 12B3, 11D5 and 9B11 did not bind to human 4-1BB associated with its ligand and are therefore termed "ligand blocking". The murine surrogate 20G2 did not bind to murine 4-1BB associated with its ligand and is also termed "ligand blocking"

**Table 57: Ligand binding property of the anti-4-1BB clones determined by surface plasmon resonance**

| Clone | Origin | First injection | Second injection (anti-4-1BB clone) | Ligand blocking |
|---|---|---|---|---|
| 12B3 | Phage display | human 4-1BB Fc(kih) | Not binding | YES |
| 25G7 | Phage display | human 4-1BB Fc(kih) | Binding | NO |
| 11D5 | Phage display | human 4-1BB Fc(kih) | Not binding | YES |
| 9B11 | Phage display | human 4-1BB Fc(kih) | Not binding | YES |
| 20G2 | Phage display | murine 4-1BB Fc(kih) | Not binding | YES |

### Example 8

### Functional properties of anti 4-1BB binding clones

4-1BB serves as a co-stimulatory receptor and improves expansion, cytokine production and functional properties of T cells in a TCR-dependent manner. TCR-activation via surface immobilized anti-CD3 antibody or peptide-pulsed antigen presenting cells induces up-regulation of 4-1BB on T cells (Pollok et al. (1993) J. Immunol. 150(3): 771-781) and support after engagement the immune response by boosting expansion and cytokine release (Hurtado et al. (1995) J Immunology 155(7), 3360-3367). To test boosting capacity of the generated anti-human 4-1BB antibodies, round-bottom suspension 96-well plates (Greiner bio-one, cellstar, Cat.-No. 650185) were coated over night with DPBS containing 2 µg/mL AffiniPure F(ab')2 fragment goat anti-human IgG, Fcy-fragment-specific (Jackson Immunoresearch, Cat.-No. 109-006-008) and 2 µg/mL AffiniPure goat anti-mouse IgG, Fcy-fragment-specific (Jackson Immunoresearch, Cat.-No 115-005-008). Plates were washed with DPBS to remove excessive molecules and were blocked for 90 min at 37 °C with DPBS containing 1 % (w/v) BSA (SIGMA-Aldrich, Cat-No. A3059-100G). Supernatant was removed and plates were incubated with DPBS supplied with 1% (w/v) BSA and with or without 10 ng/mL anti-human CD3 antibody (BioLegend, Cat.-No. 317315, clone OKT3) for 90 min at 37 °C. Plates were washed with DPBS and incubated with DPBS supplied with 1% (w/v) BSA and different concentrations of titrated anti-human 4-1BB IgG1 P329 G LALA antibodies. Plates were washed with DPBS and supernatant was aspirated.

Human PBMCs were isolated as described before (**7.1.2**) and activated in RPMI 1640 containing 10 % (v/v) FBS, 1% (v/v) GlutaMAX-I, 2 ug/mL PHA-L and 200 U/mL Proleukin for 5 days. Cells were further cultured in RPMI 1640 containing 10 % (v/v) FBS, 1% (v/v) GlutaMAX-I and 200 U/mL Proleukin for further 21 days at a density of 1-2 x 10⁶ cells/mL. Long time cultured PBMCs were harvested washed and CD8 T cells were isolated according to manufactures protocol using human CD8⁺ T cell isolation kit (Miltenyi Biotec, Cat.-No. 130-096-495). Preactivated and sorted CD8⁺ T cells were seeded to 7 x 10⁴ cells/well in 200 µL/well T cell medium consisting of RPMI 1640 medium supplied with 10 % FBS, 1 % (v/v) GlutaMAX-I, 1 mM Sodium Pyruvate, 1 % (v/v) MEM non-essential amino acids and 50 µM β-Mercaptoethanol. Cells were incubated for 72 h , the last 4 h in the presence of Golgi-Stop (BD Bioscience, Cat.-No. 554724). Cells were washed with DPBS and incubated for 30 min at 4 °C in 100 µL/well DPBS containing 1:5000 diluted LIVE/DEAD Fixable Green Dead Cell Stain (Molecular Probes, Life Technologies, Cat.-No. L-23101). Afterwards cells were washed and incubated for 30 min at 4 °C in 50 µL/well FACS buffer containing 0.5 µg/mL PerCP/Cy5.5-conjugated anti-human CD8 (mouse IgG1 κ, clone RPA-T8, BioLegend, Cat.-No. 301032), 0.5 µg/mL PE/Cy7-conjugated anti-human CD25 (mouse IgG1 κ, clone BC96, BioLegend, Cat.-No. 302612) and 1 µg/mL APC/Cy7-conjugated anti-human PD-1 (mouse IgG1 κ, clone EH12.2H7, BioLegend, Cat.-No. 329922). Cells were washed with FACS buffer and resuspended in 50 µL/well in freshly prepared fixation/permeabilization solution (eBioscience, Cat.-No. 00-5523-00). After incubation for 30 min at 4°C, cells were washed with freshly prepared permeabilization buffer (eBioscience, Cat.-No. 00-5523-00) and incubated for 1 h at 4 °C with 50 µL/well Perm-buffer containing 2 µg/mL APC-labeled anti-human-IFNy (mo IgG1 κ, clone B27, BD Pharmingen, Cat.-No. 554702) and 2 µg/mL PE-conjugated anti-human-TNFα (mo IgG1 κ, clone MAb11, BD Pharmingen, Cat.-No. 554513). Cells were washed and fixed with DPBS containing 1% formaldehyde. Cells were acquired the next day using 2-laser Canto II (BD, DIVA software). Gates were set on CD8⁺ T cells and frequency of TNFα and IFNγ secreting CD8⁺ T cells were determined. Using Graph Pad Prism (Graph Pad Software Inc.) data was blotted and curves were calculated using non-linear regression curve fit (robust fit).

As described before in the absence of TCR- or CD3-stimulation 4-1BB engagement has no effect on CD8⁺ T cell function (Pollok 1995), whereas in the presence of suboptimal CD3-activation co-stimulation of 4-1BB increase cytokine secretion **(****Figures 39A or 39C****).** Suboptimal activation via CD3-antibody induces IFNγ-secretion in 30% of CD8⁺ T cells in the total CD8⁺ T cell population. Addition of 4-1BB-co-stimulation increases the IFNγ+ CD8 T cell population up to 55 % in a concentration dependent manner **(****Figure 39B****). Table 58** shows the corresponding EC₅₀ values. TNFα secretion could be increased from 23 % to 39 % of total CD8+ T cell population **(****Figure 39D****).** Similar to their binding properties clones 12B3 and 11D5 increase INFγ expression superior to 25G7 and 9B11 in frequency and EC₅₀. Therefore our generated clones are functional and can improve TCR-mediated T cell activation and function. If the anti-4-1BB-specific antibodies were not surface immobilized, they did not improve CD8⁺ T cell activation (not shown).

**Table 58: EC₅₀ values of increase of IFNγ secretion in activated CD8⁺ T cells**

| Clone | EC₅₀ CD8 [nM] |
|---|---|
| 25G7 | 0.12 |
| 12B3 | 0.07 |
| 11D5 | 0.06 |
| 9B11 | 0.12 |

### Example 9

### Preparation, purification and characterization of bispecific bivalent antibodies targeting 4-1BB and a tumor associated antigen (TAA)

### 9.1 Generation of bispecific bivalent antibodies targeting 4-1BB and fibroblast activation protein (FAP) (2+2 format, comparative examples)

Bispecific agonistic 4-1BB antibodies with bivalent binding for 4-1BB and for FAP were prepared. The crossmab technology was applied to reduce the formation of wrongly paired light chains as described in International patent application No. WO 2010/145792 A1.

The generation and preparation of the FAP binders is described in WO 2012/020006 A2.

In this example, a crossed Fab unit (VHCL) of the FAP binder 28H1 was C-terminally fused to the heavy chain of an anti-4-1BB hu IgG1 using a (G4S)₄ connector sequence. This heavy chain fusion was co-expressed with the light chain of the anti-4-1BB and the corresponding FAP crossed light chain (VLCH1). The Pro329Gly, Leu234Ala and Leu235Ala mutations have been introduced in the constant region of the heavy chains to abrogate binding to Fc gamma receptors according to the method described in International Patent Appl. Publ. No. WO 2012/130831 A1. The resulting bispecific, bivalent construct is analogous to the one depicted in **Figure 40A****.**

**Table 59** shows, respectively, the nucleotide and amino acid sequences of mature bispecific, bivalent anti-4-1BB/anti-FAP human IgG1 P329GLALA antibodies.

**Table 59: Sequences of bispecific, bivalent anti-4-1BB /anti-FAP human IgG1 P329GLALA antigen binding molecules**

| SEQ ID NO: | Description | Sequence |
|---|---|---|
| 307 | (12B3) VHCH1-Heavy chain-(28H1) VHCL (nucleotide sequence) | CAGGTGCAATTGGTGCAGTCTGGGGCTGAGGTGAAGAAG CCTGGGTCCTCGGTGAAGGTCTCCTGCAAGGCCTCCGGAG GCACATTCAGCAGCTACGCTATAAGCTGGGTGCGACAGG CCCCTGGACAAGGGCTCGAGTGGATGGGAGGGATCATCC CTATCTTTGGTACAGCAAACTACGCACAGAAGTTCCAGGG CAGGGTCACCATTACTGCAGACAAATCCACGAGCACAGC CTACATGGAGCTGAGCAGCCTGAGATCTGAGGACACCGC CGTGTATTACTGTGCGAGATCTGAATTCCGTTTCTACGCT GACTTCGACTACTGGGGCCAAGGGACCACCGTGACCGTC TCCTCAGCTAGCACCAAGGGCCCATCCGTGTTCCCTCTGG CCCCTTCCAGCAAGTCTACCTCTGGCGGCACAGCCGCTCT GGGCTGCCTCGTGAAGGACTACTTCCCCGAGCCTGTGACA GTGTCCTGGAACTCTGGCGCCCTGACATCCGGCGTGCACA CCTTTCCAGCTGTGCTGCAGTCCTCCGGCCTGTACTCCCTG TCCTCCGTCGTGACAGTGCCCTCCAGCTCTCTGGGCACCC AGACCTACATCTGCAACGTGAACCACAAGCCCTCCAACA CCAAGGTGGACAAGAAGGTGGAACCCAAGTCCTGCGACA AGACCCACACCTGTCCCCCTTGTCCTGCCCCTGAAGCTGC TGGCGGCCCTAGCGTGTTCCTGTTCCCCCCAAAGCCCAAG GACACCCTGATGATCTCCCGGACCCCCGAAGTGACCTGCG TGGTGGTGGATGTGTCCCACGAGGACCCTGAAGTGAAGT TCAATTGGTACGTGGACGGCGTGGAAGTGCACAATGCCA AGACCAAGCCTAGAGAGGAACAGTACAACTCCACCTACC GGGTGGTGTCCGTGCTGACCGTGCTGCACCAGGATTGGCT GAACGGCAAAGAGTACAAGTGCAAGGTGTCCAACAAGGC CCTGGGAGCCCCCATCGAAAAGACCATCTCCAAGGCCAA GGGCCAGCCTCGCGAGCCTCAGGTGTACACCCTGCCCCCT AGCAGAGATGAGCTGACCAAGAACCAGGTGTCCCTGACC TGTCTCGTGAAAGGCTTCTACCCCTCCGATATCGCCGTGG AATGGGAGAGCAACGGCCAGCCCGAGAACAACTACAAG ACCACCCCCCCTGTGCTGGACTCCGACGGCTCATTCTTCC TGTACTCTAAGCTGACAGTGGACAAGTCCCGGTGGCAGC AGGGCAACGTGTTCTCCTGCTCCGTGATGCACGAGGCCCT |
| | | GCACAACCACTACACCCAGAAGTCCCTGTCCCTGTCTCCC GGGGGAGGCGGAGGATCTGGCGGAGGCGGATCCGGTGGT GGCGGATCTGGGGGCGGTGGATCTGAGGTGCAGCTGCTG GAATCTGGGGGAGGACTGGTGCAGCCAGGCGGATCTCTG AGGCTGTCCTGCGCTGCTTCCGGCTTTACCTTCTCCAGCC ACGCCATGAGTTGGGTGCGCCAGGCACCCGGAAAAGGAC TGGAATGGGTGTCAGCCATCTGGGCCTCCGGCGAGCAGT ACTACGCCGATAGCGTGAAGGGCCGGTTCACCATCTCTCG GGATAACAGCAAGAATACTCTGTACCTGCAGATGAACTC CCTGCGCGCTGAAGATACCGCTGTGTATTACTGCGCCAAG GGCTGGCTGGGCAACTTCGATTACTGGGGCCAGGGAACC CTCGTGACTGTCTCGAGCGCTTCTGTGGCCGCTCCCTCCG TGTTCATCTTCCCACCTTCCGACGAGCAGCTGAAGTCCGG CACTGCCTCTGTCGTGTGCCTGCTGAACAACTTCTACCCT CGGGAAGCCAAGGTGCAGTGGAAAGTGGATAACGCCCTG CAGTCCGGCAACTCCCAGGAATCCGTGACCGAGCAGGAC TCCAAGGACAGCACCTACTCCCTGAGCAGCACCCTGACCC TGTCCAAGGCCGACTACGAGAAGCACAAGGTGTACGCCT GTGAAGTGACCCACCAGGGCCTGTCCAGCCCCGTGACCA AGTCCTTCAACCGGGGCGAGTGC |
| 287 | VLCL- Light chain 1 (12B3) (nucleotide sequence) | see Table 48 |
| 184 | VLCH1-Light chain 2 (28H1) (nucleotide sequence) | GAGATCGTGCTGACCCAGTCTCCCGGCACCCTGAGCCTGA GCCCTGGCGAGAGAGCCACCCTGAGCTGCAGAGCCAGCC AGAGCGTGAGCCGGAGCTACCTGGCCTGGTATCAGCAGA AGCCCGGCCAGGCCCCCAGACTGCTGATCATCGGCGCCA GCACCCGGGCCACCGGCATCCCCGATAGATTCAGCGGCA GCGGCTCCGGCACCGACTTCACCCTGACCATCAGCCGGCT GGAACCCGAGGACTTCGCCGTGTACTACTGCCAGCAGGG CCAGGTGATCCCCCCCACCTTCGGCCAGGGCACCAAGGT GGAAATCAAGAGCTCCGCTAGCACCAAGGGCCCCTCCGT GTTTCCTCTGGCCCCCAGCAGCAAGAGCACCTCTGGCGGA ACAGCCGCCCTGGGCTGCCTGGTGAAAGACTACTTCCCCG AGCCCGTGACCGTGTCCTGGAACTCTGGCGCCCTGACCAG CGGCGTGCACACCTTTCCAGCCGTGCTGCAGAGCAGCGG CCTGTACTCCCTGAGCAGCGTGGTGACAGTGCCCTCCAGC AGCCTGGGCACCCAGACCTACATCTGCAACGTGAACCAC AAGCCCAGCAACACCAAAGTGGACAAGAAGGTGGAACCC AAGAGCTGCGAC |
| 308 | (12B3) VHCH1-Heavy chain-(28H1) VHCL | QVQLVQSGAEVKKPGSSVKVSCKASGGTFSSYAISWVRQAP GQGLEWMGGIIPIFGTANYAQKFQGRVTITADKSTSTAYME LSSLRSEDTAVYYCARSEFRFYADFDYWGQGTTVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKKVEPKSCDKTHTCPPCPAPEAAGGPSVFLFPP KPKDTLMISRTPEVTCVVVDVSHEDPEVKFNWYVDGVEVH NAKTKPREEQYNSTYRVVSVLTVLHQDWLNGKEYKCKVSN KALGAPIEKTISKAKGQPREPQVYTLPPSRDELTKNQVSLTC LVKGFYPSDIAVEWESNGQPENNYKTTPPVLDSDGSFFLYSK LTVDKSRWQQGNVFSCSVMHEALHNHYTQKSLSLSPGGGG GSGGGGSGGGGSGGGGSEVQLLESGGGLVQPGGSLRLSCA ASGFTFSSHAMSWVRQAPGKGLEWVSAIWASGEQYYADSV |
| | | KGRFTISRDNSKNTLYLQMNSLRAEDTAVYYCAKGWLGNF DYWGQGTLVTVSSASVAAPSVFIFPPSDEQLKSGTASVVCLL NNFYPREAKVQWKVDNALQSGNSQESVTEQDSKDSTYSLSS TLTLSKADYEKHKVYACEVTHQGLSSPVTKSFNRGEC |
| 289 | VLCL- Light chain 1 (12B3) | see Table 48 |
| 186 | VLCH1-Light chain 2 (28H1) | EIVLTQSPGTLSLSPGERATLSCRASQSVSRSYLAWYQQKPG QAPRLLIIGASTRATGIPDRFSGSGSGTDFTLTISRLEPEDFAV YYCQQGQVIPPTFGQGTKVEIKSSASTKGPSVFPLAPSSKSTS GGTAALGCLVKDYFPEPVTVSWNSGALTSGVHTFPAVLQSS GLYSLSSVVTVPSSSLGTQTYICNVNHKPSNTKVDKKVEPKS CD |
| 309 | (25G7) VHCH1-Heavy chain-(28H1) VHCL (nucleotide sequence) | GAGGTGCAATTGTTGGAGTCTGGGGGAGGCTTGGTACAG CCTGGGGGGTCCCTGAGACTCTCCTGTGCAGCCTCCGGAT TCACCTTTAGCAGTTATGCCATGAGCTGGGTCCGCCAGGC TCCAGGGAAGGGGCTGGAGTGGGTCTCAGCTATTAGTGG TAGTGGTGGTAGCACATACTACGCAGACTCCGTGAAGGG CCGGTTCACCATCTCCAGAGACAATTCCAAGAACACGCTG TATCTGCAGATGAACAGCCTGAGAGCCGAGGACACGGCC GTATATTACTGTGCGCGTGACGACCCGTGGCCGCCGTTCG ACTACTGGGGCCAAGGAACCCTGGTCACCGTCTCGAGTG CTAGCACCAAGGGCCCATCCGTGTTCCCTCTGGCCCCTTC CAGCAAGTCTACCTCTGGCGGCACAGCCGCTCTGGGCTGC CTCGTGAAGGACTACTTCCCCGAGCCTGTGACAGTGTCCT GGAACTCTGGCGCCCTGACATCCGGCGTGCACACCTTTCC AGCTGTGCTGCAGTCCTCCGGCCTGTACTCCCTGTCCTCC GTCGTGACAGTGCCCTCCAGCTCTCTGGGCACCCAGACCT ACATCTGCAACGTGAACCACAAGCCCTCCAACACCAAGG TGGACAAGAAGGTGGAACCCAAGTCCTGCGACAAGACCC ACACCTGTCCCCCTTGTCCTGCCCCTGAAGCTGCTGGCGG CCCTAGCGTGTTCCTGTTCCCCCCAAAGCCCAAGGACACC CTGATGATCTCCCGGACCCCCGAAGTGACCTGCGTGGTGG TGGATGTGTCCCACGAGGACCCTGAAGTGAAGTTCAATTG GTACGTGGACGGCGTGGAAGTGCACAATGCCAAGACCAA GCCTAGAGAGGAACAGTACAACTCCACCTACCGGGTGGT GTCCGTGCTGACCGTGCTGCACCAGGATTGGCTGAACGGC AAAGAGTACAAGTGCAAGGTGTCCAACAAGGCCCTGGGA GCCCCCATCGAAAAGACCATCTCCAAGGCCAAGGGCCAG CCTCGCGAGCCTCAGGTGTACACCCTGCCCCCTAGCAGAG ATGAGCTGACCAAGAACCAGGTGTCCCTGACCTGTCTCGT GAAAGGCTTCTACCCCTCCGATATCGCCGTGGAATGGGA GAGCAACGGCCAGCCCGAGAACAACTACAAGACCACCCC CCCTGTGCTGGACTCCGACGGCTCATTCTTCCTGTACTCTA AGCTGACAGTGGACAAGTCCCGGTGGCAGCAGGGCAACG TGTTCTCCTGCTCCGTGATGCACGAGGCCCTGCACAACCA CTACACCCAGAAGTCCCTGTCCCTGTCTCCCGGGGGAGGC GGAGGATCTGGCGGAGGCGGATCCGGTGGTGGCGGATCT GGGGGCGGTGGATCTGAGGTGCAGCTGCTGGAATCTGGG GGAGGACTGGTGCAGCCAGGCGGATCTCTGAGGCTGTCC TGCGCTGCTTCCGGCTTTACCTTCTCCAGCCACGCCATGA GTTGGGTGCGCCAGGCACCCGGAAAAGGACTGGAATGGG TGTCAGCCATCTGGGCCTCCGGCGAGCAGTACTACGCCGA TAGCGTGAAGGGCCGGTTCACCATCTCTCGGGATAACAG CAAGAATACTCTGTACCTGCAGATGAACTCCCTGCGCGCT |
| | | GAAGATACCGCTGTGTATTACTGCGCCAAGGGCTGGCTG GGCAACTTCGATTACTGGGGCCAGGGAACCCTCGTGACT GTCTCGAGCGCTTCTGTGGCCGCTCCCTCCGTGTTCATCTT CCCACCTTCCGACGAGCAGCTGAAGTCCGGCACTGCCTCT GTCGTGTGCCTGCTGAACAACTTCTACCCTCGGGAAGCCA AGGTGCAGTGGAAAGTGGATAACGCCCTGCAGTCCGGCA ACTCCCAGGAATCCGTGACCGAGCAGGACTCCAAGGACA GCACCTACTCCCTGAGCAGCACCCTGACCCTGTCCAAGGC CGACTACGAGAAGCACAAGGTGTACGCCTGTGAAGTGAC CCACCAGGGCCTGTCCAGCCCCGTGACCAAGTCCTTCAAC CGGGGCGAGTGC |
| 291 | VLCL- Light chain 1 (25G7) (nucleotide sequence) | see Table 48 |
| 184 | VLCH1-Light chain 2 (28H1) (nucleotide sequence) | see above |
| 310 | (25G7) VHCH1-Heavy chain-(28H1) VHCL | EVQLLESGGGLVQPGGSLRLSCAASGFTFSSYAMSWVRQAP GKGLEWVSAISGSGGSTYYADSVKGRFTISRDNSKNTLYLQ MNSLRAEDTAVYYCARDDPWPPFDYWGQGTLVTVSSASTK GPSVFPLAPSSKSTSGGTAALGCLVKDYFPEPVTVSWNSGAL TSGVHTFPAVLQSSGLYSLSSVVTVPSSSLGTQTYICNVNHK PSNTKVDKKVEPKSCDKTHTCPPCPAPEAAGGPSVFLFPPKP KDTLMISRTPEVTCVVVDVSHEDPEVKFNWYVDGVEVHNA KTKPREEQYNSTYRVVSVLTVLHQDWLNGKEYKCKVSNKA LGAPIEKTISKAKGQPREPQVYTLPPSRDELTKNQVSLTCLV KGFYPSDIAVEWESNGQPENNYKTTPPVLDSDGSFFLYSKLT VDKSRWQQGNVFSCSVMHEALHNHYTQKSLSLSPGGGGGS GGGGSGGGGSGGGGSEVQLLESGGGLVQPGGSLRLSCAAS GFTFSSHAMSWVRQAPGKGLEWVSAIWASGEQYYADSVKG RFTISRDNSKNTLYLQMNSLRAEDTAVYYCAKGWLGNFDY WGQGTLVTVSSASVAAPSVFIFPPSDEQLKSGTASVVCLLNN FYPREAKVQWKVDNALQSGNSQESVTEQDSKDSTYSLSSTL TLSKADYEKHKVYACEVTHQGLSSPVTKSFNRGEC |
| 293 | VLCL- Light chain 1 (25G7) | see Table 48 |
| 186 | VLCH1-Light chain 2 (28H1) | see above |
| 311 | (11D5) VHCH1-Heavy chain-(28H1) VHCL (nucleotide sequence) | CAGGTGCAATTGGTGCAGTCTGGGGCTGAGGTGAAGAAG CCTGGGTCCTCGGTGAAGGTCTCCTGCAAGGCCTCCGGAG GCACATTCAGCAGCTACGCTATAAGCTGGGTGCGACAGG CCCCTGGACAAGGGCTCGAGTGGATGGGAGGGATCATCC CTATCTTTGGTACAGCAAACTACGCACAGAAGTTCCAGGG CAGGGTCACCATTACTGCAGACAAATCCACGAGCACAGC CTACATGGAGCTGAGCAGCCTGAGATCTGAGGACACCGC CGTGTATTACTGTGCGAGATCTACTCTGATCTACGGTTAC TTCGACTACTGGGGCCAAGGGACCACCGTGACCGTCTCCT CAGCTAGCACCAAGGGCCCATCCGTGTTCCCTCTGGCCCC TTCCAGCAAGTCTACCTCTGGCGGCACAGCCGCTCTGGGC TGCCTCGTGAAGGACTACTTCCCCGAGCCTGTGACAGTGT CCTGGAACTCTGGCGCCCTGACATCCGGCGTGCACACCTT |
| | | TCCAGCTGTGCTGCAGTCCTCCGGCCTGTACTCCCTGTCCT CCGTCGTGACAGTGCCCTCCAGCTCTCTGGGCACCCAGAC CTACATCTGCAACGTGAACCACAAGCCCTCCAACACCAA GGTGGACAAGAAGGTGGAACCCAAGTCCTGCGACAAGAC CCACACCTGTCCCCCTTGTCCTGCCCCTGAAGCTGCTGGC GGCCCTAGCGTGTTCCTGTTCCCCCCAAAGCCCAAGGACA CCCTGATGATCTCCCGGACCCCCGAAGTGACCTGCGTGGT GGTGGATGTGTCCCACGAGGACCCTGAAGTGAAGTTCAA TTGGTACGTGGACGGCGTGGAAGTGCACAATGCCAAGAC CAAGCCTAGAGAGGAACAGTACAACTCCACCTACCGGGT GGTGTCCGTGCTGACCGTGCTGCACCAGGATTGGCTGAAC GGCAAAGAGTACAAGTGCAAGGTGTCCAACAAGGCCCTG GGAGCCCCCATCGAAAAGACCATCTCCAAGGCCAAGGGC CAGCCTCGCGAGCCTCAGGTGTACACCCTGCCCCCTAGCA GAGATGAGCTGACCAAGAACCAGGTGTCCCTGACCTGTC TCGTGAAAGGCTTCTACCCCTCCGATATCGCCGTGGAATG GGAGAGCAACGGCCAGCCCGAGAACAACTACAAGACCAC CCCCCCTGTGCTGGACTCCGACGGCTCATTCTTCCTGTACT CTAAGCTGACAGTGGACAAGTCCCGGTGGCAGCAGGGCA ACGTGTTCTCCTGCTCCGTGATGCACGAGGCCCTGCACAA CCACTACACCCAGAAGTCCCTGTCCCTGTCTCCCGGGGGA GGCGGAGGATCTGGCGGAGGCGGATCCGGTGGTGGCGGA TCTGGGGGCGGTGGATCTGAGGTGCAGCTGCTGGAATCT GGGGGAGGACTGGTGCAGCCAGGCGGATCTCTGAGGCTG TCCTGCGCTGCTTCCGGCTTTACCTTCTCCAGCCACGCCAT GAGTTGGGTGCGCCAGGCACCCGGAAAAGGACTGGAATG GGTGTCAGCCATCTGGGCCTCCGGCGAGCAGTACTACGCC GATAGCGTGAAGGGCCGGTTCACCATCTCTCGGGATAAC AGCAAGAATACTCTGTACCTGCAGATGAACTCCCTGCGCG CTGAAGATACCGCTGTGTATTACTGCGCCAAGGGCTGGCT GGGCAACTTCGATTACTGGGGCCAGGGAACCCTCGTGAC TGTCTCGAGCGCTTCTGTGGCCGCTCCCTCCGTGTTCATCT TCCCACCTTCCGACGAGCAGCTGAAGTCCGGCACTGCCTC TGTCGTGTGCCTGCTGAACAACTTCTACCCTCGGGAAGCC AAGGTGCAGTGGAAAGTGGATAACGCCCTGCAGTCCGGC AACTCCCAGGAATCCGTGACCGAGCAGGACTCCAAGGAC AGCACCTACTCCCTGAGCAGCACCCTGACCCTGTCCAAGG CCGACTACGAGAAGCACAAGGTGTACGCCTGTGAAGTGA CCCACCAGGGCCTGTCCAGCCCCGTGACCAAGTCCTTCAA CCGGGGCGAGTGC |
| 295 | VLCL-Light chain 1 (11D5) (nucleotide sequence) | see Table 48 |
| 184 | VLCH1-Light chain 2 (28H1) (nucleotide sequence) | see above |
| 312 | (11D5) VHCH1-Heavy chain-(28H1) VHCL | QVQLVQSGAEVKKPGSSVKVSCKASGGTFSSYAISWVRQAP GQGLEWMGGIIPIFGTANYAQKFQGRVTITADKSTSTAYME LSSLRSEDTAVYYCARSTLIYGYFDYWGQGTTVTVSSASTK GPSVFPLAPSSKSTSGGTAALGCLVKDYFPEPVTVSWNSGAL TSGVHTFPAVLQSSGLYSLSSVVTVPSSSLGTQTYICNVNHK |
| | | PSNTKVDKKVEPKSCDKTHTCPPCPAPEAAGGPSVFLFPPKP KDTLMISRTPEVTCVVVDVSHEDPEVKFNWYVDGVEVHNA KTKPREEQYNSTYRVVSVLTVLHQDWLNGKEYKCKVSNKA LGAPIEKTISKAKGQPREPQVYTLPPSRDELTKNQVSLTCLV KGFYPSDIAVEWESNGQPENNYKTTPPVLDSDGSFFLYSKLT VDKSRWQQGNVFSCSVMHEALHNHYTQKSLSLSPGGGGGS GGGGSGGGGSGGGGSEVQLLESGGGLVQPGGSLRLSCAAS GFTFSSHAMSWVRQAPGKGLEWVSAIWASGEQYYADSVKG RFTISRDNSKNTLYLQMNSLRAEDTAVYYCAKGWLGNFDY WGQGTLVTVSSASVAAPSVFIFPPSDEQLKSGTASVVCLLNN FYPREAKVQWKVDNALQSGNSQESVTEQDSKDSTYSLSSTL TLSKADYEKHKVYACEVTHQGLSSPVTKSFNRGEC |
| 297 | VLCL- Light chain 1 (11D5) | see Table 48 |
| 186 | VLCH1-Light chain 2 (28H1) | see above |

All genes were transiently expressed under control of a chimeric MPSV promoter consisting of the MPSV core promoter combined with the CMV promoter enhancer fragment. The expression vector also contains the oriP region for episomal replication in EBNA (Epstein Barr Virus Nuclear Antigen) containing host cells.

The bispecific anti-4-1BB/ anti-FAP constructs were produced by co-transfecting HEK293-EBNA cells with the mammalian expression vectors using polyethylenimine. The cells were transfected with the corresponding expression vectors in a 1:1:1 ratio ("vector heavy chain" : "vector light chain1": "vector light chain2").

For production in 500 mL shake flasks, 400 million HEK293 EBNA cells were seeded 24 hours before transfection. For transfection cells were centrifuged for 5 minutes by 210 x g, and supernatant was replaced by pre-warmed CD CHO medium. Expression vectors were mixed in 20 mL CD CHO medium to a final amount of 200 µg DNA. After addition of 540 µL PEI, the solution was vortexed for 15 seconds and incubated for 10 minutes at room temperature. Afterwards, cells were mixed with the DNA/PEI solution, transferred to a 500 mL shake flask and incubated for 3 hours at 37°C in an incubator with a 5% CO₂ atmosphere. After the incubation, 160 mL F17 medium was added and cells were cultured for 24 hours. One day after transfection 1 mM valproic acid and 7% Feed were added. After culturingfor 7 days, the cell supernatant was collected by centrifugation for 15 minutes at 210 x g. The solution was sterile filtered (0.22 µm filter), supplemented with sodium azide to a final concentration of 0.01 % (w/v), and kept at 4°C.

Purification of bispecific constructs from cell culture supernatants was carried out by affinity chromatography using Protein A as described above for purification of antigen-Fc fusions and antibodies.

The protein was concentrated and filtered prior to loading on a HiLoad Superdex 200 column (GE Healthcare) equilibrated with 20mM Histidine, 140mM NaCl solution of pH 6.0.

The protein concentration of purified bispecific constructs was determined by measuring the OD at 280 nm, using the molar extinction coefficient calculated on the basis of the amino acid sequence. Purity and molecular weight of the bispecific constructs were analyzed by CE-SDS in the presence and absence of a reducing agent (Invitrogen, USA) using a LabChipGXII (Caliper). The aggregate content of bispecific constructs was analyzed using a TSKgel G3000 SW XL analytical size-exclusion column (Tosoh) equilibrated in a 25 mM K₂HPO₄, 125 mM NaCl, 200mM L-Arginine Monohydrocloride, 0.02 % (w/v) NaN₃, pH 6.7 running buffer at 25°C (Table 60).

**Table 60: Biochemical analysis of bispecific, bivalent anti-4-1BB/anti-FAP IgG1 P329G LALA antigen binding molecules**

| Clone | Monomer [%] | Yield [mg/l] |
|---|---|---|
| 12B3/FAP P329GLALA IgG1 2+2 | 97.6 | 6.8 |
| 25G7/FAP P329GLALA IgG1 2+2 | 98.4 | 13 |
| 11D5/FAP P329GLALA IgG1 2+2 | 100 | 8.7 |

### 9.2 Generation of bispecific antibodies targeting 4-1BB and fibroblast activation protein (FAP) in monovalent format (1+1 format, comparative examples)

Bispecific agonistic 4-1BB antibodies with monovalent binding for 4-1BB and for FAP were prepared. The crossmab technology was applied to reduce the formation of wrongly paired light chains as described in International patent application No. WO 2010/145792 A1.

The generation and preparation of the FAP binders is described in WO2012/020006 A2.

The bispecific construct binds monovalently to 4-1BB and to FAP (Figure 40B). It contains a crossed Fab unit (VHCL) of the FAP binder fused to the knob heavy chain of an anti-4-1BB huIgG1 (containing the S354C/T366W mutations). The Fc hole heavy chain (containing the Y349C/T366S/L368A/Y407V mutations) is fused to a Fab against anti-4-1BB. Combination of the targeted anti-FAP-Fc knob with the anti-4-1BB-Fc hole chain allows generation of a heterodimer, which includes a Fab that specifically binds to FAP and a Fab that specifically binds to 4-1BB.

The Pro329Gly, Leu234Ala and Leu235Ala mutations have been introduced in the constant region of the knob and hole heavy chains to abrogate binding to Fc gamma receptors according to the method described in International Patent Appl. Publ. No. WO 2012/130831 A1.

The bispecific monovalent anti-4-1BB and anti-FAP huIgG1 P329GLALA were produced by co-transfecting HEK293-EBNA cells with the mammalian expression vectors using polyethylenimine. The cells were transfected with the corresponding expression vectors in a 1:1:1:1 ratio ("vector knob heavy chain" : "vector light chain 1" : "vector hole heavy chain" : "vector light chain2").

The resulting bispecific, monovalent constructs were produced and purified as described for the bispecific bivalent anti-4-1BB and anti-FAP huIgG1 P329GLALA (see Example 9.1). The nucleotide and amino acid sequences can be found in **Table 61.**

**Table 61: cDNA and amino acid sequences of mature bispecific monovalent anti-4-1BB / anti-FAP huIgG1 P329GLALA kih antibodies**

| SEQ ID NO: | Description | Sequence |
|---|---|---|
| 197 | (28H1) VHCL-heavy chain hole (nucleotide sequence) | GAAGTGCAGCTGCTGGAATCCGGCGGAGGCCTGGTGC AGCCTGGCGGATCTCTGAGACTGTCCTGCGCCGCCTCC GGCTTCACCTTCTCCTCCCACGCCATGTCCTGGGTCCG ACAGGCTCCTGGCAAAGGCCTGGAATGGGTGTCCGCC ATCTGGGCCTCCGGCGAGCAGTACTACGCCGACTCTGT GAAGGGCCGGTTCACCATCTCCCGGGACAACTCCAAG AACACCCTGTACCTGCAGATGAACTCCCTGCGGGCCGA GGACACCGCCGTGTACTACTGTGCCAAGGGCTGGCTGG GCAACTTCGACTACTGGGGACAGGGCACCCTGGTCACC GTGTCCAGCGCTAGCGTGGCCGCTCCCAGCGTGTTCAT CTTCCCACCCAGCGACGAGCAGCTGAAGTCCGGCACA GCCAGCGTGGTGTGCCTGCTGAACAACTTCTACCCCCG CGAGGCCAAGGTGCAGTGGAAGGTGGACAACGCCCTG CAGAGCGGCAACAGCCAGGAATCCGTGACCGAGCAGG ACAGCAAGGACTCCACCTACAGCCTGAGCAGCACCCT GACCCTGAGCAAGGCCGACTACGAGAAGCACAAGGTG TACGCCTGCGAAGTGACCCACCAGGGCCTGTCCAGCCC CGTGACCAAGAGCTTCAACCGGGGCGAGTGCGACAAG ACCCACACCTGTCCCCCTTGCCCTGCCCCTGAAGCTGC TGGTGGCCCTTCCGTGTTCCTGTTCCCCCCAAAGCCCA AGGACACCCTGATGATCAGCCGGACCCCCGAAGTGAC CTGCGTGGTGGTCGATGTGTCCCACGAGGACCCTGAAG TGAAGTTCAATTGGTACGTGGACGGCGTGGAAGTGCA CAATGCCAAGACCAAGCCGCGGGAGGAGCAGTACAAC AGCACGTACCGTGTGGTCAGCGTCCTCACCGTCCTGCA CCAGGACTGGCTGAATGGCAAGGAGTACAAGTGCAAG GTCTCCAACAAAGCCCTCGGCGCCCCCATCGAGAAAA CCATCTCCAAAGCCAAAGGGCAGCCCCGAGAACCACA GGTGTGCACCCTGCCCCCATCCCGGGATGAGCTGACCA AGAACCAGGTCAGCCTCTCGTGCGCAGTCAAAGGCTTC |
| | | TATCCCAGCGACATCGCCGTGGAGTGGGAGAGCAATG GGCAGCCGGAGAACAACTACAAGACCACGCCTCCCGT GCTGGACTCCGACGGCTCCTTCTTCCTCGTGAGCAAGC TCACCGTGGACAAGAGCAGGTGGCAGCAGGGGAACGT CTTCTCATGCTCCGTGATGCATGAGGCTCTGCACAACC ACTACACGCAGAAGAGCCTCTCCCTGTCTCCGGGTAAA |
| 184 | (28H1) VLCH1-Light chain 2 (nucleotide sequence) | GAGATCGTGCTGACCCAGTCTCCCGGCACCCTGAGCCT GAGCCCTGGCGAGAGAGCCACCCTGAGCTGCAGAGCC AGCCAGAGCGTGAGCCGGAGCTACCTGGCCTGGTATC AGCAGAAGCCCGGCCAGGCCCCCAGACTGCTGATCAT CGGCGCCAGCACCCGGGCCACCGGCATCCCCGATAGA TTCAGCGGCAGCGGCTCCGGCACCGACTTCACCCTGAC CATCAGCCGGCTGGAACCCGAGGACTTCGCCGTGTACT ACTGCCAGCAGGGCCAGGTGATCCCCCCCACCTTCGGC CAGGGCACCAAGGTGGAAATCAAGAGCTCCGCTAGCA CCAAGGGCCCCTCCGTGTTTCCTCTGGCCCCCAGCAGC AAGAGCACCTCTGGCGGAACAGCCGCCCTGGGCTGCC TGGTGAAAGACTACTTCCCCGAGCCCGTGACCGTGTCC TGGAACTCTGGCGCCCTGACCAGCGGCGTGCACACCTT TCCAGCCGTGCTGCAGAGCAGCGGCCTGTACTCCCTGA GCAGCGTGGTGACAGTGCCCTCCAGCAGCCTGGGCAC CCAGACCTACATCTGCAACGTGAACCACAAGCCCAGC AACACCAAAGTGGACAAGAAGGTGGAACCCAAGAGCT GCGAC |
| 198 | (28H1) VHCL-heavy chain hole | EVQLLESGGGLVQPGGSLRLSCAASGFTFSSHAMSWVRQ APGKGLEWVSAIWASGEQYYADSVKGRFTISRDNSKNTL YLQMNSLRAEDTAVYYCAKGWLGNFDYWGQGTLVTVS SASVAAPSVFIFPPSDEQLKSGTASVVCLLNNFYPREAKV QWKVDNALQSGNSQESVTEQDSKDSTYSLSSTLTLSKAD YEKHKVYACEVTHQGLSSPVTKSFNRGECDKTHTCPPCP APEAAGGPSVFLFPPKPKDTLMISRTPEVTCVVVDVSHED PEVKFNWYVDGVEVHNAKTKPREEQYNSTYRVVSVLTV LHQDWLNGKEYKCKVSNKALGAPIEKTISKAKGQPREPQ VCTLPPSRDELTKNQVSLSCAVKGFYPSDIAVEWESNGQP ENNYKTTPPVLDSDGSFFLVSKLTVDKSRWQQGNVFSCS VMHEALHNHYTQKSLSLSPGK |
| 186 | (28H1) VLCH1-Light chain 2 | EIVLTQSPGTLSLSPGERATLSCRASQSVSRSYLAWYQQK PGQAPRLLIIGASTRATGIPDRFSGSGSGTDFTLTISRLEPE DFAVYYCQQGQVIPPTFGQGTKVEIKSSASTKGPSVFPLA PSSKSTSGGTAALGCLVKDYFPEPVTVSWNSGALTSGVH TFPAVLQSSGLYSLSSVVTVPSSSLGTQTYICNVNHKPSNT KVDKKVEPKSCD |
| 313 | (12B3) VHCH1-heavy chain knob (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGATCTGAA TTCCGTTTCTACGCTGACTTCGACTACTGGGGCCAAGG GACCACCGTGACCGTCTCCTCAGCTAGCACCAAGGGCC CTAGCGTGTTCCCTCTGGCCCCTAGCAGCAAGAGCACA AGTGGAGGAACAGCCGCCCTGGGCTGCCTGGTCAAGG |
| | | ACTACTTCCCCGAGCCCGTGACCGTGTCCTGGAATTCT GGCGCCCTGACAAGCGGCGTGCACACATTTCCAGCCGT GCTGCAGAGCAGCGGCCTGTACTCTCTGAGCAGCGTCG TGACCGTGCCCTCTAGCTCTCTGGGCACCCAGACCTAC ATCTGCAACGTGAACCACAAGCCCAGCAACACCAAAG TGGACAAGAAGGTGGAACCCAAGAGCTGCGACAAGAC CCACACCTGTCCCCCTTGCCCTGCCCCTGAAGCTGCTG GTGGCCCTTCCGTGTTCCTGTTCCCCCCAAAGCCCAAG GACACCCTGATGATCAGCCGGACCCCCGAAGTGACCT GCGTGGTGGTCGATGTGTCCCACGAGGACCCTGAAGTG AAGTTCAATTGGTACGTGGACGGCGTGGAAGTGCACA ATGCCAAGACCAAGCCGCGGGAGGAGCAGTACAACAG CACGTACCGTGTGGTCAGCGTCCTCACCGTCCTGCACC AGGACTGGCTGAATGGCAAGGAGTACAAGTGCAAGGT CTCCAACAAAGCCCTCGGCGCCCCCATCGAGAAAACC ATCTCCAAAGCCAAAGGGCAGCCCCGAGAACCACAGG TGTACACCCTGCCCCCATGCCGGGATGAGCTGACCAAG AACCAGGTCAGCCTGTGGTGCCTGGTCAAAGGCTTCTA TCCCAGCGACATCGCCGTGGAGTGGGAGAGCAATGGG CAGCCGGAGAACAACTACAAGACCACGCCTCCCGTGC TGGACTCCGACGGCTCCTTCTTCCTCTACAGCAAGCTC ACCGTGGACAAGAGCAGGTGGCAGCAGGGGAACGTCT TCTCATGCTCCGTGATGCATGAGGCTCTGCACAACCAC TACACGCAGAAGAGCCTCTCCCTGTCTCCGGGTAAA |
| 287 | (12B3) VLCL-Light chain 1 (nucleotide sequence) | see Table 48 |
| 314 | (12B3) VHCH1-heavy chain knob | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSEFRFYADFDYWGQGTTVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDKTHTCPPCPAPE AAGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALGAPIEKTISKAKGQPREPQVYT LPPCRDELTKNQVSLWCLVKGFYPSDIAVEWESNGQPEN NYKTTPPVLDSDGSFFLYSKLTVDKSRWQQGNVFSCSVM HEALHNHYTQKSLSLSPGK |
| 289 | (12B3) VLCL-Light chain 1 | see Table 48 |
| 315 | (25G7) VHCH1-heavy chain knob (nucleotide sequence) | GAGGTGCAATTGTTGGAGTCTGGGGGAGGCTTGGTAC AGCCTGGGGGGTCCCTGAGACTCTCCTGTGCAGCCTCC GGATTCACCTTTAGCAGTTATGCCATGAGCTGGGTCCG CCAGGCTCCAGGGAAGGGGCTGGAGTGGGTCTCAGCT ATTAGTGGTAGTGGTGGTAGCACATACTACGCAGACTC CGTGAAGGGCCGGTTCACCATCTCCAGAGACAATTCCA AGAACACGCTGTATCTGCAGATGAACAGCCTGAGAGC CGAGGACACGGCCGTATATTACTGTGCGCGTGACGACC CGTGGCCGCCGTTCGACTACTGGGGCCAAGGAACCCTG GTCACCGTCTCGAGTGCTAGCACCAAGGGCCCTAGCGT GTTCCCTCTGGCCCCTAGCAGCAAGAGCACAAGTGGA GGAACAGCCGCCCTGGGCTGCCTGGTCAAGGACTACTT CCCCGAGCCCGTGACCGTGTCCTGGAATTCTGGCGCCC |
| | | TGACAAGCGGCGTGCACACATTTCCAGCCGTGCTGCAG AGCAGCGGCCTGTACTCTCTGAGCAGCGTCGTGACCGT GCCCTCTAGCTCTCTGGGCACCCAGACCTACATCTGCA ACGTGAACCACAAGCCCAGCAACACCAAAGTGGACAA GAAGGTGGAACCCAAGAGCTGCGACAAGACCCACACC TGTCCCCCTTGCCCTGCCCCTGAAGCTGCTGGTGGCCC TTCCGTGTTCCTGTTCCCCCCAAAGCCCAAGGACACCC TGATGATCAGCCGGACCCCCGAAGTGACCTGCGTGGTG GTCGATGTGTCCCACGAGGACCCTGAAGTGAAGTTCAA TTGGTACGTGGACGGCGTGGAAGTGCACAATGCCAAG ACCAAGCCGCGGGAGGAGCAGTACAACAGCACGTACC GTGTGGTCAGCGTCCTCACCGTCCTGCACCAGGACTGG CTGAATGGCAAGGAGTACAAGTGCAAGGTCTCCAACA AAGCCCTCGGCGCCCCCATCGAGAAAACCATCTCCAA AGCCAAAGGGCAGCCCCGAGAACCACAGGTGTACACC CTGCCCCCATGCCGGGATGAGCTGACCAAGAACCAGG TCAGCCTGTGGTGCCTGGTCAAAGGCTTCTATCCCAGC GACATCGCCGTGGAGTGGGAGAGCAATGGGCAGCCGG AGAACAACTACAAGACCACGCCTCCCGTGCTGGACTCC GACGGCTCCTTCTTCCTCTACAGCAAGCTCACCGTGGA CAAGAGCAGGTGGCAGCAGGGGAACGTCTTCTCATGC TCCGTGATGCATGAGGCTCTGCACAACCACTACACGCA GAAGAGCCTCTCCCTGTCTCCGGGTAAA |
| 291 | (25G7) VLCL-Light chain 1 (nucleotide sequence) | see Table 48 |
| 316 | (25G7) VHCH1-heavy chain knob | EVQLLESGGGLVQPGGSLRLSCAASGFTFSSYAMSWVRQ APGKGLEWVSAISGSGGSTYYADSVKGRFTISRDNSKNTL YLQMNSLRAEDTAVYYCARDDPWPPFDYWGQGTLVTV SSASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDKTHTCPPCPAPEA AGGPSVFLFPPKPKDTLMISRTPEVTCVVVDVSHEDPEVK FNWYVDGVEVHNAKTKPREEQYNSTYRVVSVLTVLHQD WLNGKEYKCKVSNKALGAPIEKTISKAKGQPREPQVYTL PPCRDELTKNQVSLWCLVKGFYPSDIAVEWESNGQPENN YKTTPPVLDSDGSFFLYSKLTVDKSRWQQGNVFSCSVMH EALHNHYTQKSLSLSPGK |
| 293 | (25G7) VLCL-Light chain 1 | see Table 48 |

All genes were transiently expressed under control of a chimeric MPSV promoter consisting of the MPSV core promoter combined with the CMV promoter enhancer fragment. The expression vector also contains the oriP region for episomal replication in EBNA (Epstein Barr Virus Nuclear Antigen) containing host cells.

The bispecific anti-4-1BB/ anti-FAP constructs were produced by co-transfecting HEK293-EBNA cells with the mammalian expression vectors using polyethylenimine. The cells were transfected with the corresponding expression vectors in a 1:1:1:1 ratio ("vector heavy knob chain" : "vector heavy hole chain " : "vector light chain 1": "vector light chain2").

For production in 500 mL shake flasks, 400 million HEK293 EBNA cells were seeded 24 hours before transfection. For transfection cells were centrifuged for 5 minutes by 210 x g, and supernatant was replaced by pre-warmed CD CHO medium. Expression vectors were mixed in 20 mL CD CHO medium to a final amount of 200 µg DNA. After addition of 540 µL PEI, the solution was vortexed for 15 seconds and incubated for 10 minutes at room temperature. Afterwards, cells were mixed with the DNA/PEI solution, transferred to a 500 mL shake flask and incubated for 3 hours at 37 °C in an incubator with a 5% CO₂ atmosphere. After the incubation, 160 mL F17 medium was added and cells were cultured for 24 hours. One day after transfection 1 mM valproic acid and 7% Feed were added. After culturingfor 7 days, the cell supernatant was collected by centrifugation for 15 minutes at 210 x g. The solution was sterile filtered (0.22 µm filter), supplemented with sodium azide to a final concentration of 0.01 % (w/v), and kept at 4 °C.

Purification of bispecific constructs from cell culture supernatants was carried out by affinity chromatography using Protein A as described above for purification of antigen/Fc fusion molecules or antibodies. The protein concentration of purified bispecific constructs was determined by measuring the OD at 280 nm, using the molar extinction coefficient calculated on the basis of the amino acid sequence. Purity and molecular weight of the bispecific constructs were analyzed by CE-SDS in the presence and absence of a reducing agent (Invitrogen, USA) using a LabChipGXII (Caliper). The aggregate content of bispecific constructs was analyzed using a TSKgel G3000 SW XL analytical size-exclusion column (Tosoh) equilibrated in a 25 mM K2HPO4, 125 mM NaCl, 200mM L-Arginine Monohydrocloride, 0.02 % (w/v) NaN3, pH 6.7 running buffer at 25°C.

**Table 62: Biochemical analysis of bispecific, monovalent anti-4-1BB/anti-FAP IgG1 P329G LALA antigen binding molecules**

| Clone | Yield [mg/l] | Monomer [%] |
|---|---|---|
| 12B3/FAP P329GLALA IgG1 1+1 | 14 | 94.8 |
| 25G7/FAP P329GLALA IgG1 1+1 | 33 | 92.2 |

### 9.3 Generation of bispecific antibodies with a bivalent binding to 4-1BB and a monovalent binding to tumor associated antigen (TAA) (2+1 format, comparative examples)

Bispecific agonistic 4-1BB antibodies with bivalent binding for 4-1BB and monovalent binding for FAP, also termed 2+1, have been prepared as depicted in **Figures 40C and 40D****.**

In this example, the first heavy chain HC1 of the construct was comprised of the following components: VHCH1 of anti-4-1BB binder, followed by Fc knob, at which C-terminus a VL or VH of anti-FAP binder was fused. The second heavy chain HC2 was comprised of VHCH1 of anti-4-1BB followed by Fc hole, at which C-terminus a VH or VL, respectively, of anti-FAP binder (clone 4B9) was fused. The generation and preparation of FAP binder 4B9 is described in WO 2012/020006 A2. Binders against 4-1BB(12B3, 9B11, 11D5 and 25G7), were generated as described in Example 6. Combination of the targeted anti-FAP-Fc knob with the anti-4-1BB-Fc hole chain allows generation of a heterodimer, which includes a FAP binding moiety and two 4-1BB binding Fabs (**Figures 40C and 40D****).**

The Pro329Gly, Leu234Ala and Leu235Ala mutations have been introduced in the constant region of the knob and hole heavy chains to abrogate binding to Fcgamma receptors according to the method described in International Patent Appl. Publ. No.WO2012/130831A1.

The bispecific 2+1 anti-4-1BB anti-FAP huIgG1 P329GLALA antibodies were produced by co-transfecting HEK293-EBNA cells with the mammalian expression vectors using polyethylenimine. The cells were transfected with the corresponding expression vectors in a 1:1:1 ratio ("vector knob heavy chain": "vector light chain" :"vector hole heavy chain"). The constructs were produced and purified as described for the bispecific bivalent anti-4-1BB and anti-FAP huIgG1 P329GLALA antibodies (see Example 9.1).

The base pair and amino acid sequences for 2+1 anti-4-1BB, anti-FAP constructs with a-FAP VH fused to knob and VL fused to hole chain can be found respectively in **Table 63.**

**Table 63: cDNA and amino acid sequences of mature bispecific 2+1 anti-4-1BB, anti-FAP human IgG1 P329GLALA. (Constructs with a-FAP VL fused to hole and VH fused to knob chain, termed in Table 64 below hole-VL)**

| SEQ ID NO: | Description | Sequence |
|---|---|---|
| 291 | (25G7) VLCL-light chain (nucleotide sequence) | see Table 48 |
| 317 | (25G7) VHCH1 Fc knob VH (4B9) (nucleotide sequence, heavy chain 1) | GAGGTGCAGCTGCTGGAATCTGGCGGCGGACTGGTGC AGCCTGGCGGATCTCTGAGACTGAGCTGTGCCGCCAGC GGCTTCACCTTCAGCAGCTACGCCATGAGCTGGGTGCG CCAGGCCCCTGGAAAAGGCCTGGAATGGGTGTCCGCC |
| | | ATCTCTGGCAGCGGCGGCAGCACCTACTACGCCGATTC TGTGAAGGGCCGGTTCACCATCAGCCGGGACAACAGC AAGAACACCCTGTACCTGCAGATGAACAGCCTGCGGG CCGAGGACACCGCCGTGTACTATTGCGCCAGGGACGA CCCCTGGCCCCCCTTTGATTATTGGGGACAGGGCACCC TCGTGACCGTGTCCAGCGCTAGCACCAAGGGCCCATCG GTCTTCCCCCTGGCACCCTCCTCCAAGAGCACCTCTGG GGGCACAGCGGCCCTGGGCTGCCTGGTCAAGGACTAC TTCCCCGAACCGGTGACGGTGTCGTGGAACTCAGGCGC CCTGACCAGCGGCGTGCACACCTTCCCGGCTGTCCTAC AGTCCTCAGGACTCTACTCCCTCAGCAGCGTGGTGACC GTGCCCTCCAGCAGCTTGGGCACCCAGACCTACATCTG CAACGTGAATCACAAGCCCAGCAACACCAAGGTGGAC AAGAAAGTTGAGCCCAAATCTTGTGACAAAACTCACA CATGCCCACCGTGCCCAGCACCTGAAGCTGCAGGGGG ACCGTCAGTCTTCCTCTTCCCCCCAAAACCCAAGGACA CCCTCATGATCTCCCGGACCCCTGAGGTCACATGCGTG GTGGTGGACGTGAGCCACGAAGACCCTGAGGTCAAGT TCAACTGGTACGTGGACGGCGTGGAGGTGCATAATGC CAAGACAAAGCCGCGGGAGGAGCAGTACAACAGCACG TACCGTGTGGTCAGCGTCCTCACCGTCCTGCACCAGGA CTGGCTGAATGGCAAGGAGTACAAGTGCAAGGTCTCC AACAAAGCCCTCGGCGCCCCCATCGAGAAAACCATCT CCAAAGCCAAAGGGCAGCCCCGAGAACCACAGGTGTA CACCCTGCCCCCCTGCAGAGATGAGCTGACCAAGAAC CAGGTGTCCCTGTGGTGTCTGGTCAAGGGCTTCTACCC CAGCGATATCGCCGTGGAGTGGGAGAGCAACGGCCAG CCTGAGAACAACTACAAGACCACCCCCCCTGTGCTGGA CAGCGACGGCAGCTTCTTCCTGTACTCCAAACTGACCG TGGACAAGAGCCGGTGGCAGCAGGGCAACGTGTTCAG CTGCAGCGTGATGCACGAGGCCCTGCACAACCACTAC ACCCAGAAGTCCCTGAGCCTGAGCCCCGGCGGAGGCG GCGGAAGCGGAGGAGGAGGATCTGGGGGCGGAGGTTC CGGAGGCGGTGGATCTGAGGTGCAGCTGCTCGAAAGC GGCGGAGGACTGGTGCAGCCTGGCGGCAGCCTGAGAC TGTCTTGCGCCGCCAGCGGCTTCACCTTCAGCAGCTAC GCCATGAGCTGGGTCCGCCAGGCCCCTGGCAAGGGAC TGGAATGGGTGTCCGCCATCATCGGCTCTGGCGCCAGC ACCTACTACGCCGACAGCGTGAAGGGCCGGTTCACCAT CAGCCGGGACAACAGCAAGAACACCCTGTACCTGCAG ATGAACAGCCTGCGGGCCGAGGACACCGCCGTGTACT ACTGCGCCAAGGGATGGTTCGGCGGCTTCAACTACTGG GGACAGGGCACCCTGGTCACCGTGTCCAGC |
| 318 | (25G7) VHCH1 Fc hole VL (4B9) (nucleotide sequence, heavy chain 2) | GAGGTGCAGCTGCTGGAATCTGGCGGCGGACTGGTGC AGCCTGGCGGATCTCTGAGACTGAGCTGTGCCGCCAGC GGCTTCACCTTCAGCAGCTACGCCATGAGCTGGGTGCG CCAGGCCCCTGGAAAAGGCCTGGAATGGGTGTCCGCC ATCTCTGGCAGCGGCGGCAGCACCTACTACGCCGATTC TGTGAAGGGCCGGTTCACCATCAGCCGGGACAACAGC AAGAACACCCTGTACCTGCAGATGAACAGCCTGCGGG CCGAGGACACCGCCGTGTACTATTGCGCCAGGGACGA CCCCTGGCCCCCCTTTGATTATTGGGGACAGGGCACCC TCGTGACCGTGTCCAGCGCTAGCACCAAGGGCCCATCG GTCTTCCCCCTGGCACCCTCCTCCAAGAGCACCTCTGG |
| | | GGGCACAGCGGCCCTGGGCTGCCTGGTCAAGGACTAC TTCCCCGAACCGGTGACGGTGTCGTGGAACTCAGGCGC CCTGACCAGCGGCGTGCACACCTTCCCGGCTGTCCTAC AGTCCTCAGGACTCTACTCCCTCAGCAGCGTGGTGACC GTGCCCTCCAGCAGCTTGGGCACCCAGACCTACATCTG CAACGTGAATCACAAGCCCAGCAACACCAAGGTGGAC AAGAAAGTTGAGCCCAAATCTTGTGACAAAACTCACA CATGCCCACCGTGCCCAGCACCTGAAGCTGCAGGGGG ACCGTCAGTCTTCCTCTTCCCCCCAAAACCCAAGGACA CCCTCATGATCTCCCGGACCCCTGAGGTCACATGCGTG GTGGTGGACGTGAGCCACGAAGACCCTGAGGTCAAGT TCAACTGGTACGTGGACGGCGTGGAGGTGCATAATGC CAAGACAAAGCCGCGGGAGGAGCAGTACAACAGCACG TACCGTGTGGTCAGCGTCCTCACCGTCCTGCACCAGGA CTGGCTGAATGGCAAGGAGTACAAGTGCAAGGTCTCC AACAAAGCCCTCGGCGCCCCCATCGAGAAAACCATCT CCAAAGCCAAAGGGCAGCCCCGAGAACCACAGGTGTG CACCCTGCCCCCATCCCGGGATGAGCTGACCAAGAACC AGGTCAGCCTCTCGTGCGCAGTCAAAGGCTTCTATCCC AGCGACATCGCCGTGGAGTGGGAGAGCAATGGGCAGC CGGAGAACAACTACAAGACCACGCCTCCCGTGCTGGA CTCCGACGGCTCCTTCTTCCTCGTGAGCAAGCTCACCG TGGACAAGAGCAGGTGGCAGCAGGGGAACGTCTTCTC ATGCTCCGTGATGCATGAGGCTCTGCACAACCACTACA CGCAGAAGAGCCTCTCCCTGTCTCCGGGTGGAGGCGGC GGAAGCGGAGGAGGAGGATCCGGCGGCGGAGGTTCCG GAGGCGGAGGATCCGAGATCGTGCTGACCCAGTCTCC CGGCACCCTGTCTCTGAGCCCTGGCGAGAGAGCCACCC TGTCCTGCAGAGCCTCCCAGTCCGTGACCTCCTCCTAC CTCGCCTGGTATCAGCAGAAGCCCGGCCAGGCCCCTCG GCTGCTGATCAACGTGGGCAGTCGGAGAGCCACCGGC ATCCCTGACCGGTTCTCCGGCTCTGGCTCCGGCACCGA CTTCACCCTGACCATCTCCCGGCTGGAACCCGAGGACT TCGCCGTGTACTACTGCCAGCAGGGCATCATGCTGCCC CCCACCTTTGGCCAGGGCACCAAGGTGGAAATCAAG |
| 293 | (25G7) VLCL-light chain | see Table 48 |
| 319 | (25G7) VHCH1 Fc knob VH (4B9) (heavy chain 1) | EVQLLESGGGLVQPGGSLRLSCAASGFTFSSYAMSWVRQ APGKGLEWVSAISGSGGSTYYADSVKGRFTISRDNSKNTL YLQMNSLRAEDTAVYYCARDDPWPPFDYWGQGTLVTV SSASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDKTHTCPPCPAPEA AGGPSVFLFPPKPKDTLMISRTPEVTCVVVDVSHEDPEVK FNWYVDGVEVHNAKTKPREEQYNSTYRVVSVLTVLHQD WLNGKEYKCKVSNKALGAPIEKTISKAKGQPREPQVYTL PPCRDELTKNQVSLWCLVKGFYPSDIAVEWESNGQPENN YKTTPPVLDSDGSFFLYSKLTVDKSRWQQGNVFSCSVMH EALHNHYTQKSLSLSPGGGGGSGGGGSGGGGSGGGGSE VQLLESGGGLVQPGGSLRLSCAASGFTFSSYAMSWVRQA PGKGLEWVSAIIGSGASTYYADSVKGRFTISRDNSKNTLY LQMNSLRAEDTAVYYCAKGWFGGFNYWGQGTLVTVSS EVQLLESGGGLVQPGGSLRLSCAASGFTFSSYAMSWVRQ APGKGLEWVSAISGSGGSTYYADSVKGRFTISRDNSKNTL |
| 320 | (25G7) VHCH1 Fc hole VL (4B9) | EVQLLESGGGLVQPGGSLRLSCAASGFTFSSYAMSWVRQ APGKGLEWVSAISGSGGSTYYADSVKGRFTISRDNSKNTL |
| | (heavy chain 2) | YLQMNSLRAEDTAVYYCARDDPWPPFDYWGQGTLVTV SSASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDKTHTCPPCPAPEA AGGPSVFLFPPKPKDTLMISRTPEVTCVVVDVSHEDPEVK FNWYVDGVEVHNAKTKPREEQYNSTYRVVSVLTVLHQD WLNGKEYKCKVSNKALGAPIEKTISKAKGQPREPQVCTL PPSRDELTKNQVSLSCAVKGFYPSDIAVEWESNGQPENN YKTTPPVLDSDGSFFLVSKLTVDKSRWQQGNVFSCSVMH EALHNHYTQKSLSLSPGGGGGSGGGGSGGGGSGGGGSEI VLTQSPGTLSLSPGERATLSCRASQSVTSSYLAWYQQKPG QAPRLLINVGSRRATGIPDRFSGSGSGTDFTLTISRLEPEDF AVYYCQQGIMLPPTFGQGTKVEIK |
| 295 | (11D5) VLCL-light chain (nucleotide sequence) | GACATCCAGATGACCCAGTCTCCTTCCACCCTGTCTGC ATCTGTAGGAGACCGTGTCACCATCACTTGCCGTGCCA GTCAGAGTATTAGTAGCTGGTTGGCCTGGTATCAGCAG AAACCAGGGAAAGCCCCTAAGCTCCTGATCTATGATGC CTCCAGTTTGGAAAGTGGGGTCCCATCACGTTTCAGCG GCAGTGGATCCGGGACAGAATTCACTCTCACCATCAGC AGCTTGCAGCCTGATGATTTTGCAACTTATTACTGCCA ACAGCTTAATTCGTATCCTCAGACGTTTGGCCAGGGCA CCAAAGTCGAGATCAAGCGTACGGTGGCTGCACCATCT GTCTTCATCTTCCCGCCATCTGATGAGCAGTTGAAATC TGGAACTGCCTCTGTTGTGTGCCTGCTGAATAACTTCT ATCCCAGAGAGGCCAAAGTACAGTGGAAGGTGGATAA CGCCCTCCAATCGGGTAACTCCCAGGAGAGTGTCACAG AGCAGGACAGCAAGGACAGCACCTACAGCCTCAGCAG CACCCTGACGCTGAGCAAAGCAGACTACGAGAAACAC AAAGTCTACGCCTGCGAAGTCACCCATCAGGGCCTGA GCTCGCCCGTCACAAAGAGCTTCAACAGGGGAGAGTG T |
| 321 | (11D5) VHCH1 Fc knob VH (4B9) (nucleotide sequence, heavy chain 1) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTACTGTGCCAGAAGCA CCCTGATCTACGGCTACTTCGACTACTGGGGCCAGGGC ACCACCGTGACCGTGTCTAGCGCTAGCACCAAGGGCCC ATCGGTCTTCCCCCTGGCACCCTCCTCCAAGAGCACCT CTGGGGGCACAGCGGCCCTGGGCTGCCTGGTCAAGGA CTACTTCCCCGAACCGGTGACGGTGTCGTGGAACTCAG GCGCCCTGACCAGCGGCGTGCACACCTTCCCGGCTGTC CTACAGTCCTCAGGACTCTACTCCCTCAGCAGCGTGGT GACCGTGCCCTCCAGCAGCTTGGGCACCCAGACCTACA TCTGCAACGTGAATCACAAGCCCAGCAACACCAAGGT GGACAAGAAAGTTGAGCCCAAATCTTGTGACAAAACT CACACATGCCCACCGTGCCCAGCACCTGAAGCTGCAG GGGGACCGTCAGTCTTCCTCTTCCCCCCAAAACCCAAG GACACCCTCATGATCTCCCGGACCCCTGAGGTCACATG CGTGGTGGTGGACGTGAGCCACGAAGACCCTGAGGTC AAGTTCAACTGGTACGTGGACGGCGTGGAGGTGCATA |
| | | ATGCCAAGACAAAGCCGCGGGAGGAGCAGTACAACAG CACGTACCGTGTGGTCAGCGTCCTCACCGTCCTGCACC AGGACTGGCTGAATGGCAAGGAGTACAAGTGCAAGGT CTCCAACAAAGCCCTCGGCGCCCCCATCGAGAAAACC ATCTCCAAAGCCAAAGGGCAGCCCCGAGAACCACAGG TGTACACCCTGCCCCCCTGCAGAGATGAGCTGACCAAG AACCAGGTGTCCCTGTGGTGTCTGGTCAAGGGCTTCTA CCCCAGCGATATCGCCGTGGAGTGGGAGAGCAACGGC CAGCCTGAGAACAACTACAAGACCACCCCCCCTGTGCT GGACAGCGACGGCAGCTTCTTCCTGTACTCCAAACTGA CCGTGGACAAGAGCCGGTGGCAGCAGGGCAACGTGTT CAGCTGCAGCGTGATGCACGAGGCCCTGCACAACCAC TACACCCAGAAGTCCCTGAGCCTGAGCCCCGGCGGAG GCGGCGGAAGCGGAGGAGGAGGATCTGGGGGCGGAG GTTCCGGAGGCGGTGGATCTGAGGTGCAGCTGCTCGA AAGCGGCGGAGGACTGGTGCAGCCTGGCGGCAGCCTG AGACTGTCTTGCGCCGCCAGCGGCTTCACCTTCAGCAG CTACGCCATGAGCTGGGTCCGCCAGGCCCCTGGCAAG GGACTGGAATGGGTGTCCGCCATCATCGGCTCTGGCGC CAGCACCTACTACGCCGACAGCGTGAAGGGCCGGTTC ACCATCAGCCGGGACAACAGCAAGAACACCCTGTACC TGCAGATGAACAGCCTGCGGGCCGAGGACACCGCCGT GTACTACTGCGCCAAGGGATGGTTCGGCGGCTTCAACT ACTGGGGACAGGGCACCCTGGTCACCGTGTCCAGC |
| 322 | (11D5) VHCH1 Fc hole VL (4B9) (nucleotide sequence, heavy chain 2) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTACTGTGCCAGAAGCA CCCTGATCTACGGCTACTTCGACTACTGGGGCCAGGGC ACCACCGTGACCGTGTCTAGCGCTAGCACCAAGGGCCC ATCGGTCTTCCCCCTGGCACCCTCCTCCAAGAGCACCT CTGGGGGCACAGCGGCCCTGGGCTGCCTGGTCAAGGA CTACTTCCCCGAACCGGTGACGGTGTCGTGGAACTCAG GCGCCCTGACCAGCGGCGTGCACACCTTCCCGGCTGTC CTACAGTCCTCAGGACTCTACTCCCTCAGCAGCGTGGT GACCGTGCCCTCCAGCAGCTTGGGCACCCAGACCTACA TCTGCAACGTGAATCACAAGCCCAGCAACACCAAGGT GGACAAGAAAGTTGAGCCCAAATCTTGTGACAAAACT CACACATGCCCACCGTGCCCAGCACCTGAAGCTGCAG GGGGACCGTCAGTCTTCCTCTTCCCCCCAAAACCCAAG GACACCCTCATGATCTCCCGGACCCCTGAGGTCACATG CGTGGTGGTGGACGTGAGCCACGAAGACCCTGAGGTC AAGTTCAACTGGTACGTGGACGGCGTGGAGGTGCATA ATGCCAAGACAAAGCCGCGGGAGGAGCAGTACAACAG CACGTACCGTGTGGTCAGCGTCCTCACCGTCCTGCACC AGGACTGGCTGAATGGCAAGGAGTACAAGTGCAAGGT CTCCAACAAAGCCCTCGGCGCCCCCATCGAGAAAACC ATCTCCAAAGCCAAAGGGCAGCCCCGAGAACCACAGG TGTGCACCCTGCCCCCATCCCGGGATGAGCTGACCAAG AACCAGGTCAGCCTCTCGTGCGCAGTCAAAGGCTTCTA |
| | | TCCCAGCGACATCGCCGTGGAGTGGGAGAGCAATGGG CAGCCGGAGAACAACTACAAGACCACGCCTCCCGTGC TGGACTCCGACGGCTCCTTCTTCCTCGTGAGCAAGCTC ACCGTGGACAAGAGCAGGTGGCAGCAGGGGAACGTCT TCTCATGCTCCGTGATGCATGAGGCTCTGCACAACCAC TACACGCAGAAGAGCCTCTCCCTGTCTCCGGGTGGAGG CGGCGGAAGCGGAGGAGGAGGATCCGGCGGCGGAGG TTCCGGAGGCGGAGGATCCGAGATCGTGCTGACCCAG TCTCCCGGCACCCTGTCTCTGAGCCCTGGCGAGAGAGC CACCCTGTCCTGCAGAGCCTCCCAGTCCGTGACCTCCT CCTACCTCGCCTGGTATCAGCAGAAGCCCGGCCAGGCC CCTCGGCTGCTGATCAACGTGGGCAGTCGGAGAGCCA CCGGCATCCCTGACCGGTTCTCCGGCTCTGGCTCCGGC ACCGACTTCACCCTGACCATCTCCCGGCTGGAACCCGA GGACTTCGCCGTGTACTACTGCCAGCAGGGCATCATGC TGCCCCCCACCTTTGGCCAGGGCACCAAGGTGGAAATC AAG |
| 297 | (11D5) VLCL-light chain | DIQMTQSPSTLSASVGDRVTITCRASQSISSWLAWYQQKP GKAPKLLIYDASSLESGVPSRFSGSGSGTEFTLTISSLQPDD FATYYCQQLNSYPQTFGQGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 323 | (11D5) VHCH1 Fc knob VH (4B9) (heavy chain 1) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSTLIYGYFDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPEAA GGPSVFLFPPKPKDTLMISRTPEVTCVVVDVSHEDPEVKF NWYVDGVEVHNAKTKPREEQYNSTYRVVSVLTVLHQD WLNGKEYKCKVSNKALGAPIEKTISKAKGQPREPQVYTL PPCRDELTKNQVSLWCLVKGFYPSDIAVEWESNGQPENN YKTTPPVLDSDGSFFLYSKLTVDKSRWQQGNVFSCSVMH EALHNHYTQKSLSLSPGGGGGSGGGGSGGGGSGGGGSE VQLLESGGGLVQPGGSLRLSCAASGFTFSSYAMSWVRQA PGKGLEWVSAIIGSGASTYYADSVKGRFTISRDNSKNTLY LQMNSLRAEDTAVYYCAKGWFGGFNYWGQGTLVTVSS |
| 324 | (11D5) VHCH1 Fc hole VL (4B9) (heavy chain 2) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSTLIYGYFDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPEAA GGPSVFLFPPKPKDTLMISRTPEVTCVVVDVSHEDPEVKF NWYVDGVEVHNAKTKPREEQYNSTYRVVSVLTVLHQD WLNGKEYKCKVSNKALGAPIEKTISKAKGQPREPQVCTL PPSRDELTKNQVSLSCAVKGFYPSDIAVEWESNGQPENN YKTTPPVLDSDGSFFLVSKLTVDKSRWQQGNVFSCSVMH EALHNHYTQKSLSLSPGGGGGSGGGGSGGGGSGGGGSEI VLTQSPGTLSLSPGERATLSCRASQSVTSSYLAWYQQKPG QAPRLLINVGSRRATGIPDRFSGSGSGTDFTLTISRLEPEDF AVYYCQQGIMLPPTFGQGTKVEIK |

**Table 64: Biochemical analysis of bispecific constructs with a bivalent binding to 4-1BB and a monovalent binding to FAP (2+1 4-1BB/FAP human IgG1 P329GLALA)**

| **Clone** | **Yield [mg/l]** | **Monomer [%]** | **CE-SDS (nonred)** |
|---|---|---|---|
| **2+1 25G7/FAP (hole-VH)** | 25.6 | 96.7 | 95.4 |
| **2+1 11D5/FAP (hole-VH)** | 6.3 | 97 | 89.2 |

### 9.4 Preparation, purification and characterization of FAP antigens as screening tools

In order to test the binding to FAP, DNA sequences encoding the ectodomains of human, mouse or cynomolgus FAP fused to a C-terminal HisTag were cloned in an expression vector containing a chimeric MPSV promoter consisting of the MPSV core promoter combined with the CMV promoter enhancer fragment. The expression vector also contains the oriP region for episomal replication in EBNA (Epstein Barr Virus Nuclear Antigen) containing host cells. The amino acid and nucleotide sequences of a His-tagged human FAP ECD is shown in SEQ ID NOs 85 and 86, respectively. SEQ ID NOs 88 and 89 show the amino acid and nucleotide sequences, respectively, of a His-tagged mouse FAP ECD. SEQ ID NOs 90 and 91 show the amino acid and nucleotide sequences, respectively, of a His-tagged cynomolgus FAP ECD.

The FAP antigens were produced by co-transfecting HEK293-EBNA cells with the mammalian expression vectors using polyethylenimine (PEI; Polysciences Inc.).

For a 200 mL production in 500 mL shake flasks, 300 million HEK293 EBNA cells were seeded 24 hours before transfection in 100% F17 + 6mM Glutamine. For transfection, 400 million cells were centrifuged for 5 minutes at 210 x g, and supernatant was replaced by 20 mL pre-warmed CD-CHO medium (Gibco). Expression vectors were mixed in 20 mL CD-CHO medium to a final amount of 200 µg DNA. After addition of 540 µL PEI (1 mg/mL) (Polysciences Inc.), the solution was vortexed for 15 seconds and incubated for 10 minutes at room temperature. Afterwards, resuspended cells were mixed with the DNA/PEI solution, transferred to a 500 mL shake flask and incubated for 3 hours at 37°C in an incubator with a 5% CO2 atmosphere and shaking at 165 rpm. After the incubation, 160 mL F17 medium and supplements (1mM valproic acid, 5g/l Pepsoy and 6 mM L-Glutamine) were added and cells cultivated for 24 hours. 24h after transfection the cells were then supplement with an amino acid and glucose feed at 12% final volume (24 mL). After cultivation for 7 days, the cell supernatant was collected by centrifugation for 45 minutes at 2000-3000 x g. The solution was sterile filtered (0.22 µm filter), supplemented with sodium azide to a final concentration of 0.01 % (w/v), and kept at 4°C.

Purification of the antigens from cell culture supernatants was carried out in a two step purification with first an affinity chromatography step using either a 5 ml IMAC column (Roche) or a 5 ml NiNTA column (Qiagen) followed by a size exclusion chromatography using a HiLoad Superdex 200 column (GE Healthcare) equilibrated with 20mM Histidine, 140mM NaCl, pH6.0 or 2mM MOPS 150 mM NaCl 0.02% NaN3 pH 7.3, respectively.

For affinity chromatography, using the IMAC column (Roche), equilibration was performed with 25 mM Tris-HCl, 500 mM sodium Chloride, 20 mM imidazole, pH8.0 for 8 CVs. The supernatant was loaded and unbound protein washed out by washing with 10 CVs of 25 mM Tris-HCl, 500 mM sodium Chloride, 20 mM imidazole, pH8.0. The bound protein was eluted using a linear gradient of 20 CVs (from 0-100% ) of 25 mM Tris-HCl, 500 mM sodium Chloride, 500 mM imidazole, pH8.0 followed by a step at 100% for 8 CVs.

For the affinity chromatography using the NiNTA column (Qiagen), equilibration was performed with 50 mM Sodium Phosphate, 300 mM Sodium Chloride, pH8.0 for 8 CVs. The supernatant was loaded and unbound protein was removed by washing with 10 column volumes of 50 mM Sodium Phosphate, 300 mM Sodium Chloride, pH8.0. The bound protein was eluted using a linear gradient of 20 CVs (from 0 to 100%) of 50 mM Sodium Phosphate, 300 mM Sodium Chloride, 500 mM imidazole, pH7.4 followed by a step of 5CVs of 50 mM Sodium Phosphate, 300 mM Sodium Chloride, 500 mM imidazole, pH7.4. The column was then reequilibrated with 8 CVs of 50 mM Sodium Phosphate, 300 mM Sodium Chloride, pH8.0.

The collected fractions were then supplemented with 1/10 (v/v) of 0.5 M EDTA, pH8.0. The protein was concentrated in Vivaspin columns (30 kD cut off, Sartorius) and filtered prior to loading on a HiLoad Superdex 200 column (GE Healthcare) equilibrated with 2mM MOPS 150 mM NaCl 0.02% NaN₃ pH 7.3 or 20mM Histidine, 140mM NaCl, pH6.0.

The protein concentration of purified antigens was determined by measuring the OD at 280 nm, using the molar extinction coefficient calculated on the basis of the amino acid sequence. Purity and molecular weight of the antigens were analyzed by CE-SDS in the presence and absence of a reducing agent (Invitrogen) using a LabChipGXII (Caliper). The aggregate content of the antigens was analyzed using a TSKgel G3000 SW XL analytical size-exclusion column (Tosoh) equilibrated in a 25 mM potassiumphosphate, 125 mM sodium chloride, 200 mM L-Arginine Monohydrocloride, 0.02 % (w/v) NaN₃, pH 6.7 running buffer at 25°C.

### 9.5 Generation of bispecific tetravalent antigen binding molecules targeting 4-1BB and fibroblast activation protein (FAP) (4+1 format)

Bispecific agonistic 4-1BB antibodies with tetravalent binding for 4-1BB and monovalent binding for FAP, also termed 4+1 bispecific antigen binding molecules, were prepared as depicted in **Figures 40E and 40F****.**

In this example the HC1 of the construct was comprised of the following components, VHCH1_VHCH1 of an anti-4-1BB followed by Fc hole, at which C-terminus a VL or VH, respectively, of an anti-FAP binder (clone 4B9) was fused. HC2 was comprised of VHCH1_VHCH1 of anti-4-1BB followed by Fc knob, at which C-terminus a VH or VL, of the anti-FAP binder was fused.

Binders against 4-1BB, 12B3, 9B11, 11D5 and 25G7, were generated as described in Example 6. The generation and preparation of the FAP binders is described in WO 2012/020006 A2.

Combination of the targeted anti-FAP-Fc knob with the anti-4-1BB-Fc hole chain allows generation of a heterodimer, which includes a FAP binding moiety and four 4-1BB binding Fabs. The Pro329Gly, Leu234Ala and Leu235Ala mutations were introduced in the constant region of the heavy chains to abrogate binding to Fc gamma receptors according to the method described in International Patent Appl. Publ. No. WO 2012/130831 A1. The heavy chain fusion proteins were co-expressed with the light chain of the anti-4-1BB binder (CLVL). The resulting bispecific, tetravalent construct is depicted in **Figures 40E and 40F** and the nucleotide and amino acid sequences can be found in **Table 65.**

The bispecific 4+1 anti-4-1BB anti-FAP huIgG1 P329GLALA were produced by co-transfecting HEK293-EBNA cells with the mammalian expression vectors using polyethylenimine. The cells were transfected with the corresponding expression vectors in a 1:1:1 ratio ("vector knob heavy chain" : "vector light chain" : "vector hole heavy chain"). The 4+1 bispecific antigen binding molecules were produced and purified as described for the bispecific bivalent anti-4-1BB and anti-FAP huIgG1 P329GLALA (see Example 9.1).

In addition, an "untargeted" 4+1 construct was prepared, wherein the VH and VL domain of the anti-FAP binder were replaced by a germline control, termed DP47, not binding to the antigen.

**Table 65: cDNA and amino acid sequences of mature bispecific 4+1 anti-4-1BB, anti-FAP human IgG1 P329GLALA kih antibodies (constructs with a-FAP VH fused to knob and VL fused to hole chain, termed below hole-VL)t**

| SEQ ID NO: | Description | Sequence |
|---|---|---|
| 287 | (12B3) VLCL-light chain (nucleotide sequence) | see Table 48 |
| 325 | HC 1 (12B3) VHCH1 VHCH1 Fc knob VH (4B9) (nucleotide sequence) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTACTGTGCCAGAAGCG AGTTCCGGTTCTACGCCGACTTCGACTACTGGGGCCAG GGCACCACCGTGACCGTGTCTAGCGCTTCTACCAAGGG CCCCAGCGTGTTCCCTCTGGCCCCTAGCAGCAAGAGCA CATCTGGCGGAACAGCCGCCCTGGGCTGCCTCGTGAAG GACTACTTTCCCGAGCCCGTGACAGTGTCCTGGAACTC TGGCGCCCTGACAAGCGGCGTGCACACCTTTCCAGCCG TGCTGCAGAGCAGCGGCCTGTACTCTCTGAGCAGCGTC GTGACTGTGCCCAGCAGCAGCCTGGGAACCCAGACCT ACATCTGCAACGTGAACCACAAGCCCAGCAACACCAA GGTGGACAAGAAGGTGGAACCCAAGAGCTGCGACGGC GGAGGCGGATCTGGCGGCGGAGGATCCCAGGTGCAGC TGGTGCAGAGCGGAGCTGAAGTGAAAAAGCCTGGCTC CTCCGTGAAAGTGTCTTGTAAAGCCAGCGGCGGCACAT TCTCATCCTACGCCATCAGCTGGGTGCGGCAGGCTCCA GGCCAGGGACTGGAATGGATGGGAGGAATTATCCCTA TTTTTGGGACAGCCAATTATGCTCAGAAATTTCAGGGG CGCGTGACAATTACAGCCGACAAGTCCACCTCTACAGC TTATATGGAACTGTCCTCCCTGCGCTCCGAGGATACAG CTGTGTATTACTGCGCTCGGAGCGAGTTTAGATTCTAT GCCGATTTTGATTATTGGGGGCAGGGAACAACAGTGA CTGTGTCCTCCGCTAGCACCAAGGGCCCATCGGTCTTC CCCCTGGCACCCTCCTCCAAGAGCACCTCTGGGGGCAC AGCGGCCCTGGGCTGCCTGGTCAAGGACTACTTCCCCG AACCGGTGACGGTGTCGTGGAACTCAGGCGCCCTGAC CAGCGGCGTGCACACCTTCCCGGCTGTCCTACAGTCCT CAGGACTCTACTCCCTCAGCAGCGTGGTGACCGTGCCC TCCAGCAGCTTGGGCACCCAGACCTACATCTGCAACGT GAATCACAAGCCCAGCAACACCAAGGTGGACAAGAAA GTTGAGCCCAAATCTTGTGACAAAACTCACACATGCCC ACCGTGCCCAGCACCTGAAGCTGCAGGGGGACCGTCA GTCTTCCTCTTCCCCCCAAAACCCAAGGACACCCTCAT GATCTCCCGGACCCCTGAGGTCACATGCGTGGTGGTGG ACGTGAGCCACGAAGACCCTGAGGTCAAGTTCAACTG GTACGTGGACGGCGTGGAGGTGCATAATGCCAAGACA AAGCCGCGGGAGGAGCAGTACAACAGCACGTACCGTG TGGTCAGCGTCCTCACCGTCCTGCACCAGGACTGGCTG |
| | | AATGGCAAGGAGTACAAGTGCAAGGTCTCCAACAAAG CCCTCGGCGCCCCCATCGAGAAAACCATCTCCAAAGCC AAAGGGCAGCCCCGAGAACCACAGGTGTACACCCTGC CCCCCTGCAGAGATGAGCTGACCAAGAACCAGGTGTC CCTGTGGTGTCTGGTCAAGGGCTTCTACCCCAGCGATA TCGCCGTGGAGTGGGAGAGCAACGGCCAGCCTGAGAA CAACTACAAGACCACCCCCCCTGTGCTGGACAGCGAC GGCAGCTTCTTCCTGTACTCCAAACTGACCGTGGACAA GAGCCGGTGGCAGCAGGGCAACGTGTTCAGCTGCAGC GTGATGCACGAGGCCCTGCACAACCACTACACCCAGA AGTCCCTGAGCCTGAGCCCCGGCGGAGGCGGCGGAAG CGGAGGAGGAGGATCTGGGGGCGGAGGTTCCGGAGGC GGAGGATCCGAGGTGCAGCTGCTCGAAAGCGGCGGAG GACTGGTGCAGCCTGGCGGCAGCCTGAGACTGTCTTGC GCCGCCAGCGGCTTCACCTTCAGCAGCTACGCCATGAG CTGGGTCCGCCAGGCCCCTGGCAAGGGACTGGAATGG GTGTCCGCCATCATCGGCTCTGGCGCCAGCACCTACTA CGCCGACAGCGTGAAGGGCCGGTTCACCATCAGCCGG GACAACAGCAAGAACACCCTGTACCTGCAGATGAACA GCCTGCGGGCCGAGGACACCGCCGTGTACTACTGCGCC AAGGGATGGTTCGGCGGCTTCAACTACTGGGGACAGG GCACCCTGGTCACCGTGTCCAGC |
| 326 | HC 2 (12B3) VHCH1 VHCH1 Fc hole VL (4B9) (nucleotide sequence) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTACTGTGCCAGAAGCG AGTTCCGGTTCTACGCCGACTTCGACTACTGGGGCCAG GGCACCACCGTGACCGTGTCTAGCGCTTCTACCAAGGG CCCCAGCGTGTTCCCTCTGGCCCCTAGCAGCAAGAGCA CATCTGGCGGAACAGCCGCCCTGGGCTGCCTCGTGAAG GACTACTTTCCCGAGCCCGTGACAGTGTCCTGGAACTC TGGCGCCCTGACAAGCGGCGTGCACACCTTTCCAGCCG TGCTGCAGAGCAGCGGCCTGTACTCTCTGAGCAGCGTC GTGACTGTGCCCAGCAGCAGCCTGGGAACCCAGACCT ACATCTGCAACGTGAACCACAAGCCCAGCAACACCAA GGTGGACAAGAAGGTGGAACCCAAGAGCTGCGACGGC GGAGGCGGATCTGGCGGCGGAGGATCCCAGGTGCAGC TGGTGCAGAGCGGAGCTGAAGTGAAAAAGCCTGGCTC CTCCGTGAAAGTGTCTTGTAAAGCCAGCGGCGGCACAT TCTCATCCTACGCCATCAGCTGGGTGCGGCAGGCTCCA GGCCAGGGACTGGAATGGATGGGAGGAATTATCCCTA TTTTTGGGACAGCCAATTATGCTCAGAAATTTCAGGGG CGCGTGACAATTACAGCCGACAAGTCCACCTCTACAGC TTATATGGAACTGTCCTCCCTGCGCTCCGAGGATACAG CTGTGTATTACTGCGCTCGGAGCGAGTTTAGATTCTAT GCCGATTTTGATTATTGGGGGCAGGGAACAACAGTGA CTGTGTCCTCCGCTAGCACCAAGGGCCCATCGGTCTTC CCCCTGGCACCCTCCTCCAAGAGCACCTCTGGGGGCAC AGCGGCCCTGGGCTGCCTGGTCAAGGACTACTTCCCCG AACCGGTGACGGTGTCGTGGAACTCAGGCGCCCTGAC |
| | | CAGCGGCGTGCACACCTTCCCGGCTGTCCTACAGTCCT CAGGACTCTACTCCCTCAGCAGCGTGGTGACCGTGCCC TCCAGCAGCTTGGGCACCCAGACCTACATCTGCAACGT GAATCACAAGCCCAGCAACACCAAGGTGGACAAGAAA GTTGAGCCCAAATCTTGTGACAAAACTCACACATGCCC ACCGTGCCCAGCACCTGAAGCTGCAGGGGGACCGTCA GTCTTCCTCTTCCCCCCAAAACCCAAGGACACCCTCAT GATCTCCCGGACCCCTGAGGTCACATGCGTGGTGGTGG ACGTGAGCCACGAAGACCCTGAGGTCAAGTTCAACTG GTACGTGGACGGCGTGGAGGTGCATAATGCCAAGACA AAGCCGCGGGAGGAGCAGTACAACAGCACGTACCGTG TGGTCAGCGTCCTCACCGTCCTGCACCAGGACTGGCTG AATGGCAAGGAGTACAAGTGCAAGGTCTCCAACAAAG CCCTCGGCGCCCCCATCGAGAAAACCATCTCCAAAGCC AAAGGGCAGCCCCGAGAACCACAGGTGTACACCCTGC CCCCATCCCGGGATGAGCTGACCAAGAACCAGGTCAG CCTCTCGTGCGCAGTCAAAGGCTTCTATCCCAGCGACA TCGCCGTGGAGTGGGAGAGCAATGGGCAGCCGGAGAA CAACTACAAGACCACGCCTCCCGTGCTGGACTCCGACG GCTCCTTCTTCCTCGTGAGCAAGCTCACCGTGGACAAG AGCAGGTGGCAGCAGGGGAACGTCTTCTCATGCTCCGT GATGCATGAGGCTCTGCACAACCACTACACGCAGAAG AGCCTCTCCCTGTCTCCGGGTGGAGGCGGCGGAAGCG GAGGAGGAGGATCCGGCGGCGGAGGTTCCGGAGGCGG TGGATCTGAGATCGTGCTGACCCAGTCTCCCGGCACCC TGTCTCTGAGCCCTGGCGAGAGAGCCACCCTGTCCTGC AGAGCCTCCCAGTCCGTGACCTCCTCCTACCTCGCCTG GTATCAGCAGAAGCCCGGCCAGGCCCCTCGGCTGCTG ATCAACGTGGGCAGTCGGAGAGCCACCGGCATCCCTG ACCGGTTCTCCGGCTCTGGCTCCGGCACCGACTTCACC CTGACCATCTCCCGGCTGGAACCCGAGGACTTCGCCGT GTACTACTGCCAGCAGGGCATCATGCTGCCCCCCACCT TTGGCCAGGGCACCAAGGTGGAAATCAAG |
| 289 | (12B3) VLCL-light chain | see Table 48 |
| 327 | HC 1 (12B3) VHCH1 VHCH1 Fc knob VH (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSEFRFYADFDYWGQGTTVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDGGGGSGGGGSQ VQLVQSGAEVKKPGSSVKVSCKASGGTFSSYAISWVRQA PGQGLEWMGGIIPIFGTANYAQKFQGRVTITADKSTSTAY MELSSLRSEDTAVYYCARSEFRFYADFDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPEAA GGPSVFLFPPKPKDTLMISRTPEVTCVVVDVSHEDPEVKF NWYVDGVEVHNAKTKPREEQYNSTYRVVSVLTVLHQD WLNGKEYKCKVSNKALGAPIEKTISKAKGQPREPQVYTL PPCRDELTKNQVSLWCLVKGFYPSDIAVEWESNGQPENN YKTTPPVLDSDGSFFLYSKLTVDKSRWQQGNVFSCSVMH EALHNHYTQKSLSLSPGGGGGSGGGGSGGGGSGGGGSE VQLLESGGGLVQPGGSLRLSCAASGFTFSSYAMSWVRQA |
| | | PGKGLEWVSAIIGSGASTYYADSVKGRFTISRDNSKNTLY LQMNSLRAEDTAVYYCAKGWFGGFNYWGQGTLVTVSS |
| 328 | HC 2 (12B3) VHCH1 VHCH1 Fc hole VL (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSEFRFYADFDYWGQGTTVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDGGGGSGGGGSQ VQLVQSGAEVKKPGSSVKVSCKASGGTFSSYAISWVRQA PGQGLEWMGGIIPIFGTANYAQKFQGRVTITADKSTSTAY MELSSLRSEDTAVYYCARSEFRFYADFDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPEAA GGPSVFLFPPKPKDTLMISRTPEVTCVVVDVSHEDPEVKF NWYVDGVEVHNAKTKPREEQYNSTYRVVSVLTVLHQD WLNGKEYKCKVSNKALGAPIEKTISKAKGQPREPQVCTL PPSRDELTKNQVSLSCAVKGFYPSDIAVEWESNGQPENN YKTTPPVLDSDGSFFLVSKLTVDKSRWQQGNVFSCSVMH EALHNHYTQKSLSLSPGGGGGSGGGGSGGGGSGGGGSEI VLTQSPGTLSLSPGERATLSCRASQSVTSSYLAWYQQKPG QAPRLLINVGSRRATGIPDRFSGSGSGTDFTLTISRLEPEDF AVYYCQQGIMLPPTFGQGTKVEIK |
| 291 | (25G7) VLCL-light chain (nucleotide sequence) | see Table 48 |
| 329 | HC 1 (25G7) VHCH1 VHCH1 Fc knob VH (4B9) (nucleotide sequence) | GAGGTGCAGCTGCTGGAATCTGGCGGCGGACTGGTGC AGCCTGGCGGATCTCTGAGACTGAGCTGTGCCGCCAGC GGCTTCACCTTCAGCAGCTACGCCATGAGCTGGGTGCG CCAGGCCCCTGGAAAAGGCCTGGAATGGGTGTCCGCC ATCTCTGGCAGCGGCGGCAGCACCTACTACGCCGATTC TGTGAAGGGCCGGTTCACCATCAGCCGGGACAACAGC AAGAACACCCTGTACCTGCAGATGAACAGCCTGCGGG CCGAGGACACCGCCGTGTACTATTGCGCCAGGGACGA CCCCTGGCCCCCCTTTGATTATTGGGGACAGGGCACCC TCGTGACCGTGTCCAGCGCTTCTACCAAGGGCCCCAGC GTGTTCCCTCTGGCCCCTAGCAGCAAGAGCACATCTGG CGGAACAGCCGCCCTGGGCTGCCTCGTGAAGGACTACT TTCCCGAGCCCGTGACAGTGTCCTGGAACTCTGGCGCC CTGACAAGCGGCGTGCACACCTTTCCAGCCGTGCTGCA GAGCAGCGGCCTGTACTCTCTGAGCAGCGTCGTGACTG TGCCCAGCAGCAGCCTGGGAACCCAGACCTACATCTGC AACGTGAACCACAAGCCCAGCAACACCAAGGTGGACA AGAAGGTGGAACCCAAGAGCTGCGACGGCGGAGGCGG ATCTGGCGGCGGAGGATCCGAAGTGCAGCTGCTGGAA AGTGGGGGAGGCCTGGTGCAGCCAGGGGGAAGCCTGA GACTGTCTTGTGCCGCTTCCGGCTTTACCTTTAGCTCTT ACGCCATGTCTTGGGTGCGGCAGGCTCCAGGCAAGGG ACTGGAATGGGTGTCAGCTATCAGCGGCTCTGGCGGCT CCACATATTACGCCGACAGCGTGAAGGGCAGATTCAC AATCTCCAGAGACAACTCCAAGAATACTCTGTACCTGC AGATGAATTCCCTGCGCGCCGAAGATACAGCTGTGTAT TACTGTGCCCGCGACGATCCTTGGCCCCCTTTCGACTA CTGGGGGCAGGGAACACTCGTGACAGTGTCATCCGCT |
| | | AGCACCAAGGGCCCATCGGTCTTCCCCCTGGCACCCTC CTCCAAGAGCACCTCTGGGGGCACAGCGGCCCTGGGC TGCCTGGTCAAGGACTACTTCCCCGAACCGGTGACGGT GTCGTGGAACTCAGGCGCCCTGACCAGCGGCGTGCAC ACCTTCCCGGCTGTCCTACAGTCCTCAGGACTCTACTC CCTCAGCAGCGTGGTGACCGTGCCCTCCAGCAGCTTGG GCACCCAGACCTACATCTGCAACGTGAATCACAAGCCC AGCAACACCAAGGTGGACAAGAAAGTTGAGCCCAAAT CTTGTGACAAAACTCACACATGCCCACCGTGCCCAGCA CCTGAAGCTGCAGGGGGACCGTCAGTCTTCCTCTTCCC CCCAAAACCCAAGGACACCCTCATGATCTCCCGGACCC CTGAGGTCACATGCGTGGTGGTGGACGTGAGCCACGA AGACCCTGAGGTCAAGTTCAACTGGTACGTGGACGGC GTGGAGGTGCATAATGCCAAGACAAAGCCGCGGGAGG AGCAGTACAACAGCACGTACCGTGTGGTCAGCGTCCTC ACCGTCCTGCACCAGGACTGGCTGAATGGCAAGGAGT ACAAGTGCAAGGTCTCCAACAAAGCCCTCGGCGCCCC CATCGAGAAAACCATCTCCAAAGCCAAAGGGCAGCCC CGAGAACCACAGGTGTACACCCTGCCCCCCTGCAGAG ATGAGCTGACCAAGAACCAGGTGTCCCTGTGGTGTCTG GTCAAGGGCTTCTACCCCAGCGATATCGCCGTGGAGTG GGAGAGCAACGGCCAGCCTGAGAACAACTACAAGACC ACCCCCCCTGTGCTGGACAGCGACGGCAGCTTCTTCCT GTACTCCAAACTGACCGTGGACAAGAGCCGGTGGCAG CAGGGCAACGTGTTCAGCTGCAGCGTGATGCACGAGG CCCTGCACAACCACTACACCCAGAAGTCCCTGAGCCTG AGCCCCGGCGGAGGCGGCGGAAGCGGAGGAGGAGGA TCTGGGGGCGGAGGTTCCGGAGGCGGAGGATCCGAGG TGCAGCTGCTCGAAAGCGGCGGAGGACTGGTGCAGCC TGGCGGCAGCCTGAGACTGTCTTGCGCCGCCAGCGGCT TCACCTTCAGCAGCTACGCCATGAGCTGGGTCCGCCAG GCCCCTGGCAAGGGACTGGAATGGGTGTCCGCCATCAT CGGCTCTGGCGCCAGCACCTACTACGCCGACAGCGTGA AGGGCCGGTTCACCATCAGCCGGGACAACAGCAAGAA CACCCTGTACCTGCAGATGAACAGCCTGCGGGCCGAG GACACCGCCGTGTACTACTGCGCCAAGGGATGGTTCGG CGGCTTCAACTACTGGGGACAGGGCACCCTGGTCACCG TGTCCAGC |
| 330 | HC 2 (25G7) VHCH1 VHCH1 Fc hole VL (4B9) (nucleotide sequence) | GAGGTGCAGCTGCTGGAATCTGGCGGCGGACTGGTGC AGCCTGGCGGATCTCTGAGACTGAGCTGTGCCGCCAGC GGCTTCACCTTCAGCAGCTACGCCATGAGCTGGGTGCG CCAGGCCCCTGGAAAAGGCCTGGAATGGGTGTCCGCC ATCTCTGGCAGCGGCGGCAGCACCTACTACGCCGATTC TGTGAAGGGCCGGTTCACCATCAGCCGGGACAACAGC AAGAACACCCTGTACCTGCAGATGAACAGCCTGCGGG CCGAGGACACCGCCGTGTACTATTGCGCCAGGGACGA CCCCTGGCCCCCCTTTGATTATTGGGGACAGGGCACCC TCGTGACCGTGTCCAGCGCTTCTACCAAGGGCCCCAGC GTGTTCCCTCTGGCCCCTAGCAGCAAGAGCACATCTGG CGGAACAGCCGCCCTGGGCTGCCTCGTGAAGGACTACT TTCCCGAGCCCGTGACAGTGTCCTGGAACTCTGGCGCC CTGACAAGCGGCGTGCACACCTTTCCAGCCGTGCTGCA GAGCAGCGGCCTGTACTCTCTGAGCAGCGTCGTGACTG TGCCCAGCAGCAGCCTGGGAACCCAGACCTACATCTGC |
| | | AACGTGAACCACAAGCCCAGCAACACCAAGGTGGACA AGAAGGTGGAACCCAAGAGCTGCGACGGCGGAGGCGG ATCTGGCGGCGGAGGATCCGAAGTGCAGCTGCTGGAA AGTGGGGGAGGCCTGGTGCAGCCAGGGGGAAGCCTGA GACTGTCTTGTGCCGCTTCCGGCTTTACCTTTAGCTCTT ACGCCATGTCTTGGGTGCGGCAGGCTCCAGGCAAGGG ACTGGAATGGGTGTCAGCTATCAGCGGCTCTGGCGGCT CCACATATTACGCCGACAGCGTGAAGGGCAGATTCAC AATCTCCAGAGACAACTCCAAGAATACTCTGTACCTGC AGATGAATTCCCTGCGCGCCGAAGATACAGCTGTGTAT TACTGTGCCCGCGACGATCCTTGGCCCCCTTTCGACTA CTGGGGGCAGGGAACACTCGTGACAGTGTCATCCGCT AGCACCAAGGGCCCATCGGTCTTCCCCCTGGCACCCTC CTCCAAGAGCACCTCTGGGGGCACAGCGGCCCTGGGC TGCCTGGTCAAGGACTACTTCCCCGAACCGGTGACGGT GTCGTGGAACTCAGGCGCCCTGACCAGCGGCGTGCAC ACCTTCCCGGCTGTCCTACAGTCCTCAGGACTCTACTC CCTCAGCAGCGTGGTGACCGTGCCCTCCAGCAGCTTGG GCACCCAGACCTACATCTGCAACGTGAATCACAAGCCC AGCAACACCAAGGTGGACAAGAAAGTTGAGCCCAAAT CTTGTGACAAAACTCACACATGCCCACCGTGCCCAGCA CCTGAAGCTGCAGGGGGACCGTCAGTCTTCCTCTTCCC CCCAAAACCCAAGGACACCCTCATGATCTCCCGGACCC CTGAGGTCACATGCGTGGTGGTGGACGTGAGCCACGA AGACCCTGAGGTCAAGTTCAACTGGTACGTGGACGGC GTGGAGGTGCATAATGCCAAGACAAAGCCGCGGGAGG AGCAGTACAACAGCACGTACCGTGTGGTCAGCGTCCTC ACCGTCCTGCACCAGGACTGGCTGAATGGCAAGGAGT ACAAGTGCAAGGTCTCCAACAAAGCCCTCGGCGCCCC CATCGAGAAAACCATCTCCAAAGCCAAAGGGCAGCCC CGAGAACCACAGGTGTGCACCCTGCCCCCATCCCGGG ATGAGCTGACCAAGAACCAGGTCAGCCTCTCGTGCGC AGTCAAAGGCTTCTATCCCAGCGACATCGCCGTGGAGT GGGAGAGCAATGGGCAGCCGGAGAACAACTACAAGAC CACGCCTCCCGTGCTGGACTCCGACGGCTCCTTCTTCCT CGTGAGCAAGCTCACCGTGGACAAGAGCAGGTGGCAG CAGGGGAACGTCTTCTCATGCTCCGTGATGCATGAGGC TCTGCACAACCACTACACGCAGAAGAGCCTCTCCCTGT CTCCGGGTGGAGGCGGCGGAAGCGGAGGAGGAGGATC CGGCGGCGGAGGTTCCGGAGGCGGTGGATCTGAGATC GTGCTGACCCAGTCTCCCGGCACCCTGTCTCTGAGCCC TGGCGAGAGAGCCACCCTGTCCTGCAGAGCCTCCCAGT CCGTGACCTCCTCCTACCTCGCCTGGTATCAGCAGAAG CCCGGCCAGGCCCCTCGGCTGCTGATCAACGTGGGCAG TCGGAGAGCCACCGGCATCCCTGACCGGTTCTCCGGCT CTGGCTCCGGCACCGACTTCACCCTGACCATCTCCCGG CTGGAACCCGAGGACTTCGCCGTGTACTACTGCCAGCA GGGCATCATGCTGCCCCCCACCTTTGGCCAGGGCACCA AGGTGGAAATCAAG |
| 293 | (25G7) VLCL-light chain | see Table 48 |
| 331 | HC 1 (25G7) VHCH1_VHCH1 Fc knob VH (4B9) | EVQLLESGGGLVQPGGSLRLSCAASGFTFSSYAMSWVRQ APGKGLEWVSAISGSGGSTYYADSVKGRFTISRDNSKNTL YLQMNSLRAEDTAVYYCARDDPWPPFDYWGQGTLVTV |
| | | SSASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDGGGGSGGGGSEV QLLESGGGLVQPGGSLRLSCAASGFTFSSYAMSWVRQAP GKGLEWVSAISGSGGSTYYADSVKGRFTISRDNSKNTLY LQMNSLRAEDTAVYYCARDDPWPPFDYWGQGTLVTVSS ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALGAPIEKTISKAKGQPREPQVYTLPP CRDELTKNQVSLWCLVKGFYPSDIAVEWESNGQPENNY KTTPPVLDSDGSFFLYSKLTVDKSRWQQGNVFSCSVMHE ALHNHYTQKSLSLSPGGGGGSGGGGSGGGGSGGGGSEV QLLESGGGLVQPGGSLRLSCAASGFTFSSYAMSWVRQAP GKGLEWVSAIIGSGASTYYADSVKGRFTISRDNSKNTLYL QMNSLRAEDTAVYYCAKGWFGGFNYWGQGTLVTVSS |
| 332 | HC 2 (25G7) VHCH1 VHCH1 Fc hole VL (4B9) | EVQLLESGGGLVQPGGSLRLSCAASGFTFSSYAMSWVRQ APGKGLEWVSAISGSGGSTYYADSVKGRFTISRDNSKNTL YLQMNSLRAEDTAVYYCARDDPWPPFDYWGQGTLVTV SSASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDGGGGSGGGGSEV QLLESGGGLVQPGGSLRLSCAASGFTFSSYAMSWVRQAP GKGLEWVSAISGSGGSTYYADSVKGRFTISRDNSKNTLY LQMNSLRAEDTAVYYCARDDPWPPFDYWGQGTLVTVSS ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALGAPIEKTISKAKGQPREPQVCTLPP SRDELTKNQVSLSCAVKGFYPSDIAVEWESNGQPENNYK TTPPVLDSDGSFFLVSKLTVDKSRWQQGNVFSCSVMHEA LHNHYTQKSLSLSPGGGGGSGGGGSGGGGSGGGGSEIVL TQSPGTLSLSPGERATLSCRASQSVTSSYLAWYQQKPGQ APRLLINVGSRRATGIPDRFSGSGSGTDFTLTISRLEPEDFA VYYCQQGIMLPPTFGQGTKVEIK |
| 295 | (11D5) VLCL-light chain (nucleotide sequence) | see Table 48 |
| 333 | HC 1 (11D5) VHCH1 VHCH1 Fc knob VH (4B9) (nucleotide sequence) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTACTGTGCCAGAAGCA CCCTGATCTACGGCTACTTCGACTACTGGGGCCAGGGC ACCACCGTGACCGTGTCTAGCGCTTCTACCAAGGGCCC CAGCGTGTTCCCTCTGGCCCCTAGCAGCAAGAGCACAT CTGGCGGAACAGCCGCCCTGGGCTGCCTCGTGAAGGA |
| | | CTACTTTCCCGAGCCCGTGACAGTGTCCTGGAACTCTG GCGCCCTGACAAGCGGCGTGCACACCTTTCCAGCCGTG CTGCAGAGCAGCGGCCTGTACTCTCTGAGCAGCGTCGT GACTGTGCCCAGCAGCAGCCTGGGAACCCAGACCTAC ATCTGCAACGTGAACCACAAGCCCAGCAACACCAAGG TGGACAAGAAGGTGGAACCCAAGAGCTGCGACGGCGG AGGCGGATCTGGCGGCGGAGGATCCCAGGTGCAGCTG GTGCAGAGCGGAGCTGAAGTGAAAAAGCCTGGCTCCT CCGTGAAAGTGTCTTGTAAAGCCAGCGGCGGCACATTC TCATCCTACGCCATCAGCTGGGTGCGGCAGGCTCCAGG CCAGGGACTGGAATGGATGGGAGGAATTATCCCTATTT TTGGGACAGCCAATTATGCTCAGAAATTTCAGGGGCGC GTGACAATTACAGCCGACAAGTCCACCTCTACAGCTTA TATGGAACTGTCCTCCCTGCGCTCCGAGGATACAGCTG TGTATTACTGCGCCCGGTCCACACTGATCTATGGATAC TTTGATTATTGGGGGCAGGGAACAACAGTGACTGTGTC CTCCGCTAGCACCAAGGGCCCATCGGTCTTCCCCCTGG CACCCTCCTCCAAGAGCACCTCTGGGGGCACAGCGGCC CTGGGCTGCCTGGTCAAGGACTACTTCCCCGAACCGGT GACGGTGTCGTGGAACTCAGGCGCCCTGACCAGCGGC GTGCACACCTTCCCGGCTGTCCTACAGTCCTCAGGACT CTACTCCCTCAGCAGCGTGGTGACCGTGCCCTCCAGCA GCTTGGGCACCCAGACCTACATCTGCAACGTGAATCAC AAGCCCAGCAACACCAAGGTGGACAAGAAAGTTGAGC CCAAATCTTGTGACAAAACTCACACATGCCCACCGTGC CCAGCACCTGAAGCTGCAGGGGGACCGTCAGTCTTCCT CTTCCCCCCAAAACCCAAGGACACCCTCATGATCTCCC GGACCCCTGAGGTCACATGCGTGGTGGTGGACGTGAG CCACGAAGACCCTGAGGTCAAGTTCAACTGGTACGTG GACGGCGTGGAGGTGCATAATGCCAAGACAAAGCCGC GGGAGGAGCAGTACAACAGCACGTACCGTGTGGTCAG CGTCCTCACCGTCCTGCACCAGGACTGGCTGAATGGCA AGGAGTACAAGTGCAAGGTCTCCAACAAAGCCCTCGG CGCCCCCATCGAGAAAACCATCTCCAAAGCCAAAGGG CAGCCCCGAGAACCACAGGTGTACACCCTGCCCCCCTG CAGAGATGAGCTGACCAAGAACCAGGTGTCCCTGTGG TGTCTGGTCAAGGGCTTCTACCCCAGCGATATCGCCGT GGAGTGGGAGAGCAACGGCCAGCCTGAGAACAACTAC AAGACCACCCCCCCTGTGCTGGACAGCGACGGCAGCTT CTTCCTGTACTCCAAACTGACCGTGGACAAGAGCCGGT GGCAGCAGGGCAACGTGTTCAGCTGCAGCGTGATGCA CGAGGCCCTGCACAACCACTACACCCAGAAGTCCCTG AGCCTGAGCCCCGGCGGAGGCGGCGGAAGCGGAGGAG GAGGATCTGGGGGCGGAGGTTCCGGAGGCGGAGGATC CGAGGTGCAGCTGCTCGAAAGCGGCGGAGGACTGGTG CAGCCTGGCGGCAGCCTGAGACTGTCTTGCGCCGCCAG CGGCTTCACCTTCAGCAGCTACGCCATGAGCTGGGTCC GCCAGGCCCCTGGCAAGGGACTGGAATGGGTGTCCGC CATCATCGGCTCTGGCGCCAGCACCTACTACGCCGACA GCGTGAAGGGCCGGTTCACCATCAGCCGGGACAACAG CAAGAACACCCTGTACCTGCAGATGAACAGCCTGCGG GCCGAGGACACCGCCGTGTACTACTGCGCCAAGGGAT GGTTCGGCGGCTTCAACTACTGGGGACAGGGCACCCTG GTCACCGTGTCCAGC |
| 334 | HC 2 (11D5) VHCH1 VHCH1 Fc hole VL (4B9) (nucleotide sequence) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTACTGTGCCAGAAGCA CCCTGATCTACGGCTACTTCGACTACTGGGGCCAGGGC ACCACCGTGACCGTGTCTAGCGCTTCTACCAAGGGCCC CAGCGTGTTCCCTCTGGCCCCTAGCAGCAAGAGCACAT CTGGCGGAACAGCCGCCCTGGGCTGCCTCGTGAAGGA CTACTTTCCCGAGCCCGTGACAGTGTCCTGGAACTCTG GCGCCCTGACAAGCGGCGTGCACACCTTTCCAGCCGTG CTGCAGAGCAGCGGCCTGTACTCTCTGAGCAGCGTCGT GACTGTGCCCAGCAGCAGCCTGGGAACCCAGACCTAC ATCTGCAACGTGAACCACAAGCCCAGCAACACCAAGG TGGACAAGAAGGTGGAACCCAAGAGCTGCGACGGCGG AGGCGGATCTGGCGGCGGAGGATCCCAGGTGCAGCTG GTGCAGAGCGGAGCTGAAGTGAAAAAGCCTGGCTCCT CCGTGAAAGTGTCTTGTAAAGCCAGCGGCGGCACATTC TCATCCTACGCCATCAGCTGGGTGCGGCAGGCTCCAGG CCAGGGACTGGAATGGATGGGAGGAATTATCCCTATTT TTGGGACAGCCAATTATGCTCAGAAATTTCAGGGGCGC GTGACAATTACAGCCGACAAGTCCACCTCTACAGCTTA TATGGAACTGTCCTCCCTGCGCTCCGAGGATACAGCTG TGTATTACTGCGCCCGGTCCACACTGATCTATGGATAC TTTGATTATTGGGGGCAGGGAACAACAGTGACTGTGTC CTCCGCTAGCACCAAGGGCCCATCGGTCTTCCCCCTGG CACCCTCCTCCAAGAGCACCTCTGGGGGCACAGCGGCC CTGGGCTGCCTGGTCAAGGACTACTTCCCCGAACCGGT GACGGTGTCGTGGAACTCAGGCGCCCTGACCAGCGGC GTGCACACCTTCCCGGCTGTCCTACAGTCCTCAGGACT CTACTCCCTCAGCAGCGTGGTGACCGTGCCCTCCAGCA GCTTGGGCACCCAGACCTACATCTGCAACGTGAATCAC AAGCCCAGCAACACCAAGGTGGACAAGAAAGTTGAGC CCAAATCTTGTGACAAAACTCACACATGCCCACCGTGC CCAGCACCTGAAGCTGCAGGGGGACCGTCAGTCTTCCT CTTCCCCCCAAAACCCAAGGACACCCTCATGATCTCCC GGACCCCTGAGGTCACATGCGTGGTGGTGGACGTGAG CCACGAAGACCCTGAGGTCAAGTTCAACTGGTACGTG GACGGCGTGGAGGTGCATAATGCCAAGACAAAGCCGC GGGAGGAGCAGTACAACAGCACGTACCGTGTGGTCAG CGTCCTCACCGTCCTGCACCAGGACTGGCTGAATGGCA AGGAGTACAAGTGCAAGGTCTCCAACAAAGCCCTCGG CGCCCCCATCGAGAAAACCATCTCCAAAGCCAAAGGG CAGCCCCGAGAACCACAGGTGTGCACCCTGCCCCCATC CCGGGATGAGCTGACCAAGAACCAGGTCAGCCTCTCG TGCGCAGTCAAAGGCTTCTATCCCAGCGACATCGCCGT GGAGTGGGAGAGCAATGGGCAGCCGGAGAACAACTAC AAGACCACGCCTCCCGTGCTGGACTCCGACGGCTCCTT CTTCCTCGTGAGCAAGCTCACCGTGGACAAGAGCAGGT GGCAGCAGGGGAACGTCTTCTCATGCTCCGTGATGCAT GAGGCTCTGCACAACCACTACACGCAGAAGAGCCTCT |
| | | CCCTGTCTCCGGGTGGAGGCGGCGGAAGCGGAGGAGG AGGATCCGGCGGCGGAGGTTCCGGAGGCGGTGGATCT GAGATCGTGCTGACCCAGTCTCCGGCACCCTGTCTCT GAGCCCTGGCGAGAGAGCCACCCTGTCCTGCAGAGCC TCCCAGTCCGTGACCTCCTCCTACCTCGCCTGGTATCA GCAGAAGCCCGGCCAGGCCCCTCGGCTGCTGATCAAC GTGGGCAGTCGGAGAGCCACCGGCATCCCTGACCGGT TCTCCGGCTCTGGCTCCGGCACCGACTTCACCCTGACC ATCTCCCGGCTGGAACCCGAGGACTTCGCCGTGTACTA CTGCCAGCAGGGCATCATGCTGCCCCCCACCTTTGGCC AGGGCACCAAGGTGGAAATCAAG |
| 297 | (11D5) VLCL-light chain | see Table 48 |
| 335 | HC 1 (11D5) VHCH1_VHCH1_Fc knob VH (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSTLIYGYFDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCARSTLIYGYFDYWGQGTTVTVSSA STKGPSVFPLAPSSKSTSGGTAALGCLVKDYFPEPVTVSW NSGALTSGVHTFPAVLQSSGLYSLSSVVTVPSSSLGTQTYI CNVNHKPSNTKVDKKVEPKSCDKTHTCPPCPAPEAAGGP SVFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVYTLPPCRD ELTKNQVSLWCLVKGFYPSDIAVEWESNGQPENNYKTTP PVLDSDGSFFLYSKLTVDKSRWQQGNVFSCSVMHEALH NHYTQKSLSLSPGGGGGSGGGGSGGGGSGGGGSEVQLLE SGGGLVQPGGSLRLSCAASGFTFSSYAMSWVRQAPGKGL EWVSAIIGSGASTYYADSVKGRFTISRDNSKNTLYLQMNS LRAEDTAVYYCAKGWFGGFNYWGQGTLVTVSS |
| 336 | HC 2 (11D5) VHCH1_VHCH1_Fc hole VL (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSTLIYGYFDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCARSTLIYGYFDYWGQGTTVTVSSA STKGPSVFPLAPSSKSTSGGTAALGCLVKDYFPEPVTVSW NSGALTSGVHTFPAVLQSSGLYSLSSVVTVPSSSLGTQTYI CNVNHKPSNTKVDKKVEPKSCDKTHTCPPCPAPEAAGGP SVFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVCTLPPSRD ELTKNQVSLSCAVKGFYPSDIAVEWESNGQPENNYKTTP PVLDSDGSFFLVSKLTVDKSRWQQGNVFSCSVMHEALH NHYTQKSLSLSPGGGGGSGGGGSGGGGSGGGGSEIVLTQ SPGTLSLSPGERATLSCRASQSVTSSYLAWYQQKPGQAPR LLINVGSRRATGIPDRFSGSGSGTDFTLTISRLEPEDFAVY |
| | | YCQQGIMLPPTFGQGTKVEIK |
| 299 | (9B11) VLCL-light chain (nucleotide sequence) | see Table 48 |
| 337 | HC 1 (9B11) VHCH1_VHCH1_Fc knob VH (4B9) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGATCTTCT GGTGCTTACCCGGGTTACTTCGACTACTGGGGCCAAGG GACCACCGTGACCGTCTCCTCAGCTTCTACCAAGGGCC CCAGCGTGTTCCCTCTGGCCCCTAGCAGCAAGAGCACA TCTGGCGGAACAGCCGCCCTGGGCTGCCTCGTGAAGG ACTACTTTCCCGAGCCCGTGACAGTGTCCTGGAACTCT GGCGCCCTGACAAGCGGCGTGCACACCTTTCCAGCCGT GCTGCAGAGCAGCGGCCTGTACTCTCTGAGCAGCGTCG TGACTGTGCCCAGCAGCAGCCTGGGAACCCAGACCTA CATCTGCAACGTGAACCACAAGCCCAGCAACACCAAG GTGGACAAGAAGGTGGAACCCAAGAGCTGCGACGGCG GAGGCGGATCTGGCGGCGGAGGATCCCAGGTGCAATT GGTGCAGTCTGGGGCTGAGGTGAAGAAGCCTGGGTCC TCGGTGAAGGTCTCCTGCAAGGCCTCCGGAGGCACATT CAGCAGCTACGCTATAAGCTGGGTGCGACAGGCCCCT GGACAAGGGCTCGAGTGGATGGGAGGGATCATCCCTA TCTTTGGTACAGCAAACTACGCACAGAAGTTCCAGGGC AGGGTCACCATTACTGCAGACAAATCCACGAGCACAG CCTACATGGAGCTGAGCAGCCTGAGATCTGAGGACAC CGCCGTGTATTACTGTGCGAGATCTTCTGGTGCTTACC CGGGTTACTTCGACTACTGGGGCCAAGGGACCACCGTG ACCGTCTCCTCAGCTAGCACCAAGGGCCCATCGGTCTT CCCCCTGGCACCCTCCTCCAAGAGCACCTCTGGGGGCA CAGCGGCCCTGGGCTGCCTGGTCAAGGACTACTTCCCC GAACCGGTGACGGTGTCGTGGAACTCAGGCGCCCTGA CCAGCGGCGTGCACACCTTCCCGGCTGTCCTACAGTCC TCAGGACTCTACTCCCTCAGCAGCGTGGTGACCGTGCC CTCCAGCAGCTTGGGCACCCAGACCTACATCTGCAACG TGAATCACAAGCCCAGCAACACCAAGGTGGACAAGAA AGTTGAGCCCAAATCTTGTGACAAAACTCACACATGCC CACCGTGCCCAGCACCTGAAGCTGCAGGGGGACCGTC AGTCTTCCTCTTCCCCCCAAAACCCAAGGACACCCTCA TGATCTCCCGGACCCCTGAGGTCACATGCGTGGTGGTG GACGTGAGCCACGAAGACCCTGAGGTCAAGTTCAACT GGTACGTGGACGGCGTGGAGGTGCATAATGCCAAGAC AAAGCCGCGGGAGGAGCAGTACAACAGCACGTACCGT GTGGTCAGCGTCCTCACCGTCCTGCACCAGGACTGGCT GAATGGCAAGGAGTACAAGTGCAAGGTCTCCAACAAA GCCCTCGGCGCCCCCATCGAGAAAACCATCTCCAAAGC CAAAGGGCAGCCCCGAGAACCACAGGTGTACACCCTG CCCCCCTGCAGAGATGAGCTGACCAAGAACCAGGTGT CCCTGTGGTGTCTGGTCAAGGGCTTCTACCCCAGCGAT ATCGCCGTGGAGTGGGAGAGCAACGGCCAGCCTGAGA |
| | | ACAACTACAAGACCACCCCCCCTGTGCTGGACAGCGA CGGCAGCTTCTTCCTGTACTCCAAACTGACCGTGGACA AGAGCCGGTGGCAGCAGGGCAACGTGTTCAGCTGCAG CGTGATGCACGAGGCCCTGCACAACCACTACACCCAG AAGTCCCTGAGCCTGAGCCCCGGCGGAGGCGGCGGAA GCGGAGGAGGAGGATCTGGGGGCGGAGGTTCCGGAGG CGGAGGATCCGAGGTGCAGCTGCTCGAAAGCGGCGGA GGACTGGTGCAGCCTGGCGGCAGCCTGAGACTGTCTTG CGCCGCCAGCGGCTTCACCTTCAGCAGCTACGCCATGA GCTGGGTCCGCCAGGCCCCTGGCAAGGGACTGGAATG GGTGTCCGCCATCATCGGCTCTGGCGCCAGCACCTACT ACGCCGACAGCGTGAAGGGCCGGTTCACCATCAGCCG GGACAACAGCAAGAACACCCTGTACCTGCAGATGAAC AGCCTGCGGGCCGAGGACACCGCCGTGTACTACTGCG CCAAGGGATGGTTCGGCGGCTTCAACTACTGGGGACA GGGCACCCTGGTCACCGTGTCCAGC |
| 338 | HC 2 (9B11) VHCH1_VHCH1_Fc hole VL (4B9) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGATCTTCT GGTGCTTACCCGGGTTACTTCGACTACTGGGGCCAAGG GACCACCGTGACCGTCTCCTCAGCTTCTACCAAGGGCC CCAGCGTGTTCCCTCTGGCCCCTAGCAGCAAGAGCACA TCTGGCGGAACAGCCGCCCTGGGCTGCCTCGTGAAGG ACTACTTTCCCGAGCCCGTGACAGTGTCCTGGAACTCT GGCGCCCTGACAAGCGGCGTGCACACCTTTCCAGCCGT GCTGCAGAGCAGCGGCCTGTACTCTCTGAGCAGCGTCG TGACTGTGCCCAGCAGCAGCCTGGGAACCCAGACCTA CATCTGCAACGTGAACCACAAGCCCAGCAACACCAAG GTGGACAAGAAGGTGGAACCCAAGAGCTGCGACGGCG GAGGCGGATCTGGCGGCGGAGGATCCCAGGTGCAATT GGTGCAGTCTGGGGCTGAGGTGAAGAAGCCTGGGTCC TCGGTGAAGGTCTCCTGCAAGGCCTCCGGAGGCACATT CAGCAGCTACGCTATAAGCTGGGTGCGACAGGCCCCT GGACAAGGGCTCGAGTGGATGGGAGGGATCATCCCTA TCTTTGGTACAGCAAACTACGCACAGAAGTTCCAGGGC AGGGTCACCATTACTGCAGACAAATCCACGAGCACAG CCTACATGGAGCTGAGCAGCCTGAGATCTGAGGACAC CGCCGTGTATTACTGTGCGAGATCTTCTGGTGCTTACC CGGGTTACTTCGACTACTGGGGCCAAGGGACCACCGTG ACCGTCTCCTCAGCTAGCACCAAGGGCCCATCGGTCTT CCCCCTGGCACCCTCCTCCAAGAGCACCTCTGGGGGCA CAGCGGCCCTGGGCTGCCTGGTCAAGGACTACTTCCCC GAACCGGTGACGGTGTCGTGGAACTCAGGCGCCCTGA CCAGCGGCGTGCACACCTTCCCGGCTGTCCTACAGTCC TCAGGACTCTACTCCCTCAGCAGCGTGGTGACCGTGCC CTCCAGCAGCTTGGGCACCCAGACCTACATCTGCAACG TGAATCACAAGCCCAGCAACACCAAGGTGGACAAGAA AGTTGAGCCCAAATCTTGTGACAAAACTCACACATGCC CACCGTGCCCAGCACCTGAAGCTGCAGGGGGACCGTC |
| | | AGTCTTCCTCTTCCCCCCAAAACCCAAGGACACCCTCA TGATCTCCCGGACCCCTGAGGTCACATGCGTGGTGG GACGTGAGCCACGAAGACCCTGAGGTCAAGTTCAACT GGTACGTGGACGGCGTGGAGGTGCATAATGCCAAGAC AAAGCCGCGGGAGGAGCAGTACAACAGCACGTACCGT GTGGTCAGCGTCCTCACCGTCCTGCACCAGGACTGGCT GAATGGCAAGGAGTACAAGTGCAAGGTCTCCAACAAA GCCCTCGGCGCCCCCATCGAGAAAACCATCTCCAAAGC CAAAGGGCAGCCCCGAGAACCACAGGTGTGCACCCTG CCCCCATCCCGGGATGAGCTGACCAAGAACCAGGTCA GCCTCTCGTGCGCAGTCAAAGGCTTCTATCCCAGCGAC ATCGCCGTGGAGTGGGAGAGCAATGGGCAGCCGGAGA ACAACTACAAGACCACGCCTCCCGTGCTGGACTCCGAC GGCTCCTTCTTCCTCGTGAGCAAGCTCACCGTGGACAA GAGCAGGTGGCAGCAGGGGAACGTCTTCTCATGCTCC GTGATGCATGAGGCTCTGCACAACCACTACACGCAGA AGAGCCTCTCCCTGTCTCCGGGTGGAGGCGGCGGAAG CGGAGGAGGAGGATCCGGCGGCGGAGGTTCCGGAGGC GGTGGATCTGAGATCGTGCTGACCCAGTCTCCGGCAC CCTGTCTCTGAGCCCTGGCGAGAGAGCCACCCTGTCCT GCAGAGCCTCCCAGTCCGTGACCTCCTCCTACCTCGCC TGGTATCAGCAGAAGCCCGGCCAGGCCCCTCGGCTGCT GATCAACGTGGGCAGTCGGAGAGCCACCGGCATCCCT GACCGGTTCTCCGGCTCTGGCTCCGGCACCGACTTCAC CCTGACCATCTCCCGGCTGGAACCCGAGGACTTCGCCG TGTACTACTGCCAGCAGGGCATCATGCTGCCCCCCACC TTTGGCCAGGGCACCAAGGTGGAAATCAAG |
| 301 | (9B11) VLCL-light chain | see Table 48 |
| 339 | HC 1 (9B11) VHCH1_VHCH1_Fc knob VH (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSSGAYPGYFDYWGQGTTVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDGGGGSGGGGSQ VQLVQSGAEVKKPGSSVKVSCKASGGTFSSYAISWVRQA PGQGLEWMGGIIPIFGTANYAQKFQGRVTITADKSTSTAY MELSSLRSEDTAVYYCARSSGAYPGYFDYWGQGTTVTV SSASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDKTHTCPPCPAPEA AGGPSVFLFPPKPKDTLMISRTPEVTCVVVDVSHEDPEVK FNWYVDGVEVHNAKTKPREEQYNSTYRVVSVLTVLHQD WLNGKEYKCKVSNKALGAPIEKTISKAKGQPREPQVYTL PPCRDELTKNQVSLWCLVKGFYPSDIAVEWESNGQPENN YKTTPPVLDSDGSFFLYSKLTVDKSRWQQGNVFSCSVMH EALHNHYTQKSLSLSPGGGGGSGGGGSGGGGSGGGGSE VQLLESGGGLVQPGGSLRLSCAASGFTFSSYAMSWVRQA PGKGLEWVSAIIGSGASTYYADSVKGRFTISRDNSKNTLY LQMNSLRAEDTAVYYCAKGWFGGFNYWGQGTLVTVSS |
| 340 | HC 2 (9B11) VHCH1_VHCH1_Fc hole VL (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSSGAYPGYFDYWGQGTTVT VSSASTKGPSVFPLAPSSKSTSGGTAALGCLVKDYFPEPV |
| | | TVSWNSGALTSGVHTFPAVLQSSGLYSLSSVVTVPSSSLG TQTYICNVNHKPSNTKVDKKVEPKSCDGGGGSGGGGSQ VQLVQSGAEVKKPGSSVKVSCKASGGTFSSYAISWVRQA PGQGLEWMGGIIPIFGTANYAQKFQGRVTITADKSTSTAY MELSSLRSEDTAVYYCARSSGAYPGYFDYWGQGTTVTV SSASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDKTHTCPPCPAPEA AGGPSVFLFPPKPKDTLMISRTPEVTCVVVDVSHEDPEVK FNWYVDGVEVHNAKTKPREEQYNSTYRVVSVLTVLHQD WLNGKEYKCKVSNKALGAPIEKTISKAKGQPREPQVCTL PPSRDELTKNQVSLSCAVKGFYPSDIAVEWESNGQPENN YKTTPPVLDSDGSFFLVSKLTVDKSRWQQGNVFSCSVMH EALHNHYTQKSLSLSPGGGGGSGGGGSGGGGSGGGGSEI VLTQSPGTLSLSPGERATLSCRASQSVTSSYLAWYQQKPG QAPRLLINVGSRRATGIPDRFSGSGSGTDFTLTISRLEPEDF AVYYCQQGIMLPPTFGQGTKVEIK |

The base pair and amino acid sequences for 4+1 anti-4-1BB, anti-FAP constructs with a-FAP VL fused to knob and VH fused to hole chain can be found respectively in **Table 66.**

**Table 66: Amino acid sequences of mature bispecific tetravalent anti-4-1BB/ monovalent anti-FAP huIgG1 P329GLALA kih antibody (4+1 format) anti-FAP VH fused to hole and anti-FAP VL fused to knob chain**

| SEQ ID NO: | Description | Sequence |
|---|---|---|
| 287 | (12B3) VLCL-light chain (nucleotide sequence) | see Table 48 |
| 341 | HC 1 (12B3) VHCH1 VHCH1 Fc knob VL (4B9) (nucleotide sequence) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTACTGTGCCAGAAGCG AGTTCCGGTTCTACGCCGACTTCGACTACTGGGGCCAG GGCACCACCGTGACCGTGTCTAGCGCTTCTACAAAGGG CCCCAGCGTGTTCCCTCTGGCCCCTAGCAGCAAGTCTA CCAGCGGAGGAACAGCCGCCCTGGGCTGCCTCGTGAA GGACTACTTTCCCGAGCCCGTGACAGTGTCCTGGAACA GCGGAGCCCTGACAAGCGGCGTGCACACCTTTCCAGCC GTGCTGCAGAGCAGCGGCCTGTACTCTCTGAGCAGCGT CGTGACTGTGCCCAGCAGCAGCCTGGGAACCCAGACC TACATCTGCAACGTGAACCACAAGCCCAGCAACACCA AGGTGGACAAGAAGGTGGAACCCAAGAGCTGCGACGG CGGAGGCGGATCAGGCGGCGGAGGATCCCAGGTGCAG CTGGTGCAGAGCGGAGCTGAAGTGAAAAAGCCTGGCT CCTCCGTGAAAGTGTCTTGTAAAGCCAGCGGCGGCACA |
| | | TTCTCATCCTACGCCATCAGCTGGGTGCGGCAGGCTCC AGGCCAGGGACTGGAATGGATGGGAGGAATTATCCCT ATTTTTGGACAGCCAATTATGCTCAGAAATTTCAGGG GCGCGTGACAATTACAGCCGACAAGTCCACCTCTACAG CTTATATGGAACTGTCCTCCCTGCGCTCCGAGGATACA GCTGTGTATTACTGCGCTCGGAGCGAGTTTAGATTCTA TGCCGATTTTGATTATTGGGGGCAGGGAACAACAGTGA CTGTGTCCTCCGCTAGCACCAAGGGCCCATCGGTCTTC CCCCTGGCACCCTCCTCCAAGAGCACCTCTGGGGGCAC AGCGGCCCTGGGCTGCCTGGTCAAGGACTACTTCCCCG AACCGGTGACGGTGTCGTGGAACTCAGGCGCCCTGAC CAGCGGCGTGCACACCTTCCCGGCTGTCCTACAGTCCT CAGGACTCTACTCCCTCAGCAGCGTGGTGACCGTGCCC TCCAGCAGCTTGGGCACCCAGACCTACATCTGCAACGT GAATCACAAGCCCAGCAACACCAAGGTGGACAAGAAA GTTGAGCCCAAATCTTGTGACAAAACTCACACATGCCC ACCGTGCCCAGCACCTGAAGCTGCAGGGGGACCGTCA GTCTTCCTCTTCCCCCCAAAACCCAAGGACACCCTCAT GATCTCCCGGACCCCTGAGGTCACATGCGTGGTGGTGG ACGTGAGCCACGAAGACCCTGAGGTCAAGTTCAACTG GTACGTGGACGGCGTGGAGGTGCATAATGCCAAGACA AAGCCGCGGGAGGAGCAGTACAACAGCACGTACCGTG TGGTCAGCGTCCTCACCGTCCTGCACCAGGACTGGCTG AATGGCAAGGAGTACAAGTGCAAGGTCTCCAACAAAG CCCTCGGCGCCCCCATCGAGAAAACCATCTCCAAAGCC AAAGGGCAGCCCCGAGAACCACAGGTGTACACCCTGC CCCCCTGCAGAGATGAGCTGACCAAGAACCAGGTGTC CCTGTGGTGTCTGGTCAAGGGCTTCTACCCCAGCGATA TCGCCGTGGAGTGGGAGAGCAACGGCCAGCCTGAGAA CAACTACAAGACCACCCCCCCTGTGCTGGACAGCGAC GGCAGCTTCTTCCTGTACTCCAAACTGACCGTGGACAA GAGCCGGTGGCAGCAGGGCAACGTGTTCAGCTGCAGC GTGATGCACGAGGCCCTGCACAACCACTACACCCAGA AGTCCCTGAGCCTGAGCCCCGGCGGAGGCGGCGGAAG CGGAGGAGGAGGATCTGGGGGCGGAGGTTCCGGAGGC GGTGGATCTGAGATCGTGCTGACCCAGTCTCCCGGCAC CCTGTCTCTGAGCCCTGGCGAGAGAGCCACCCTGTCCT GCAGAGCCTCCCAGTCCGTGACCTCCTCCTACCTCGCC TGGTATCAGCAGAAGCCCGGCCAGGCCCCTCGGCTGCT GATCAACGTGGGCAGTCGGAGAGCCACCGGCATCCCT GACCGGTTCTCCGGCTCTGGCTCCGGCACCGACTTCAC CCTGACCATCTCCCGGCTGGAACCCGAGGACTTCGCCG TGTACTACTGCCAGCAGGGCATCATGCTGCCCCCCACC TTTGGCCAGGGCACCAAGGTGGAAATCAAG |
| 342 | HC 2 (12B3) VHCH1 VHCH1 Fc hole VH (4B9) (nucleotide sequence) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTACTGTGCCAGAAGCG AGTTCCGGTTCTACGCCGACTTCGACTACTGGGGCCAG GGCACCACCGTGACCGTGTCTAGCGCTTCTACAAAGGG |
| | | CCCCAGCGTGTTCCCTCTGGCCCCTAGCAGCAAGTCTA CCAGCGGAGGAACAGCCGCCCTGGGCTGCCTCGTGAA GGACTACTTTCCCGAGCCCGTGACAGTGTCCTGGAACA GCGGAGCCCTGACAAGCGGCGTGCACACCTTTCCAGCC GTGCTGCAGAGCAGCGGCCTGTACTCTCTGAGCAGCGT CGTGACTGTGCCCAGCAGCAGCCTGGGAACCCAGACC TACATCTGCAACGTGAACCACAAGCCCAGCAACACCA AGGTGGACAAGAAGGTGGAACCCAAGAGCTGCGACGG CGGAGGCGGATCAGGCGGCGGAGGATCCCAGGTGCAG CTGGTGCAGAGCGGAGCTGAAGTGAAAAAGCCTGGCT CCTCCGTGAAAGTGTCTTGTAAAGCCAGCGGCGGCACA TTCTCATCCTACGCCATCAGCTGGGTGCGGCAGGCTCC AGGCCAGGGACTGGAATGGATGGGAGGAATTATCCCT ATTTTTGGGACAGCCAATTATGCTCAGAAATTTCAGGG GCGCGTGACAATTACAGCCGACAAGTCCACCTCTACAG CTTATATGGAACTGTCCTCCCTGCGCTCCGAGGATACA GCTGTGTATTACTGCGCTCGGAGCGAGTTTAGATTCTA TGCCGATTTTGATTATTGGGGGCAGGGAACAACAGTGA CTGTGTCCTCCGCTAGCACCAAGGGCCCATCGGTCTTC CCCCTGGCACCCTCCTCCAAGAGCACCTCTGGGGGCAC AGCGGCCCTGGGCTGCCTGGTCAAGGACTACTTCCCCG AACCGGTGACGGTGTCGTGGAACTCAGGCGCCCTGAC CAGCGGCGTGCACACCTTCCCGGCTGTCCTACAGTCCT CAGGACTCTACTCCCTCAGCAGCGTGGTGACCGTGCCC TCCAGCAGCTTGGGCACCCAGACCTACATCTGCAACGT GAATCACAAGCCCAGCAACACCAAGGTGGACAAGAAA GTTGAGCCCAAATCTTGTGACAAAACTCACACATGCCC ACCGTGCCCAGCACCTGAAGCTGCAGGGGGACCGTCA GTCTTCCTCTTCCCCCCAAAACCCAAGGACACCCTCAT GATCTCCCGGACCCCTGAGGTCACATGCGTGGTGGTGG ACGTGAGCCACGAAGACCCTGAGGTCAAGTTCAACTG GTACGTGGACGGCGTGGAGGTGCATAATGCCAAGACA AAGCCGCGGGAGGAGCAGTACAACAGCACGTACCGTG TGGTCAGCGTCCTCACCGTCCTGCACCAGGACTGGCTG AATGGCAAGGAGTACAAGTGCAAGGTCTCCAACAAAG CCCTCGGCGCCCCCATCGAGAAAACCATCTCCAAAGCC AAAGGGCAGCCCCGAGAACCACAGGTGTGCACCCTGC CCCCATCCCGGGATGAGCTGACCAAGAACCAGGTCAG CCTCTCGTGCGCAGTCAAAGGCTTCTATCCCAGCGACA TCGCCGTGGAGTGGGAGAGCAATGGGCAGCCGGAGAA CAACTACAAGACCACGCCTCCCGTGCTGGACTCCGACG GCTCCTTCTTCCTCGTGAGCAAGCTCACCGTGGACAAG AGCAGGTGGCAGCAGGGGAACGTCTTCTCATGCTCCGT GATGCATGAGGCTCTGCACAACCACTACACGCAGAAG AGCCTCTCCCTGTCTCCGGGTGGAGGCGGCGGAAGCG GAGGAGGAGGATCCGGCGGCGGAGGTTCCGGAGGCGG AGGATCCGAGGTGCAGCTGCTCGAAAGCGGCGGAGGA CTGGTGCAGCCTGGCGGCAGCCTGAGACTGTCTTGCGC CGCCAGCGGCTTCACCTTCAGCAGCTACGCCATGAGCT GGGTCCGCCAGGCCCCTGGCAAGGGACTGGAATGGGT GTCCGCCATCATCGGCTCTGGCGCCAGCACCTACTACG CCGACAGCGTGAAGGGCCGGTTCACCATCAGCCGGGA CAACAGCAAGAACACCCTGTACCTGCAGATGAACAGC CTGCGGGCCGAGGACACCGCCGTGTACTACTGCGCCA |
| | | AGGGATGGTTCGGCGGCTTCAACTACTGGGGACAGGG CACCCTGGTCACCGTGTCCAGC |
| 289 | (12B3) VLCL-light chain | see Table 48 |
| 343 | HC 1 (12B3) VHCH1 VHCH1 Fc knob VL (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSEFRFYADFDYWGQGTTVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDGGGGSGGGGS QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSEFRFYADFDYWGQGTTVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDKTHTCPPCPAPE AAGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALGAPIEKTISKAKGQPREPQVYT LPPCRDELTKNQVSLWCLVKGFYPSDIAVEWESNGQPEN NYKTTPPVLDSDGSFFLYSKLTVDKSRWQQGNVFSCSVM HEALHNHYTQKSLSLSPGGGGGSGGGGSGGGGSGGGGS EIVLTQSPGTLSLSPGERATLSCRASQSVTSSYLAWYQQK PGQAPRLLINVGSRRATGIPDRFSGSGSGTDFTLTISRLEPE DFAVYYCQQGIMLPPTFGQGTKVEIK |
| 344 | HC 2 (12B3) VHCH1 VHCH1 Fc hole VH (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSEFRFYADFDYWGQGTTVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDGGGGSGGGGSQ VQLVQSGAEVKKPGSSVKVSCKASGGTFSSYAISWVRQA PGQGLEWMGGIIPIFGTANYAQKFQGRVTITADKSTSTAY MELSSLRSEDTAVYYCARSEFRFYADFDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPEAA GGPSVFLFPPKPKDTLMISRTPEVTCVVVDVSHEDPEVKF NWYVDGVEVHNAKTKPREEQYNSTYRVVSVLTVLHQD WLNGKEYKCKVSNKALGAPIEKTISKAKGQPREPQVCTL PPSRDELTKNQVSLSCAVKGFYPSDIAVEWESNGQPENN YKTTPPVLDSDGSFFLVSKLTVDKSRWQQGNVFSCSVMH EALHNHYTQKSLSLSPGGGGGSGGGGSGGGGSGGGGSE VQLLESGGGLVQPGGSLRLSCAASGFTFSSYAMSWVRQA PGKGLEWVSAIIGSGASTYYADSVKGRFTISRDNSKNTLY LQMNSLRAEDTAVYYCAKGWFGGFNYWGQGTLVTVSS |
| 291 | (25G7) VLCL-light chain (nucleotide sequence) | see Table 48 |
| 345 | HC 1 (25G7) VHCH1 VHCH1 Fc knob VL (4B9) | GAGGTGCAGCTGCTGGAATCTGGCGGCGGACTGGTGC AGCCTGGCGGATCTCTGAGACTGAGCTGTGCCGCCAGC GGCTTCACCTTCAGCAGCTACGCCATGAGCTGGGTGCG CCAGGCCCCTGGAAAGGCCTGGAATGGGTGTCCGCC ATCTCTGGCAGCGGCGGCAGCACCTACTACGCCGATTC |
| | (nucleotide sequence) | TGTGAAGGGCCGGTTCACCATCAGCCGGGACAACAGC AAGAACACCCTGTACCTGCAGATGAACAGCCTGCGGG CCGAGGACACCGCCGTGTACTATTGCGCCAGGGACGA CCCCTGGCCCCCTTTGATTATTGGGGACAGGGCACCC TCGTGACCGTGTCCAGCGCTTCTACAAAGGGCCCCAGC GTGTTCCCTCTGGCCCCTAGCAGCAAGTCTACCAGCGG AGGAACAGCCGCCCTGGGCTGCCTCGTGAAGGACTAC TTTCCCGAGCCCGTGACAGTGTCCTGGAACAGCGGAGC CCTGACAAGCGGCGTGCACACCTTTCCAGCCGTGCTGC AGAGCAGCGGCCTGTACTCTCTGAGCAGCGTCGTGACT GTGCCCAGCAGCAGCCTGGGAACCCAGACCTACATCT GCAACGTGAACCACAAGCCCAGCAACACCAAGGTGGA CAAGAAGGTGGAACCCAAGAGCTGCGACGGCGGAGGC GGATCAGGCGGCGGAGGATCCGAAGTGCAGCTGCTGG AAAGTGGGGGAGGCCTGGTGCAGCCAGGGGGAAGCCT GAGACTGTCTTGTGCCGCTTCCGGCTTTACCTTTAGCTC TTACGCCATGTCTTGGGTGCGGCAGGCTCCAGGCAAGG GACTGGAATGGGTGTCAGCTATCAGCGGCTCTGGCGGC TCCACATATTACGCCGACAGCGTGAAGGGCAGATTCAC AATCTCCAGAGACAACTCCAAGAATACTCTGTACCTGC AGATGAATTCCCTGCGCGCCGAAGATACAGCTGTGTAT TACTGTGCCCGCGACGATCCTTGGCCCCCTTTCGACTA CTGGGGGCAGGGAACACTCGTGACAGTGTCATCCGCT AGCACCAAGGGCCCATCGGTCTTCCCCCTGGCACCCTC CTCCAAGAGCACCTCTGGGGGCACAGCGGCCCTGGGC TGCCTGGTCAAGGACTACTTCCCCGAACCGGTGACGGT GTCGTGGAACTCAGGCGCCCTGACCAGCGGCGTGCAC ACCTTCCCGGCTGTCCTACAGTCCTCAGGACTCTACTC CCTCAGCAGCGTGGTGACCGTGCCCTCCAGCAGCTTGG GCACCCAGACCTACATCTGCAACGTGAATCACAAGCCC AGCAACACCAAGGTGGACAAGAAAGTTGAGCCCAAAT CTTGTGACAAAACTCACACATGCCCACCGTGCCCAGCA CCTGAAGCTGCAGGGGGACCGTCAGTCTTCCTCTTCCC CCCAAAACCCAAGGACACCCTCATGATCTCCCGGACCC CTGAGGTCACATGCGTGGTGGTGGACGTGAGCCACGA AGACCCTGAGGTCAAGTTCAACTGGTACGTGGACGGC GTGGAGGTGCATAATGCCAAGACAAAGCCGCGGGAGG AGCAGTACAACAGCACGTACCGTGTGGTCAGCGTCCTC ACCGTCCTGCACCAGGACTGGCTGAATGGCAAGGAGT ACAAGTGCAAGGTCTCCAACAAAGCCCTCGGCGCCCC CATCGAGAAAACCATCTCCAAAGCCAAAGGGCAGCCC CGAGAACCACAGGTGTACACCCTGCCCCCCTGCAGAG ATGAGCTGACCAAGAACCAGGTGTCCCTGTGGTGTCTG GTCAAGGGCTTCTACCCCAGCGATATCGCCGTGGAGTG GGAGAGCAACGGCCAGCCTGAGAACAACTACAAGACC ACCCCCCCTGTGCTGGACAGCGACGGCAGCTTCTTCCT GTACTCCAAACTGACCGTGGACAAGAGCCGGTGGCAG CAGGGCAACGTGTTCAGCTGCAGCGTGATGCACGAGG CCCTGCACAACCACTACACCCAGAAGTCCCTGAGCCTG AGCCCCGGCGGAGGCGGCGGAAGCGGAGGAGGAGGA TCTGGGGGCGGAGGTTCCGGAGGCGGTGGATCTGAGA TCGTGCTGACCCAGTCTCCCGGCACCCTGTCTCTGAGC CCTGGCGAGAGAGCCACCCTGTCCTGCAGAGCCTCCCA GTCCGTGACCTCCTCCTACCTCGCCTGGTATCAGCAGA |
| | | AGCCCGGCCAGGCCCCTCGGCTGCTGATCAACGTGGGC AGTCGGAGAGCCACCGGCATCCCTGACCGGTTCTCCGG CTCTGGCTCCGGCACCGACTTCACCCTGACCATCTCCC GGCTGGAACCCGAGGACTTCGCCGTGTACTACTGCCAG CAGGGCATCATGCTGCCCCCCACCTTTGGCCAGGGCAC CAAGGTGGAAATCAAG |
| 346 | HC 2 (25G7) VHCH1 VHCH1 Fc hole VH (4B9) (nucleotide sequence) | GAGGTGCAGCTGCTGGAATCTGGCGGCGGACTGGTGC AGCCTGGCGGATCTCTGAGACTGAGCTGTGCCGCCAGC GGCTTCACCTTCAGCAGCTACGCCATGAGCTGGGTGCG CCAGGCCCCTGGAAAGGCCTGGAATGGGTGTCCGCC ATCTCTGGCAGCGGCGGCAGCACCTACTACGCCGATTC TGTGAAGGGCCGGTTCACCATCAGCCGGGACAACAGC AAGAACACCCTGTACCTGCAGATGAACAGCCTGCGGG CCGAGGACACCGCCGTGTACTATTGCGCCAGGGACGA CCCCTGGCCCCCCTTTGATTATTGGGGACAGGGCACCC TCGTGACCGTGTCCAGCGCTTCTACAAAGGGCCCCAGC GTGTTCCCTCTGGCCCCTAGCAGCAAGTCTACCAGCGG AGGAACAGCCGCCCTGGGCTGCCTCGTGAAGGACTAC TTTCCCGAGCCCGTGACAGTGTCCTGGAACAGCGGAGC CCTGACAAGCGGCGTGCACACCTTTCCAGCCGTGCTGC AGAGCAGCGGCCTGTACTCTCTGAGCAGCGTCGTGACT GTGCCCAGCAGCAGCCTGGGAACCCAGACCTACATCT GCAACGTGAACCACAAGCCCAGCAACACCAAGGTGGA CAAGAAGGTGGAACCCAAGAGCTGCGACGGCGGAGGC GGATCAGGCGGCGGAGGATCCGAAGTGCAGCTGCTGG AAAGTGGGGGAGGCCTGGTGCAGCCAGGGGGAAGCCT GAGACTGTCTTGTGCCGCTTCCGGCTTTACCTTTAGCTC TTACGCCATGTCTTGGGTGCGGCAGGCTCCAGGCAAGG GACTGGAATGGGTGTCAGCTATCAGCGGCTCTGGCGGC TCCACATATTACGCCGACAGCGTGAAGGGCAGATTCAC AATCTCCAGAGACAACTCCAAGAATACTCTGTACCTGC AGATGAATTCCCTGCGCGCCGAAGATACAGCTGTGTAT TACTGTGCCCGCGACGATCCTTGGCCCCCTTTCGACTA CTGGGGGCAGGGAACACTCGTGACAGTGTCATCCGCT AGCACCAAGGGCCCATCGGTCTTCCCCCTGGCACCCTC CTCCAAGAGCACCTCTGGGGGCACAGCGGCCCTGGGC TGCCTGGTCAAGGACTACTTCCCCGAACCGGTGACGGT GTCGTGGAACTCAGGCGCCCTGACCAGCGGCGTGCAC ACCTTCCCGGCTGTCCTACAGTCCTCAGGACTCTACTC CCTCAGCAGCGTGGTGACCGTGCCCTCCAGCAGCTTGG GCACCCAGACCTACATCTGCAACGTGAATCACAAGCCC AGCAACACCAAGGTGGACAAGAAAGTTGAGCCCAAAT CTTGTGACAAAACTCACACATGCCCACCGTGCCCAGCA CCTGAAGCTGCAGGGGGACCGTCAGTCTTCCTCTTCCC CCCAAAACCCAAGGACACCCTCATGATCTCCCGGACCC CTGAGGTCACATGCGTGGTGGTGGACGTGAGCCACGA AGACCCTGAGGTCAAGTTCAACTGGTACGTGGACGGC GTGGAGGTGCATAATGCCAAGACAAAGCCGCGGGAGG AGCAGTACAACAGCACGTACCGTGTGGTCAGCGTCCTC ACCGTCCTGCACCAGGACTGGCTGAATGGCAAGGAGT ACAAGTGCAAGGTCTCCAACAAAGCCCTCGGCGCCCC CATCGAGAAAACCATCTCCAAAGCCAAAGGGCAGCCC CGAGAACCACAGGTGTGCACCCTGCCCCCATCCCGGG ATGAGCTGACCAAGAACCAGGTCAGCCTCTCGTGCGC |
| | | AGTCAAAGGCTTCTATCCCAGCGACATCGCCGTGGAGT GGGAGAGCAATGGGCAGCCGGAGAACAACTACAAGAC CACGCCTCCCGTGCTGGACTCCGACGGCTCCTTCTTCCT CGTGAGCAAGCTCACCGTGGACAAGAGCAGGTGGCAG CAGGGGAACGTCTTCTCATGCTCCGTGATGCATGAGGC TCTGCACAACCACTACACGCAGAAGAGCCTCTCCCTGT CTCCGGGTGGAGGCGGCGGAAGCGGAGGAGGAGGATC CGGCGGCGGAGGTTCCGGAGGCGGAGGATCCGAGGTG CAGCTGCTCGAAAGCGGCGGAGGACTGGTGCAGCCTG GCGGCAGCCTGAGACTGTCTTGCGCCGCCAGCGGCTTC ACCTTCAGCAGCTACGCCATGAGCTGGGTCCGCCAGGC CCCTGGCAAGGGACTGGAATGGGTGTCCGCCATCATCG GCTCTGGCGCCAGCACCTACTACGCCGACAGCGTGAA GGGCCGGTTCACCATCAGCCGGGACAACAGCAAGAAC ACCCTGTACCTGCAGATGAACAGCCTGCGGGCCGAGG ACACCGCCGTGTACTACTGCGCCAAGGGATGGTTCGGC GGCTTCAACTACTGGGGACAGGGCACCCTGGTCACCGT GTCCAGC |
| 293 | (25G7) VLCL-light chain | see Table 48 |
| 347 | HC 1 (25G7) VHCH1 VHCH1 Fc knob VL (4B9) | EVQLLESGGGLVQPGGSLRLSCAASGFTFSSYAMSWVRQ APGKGLEWVSAISGSGGSTYYADSVKGRFTISRDNSKNTL YLQMNSLRAEDTAVYYCARDDPWPPFDYWGQGTLVTV SSASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDGGGGSGGGGSEV QLLESGGGLVQPGGSLRLSCAASGFTFSSYAMSWVRQAP GKGLEWVSAISGSGGSTYYADSVKGRFTISRDNSKNTLY LQMNSLRAEDTAVYYCARDDPWPPFDYWGQGTLVTVSS ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALGAPIEKTISKAKGQPREPQVYTLPP CRDELTKNQVSLWCLVKGFYPSDIAVEWESNGQPENNY KTTPPVLDSDGSFFLYSKLTVDKSRWQQGNVFSCSVMHE ALHNHYTQKSLSLSPGGGGGSGGGGSGGGGSGGGGSEIV LTQSPGTLSLSPGERATLSCRASQSVTSSYLAWYQQKPGQ APRLLINVGSRRATGIPDRFSGSGSGTDFTLTISRLEPEDFA VYYCQQGIMLPPTFGQGTKVEIK |
| 348 | HC 2 (25G7) VHCH1 VHCH1 Fc hole VH (4B9) | EVQLLESGGGLVQPGGSLRLSCAASGFTFSSYAMSWVRQ APGKGLEWVSAISGSGGSTYYADSVKGRFTISRDNSKNTL YLQMNSLRAEDTAVYYCARDDPWPPFDYWGQGTLVTV SSASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDGGGGSGGGGSEV QLLESGGGLVQPGGSLRLSCAASGFTFSSYAMSWVRQAP GKGLEWVSAISGSGGSTYYADSVKGRFTISRDNSKNTLY LQMNSLRAEDTAVYYCARDDPWPPFDYWGQGTLVTVSS ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAG GPSVFLFPPKPKDTLMISRTPEVTCVVVDVSHEDPEVKFN |
| | | WYVDGVEVHNAKTKPREEQYNSTYRVVSVLTVLHQDW LNGKEYKCKVSNKALGAPIEKTISKAKGQPREPQVCTLPP SRDELTKNQVSLSCAVKGFYPSDIAVEWESNGQPENNYK TTPPVLDSDGSFFLVSKLTVDKSRWQQGNVFSCSVMHEA LHNHYTQKSLSLSPGGGGGSGGGGSGGGGSGGGGSEVQ LLESGGGLVQPGGSLRLSCAASGFTFSSYAMSWVRQAPG KGLEWVSAIIGSGASTYYADSVKGRFTISRDNSKNTLYLQ MNSLRAEDTAVYYCAKGWFGGFNYWGQGTLVTVSS |
| 295 | (11D5) VLCL-light chain (nucleotide sequence) | see Table 48 |
| 349 | HC 1 (11D5) VHCH1 VHCH1 Fc knob VL (4B9) (nucleotide sequence) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTACTGTGCCAGAAGCA CCCTGATCTACGGCTACTTCGACTACTGGGGCCAGGGC ACCACCGTGACCGTGTCTAGCGCTTCTACCAAGGGCCC CAGCGTGTTCCCTCTGGCCCCTAGCAGCAAGAGCACAT CTGGCGGAACAGCCGCCCTGGGCTGCCTCGTGAAGGA CTACTTTCCCGAGCCCGTGACAGTGTCCTGGAACTCTG GCGCCCTGACAAGCGGCGTGCACACCTTTCCAGCCGTG CTGCAGAGCAGCGGCCTGTACTCTCTGAGCAGCGTCGT GACTGTGCCCAGCAGCAGCCTGGGAACCCAGACCTAC ATCTGCAACGTGAACCACAAGCCCAGCAACACCAAGG TGGACAAGAAGGTGGAACCCAAGAGCTGCGACGGCGG AGGCGGATCTGGCGGCGGAGGATCCCAGGTGCAGCTG GTGCAGAGCGGAGCTGAAGTGAAAAAGCCTGGCTCCT CCGTGAAAGTGTCTTGTAAAGCCAGCGGCGGCACATTC TCATCCTACGCCATCAGCTGGGTGCGGCAGGCTCCAGG CCAGGGACTGGAATGGATGGGAGGAATTATCCCTATTT TTGGGACAGCCAATTATGCTCAGAAATTTCAGGGGCGC GTGACAATTACAGCCGACAAGTCCACCTCTACAGCTTA TATGGAACTGTCCTCCCTGCGCTCCGAGGATACAGCTG TGTATTACTGCGCCCGGTCCACACTGATCTATGGATAC TTTGATTATTGGGGGCAGGGAACAACAGTGACTGTGTC CTCCGCTAGCACCAAGGGCCCATCGGTCTTCCCCCTGG CACCCTCCTCCAAGAGCACCTCTGGGGGCACAGCGGCC CTGGGCTGCCTGGTCAAGGACTACTTCCCCGAACCGGT GACGGTGTCGTGGAACTCAGGCGCCCTGACCAGCGGC GTGCACACCTTCCCGGCTGTCCTACAGTCCTCAGGACT CTACTCCCTCAGCAGCGTGGTGACCGTGCCCTCCAGCA GCTTGGGCACCCAGACCTACATCTGCAACGTGAATCAC AAGCCCAGCAACACCAAGGTGGACAAGAAAGTTGAGC CCAAATCTTGTGACAAAACTCACACATGCCCACCGTGC CCAGCACCTGAAGCTGCAGGGGGACCGTCAGTCTTCCT CTTCCCCCCAAAACCCAAGGACACCCTCATGATCTCCC GGACCCCTGAGGTCACATGCGTGGTGGTGGACGTGAG CCACGAAGACCCTGAGGTCAAGTTCAACTGGTACGTG GACGGCGTGGAGGTGCATAATGCCAAGACAAAGCCGC GGGAGGAGCAGTACAACAGCACGTACCGTGTGGTCAG |
| | | CGTCCTCACCGTCCTGCACCAGGACTGGCTGAATGGCA AGGAGTACAAGTGCAAGGTCTCCAACAAAGCCCTCGG CGCCCCCATCGAGAAAACCATCTCCAAAGCCAAAGGG CAGCCCCGAGAACCACAGGTGTACACCCTGCCCCCCTG CAGAGATGAGCTGACCAAGAACCAGGTGTCCCTGTGG TGTCTGGTCAAGGGCTTCTACCCCAGCGATATCGCCGT GGAGTGGGAGAGCAACGGCCAGCCTGAGAACAACTAC AAGACCACCCCCCCTGTGCTGGACAGCGACGGCAGCTT CTTCCTGTACTCCAAACTGACCGTGGACAAGAGCCGGT GGCAGCAGGGCAACGTGTTCAGCTGCAGCGTGATGCA CGAGGCCCTGCACAACCACTACACCCAGAAGTCCCTG AGCCTGAGCCCCGGCGGAGGCGGCGGAAGCGGAGGAG GAGGATCTGGGGGCGGAGGTTCCGGAGGCGGTGGATC TGAGATCGTGCTGACCCAGTCTCCCGGCACCCTGTCTC TGAGCCCTGGCGAGAGAGCCACCCTGTCCTGCAGAGC CTCCCAGTCCGTGACCTCCTCCTACCTCGCCTGGTATCA GCAGAAGCCCGGCCAGGCCCCTCGGCTGCTGATCAAC GTGGGCAGTCGGAGAGCCACCGGCATCCCTGACCGGT TCTCCGGCTCTGGCTCCGGCACCGACTTCACCCTGACC ATCTCCCGGCTGGAACCCGAGGACTTCGCCGTGTACTA CTGCCAGCAGGGCATCATGCTGCCCCCCACCTTTGGCC AGGGCACCAAGGTGGAAATCAAG |
| 350 | HC 2 (11D5) VHCH1 VHCH1 Fc hole VH (4B9) (nucleotide sequence) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTACTGTGCCAGAAGCA CCCTGATCTACGGCTACTTCGACTACTGGGGCCAGGGC ACCACCGTGACCGTGTCTAGCGCTTCTACCAAGGGCCC CAGCGTGTTCCCTCTGGCCCCTAGCAGCAAGAGCACAT CTGGCGGAACAGCCGCCCTGGGCTGCCTCGTGAAGGA CTACTTTCCCGAGCCCGTGACAGTGTCCTGGAACTCTG GCGCCCTGACAAGCGGCGTGCACACCTTTCCAGCCGTG CTGCAGAGCAGCGGCCTGTACTCTCTGAGCAGCGTCGT GACTGTGCCCAGCAGCAGCCTGGGAACCCAGACCTAC ATCTGCAACGTGAACCACAAGCCCAGCAACACCAAGG TGGACAAGAAGGTGGAACCCAAGAGCTGCGACGGCGG AGGCGGATCTGGCGGCGGAGGATCCCAGGTGCAGCTG GTGCAGAGCGGAGCTGAAGTGAAAAAGCCTGGCTCCT CCGTGAAAGTGTCTTGTAAAGCCAGCGGCGGCACATTC TCATCCTACGCCATCAGCTGGGTGCGGCAGGCTCCAGG CCAGGGACTGGAATGGATGGGAGGAATTATCCCTATTT TTGGGACAGCCAATTATGCTCAGAAATTTCAGGGGCGC GTGACAATTACAGCCGACAAGTCCACCTCTACAGCTTA TATGGAACTGTCCTCCCTGCGCTCCGAGGATACAGCTG TGTATTACTGCGCCCGGTCCACACTGATCTATGGATAC TTTGATTATTGGGGGCAGGGAACAACAGTGACTGTGTC CTCCGCTAGCACCAAGGGCCCATCGGTCTTCCCCCTGG CACCCTCCTCCAAGAGCACCTCTGGGGGCACAGCGGCC CTGGGCTGCCTGGTCAAGGACTACTTCCCCGAACCGGT GACGGTGTCGTGGAACTCAGGCGCCCTGACCAGCGGC |
| | | GTGCACACCTTCCCGGCTGTCCTACAGTCCTCAGGACT CTACTCCCTCAGCAGCGTGGTGACCGTGCCCTCCAGCA GCTTGGGCACCCAGACCTACATCTGCAACGTGAATCAC AAGCCCAGCAACACCAAGGTGGACAAGAAAGTTGAGC CCAAATCTTGTGACAAAACTCACACATGCCCACCGTGC CCAGCACCTGAAGCTGCAGGGGGACCGTCAGTCTTCCT CTTCCCCCCAAAACCCAAGGACACCCTCATGATCTCCC GGACCCCTGAGGTCACATGCGTGGTGGTGGACGTGAG CCACGAAGACCCTGAGGTCAAGTTCAACTGGTACGTG GACGGCGTGGAGGTGCATAATGCCAAGACAAAGCCGC GGGAGGAGCAGTACAACAGCACGTACCGTGTGGTCAG CGTCCTCACCGTCCTGCACCAGGACTGGCTGAATGGCA AGGAGTACAAGTGCAAGGTCTCCAACAAAGCCCTCGG CGCCCCCATCGAGAAAACCATCTCCAAAGCCAAAGGG CAGCCCCGAGAACCACAGGTGTGCACCCTGCCCCCATC CCGGGATGAGCTGACCAAGAACCAGGTCAGCCTCTCG TGCGCAGTCAAAGGCTTCTATCCCAGCGACATCGCCGT GGAGTGGGAGAGCAATGGGCAGCCGGAGAACAACTAC AAGACCACGCCTCCCGTGCTGGACTCCGACGGCTCCTT CTTCCTCGTGAGCAAGCTCACCGTGGACAAGAGCAGGT GGCAGCAGGGGAACGTCTTCTCATGCTCCGTGATGCAT GAGGCTCTGCACAACCACTACACGCAGAAGAGCCTCT CCCTGTCTCCGGGTGGAGGCGGCGGAAGCGGAGGAGG AGGATCCGGCGGCGGAGGTTCCGGAGGCGGAGGATCC GAGGTGCAGCTGCTCGAAAGCGGCGGAGGACTGGTGC AGCCTGGCGGCAGCCTGAGACTGTCTTGCGCCGCCAGC GGCTTCACCTTCAGCAGCTACGCCATGAGCTGGGTCCG CCAGGCCCCTGGCAAGGGACTGGAATGGGTGTCCGCC ATCATCGGCTCTGGCGCCAGCACCTACTACGCCGACAG CGTGAAGGGCCGGTTCACCATCAGCCGGGACAACAGC AAGAACACCCTGTACCTGCAGATGAACAGCCTGCGGG CCGAGGACACCGCCGTGTACTACTGCGCCAAGGGATG GTTCGGCGGCTTCAACTACTGGGGACAGGGCACCCTGG TCACCGTGTCCAGC |
| 297 | (11D5) VLCL-light chain | see Table 48 |
| 351 | HC 1 (11D5) VHCH1 VHCH1 Fc knob VL (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSTLIYGYFDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCARSTLIYGYFDYWGQGTTVTVSSA STKGPSVFPLAPSSKSTSGGTAALGCLVKDYFPEPVTVSW NSGALTSGVHTFPAVLQSSGLYSLSSVVTVPSSSLGTQTYI CNVNHKPSNTKVDKKVEPKSCDKTHTCPPCPAPEAAGGP SVFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVYTLPPCRD ELTKNQVSLWCLVKGFYPSDIAVEWESNGQPENNYKTTP PVLDSDGSFFLYSKLTVDKSRWQQGNVFSCSVMHEALH NHYTQKSLSLSPGGGGGSGGGGSGGGGSGGGGSEIVLTQ |
| | | SPGTLSLSPGERATLSCRASQSVTSSYLAWYQQKPGQAPR LLINVGSRRATGIPDRFSGSGSGTDFTLTISRLEPEDFAVY YCQQGIMLPPTFGQGTKVEIK |
| 352 | HC 2 (11D5) VHCH1_VHCH1_Fc hole VH (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSTLIYGYFDYWGQGTTVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDGGGGSGGGGSQVQ LVQSGAEVKKPGSSVKVSCKASGGTFSSYAISWVRQAPG QGLEWMGGIIPIFGTANYAQKFQGRVTITADKSTSTAYM ELSSLRSEDTAVYYCARSTLIYGYFDYWGQGTTVTVSSA STKGPSVFPLAPSSKSTSGGTAALGCLVKDYFPEPVTVSW NSGALTSGVHTFPAVLQSSGLYSLSSVVTVPSSSLGTQTYI CNVNHKPSNTKVDKKVEPKSCDKTHTCPPCPAPEAAGGP SVFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVCTLPPSRD ELTKNQVSLSCAVKGFYPSDIAVEWESNGQPENNYKTTP PVLDSDGSFFLVSKLTVDKSRWQQGNVFSCSVMHEALH NHYTQKSLSLSPGGGGGSGGGGSGGGGSGGGGSEVQLLE SGGGLVQPGGSLRLSCAASGFTFSSYAMSWVRQAPGKGL EWVSAIIGSGASTYYADSVKGRFTISRDNSKNTLYLQMNS LRAEDTAVYYCAKGWFGGFNYWGQGTLVTVSS |
| 301 | (9B11) VLCL-light chain (nucleotide sequence) | see Table 48 |
| 353 | HC 1 (9B11) VHCH1_VHCH1_Fc knob VL (4B9) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGATCTTCT GGTGCTTACCCGGGTTACTTCGACTACTGGGGCCAAGG GACCACCGTGACCGTCTCCTCAGCTTCTACCAAGGGCC CCAGCGTGTTCCCTCTGGCCCCTAGCAGCAAGAGCACA TCTGGCGGAACAGCCGCCCTGGGCTGCCTCGTGAAGG ACTACTTTCCCGAGCCCGTGACAGTGTCCTGGAACTCT GGCGCCCTGACAAGCGGCGTGCACACCTTTCCAGCCGT GCTGCAGAGCAGCGGCCTGTACTCTCTGAGCAGCGTCG TGACTGTGCCCAGCAGCAGCCTGGGAACCCAGACCTA CATCTGCAACGTGAACCACAAGCCCAGCAACACCAAG GTGGACAAGAAGGTGGAACCCAAGAGCTGCGACGGCG GAGGCGGATCTGGCGGCGGAGGATCCCAGGTGCAATT GGTGCAGTCTGGGGCTGAGGTGAAGAAGCCTGGGTCC TCGGTGAAGGTCTCCTGCAAGGCCTCCGGAGGCACATT CAGCAGCTACGCTATAAGCTGGGTGCGACAGGCCCCT GGACAAGGGCTCGAGTGGATGGGAGGGATCATCCCTA TCTTTGGTACAGCAAACTACGCACAGAAGTTCCAGGGC AGGGTCACCATTACTGCAGACAAATCCACGAGCACAG CCTACATGGAGCTGAGCAGCCTGAGATCTGAGGACAC CGCCGTGTATTACTGTGCGAGATCTTCTGGTGCTTACC |
| | | CGGGTTACTTCGACTACTGGGGCCAAGGGACCACCGTG ACCGTCTCCTCAGCTAGCACCAAGGGCCCATCGGTCTT CCCCCTGGCACCCTCCTCCAAGAGCACCTCTGGGGGCA CAGCGGCCCTGGGCTGCCTGGTCAAGGACTACTTCCCC GAACCGGTGACGGTGTCGTGGAACTCAGGCGCCCTGA CCAGCGGCGTGCACACCTTCCCGGCTGTCCTACAGTCC TCAGGACTCTACTCCCTCAGCAGCGTGGTGACCGTGCC CTCCAGCAGCTTGGGCACCCAGACCTACATCTGCAACG TGAATCACAAGCCCAGCAACACCAAGGTGGACAAGAA AGTTGAGCCCAAATCTTGTGACAAAACTCACACATGCC CACCGTGCCCAGCACCTGAAGCTGCAGGGGGACCGTC AGTCTTCCTCTTCCCCCCAAAACCCAAGGACACCCTCA TGATCTCCCGGACCCCTGAGGTCACATGCGTGGTGGTG GACGTGAGCCACGAAGACCCTGAGGTCAAGTTCAACT GGTACGTGGACGGCGTGGAGGTGCATAATGCCAAGAC AAAGCCGCGGGAGGAGCAGTACAACAGCACGTACCGT GTGGTCAGCGTCCTCACCGTCCTGCACCAGGACTGGCT GAATGGCAAGGAGTACAAGTGCAAGGTCTCCAACAAA GCCCTCGGCGCCCCCATCGAGAAAACCATCTCCAAAGC CAAAGGGCAGCCCCGAGAACCACAGGTGTACACCCTG CCCCCCTGCAGAGATGAGCTGACCAAGAACCAGGTGT CCCTGTGGTGTCTGGTCAAGGGCTTCTACCCCAGCGAT ATCGCCGTGGAGTGGGAGAGCAACGGCCAGCCTGAGA ACAACTACAAGACCACCCCCCCTGTGCTGGACAGCGA CGGCAGCTTCTTCCTGTACTCCAAACTGACCGTGGACA AGAGCCGGTGGCAGCAGGGCAACGTGTTCAGCTGCAG CGTGATGCACGAGGCCCTGCACAACCACTACACCCAG AAGTCCCTGAGCCTGAGCCCCGGCGGAGGCGGCGGAA GCGGAGGAGGAGGATCTGGGGGCGGAGGTTCCGGAGG CGGTGGATCTGAGATCGTGCTGACCCAGTCTCCCGGCA CCCTGTCTCTGAGCCCTGGCGAGAGAGCCACCCTGTCC TGCAGAGCCTCCCAGTCCGTGACCTCCTCCTACCTCGC CTGGTATCAGCAGAAGCCCGGCCAGGCCCCTCGGCTGC TGATCAACGTGGGCAGTCGGAGAGCCACCGGCATCCC TGACCGGTTCTCCGGCTCTGGCTCCGGCACCGACTTCA CCCTGACCATCTCCCGGCTGGAACCCGAGGACTTCGCC GTGTACTACTGCCAGCAGGGCATCATGCTGCCCCCCAC CTTTGGCCAGGGCACCAAGGTGGAAATCAAG |
| 354 | HC 2 (9B11) VHCH1_VHCH1_Fc hole VH (4B9) (nucleotide sequence) | CAGGTGCAATTGGTGCAGTCTGGGGCTGAGGTGAAGA AGCCTGGGTCCTCGGTGAAGGTCTCCTGCAAGGCCTCC GGAGGCACATTCAGCAGCTACGCTATAAGCTGGGTGC GACAGGCCCCTGGACAAGGGCTCGAGTGGATGGGAGG GATCATCCCTATCTTTGGTACAGCAAACTACGCACAGA AGTTCCAGGGCAGGGTCACCATTACTGCAGACAAATCC ACGAGCACAGCCTACATGGAGCTGAGCAGCCTGAGAT CTGAGGACACCGCCGTGTATTACTGTGCGAGATCTTCT GGTGCTTACCCGGGTTACTTCGACTACTGGGGCCAAGG GACCACCGTGACCGTCTCCTCAGCTTCTACCAAGGGCC CCAGCGTGTTCCCTCTGGCCCCTAGCAGCAAGAGCACA TCTGGCGGAACAGCCGCCCTGGGCTGCCTCGTGAAGG ACTACTTTCCCGAGCCCGTGACAGTGTCCTGGAACTCT GGCGCCCTGACAAGCGGCGTGCACACCTTTCCAGCCGT GCTGCAGAGCAGCGGCCTGTACTCTCTGAGCAGCGTCG TGACTGTGCCCAGCAGCAGCCTGGGAACCCAGACCTA |
| | | CATCTGCAACGTGAACCACAAGCCCAGCAACACCAAG GTGGACAAGAAGGTGGAACCCAAGAGCTGCGACGGCG GAGGCGGATCTGGCGGCGGAGGATCCCAGGTGCAATT GGTGCAGTCTGGGGCTGAGGTGAAGAAGCCTGGGTCC TCGGTGAAGGTCTCCTGCAAGGCCTCCGGAGGCACATT CAGCAGCTACGCTATAAGCTGGGTGCGACAGGCCCCT GGACAAGGGCTCGAGTGGATGGGAGGGATCATCCCTA TCTTTGGTACAGCAAACTACGCACAGAAGTTCCAGGGC AGGGTCACCATTACTGCAGACAAATCCACGAGCACAG CCTACATGGAGCTGAGCAGCCTGAGATCTGAGGACAC CGCCGTGTATTACTGTGCGAGATCTTCTGGTGCTTACC CGGGTTACTTCGACTACTGGGGCCAAGGGACCACCGTG ACCGTCTCCTCAGCTAGCACCAAGGGCCCATCGGTCTT CCCCCTGGCACCCTCCTCCAAGAGCACCTCTGGGGGCA CAGCGGCCCTGGGCTGCCTGGTCAAGGACTACTTCCCC GAACCGGTGACGGTGTCGTGGAACTCAGGCGCCCTGA CCAGCGGCGTGCACACCTTCCCGGCTGTCCTACAGTCC TCAGGACTCTACTCCCTCAGCAGCGTGGTGACCGTGCC CTCCAGCAGCTTGGGCACCCAGACCTACATCTGCAACG TGAATCACAAGCCCAGCAACACCAAGGTGGACAAGAA AGTTGAGCCCAAATCTTGTGACAAAACTCACACATGCC CACCGTGCCCAGCACCTGAAGCTGCAGGGGGACCGTC AGTCTTCCTCTTCCCCCCAAAACCCAAGGACACCCTCA TGATCTCCCGGACCCCTGAGGTCACATGCGTGGTGGTG GACGTGAGCCACGAAGACCCTGAGGTCAAGTTCAACT GGTACGTGGACGGCGTGGAGGTGCATAATGCCAAGAC AAAGCCGCGGGAGGAGCAGTACAACAGCACGTACCGT GTGGTCAGCGTCCTCACCGTCCTGCACCAGGACTGGCT GAATGGCAAGGAGTACAAGTGCAAGGTCTCCAACAAA GCCCTCGGCGCCCCCATCGAGAAAACCATCTCCAAAGC CAAAGGGCAGCCCCGAGAACCACAGGTGTGCACCCTG CCCCCATCCCGGGATGAGCTGACCAAGAACCAGGTCA GCCTCTCGTGCGCAGTCAAAGGCTTCTATCCCAGCGAC ATCGCCGTGGAGTGGGAGAGCAATGGGCAGCCGGAGA ACAACTACAAGACCACGCCTCCCGTGCTGGACTCCGAC GGCTCCTTCTTCCTCGTGAGCAAGCTCACCGTGGACAA GAGCAGGTGGCAGCAGGGGAACGTCTTCTCATGCTCC GTGATGCATGAGGCTCTGCACAACCACTACACGCAGA AGAGCCTCTCCCTGTCTCCGGGTGGAGGCGGCGGAAG CGGAGGAGGAGGATCCGGCGGCGGAGGTTCCGGAGGC GGAGGATCCGAGGTGCAGCTGCTCGAAAGCGGCGGAG GACTGGTGCAGCCTGGCGGCAGCCTGAGACTGTCTTGC GCCGCCAGCGGCTTCACCTTCAGCAGCTACGCCATGAG CTGGGTCCGCCAGGCCCCTGGCAAGGGACTGGAATGG GTGTCCGCCATCATCGGCTCTGGCGCCAGCACCTACTA CGCCGACAGCGTGAAGGGCCGGTTCACCATCAGCCGG GACAACAGCAAGAACACCCTGTACCTGCAGATGAACA GCCTGCGGGCCGAGGACACCGCCGTGTACTACTGCGCC AAGGGATGGTTCGGCGGCTTCAACTACTGGGGACAGG GCACCCTGGTCACCGTGTCCAGC |
| 301 | (9B11) VLCL-light chain | see Table 48 |
| 355 | HC 1 (9B11) | QVQLVQSGAEVKKPGSSVKVSCKASGGTFSSYAISWVRQ APGQGLEWMGGIIPIFGTANYAQKFQGRVTITADKSTSTA |
| | VHCH1_VHCH1_Fc knob VL (4B9) | YMELSSLRSEDTAVYYCARSSGAYPGYFDYWGQGTTVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDGGGGSGGGGSQ VQLVQSGAEVKKPGSSVKVSCKASGGTFSSYAISWVRQA PGQGLEWMGGIIPIFGTANYAQKFQGRVTITADKSTSTAY MELSSLRSEDTAVYYCARSSGAYPGYFDYWGQGTTVTV SSASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDKTHTCPPCPAPEA AGGPSVFLFPPKPKDTLMISRTPEVTCVVVDVSHEDPEVK FNWYVDGVEVHNAKTKPREEQYNSTYRVVSVLTVLHQD WLNGKEYKCKVSNKALGAPIEKTISKAKGQPREPQVYTL PPCRDELTKNQVSLWCLVKGFYPSDIAVEWESNGQPENN YKTTPPVLDSDGSFFLYSKLTVDKSRWQQGNVFSCSVMH EALHNHYTQKSLSLSPGGGGGSGGGGSGGGGSGGGGSEI VLTQSPGTLSLSPGERATLSCRASQSVTSSYLAWYQQKPG QAPRLLINVGSRRATGIPDRFSGSGSGTDFTLTISRLEPEDF AVYYCQQGIMLPPTFGQGTKVEIK |
| 356 | HC 2 (9B11) VHCH1_VHCH1_Fc hole VH (4B9) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARSSGAYPGYFDYWGQGTTVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDGGGGSGGGGSQ VQLVQSGAEVKKPGSSVKVSCKASGGTFSSYAISWVRQA PGQGLEWMGGIIPIFGTANYAQKFQGRVTITADKSTSTAY MELSSLRSEDTAVYYCARSSGAYPGYFDYWGQGTTVTV SSASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDKTHTCPPCPAPEA AGGPSVFLFPPKPKDTLMISRTPEVTCVVVDVSHEDPEVK FNWYVDGVEVHNAKTKPREEQYNSTYRVVSVLTVLHQD WLNGKEYKCKVSNKALGAPIEKTISKAKGQPREPQVCTL PPSRDELTKNQVSLSCAVKGFYPSDIAVEWESNGQPENN YKTTPPVLDSDGSFFLVSKLTVDKSRWQQGNVFSCSVMH EALHNHYTQKSLSLSPGGGGGSGGGGSGGGGSGGGGSE VQLLESGGGLVQPGGSLRLSCAASGFTFSSYAMSWVRQA PGKGLEWVSAIIGSGASTYYADSVKGRFTISRDNSKNTLY LQMNSLRAEDTAVYYCAKGWFGGFNYWGQGTLVTVSS |

**Table 67: Biochemical analysis of exemplary bispecific, tetravalent anti-4-1BB/anti-FAP IgG1 P329G LALA antigen binding molecules (4+1 constructs)**

| **Clone** | **Yield [mg/l]** | **Monomer [%]** | **CE-SDS (nonred)** |
|---|---|---|---|
| 4+1 2B3/FAP (hole-VL) | 0.7 | 97 | 96 |
| 4+1 25G7/FAP (hole-VH) | 10 | 95 | 90 |
| 4+1 11D5/FAP (hole-VH) | 0.4 | 94 | 95 |

### 9.6 Characterization of bispecific tetravalent antibodies targeting 4-1BB and fibroblast activation protein (FAP)

### 9.6.1 Surface plasmon resonance (simultaneous binding)

The capacity of binding simultaneously human 4-1BB Fc(kih) and human FAP was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany).

Biotinylated human 4-1BB Fc(kih) was directly coupled to a flow cell of a streptavidin (SA) sensor chip. Immobilization levels up to 400 resonance units (RU) were used. The bispecific antibodies targeting 4-1BB and FAP were passed at a concentration range of 200 nM with a flow of 30 µL/minute through the flow cells over 90 seconds and dissociation was set to zero sec. Human FAP was injected as second analyte with a flow of 30 µL/minute through the flow cells over 90 seconds at a concentration of 500 nM. The dissociation was monitored for 120 sec. Bulk refractive index differences were corrected for by subtracting the response obtained in a reference flow cell, where no protein was immobilized.

All bispecific constructs could bind simultaneously to human 4-1BB and human FAP as shown in **Figures 41B-41D****.**

### 9.6.2 Binding to human 4-1BB - Competition assay of bivalent 4-1BB antibody vs tetravalent anti-4-1BB antigen binding molecules

To confirm the ability of all four anti-4-1BB Fab domains to bind to human 4-1BB, a cell-based FRET assay (TagLite) was applied. Therefore, 2500 Hek293 EBNA cells/well transfected with a hu4-1BB-SNAP fusion protein and labeled with the FRET donor Terbium (Cisbio) were mixed with either 0.6 nM anti-4-1BB Urelumab or 0.39 nM anti-4-1BB 12B3 labeled with the FRET acceptor d2 (Cisbio). Additionally, a concentration dilution ranging from 0.006-1000 nM of the unlabeled IgG (12B3) or tetravalent (12B3/4B9 4+1) bispecific antibody was added and incubated for 2 to 4 hours at RT. The fluorescent signal was measured at 620 nm for the fluorescent donor (Terbium) and at 665 nm for the fluorescent acceptor dye (M100 Pro, Tecan). The ratio of 665/620^{∗}1000 was calculated, and the reference (cells only) was subtracted. For EC₅₀ determination the results were analysed in Graph Pad Prism6. The observed EC₅₀ values after 4 hour incubation are shown in **Table 68.** The corresponding curves are shown in **Figure 42**.

**Table 68: EC₅₀ values for competitive binding of bivalent vs tetravalent 4-1BB binding molecules**

| | |
|---|---|
| Construct | EC₅₀ (nM) |
| 12B3 IgG | 1.4 (0.9-2.1) |
| 4-1BB(12B3)/ FAP(4B9) 4+1 | 0.3 (0.2-0.4) |

### 9.6.3 Binding on cells

### 9.6.3.1 Binding on human 4-1BB expressing cells: resting and activated human peripheral mononuclear blood leukocytes (PBMC)

Expression of human 4-1BB is absent on resting (naive) human T cells (Kienzle G. and von Kempis J (2000), Int. Immunol. 12(1):, 73-82, Wen T. et al. (2002), J. Immunol. 168, 4897-4906). After activation with immobilized anti-human CD3 agonistic antibody, 4-1BB is upregulated on CD4⁺ and CD8⁺ T cells. 4-1BB expression has also been reported on activated human NK cells (Baessler T. et. al. (2010) Blood 115(15), 3058-3069), activated human NKT cells (Cole S.L. et al. (2014) J. Immunol. 192(8), 3898-3907), activated human B cells (Zhang et al. (2010) J. Immunol. 184(2), 787-795), activated human eosinophils (Heinisch et al. 2001), constitutively on human neutrophils (Heinisch I.V. (2000) J Allergy Clin Immunol. 108(1), 21-28), activated human monocytes (Langstein J.et al. (1998) J Immunol. 160(5), 2488-2494, Kwajah M. and Schwarz H. (2010) Eur J Immunol. 40(7), 1938-1949), constitutively on human regulatory T cells (Bacher P. et al. (2014) Mucosal Immunol. 7(4), 916-928), human follicular dendritic cells (Pauly S. et al. (2002) J Leukoc Biol. 72(1), 35-42), activated human dendritic cells (Zhang L. et al. (2004) Cell Mol Immunol. 1(1), 71-76) and on blood vessels of malignant human tumors (Broll K. et al. (2001) Am J Clin Pathol. 115(4), 543-549).

To test binding of our anti-4-1BB clones to naturally cell-expressed human 4-1BB, resting peripheral blood mononuclear cells (PBMCs) or PHA-L/Proleukin pre-activated and CD3/CD28-reactivated PBMC were used. PBMCs from buffy coats obtained from the Zurich blood donation center were isolated by ficoll density centrifugation using Histopaque 1077 (SIGMA Life Science, Cat.-No. 10771, polysucrose and sodium diatrizoate, adjusted to a density of 1.077 g/mL) and resuspended in T cell medium consisting of RPMI 1640 medium (Gibco by Life Technology, Cat.-No. 42401-042) supplied with 10 % Fetal Bovine Serum (FBS, Gibco by Life Technology, Cat.-No. 16000-044, Lot 941273, gamma-irradiated, mycoplasma-free and heat inactivated at 56 °C for 35 min), 1 % (v/v) GlutaMAX-I (GIBCO by Life Technologies, Cat.-No. 35050 038), 1 mM Sodium Pyruvate (SIGMA, Cat.-No. S8636), 1 % (v/v) MEM non-essential amino acids (SIGMA, Cat.-No. M7145) and 50 µM β-Mercaptoethanol (SIGMA, M3148). PBMCs were used directly after isolation (resting cells) or stimulated to induce 4-1BB expression at the cell surface of T cells by culturing for 3 to 5 days in T cell medium supplemented with 200 U/mL Proleukin (Novartis Pharma Schweiz AG, CHCLB-P-476-700-10340) and 2 µg/mL PHA-L (SIGMA Cat.-No. L2769) in a 6-well tissue culture plate and then 2 day in a 6-well tissue culture plate coated with 10 µg/mL anti-human CD3 (clone OKT3, BioLegend, Cat.-No. 317315) and 2 µg/mL anti-human CD28 (clone CD28.2, BioLegend, Cat.-No.: 302928) in T cell medium at 37 °C and 5% CO₂.

To determine binding to human 4-1BB expressed by human PBMCs, 0.1-0.2 × 10⁶ freshly isolated e.g. resting or activated PBMCs were added to each well of a round-bottom suspension cell 96-well plates (Greiner bio-one, cellstar, Cat.-No. 650185). Plates were centrifuged 4 minutes with 400 x g at 4 °C and supernatant was discarded. Cells were washed with 200 µL/well DPBS and then incubated for 30 min at 4°C with 100 µL/mL DPBS containing 1:5000 diluted Fixable Viability Dye eFluor 660 (eBioscience, Cat.-No. 65-0864-18). Afterwards cells were washed once with 200 µL/well cold FACS buffer (DPBS supplied with 2 % (v/v) FBS, 5 mM EDTA pH8 (Amresco, Cat. No. E177) and 7.5 mM sodium azide (Sigma-Aldrich S2002)). Next, 50 µL/well of 4 °C cold FACS buffer containing titrated anti-human 4-1BB binders were added and cells were incubated for 120 minutes at 4 °C. Cells were washed four times with 200 µL/well 4 °C FACS buffer to remove onbound molecules. Afterwards cells were further incubated with 50 µL/well of 4 °C cold FACS buffer containing 2.5 µg/mL PE-conjugated AffiniPure anti-human IgG Fcγ-fragment-specific goat F(ab')2 fragment (Jackson ImmunoResearch, Cat.-No. 109-116-098), anti-human CD45 AF488 (clone HI30, BioLegend, Cat.-No. 304019), 0.67 µg/mL PerCP/Cy5.5-conjugated anti-human CD3 mouse IgG1 κ (clone UCHT1, BioLegend, Cat.-No. 300430), 0.125 µg/mL BV421-conjugated anti-human CD4 moIgG1κ (clone RPA-T4, BioLegend, Cat.-No. 300532) or 0.23 µg/mL BV421-conjugated anti-human CD4 mouse IgG2b κ (clone OKT4, BioLegend, Cat.-No. 317434, 0.33 µg/mL anti-human CD8-BV510 (moIgG1κ, clone SKI, BioLegend, Cat.-No. 344732) and 0.67 µg/mL anti-human CD19-PE/Cy7 (moIgG1κ, clone HIB19, BioLegend, Cat.-No. 302216) and incubated for 30 minutes at 4 °C.

Cells were washed twice with 200 µL FACS buffer/well and fixated by resuspending in 50 µL/well DPBS containing 1 % Formaldehyde (Sigma, HT501320-9.5L). Cells were acquired the same or next day using 3-laser MACSQuant Analyzer 10 (Miltenyi Biotech). Gates were set on CD8⁺ and CD4⁺ T cells and the geo mean of fluorescence intensity (MFI) of the secondary detection antibody was used to analyze binding of primary antibodies. Using Graph Pad Prism (Graph Pad Software Inc.) data was baselined by subtracting the blank values (no primary antibody added) and the EC₅₀ values were calculated using non-linear regression curve fit (robust fit).

Human T cells lack **4-1BB** expression in a resting status but upregulate **4-1BB** after activation. Human CD8⁺ T cells show a stronger up-regulation than CD4⁺ T cells. The generated anti-human 4-1BB-specific antibodies can bind to human **4-1BB** expressed by activated human T cells as shown in **Figure 43B and 43D** whereas on resting CD4 and CD8 T cells no significant binding can be detected **(****Figure 43A and 43C****).** Binding is influenced by the **4-1BB** expression level of the cells, e.g. 4-1BB-specific molecules bind stronger to activated CD8⁺ T cells **(****Figure 43D****)** than to activated CD4⁺ T cells **(****Figure 43B****).** There is also a difference observed by the format of the molecules, e.g. monovalent 4-1BB-binders bind differently than bivalent or tetravalent 4-1BB antigen binding molecules. Monovalent 4-1BB-binding leads to an increase of the EC₅₀ value and decrease of MFI due to lower binding affinity, the tetravalent 4-1BB-binders may show a different binding curve because of stronger affinity but also due to internal competition for 4-1BB-specific epitope. Molecules containing Fc-fused FAP-targeting domains (e.g. FAP-targeted 2+2 or 4+1 but not 1+1 formats) may mask epitopes of the polyclonal secondary detection antibody which could influence the MFI and EC₅₀ values. The EC₅₀ values of binding to activated CD8⁺ T cells are shown in **Table 69.** In **Figure 44** the area under the curve (AUC) of binding curves shown in **Figures 43** are summarized.

**Table 69: EC₅₀ values of binding to activated human CD8⁺ T cells**

| | |
|---|---|
| Clone | EC₅₀ [nM] |
| 12B3 IgG | 0.4 |
| 4-1BB(12B3)/ FAP(4B9) 1+1 | 8 |
| 4-1BB(12B3)/ FAP(4B9) 2+2 | 2 |
| 4-1BB(12B3)/ FAP(4B9) 4+1 | n.d. (plateau not reached) |

### 9.6.3.2 Binding to human FAP-expressing tumor cells

For binding to cell-surface-expressed human Fibroblast Activation Protein (FAP) NIH/3T3-huFAP clone 19 cells or human melanoma cell line WM-266-4 (ATCC CRL-1676) were used. NIH/3T3-huFAP clone 19 was generated by transfection of mouse embryonic fibroblast NIH/3T3 cells (ATCC CRL-1658) with the expression pETR4921 plasmid encoding human FAP under a CMV promoter. Cells were maintained in the presence of 1.5 µg/mL puromycin (InvivoGen, Cat.-No.: ant-pr-5). 2 × 10⁵ of FAP expressing tumor cells were added to each well of a round-bottom suspension cell 96-well plates (Greiner bio-one, cellstar, Cat.-No. 650185). Cells were washed once with 200 µL DPBS and pellets were resuspended in 100 µL/well of 4 °C cold DPBS buffer containing 1:5000 diluted Fixable Viability Dye eFluor 450 (eBioscience, Cat. No. 65 0863 18) or or Fixable Viability Dye eFluor 660 (eBioscience, Cat.-No. 65-0864-18). Plates were incubated for 30 minutes at 4°C and washed once with 200 µL 4 °C cold DPBS buffer. Afterwards cells were resuspended in 50 µL/well of 4 °C cold FACS buffer containing different titrated concentrations of 4-1BB-specific FAP-targeted and non-targeted antibodies, followed by incubation for 1 hour at 4 °C. After washing four times with with 200 µL/well, cells were stained with 50 µL/well of 4 °C cold FACS buffer containing 2.5 µg/mL PE-conjugated AffiniPure anti-human IgG Fcy-fragment-specific goat F(ab')2 fragment (Jackson ImmunoResearch, Cat.-No. 109-116-098) or 30 µg/mL FITC-conjugated AffiniPure anti-human IgG Fcy-fragment-specific goat F(ab')2 fragment (Jackson ImmunoResearch, Cat. No. 109 096 098) for 30 minutes at 4 °C. Cells were washed twice with 200 µL 4 °C FACS buffer and then resuspended in 50 µL/well DPBS containing 1 % Formaldehyde for fixation. The same or the next day cells were resuspended in 100 µL FACS-buffer and acquired using 5-laser LSR-Fortessa (BD Bioscience with DIVA software) or MACSQuant Analyzer 10 (Miltenyi Biotec).

As shown in **Figures 45A and 45B****,** the FAP-targeted molecules, but not the DP47-targeted or parental huIgG1 P293G LALA clone 12B3 bind efficiently to human FAP-expressing WM-266-4 (A) and NIH/3T3-huFAP clone 19 cells (B). Therefore including a FAP-binding side induces an efficient targeting effect to human FAP-expressing cells. The moiety of FAP-binding sides (e.g. 1+1 or 2+2 or 4+1) as well as the FAP-binding clone (28H1 or 4B9) play a role and influence the EC₅₀ **(Table 70)** and AUC **(****Figure 46****)** of binding to FAP-expressing cells. The 4-1BB (12B3) x FAP (28H1) 2+2 (black filled triangle), 4-1BB (12B3) x FAP (28H1) 1+1 (grey half-filled circle) and 4-1BB (12B3) x FAP (4B9) 4+1 (grey half-filled square) show differences in binding **(****Figure 45B****)** which may also influence their cross-linking and therefore also the activation potential (shown in **Figures 47A****-47K** and discussed in Example 10).

**Table 70: EC₅₀ values of binding to FAP expressing cell line NIH/3T3-huFAP clone 19 and WM-266-4**

| Clone | EC₅₀ [nM] with NIH/3T3-huFAP clone 19 cells | EC₅₀ [nM] with WM-266-4 cells |
|---|---|---|
| 12B3 IgG | n.d. | n.d. |
| 4-1BB(12B3)/ FAP(4B9) 1+1 | 2.4 | 1.7 |
| 4-1BB(12B3)/ FAP(4B9) 2+2 | 12.2 | n.d. (plateau not reached) |
| 4-1BB(12B3)/ FAP(4B9) 4+1 | n.d. (plateau not reached) | 9.4 |

### Example 10

### Functional properties of bispecific anti-human 4-1BB binding molecules 10.1 NFκB activation

### 10.1.1 Generation of HeLa cells expressing human 4-1BB and NF-κB-luciferase

The cervix carcinoma cell line HeLa (ATCC CCL-2) was transduced with a plasmid based on the expression vector pETR10829, which contains the sequence of human 4-1BB (Uniprot accession Q07011) under control of a CMV-promoter and a puromycin resistance gene. Cells were cultured in DMEM medium supplemented with 10 % (v/v) FBS, 1 % (v/v) GlutaMAX-I and 3 µg/mL puromycin.

4-1BB-transduced HeLa cells were tested for 4-1BB expression by flow cytometry: 02×10⁶ living cells were resuspended in 100 µL FACS buffer containing 0.1 µg PerCP/Cy5.5 conjugated anti-human 4-1BB mouse IgG1κ clone 4B4-1 (BioLegend Cat. No.309814) or its isotype control (PerCP/Cy5.5 conjugated mouse IgG1κ isotype control antibody clone MOPC 21, BioLegend Cat. No.400150) and incubated for 30 minutes at 4 °C. Cells were washed twice with FACS buffer, resuspended in 300 µL FACS buffer containing 0.06 µg DAPI (Santa Cruz Biotec, Cat. No. Sc-3598) and acquired using a 5-laser LSR-Fortessa (BD Bioscience, DIVA software). Limited dilutions were performed to generate single clones as described: human-4-1BB-transduced HeLa cells were resuspended in medium to a density of 10, 5 and 2.5 cells/ml and 200 µl of cell suspensions were transferred to round bottom tissue-culture treated 96-well plates (6 plates/cell concentration, TPP Cat. No. 92697). Single clones were harvested, expanded and tested for 4-1BB expression as described above. The clone with the highest expression of 4-1BB (clone 5) was chosen for subsequent transfection with the NF-κB-luciferase expression-vector 5495p Tranlucent HygB. The vector confers transfected cells both with resistance to Hygromycin B and capacity to express luciferase under control of NF-kB-response element (Panomics, Cat. No. LR0051). For transfection Human-4-1BB HeLa clone 5 cells were cultured to 70 % confluence. 50 µg (40 µL) linearized (restriction enzymes AseI and SalI) 5495p Tranlucent HygB expression vector were added to a sterile 0.4 cm Gene Pulser/MicroPulser Cuvette (Biorad, Cat.-No, 165-2081). 2.5×10⁶ human-4-1BB HeLa clone 5 cells in 400 µl supplement-free DMEM medium were added and mixed carefully with the plasmid solution. Transfection of cells was performed using a Gene Pulser Xcell total system (Biorad, Cat No. 165 2660) under the following settings: exponential pulse, capacitance 500 µF, voltage 160 V, resistance ∞. Immediately after the pulse transfected cells were transferred to a 75 cm² tissue culture flask (TPP, Cat. No. 90075) with 15 mL 37°C warm DMEM medium supplied with 10 % (v/v) FBS and 1 % (v/v) GlutaMAX I. On the next day, culture medium containing 3 µg/mL Puromycin and 200 µg/mL Hygromycin B (Roche, Cat. No. 10843555001) was added. Surviving cells were expanded and limited dilution was performed as described above to generate single clones.

Clones were tested for 4-1BB expression as described above and for NF-κB-Luciferase activity as following: Clones were harvested in selection medium and counted using a Cell Counter Vi-cell xr 2.03 (Beckman Coulter, Cat. No. 731050). Cells were set to a cell density of 0.33×10⁶ cells/mL and 150 µL of this cell suspension were transferred to each well of a sterile white 96-well flat bottom tissue culture plate with lid (greiner bio one, Cat. No. 655083). Cells were incubated at 37°C and 5 % CO₂ overnight in a cell incubator (Hera Cell). The next day 50 µL of medium containing different concentrations of recombinant human tumor necrosis factor alpha (rhTNFα, PeproTech, Cat.-No. 300 01A) were added to each well of a 96-well plate resulting in final concentration of rhTNFα of 100, 50, 25, 12.5, 6.25 and 0 ng/well. Cells were incubated for 6 hours at 37°C and 5 % CO₂ and then washed three times with 200µL/well DPBS. Reporter Lysis Buffer (Promega, Cat-No: E3971) was added to each well (40 µl) and the plates were stored over night at -20°C. The next day frozen cell and Detection Buffer (Luciferase 1000 Assay System, Promega, Cat. No. E4550) were thawed to room temperature. 100 uL of detection buffer were added to each well and the plate was measured as fast as possible using a SpectraMax M5/M5e microplate reader and the SoftMax Pro Software (Molecular Devices). Measured units of released light for 500 ms/well (URLs) above control (no rhTNFα added) were taken as luciferase activity. The HeLa-hu4-1BB-NF-κB-luc clone 26 exhibiting the highest luciferase activity and a considerable level of 4-1BB-expression and was chosen for further use.

### 10.1.2 NFκB activation in HeLa cells expressing human 4-1BB co-cultured with FAP-expressing tumor cells

HeLa-hu4-1BB-NF-κB-luc clone 26 cells were harvested and resuspended in DMEM medium supplied with 10 % (v/v) FBS and 1 % (v/v) GlutaMAX-I to a concentration of 0.2 × 10⁶ cells/ml. 100 µl (2 × 10⁴ cells) of this cell suspension were transferred to each well of a sterile white 96-well flat bottom tissue culture plate with lid (greiner bio one, Cat. No. 655083) and the plate were incubated at 37 °C and 5 % CO₂ overnight. The next day 50 µL of medium containing titrated FAP-targeted anti-human 4-1BB constructs or their parental huIgG1 P329G LALA antibodies were added. FAP-expressing NIH/3T3-huFAP clone 19 or WM-266-4 were resuspended in DMEM medium supplied with 10 % (v/v) FBS and 1 % (v/v) GlutaMAX-I to a concentration of 2 × 10⁶ cells/ml.

Suspension of FAP-expressing tumor cell (50 µl) or medium as negative control was added to each well and plates were incubated for 6 hours at 37 °C and 5 % CO₂ in the cell incubator. Cells were washed twice with 200 µL/well DPBS. 40 µl freshly prepared Reporter Lysis Buffer (Promega, Cat-No: E3971) were added to each well and the plate were stored over night at -20 °C. The next day frozen cell plates and Detection Buffer (Luciferase 1000 Assay System, Promega, Cat. No. E4550) were thawed at room temperature. 100 µL of detection buffer were added to each well and luciferase activity was measured as fast as possible using a SpectraMax M5/M5e microplate reader and a SoftMax Pro Software (Molecular Devices).

In **Figures 47A****-47K** the activation property of different FAP-targeted 4-1BB-specific constructs are shown. In the upper panels the activation property in the absence of FAP is shown (**Figure 47A****, D and G**). In the absence of FAP no activation can be detected independent of the 4-1BB-binding clone. In the middle panels the activation in the presence of intermediate FAP-expressing human WM-266-4 is shown. Only in the presence of 4+1 and 2+1 FAP (4B9)-targeted constructs a NFκB-mediated luciferase activation can be observed; namely: 4-1BB (12B3) x FAP (4B9) 4+1 (half filled grey squares, half dotted line) in **Figure 47B****,** 4-1BB (11D5) x FAP (4B9) 4+1 (grey star, dotted line) and) and 4-1BB (11D5) x FAP (4B9) 2+1 (black cross, dotted line) in **Figure 47E** as well as 4-1BB (25G7) x FAP (4B9) 4+1 (up-showing half filed black triangle, dotted line) and 4-1BB (25G7) x FAP (4B9) 2+1 (half filled down-showing black triangle) in **Figure 47I.** In the lower panels the activation in the presence of high FAP-expressing NIH/3T3-huFAP clone 19 is shown. Here not only the 4+1 and 2+1 FAP (4B9)-targeted constructs but also the 2+2 and 1+1 FAP (28H1)-targeted constructs can induce a NFκB-mediated luciferase activation in the reporter cell line. These results are also summarized in **Figures 48A** **and B** as area under the curve (AUC) of the activation curves. The used formats and FAP-binding clones are indicated as pictograms below the graph, the used clones are indicated by the column pattern. Clearly the tetravalent 4-1BB x FAP 4+1 molecules induced the strongest NFkB/Luciferase-activation in the 4-1BB expressing reporter cell line.

**Table 71: EC₅₀ values of activation of the NFκB signaling pathway in the presence of FAP-expressing tumor cells**

| Clone | EC₅₀ [nM] with NIH/3T3-huFAP clone 19 | EC₅₀ [nM] with WM-266-4 |
|---|---|---|
| 12B3 IgG | n.d. | n.d. |
| 4-1BB(12B3)/ FAP(4B9) 1+1 | 0.9 | n.d. |
| 4-1BB(12B3)/ FAP(4B9) 2+2 | 0.2 | n.d. |
| 4-1BB(12B3)/ FAP(4B9) 4+1 | 0.09 | 0.1 |
| 25G7 IgG | n.d. | n.d. |
| 4-1BB(25G7)/ FAP(28H1) 1+1 | 3.2 | n.d. |
| 4-1BB(25G7)/ FAP(28H1) 2+2 | 0.05 | n.d. |
| 4-1BB(25G7)/ FAP(4B9) 2+1 | 0.1 | 0.3 |
| 4-1BB(25G7)/FAP(4B9) 4+1 | 0.04 | 0.2 |
| 11D5 IgG | n.d. | n.d. |
| 4-1BB(11D5)/ FAP(28H1) 2+2 | 0.05 | n.d. |
| 4-1BB(11D5)/ FAP(4B9) 2+1 | 0.3 | 0.4 |
| 4-1BB(11D5)/ FAP(4B9) 4+1 | 0.06 | 0.1 |

### Example 11

### Generation of GITR antibodies and tool binders

### 11.1 Preparation, purification and characterization of antigens Fc fusion as screening tools for the generation of novel GITR binders by Phage Display

DNA sequences encoding the ectodomains of human, mouse or cynomolgus GITR (Table 72) were fused in frame with the human IgG1 heavy chain CH2 and CH3 domains on the Fc-knob (Merchant et al., 1998). An IgAse protease cleavage site was introduced between an antigen ectodomain and the Fc of human IgG1. An Avi tag for directed biotinylation was introduced at the C-terminus of the antigen-Fc-knob fusion. Combination of the antigen-Fc knob chain containing the S354C/T366W mutations, with an Fc-hole chain containing the Y349C/T366S/L368A/Y407V mutations allows generation of a heterodimer with one GITR ectodomain chain, thus creating a monomeric form of Fc-linked (**Figure 1A**). **Table 73** shows the cDNA and amino acid sequences of the antigen Fc-fusion constructs.

**Table 72: Amino acid numbering of antigen ectodomains (ECD) and their origin**

| **SEQ ID NO:** | **Construct** | **Origin** | **ECD** |
|---|---|---|---|
| 357 | human GITR ECD | synthetized according to Q9Y5U5 | aa 26-162 |
| 358 | cynomolgus GITR ECD | isolated from cynomolgus blood | aa 20-156 |
| 359 | murine GITR ECD | synthetized according to 035714 | aa 20-153 |

**Table 73: cDNA and amino acid sequences of monomeric antigen Fc(kih) fusion molecules (produced by combination of one Fc hole chain with one antigen Fc knob chain)**

| SEQ ID NO: | Antigen | Sequence |
|---|---|---|
| 95 | Nucleotide sequence Fc hole chain | |
| 360 | Nucleotide sequence human GITR antigen Fc knob chain | GACAAAACTCACACATGCCCACCGTGCCCAGCACCTGAA CTCCTGGGGGGACCGTCAGTCTTCCTCTTCCCCCCAAAAC CCAAGGACACCCTCATGATCTCCCGGACCCCTGAGGTCAC ATGCGTGGTGGTGGACGTGAGCCACGAAGACCCTGAGGT CAAGTTCAACTGGTACGTGGACGGCGTGGAGGTGCATAA TGCCAAGACAAAGCCGCGGGAGGAGCAGTACAACAGCAC GTACCGTGTGGTCAGCGTCCTCACCGTCCTGCACCAGGAC TGGCTGAATGGCAAGGAGTACAAGTGCAAGGTCTCCAAC AAAGCCCTCCCAGCCCCCATCGAGAAAACCATCTCCAAA GCCAAAGGGCAGCCCCGAGAACCACAGGTGTGCACCCTG CCCCCATCCCGGGATGAGCTGACCAAGAACCAGGTCAGC CTCTCGTGCGCAGTCAAAGGCTTCTATCCCAGCGACATCG CCGTGGAGTGGGAGAGCAATGGGCAGCCGGAGAACAACT ACAAGACCACGCCTCCCGTGCTGGACTCCGACGGCTCCTT CTTCCTCGTGAGCAAGCTCACCGTGGACAAGAGCAGGTG GCAGCAGGGGAACGTCTTCTCATGCTCCGTGATGCATGAG GCTCTGCACAACCACTACACGCAGAAGAGCCTCTCCCTGT CTCCGGGTAAA CAGAGGCCCACAGGCGGCCCTGGCTGTGGACCTGGCAGA CTGCTGCTGGGCACCGGCACCGATGCAAGATGCTGTAGA GTGCACACCACCAGATGCTGCCGGGACTACCCTGGCGAA GAGTGCTGCAGCGAGTGGGACTGTATGTGCGTGCAGCCC GAGTTCCACTGCGGCGACCCCTGCTGCACCACCTGTAGAC ACCACCCTTGCCCTCCCGGCCAGGGCGTGCAGAGCCAGG GCAAGTTCAGCTTCGGCTTCCAGTGCATCGACTGCGCCAG CGGCACCTTCTCTGGCGGCCACGAGGGACACTGCAAGCC CTGGACCGACTGTACCCAGTTCGGCTTCCTGACCGTGTTC CCCGGCAACAAGACCCACAACGCCGTGTGCGTGCCCGGC AGCCCTCCTGCTGAAGTCGACGGTGGTAGTCCGACACCTC CGACACCCGGGGGTGGTTCTGCAGACAAAACTCACACAT GCCCACCGTGCCCAGCACCTGAACTCCTGGGGGGACCGT CAGTCTTCCTCTTCCCCCCAAAACCCAAGGACACCCTCAT GATCTCCCGGACCCCTGAGGTCACATGCGTGGTGGTGGAC GTGAGCCACGAAGACCCTGAGGTCAAGTTCAACTGGTAC GTGGACGGCGTGGAGGTGCATAATGCCAAGACAAAGCCG CGGGAGGAGCAGTACAACAGCACGTACCGTGTGGTCAGC GTCCTCACCGTCCTGCACCAGGACTGGCTGAATGGCAAG GAGTACAAGTGCAAGGTCTCCAACAAAGCCCTCCCAGCC CCCATCGAGAAAACCATCTCCAAAGCCAAAGGGCAGCCC CGAGAACCACAGGTGTACACCCTGCCCCCATGCCGGGAT GAGCTGACCAAGAACCAGGTCAGCCTGTGGTGCCTGGTC AAAGGCTTCTATCCCAGCGACATCGCCGTGGAGTGGGAG AGCAATGGGCAGCCGGAGAACAACTACAAGACCACGCCT CCCGTGCTGGACTCCGACGGCTCCTTCTTCCTCTACAGCA AGCTCACCGTGGACAAGAGCAGGTGGCAGCAGGGGAACG TCTTCTCATGCTCCGTGATGCATGAGGCTCTGCACAACCA CTACACGCAGAAGAGCCTCTCCCTGTCTCCGGGTAAATCC GGAGGCCTGAACGACATCTTCGAGGCCCAGAAGATTGAA TGGCACGAG |
| 361 | Nucleotide sequence cynomolgus GITR antigen Fc knob chain | CAGAGGCCTACAGGCGGCCCTGGATGTGGACCTGGCAGA CTGCTGCTGGGCACAGGCAAGGATGCCCGGTGCTGTAGA GTGCACCCCACCAGATGCTGCCGGGACTACCAGGGCGAG GAGTGCTGCAGCGAGTGGGACTGCGTGTGCGTGCAGCCT GAGTTCCACTGCGGCAACCCCTGCTGCACCACCTGTCAGC ACCACCCTTGTCCTAGCGGACAGGGCGTGCAGCCCCAGG GCAAGTTCAGCTTCGGCTTCAGATGCGTGGACTGCGCCCT GGGCACCTTCAGCAGAGGACACGATGGCCACTGCAAGCC CTGGACCGACTGTACCCAGTTCGGCTTCCTGACCGTGTTC CCCGGCAACAAGACCCACAACGCCGTGTGTGTGCCTGGC AGCCCTCCTGCTGAACCTGTCGACGGTGGTAGTCCGACAC CTCCGACACCCGGGGGTGGTTCTGCAGACAAAACTCACA CATGCCCACCGTGCCCAGCACCTGAACTCCTGGGGGGAC CGTCAGTCTTCCTCTTCCCCCCAAAACCCAAGGACACCCT CATGATCTCCCGGACCCCTGAGGTCACATGCGTGGTGG GACGTGAGCCACGAAGACCCTGAGGTCAAGTTCAACTGG TACGTGGACGGCGTGGAGGTGCATAATGCCAAGACAAAG CCGCGGGAGGAGCAGTACAACAGCACGTACCGTGTGGTC AGCGTCCTCACCGTCCTGCACCAGGACTGGCTGAATGGCA AGGAGTACAAGTGCAAGGTCTCCAACAAAGCCCTCCCAG CCCCCATCGAGAAAACCATCTCCAAAGCCAAAGGGCAGC CCCGAGAACCACAGGTGTACACCCTGCCCCCATGCCGGG ATGAGCTGACCAAGAACCAGGTCAGCCTGTGGTGCCTGG TCAAAGGCTTCTATCCCAGCGACATCGCCGTGGAGTGGG AGAGCAATGGGCAGCCGGAGAACAACTACAAGACCACGC CTCCCGTGCTGGACTCCGACGGCTCCTTCTTCCTCTACAG CAAGCTCACCGTGGACAAGAGCAGGTGGCAGCAGGGGAA CGTCTTCTCATGCTCCGTGATGCATGAGGCTCTGCACAAC CACTACACGCAGAAGAGCCTCTCCCTGTCTCCGGGTAAAT CCGGAGGCCTGAACGACATCTTCGAGGCCCAGAAGATTG AATGGCACGAG |
| 362 | Nucleotide sequence murine GITR antigen Fc knob chain | AGCGTGGTGGAAGAACCCGGCTGCGGCCCTGGCAAGGTG CAGAATGGCAGCGGCAACAACACCCGGTGCTGCAGCCTG TACGCCCCTGGCAAAGAGGACTGCCCCAAAGAACGGTGC ATCTGCGTGACCCCCGAGTACCACTGCGGCGACCCCCAGT GCAAAATCTGCAAGCACTACCCCTGCCAGCCCGGCCAGC GGGTCGAAAGCCAGGGCGATATCGTGTTCGGCTTCAGAT GCGTGGCCTGCGCCATGGGCACCTTCAGCGCCGGCAGAG ATGGCCACTGCAGACTGTGGACCAACTGCAGCCAGTTCG GCTTCCTGACCATGTTCCCCGGCAACAAGACCCACAACGC CGTGTGCATCCCCGAGCCCCTGCCCACAGAGCAGTACGG CCATGTCGACGGTGGTAGTCCGACACCTCCGACACCCGG GGGTGGTTCTGCAGACAAAACTCACACATGCCCACCGTG CCCAGCACCTGAACTCCTGGGGGGACCGTCAGTCTTCCTC TTCCCCCCAAAACCCAAGGACACCCTCATGATCTCCCGGA CCCCTGAGGTCACATGCGTGGTGGTGGACGTGAGCCACG AAGACCCTGAGGTCAAGTTCAACTGGTACGTGGACGGCG TGGAGGTGCATAATGCCAAGACAAAGCCGCGGGAGGAGC AGTACAACAGCACGTACCGTGTGGTCAGCGTCCTCACCGT CCTGCACCAGGACTGGCTGAATGGCAAGGAGTACAAGTG CAAGGTCTCCAACAAAGCCCTCCCAGCCCCCATCGAGAA AACCATCTCCAAAGCCAAAGGGCAGCCCCGAGAACCACA GGTGTACACCCTGCCCCCATGCCGGGATGAGCTGACCAA |
| | | GAACCAGGTCAGCCTGTGGTGCCTGGTCAAAGGCTTCTAT CCCAGCGACATCGCCGTGGAGTGGGAGAGCAATGGGCAG CCGGAGAACAACTACAAGACCACGCCTCCCGTGCTGGAC TCCGACGGCTCCTTCTTCCTCTACAGCAAGCTCACCGTGG ACAAGAGCAGGTGGCAGCAGGGGAACGTCTTCTCATGCT CCGTGATGCATGAGGCTCTGCACAACCACTACACGCAGA AGAGCCTCTCCCTGTCTCCGGGTAAATCCGGAGGCCTGAA CGACATCTTCGAGGCCCAGAAGATTGAATGGCACGAG |
| 99 | Fc hole chain | DKTHTCPPCPAPELLGGPSVFLFPPKPKDTLMISRTPEVTCVV VDVSHEDPEVKFNWYVDGVEVHNAKTKPREEQYNSTYRV VSVLTVLHQDWLNGKEYKCKVSNKALPAPIEKTISKAKGQP REPQVCTLPPSRDELTKNQVSLSCAVKGFYPSDIAVEWESNG QPENNYKTTPPVLDSDGSFFLVSKLTVDKSRWQQGNVFSCS VMHEALHNHYTQKSLSLSPGK |
| 363 | human GITR antigen Fc knob chain | QRPTGGPGCGPGRLLLGTGTDARCCRVHTTRCCRDYPGEEC CSEWDCMCVQPEFHCGDPCCTTCRHHPCPPGQGVQSQGKF SFGFQCIDCASGTFSGGHEGHCKPWTDCTQFGFLTVFPGNKT HNAVCVPGSPPAEVDGGSPTPPTPGGGSADKTHTCPPCPAPE LLGGPSVFLFPPKPKDTLMISRTPEVTCVVVDVSHEDPEVKF NWYVDGVEVHNAKTKPREEQYNSTYRVVSVLTVLHQDWL NGKEYKCKVSNKALPAPIEKTISKAKGQPREPQVYTLPPCRD ELTKNQVSLWCLVKGFYPSDIAVEWESNGQPENNYKTTPPV LDSDGSFFLYSKLTVDKSRWQQGNVFSCSVMHEALHNHYT QKSLSLSPGKSGGLNDIFEAQKIEWHE |
| 364 | cynomolgus GITR antigen Fc knob chain | QRPTGGPGCGPGRLLLGTGKDARCCRVHPTRCCRDYQGEE CCSEWDCVCVQPEFHCGNPCCTTCQHHPCPSGQGVQPQGK FSFGFRCVDCALGTFSRGHDGHCKPWTDCTQFGFLTVFPGN KTHNAVCVPGSPPAEPVDGGSPTPPTPGGGSADKTHTCPPCP APELLGGPSVFLFPPKPKDTLMISRTPEVTCVVVDVSHEDPE VKFNWYVDGVEVHNAKTKPREEQYNSTYRVVSVLTVLHQ DWLNGKEYKCKVSNKALPAPIEKTISKAKGQPREPQVYTLP PCRDELTKNQVSLWCLVKGFYPSDIAVEWESNGQPENNYKT TPPVLDSDGSFFLYSKLTVDKSRWQQGNVFSCSVMHEALHN HYTQKSLSLSPGKSGGLNDIFEAQKIEWHE |
| 365 | murine GITR antigen Fc knob chain | SVVEEPGCGPGKVQNGSGNNTRCCSLYAPGKEDCPKERCIC VTPEYHCGDPQCKICKHYPCQPGQRVESQGDIVFGFRCVAC AMGTFSAGRDGHCRLWTNCSQFGFLTMFPGNKTHNAVCIP EPLPTEQYGHVDGGSPTPPTPGGGSADKTHTCPPCPAPELLG GPSVFLFPPKPKDTLMISRTPEVTCVVVDVSHEDPEVKFNWY VDGVEVHNAKTKPREEQYNSTYRVVSVLTVLHQDWLNGK EYKCKVSNKALPAPIEKTISKAKGQPREPQVYTLPPCRDELT KNQVSLWCLVKGFYPSDIAVEWESNGQPENNYKTTPPVLDS DGSFFLYSKLTVDKSRWQQGNVFSCSVMHEALHNHYTQKS LSLSPGKSGGLNDIFEAQKIEWHE |

All GITR-Fc-fusion molecule encoding sequences were cloned into a plasmid vector, which drives expression of the insert from a chimeric MPSV promoter and contains a synthetic polyA sequence located at the 3' end of the CDS. In addition, the vector contains an EBV oriP sequence for episomal maintenance of the plasmid.

For preparation of the biotinylated monomeric antigen/Fc fusion molecules, exponentially growing suspension HEK293 EBNA cells were co-transfected with three vectors encoding the two components of fusion protein (knob and hole chains) as well as BirA, an enzyme necessary for the biotinylation reaction. The corresponding vectors were used at a 2 : 1 : 0.05 ratio ("antigen ECD-IgAse- Fc knob" : "Fc hole" : "BirA").

Antigen production and purification was performed analogously to the procedure described in example 1 (1.1). Yield, concentration and quality of the three different GITR-Fc fusion proteins are summarized in **Table 74**.

**Table 74: Characterization of GITR antigens produced as monomeric Fc fusion molecules**

| Antigen | Yield [mg/L] | Concentration [mg/L] | Monomer content [%] | Biotinylation [%] |
|---|---|---|---|---|
| hu GITR-Fc | 9.07 | 1.94 | 98.3 | 57.6 |
| mu GITR-Fc | 5.72 | 3.09 | 99.6 | 94.5 |
| cy GITR-Fc | 1.13 | 1.78 | 100 | 96.9 |

### 11.2 Selection of anti-GITR antibodies from generic F(ab) libraries

The anti-GITR antibody 8A06 was selected in Fab-format from the generic phage display library λ -DP88.

### Library construction

The λ-DP88 library was constructed on the basis of human germline genes using the V-domain pairing Vl3_19 (lambda light chain) and VH1_69 (heavy chain) comprising randomized sequence space in CDR3 of the light chain (L3, 3 different lengths) and CDR3 of the heavy chain (H3, 3 different lengths). Library generation was performed by assembly of 3 PCR-amplified fragments applying splicing by overlapping extension (SOE) PCR. Fragment 1 comprises the 5' end of the antibody gene including randomized L3, fragment 2 is a central constant fragment spanning from L3 to H3 whereas fragment 3 comprises randomized H3 and the 3' end of the antibody gene. The following primer combinations were used to generate these library fragments for λ-DP88 library: fragment 1 (forward primer LMB3 combined with reverse primers Vl_3_19_L3r_V or Vl_3_19_L3r_HV or Vl_3_19_L3r_HLV), fragment 2 (forward primer RJH80 combined with reverse primer RJH32) and fragment 3 (forward primers DP88-v4-4 or DP88-v4-6 or DP88-v4-8 combined with reverse primer fdseqlong), respectively. PCR parameters for production of library fragments were 5 min initial denaturation at 94 °C, 25 cycles of 1 min 94 °C, 1 min 58 °C, 1 min 72 °C and terminal elongation for 10 min at 72 °C. For assembly PCR, using equimolar ratios of the gel-purified single fragments as template, parameters were 3 min initial denaturation at 94 °C and 5 cycles of 30 s 94 °C, 1 min 58 °C, 2 min 72 °C. At this stage, outer primers (LMB3 and fdseqlong) were added and additional 20 cycles were performed prior to a terminal elongation for 10 min at 72 °C. After assembly of sufficient amounts of full length randomized Fab constructs, they were digested NcoI / NheI and ligated into similarly treated acceptor phagemid vector. Purified ligations were used for ~60 transformations into electrocompetent E. coli TG1. Phagemid particles displaying the Fab library were rescued and purified by PEG/NaCl purification to be used for selections. A final library size of 9.0 × 109 was obtained. Percentages of functional clones, as determined by C-terminal tag detection in dot blot, were 73.7% for the light chain and 80.0% for the heavy chain, respectively.

### Phage display selections & ELISA screening

Human GITR as antigen for the phage display selections was transiently expressed as N-terminal monomeric Fc-fusion (knob-into-hole heterodimerization of Fc) in HEK EBNA cells and in vivo site-specifically biotinylated via co-expression of BirA biotin ligase at the avi-tag recognition sequence located at the C-terminus of the Fc portion carrying the receptor chain (Fc knob chain).

Selection rounds (biopanning) were performed in solution according to the following pattern: 1. pre-clearing of ~ 1012 phagemid particles with human IgG coated onto NUNC Maxisorp plates at 10µg/ml to deplete the library of antibodies recognizing the Fc-portion of the antigen, 2. incubation of the pre-cleared phagemid particles in the supernatant with 100nM biotinylated human GITR for 0.5 h in a total volume of 1ml, 3. capture of biotinylated human GITR and specifically binding phage by transfer to 4 wells of a neutravidin pre-coated microtiter plate for 10 min (in rounds 1 & 3), 4. washing of respective wells using 5x PBS/Tween20 and 5x PBS, 5. elution of phage particles by addition of 250ul 100mM TEA (triethylamine) per well for 10 min and neutralization by addition of 500ul 1M Tris/HCl pH 7.4 to the pooled eluates from 4 wells, 6. re-infection of log-phase E. coli TG1 cells with the supernatant of eluted phage particles, infection with helperphage VCSM13, incubation on a shaker at 30 °C over night and subsequent PEG/NaCl precipitation of phagemid particles to be used in the next selection round. Selections were carried out over 4 rounds using constant antigen concentrations of 100nM. In round 2 and 4, in order to avoid enrichment of binders to neutravidin, capture of antigen: phage complexes was performed by addition of 5.4 × 107 streptavidin-coated magnetic beads. Specific binders were identified by ELISA after round 4 as follows: 100ul of 100nM biotinylated human GITR were coated on neutravidin plates. Fab-containing bacterial supernatants were added and binding Fabs were detected via their Flag-tags using an anti-Flag/HRP secondary antibody. Clones exhibiting signals on human GITR and being negative on human Fc, were short-listed for further analyses. They were bacterially expressed in a 0.5 liter culture volume, affinity purified and characterized by SPR-analysis using BioRad's ProteOn XPR36 biosensor to determine binding kinetics and to test cross-reactivity to cynomolgus and murine GITR.

### SPR-analysis using BioRad's ProteOn XPR36 biosensor

The affinities (K_{D}) of clone 8A06 to human, cynomolgus, and murine GITR were measured by surface plasmon resonance (SPR) using a ProteOn XPR36 instrument (Biorad) at 25 °C with biotinylated human, cynomolgus and murine GITR antigens immobilized on NLC chips by neutravidin capture. Immobilization of antigens (ligand): Recombinant antigens were diluted with PBST (10 mM phosphate, 150 mM sodium chloride pH 7.4, 0.005% Tween 20) to 10 µg/ml, then injected at 30 µl/minute in vertical orientation. Injection of analytes: For 'one-shot kinetics' measurements, injection direction was changed to horizontal orientation, two-fold dilution series of purified Fab were injected simultaneously along separate channels 1-5, with association times of 200 s, and dissociation times of 240 s, respectively. Buffer (PBST) was injected along the sixth channel to provide an "in-line" blank for referencing. Association rate constants (kon) and dissociation rate constants (koff) were calculated using a simple one-to-one Langmuir binding model in ProteOn Manager software by simultaneously fitting the association and dissociation sensorgrams. The equilibrium dissociation constant (KD) was calculated as the ratio koff/kon. Clone 8A06 bound to the human and cynomolgus monomeric GITR-Fc fusions with affinities of 4.4 and 67 nM, respectively. No binding could be detected to the murine monomeric GITR-Fc fusion.

The pRJH52 library template λ-DP88 library is based on the complete Fab coding region comprising PelB leader sequence + Vl3_19 lambda V-domain + CL constant domain for light chain and pelB + VH1_69 V-domain + CH1 constant domain for heavy chain including tags. **Table 75** shows the sequences of generic phage-displayed λ-DP88 library (Vl3_19/VH1_69) template used for PCRs and **Table 76** shows the Primer sequences used for generation of the λ-DP88 library template.

**Table 75: Sequences of generic phage- displayed pRJH52 library template λ-DP88 library**

| Molecule | Sequence | Seq ID No |
|---|---|---|
| Amino acid sequence | | |
| Fab light chain Vl3_19 template | SSELTQDPAVSVALGQTVRITCQGDSLRSYYASWYQQKPG QAPVLVIYGKNNRPSGIPDRFSGSSSGNTASLTITGAQAEDE ADYYCNSRDSSGNHVVFGGGTKLTVLGQPKAAPSVTLFPP SSEELQANKATLVCLISDFYPGAVTVAWKADSSPVKAGVE TTTPSKQSNNKYAASSYLSLTPEQWKSHRSYSCQVTHEGS | 366 |
| | TVEKTVAPTECSGAAEQKLISEEDLNGAADYKDDDDKGA A | |
| Fab heavy chain VH1_69 template | QVQLVQSGAEVKKPGSSVKVSCKASGGTFSSYAISW VRQAPGQGLEWMGGIIPIFGTANYAQKFQGRVTITAD KSTSTAYMELSSLRSEDTAVYYCARLSPGGYYVMDA WGQGTTVTVSSASTKGPSVFPLAPSSKSTSGGTAALG CLVKDYFPEPVTVSWNSGALTSGVHTFPAVLQSSGLY SLSSVVTVPSSSLGTQTYICNVNHKPSNTKVDKKVEP KSCDAAASTSAHHHHHHAAA | 367 |
| Nucleotide sequence | | |
| Fab light chain Vl3_19 template | TCGTCTGAGCTGACTCAGGACCCTGCTGTGTCTGTGGCC TTGGGACAGACAGTCAGGATCACATGCCAAGGAGACAG CCTCAGAAGTTATTATGCAAGCTGGTACCAGCAGAAGCC AGGACAGGCCCCTGTACTTGTCATCTATGGTAAAAACAA CCGGCCCTCAGGGATCCCAGACCGATTCTCTGGCTCCAG CTCAGGAAACACAGCTTCCTTGACCATCACTGGGGCTCA GGCGGAAGATGAGGCTGACTATTACTGTAACTCCCGTGA TAGTAGCGGTAATCATGTGGTATTCGGCGGAGGGACCA AGCTGACCGTCCTAGGACAACCCAAGGCTGCCCCCAGC GTGACCCTGTTCCCCCCCAGCAGCGAGGAATTGCAGGCC AACAAGGCCACCCTGGTCTGCCTGATCAGCGACTTCTAC CCAGGCGCCGTGACCGTGGCCTGGAAGGCCGACAGCAG CCCCGTGAAGGCCGGCGTGGAGACCACCACCCCCAGCA AGCAGAGCAACAACAAGTACGCCGCCAGCAGCTACCTG AGCCTGACCCCCGAGCAGTGGAAGAGCCACAGGTCCTA CAGCTGCCAGGTGACCCACGAGGGCAGCACCGTGGAGA AAACCGTGGCCCCCACCGAGTGCAGCGGAGCCGCAGAA CAAAAACTCATCTCAGAAGAGGATCTGAATGGAGCCGC AGACTACAAGGACGACGACGACAAGGGTGCCGCA | 368 |
| Fab heavy chain VH1_69 template | CAGGTGCAATTGGTGCAGTCTGGGGCTGAGGTGAA GAAGCCTGGGTCCTCGGTGAAGGTCTCCTGCAAGG CCTCCGGAGGCACATTCAGCAGCTACGCTATAAGC TGGGTGCGACAGGCCCCTGGACAAGGGCTCGAGTG GATGGGAGGGATCATCCCTATCTTTGGTACAGCAA ACTACGCACAGAAGTTCCAGGGCAGGGTCACCATT ACTGCAGACAAATCCACGAGCACAGCCTACATGGA GCTGAGCAGCCTGAGATCTGAGGACACCGCCGTGT ATTACTGTGCGAGACTATCCCCAGGCGGTTACTATG TTATGGATGCCTGGGGCCAAGGGACCACCGTGACC GTCTCCTCAGCTAGCACCAAAGGCCCATCGGTCTTC CCCCTGGCACCCTCCTCCAAGAGCACCTCTGGGGG CACAGCGGCCCTGGGCTGCCTGGTCAAGGACTACT TCCCCGAACCGGTGACGGTGTCGTGGAACTCAGGC GCCCTGACCAGCGGCGTGCACACCTTCCCGGCTGTC CTACAGTCCTCAGGACTCTACTCCCTCAGCAGCGTG GTGACCGTGCCCTCCAGCAGCTTGGGCACCCAGAC CTACATCTGCAACGTGAATCACAAGCCCAGCAACA CCAAAGTGGACAAGAAAGTTGAGCCCAAATCTTGT GACGCGGCCGCAAGCACTAGTGCCCATCACCATCA CCATCACGCCGCGGCA | 369 |
| Complete | ATGAAATACCTATTGCCTACGGCAGCCGCTGGATT | 370 |
| pRJH52 Fab sequence | GTTATTACTCGCGGCCCAGCCGGCCATGGCCTCGTC TGAGCTGACTCAGGACCCTGCTGTGTCTGTGGCCTT GGGACAGACAGTCAGGATCACATGCCAAGGAGAC AGCCTCAGAAGTTATTATGCAAGCTGGTACCAGCA GAAGCCAGGACAGGCCCCTGTACTTGTCATCTATG GTAAAAACAACCGGCCCTCAGGGATCCCAGACCGA TTCTCTGGCTCCAGCTCAGGAAACACAGCTTCCTTG ACCATCACTGGGGCTCAGGCGGAAGATGAGGCTGA CTATTACTGTAACTCCCGTGATAGTAGCGGTAATCA TGTGGTATTCGGCGGAGGGACCAAGCTGACCGTCC TAGGACAACCCAAGGCTGCCCCCAGCGTGACCCTG TTCCCCCCCAGCAGCGAGGAATTGCAGGCCAACAA GGCCACCCTGGTCTGCCTGATCAGCGACTTCTACCC AGGCGCCGTGACCGTGGCCTGGAAGGCCGACAGCA GCCCCGTGAAGGCCGGCGTGGAGACCACCACCCCC AGCAAGCAGAGCAACAACAAGTACGCCGCCAGCA GCTACCTGAGCCTGACCCCCGAGCAGTGGAAGAGC CACAGGTCCTACAGCTGCCAGGTGACCCACGAGGG CAGCACCGTGGAGAAAACCGTGGCCCCCACCGAGT GCAGCGGAGCCGCAGAACAAAAACTCATCTCAGAA GAGGATCTGAATGGAGCCGCAGACTACAAGGACGA CGACGACAAGGGTGCCGCATAATAAGGCGCGCCAA TTCTATTTCAAGGAGACAGTCATATGAAATACCTGC TGCCGACCGCTGCTGCTGGTCTGCTGCTCCTCGCTG CCCAGCCGGCGATGGCCCAGGTGCAATTGGTGCAG TCTGGGGCTGAGGTGAAGAAGCCTGGGTCCTCGGT GAAGGTCTCCTGCAAGGCCTCCGGAGGCACATTCA GCAGCTACGCTATAAGCTGGGTGCGACAGGCCCCT GGACAAGGGCTCGAGTGGATGGGAGGGATCATCCC TATCTTTGGTACAGCAAACTACGCACAGAAGTTCC AGGGCAGGGTCACCATTACTGCAGACAAATCCACG AGCACAGCCTACATGGAGCTGAGCAGCCTGAGATC TGAGGACACCGCCGTGTATTACTGTGCGAGACTAT CCCCAGGCGGTTACTATGTTATGGATGCCTGGGGCC AAGGGACCACCGTGACCGTCTCCTCAGCTAGCACC AAAGGCCCATCGGTCTTCCCCCTGGCACCCTCCTCC AAGAGCACCTCTGGGGGCACAGCGGCCCTGGGCTG CCTGGTCAAGGACTACTTCCCCGAACCGGTGACGG TGTCGTGGAACTCAGGCGCCCTGACCAGCGGCGTG CACACCTTCCCGGCTGTCCTACAGTCCTCAGGACTC TACTCCCTCAGCAGCGTGGTGACCGTGCCCTCCAGC AGCTTGGGCACCCAGACCTACATCTGCAACGTGAA TCACAAGCCCAGCAACACCAAAGTGGACAAGAAA GTTGAGCCCAAATCTTGTGACGCGGCCGCAAGCAC TAGTGCCCATCACCATCACCATCACGCCGCGGCA | |

**Table 76: Primer sequences used for generation of lambda-DP47 library (Vl3_19/VH3_23)**

| **SEQ ID NO:** | **Primer name** | **Primer sequence 5' - 3'** |
|---|---|---|
| 132 | LMB3 | CAGGAAACAGCTATGACCATGATTAC |
| 133 | Vl_3_19_L3r_V | GGACGGTCAGCTTGGTCCCTCCGCCGAATAC VHV ATT ACC GCT ACT ATC ACG GGAGTTACAGTAATAGTCAGCCTCATCTTCCGC |
| | | underlined: 60% original base and 40% randomization as M bold and italic: 60% original base and 40% randomization as N |
| 134 | Vl_3_19_L3r_HV | GGACGGTCAGCTTGGTCCCTCCGCCGAATAC CMM ATG ATT ACC GCT ACT ATC ACG GGAGTTACAGTAATAGTCAGCCTCATCTTCCGC |
| | | underlined: 60% original base and 40% randomization as M bolded and italic: 60% original base and 40% randomization as N |
| 135 | Vl_3_19_L3r_HL V | GGACGGTCAGCTTGGTCCCTCCGCCGAATAC RHM VWG ATG ATT ACC GCT ACT ATC ACG GGAGTTACAGTAATAGTCAGCCTCATCTTC CGC |
| | | underlined: 60% original base and 40% randomization as M bolded and italic: 60% original base and 40% randomization as N |
| 136 | RJH80 | TTCGGCGGAGGGACCAAGCTGACCGTCC |
| 113 | RJH32 | TCTCGCACAGTAATACACGGCGGTGTCC |
| 114 | DP88-v4-4 | |
| 115 | DP88-v4-6 | GGACACCGCCGTGTATTACTGTGCGAGA-1-2-2-3-4-GAC- TAC-TGGGGCCAAGGGACCACCGTGACCGTCTCC GGACACCGCCGTGTATTACTGTGCGAGA-1-2-2-2-2-3-4-GAC- TAC-TGGGGCCAAGGGACCACCGTGACCGTCTCC GGACACCGCCGTGTATTACTGTGCGAGA-1-2-2-2-2-2-2-3-4- GAC-TAC-TGGGGCCAAGGGACCACCGTGACCGTCTCC |
| 116 | DP88-v4-8 | |
| 117 | fdseqlong | GACGTTAGTAAATGAATTTTCTGTATGAGG |

Clone 8A06 was identified as human GITR-specific binder through the procedure described above. The cDNA sequences of their variable regions are shown in **Table 77** below, the corresponding amino acid sequences can be found in Table C. In addition, the cDNA sequences of the variable regions of benchmark antibody 6C8 (prepared according to WO 2006/105021) are shown in **Table 77.**

**Table 77: Variable region base pair sequences for anti-GITR antibodies.**

| Clone | SEQ ID NO: | Sequence |
|---|---|---|
| 8A06 | 387 (VL) | TCGTCTGAGCTGACTCAGGACCCTGCTGTGTCTGTGGCCTTGGGACA GACAGTCAGGATCACATGCCAAGGAGACAGCCTCAGAAGTTATTAT GCAAGCTGGTACCAGCAGAAGCCAGGACAGGCCCCTGTACTTGTCA TCTATGGTAAAAACAACCGGCCCTCAGGGATCCCAGACCGATTCTC TGGCTCCAGCTCAGGAAACACAGCTTCCTTGACCATCACTGGGGCT CAGGCGGAAGATGAGGCTGACTATTACTGTAACTCCCCTCAGACTA |
| | | GCGGTAGTGCCGTATTCGGCGGAGGGACCAAGCTGACCGTCCTA |
| | 388 (VH) | CAGGTGCAATTGGTGCAGTCTGGGGCTGAGGTGAAGAAGCCTGGGT CCTCGGTGAAGGTCTCCTGCAAGGCCTCCGGAGGCACATTCAGCAG CTACGCTATAAGCTGGGTGCGACAGGCCCCTGGACAAGGGCTCGAG TGGATGGGAGGGATCATCCCTATCTTTGGTACAGCAAACTACGCAC AGAAGTTCCAGGGCAGGGTCACCATTACTGCAGACAAATCCACGAG CACAGCCTACATGGAGCTGAGCAGCCTGAGATCTGAGGACACCGCC GTGTATTACTGTGCGAGAGGTTACTACGCTATCGACTACTGGGGTC AAGGGACCACCGTGACCGTCTCCTCA |
| 6C8 | 389 (VL) | GAGATCGTGATGACCCAGTCCCCCGCCACCCTGTCCGTGTCTCCAG GCGAGAGAGCCACCCTGAGCTGCAAGGCCTCCCAGAACGTGGGCA CCAACGTGGCCTGGTATCAGCAGAAGCCCGGCCAGGCCCCTCGGCT GCTGATCTACTCCGCCTCCTACCGGTACTCCGGCATCCCTGCCCGGT TCTCCGGCTCTGGCTCTGGCACCGAGTTTACCCTGACCATCTCCAGC CTGCAGTCCGAGGACTTCGCCGTGTACTACTGCCAGCAGTACAACA CCGACCCCCTGACCTTCGGCGGAGGCACCAAGGTGGAAATCAAA |
| | 390 (VH) | CAGGTCACACTGAGAGAGTCCGGCCCTGCCCTGGTCAAGCCCACCC AGACCCTGACCCTGACATGCACCTTCTCCGGCTTCTCCCTGTCCACC TCCGGCATGGGCGTGGGCTGGATCAGACAGCCTCCTGGCAAGGCCC TGGAATGGCTGGCCCACATTTGGTGGGACGACGACAAGTACTACCA GCCCTCCCTGAAGTCCCGGCTGACCATCTCCAAGGACACCTCCAAG AACCAGGTGGTGCTGACCATGACCAACATGGACCCCGTGGACACCG CCACCTACTACTGCGCCCGGACCCGGCGGTACTTCCCCTTTGCTTAT TGGGGCCAGGGCACCCTGGTCACCGTCTCGAGT |

### 11.3 Preparation, purification and characterization of anti-GITR IgG1 P329G LALA antibodies

The variable regions of heavy and light chain DNA sequences of selected anti-GITR binders were subcloned in frame with either the constant heavy chain or the constant light chain of human IgG1. The Pro329Gly, Leu234Ala and Leu235Ala mutations have been introduced in the constant region of the knob and hole heavy chains to abrogate binding to Fc gamma receptors according to the method described in International Patent Appl. Publ. No. WO 2012/130831 A1.

The nucleotide and amino acid sequences of the anti-GITR clones are shown in **Table 78**. The anti-GITR antibodies were produced by co-transfecting HEK293-EBNA cells with the mammalian expression vectors using polyethylenimine. The cells were transfected with the corresponding expression vectors in a 1:1 ratio ("vector heavy chain": "vector light chain"). Production and purification of GITR specific antibody 8A06 was performed analogously as described in example 1.3. **Table 79** summarizes the results of production and purification of clone 8A06 selected by phage display.

**Table 78: Sequences of anti-GITR clones in P329GLALA human IgG1 format**

| **Clone** | **SEQ ID No.** | **Sequence** |
|---|---|---|
| | 391 (nucleotide sequence light chain) | TCGTCTGAGCTGACTCAGGACCCTGCTGTGTCTGTGGCCTTGGG ACAGACAGTCAGGATCACATGCCAAGGAGACAGCCTCAGAAG TTATTATGCAAGCTGGTACCAGCAGAAGCCAGGACAGGCCCCT GTACTTGTCATCTATGGTAAAAACAACCGGCCCTCAGGGATCC CAGACCGATTCTCTGGCTCCAGCTCAGGAAACACAGCTTCCTT GACCATCACTGGGGCTCAGGCGGAAGATGAGGCTGACTATTAC TGTAACTCCCCTCAGACTAGCGGTAGTGCCGTATTCGGCGGAG GGACCAAGCTGACCGTCCTAGGTCAACCCAAGGCTGCCCCCAG CGTGACCCTGTTCCCCCCCAGCAGCGAGGAACTGCAGGCCAAC AAGGCCACCCTGGTCTGCCTGATCAGCGACTTCTACCCAGGCG CCGTGACCGTGGCCTGGAAGGCCGACAGCAGCCCCGTGAAGG CCGGCGTGGAGACCACCACCCCCAGCAAGCAGAGCAACAACA AGTACGCCGCCAGCAGCTACCTGAGCCTGACCCCCGAGCAGTG GAAGAGCCACAGGTCCTACAGCTGCCAGGTGACCCACGAGGG CAGCACCGTGGAGAAAACCGTGGCCCCCACCGAGTGCAGC |
| **8A06** | 392 (nucleotide sequence heavy chain) | CAGGTGCAATTGGTGCAGTCTGGGGCTGAGGTGAAGAAGCCTG GGTCCTCGGTGAAGGTCTCCTGCAAGGCCTCCGGAGGCACATT CAGCAGCTACGCTATAAGCTGGGTGCGACAGGCCCCTGGACAA GGGCTCGAGTGGATGGGAGGGATCATCCCTATCTTTGGTACAG CAAACTACGCACAGAAGTTCCAGGGCAGGGTCACCATTACTGC AGACAAATCCACGAGCACAGCCTACATGGAGCTGAGCAGCCT GAGATCTGAGGACACCGCCGTGTATTACTGTGCGAGAGGTTAC TACGCTATCGACTACTGGGGTCAAGGGACCACCGTGACCGTCT CCTCAGCTAGCACCAAGGGCCCATCGGTCTTCCCCCTGGCACC CTCCTCCAAGAGCACCTCTGGGGGCACAGCGGCCCTGGGCTGC CTGGTCAAGGACTACTTCCCCGAACCGGTGACGGTGTCGTGGA ACTCAGGCGCCCTGACCAGCGGCGTGCACACCTTCCCGGCTGT CCTACAGTCCTCAGGACTCTACTCCCTCAGCAGCGTGGTGACC GTGCCCTCCAGCAGCTTGGGCACCCAGACCTACATCTGCAACG TGAATCACAAGCCCAGCAACACCAAGGTGGACAAGAAAGTTG AGCCCAAATCTTGTGACAAAACTCACACATGCCCACCGTGCCC AGCACCTGAAGCTGCAGGGGGACCGTCAGTCTTCCTCTTCCCC CCAAAACCCAAGGACACCCTCATGATCTCCCGGACCCCTGAGG TCACATGCGTGGTGGTGGACGTGAGCCACGAAGACCCTGAGGT CAAGTTCAACTGGTACGTGGACGGCGTGGAGGTGCATAATGCC AAGACAAAGCCGCGGGAGGAGCAGTACAACAGCACGTACCGT GTGGTCAGCGTCCTCACCGTCCTGCACCAGGACTGGCTGAATG GCAAGGAGTACAAGTGCAAGGTCTCCAACAAAGCCCTCGGCG CCCCCATCGAGAAAACCATCTCCAAAGCCAAAGGGCAGCCCC GAGAACCACAGGTGTACACCCTGCCCCCATCCCGGGATGAGCT GACCAAGAACCAGGTCAGCCTGACCTGCCTGGTCAAAGGCTTC TATCCCAGCGACATCGCCGTGGAGTGGGAGAGCAATGGGCAG CCGGAGAACAACTACAAGACCACGCCTCCCGTGCTGGACTCCG ACGGCTCCTTCTTCCTCTACAGCAAGCTCACCGTGGACAAGAG CAGGTGGCAGCAGGGGAACGTCTTCTCATGCTCCGTGATGCAT GAGGCTCTGCACAACCACTACACGCAGAAGAGCCTCTCCCTGT CTCCGGGTAAA |
| | 393 (Light chain) | SSELTQDPAVSVALGQTVRITCQGDSLRSYYASWYQQKPGQAPV LVIYGKNNRPSGIPDRFSGSSSGNTASLTITGAQAEDEADYYCNSP QTSGSAVFGGGTKLTVLGQPKAAPSVTLFPPSSEELQANKATLVC LISDFYPGAVTVAWKADSSPVKAGVETTTPSKQSNNKYAASSYLS |
| | | LTPEQWKSHRSYSCQVTHEGSTVEKTVAPTECS |
| | 394 (Heavy chain) | QVQLVQSGAEVKKPGSSVKVSCKASGGTFSSYAISWVRQAPGQG LEWMGGIIPIFGTANYAQKFQGRVTITADKSTSTAYMELSSLRSED TAVYYCARGYYAIDYWGQGTTVTVSSASTKGPSVFPLAPSSKSTS GGTAALGCLVKDYFPEPVTVSWNSGALTSGVHTFPAVLQSSGLY SLSSVVTVPSSSLGTQTYICNVNHKPSNTKVDKKVEPKSCDKTHT CPPCPAPEAAGGPSVFLFPPKPKDTLMISRTPEVTCVVVDVSHEDP EVKFNWYVDGVEVHNAKTKPREEQYNSTYRVVSVLTVLHQDWL NGKEYKCKVSNKALGAPIEKTISKAKGQPREPQVYTLPPSRDELT KNQVSLTCLVKGFYPSDIAVEWESNGQPENNYKTTPPVLDSDGSF FLYSKLTVDKSRWQQGNVFSCSVMHEALHNHYTQKSLSLSPGK |
| **6C8** | 395 (nucleotide sequence light chain) | GAGATCGTGATGACCCAGTCCCCCGCCACCCTGTCCGTGTCTC CAGGCGAGAGAGCCACCCTGAGCTGCAAGGCCTCCCAGAACG TGGGCACCAACGTGGCCTGGTATCAGCAGAAGCCCGGCCAGG CCCCTCGGCTGCTGATCTACTCCGCCTCCTACCGGTACTCCGGC ATCCCTGCCCGGTTCTCCGGCTCTGGCTCTGGCACCGAGTTTAC CCTGACCATCTCCAGCCTGCAGTCCGAGGACTTCGCCGTGTAC TACTGCCAGCAGTACAACACCGACCCCCTGACCTTCGGCGGAG GCACCAAGGTGGAAATCAAACGTACGGTGGCTGCACCATCTGT CTTCATCTTCCCGCCATCTGATGAGCAGTTGAAATCTGGAACTG CCTCTGTTGTGTGCCTGCTGAATAACTTCTATCCCAGAGAGGCC AAAGTACAGTGGAAGGTGGATAACGCCCTCCAATCGGGTAACT CCCAGGAGAGTGTCACAGAGCAGGACAGCAAGGACAGCACCT ACAGCCTCAGCAGCACCCTGACGCTGAGCAAAGCAGACTACG AGAAACACAAAGTCTACGCCTGCGAAGTCACCCATCAGGGCCT GAGCTCGCCCGTCACAAAGAGCTTCAACAGGGGAGAGTGT |
| | 396 (nucleotide sequence heavy chain) | CAGGTCACACTGAGAGAGTCCGGCCCTGCCCTGGTCAAGCCCA CCCAGACCCTGACCCTGACATGCACCTTCTCCGGCTTCTCCCTG TCCACCTCCGGCATGGGCGTGGGCTGGATCAGACAGCCTCCTG GCAAGGCCCTGGAATGGCTGGCCCACATTTGGTGGGACGACGA CAAGTACTACCAGCCCTCCCTGAAGTCCCGGCTGACCATCTCC AAGGACACCTCCAAGAACCAGGTGGTGCTGACCATGACCAAC ATGGACCCCGTGGACACCGCCACCTACTACTGCGCCCGGACCC GGCGGTACTTCCCCTTTGCTTATTGGGGCCAGGGCACCCTGGTC ACCGTCTCGAGTGCTAGCACCAAGGGCCCATCGGTCTTCCCCC TGGCACCCTCCTCCAAGAGCACCTCTGGGGGCACAGCGGCCCT GGGCTGCCTGGTCAAGGACTACTTCCCCGAACCGGTGACGGTG TCGTGGAACTCAGGCGCCCTGACCAGCGGCGTGCACACCTTCC CGGCTGTCCTACAGTCCTCAGGACTCTACTCCCTCAGCAGCGT GGTGACCGTGCCCTCCAGCAGCTTGGGCACCCAGACCTACATC TGCAACGTGAATCACAAGCCCAGCAACACCAAGGTGGACAAG AAAGTTGAGCCCAAATCTTGTGACAAAACTCACACATGCCCAC CGTGCCCAGCACCTGAAGCTGCAGGGGGACCGTCAGTCTTCCT CTTCCCCCCAAAACCCAAGGACACCCTCATGATCTCCCGGACC CCTGAGGTCACATGCGTGGTGGTGGACGTGAGCCACGAAGACC CTGAGGTCAAGTTCAACTGGTACGTGGACGGCGTGGAGGTGCA TAATGCCAAGACAAAGCCGCGGGAGGAGCAGTACAACAGCAC GTACCGTGTGGTCAGCGTCCTCACCGTCCTGCACCAGGACTGG CTGAATGGCAAGGAGTACAAGTGCAAGGTCTCCAACAAAGCC CTCGGCGCCCCCATCGAGAAAACCATCTCCAAAGCCAAAGGGC AGCCCCGAGAACCACAGGTGTACACCCTGCCCCCATCCCGGGA TGAGCTGACCAAGAACCAGGTCAGCCTGACCTGCCTGGTCAAA GGCTTCTATCCCAGCGACATCGCCGTGGAGTGGGAGAGCAATG |
| | | GGCAGCCGGAGAACAACTACAAGACCACGCCTCCCGTGCTGG ACTCCGACGGCTCCTTCTTCCTCTACAGCAAGCTCACCGTGGAC AAGAGCAGGTGGCAGCAGGGGAACGTCTTCTCATGCTCCGTGA TGCATGAGGCTCTGCACAACCACTACACGCAGAAGAGCCTCTC CCTGTCTCCGGGTAAA |
| | 397 (Light chain) | EIVMTQSPATLSVSPGERATLSCKASQNVGTNVAWYQQKPGQAP RLLIYSASYRYSGIPARFSGSGSGTEFTLTISSLQSEDFAVYYCQQY NTDPLTFGGGTKVEIKRTVAAPSVFIFPPSDEQLKSGTASVVCLLN NFYPREAKVQWKVDNALQSGNSQESVTEQDSKDSTYSLSSTLTLS KADYEKHKVYACEVTHQGLSSPVTKSFNRGEC |
| | 398 (Heavy chain) | QVTLRESGPALVKPTQTLTLTCTFSGFSLSTSGMGVGWIRQPPGK ALEWLAHIWWDDDKYYQPSLKSRLTISKDTSKNQVVLTMTNMD PVDTATYYCARTRRYFPFAYWGQGTLVTVSSASTKGPSVFPLAPS SKSTSGGTAALGCLVKDYFPEPVTVSWNSGALTSGVHTFPAVLQS SGLYSLSSVVTVPSSSLGTQTYICNVNHKPSNTKVDKKVEPKSCD KTHTCPPCPAPEAAGGPSVFLFPPKPKDTLMISRTPEVTCVVVDVS HEDPEVKFNWYVDGVEVHNAKTKPREEQYNSTYRVVSVLTVLH QDWLNGKEYKCKVSNKALGAPIEKTISKAKGQPREPQVYTLPPSR DELTKNQVSLTCLVKGFYPSDIAVEWESNGQPENNYKTTPPVLDS DGSFFLYSKLTVDKSRWQQGNVFSCSVMHEALHNHYTQKSLSLS PGK |

**Table 79: Biochemical analysis of anti-GITR P329G LALA IgG1 antibodies**

| Clone | Yield [mg/L] | Monomer[%] | Purity (non-red) [%] | CE-SDS (red) |
|---|---|---|---|---|
| 8A06 P329GLALA IgG | 6.74 | 100 | 98.13 | 98.66 |
| 6C8 P329GLALA IgG | 14.71 | 100 | 97.62 | 98.95 |

### 11.4 Characterization of GITR clone 8A06

### 11.4.1 Surface plasmon resonance (avidity + affinity)

### 11.4.1.1 Species cross-reactivity and affinity of clones 8A06 and 6C8

Binding of GITR-specific antibodies (clone 8A06 and 6C8) to the recombinant GITR Fc (kih) was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany). In the same experiment, the species selectivity and the avidity of the interaction between the GITR binder (human IgG1 P329GLALA), and recombinant GITR (human, cyno and murine) was determined. Biotinylated human, cynomolgus and murine GITR Fc (kih) were directly coupled to different flow cells of a streptavidin (SA) sensor chip. Immobilization levels up to 100 resonance units (RU) were used.

Anti-GITR human IgG1 P329GLALA antibody was passed at a concentration range from 0.2 to 200 nM (4-fold dilution) with a flow of 30 µL/minute through the flow cells over 120 seconds. Complex dissociation was monitored for 180 seconds. Bulk refractive index differences were corrected for by subtracting the response obtained in a reference flow cell, where no protein was immobilized. Kinetic constants were derived using the Biacore T200 Evaluation Software (vAA, Biacore AB, Uppsala/Sweden), to fit rate equations for 1:1 Langmuir binding by numerical integration and used to estimate qualitatively the avidity **(****Figures 49A-49D****).**

In the same experiment, the affinities of the interaction between the GITR antibody (human IgG1 P329GLALA) to recombinant GITR (human and cyno) were determined. Anti-human Fab antibody (Biacore, Freiburg/Germany) was directly coupled on a CM5 chip at pH 5.0 using the standard amine coupling kit (Biacore, Freiburg/Germany). The immobilization level was approximately 7000 RU. The GITR antibody was captured for 90 seconds at 5 nM. Recombinant human and cynomolgus GITR Fc(kih) was passed at a concentration range from 2.7 to 2000 nM with a flow of 30 µL/minutes through the flow cells over 180 seconds. The dissociation was monitored for 180 seconds. Bulk refractive index differences were corrected for by subtracting the response obtained on reference flow cell. Here, the antigens were flown over a surface with immobilized anti-human Fab antibody but on which HBS-EP has been injected rather than the antibodies **(****Figure 50A****).** Kinetic constants were derived using the Biacore T200 Evaluation Software (vAA, Biacore AB, Uppsala/Sweden), to fit rate equations for 1:1 Langmuir binding by numerical integration. Affinity constants for the interaction between anti-GITR P329GLALA IgG1 (clone 8A06) and GITR Fc(kih) were determined by fitting to a 1:1 Langmuir binding (Table 80).

Clones 8A06 and 6C8 bind to human and cynomolgus GITR Fc (kih), but are not cross reactive for murine GITR.

**Table 80: Binding of anti-GITR antibodies to recombinant GITR**

| | Clone 8A06 | **Recombinant human GITR (affinity format)** | | |
|---|---|---|---|---|
| | | ka (1/Ms) | kd (1/s) | KD (M) |
| **Human GITR** | +++ | 1.2E+04 | 3.6E-04 | 2.9E-08 |
| **Cyno GITR** | ++ | 2.7E+04 | 3.3E-02 | 1.2E-06 |

| **Murine GITR** | Not binding | Not binding | | |
|---|---|---|---|---|
| | Clone 6C8 | **Recombinant human GITR (affinity format)** | | |
| | | ka (1/Ms) | kd (1/s) | KD (M) |
| **Human GITR** | +++ | 3.8E+04 | 1.1E-03 | 3.0E-08 |
| **Cyno GITR** | ++ | 7.3E+04 | 1.4E-02 | 1.9E-07 |
| **Murine GITR** | Not binding | Not binding | | |

### 11.4.1.2 Ligand blocking property

To determine the capacity of the GITR-specific human IgG1 P329GLALA antibody 8A06 to interfere with GITR/GITR-ligand interaction we used human GITR ligand (R&D systems).

Human GITR ligand was directly coupled to two flow cells of a CM5 chip at approximately 2000 RU by pH 5.0 using the standard amine coupling kit (Biacore, Freiburg/Germany). Recombinant human GITR Fc (kih) was passed on the second flow cell at a concentration of 100 nM with a flow of 30 µL/minute over 90 seconds. The dissociation was omitted and anti-GITR human IgG1P329LALA was passed on both flow cells at a concentration of 500 nM with a flow of 30 uL/ minute over 90 seconds. The dissociation was monitored for 60 seconds. Bulk refractive index differences were corrected for by subtracting the response obtained on reference flow cell. Here, the antibodies were flown over a surface with immobilized human GITR ligand but on which HBS-EP has been injected instead of recombinant human GITR Fc (kih).

The GITR clone 8A06 bound to the complex of human GITR with its GITR ligand (Figure **51****).** Thus, this antibody does not compete with the ligand for binding to human GITR and is therefore termed "non-ligand blocking".

**Table 81: Ligand binding property of the anti-GITR clones 8A06 and 6C8 determined by surface plasmon resonance**

| **Clone** | **Ligand** | **First injection** | **Second injection** | **Ligand blocking** |
|---|---|---|---|---|
| **8A06** | **Hu GITR Ligand** | human GITR Fc(kih) (binding observed) | anti-GITR 8A06 IgG (binding observed) | NO |
| **6C8** | **Hu GITR Ligand** | human GITR Fc(kih) (binding observed) | anti-GITR 6C8 IgG (binding observed) | yes |

### 11.4.1.3 Epitope Binning

The epitope recognized by the anti-GITR antibodies was characterized by surface plasmon resonance. First, the ability of the antibodies to compete for binding to human GITR was assessed by surface plasmon resonance. The aim of these experiments was to define an "epitope bin". Antibodies that compete for a similar or an overlapping epitope are not able to bind simultaneously to GITR and belong to an "epitope bin". Anti-GITR antibodies that can bind simultaneously to GITR do not share an epitope, or part of it, and are therefore grouped into a different epitope bin.

To analyze competitive binding for human or murine receptor of the anti-GITR human IgG1 P329GLALA, the phage derived anti-GITR clone 8A06 and benchmark antibody 6C8 were directly coupled to CM5 chip at approx. 1000 RU by pH 5.5 using the standard amine coupling kit (Biacore, Freiburg/Germany). Recombinant human GITR Fc (kih) was injected at a concentration of 200 nM with a flow of 30 µL/min over 180 seconds. The dissociation was omitted and a second anti-GITR human IgG1 P329GLALA antibody was passed at a concentration of 200 nM with a flow of 30 µL/min over 90 seconds. The dissociation was monitored for 90 sec. Bulk refractive index differences were corrected for by subtracting the response obtained on reference flow cell.

The SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany).

The competition binding experiment shows that the anti-GITR clone 8A06 and 6C8 bind to a different spatial epitope, since the two antibodies can bind simultaneously to human GITR Fc(kih) **(Table 82,** **Figures 52B and 52C****).**

**Table 82: Summary of competition binding experiments**

| Immobilized on chip | First injection | Second injection | |
|---|---|---|---|
| | | 8A06 | 6C8 |
| 8A06 | Human GITR Fc(kih) | 0 | 1 |
| 6C8 | Human GITR Fc(kih) | 1 | 0 |

| | | | |
|---|---|---|---|
| *O* = *no binding; 1, binding* | | | |

### Example 12 Preparation, purification and characterization of bispecific bivalent antibodies targeting GITR and a tumor associated antigen (TAA)

### 12.1 Generation of bispecific tetravalent constructs targeting GITR and fibroblast activation protein (FAP) (4+1 and 4+2 formats)

Bispecific agonistic GITR antibodies with tetravalent binding for GITR and monovalent binding for FAP, also termed 4+1 bispecific antigen binding molecules, were prepared as depicted in **Figure 53A****.**

One heavy chain HC1 of the construct was comprised of the following components: VHCH1_VHCH1 of an anti-GITR followed by Fc hole, at which C-terminus a VL or VH, respectively, of an anti-FAP binder (clone 28H1) was fused. Heavy chain HC2 was comprised of VHCH1VHCH1 of anti-GITR followed by Fc knob, at which C-terminus a VH or VL, of the anti-FAP binder was fused.

Binders against GITR were generated as described in Example 11. The generation and preparation of the FAP binders is described in WO 2012/020006 A2.

Combination of both heavy chains allows generation of a heterodimer, which includes a FAP binding moiety and four GITR binding Fabs. The Pro329Gly, Leu234Ala and Leu235Ala mutations were introduced in the constant region of the heavy chains to abrogate binding to Fc gamma receptors according to the method described in International Patent Appl. Publ. No. WO 2012/130831 A1. The heavy chain fusion proteins were co-expressed with the light chain of the anti-GITR binder (CLVL). The nucleotide and amino acid sequences of the resulting bispecific, tetravalent construct can be found in **Table 83.**

**Table 83: cDNA and amino acid sequences of mature bispecific 4+1 anti-GITR, anti-FAP human IgG1 P329GLALA kih antibodies (constructs with a-FAP VH fused to knob and VL fused to hole chain)**

| SEQ ID NO: | Description | Sequence |
|---|---|---|
| 391 | (8A06) VLCL-light chain (nucleotide sequence) | TCGTCTGAGCTGACTCAGGACCCTGCTGTGTCTGTGGC CTTGGGACAGACAGTCAGGATCACATGCCAAGGAGAC AGCCTCAGAAGTTATTATGCAAGCTGGTACCAGCAGA AGCCAGGACAGGCCCCTGTACTTGTCATCTATGGTAAA AACAACCGGCCCTCAGGGATCCCAGACCGATTCTCTGG CTCCAGCTCAGGAAACACAGCTTCCTTGACCATCACTG GGGCTCAGGCGGAAGATGAGGCTGACTATTACTGTAA CTCCCCTCAGACTAGCGGTAGTGCCGTATTCGGCGGAG GGACCAAGCTGACCGTCCTAGGTCAACCCAAGGCTGC CCCCAGCGTGACCCTGTTCCCCCCCAGCAGCGAGGAAC TGCAGGCCAACAAGGCCACCCTGGTCTGCCTGATCAGC GACTTCTACCCAGGCGCCGTGACCGTGGCCTGGAAGGC CGACAGCAGCCCCGTGAAGGCCGGCGTGGAGACCACC ACCCCCAGCAAGCAGAGCAACAACAAGTACGCCGCCA GCAGCTACCTGAGCCTGACCCCCGAGCAGTGGAAGAG CCACAGGTCCTACAGCTGCCAGGTGACCCACGAGGGC AGCACCGTGGAGAAAACCGTGGCCCCCACCGAGTGCA GC |
| 399 | HC 1 (8A06) VHCH1-_VHCH1-_Fc knob VH (28H1) (nucleotide sequence) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTATTGCGCCAGAGGCT ACTACGCCATCGACTACTGGGGCCAGGGCACCACCGT GACCGTGTCTAGCGCTTCTACCAAGGGCCCCAGCGTGT TCCCTCTGGCCCCTAGCAGCAAGAGCACATCTGGCGGA ACAGCCGCCCTGGGCTGCCTCGTGAAGGACTACTTTCC CGAGCCCGTGACAGTGTCCTGGAACTCTGGCGCCCTGA CAAGCGGCGTGCACACCTTTCCAGCCGTGCTCCAGAGC AGCGGCCTGTACTCTCTGAGCAGCGTCGTGACTGTGCC CAGCAGCAGCCTGGGAACCCAGACCTACATCTGCAAC GTGAACCACAAGCCCAGCAACACCAAGGTGGACAAGA AGGTGGAACCCAAGAGCTGCGACGGCGGAGGCGGATC TGGCGGCGGAGGATCCCAGGTGCAGCTGGTGCAGAGC GGAGCTGAAGTGAAAAAGCCTGGCTCCTCCGTGAAAG TGTCTTGTAAAGCCAGCGGCGGCACATTCTCTAGCTAT GCCATCAGCTGGGTGCGGCAGGCTCCAGGCCAGGGAC TGGAATGGATGGGAGGAATTATCCCTATTTTTGGGACA GCCAATTATGCTCAGAAATTTCAGGGGCGCGTGACAAT TACAGCCGACAAGTCCACCTCTACAGCTTATATGGAAC TGTCCTCCCTGCGCTCCGAGGATACAGCTGTGTATTAC TGCGCTCGGGGCTATTATGCCATTGATTATTGGGGACA GGGAACAACAGTGACTGTGTCCTCCGCTAGCACCAAG GGCCCATCGGTCTTCCCCCTGGCACCCTCCTCCAAGAG |
| | | CACCTCTGGGGGCACAGCGGCCCTGGGCTGCCTGGTCA AGGACTACTTCCCCGAACCGGTGACGGTGTCGTGGAAC TCAGGCGCCCTGACCAGCGGCGTGCACACCTTCCCGGC TGTCCTACAGTCCTCAGGACTCTACTCCCTCAGCAGCG TGGTGACCGTGCCCTCCAGCAGCTTGGGCACCCAGACC TACATCTGCAACGTGAATCACAAGCCCAGCAACACCA AGGTGGACAAGAAAGTTGAGCCCAAATCTTGTGACAA AACTCACACATGCCCACCGTGCCCAGCACCTGAAGCTG CAGGGGGACCGTCAGTCTTCCTCTTCCCCCCAAAACCC AAGGACACCCTCATGATCTCCCGGACCCCTGAGGTCAC ATGCGTGGTGGTGGACGTGAGCCACGAAGACCCTGAG GTCAAGTTCAACTGGTACGTGGACGGCGTGGAGGTGC ATAATGCCAAGACAAAGCCGCGGGAGGAGCAGTACAA CAGCACGTACCGTGTGGTCAGCGTCCTCACCGTCCTGC ACCAGGACTGGCTGAATGGCAAGGAGTACAAGTGCAA GGTCTCCAACAAAGCCCTCGGCGCCCCCATCGAGAAA ACCATCTCCAAAGCCAAAGGGCAGCCCCGAGAACCAC AGGTGTACACCCTGCCCCCCTGCAGAGATGAGCTGACC AAGAACCAGGTGTCCCTGTGGTGTCTGGTCAAGGGCTT CTACCCCAGCGATATCGCCGTGGAGTGGGAGAGCAAC GGCCAGCCTGAGAACAACTACAAGACCACCCCCCCTG TGCTGGACAGCGACGGCAGCTTCTTCCTGTACTCCAAA CTGACCGTGGACAAGAGCCGGTGGCAGCAGGGCAACG TGTTCAGCTGCAGCGTGATGCACGAGGCCCTGCACAAC CACTACACCCAGAAGTCCCTGAGCCTGAGCCCCGGCG GAGGCGGCGGAAGCGGAGGAGGAGGATCCGGAGGAG GGGGAAGTGGCGGCGGAGGATCTGAGGTGCAGCTGCT GGAATCCGGCGGAGGCCTGGTGCAGCCTGGCGGATCT CTGAGACTGTCCTGCGCCGCCTCCGGCTTCACCTTCTCC TCCCACGCCATGTCCTGGGTCCGACAGGCTCCTGGCAA AGGCCTGGAATGGGTGTCCGCCATCTGGGCCTCCGGCG AGCAGTACTACGCCGACTCTGTGAAGGGCCGGTTCACC ATCTCCCGGGACAACTCCAAGAACACCCTGTACCTGCA GATGAACTCCCTGCGGGCCGAGGACACCGCCGTGTACT ACTGTGCCAAGGGCTGGCTGGGCAACTTCGACTACTGG GGCCAGGGCACCCTGGTCACCGTGTCCAGC |
| 400 | HC 2 (8A06) VHCH1-_VHCH1-_Fc hole VL (28H1) (nucleotide sequence) | CAGGTGCAGCTGGTGCAGTCTGGCGCCGAAGTGAAGA AACCCGGCAGCAGCGTGAAGGTGTCCTGCAAGGCTTC CGGCGGCACCTTCAGCAGCTACGCCATTTCTTGGGTGC GCCAGGCCCCTGGACAGGGCCTGGAATGGATGGGCGG CATCATCCCCATCTTCGGCACCGCCAACTACGCCCAGA AATTCCAGGGCAGAGTGACCATCACCGCCGACAAGAG CACCAGCACCGCCTACATGGAACTGAGCAGCCTGCGG AGCGAGGACACCGCCGTGTACTATTGCGCCAGAGGCT ACTACGCCATCGACTACTGGGGCCAGGGCACCACCGT GACCGTGTCTAGCGCTTCTACCAAGGGCCCCAGCGTGT TCCCTCTGGCCCCTAGCAGCAAGAGCACATCTGGCGGA ACAGCCGCCCTGGGCTGCCTCGTGAAGGACTACTTTCC CGAGCCCGTGACAGTGTCCTGGAACTCTGGCGCCCTGA CAAGCGGCGTGCACACCTTTCCAGCCGTGCTCCAGAGC AGCGGCCTGTACTCTCTGAGCAGCGTCGTGACTGTGCC CAGCAGCAGCCTGGGAACCCAGACCTACATCTGCAAC GTGAACCACAAGCCCAGCAACACCAAGGTGGACAAGA AGGTGGAACCCAAGAGCTGCGACGGCGGAGGCGGATC |
| | | TGGCGGCGGAGGATCCCAGGTGCAGCTGGTGCAGAGC GGAGCTGAAGTGAAAAAGCCTGGCTCCTCCGTGAAAG TGTCTTGTAAAGCCAGCGGCGGCACATTCTCTAGCTAT GCCATCAGCTGGGTGCGGCAGGCTCCAGGCCAGGGAC TGGAATGGATGGGAGGAATTATCCCTATTTTGGGACA GCCAATTATGCTCAGAAATTTCAGGGGCGCGTGACAAT TACAGCCGACAAGTCCACCTCTACAGCTTATATGGAAC TGTCCTCCCTGCGCTCCGAGGATACAGCTGTGTATTAC TGCGCTCGGGGCTATTATGCCATTGATTATTGGGGACA GGGAACAACAGTGACTGTGTCCTCCGCTAGCACCAAG GGCCCATCGGTCTTCCCCCTGGCACCCTCCTCCAAGAG CACCTCTGGGGGCACAGCGGCCCTGGGCTGCCTGGTCA AGGACTACTTCCCCGAACCGGTGACGGTGTCGTGGAAC TCAGGCGCCCTGACCAGCGGCGTGCACACCTTCCCGGC TGTCCTACAGTCCTCAGGACTCTACTCCCTCAGCAGCG TGGTGACCGTGCCCTCCAGCAGCTTGGGCACCCAGACC TACATCTGCAACGTGAATCACAAGCCCAGCAACACCA AGGTGGACAAGAAAGTTGAGCCCAAATCTTGTGACAA AACTCACACATGCCCACCGTGCCCAGCACCTGAAGCTG CAGGGGGACCGTCAGTCTTCCTCTTCCCCCCAAAACCC AAGGACACCCTCATGATCTCCCGGACCCCTGAGGTCAC ATGCGTGGTGGTGGACGTGAGCCACGAAGACCCTGAG GTCAAGTTCAACTGGTACGTGGACGGCGTGGAGGTGC ATAATGCCAAGACAAAGCCGCGGGAGGAGCAGTACAA CAGCACGTACCGTGTGGTCAGCGTCCTCACCGTCCTGC ACCAGGACTGGCTGAATGGCAAGGAGTACAAGTGCAA GGTCTCCAACAAAGCCCTCGGCGCCCCCATCGAGAAA ACCATCTCCAAAGCCAAAGGGCAGCCCCGAGAACCAC AGGTGTGCACCCTGCCCCCATCCCGGGATGAGCTGACC AAGAACCAGGTCAGCCTCTCGTGCGCAGTCAAAGGCTT CTATCCCAGCGACATCGCCGTGGAGTGGGAGAGCAAT GGGCAGCCGGAGAACAACTACAAGACCACGCCTCCCG TGCTGGACTCCGACGGCTCCTTCTTCCTCGTGAGCAAG CTCACCGTGGACAAGAGCAGGTGGCAGCAGGGGAACG TCTTCTCATGCTCCGTGATGCATGAGGCTCTGCACAAC CACTACACGCAGAAGAGCCTCTCCCTGTCTCCGGGTGG AGGCGGCGGAAGCGGAGGAGGAGGATCCGGTGGTGGC GGATCTGGGGGCGGTGGATCTGAGATCGTGCTGACCC AGTCTCCGGCACCCTGAGCCTGAGCCCTGGCGAGAG AGCCACCCTGAGCTGCAGAGCCAGCCAGAGCGTGAGC CGGAGCTACCTGGCCTGGTATCAGCAGAAGCCCGGCC AGGCCCCCAGACTGCTGATCATCGGCGCCAGCACCCG GGCCACCGGCATCCCCGATAGATTCAGCGGCAGCGGC TCCGGCACCGACTTCACCCTGACCATCAGCCGGCTGGA ACCCGAGGACTTCGCCGTGTACTACTGCCAGCAGGGCC AGGTGATCCCCCCCACCTTCGGCCAGGGCACCAAGGTG GAAATCAAG |
| 393 | (8A06) VLCL-light chain | SSELTQDPAVSVALGQTVRITCQGDSLRSYYASWYQQKP GQAPVLVIYGKNNRPSGIPDRFSGSSSGNTASLTITGAQAE DEADYYCNSPQTSGSAVFGGGTKLTVLGQPKAAPSVTLF PPSSEELQANKATLVCLISDFYPGAVTVAWKADSSPVKA GVETTTPSKQSNNKYAASSYLSLTPEQWKSHRSYSCQVT HEGSTVEKTVAPTECS |
| 401 | HC 1 | QVQLVQSGAEVKKPGSSVKVSCKASGGTFSSYAISWVRQ |
| | (8A06) VHCH1-_VHCH1-_Fc knob VH (28H1) | APGQGLEWMGGIIPIFGTANYAQKFQGRVTITADKSTSTA YMELSSLRSEDTAVYYCARGYYAIDYWGQGTTVTVSSA STKGPSVFPLAPSSKSTSGGTAALGCLVKDYFPEPVTVSW NSGALTSGVHTFPAVLQSSGLYSLSSVVTVPSSSLGTQTYI CNVNHKPSNTKVDKKVEPKSCDGGGGSGGGGSQVQLVQ SGAEVKKPGSSVKVSCKASGGTFSSYAISWVRQAPGQGL EWMGGIIPIFGTANYAQKFQGRVTITADKSTSTAYMELSS LRSEDTAVYYCARGYYAIDYWGQGTTVTVSSASTKGPSV FPLAPSSKSTSGGTAALGCLVKDYFPEPVTVSWNSGALTS GVHTFPAVLQSSGLYSLSSVVTVPSSSLGTQTYICNVNHK PSNTKVDKKVEPKSCDKTHTCPPCPAPEAAGGPSVFLFPP KPKDTLMISRTPEVTCVVVDVSHEDPEVKFNWYVDGVE VHNAKTKPREEQYNSTYRVVSVLTVLHQDWLNGKEYKC KVSNKALGAPIEKTISKAKGQPREPQVYTLPPCRDELTKN QVSLWCLVKGFYPSDIAVEWESNGQPENNYKTTPPVLDS DGSFFLYSKLTVDKSRWQQGNVFSCSVMHEALHNHYTQ KSLSLSPGGGGGSGGGGSGGGGSGGGGSEVQLLESGGGL VQPGGSLRLSCAASGFTFSSHAMSWVRQAPGKGLEWVS AIWASGEQYYADSVKGRFTISRDNSKNTLYLQMNSLRAE DTAVYYCAKGWLGNFDYWGQGTLVTVSS |
| 402 | HC 2 (8A06) VHCH1-_----VHCH1 Fc hole VL (28H1) | QVQLVQSGAEVKKPGSSVKVSCKASGGTFSSYAISWVRQ APGQGLEWMGGIIPIFGTANYAQKFQGRVTITADKSTSTA YMELSSLRSEDTAVYYCARGYYAIDYWGQGTTVTVSSA STKGPSVFPLAPSSKSTSGGTAALGCLVKDYFPEPVTVSW NSGALTSGVHTFPAVLQSSGLYSLSSVVTVPSSSLGTQTYI CNVNHKPSNTKVDKKVEPKSCDGGGGSGGGGSQVQLVQ SGAEVKKPGSSVKVSCKASGGTFSSYAISWVRQAPGQGL EWMGGIIPIFGTANYAQKFQGRVTITADKSTSTAYMELSS LRSEDTAVYYCARGYYAIDYWGQGTTVTVSSASTKGPSV FPLAPSSKSTSGGTAALGCLVKDYFPEPVTVSWNSGALTS GVHTFPAVLQSSGLYSLSSVVTVPSSSLGTQTYICNVNHK PSNTKVDKKVEPKSCDKTHTCPPCPAPEAAGGPSVFLFPP KPKDTLMISRTPEVTCVVVDVSHEDPEVKFNWYVDGVE VHNAKTKPREEQYNSTYRVVSVLTVLHQDWLNGKEYKC KVSNKALGAPIEKTISKAKGQPREPQVCTLPPSRDELTKN QVSLSCAVKGFYPSDIAVEWESNGQPENNYKTTPPVLDS DGSFFLVSKLTVDKSRWQQGNVFSCSVMHEALHNHYTQ KSLSLSPGGGGGSGGGGSGGGGSGGGGSEIVLTQSPGTLS LSPGERATLSCRASQSVSRSYLAWYQQKPGQAPRLLIIGA STRATGIPDRFSGSGSGTDFTLTISRLEPEDFAVYYCQQGQ VIPPTFGQGTKVEIK |
| 395 | (6C8) VLCL-light chain (nucleotide sequence) | GAGATCGTGATGACCCAGTCCCCCGCCACCCTGTCCGT GTCTCCAGGCGAGAGAGCCACCCTGAGCTGCAAGGCC TCCCAGAACGTGGGCACCAACGTGGCCTGGTATCAGC AGAAGCCCGGCCAGGCCCCTCGGCTGCTGATCTACTCC GCCTCCTACCGGTACTCCGGCATCCCTGCCCGGTTCTC CGGCTCTGGCTCTGGCACCGAGTTTACCCTGACCATCT CCAGCCTGCAGTCCGAGGACTTCGCCGTGTACTACTGC CAGCAGTACAACACCGACCCCCTGACCTTCGGCGGAG GCACCAAGGTGGAAATCAAACGTACGGTGGCTGCACC ATCTGTCTTCATCTTCCCGCCATCTGATGAGCAGTTGA AATCTGGAACTGCCTCTGTTGTGTGCCTGCTGAATAAC TTCTATCCCAGAGAGGCCAAAGTACAGTGGAAGGTGG ATAACGCCCTCCAATCGGGTAACTCCCAGGAGAGTGTC |
| | | ACAGAGCAGGACAGCAAGGACAGCACCTACAGCCTCA GCAGCACCCTGACGCTGAGCAAAGCAGACTACGAGAA ACACAAAGTCTACGCCTGCGAAGTCACCCATCAGGGC CTGAGCTCGCCCGTCACAAAGAGCTTCAACAGGGGAG AGTGT |
| 403 | HC 1 (6C8) VHCH1-_VHCH1-_Fc knob VH (28H1) (nucleotide sequence) | CAGGTCACACTGAGAGAGTCCGGCCCTGCCCTGGTCAA GCCCACCCAGACCCTGACCCTGACATGCACCTTCTCCG GCTTCTCCCTGTCCACCTCCGGCATGGGCGTGGGCTGG ATCAGACAGCCTCCTGGCAAGGCCCTGGAATGGCTGG CCCACATTTGGTGGGACGACGACAAGTACTACCAGCCC TCCCTGAAGTCCCGGCTGACCATCTCCAAGGACACCTC CAAGAACCAGGTGGTGCTGACCATGACCAACATGGAC CCCGTGGACACCGCCACCTACTACTGCGCCCGGACCCG GCGGTACTTCCCCTTTGCTTATTGGGGCCAGGGCACCC TGGTCACCGTCTCGAGCGCTTCTACCAAGGGCCCCAGC GTGTTCCCTCTGGCCCCTAGCAGCAAGAGCACATCTGG CGGAACAGCCGCCCTGGGCTGCCTCGTGAAGGACTACT TTCCCGAGCCCGTGACAGTGTCCTGGAACTCTGGCGCC CTGACAAGCGGCGTGCACACCTTTCCAGCCGTGCTCCA GAGCAGCGGCCTGTACTCTCTGAGCAGCGTCGTGACTG TGCCCAGCAGCAGCCTGGGAACCCAGACCTACATCTGC AACGTGAACCACAAGCCCAGCAACACCAAGGTGGACA AGAAGGTGGAACCCAAGAGCTGCGACGGCGGAGGCGG ATCTGGCGGCGGAGGATCCCAGGTCACACTGAGAGAG TCCGGCCCTGCCCTGGTCAAGCCCACCCAGACCCTGAC CCTGACATGCACCTTCTCCGGCTTCTCCCTGTCCACCTC CGGCATGGGCGTGGGCTGGATCAGACAGCCTCCTGGC AAGGCCCTGGAATGGCTGGCCCACATTTGGTGGGACG ACGACAAGTACTACCAGCCCTCCCTGAAGTCCCGGCTG ACCATCTCCAAGGACACCTCCAAGAACCAGGTGGTGCT GACCATGACCAACATGGACCCCGTGGACACCGCCACC TACTACTGCGCCCGGACCCGGCGGTACTTCCCCTTTGC TTATTGGGGCCAGGGCACCCTGGTCACCGTCTCGAGTG CTAGCACCAAGGGCCCATCGGTCTTCCCCCTGGCACCC TCCTCCAAGAGCACCTCTGGGGGCACAGCGGCCCTGG GCTGCCTGGTCAAGGACTACTTCCCCGAACCGGTGACG GTGTCGTGGAACTCAGGCGCCCTGACCAGCGGCGTGC ACACCTTCCCGGCTGTCCTACAGTCCTCAGGACTCTAC TCCCTCAGCAGCGTGGTGACCGTGCCCTCCAGCAGCTT GGGCACCCAGACCTACATCTGCAACGTGAATCACAAG CCCAGCAACACCAAGGTGGACAAGAAAGTTGAGCCCA AATCTTGTGACAAAACTCACACATGCCCACCGTGCCCA GCACCTGAAGCTGCAGGGGGACCGTCAGTCTTCCTCTT CCCCCCAAAACCCAAGGACACCCTCATGATCTCCCGGA CCCCTGAGGTCACATGCGTGGTGGTGGACGTGAGCCAC GAAGACCCTGAGGTCAAGTTCAACTGGTACGTGGACG GCGTGGAGGTGCATAATGCCAAGACAAAGCCGCGGGA GGAGCAGTACAACAGCACGTACCGTGTGGTCAGCGTC CTCACCGTCCTGCACCAGGACTGGCTGAATGGCAAGG AGTACAAGTGCAAGGTCTCCAACAAAGCCCTCGGCGC CCCCATCGAGAAAACCATCTCCAAAGCCAAAGGGCAG CCCCGAGAACCACAGGTGTACACCCTGCCCCCCTGCAG AGATGAGCTGACCAAGAACCAGGTGTCCCTGTGGTGTC TGGTCAAGGGCTTCTACCCCAGCGATATCGCCGTGGAG |
| | | TGGGAGAGCAACGGCCAGCCTGAGAACAACTACAAGA CCACCCCCCCTGTGCTGGACAGCGACGGCAGCTTCTTC CTGTACTCCAAACTGACCGTGGACAAGAGCCGGTGGC AGCAGGGCAACGTGTTCAGCTGCAGCGTGATGCACGA GGCCCTGCACAACCACTACACCCAGAAGTCCCTGAGCC TGAGCCCCGGCGGAGGCGGCGGAAGCGGAGGAGGAG GATCCGGAGGAGGGGGAAGTGGCGGCGGAGGATCTGA GGTGCAGCTGCTGGAATCCGGCGGAGGCCTGGTGCAG CCTGGCGGATCTCTGAGACTGTCCTGCGCCGCCTCCGG CTTCACCTTCTCCTCCCACGCCATGTCCTGGGTCCGACA GGCTCCTGGCAAAGGCCTGGAATGGGTGTCCGCCATCT GGGCCTCCGGCGAGCAGTACTACGCCGACTCTGTGAA GGGCCGGTTCACCATCTCCCGGGACAACTCCAAGAAC ACCCTGTACCTGCAGATGAACTCCCTGCGGGCCGAGGA CACCGCCGTGTACTACTGTGCCAAGGGCTGGCTGGGCA ACTTCGACTACTGGGGCCAGGGCACCCTGGTCACCGTG TCCAGC |
| 404 | HC 2 (6C8) VHCH1-_VHCH1-_Fc hole VL (28H1) (nucleotide sequence) | CAGGTCACACTGAGAGAGTCCGGCCCTGCCCTGGTCAA GCCCACCCAGACCCTGACCCTGACATGCACCTTCTCCG GCTTCTCCCTGTCCACCTCCGGCATGGGCGTGGGCTGG ATCAGACAGCCTCCTGGCAAGGCCCTGGAATGGCTGG CCCACATTTGGTGGGACGACGACAAGTACTACCAGCCC TCCCTGAAGTCCCGGCTGACCATCTCCAAGGACACCTC CAAGAACCAGGTGGTGCTGACCATGACCAACATGGAC CCCGTGGACACCGCCACCTACTACTGCGCCCGGACCCG GCGGTACTTCCCCTTTGCTTATTGGGGCCAGGGCACCC TGGTCACCGTCTCGAGCGCTTCTACCAAGGGCCCCAGC GTGTTCCCTCTGGCCCCTAGCAGCAAGAGCACATCTGG CGGAACAGCCGCCCTGGGCTGCCTCGTGAAGGACTACT TTCCCGAGCCCGTGACAGTGTCCTGGAACTCTGGCGCC CTGACAAGCGGCGTGCACACCTTTCCAGCCGTGCTCCA GAGCAGCGGCCTGTACTCTCTGAGCAGCGTCGTGACTG TGCCCAGCAGCAGCCTGGGAACCCAGACCTACATCTGC AACGTGAACCACAAGCCCAGCAACACCAAGGTGGACA AGAAGGTGGAACCCAAGAGCTGCGACGGCGGAGGCGG ATCTGGCGGCGGAGGATCCCAGGTCACACTGAGAGAG TCCGGCCCTGCCCTGGTCAAGCCCACCCAGACCCTGAC CCTGACATGCACCTTCTCCGGCTTCTCCCTGTCCACCTC CGGCATGGGCGTGGGCTGGATCAGACAGCCTCCTGGC AAGGCCCTGGAATGGCTGGCCCACATTTGGTGGGACG ACGACAAGTACTACCAGCCCTCCCTGAAGTCCCGGCTG ACCATCTCCAAGGACACCTCCAAGAACCAGGTGGTGCT GACCATGACCAACATGGACCCCGTGGACACCGCCACC TACTACTGCGCCCGGACCCGGCGGTACTTCCCCTTTGC TTATTGGGGCCAGGGCACCCTGGTCACCGTCTCGAGTG CTAGCACCAAGGGCCCATCGGTCTTCCCCCTGGCACCC TCCTCCAAGAGCACCTCTGGGGGCACAGCGGCCCTGG GCTGCCTGGTCAAGGACTACTTCCCCGAACCGGTGACG GTGTCGTGGAACTCAGGCGCCCTGACCAGCGGCGTGC ACACCTTCCCGGCTGTCCTACAGTCCTCAGGACTCTAC TCCCTCAGCAGCGTGGTGACCGTGCCCTCCAGCAGCTT GGGCACCCAGACCTACATCTGCAACGTGAATCACAAG CCCAGCAACACCAAGGTGGACAAGAAAGTTGAGCCCA AATCTTGTGACAAAACTCACACATGCCCACCGTGCCCA |
| | | GCACCTGAAGCTGCAGGGGGACCGTCAGTCTTCCTCTT CCCCCCAAAACCCAAGGACACCCTCATGATCTCCCGGA CCCCTGAGGTCACATGCGTGGTGGTGGACGTGAGCCAC GAAGACCCTGAGGTCAAGTTCAACTGGTACGTGGACG GCGTGGAGGTGCATAATGCCAAGACAAAGCCGCGGGA GGAGCAGTACAACAGCACGTACCGTGTGGTCAGCGTC CTCACCGTCCTGCACCAGGACTGGCTGAATGGCAAGG AGTACAAGTGCAAGGTCTCCAACAAAGCCCTCGGCGC CCCCATCGAGAAAACCATCTCCAAAGCCAAAGGGCAG CCCCGAGAACCACAGGTGTGCACCCTGCCCCCATCCCG GGATGAGCTGACCAAGAACCAGGTCAGCCTCTCGTGC GCAGTCAAAGGCTTCTATCCCAGCGACATCGCCGTGGA GTGGGAGAGCAATGGGCAGCCGGAGAACAACTACAAG ACCACGCCTCCCGTGCTGGACTCCGACGGCTCCTTCTT CCTCGTGAGCAAGCTCACCGTGGACAAGAGCAGGTGG CAGCAGGGGAACGTCTTCTCATGCTCCGTGATGCATGA GGCTCTGCACAACCACTACACGCAGAAGAGCCTCTCCC TGTCTCCGGGTGGAGGCGGCGGAAGCGGAGGAGGAGG ATCCGGTGGTGGCGGATCTGGGGGCGGTGGATCTGAG ATCGTGCTGACCCAGTCTCCCGGCACCCTGAGCCTGAG CCCTGGCGAGAGAGCCACCCTGAGCTGCAGAGCCAGC CAGAGCGTGAGCCGGAGCTACCTGGCCTGGTATCAGC AGAAGCCCGGCCAGGCCCCCAGACTGCTGATCATCGG CGCCAGCACCCGGGCCACCGGCATCCCCGATAGATTCA GCGGCAGCGGCTCCGGCACCGACTTCACCCTGACCATC AGCCGGCTGGAACCCGAGGACTTCGCCGTGTACTACTG CCAGCAGGGCCAGGTGATCCCCCCCACCTTCGGCCAGG GCACCAAGGTGGAAATCAAG |
| 397 | (6C8) VLCL-light chain | EIVMTQSPATLSVSPGERATLSCKASQNVGTNVAWYQQK PGQAPRLLIYSASYRYSGIPARFSGSGSGTEFTLTISSLQSE DFAVYYCQQYNTDPLTFGGGTKVEIKRTVAAPSVFIFPPS DEQLKSGTASVVCLLNNFYPREAKVQWKVDNALQSGNS QESVTEQDSKDSTYSLSSTLTLSKADYEKHKVYACEVTH QGLSSPVTKSFNRGEC |
| 405 | HC 1 (6C8) VHCH1-_VHCH1-_Fc knob VH (28H1) | QVTLRESGPALVKPTQTLTLTCTFSGFSLSTSGMGVGWIR QPPGKALEWLAHIWWDDDKYYQPSLKSRLTISKDTSKNQ VVLTMTNMDPVDTATYYCARTRRYFPFAYWGQGTLVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDGGGGSGGGGSQ VTLRESGPALVKPTQTLTLTCTFSGFSLSTSGMGVGWIRQ PPGKALEWLAHIWWDDDKYYQPSLKSRLTISKDTSKNQV VLTMTNMDPVDTATYYCARTRRYFPFAYWGQGTLVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPEAA GGPSVFLFPPKPKDTLMISRTPEVTCVVVDVSHEDPEVKF NWYVDGVEVHNAKTKPREEQYNSTYRVVSVLTVLHQD WLNGKEYKCKVSNKALGAPIEKTISKAKGQPREPQVYTL PPCRDELTKNQVSLWCLVKGFYPSDIAVEWESNGQPENN YKTTPPVLDSDGSFFLYSKLTVDKSRWQQGNVFSCSVMH EALHNHYTQKSLSLSPGGGGGSGGGGSGGGGSGGGGSE VQLLESGGGLVQPGGSLRLSCAASGFTFSSHAMSWVRQA PGKGLEWVSAIWASGEQYYADSVKGRFTISRDNSKNTLY |
| | | LQMNSLRAEDTAVYYCAKGWLGNFDYWGQGTLVTVSS |
| 406 | HC 2 (6C8) VHCH1-_VHCH1-_Fc hole VL (28H1) | QVTLRESGPALVKPTQTLTLTCTFSGFSLSTSGMGVGWIR QPPGKALEWLAHIWWDDDKYYQPSLKSRLTISKDTSKNQ VVLTMTNMDPVDTATYYCARTRRYFPFAYWGQGTLVT VSSASTKGPSVFPLAPSSKSTSGGTAALGCLVKDYFPEPV TVSWNSGALTSGVHTFPAVLQSSGLYSLSSVVTVPSSSLG TQTYICNVNHKPSNTKVDKKVEPKSCDGGGGSGGGGSQ VTLRESGPALVKPTQTLTLTCTFSGFSLSTSGMGVGWIRQ PPGKALEWLAHIWWDDDKYYQPSLKSRLTISKDTSKNQV VLTMTNMDPVDTATYYCARTRRYFPFAYWGQGTLVTVS SASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPEAA GGPSVFLFPPKPKDTLMISRTPEVTCVVVDVSHEDPEVKF NWYVDGVEVHNAKTKPREEQYNSTYRVVSVLTVLHQD WLNGKEYKCKVSNKALGAPIEKTISKAKGQPREPQVCTL PPSRDELTKNQVSLSCAVKGFYPSDIAVEWESNGQPENN YKTTPPVLDSDGSFFLVSKLTVDKSRWQQGNVFSCSVMH EALHNHYTQKSLSLSPGGGGGSGGGGSGGGGSGGGGSEI VLTQSPGTLSLSPGERATLSCRASQSVSRSYLAWYQQKPG QAPRLLIIGASTRATGIPDRFSGSGSGTDFTLTISRLEPEDF AVYYCQQGQVIPPTFGQGTKVEIK |

Bispecific agonistic GITR antibodies with tetravalent binding for GITR and with bivalent binding for FAP (4+2 constructs) were also prepared. The crossmab technology in accordance with International patent application No. WO 2010/145792 A1 was applied to reduce the formation of wrongly paired light chains. In this example, a crossed Fab unit (VHCL) of the FAP binder 28H1 was fused to the C-terminus of the Fc part of both heavy chains. Two Fabs against GITR were fused to the N-terminus of each heavy chain as described in Example 4.2. In this case the introduction of a knob into hole was not necessary as both heavy chains contained the same domains.

The Pro329Gly, Leu234Ala and Leu235Ala mutations were introduced in the constant region of the heavy chains to abrogate binding to Fc gamma receptors according to the method described in International Patent Appl. Publ. No. WO 2012/130831 A1. The resulting bispecific, tetravalent construct is depicted in **Figure 53B****. Table 84** shows, respectively, the nucleotide and amino acid sequences of mature bispecific, tetravalent anti-OX40/anti-FAP human IgG1 P329GLALA antibodies.

**Table 84: cDNA and amino acid sequences of mature bispecific 4+2 anti-GITR, anti-FAP human IgG1 P329GLALA kih antibodies**

| SEQ ID NO: | Description | Sequence |
|---|---|---|
| 391 | (8A06) VLCL-light chain (nucleotide sequence) | see Table 78 |
| 407 | heavy chain (8A06) VHCH1-_VHCH1-_ Fc VHCL (28H1) (nucleotide sequence) | CAGGTGCAGCTGGTGCAGTCTGGCGCCGAAGTGAAGAA CCCGGCAGCAGCGTGAAGGTGTCCTGCAAGGCTTCCGGC GGCACCTTCAGCAGCTACGCCATTTCTTGGGTGCGCCAGG CCCCTGGACAGGGCCTGGAATGGATGGGCGGCATCATCC CCATCTTCGGCACCGCCAACTACGCCCAGAAATTCCAGGG CAGAGTGACCATCACCGCCGACAAGAGCACCAGCACCGC CTACATGGAACTGAGCAGCCTGCGGAGCGAGGACACCGC CGTGTACTATTGCGCCAGAGGCTACTACGCCATCGACTAC TGGGGCCAGGGCACCACCGTGACCGTGTCTAGCGCTTCTA CCAAGGGCCCCAGCGTGTTCCCTCTGGCCCCTAGCAGCAA GAGCACATCTGGCGGAACAGCCGCCCTGGGCTGCCTCGT GAAGGACTACTTTCCCGAGCCCGTGACAGTGTCCTGGAAC TCTGGCGCCCTGACAAGCGGCGTGCACACCTTTCCAGCCG TGCTCCAGAGCAGCGGCCTGTACTCTCTGAGCAGCGTCGT GACTGTGCCCAGCAGCAGCCTGGGAACCCAGACCTACAT CTGCAACGTGAACCACAAGCCCAGCAACACCAAGGTGGA CAAGAAGGTGGAACCCAAGAGCTGCGACGGCGGAGGCG GATCTGGCGGCGGAGGATCCCAGGTGCAGCTGGTGCAGA GCGGAGCTGAAGTGAAAAAGCCTGGCTCCTCCGTGAAAG TGTCTTGTAAAGCCAGCGGCGGCACATTCTCTAGCTATGC CATCAGCTGGGTGCGGCAGGCTCCAGGCCAGGGACTGGA ATGGATGGGAGGAATTATCCCTATTTTTGGACAGCCAAT TATGCTCAGAAATTTCAGGGGCGCGTGACAATTACAGCC GACAAGTCCACCTCTACAGCTTATATGGAACTGTCCTCCC TGCGCTCCGAGGATACAGCTGTGTATTACTGCGCTCGGGG CTATTATGCCATTGATTATTGGGGACAGGGAACAACAGTG ACTGTGTCCTCCGCTAGCACCAAGGGCCCATCCGTGTTCC CTCTGGCCCCTTCCAGCAAGTCTACCTCTGGCGGCACAGC CGCTCTGGGCTGCCTCGTGAAGGACTACTTCCCCGAGCCT GTGACAGTGTCCTGGAACTCTGGCGCCCTGACATCCGGCG TGCACACCTTTCCAGCTGTGCTGCAGTCCTCCGGCCTGTA CTCCCTGTCCTCCGTCGTGACAGTGCCCTCCAGCTCTCTG GGCACCCAGACCTACATCTGCAACGTGAACCACAAGCCC TCCAACACCAAGGTGGACAAGAAGGTGGAACCCAAGTCC TGCGACAAGACCCACACCTGTCCCCCTTGTCCTGCCCCTG AAGCTGCTGGCGGCCCTAGCGTGTTCCTGTTCCCCCCAAA GCCCAAGGACACCCTGATGATCTCCCGGACCCCCGAAGT GACCTGCGTGGTGGTGGATGTGTCCCACGAGGACCCTGA AGTGAAGTTCAATTGGTACGTGGACGGCGTGGAAGTGCA CAATGCCAAGACCAAGCCTAGAGAGGAACAGTACAACTC CACCTACCGGGTGGTGTCCGTGCTGACCGTGCTGCACCAG GATTGGCTGAACGGCAAAGAGTACAAGTGCAAGGTGTCC AACAAGGCCCTGGGAGCCCCCATCGAAAAGACCATCTCC AAGGCCAAGGGCCAGCCTCGCGAGCCTCAGGTGTACACC CTGCCCCCTAGCAGAGATGAGCTGACCAAGAACCAGGTG TCCCTGACCTGTCTCGTGAAAGGCTTCTACCCCTCCGATA TCGCCGTGGAATGGGAGAGCAACGGCCAGCCCGAGAACA ACTACAAGACCACCCCCCCTGTGCTGGACTCCGACGGCTC ATTCTTCCTGTACTCTAAGCTGACAGTGGACAAGTCCCGG TGGCAGCAGGGCAACGTGTTCTCCTGCTCCGTGATGCACG AGGCCCTGCACAACCACTACACCCAGAAGTCCCTGTCCCT GTCTCCCGGGGGAGGCGGAGGATCTGGCGGAGGCGGATC |
| | | CGGTGGTGGCGGATCTGGGGGCGGTGGATCTGAGGTGCA GCTGCTGGAATCTGGGGGAGGACTGGTGCAGCCAGGCGG ATCTCTGAGGCTGTCCTGCGCTGCTTCCGGCTTTACCTTCT CCAGCCACGCCATGAGTTGGGTGCGCCAGGCACCCGGAA AAGGACTGGAATGGGTGTCAGCCATCTGGGCCTCCGGCG AGCAGTACTACGCCGATAGCGTGAAGGGCCGGTTCACCA TCTCTCGGGATAACAGCAAGAATACTCTGTACCTGCAGAT GAACTCCCTGCGCGCTGAAGATACCGCTGTGTATTACTGC GCCAAGGGCTGGCTGGGCAACTTCGATTACTGGGGCCAG GGAACCCTCGTGACTGTCTCGAGCGCTTCTGTGGCCGCTC CCTCCGTGTTCATCTTCCCACCTTCCGACGAGCAGCTGAA GTCCGGCACTGCCTCTGTCGTGTGCCTGCTGAACAACTTC TACCCTCGGGAAGCCAAGGTGCAGTGGAAAGTGGATAAC GCCCTGCAGTCCGGCAACTCCCAGGAATCCGTGACCGAG CAGGACTCCAAGGACAGCACCTACTCCCTGAGCAGCACC CTGACCCTGTCCAAGGCCGACTACGAGAAGCACAAGGTG TACGCCTGTGAAGTGACCCACCAGGGCCTGTCCAGCCCCG TGACCAAGTCCTTCAACCGGGGCGAGTGC |
| 408 | (28H1) VLCH1-light chain 2 (nucleotide sequence) | GAGATCGTGCTGACCCAGTCTCCCGGCACCCTGAGCCTGA GCCCTGGCGAGAGAGCCACCCTGAGCTGCAGAGCCAGCC AGAGCGTGAGCCGGAGCTACCTGGCCTGGTATCAGCAGA AGCCCGGCCAGGCCCCCAGACTGCTGATCATCGGCGCCA GCACCCGGGCCACCGGCATCCCCGATAGATTCAGCGGCA GCGGCTCCGGCACCGACTTCACCCTGACCATCAGCCGGCT GGAACCCGAGGACTTCGCCGTGTACTACTGCCAGCAGGG CCAGGTGATCCCCCCCACCTTCGGCCAGGGCACCAAGGT GGAAATCAAGAGCAGCGCTTCCACCAAAGGCCCTTCCGT GTTTCCTCTGGCTCCTAGCTCCAAGTCCACCTCTGGAGGC ACCGCTGCTCTCGGATGCCTCGTGAAGGATTATTTTCCTG AGCCTGTGACAGTGTCCTGGAATAGCGGAGCACTGACCT CTGGAGTGCATACTTTCCCCGCTGTGCTGCAGTCCTCTGG ACTGTACAGCCTGAGCAGCGTGGTGACAGTGCCCAGCAG CAGCCTGGGCACCCAGACCTACATCTGCAACGTGAACCA CAAGCCCAGCAACACCAAGGTGGACAAGAAGGTGGAACC CAAGTCTTGT |
| 393 | (8A06) VLCL-light chain | see Table 78 |
| 409 | heavy chain (8A06) VHCH1-_VHCH1-_ Fc VHCL (28H1) | QVQLVQSGAEVKKPGSSVKVSCKASGGTFSSYAISWVRQAP GQGLEWMGGIIPIFGTANYAQKFQGRVTITADKSTSTAYME LSSLRSEDTAVYYCARGYYAIDYWGQGTTVTVSSASTKGPS VFPLAPSSKSTSGGTAALGCLVKDYFPEPVTVSWNSGALTSG VHTFPAVLQSSGLYSLSSVVTVPSSSLGTQTYICNVNHKPSN TKVDKKVEPKSCDGGGGSGGGGSQVQLVQSGAEVKKPGSS VKVSCKASGGTFSSYAISWVRQAPGQGLEWMGGIIPIFGTAN YAQKFQGRVTITADKSTSTAYMELSSLRSEDTAVYYCARGY YAIDYWGQGTTVTVSSASTKGPSVFPLAPSSKSTSGGTAALG CLVKDYFPEPVTVSWNSGALTSGVHTFPAVLQSSGLYSLSSV VTVPSSSLGTQTYICNVNHKPSNTKVDKKVEPKSCDKTHTC PPCPAPEAAGGPSVFLFPPKPKDTLMISRTPEVTCVVVDVSH EDPEVKFNWYVDGVEVHNAKTKPREEQYNSTYRVVSVLTV LHQDWLNGKEYKCKVSNKALGAPIEKTISKAKGQPREPQVY TLPPSRDELTKNQVSLTCLVKGFYPSDIAVEWESNGQPENNY KTTPPVLDSDGSFFLYSKLTVDKSRWQQGNVFSCSVMHEAL HNHYTQKSLSLSPGGGGGSGGGGSGGGGSGGGGSEVQLLES |
| | | GGGLVQPGGSLRLSCAASGFTFSSHAMSWVRQAPGKGLEW VSAIWASGEQYYADSVKGRFTISRDNSKNTLYLQMNSLRAE DTAVYYCAKGWLGNFDYWGQGTLVTVSSASVAAPSVFIFP PSDEQLKSGTASVVCLLNNFYPREAKVQWKVDNALQSGNS QESVTEQDSKDSTYSLSSTLTLSKADYEKHKVYACEVTHQG LSSPVTKSFNRGEC |
| 410 | (28H1) VLCH1-light chain 2 | EIVLTQSPGTLSLSPGERATLSCRASQSVSRSYLAWYQQKPG QAPRLLIIGASTRATGIPDRFSGSGSGTDFTLTISRLEPEDFAV YYCQQGQVIPPTFGQGTKVEIKSSASTKGPSVFPLAPSSKSTS GGTAALGCLVKDYFPEPVTVSWNSGALTSGVHTFPAVLQSS GLYSLSSVVTVPSSSLGTQTYICNVNHKPSNTKVDKKVEPKS C |
| 395 | (6C8) VLCL-light chain (nucleotide sequence) | see Table 78 |
| 411 | heavy chain (6C8) VHCH1-_VHCH1-_ Fc VHCL (28H1) (nucleotide sequence) | CAGGTCACACTGAGAGAGTCCGGCCCTGCCCTGGTCAAG CCCACCCAGACCCTGACCCTGACATGCACCTTCTCCGGCT TCTCCCTGTCCACCTCCGGCATGGGCGTGGGCTGGATCAG ACAGCCTCCTGGCAAGGCCCTGGAATGGCTGGCCCACATT TGGTGGGACGACGACAAGTACTACCAGCCCTCCCTGAAG TCCCGGCTGACCATCTCCAAGGACACCTCCAAGAACCAG GTGGTGCTGACCATGACCAACATGGACCCCGTGGACACC GCCACCTACTACTGCGCCCGGACCCGGCGGTACTTCCCCT TTGCTTATTGGGGCCAGGGCACCCTGGTCACCGTCTCGAG CGCTTCTACCAAGGGCCCCAGCGTGTTCCCTCTGGCCCCT AGCAGCAAGAGCACATCTGGCGGAACAGCCGCCCTGGGC TGCCTCGTGAAGGACTACTTTCCCGAGCCCGTGACAGTGT CCTGGAACTCTGGCGCCCTGACAAGCGGCGTGCACACCTT TCCAGCCGTGCTCCAGAGCAGCGGCCTGTACTCTCTGAGC AGCGTCGTGACTGTGCCCAGCAGCAGCCTGGGAACCCAG ACCTACATCTGCAACGTGAACCACAAGCCCAGCAACACC AAGGTGGACAAGAAGGTGGAACCCAAGAGCTGCGACGG CGGAGGCGGATCTGGCGGCGGAGGATCCCAGGTCACACT GAGAGAGTCCGGCCCTGCCCTGGTCAAGCCCACCCAGAC CCTGACCCTGACATGCACCTTCTCCGGCTTCTCCCTGTCCA CCTCCGGCATGGGCGTGGGCTGGATCAGACAGCCTCCTG GCAAGGCCCTGGAATGGCTGGCCCACATTTGGTGGGACG ACGACAAGTACTACCAGCCCTCCCTGAAGTCCCGGCTGAC CATCTCCAAGGACACCTCCAAGAACCAGGTGGTGCTGAC CATGACCAACATGGACCCCGTGGACACCGCCACCTACTA CTGCGCCCGGACCCGGCGGTACTTCCCCTTTGCTTATTGG GGCCAGGGCACCCTGGTCACCGTCTCGAGTGCTAGCACC AAGGGCCCATCCGTGTTCCCTCTGGCCCCTTCCAGCAAGT CTACCTCTGGCGGCACAGCCGCTCTGGGCTGCCTCGTGAA GGACTACTTCCCCGAGCCTGTGACAGTGTCCTGGAACTCT GGCGCCCTGACATCCGGCGTGCACACCTTTCCAGCTGTGC TGCAGTCCTCCGGCCTGTACTCCCTGTCCTCCGTCGTGAC AGTGCCCTCCAGCTCTCTGGGCACCCAGACCTACATCTGC AACGTGAACCACAAGCCCTCCAACACCAAGGTGGACAAG AAGGTGGAACCCAAGTCCTGCGACAAGACCCACACCTGT CCCCCTTGTCCTGCCCCTGAAGCTGCTGGCGGCCCTAGCG TGTTCCTGTTCCCCCCAAAGCCCAAGGACACCCTGATGAT CTCCCGGACCCCCGAAGTGACCTGCGTGGTGGTGGATGTG TCCCACGAGGACCCTGAAGTGAAGTTCAATTGGTACGTG |
| | | GACGGCGTGGAAGTGCACAATGCCAAGACCAAGCCTAGA GAGGAACAGTACAACTCCACCTACCGGGTGGTGTCCGTG CTGACCGTGCTGCACCAGGATTGGCTGAACGGCAAAGAG TACAAGTGCAAGGTGTCCAACAAGGCCCTGGGAGCCCCC ATCGAAAAGACCATCTCCAAGGCCAAGGGCCAGCCTCGC GAGCCTCAGGTGTACACCCTGCCCCCTAGCAGAGATGAG CTGACCAAGAACCAGGTGTCCCTGACCTGTCTCGTGAAAG GCTTCTACCCCTCCGATATCGCCGTGGAATGGGAGAGCAA CGGCCAGCCCGAGAACAACTACAAGACCACCCCCCCTGT GCTGGACTCCGACGGCTCATTCTTCCTGTACTCTAAGCTG ACAGTGGACAAGTCCCGGTGGCAGCAGGGCAACGTGTTC TCCTGCTCCGTGATGCACGAGGCCCTGCACAACCACTACA CCCAGAAGTCCCTGTCCCTGTCTCCCGGGGGAGGCGGAG GATCTGGCGGAGGCGGATCCGGTGGTGGCGGATCTGGGG GCGGTGGATCTGAGGTGCAGCTGCTGGAATCTGGGGGAG GACTGGTGCAGCCAGGCGGATCTCTGAGGCTGTCCTGCGC TGCTTCCGGCTTTACCTTCTCCAGCCACGCCATGAGTTGG GTGCGCCAGGCACCCGGAAAAGGACTGGAATGGGTGTCA GCCATCTGGGCCTCCGGCGAGCAGTACTACGCCGATAGC GTGAAGGGCCGGTTCACCATCTCTCGGGATAACAGCAAG AATACTCTGTACCTGCAGATGAACTCCCTGCGCGCTGAAG ATACCGCTGTGTATTACTGCGCCAAGGGCTGGCTGGGCAA CTTCGATTACTGGGGCCAGGGAACCCTCGTGACTGTCTCG AGCGCTTCTGTGGCCGCTCCCTCCGTGTTCATCTTCCCACC TTCCGACGAGCAGCTGAAGTCCGGCACTGCCTCTGTCGTG TGCCTGCTGAACAACTTCTACCCTCGGGAAGCCAAGGTGC AGTGGAAAGTGGATAACGCCCTGCAGTCCGGCAACTCCC AGGAATCCGTGACCGAGCAGGACTCCAAGGACAGCACCT ACTCCCTGAGCAGCACCCTGACCCTGTCCAAGGCCGACTA CGAGAAGCACAAGGTGTACGCCTGTGAAGTGACCCACCA GGGCCTGTCCAGCCCCGTGACCAAGTCCTTCAAC CGGGGCGAGTGC |
| 408 | (28H1) VLCH1-light chain 2 (nucleotide sequence) | see above |
| 397 | (6C8) VLCL-light chain | see Table 78 |
| 412 | heavy chain (6C8) VHCH1-_VHCH1-_ Fc VHCL (28H1) | QVTLRESGPALVKPTQTLTLTCTFSGFSLSTSGMGVGWIRQP PGKALEWLAHIWWDDDKYYQPSLKSRLTISKDTSKNQVVL TMTNMDPVDTATYYCARTRRYFPFAYWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKKVEPKSCDGGGGSGGGGSQVTLRESGPALV KPTQTLTLTCTFSGFSLSTSGMGVGWIRQPPGKALEWLAHI WWDDDKYYQPSLKSRLTISKDTSKNQVVLTMTNMDPVDTA TYYCARTRRYFPFAYWGQGTLVTVSSASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKKV EPKSCDKTHTCPPCPAPEAAGGPSVFLFPPKPKDTLMISRTPE VTCVVVDVSHEDPEVKFNWYVDGVEVHNAKTKPREEQYN STYRVVSVLTVLHQDWLNGKEYKCKVSNKALGAPIEKTISK AKGQPREPQVYTLPPSRDELTKNQVSLTCLVKGFYPSDIAVE WESNGQPENNYKTTPPVLDSDGSFFLYSKLTVDKSRWQQG |
| | | NVFSCSVMHEALHNHYTQKSLSLSPGGGGGSGGGGSGGGG SGGGGSEVQLLESGGGLVQPGGSLRLSCAASGFTFSSHAMS WVRQAPGKGLEWVSAIWASGEQYYADSVKGRFTISRDNSK NTLYLQMNSLRAEDTAVYYCAKGWLGNFDYWGQGTLVTV SSASVAAPSVFIFPPSDEQLKSGTASVVCLLNNFYPREAKVQ WKVDNALQSGNSQESVTEQDSKDSTYSLSSTLTLSKADYEK HKVYACEVTHQGLSSPVTKSFNRGEC |
| 410 | (28H1) VLCH1-light chain 2 | see above |

All genes were transiently expressed under control of a chimeric MPSV promoter consisting of the MPSV core promoter combined with the CMV promoter enhancer fragment. The expression vector also contains the oriP region for episomal replication in EBNA (Epstein Barr Virus Nuclear Antigen) containing host cells. The bispecific anti-GITR/ anti-FAP constructs were produced by co-transfecting HEK293-EBNA cells with the mammalian expression vectors using polyethylenimine. The cells were transfected with the corresponding expression vectors in a 1:1:1 ratio ("vector heavy chain 1" : "vector heavy chain 2" : "vector light chain" or "vector heavy chain" : "vector light chain 1" : "vector light chain2").

Production and purification was done according to a protocol as used in section 4.5 for the production of Ox40 bispecific molecules.

**Table 85: Production Summary of bispecific GITR-FAP antibodies with tetravalent GITR binding**

| Format | Yield [mg/L] | Concentration [mg/L] | Monomer (non-red) [%] | HMW [%] | LMW [%] |
|---|---|---|---|---|---|
| (GITR-FAP) [8A06] 4+1 construct | 1.73 | 3.40 | 98.25 | 1.47 | 0.28 |
| (GITR-FAP) [8A06] 4+2 construct | 2.39 | 1.28 | 98.68 | 0.50 | 0.82 |
| (GITR-FAP) [6C8] 4+1 construct | 8.21 | 5.94 | 97.85 | 1.53 | 0.61 |
| (GITR-FAP) [6C8] 4+2 construct | 6.68 | 9.55 | 97.98 | 2.02 | -- |

### 12.2 Simultaneous binding of bispecific GITR/FAP constructs

The capacity of binding simultaneously human GITR Fc(kih) and human FAP was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany). Biotinylated human GITR Fc (kih) was directly coupled to a flow cell of a streptavidin (SA) sensor chip. Immobilization levels up to 1000 resonance units (RU) were used. The bispecific constructs targeting GITR and FAP were passed at a concentration range of 200 nM with a flow of 30 µL/minute through the flow cells over 90 seconds and dissociation was set to zero sec. Human FAP was injected as second analyte with a flow of 30 µL/minute through the flow cells over 90 seconds at a concentration of 500 nM. The dissociation was monitored for 120 sec. Bulk refractive index differences were corrected for by subtracting the response obtained in a reference flow cell, where no protein was immobilized.

All bispecific constructs could bind simultaneously human GITR and human FAP **(****Figures 54B and 54C****).**

### 12.3 Binding to GITR-expressing HEK cells and FAP-expressing 3T3 cells

The binding to cell surface GITR was tested in a similar manner as described in Example 9.6.2. for 4-1BB by using Human Embryonic Kidney 293 (HEK) cells that were engineered to overexpress human GITR protein on the cell surface (HEK-GITR). HEK-GITR cells or parental GITR negative HEK cells (HEK WT) as control were grown in DMEM medium (Gibco Cat.No. 42430-025) supplemented with 10% FBS (Gibco Cat.No. 16140-071), 50 U/mL penicillin-streptomycin (Gibco Cat.No. 15070-063) and GlutaMAX (Gibco Cat.No. 35050-061) with 1 µg/mL puromycin (Gibco Cat.No. A11138-03) for HEK-GITR cells only. The binding to cell surface FAP was tested using 3T3 cells (ATCC CRL-1658 ) that were transfected to express human fibroblast activating protein (huFAP) (see Example 9.6.3.2). Parental 3T3 cells were grown in DMEM medium supplemented with 10% CS (Sigma Cat.No. C8056). 3T3-huFAP cells were grown in DMEM + 10% CS with 1.5 µg/mL puromycin.

Cells were harvested, washed with PBS (Gibco Cat.No. 20012-019) and adjusted to a cell density of 1 × 10⁷ cells/mL in PBS. Control cells (HEK WT and 3T3 WT) were labeled with 2.5 µM of cell proliferation dye eFluor 670 (eBioscience Cat.No. 65-0840-85) in PBS for 7 minutes at 37°C. Cells were washed twice with complete media and once with PBS + 2% FBS before adjusting cell density to 1 × 10⁷ cells/mL in PBS + 2% FBS. 2 × 10⁵ cells (10⁵ HEK WT + 10⁵ HEK GITR or 10⁵ 3T3 WT + 10⁵ 3T3-huFAP) were seeded per well of round-bottom 96-well plates (TPP, Cat. No. 92097).

Plates were centrifuged at 4 °C, 3 minutes at 600 × g and supernatant was flicked off. Cells were resuspended in 50 µL/well of 4 °C cold PBS + 2% FBS containing titrated anti-GITR antibody constructs for 120 minutes at 4 °C. Plates were washed twice with 200 µL/well 4 °C PBS + 2% FBS to remove unbound construct. Cells were stained with Zombie Aqua Fixable Viability Kit (Biolegend Cat.No. 423102) and secondary antibody (Jackson ImmunoResearch Cat.No. 109-116-098) in PBS for 30 minutes at 4°C. Plates were centrifuged at 4°C for 3 minutes at 600 × g and washed twice with PBS + 2% FBS. Cells were fixed with PBS + 2% formaldehyde (Polysciences Cat.No.04018) for 20 minutes at room temperature. Plates were centrifuged at 4 °C, 3 minutes at 600 × g and supernatant was flicked off. Cells were washed with 200 µL/well FACS buffer (eBioscience Cat.No. 00-4222-26) and finally resuspended in 100 µL/well FACS buffer for acquisition the next day using a 4-laser LSR II (BD Bioscience with DIVA software).

As shown in **Figure 55A****,** all tested bispecific anti-GITR constructs bound efficiently to human GITR expressing target cells, but not, or at negligible rates, to GITR negative control cells **(****Figure 55B****)**.Therefore, anti-GITR antibodies maintain their GITR specificity also when expressed as a bispecific, FAP targeted format.

**Table 86: EC₅₀ values for binding of anti-GITR FAP targeted mono or bivalent constructs to cell surface expressed human GITR.**

| **Construct** | **GITR⁺ cell EC₅₀ [nM]** |
|---|---|
| (GITR-FAP) [8A06] 4+2 construct | 3.848 |
| (GITR-FAP) [8A06] 4+1 construct | **1.122** |
| (GITR-DP47) [8A06] 4+1 construct | 1.899 |
| (GITR-DP47) [8A06] 4+2 construct with charged residues | 1.92 |
| (GITR-FAP) [8A06] 4+2 construct with charged residues | 2.057 |

As shown in **Figure 56A****,** all tested bispecific anti-GITR constructs bound efficiently to human FAP-expressing target cells, but not, or at negligible rates, to parental 3T3 cells (negative control, **Figure 56B****)**.Therefore, the bispecific anti-GITR/anti-GITR antibodies maintain their FAP specificity in the bispecific format.

### Example 13

### Functional properties of bispecific anti-human GITR binding molecules

### 13.1 GITR mediated costimulation of human CD4 and CD8 T cells in the presence of suboptimal TCR stimulation

Ligation of GITR provides a synergistic co-stimulatory signal promoting division and survival of T-cells following suboptimal T-cell receptor (TCR) stimulation (Clouthier et al., Cytokine Growth Factor Rev. 2014 Apr;25(2):91-106).

To analyze the agonistic properties of bispecific anti-human GITR binding molecules, a selected binder (clone 8A06) in a FAP targeted monovalent format or its corresponding non-targeted version as control were tested for their ability to co-activate T cells upon surface immobilization. For this, resting ef450-labeled human PBMC were stimulated six days with a suboptimal concentration of anti-CD3 antibody in the presence of irradiated FAP⁺NIH/3T3-huFAP clone 19 cells and titrated anti-GITR constructs. Effects on T-cell proliferation were analyzed through monitoring of total cell counts and ef450 dilution in living cells by flow cytometry.

Buffy coats were obtained from the Zurich blood donation center. To isolate fresh peripheral blood mononuclear cells (PBMCs) the buffy coat was diluted with the same volume of PBS (Gibco by Life Technologies, Cat. No. 20012-019). 50 mL polypropylene centrifuge tubes (TPP, Cat.-No. 91050) were supplied with 15 mL Lymphoprep (Stemcell, Cat No 07851) and the buffy coat solution was layered above the Lymphoprep. The tubes were centrifuged for 30 min at 400 × g, room temperature and with no acceleration and no break. Afterwards the PBMCs were collected from the interface, washed three times with PBS and resuspended in T cell medium consisting of RPMI 1640 medium presuplemented with Glutamax (Gibco by Life Technology, Cat. No. 72400-021) supplied with 10 % Fetal Bovine Serum (FBS, Gibco by Life Technology, Cat. No. 16140-071), 1 % (v/v), 1 mM Sodium-Pyruvat (SIGMA, Cat. No. S8636), 1 % (v/v) MEM non-essential amino acids (SIGMA, Cat.-No. M7145) and 50 µM β-Mercaptoethanol (SIGMA, M3148). PBMCs were used directly after isolation.

PBMCs were labeled with ef450 at a cell density of 1×10⁶ cells/ mL with ef450 (eBioscience, Cat No: 65-0842-85) at a final concentration of 2,5 µM diluted in PBS for 8 minutes at 37 °C. Thereafter, cells were washed twice with T cell medium. Labeled cells were rested in T-cell media at 37 °C for 30 minutes.

Mouse embryonic fibroblast NIH/3T3-huFAP clone 19 cells (Example 4.3.2.2) were harvested using cell dissociation buffer (Invitrogen, Cat.-No. 13151-014) for 10 minutes at 37 °C. Cells were washed once with PBS. Cells were resuspended at 10⁷/ml in PBS and irradiated in an xRay irradiator by a dose ao 5000 RAD to prevent later overgrowth of human PBMC by the tumor cell line. NIH/3T3-huFAP clone 39 cells were seeded at a density of 0.25^{∗}10⁵ cells per well in T cell media in a sterile 96-well flat bottom adhesion tissue culture plate (TPP, Cat. No. 92096) over night at 37 °C and 5% CO₂ in an incubator (Hera Cell 150).

Sixteen hours later, human, ef450 labelled PBMCs were added to each well at a density of 0.75^{∗}10⁵ cells per well. Anti-human CD3 antibody (eBioscience, Clone OKT3, Ref: 16-0037-85) at a final concentration of 0.12 ng/ml and FAP targeted mono- and bivalent anti-GITR antigen binding molecules were added at the indicated concentrations. Cells were cultured for six days at 37 °C and 5% CO₂ in an incubator (Hera Cell 150). Then, cells were surface-stained with fluorescent dye-conjugated antibodies anti-human CD4 (Clone L200, BD Pharmingen, Cat no: 550631), CD8 (clone RPa-T8, BioLegend, Cat.-No. 301044), CD25 (BC96, BioLegend, Cat.-No. 302632) and Zombie Aqua (Biolegend, Cat no: 423102) for live/dead discrimination, diluted in PBS and incubated for 30 min at 4 °C. Cells were resuspended in 140 µL/well FACS buffer plus 10 µL/well of absolute count beads (CountBright, Molecular Probes, C36950), and acquired using a 5-laser Fortessa flow cytometer (BD Bioscience with DIVA software).

Zombie Aqua negative living cells were analyzed for decrease in median of fluorescence of ef450 as a marker for proliferation.

As shown in **Figures 57A and 57B****,** costimulation with non-targeted anti-GITR (GITR 8A06 DP47GS 4+1 sf w(1a)) (triangle) did not have an effect on the proliferative capacity of suboptimally TCR stimulated CD4 CD8 T cells. Hyper-crosslinking of the FAP targeted anti-GITR construct (GITR 8A06 28H1 4+1 sf W(1)) (circle) by the present NIH/3T3-huFAP cells is needed for T cell costimulation and clearly promoted proliferation.

## Claims

1. A bispecific antigen binding molecule, comprising
(a) four Fab fragments capable of specific binding to OX40,
(b) at least one moiety capable of specific binding to Fibroblast Activation Protein (FAP) comprising an antibody light chain variable region (VL) and an antibody heavy chain variable region (VH), and
(c) a Fc domain composed of a first and a second subunit capable of stable association, wherein the Fc domain is of human IgG1 subclass with the amino acid mutations L234A, L235A and P329G (numbering according to Kabat EU index), and
wherein each of the Fab fragments capable of specific binding to OX40 comprises a VH domain comprising CDR-H1 comprising the amino acid sequence of SEQ ID NO:2, CDR-H2 comprising the amino acid sequence of SEQ ID NO:4, CDR-H3 comprising the amino acid sequence of SEQ ID NO:7 and a VL domain comprising CDR-L1 comprising the amino acid sequence of SEQ ID NO: 13, CDR-L2 comprising the amino acid sequence of SEQ ID NO: 16 and CDR-L3 comprising the amino acid sequence of SEQ ID NO:20, and wherein
a first Fab fragment capable of specific binding to OX40 is fused at the C-terminus of the CH1 domain to the VH domain of a second Fab fragment capable of specific binding to OX40 and a third Fab fragment capable of specific binding to OX40 is fused at the C-terminus of the CH1 domain to the VH domain of a fourth Fab fragment capable of specific binding to OX40, optionally via a peptide linker and wherein each two of the four Fab fragments capable of specific binding to OX40 are further fused at its C-terminus to the N-terminus of one of the subunits of the Fc domain, optionally via a peptide linker, and
wherein the VH domain of the moiety capable of specific binding to FAP is connected via a peptide linker to the C-terminus of the first subunit of the Fc domain and the VL domain is connected via a peptide linker to the C-terminus of the second subunit of the Fc domain or wherein two moieties capable of specific binding to FAP are VH-VL crossover Fab fragments and are each fused at the N-terminus of the VL domain via a peptide linker to the C-terminus of one of the subunits of the Fc domain.

2. The bispecific antigen binding molecule of claim 1, wherein each of the Fab fragments capable of specific binding to OX40 comprises a heavy chain variable region VH comprising an amino acid sequence of SEQ ID NO:27 and a light chain variable region VL comprising an amino acid sequence of SEQ ID NO:28.

3. The bispecific antigen binding molecule of claims 1 or 2, wherein the moiety capable of specific binding to FAP comprises a VH domain comprising
(i) a CDR-H1 comprising the amino acid sequence selected from the group consisting of SEQ ID NO:39 and SEQ ID NO:40,
(ii) a CDR-H2 comprising the amino acid sequence selected from the group consisting of SEQ ID NO:41 and SEQ ID NO:42, and
(iii) a CDR-H3 comprising the amino acid sequence selected from the group consisting of SEQ ID NO:43 and SEQ ID NO:44,
and a VL domain comprising
(iv) a CDR-L1 comprising the amino acid sequence selected from the group consisting of SEQ ID NO:45 and SEQ ID NO:46,
(v) a CDR-L2 comprising the amino acid sequence selected from the group consisting of SEQ ID NO:47 and SEQ ID NO:48, and
(vi) a CDR-L3 comprising the amino acid sequence selected from the group consisting of SEQ ID NO:49 and SEQ ID NO:50.

4. The bispecific antigen binding molecule of any one of claims 1 to 3, wherein
(i) each of the Fab fragments capable of specific binding to OX40 comprises a heavy chain variable region VH comprising an amino acid sequence of SEQ ID NO: 27 and a light chain variable region VL comprising an amino acid sequence of SEQ ID NO: 28 and
(ii) the moiety capable of specific binding to FAP comprises a heavy chain variable region VH comprising an amino acid sequence of SEQ ID NO:51 or SEQ ID NO:53 and a light chain variable region VL comprising an amino acid sequence of SEQ ID NO:52 or SEQ ID NO:54.

5. The bispecific antigen binding molecule of any one of claims 1 to 4, wherein in the Fab fragments capable of specific binding to OX40 in the constant domain CL the amino acid at position 124 is substituted independently by lysine (K), arginine (R) or histidine (H) (numbering according to Kabat EU Index), and in the constant domain CH1 the amino acids at positions 147 and 213 are substituted independently by glutamic acid (E) or aspartic acid (D) (numbering according to Kabat EU index).

6. The bispecific antigen binding molecule of any one of claims 1 to 5, wherein the bispecific antigen binding molecule is tetravalent for OX40 and monovalent for FAP.

7. The bispecific antigen binding molecule of any one of claims 1 to 6, wherein said antigen binding molecule comprises
(i) a first heavy chain comprising an amino acid sequence of SEQ ID NO:213, a second heavy chain comprising an amino acid sequence of SEQ ID NO:214, and a light chain comprising an amino acid sequence of SEQ ID NO:157, or
(ii) a first heavy chain comprising an amino acid sequence of SEQ ID NO:217, a second heavy chain comprising an amino acid sequence of SEQ ID NO:218, and a light chain comprising an amino acid sequence of SEQ ID NO:157.

8. The bispecific antigen binding molecule of any one of claims 1 to 5, wherein the bispecific antigen binding molecule is tetravalent for OX40 and bivalent for FAP.

9. A polynucleotide encoding the bispecific antigen binding molecule of any one of claims 1 to 8.

10. A vector comprising the isolated polynucleotide of claim 9.

11. A host cell comprising the isolated polynucleotide of claim 9 or the vector of claim 10.

12. A method for producing a bispecific antigen binding molecule of any one of claims 1 to 8, comprising the steps of (i) culturing the host cell of claim 11 under conditions suitable for expression of the antigen binding molecule, and (ii) recovering the antigen binding molecule.

13. A pharmaceutical composition comprising a bispecific antigen binding molecule of any one of claims 1 to 8, and at least one pharmaceutically acceptable excipient.

14. The bispecific antigen binding molecule of any one of claims 1 to 8, or the pharmaceutical composition of claim 13, for use as a medicament.

15. The bispecific antigen binding molecule of any one of claims 1 to 8, or the pharmaceutical composition of claim 13, for use in the treatment of cancer.

16. The bispecific antigen binding molecule of any one of claims 1 to 8, or the pharmaceutical composition of claim 13, for use in the treatment of cancer, wherein the bispecific antigen binding molecule is administered in combination with a chemotherapeutic agent, radiation and/ or other agents for use in cancer immunotherapy.

## Patentansprüche

1. Bispezifisches antigenbindendes Molekül, umfassend
(a) vier Fab-Fragmente, die spezifisch an OX40 binden können,
(b) mindestens eine Einheit, die spezifisch an Fibroblastenaktivierungsprotein (FAP) binden kann, umfassend eine variable Region der leichten Kette (VL) eines Antikörpers und eine variable Region der schweren Kette (VH) eines Antikörpers, und
(c) eine Fc-Domäne, bestehend aus einer ersten und einer zweiten Untereinheit, die zu stabiler Assoziation fähig sind, wobei die Fc-Domäne der humanen IgG1-Unterklasse mit den Aminosäuremutationen L234A, L235A und P329G (Nummerierung gemäß Kabat-EU-Index) angehört, und
wobei jedes der Fab-Fragmente, die spezifisch an OX40 binden können, eine VH-Domäne, umfassend CDR-H1, umfassend die Aminosäuresequenz von SEQ ID NO:2, CDR-H2, umfassend die Aminosäuresequenz von SEQ ID NO:4, CDR-H3, umfassend die Aminosäuresequenz von SEQ ID NO:7, und eine VL-Domäne, umfassend CDR-L1, umfassend die Aminosäuresequenz von SEQ ID NO:13, CDR-L2, umfassend die Aminosäuresequenz von SEQ ID NO:16, und CDR-L3, umfassend die Aminosäuresequenz von SEQ ID NO:20, umfasst, und wobei ein erstes Fab-Fragment, das spezifisch an OX40 binden kann, an dem C-Terminus der CH1-Domäne an die VH-Domäne eines zweiten Fab-Fragments, das spezifisch an OX40 binden kann, fusioniert ist und ein drittes Fab-Fragment, das spezifisch an OX40 binden kann, an dem C-Terminus der CH1-Domäne an die VH-Domäne eines vierten Fab-Fragments, das spezifisch an OX40 binden kann, fusioniert ist, gegebenenfalls über einen Peptid-Linker, und wobei jeweils zwei der vier Fab-Fragmente, die spezifisch an OX40 binden können, ferner an ihrem C-Terminus gegebenenfalls über einen Peptid-Linker an den N-Terminus einer der Untereinheiten der Fc-Domäne fusioniert sind und wobei die VH-Domäne der Einheit, die spezifisch an FAP binden kann, über einen Peptid-Linker mit dem C-Terminus der ersten Untereinheit der Fc-Domäne verbunden ist und die VL-Domäne über einen Peptid-Linker mit dem C-Terminus der zweiten Untereinheit der Fc-Domäne verbunden ist oder wobei zwei Einheiten, die spezifisch an FAP binden können, VH-VL-Crossover-Fab-Fragmente sind und jeweils an dem N-Terminus der VL-Domäne über einen Peptid-Linker an den C-Terminus einer der Untereinheiten der Fc-Domäne fusioniert sind.

2. Bispezifisches antigenbindendes Molekül nach Anspruch 1, wobei jedes der Fab-Fragmente, die spezifisch an OX40 binden können, eine variable Region der schweren Kette VH, umfassend eine Aminosäuresequenz von SEQ ID NO:27, und eine variable Region der leichten Kette VL, umfassend eine Aminosäuresequenz von SEQ ID NO:28, umfasst.

3. Bispezifisches antigenbindendes Molekül nach den Ansprüchen 1 oder 2, wobei die Einheit, die spezifisch an FAP binden kann, eine VH-Domäne, umfassend
(i) eine CDR-H1, umfassend die Aminosäuresequenz, die aus der Gruppe ausgewählt ist, bestehend aus SEQ ID NO:39 und SEQ ID NO:40,
(ii) eine CDR-H2, umfassend die Aminosäuresequenz, die aus der Gruppe ausgewählt ist, bestehend aus SEQ ID NO:41 und SEQ ID NO:42, und
(iii) eine CDR-H3, umfassend die Aminosäuresequenz, die aus der Gruppe ausgewählt ist, bestehend aus SEQ ID NO:43 und SEQ ID NO:44,
und eine VL-Domäne, umfassend
(iv) eine CDR-L1, umfassend die Aminosäuresequenz, die aus der Gruppe ausgewählt ist, bestehend aus SEQ ID NO:45 und SEQ ID NO:46,
(v) eine CDR-L2, umfassend die Aminosäuresequenz, die aus der Gruppe ausgewählt ist, bestehend aus SEQ ID NO:47 und SEQ ID NO:48, und
(vi) eine CDR-L3, umfassend die Aminosäuresequenz, die aus der Gruppe ausgewählt ist, bestehend aus SEQ ID NO:49 und SEQ ID NO:50,
umfasst.

4. Bispezifisches antigenbindendes Molekül nach einem der Ansprüche 1 bis 3, wobei
(i) jedes der Fab-Fragmente, die spezifisch an OX40 binden können, eine variable Region der schweren Kette VH, umfassend eine Aminosäuresequenz von SEQ ID NO:27, und eine variable Region der leichten Kette VL, umfassend eine Aminosäuresequenz von SEQ ID NO:28, umfasst und
(ii) die Einheit, die spezifisch an FAP binden kann, eine variable Region der schweren Kette VH, umfassend eine Aminosäuresequenz von SEQ ID NO:51 oder SEQ ID NO:53, und eine variable Region der leichten Kette VL, umfassend eine Aminosäuresequenz von SEQ ID NO:52 oder SEQ ID NO:54 umfasst.

5. Bispezifisches antigenbindendes Molekül nach einem der Ansprüche 1 bis 4, wobei in den Fab-Fragmenten, die spezifisch an OX40 binden können, in der konstanten Domäne CL die Aminosäure an Position 124 unabhängig voneinander durch Lysin (K), Arginin (R) oder Histidin (H) (Nummerierung gemäß Kabat-EU-Index) substituiert ist, und in der konstanten Domäne CH1 die Aminosäuren an Positionen 147 und 213 unabhängig voneinander durch Glutaminsäure (E) oder Asparaginsäure (D) (Nummerierung gemäß Kabat-EU-Index) substituiert sind.

6. Bispezifisches antigenbindendes Molekül nach einem der Ansprüche 1 bis 5, wobei das bispezifische antigenbindende Molekül tetravalent für OX40 und monovalent für FAP ist.

7. Bispezifisches antigenbindendes Molekül nach einem der Ansprüche 1 bis 6, wobei das antigenbindende Molekül
(i) eine erste schwere Kette, umfassend eine Aminosäuresequenz von SEQ ID NO:213, eine zweite schwere Kette, umfassend eine Aminosäuresequenz von SEQ ID NO:214, und eine leichte Kette, umfassend eine Aminosäuresequenz von SEQ ID NO: 157, oder
(ii) eine erste schwere Kette, umfassend eine Aminosäuresequenz von SEQ ID NO:217, eine zweite schwere Kette, umfassend eine Aminosäuresequenz von SEQ ID NO:218, und eine leichte Kette, umfassend eine Aminosäuresequenz von SEQ ID NO:157, umfasst.

8. Bispezifisches antigenbindendes Molekül nach einem der Ansprüche 1 bis 5, wobei das bispezifische antigenbindende Molekül tetravalent für OX40 und bivalent für FAP ist.

9. Polynukleotid, das für das bispezifische antigenbindende Molekül nach einem der Ansprüche 1 bis 8 kodiert.

10. Vektor, umfassend das isolierte Polynukleotid nach Anspruch 9.

11. Wirtszelle, umfassend das isolierte Polynukleotid nach Anspruch 9 oder den Vektor nach Anspruch 10.

12. Verfahren zum Produzieren eines bispezifischen antigenbindenden Moleküls nach einem der Ansprüche 1 bis 8, umfassend die Schritte (i) Kultivieren der Wirtszelle nach Anspruch 11 unter Bedingungen, die zur Expression des antigenbindenden Moleküls geeignet sind, und (ii) Gewinnen des antigenbindenden Moleküls.

13. Pharmazeutische Zusammensetzung, umfassend ein bispezifisches antigenbindendes Molekül nach einem der Ansprüche 1 bis 8 und mindestens einen pharmazeutisch unbedenklichen Hilfsstoff.

14. Bispezifisches antigenbindendes Molekül nach einem der Ansprüche 1 bis 8 oder pharmazeutische Zusammensetzung nach Anspruch 13 zur Verwendung als ein Medikament.

15. Bispezifisches antigenbindendes Molekül nach einem der Ansprüche 1 bis 8 oder pharmazeutische Zusammensetzung nach Anspruch 13 zur Verwendung bei der Behandlung von Krebs.

16. Bispezifisches antigenbindendes Molekül nach einem der Ansprüche 1 bis 8 oder pharmazeutische Zusammensetzung nach Anspruch 13 zur Verwendung bei der Behandlung von Krebs, wobei das bispezifische antigenbindende Molekül in Kombination mit einem Chemotherapeutikum, Bestrahlung und/oder anderen Mitteln zur Verwendung in der Krebsimmuntherapie verabreicht wird.

## Revendications

1. Molécule bispécifique de liaison à l'antigène, comprenant
(a) quatre fragments Fab capables de se lier spécifiquement à OX40,
(b)au moins une fraction capable de se lier spécifiquement à la protéine d'activation des fibroblastes (FAP) comprenant une région variable de chaîne légère (VL) d'anticorps et une région variable de chaîne lourde (VH) d'anticorps, et
(c)un domaine Fc composé d'un premier et d'un second sous-motif capables d'une association stable, le domaine Fc étant de la sous-classe IgG1 humaine avec les mutations d'acides aminés L234A, L235A et P329G (numérotation selon l'indice EU de Kabat), et
dans laquelle chacun des fragments Fab capables de se lier spécifiquement à OX40 comprend un domaine VH comprenant une CDR-H1 comprenant la séquence d'acides aminés de SEQ ID NO : 2, une CDR-H2 comprenant la séquence d'acides aminés de SEQ ID NO : 4, une CDR-H3 comprenant la séquence d'acides aminés de SEQ ID NO : 7 et un domaine VL comprenant une CDR-L1 comprenant la séquence d'acides aminés de SEQ ID NO : 13, une CDR-L2 comprenant la séquence d'acides aminés de SEQ ID NO : 16 et une CDR-L3 comprenant la séquence d'acides aminés de SEQ ID NO : 20, et dans laquelle un premier fragment Fab capable de se lier spécifiquement à OX40 est fusionné au niveau de l'extrémité C-terminale du domaine CH1 au domaine VH d'un deuxième fragment Fab capable de se lier spécifiquement à OX40 et un troisième fragment Fab capable de se lier spécifiquement à OX40 est fusionné au niveau de l'extrémité C-terminale du domaine CH1 au domaine VH d'un quatrième fragment Fab capable de se lier spécifiquement à OX40, éventuellement au moyen d'un lieur peptidique et dans laquelle chaque paire des quatre fragments Fab capables de se lier spécifiquement à OX40 est en outre fusionnée au niveau de son extrémité C-terminale à l'extrémité N-terminale de l'un des sous-motifs du domaine Fc, éventuellement au moyen d'un lieur peptidique, et
dans laquelle le domaine VH de la fraction capable de se lier spécifiquement à FAP est lié au moyen d'un lieur peptidique à l'extrémité C-terminale du premier sous-motif du domaine Fc et le domaine VL est lié au moyen d'un lieur peptidique à l'extrémité C-terminale du second sous-motif du domaine Fc ou dans laquelle deux fractions capables de se lier spécifiquement à FAP sont des fragments Fab croisés VH-VL et sont chacun fusionnés au niveau de l'extrémité N-terminale du domaine VL au moyen d'un lieur peptidique à l'extrémité C-terminale de l'un des sous-motifs du domaine Fc.

2. Molécule bispécifique de liaison à l'antigène selon la revendication 1, dans laquelle chacun des fragments Fab capables de se lier spécifiquement à OX40 comprend une région variable de chaîne lourde VH comprenant une séquence d'acides aminés de SEQ ID NO : 27 et une région variable de chaîne légère VL comprenant une séquence d'acides aminés de SEQ ID NO : 28.

3. Molécule bispécifique de liaison à l'antigène selon les revendications 1 ou 2, dans laquelle la fraction capable de se lier spécifiquement à FAP comprend un domaine VH comprenant
(i) une CDR-H1 comprenant la séquence d'acides aminés choisie dans le groupe constitué par la SEQ ID NO : 39 et la SEQ ID NO : 40,
(ii) une CDR-H2 comprenant la séquence d'acides aminés choisie dans le groupe constitué par la SEQ ID NO : 41 et la SEQ ID NO : 42, et
(iii) une CDR-H3 comprenant la séquence d'acides aminés choisie dans le groupe constitué par la SEQ ID NO : 43 et la SEQ ID NO : 44,
et un domaine VL comprenant
(iv) une CDR-L1 comprenant la séquence d'acides aminés choisie dans le groupe constitué par la SEQ ID NO : 45 et la SEQ ID NO : 46,
(v) une CDR-L2 comprenant la séquence d'acides aminés choisie dans le groupe constitué par la SEQ ID NO : 47 et la SEQ ID NO : 48, et
(vi) une CDR-L3 comprenant la séquence d'acides aminés choisie dans le groupe constitué par la SEQ ID NO : 49 et la SEQ ID NO : 50.

4. Molécule bispécifique de liaison à l'antigène selon l'une quelconque des revendications 1 à 3, dans laquelle
(i) chacun des fragments Fab capables de se lier spécifiquement à OX40 comprend une région variable de chaîne lourde VH comprenant une séquence d'acides aminés de SEQ ID NO : 27 et une région variable de chaîne légère VL comprenant une séquence d'acides aminés de SEQ ID NO : 28 et
(ii) la fraction capable de se lier spécifiquement à FAP comprend une région variable de chaîne lourde VH comprenant une séquence d'acides aminés de SEQ ID NO : 51 ou de SEQ ID NO : 53 et une région variable de chaîne légère VL comprenant une séquence d'acides aminés de SEQ ID NO : 52 ou de SEQ ID NO : 54.

5. Molécule bispécifique de liaison à l'antigène selon l'une quelconque des revendications 1 à 4, dans laquelle dans les fragments Fab capables de se lier spécifiquement à OX40 dans le domaine constant CL l'acide aminé en position 124 est substitué indépendamment par la lysine (K), l'arginine (R) ou l'histidine (H) (numérotation selon l'indice EU de Kabat), et dans le domaine constant CH1 les acides aminés en positions 147 et 213 sont substitués indépendamment par l'acide glutamique (E) ou l'acide aspartique (D) (numérotation selon l'indice EU de Kabat).

6. Molécule bispécifique de liaison à l'antigène selon l'une quelconque des revendications 1 à 5, dans laquelle la molécule bispécifique de liaison à l'antigène est tétravalente pour OX40 et monovalente pour FAP.

7. Molécule bispécifique de liaison à l'antigène selon l'une quelconque des revendications 1 à 6, dans laquelle ladite molécule de liaison à l'antigène comprend
(i) une première chaîne lourde comprenant une séquence d'acides aminés de SEQ ID NO : 213, une seconde chaîne lourde comprenant une séquence d'acides aminés de SEQ ID NO : 214 et une chaîne légère comprenant une séquence d'acides aminés de SEQ ID NO : 157, ou
(ii) une première chaîne lourde comprenant une séquence d'acides aminés de SEQ ID NO : 217, une seconde chaîne lourde comprenant une séquence d'acides aminés de SEQ ID NO : 218 et une chaîne légère comprenant une séquence d'acides aminés de SEQ ID NO : 157.

8. Molécule bispécifique de liaison à l'antigène selon l'une quelconque des revendications 1 à 5, dans laquelle la molécule bispécifique de liaison à l'antigène est tétravalente pour OX40 et bivalente pour FAP.

9. Polynucléotide codant pour la molécule bispécifique de liaison à l'antigène selon l'une quelconque des revendications 1 à 8.

10. Vecteur comprenant le polynucléotide isolé selon la revendication 9.

11. Cellule hôte comprenant le polynucléotide isolé selon la revendication 9 ou le vecteur selon la revendication 10.

12. Procédé de production d'une molécule bispécifique de liaison à l'antigène selon l'une quelconque des revendications 1 à 8, comprenant les étapes de (i) culture de la cellule hôte selon la revendication 11 dans des conditions appropriées pour l'expression de la molécule de liaison à l'antigène et (ii) récupération de la molécule de liaison à l'antigène.

13. Composition pharmaceutique comprenant une molécule bispécifique de liaison à l'antigène selon l'une quelconque des revendications 1 à 8 et au moins un excipient pharmaceutiquement acceptable.

14. Molécule bispécifique de liaison à l'antigène selon l'une quelconque des revendications 1 à 8, ou composition pharmaceutique selon la revendication 13, pour une utilisation comme médicament.

15. Molécule bispécifique de liaison à l'antigène selon l'une quelconque des revendications 1 à 8, ou composition pharmaceutique selon la revendication 13, pour une utilisation dans le traitement d'un cancer.

16. Molécule bispécifique de liaison à l'antigène selon l'une quelconque des revendications 1 à 8, ou composition pharmaceutique selon la revendication 13, pour une utilisation dans le traitement d'un cancer, dans laquelle la molécule bispécifique de liaison à l'antigène est administrée en association avec un agent chimiothérapeutique, des rayonnements et/ou d'autres agents pour une utilisation dans l'immunothérapie anticancéreuse.
